(19) Europäisches Patentamt
European Patent Office
Office européen des brevets

(11) EP 1 730 147 B1

(12) # EUROPÄISCHE PATENTSCHRIFT

(45) Veröffentlichungstag und Bekanntmachung des Hinweises auf die Patenterteilung:
**03.02.2010 Patentblatt 2010/05**

(21) Anmeldenummer: **05716285.1**

(22) Anmeldetag: **22.03.2005**

(51) Int Cl.:
***C07D 471/10*** *(2006.01)* ***A61K 31/435*** *(2006.01)*
***A61P 3/00*** *(2006.01)*

(86) Internationale Anmeldenummer:
**PCT/EP2005/003029**

(87) Internationale Veröffentlichungsnummer:
**WO 2005/095402 (13.10.2005 Gazette 2005/41)**

(54) **SUBSTITUIERTE 1,4,8-TRIAZASPIRO[4.5 DECAN-2-ON-VERBINDUNGEN ZUR BEHANDLUNG VON FETTSUCHT**

SUBSTITUTED 1,4,8-TRIAZASPIRO[4,5]DECAN-2-ON COMPOUNDS FOR USE IN THE TREATMENT OF OBESITY

COMPOSES 1,4,8-TRIAZASPIRO[4.5]DECAN-2-ONE SUBSTITUES POUR TRAITER L'OBESITE

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**LV**

(30) Priorität: **22.03.2004 DE 102004014296**

(43) Veröffentlichungstag der Anmeldung:
**13.12.2006 Patentblatt 2006/50**

(73) Patentinhaber: **Grünenthal GmbH**
**52078 Aachen (DE)**

(72) Erfinder:
- **SUNDERMANN, Corinna**
**52074 Aachen (DE)**
- **SUNDERMANN, Bernd**
**52074 Aachen (DE)**
- **OBERBÖRSCH, Stefan**
**52074 Aachen (DE)**
- **ENGLBERGER, Werner**
**52223 Stolberg (DE)**
- **HENNIES, Hagen-Heinrich**
**52152 Simmerath (DE)**

(74) Vertreter: **Brosch, Oliver et al**
**Kutzenberger & Wolff**
**Theodor-Heuss-Ring 23**
**50668 Köln (DE)**

(56) Entgegenhaltungen:
**EP-A- 0 921 125 EP-A- 0 963 985**

- **ISHIHARA Y ET AL: "CENTRAL CHOLINERGIC AGENTS. SYNTHESIS OF 2-ALKOXY-2,8-DIAZASPIRO U4.5 DECANE-1,3-DIONES AS MUSCARINIC AGONISTS" CHEMICAL AND PHARMACEUTICAL BULLETIN, PHARMACEUTICAL SOCIETY OF JAPAN, TOKYO, JP, Bd. 40, Nr. 5, 1. Mai 1992 (1992-05-01), Seiten 1177-1185, XP000651676 ISSN: 0009-2363**
- **PATENT ABSTRACTS OF JAPAN Bd. 013, Nr. 515 (C-655), 17. November 1989 (1989-11-17) & JP 01 207291 A (TAKEDA CHEM IND LTD), 21. August 1989 (1989-08-21)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft substituierte 1,4,8-Triazaspiro[4.5]decan-2-on-Verbindungen, Verfahren zu deren Herstellung, Arzneimittel enthaltend diese Verbindung sowie die Verwendung dieser Verbindungen zur Herstellung von Arzneimitteln.

Fettsucht und daraus resultierende Störungen und Erkrankungen wie beispielsweise Herzerkrankungen stellen ein ernstzunehmendes und stetig wachsendes Problem für die Gesundheit großer Bevölkerungsgruppen, insbesondere in den hochentwickelten Industrienationen dar. Neben weiteren Einflußfaktoren wie Bewegungsmangel und ungesunder Ernährung beeinflußt auch die Hemmung der 5-Hydroxy-tryptophan-(Serotonin) Serotonin-Wiederaufnahme die Entstehung und den Verlauf dieser Krankheit. Es besteht daher ein großer Bedarf an geeigneten pharmakologischen Wirkstoffen, die die 5-Hydroxy-tryptophan- (Serotonin) Serotonin-Wiederaufnahme hemmen und sich daher insbesondere zur Therapie von Fettsucht eignen. Aus JP 01 207291 A und Y. Ishihara et al. (Chem. Pharm. Bull. 1992, 40 (5): 1177-1185) sind substituierte Triazaspirodecanon-Verbindungen bekannt, die pharmakologisch aktiv sind.

Eine Aufgabe der vorliegenden Erfindung bestand daher darin, Verbindungen zur Verfügung zu stellen, die sich insbesondere als pharmazeutische Wirkstoffe in Arzneimitteln, bevorzugt in Arzneimitteln zur Prophylaxe und/oder Behandlung von Fettsucht, eignen.

Es wurde nun überraschenderweise gefunden, daß die substituierten 1,4,8-Triazaspiro[4.5]decan-2-on-Verbindungen der nachstehend angegebenen allgemeinen Formel I sich nicht nur zur Regulation, vorzugsweise zur Inhibierung, der Serotonin (5-Hydroxytryptophan)-Wiederaufnahme und/oder zur Regulation, vorzugsweise zur Inhibierung, der Noradrenalin-Wiederaufnahme eignen, sondern darüber hinaus auch eine hohe Affinität für Opioid-Rezeptoren, insbesondere für μ-Opioid-Rezeptoren sowie für Batrachotoxin-(BTX)-Rezeptoren aufweisen und sich daher insbesondere als pharmazeutische Wirkstoffe in Arzneimitteln zur Prophylaxe und/oder Behandlung von mit diesen Rezeptoren bzw. Prozessen in Verbindung stehenden Erkrankungen eignen

Ein Gegenstand der vorliegenden Erfindung sind daher substituierte 1,4,8-Triazaspiro[4.5]decan-2-on-Verbindungen der allgemeinen Formel I,

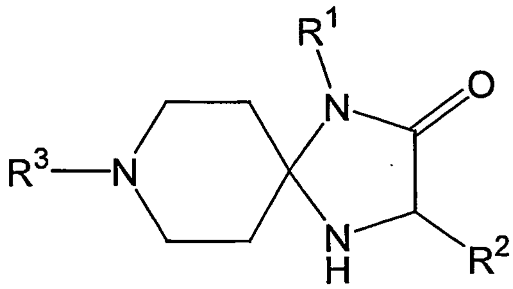

I

worin

$R^1$ für einen Wasserstoff-Rest, für einen linearen oder verzweigten, ggf. wenigstens ein Heteroatom als Kettenglied aufweisenden unsubstituierten Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, ggf. wenigstens ein Heteroatom als Kettenglied aufweisende Alkylen-Gruppe gebunden ist, für einen über eine lineare oder verzweigte Alkylen-Gruppe gebundenen $-C(=O)-OR^5$-Rest oder für einen über eine lineare oder verzweigte Alkylen-Gruppe gebundenen $-O-(C=O)-R^6$-Rest steht,

$R^2$ für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, oder für einen unsubstituierten oder wenigstens einfach substituierten Aryl- oder Heteroaryl-Rest steht, der über eine lineare oder verzweigte, ggf. wenigstens ein Heteroatom als Kettenglied aufweisende Alkylen-Gruppe gebunden sein kann,

$R^3$ für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden und gegebenenfalls mit einem unsubstituierten oder wenigstens einfach substituierten monozyklischen Ringsystem kondensiert ist, für einen unsubstituierten oder wenigstens einfach substituierten, ggf. wenigstens ein Heteroatom als Ringglied aufweisenden, ggf. über eine lineare oder verzweigte, unsubstituierte oder wenigstens einfach substituierte, ggf. wenigstens ein Heteroatom als Kettenglied aufweisende Alkylen-Gruppe gebundenen cycloaliphatischen Rest, der mit einer linearen oder verzweigten, unsubstituierten oder wenigstens einfach substituierten, ggf. wenigstens ein Heteroatom als Kettenglied aufweisenden Alkylen-Gruppe überbrückt und/oder mit einem unsubstituierten oder wenigstens einfach substituierten monozyklischen Ringsystem kondensiert sein kann, oder für eine - C(=O)-$R^4$-Gruppe steht,

$R^4$ für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest,

für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten monozyklischen Ringsystem kondensiert sein kann,

für einen unsubstituierten oder wenigstens einfach substituierten, ggf. wenigstens ein Heteroatom als Ringglied aufweisenden, ggf. über eine lineare oder verzweigte, unsubstituierte oder wenigstens einfach substituierte, ggf. wenigstens ein Heteroatom als Kettenglied aufweisende Alkylen-Gruppe gebundenen cycloaliphatischen Rest, der mit einer linearen oder verzweigten, unsubstituierten oder wenigstens einfach substituierten Alkylen-Gruppe wenigstens einfach überbrückt und/oder mit einem unsubstituierten oder wenigstens einfach substituierten monozyklischen Ringsystem kondensiert sein kann,

für einen über eine lineare oder verzweigte, unsubstituierte oder wenigstens einfach substituierte Alkylen-Gruppe gebundenen -C(=O)-$OR^7$-Rest, für einen über eine lineare oder verzweigte, unsubstituierte oder wenigstens einfach substituierte Alkylen-Gruppe gebundenen -O-(C=O)-$R^8$-Rest oder für einen über eine lineare oder verzweigte, unsubstituierte oder wenigstens einfach substituierte Alkylen-Gruppe gebundenen -(C=O)-$R^9$-Rest steht,

$R^5$ und $R^6$, unabhängig voneinander, jeweils für einen linearen oder verzweigten Alkyl-Rest, für einen linearen oder verzweigten Alkenyl-Rest oder für einen linearen oder verzweigten Alkinyl-Rest stehen,

$R^7$ und $R^8$, unabhängig voneinander, jeweils für einen Wasserstoff-Rest, für einen linearen oder verzweigten Alkyl-Rest, für einen linearen oder verzweigten Alkenyl-Rest, für einen linearen oder verzweigten Alkinyl-Rest, oder für einen unsubstituierten oder wenigstens einfach substituierten Aryl- oder Heteroaryl-Rest stehen,

$R^9$ für einen linearen oder verzweigten Alkyl-Rest, für einen linearen oder verzweigten Alkenyl-Rest oder für einen linearen oder verzweigten Alkinyl-Rest steht,

jeweils ggf. in Form eines ihrer reinen Stereoisomeren, insbesondere Enantiomeren oder Diastemomeren, ihrer Racemate oder in Form einer Mischung von Stereoisomeren, Insbesondere der Enantiomeren und/oder Diastereomeren, in einem beilebigen Mischungsverhältnis, oder jeweils in Form entsprechender Salze, oder jeweils In Form entsprechender Solvate.

Sofern einer der Reste $R^1$ bis $R^9$ für einen Alkyl-, Alkenyl- oder Alkinyl-Rest steht oder einen solchen Rest aufweist, kann dieser Rest - sofern nicht anders angegeben - unsubstituiert oder einfach oder mehrfach substituiert sein, vorzugsweise mit 1, 2, 3, 4 oder 5 Substituenten substituiert sein, wobei die Substituenten Jeweils unabhängig voneinander ausgewählt werden können aus der Gruppe bestehend aus F, Cl, Br, I, $C_{1-6}$-Alkoxy, -$NO_2$, -OH, -SH, -(C=O)-OH, -(C=O)-O-$C_{1-4}$-Alkyl, -Q-(C=O)-$C_{1-4}$-Alkyl, -$CH_2$-O-$CH_2$-Phenyl, -CN, -$CF_3$, -$CHF_2$, -$CH_2$F. unsubstituiertem Phenyl und -$NR^aR^b$, worin $R^a$ und $R^b$ jeweils unabhängig voneinander ausgewählt werden können aus der Gruppe bestehend aus H, Methyl, Ethyl, n-Propyl, iso-Propyl, n-Butyl, sec-Butyl, tert.-Butyl, -(C=O)-O-$C_{1-4}$-Alkyl und unsubstituiertem Phenyl. Alkenyl-Reste weisen wenigstens eine C-C-Doppelbindung und Alkinyl-Reste wenigstens eine C-C-Dreifachbindung auf.

Geeignete Alkyl-, Alkenyl- und Alkinyl-Reste, die einfach oder mehrfach substituiert sein können, können beispielsweise ausgewählt werden aus der Gruppe bestehend aus Methyl, Ethyl, n-Propyl, Isopropyl, n-Butyl, Isobutyl, sec-Butyl, tert-Butyl, n-Pentyl, iso-Pentyl, neo-Pentyl, sec-Pentyl, 2,2-Dimethyl-propyl, 1-Methyl-butyl, 1-Ethyl-propyl, 1-Propyl-butyl, 1-Ethyl-pentyl, Penta-1,3-dienyl, n-Hexyl, 2-Hexyl, n-Heptyl, n-Octyl, n-Nonyl, n-Decyl, -($CH_2$)-(CH)($C_2H_5$)-($CH_2$)-($CH_2$)-($CH_2$)-($CH_3$), Vinyl, Ethinyl, Propenyl, Alkyl, Propinyl, 1-Propinyl, 2-Propinyl, Butenyl, 1-Propenyl,

2-Propenyl, 1-Butenyl, 2-Butenyl, 3-Butenyl, 2-Methyl-2-butenyl, Butinyl, 1-Butinyl, 2-Butinyl, 3-Butinyl, Pentenyl, Pentinyl, Hexenyl und Hexinyl.

Die erfindungsgemäß vorliegenden Alkyl-, Alkenyl- und Alkinyl-Reste könnensofern nicht anders angegeben - auch eines oder mehrere Heteroatome, vorzugsweise eines oder mehrere Sauerstoffatome und/oder eines oder mehrere Schwefelatome, besonders bevorzugt 1 oder 2 Sauerstoffatome und/oder 1 oder 2 Schwefelatome, als Kettenglied (er) aufweisen. Vorzugsweise befinden sich diese Heteroatome in einer nicht-endständigen Position des Jeweiligen Restes. Beispielhaft seien Reste wie $-CH_2-CH_2-S-CH_3$, $-CH_2-O-CH_2-CH_2-O-CH_3$ oder $-CH_2-CH_2-O-CH_3$ genannt.

Sofern einer der Reste $R^3$ oder $R^4$ für einen cycloaliphatischen Rest steht oder einen cycloaliphatischen Rest aufweist, kann dieser Rest- sofern nicht anders angegebenunsubstituiert oder einfach oder mehrfach substituiert sein, vorzugsweise mit 1, 2, 3, 4 oder 5 Substituenten substituiert sein, wobei die Substituenten jeweils unabhängig voneinander ausgewählt werden können aus der Gruppe bestehend aus F, Cl, Br, $C_{1-6}$-Alkoxy, Oxo, $C_{1-6}$-Alkyl, Hydroxy, -CN, $-CF_3$, $-CHF_2$, $CH_2F$, unsubstituiertem Phenyl, $-NR^aR^b$, worin $R^a$ und $R^b$ jeweils unabhängig voneinander ausgewählt werden können aus der Gruppe bestehend aus H, Methyl, Ethyl, n-Propyl, iso-Propyl und unsubstituiertem Phenyl, Thioxo (=S), I, $-SF_5$, $-NO_2$, $-O-CF_3$,$-S-CF_3$, -SH, -S-$C_{1-5}$-Alkyl, -C(=O)-H, -C(=O)-$C_{1-5}$-Alkyl, -C(=O)-OH, -C(=O)-O-$C_{1-5}$-Alkyl, -($CH_2$)-C(=O)-OH, -($CH_2$)-C(=O)-O-$C_{1-5}$-Alkyl, -($CH_2$)-Benzo[b]furanyl, -O-Phenyl, -O-Benzyl, Phenyl, Benzyl, Naphthyl und -($CH_2$)-Naphthyl, wobei Jeweils der zyklische Teil der Reste -O-Phenyl, -O-Benzyl, Phenyl, -($CH_2$)-Benzo[b]furanyl, Benzyl, Naphthyl und -($CH_2$)-Naphthyl mit 1, 2, 3, 4 oder 5 Substituenten unabhängig voneinander ausgewählt aus der Gruppe bestehend aus F, Cl, Br, -OH,-CF3, $-SF_5$, -CN, $-NO_2$, -O-$C_{1-5}$-Alkyl, -$C_{1-5}$-Alkyl, $-O-CF_3$, $-S-CF_3$, Phenyl und -O-Benzyl substituiert sein kann.

Unter cycloallphatischen Resten werden im Sinne der vorliegenden Erfindung sowohl gesättigte als auch ungesättigte Reste verstanden. Die cycloaliphatischen Reste können ggf. eines oder mehrere Heteroatome, vorzugsweise 1, 2, 3, 4 oder 5 Heteroatome unabhängig voneinander ausgewählt aus der Gruppe bestehend aus Stickstoff, Sauerstoff und Schwefel, als Ringglieder aufweisen.

Geeignete cycloaliphatische Reste, die einfach oder mehrfach substituiert sein können, können beispielsweise ausgewählt werden aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuryl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl.

Die cycloaliphatischen Reste können ferner wenigstens einfach mit einer linearen oder verzweigten Alkylen-Gruppe überbrückt sein, vorzugsweise mit ein oder zwei linearen oder verzweigten $C_{1-5}$-Alkylen-Gruppen überbrückt sein. Als Beispiele für derartige cycloaliphatischen Reste seien der 4,7,7-Trimethyl-3-oxo-2-oxa-bicyclo-[2.2.1]-heptyl-bzw. der Adamantyl-Rest genannt.

Sofern einer der Reste $R^1$-$R^4$, $R^7$ und $R^8$ für einen Aryl- oder Heteroaryl-Rest steht oder einen Aryl- oder Heteroaryl-Rest aufweist, kann dieser Aryl- oder Heteroaryl-Rest - sofern nicht anders angegeben - einfach oder mehrfach substituiert sein, vorzugsweise mit 1, 2, 3, 4 oder 5 Substituenten substiuiert sein, wobei die Substituenten jeweils unabhängig voneinander ausgewählt werden können aus der Gruppe bestehend aus F, Cl, Br, I, -CN, $-NO_2$, $C_{1-5}$-Alkyl, OH, SH, $C_{1-5}$-Alkoxy, $C_{1-5}$-Perfluoroalkoxy, -C(=O)-$C_{1-5}$-Alkyl, -C(=O)-O-$C_{1-5}$-Alkyl, -$(CH_2)_{1-3}$-O-(C=O)-Phenyl, -S-$C_{1-5}$-Alkyl, -$S(=O)_2$-$C_{1-6}$-alkyl, -$S(=O)_2$-$NH_2$, -NH-(C=O)-$CH_3$, -S-$CHF_2$, -S-$CH_2F$, -C(=O)- $C_{1-5}$-Perfluoroalkyl, $-CF_3$, $CHF_2$, $CH_2F$. Phenyl, Phenoxy und $-NR^aR^b$, worin $R^a$ und $R^b$ jeweils unabhängig voneinander ausgewählt werden können aus der Gruppe bestehend aus H, Methyl, Ethyl, n-Propyl, iso-Propyl und unsubstituiertem Phenyl, $-SF_5$, -O-$C_{2-5}$-Alkenyl, $-O-CF_3$, $-O-CHF_2$, $-O-CH_2F$, -C(=O)-OH, -O-C(=O)-$C_{1-5}$-Alkyl, -O-C(=O)-Phenyl, -($CH_2$)-O-C(=O)-$C_{1-5}$-Alkyl, -NH-$C_{1-5}$-Alkyl, -N($C_{1-5}$-Alkyl$)_2$, -NH-C(=O)-O-$C_{1-5}$-Alkyl, -C(=O)-H, -C(=O)-$C_{1-5}$-Alkyl, -C(=O)-$NH_2$, -C(=O)-NH-$C_{1-5}$-Alkyl, C(=O)-N-($C_{1-5}$-Alkyl$)_2$, -$S(=O)_2$-NH-$C_{1-5}$-Alkyl, -$S(=O)_2$-NH-Phenyl, -($CH_2$)-Benzo[b]furanyl, -O-Phenyl, -S-Phenyl, -S-Benzyl, -O-Benzyl, Phenyl und Benzyl.

Der zyklische Teil der Reste -$S(=O)_2$-NH-Phenyl, -O-Phenyl, -S-Phenyl, -S-Benzyl, - O-Benzyl, Phenyl, -($CH_2$)-Benzo [b]furanyl und Benzyl kann mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -O-$C_{1-5}$-Alkyl, -$C_{1-5}$-Alkyl, $-O-CF_3$, $-S-CF_3$, Phenyl und -O-Benzyl substituiert sein.

Die Phenyl- und Phenoxy-Substituenten können selbst einfach oder mehrfach, gleich oder verschieden, mit einem Substituenten ausgewählt aus der Gruppe bestehend aus F, Cl, Br, $-S-CHF_2$, $-CF_3$, $-CHF_2$, $-CH_2F$, -CN, $-NO_2$, $C_{1-5}$-Alkyl und $C_{1-5}$-Alkoxy substituiert sein.

Die Heteroatome des Heteroaryl-Restes können bevorzugt ausgewählt werden aus der Gruppe bestehend aus Sauerstoff, Stickstoff und Schwefel. Vorzugsweise konnen die Heteroaryl-Reste 1, 2, 3, 4 oder 5 Heteroatom(e) unabhängig voneinander ausgewählt aus der Gruppe bestehend aus Sauerstoff, Stickstoff und Schwefel als Ringglied (er) aufweisen.

Geeignete Aryl-Reste können bevorzugt ausgewählt werden aus der Gruppe bestehend aus Phenyl-, 1-Naphthyl und 2-Naphthyl.

Geeignete Heteroaryl-Reste können bevorzugt ausgewählt werden aus der Gruppe bestehend aus Pyrrolyl,

Indolyl, Furyl (Furanyl), Benzofuranyl, Thienyl (Thiophenyl), Benzothienyl, Pyrazolyl, Imidazolyl, Thiazolyl, Thiadiazolyl, Triazolyl, Oxazolyl, Isoxazolyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl und Oxadiazolyl, Benzo[1,3]dioxolyl, Benzo [1,2,5]oxadiazolyl und Chromanyl und Isoindolyl.

Unter einem monozyklischen Ringsystem wird im Sinne der vorliegenden Erfindung ein monozyklischer Kohlenwasserstoffrest verstanden, der gesättigt, ungesättigt oder aromatisch sein und ggf. ein oder mehrere Heteroatome als Ringglieder aufweisen kann. Ein solches monozyklisches Ringsystem kann beispielsweise mit einem Aryl-Rest oder einem Heteroaryl-Rest kondensiert, d.h. anneliert bzw. verbunden sein.

Die Heteroatome eines solchen monozyklischen Ringsystems können jeweils bevorzugt ausgewählt werden aus der Gruppe bestehend aus Sauerstoff, Stickstoff und Schwefel. Vorzugsweise kann der Ring des Ringsystems 1, 2, 3, 4 oder 5 Heteroatom(e) unabhängig voneinander ausgewählt aus der Gruppe bestehend aus Sauerstoff, Stickstoff und Schwefel als Ringglied(er) aufweisen.

Vorzugsweise ist der Ring des Ringsystems 5- oder 6-gliedrig oder 7-gliedrig. Das Ringsystem kann einfach oder mehrfach substituiert sein, vorzugsweiste mit 1, 2, 3, 4 oder 5 Substituenten substituiert sein, wobei die Substituenten ausgewählt werden können aus der Gruppe bestehend aus F, Cl, Br, I, -CN, $-NO_2$, Oxo, $C_{1-5}$-Alkyl, $C_{1-6}$-Alkoxy, $C_{1-5}$-Perfluoralkoxy, -C(=O)-$C_{1-6}$-Alkyl, -C(=O)-O-$C_{1-5}$-Alkyl, $-S(=O)_2$-$C_{1-6}$-alkyl, -C(=O)- $C_{1-6}$-Perfluoroalkyl, $-CF_3$, $CHF_2$, $CH_2F$ und $-NR^aR^b$, worin $R^a$ und $R^b$ jeweils unabhängig voneinander ausgewählt werden können aus der Gruppe bestehend aus H, Methyl, Ethyl, n-Propyl, iso-Propyl und unsubstituiertem Phenyl, Thioxo (=S), $-SF_5$, -OH, -O-$C_{2-5}$-Alkenyl, $-NH_2$, $-NO_2$, $-O-CF_3$, $-O-CHF_2$, $-O-CH_2F$, $-S-CF_3$, $-S-CHF_2$, $-S-CH_2F$, -SH, -S-$C_{1-5}$-Alkyl, -C(=O)-OH, -O-C(=O)-$C_{1-5}$-Alkyl, -O-C(=O)-Phenyl, $-(CH_2)$-O-C(=O)-$C_{1-5}$-Alkyl, $-(CH_2)$-O-C(=O)-Phenyl, -NH-$C_{1-5}$-Alkyl, -N($C_{1-5}$-Alkyl)$_2$, -NH-C(=O)-O-$C_{1-5}$-Alkyl, -NH-C(=O)-$C_{1-5}$-Alkyl, -C(=O)-H, - C(=O)-$NH_2$, -C(=O)-NH-$C_{1-5}$-Alkyl, C(=O)-N-($C_{1-5}$-Alkyl)$_2$, $-S(=O)_2-NH_2$, $-S(=O)_2$-NH-$C_{1-5}$-Alkyl, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl, wobei Jeweils der zyklische Teil der Reste -O-C(=O)-Phenyl, $-(CH_2)$-O-C(=O)-Phenyl, $-S(=O)_2$-NH-Phenyl, -O-Phenyl, -S-Phenyl, - S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -O-$C_{1-5}$-Alkyl, -$C_{1-5}$-Alkyl, $-O-CF_3$, $-S-CF_3$, Phenyl und -O-Benzyl substituiert sein kann.

Weist einer der Reste $R^1$-$R^4$ eine lineare oder verzweigte Alkylen-, Alkenylen- oder Alkinylen-Gruppe auf, so kann diese - sofern nicht anders angegeben - unsubstituiert oder einfach oder mehrfach substituiert sein, vorzugsweise mit 1, 2, 3, 4 oder 5 Substituenten substituiert sein, wobei die Substituenten unabhängig voneinander ausgewählt werden können aus der Gruppe bestehend aus F, Cl, Br, $C_{1-6}$-Alkoxy, Hydroxy, CN, $CF_3$, $CHF_2$, $CH_2F$, unsubstitulertem Phenyl und $-NR^aR^b$, worin $R^a$ und $R^b$ jeweils unabhängig voneinander ausgewählt werden können aus der Gruppe bestehend aus H, $C_{1-3}$-Alkyl und unsubstituiertem Phenyl. Die Alkylen-Gruppe kann auch eines oder mehrere Heteroatome, vorzugsweise wenigstens ein Sauerstoffatom und/oder wenigstens ein Schwefelatom, besonders bevorzugt 1 oder 2 Sauerstoffatom(e) und/oder Schwefelatom(e), als Kettenglied(er) aufweisen. Eine Alkenylen-Gruppe weist wenigstens eine Kohlenstoff-Kohlenstoff-Doppelbindung, eine Alkinylen-Gruppe wenigstens eine Kohlenstoff-Kohlenstoff-Dreifachbindung auf.

Die jeweiligen Alkylen-, Alkenylen- oder Alkinylen- Gruppen können - sofern nicht anders angegeben - auch eines oder mehrere Heteroatome, vorzugsweise eines oder mehrere, beispielsweise 1 oder 2, Sauerstoffatome und/ oder eines oder mehrere, beispielsweise 1 oder 2, Schwefelatome, als Kettenglied(er) aufweisen.

Beispielhaft seien Alkylen-Gruppen wie $-(CH_2)-$, $-(CH(CH_3))-$, $-(CH_2)_2-$, $-(CH_2)_2-O-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$ und $-(CH_2)_{10}-$, Alkenylen-Gruppen wie -CH=CH- und Alkinylen-Gruppen wie -C≡C- genannt.

Bevorzugt sind substituierte 1,4,8-Triazaspiro[4.5]decan-2-on-Verbindungen der vorstehend angegebenen allgemeinen Formel I, in denen $R^1$ für einen Wasserstoff-Rest, für einen linearen oder verzweigten, unsubstituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest, für einen unsubstituierten oder wenigstens einfach substituierten, 5- bis 14-gliedrigen Aryl- oder Heteroaryl-Rest, der über eine lineare oder verzweigte, ggf. wenigstens ein Heteroatom als Kettenglied aufweisende $C_{1-5}$-Alkylen-Gruppe gebunden ist, für einen über eine lineare oder verzweigte $C_{1-5}$-Alkylen-Gruppe gebundenen -C(=O)-$OR^5$-Rest oder für einen über eine lineare oder verzweigte $C_{1-5}$-Alkylen-Gruppe gebundenen -O-C(=O)-$R^6$-Rest steht,

vorzugsweise für einen Wasserstoff-Rest, für einen linearen oder verzweigten, unsubstituierten $C_{1-5}$-Alkyl-Rest, für einen linearen oder verzweigten, unsubstituierten $C_{2-5}$-Alkenyl-Rest, für einen linearen oder verzweigten, unsubstituierten $C_{2-5}$-Alkinyl-Rest, für einen unsubstituierten oder wenigstens einfach substituierten Phenyl- oder Naphthyl-Rest, der über eine lineare oder verzweigte, ggf. eines oder mehrere Sauerstoffatome als Kettenglied aufweisende $C_{1-5}$-Alkylen-Gruppe gebunden ist, für einen über eine lineare oder verzweigte $C_{1-4}$-Alkylen-Gruppe gebundenen -C(=O)-$OR^5$-Rest oder für einen über eine lineare oder verzweigte $C_{1-4}$-Alkylen-Gruppe gebundenen -O-C(=O)-$R^6$-Rest steht,

besonders bevorzugt für einen Wasserstoff-Rest, für einen linearen oder verzweigten, unsubstituierten $C_{1-4}$-Alkyl-Rest, für einen linearen oder verzweigten, unsubstituierten $C_{2-5}$-Alkenyl-Rest, für einen linearen oder verzweigten, unsubstituierten $C_{2-3}$-Alkinyl-Rest, für einen Phenyl- oder Naphthyl-Rest, der über eine $-(CH_2)-$, $-(CH_2)_2-$, $-(CH_2)_3-$ oder $-(CH_2)_2$-O-Brücke gebunden und ggf. einfach oder mehrfach, gleich oder verschieden, mit einem Substituenten ausgewählt aus der Gruppe bestehend aus F, Cl, Br, CN, $NO_2$, $C_{1-5}$-Alkyl, $C_{1-5}$-Alkoxy, -C(=O)-$C_{1-5}$-Alkyl, -C(=O)-O-$C_{1-5}$-Alkyl, -S(=O)$_2$-$C_{1-6}$-alkyl, -C(=O)- $C_{1-5}$-Perfluoroalkyl, $-CF_3$, $CHF_2$ und $CH_2F$ substituiert ist, für einen über eine $-(CH_2)$-Gruppe gebundenen -C(=O)-OR$^5$-Rest oder für einen über eine $-(CH_2)-$, $-(CH_2)_2-$, $-(CH_2)_3-$ oder $-(CH_2)_4$-Gruppe gebundenen -O-C(=O)-R$^6$-Rest steht, und jeweils die übrigen Reste $R^2$-$R^9$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte 1,4,8-Triazaspiro[4.5]decan-2-on-Verbindungen der vorstehend angegebenen allgemeinen Formel I, worin der Rest $R^2$ für einen Wasserstoff-Rest, für einen linearen oder verzweigten, unsubstituierten oder wenigstens einfach substituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest, oder für einen unsubstituierten oder wenigstens einfach substituierten, 5- bis 14-gliedrigen Aryl- oder Heteroaryl-Rest steht, der über eine lineare oder verzweigte, ggf. wenigstens ein Heteroatom als Kettenglied aufweisende $C_{1-5}$-Alkylen-Gruppe gebunden sein kann,
vorzugsweise für einen Wasserstoff-Rest, für einen linearen oder verzweigten, unsubstituierten, ggf. eines oder mehrere Sauerstoffatome und/oder eines oder mehrere Schwefelatome als Kettenglied aufweisenden $C_{1-5}$-Alkyl-Rest oder für einen Phenyl- oder Naphthyl-Rest steht, der einfach oder mehrfach, gleich oder verschieden, mit einem Substituenten ausgewählt aus der Gruppe bestehend aus F, Cl, Br, CN, $NO_2$, $C_{1-5}$-Alkyl, $C_{1-5}$-Alkoxy, -C(=O)-$C_{1-5}$-Alkyl, -C(=O)-O-$C_{1-5}$-Alkyl,-S(=O)$_2$-$C_{1-6}$-alkyl, -C(=O)- $C_{1-5}$-Perfluoroalkyl, $-CF_3$, $CHF_2$ und $CH_2F$ substituiert und/oder über eine $-(CH_2)-$, $-(CH_2)_2-$, $-(CH_2)_3-$ oder $-(CH_2)_2$-O-Brücke gebunden sein kann,
besonders bevorzugt für einen Wasserstoff-Rest, für einen linearen oder verzweigten, unsubstituierten, ggf. eines oder mehrere Schwefelatome als Kettenglied aufweisenden $C_{1-5}$-Alkyl-Rest oder für einen Phenyl-Rest steht, wobei der Phenyl-Rest einfach oder mehrfach, gleich oder verschieden, mit einem Substituenten ausgewählt aus der Gruppe bestehend aus F, Cl, Br, CN, $NO_2$, $C_{1-5}$-Alkyl, $C_{1-5}$-Alkoxy, -C(=O)-$C_{1-5}$-Alkyl, -C(=O)-O-$C_{1-5}$-Alkyl, -S(=O)$_2$-$C_{1-6}$-alkyl, -C(=O)-$C_{1-5}$-Perfluoroalkyl, $-CF_3$, $CHF_2$ und $CH_2F$ substituiert und/oder über eine $-(CH_2)$-Brücke gebunden sein kann, und jeweils die übrigen Reste $R^1$ und $R^3$ bis $R^9$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Des weiteren sind substituierte 1,4,8-Triazaspiro[4.5]decan-2-on-Verbindungen der vorstehend angegebenen allgemeinen Formel I bevorzugt, in denen $R^3$ für einen Wasserstoff-Rest, für einen linearen oder verzweigten, unsubstituierten oder wenigstens einfach substituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest, für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende $C_{1-5}$-Alkylen-Gruppe gebunden und gegebenenfalls mit einem unsubstituierten oder wenigstens einfach substituierten monozyklischen Ringsystem kondensiert ist, für einen unsubstituierten oder wenigstens einfach substituierten, ggf. wenigstens ein Heteroatom als Ringglied aufweisenden $C_{3-8}$-cycloaliphatischen Rest steht, der über eine lineare oder verzweigte, unsubstituierte oder wenigstens einfach substituierte, ggf. wenigstens ein Heteroatom als Kettenglied aufweisende $C_{1-5}$-Alkylen-Gruppe gebunden und/oder mit einem unsubstituierten oder wenigstens einfach substituierten monozyklischen Ringsystem kondensiert sein kann, oder für eine -C(=O)-R$^4$-Gruppe steht,
vorzugsweise für einen Wasserstoff-Rest, für einen Biphenyl-Rest, der über eine - $(CH_2)-$, $-(CH_2)_2-$ oder $-(CH_2)_3$-Brücke gebunden ist, oder für eine C(=O)-R$^4$-Gruppe steht,
besonders bevorzugt für eine -C(=O)-R$^4$-Gruppe steht, und jeweils die übrigen $R^1$, $R^2$ und $R^4$ bis $R^9$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind solche substituierten 1,4,8-Triazaspiro[4.5]decan-2-on-Verbindungen der vorstehend

angegebenen allgemeinen Formel I, in denen der Rest $R^4$
für einen linearen oder verzweigten, unsubstituierten oder wenigstens einfach substituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest,
für einen unsubstituierten oder wenigstens einfach substituierten 5- bis 14- gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende $C_{1-5}$-Alkylen-, $C_{2-5}$-Alkenylen oder $C_{2-5}$-Alkinylen-Gruppe gebunden und/oder mit einem unsubstituierten oder wenigstens einfach substituierten monozyklischen Ringsystem kondensiert sein kann,
für einen unsubstituierten oder wenigstens einfach substituierten, ggf. wenigstens ein Heteroatom als Ringglied aufweisenden, ggf. über eine lineare oder verzweigte, unsubstituierte oder wenigstens einfach substituierte, ggf. wenigstens ein Heteroatom als Kettenglied aufweisende $C_{1-6}$-Alkylen-Gruppe gebundenen $C_{3-8}$ cycloaliphatischen Rest, der mit einer linearen oder verzweigten, unsubstituierten oder wenigstens einfach substituierten $C_{1-6}$-Alkylen-Gruppe wenigstens einfach überbrückt und/oder mit einem unsubstituierten oder wenigstens einfach substituierten monozyklischen Ringsystem kondensiert sein kann,
für einen über eine lineare oder verzweigte, unsubstituierte oder wenigstens einfach substituierte $C_{1-5}$-Alkylen-Gruppe gebundenen -C(=O)-$OR^7$-Rest, für einen über eine lineare oder verzweigte, unsubstituierte oder wenigstens einfach substituierte $C_{1-5}$-Alkylen-Gruppe gebundenen -O-(C=O)-$R^8$-Rest oder für einen über eine lineare oder verzweigte, unsubstituierte oder wenigstens einfach substituierte $C_{1-5}$-Alkylen-Gruppe gebundenen -(C=O)-$R^9$-Rest steht,
vorzugsweise für einen linearen oder verzweigten, ggf. eines oder mehrere Sauerstoffatome und/oder eines oder mehrere Schwefelatome als Kettenglied aufweisenden $C_{1-10}$-Alkyl-Rest, der unsubstituiert oder wenigstens einfach, gleich oder verschieden, mit einem Substituenten ausgewählt aus der Gruppe bestehend aus Phenyl, Di-($C_{1-5}$-Alkylamino), Carboxy und -NH-(C=O)-O-$C_{1-5}$-Alkyl substituiert sein kann, für einen linearen oder verzweigten, unsubstituierten $C_{2-5}$-Alkenyl-Rest, für einen Phenyl-Rest, Naphthyl-Rest, Furyl- (Furanyl-)-Rest, Thienyl-(Thiophenyl-)-Rest oder [1,2,3]-Triazolyl-Rest, Benzo[1.3]dioxolyl-Rest, Benzo[1,2,5]oxadiazolyl-Rest, Chromanyl-Rest, Pyrimidinyl-Rest, Pyrazolyl-Rest, Pyridinyl-Rest, Isoxazolyl-Rest oder [1,2,3]-Thiadiazolyl-Rest, wobei der zyklische Rest jeweils über eine -($CH_2$)-, - ($CH_2$)-O-, -CH=CH-, -$(CH_2)_2$-, -$(CH_2)_2$-O-, oder -$(CH_2)_3$-Brücke gebunden sein kann und/oder einfach oder mehrfach, gleich oder verschieden, mit einem Substituenten ausgewählt aus der Gruppe bestehend aus F, Cl, Br, I, -CN, -$NO_2$, $C_{1-5}$-Alkyl, $C_{1-5}$-Alkoxy, $C_{1-5}$-Perfluoralkoxy, -C(=O)-$C_{1-5}$-Alkyl, -C(=O)-O-$C_{1-5}$-Alkyl, -$(CH_2)_{1-3}$-O-C(=O)-Phenyl, -$N(CH_3)_2$, -S (=O)$_2$-$C_{1-6}$-alkyl, -S(=O)$_2$-$NH_2$, -C(=O)-$C_{1-5}$-Perfluoroalkyl, -S-$C_{1-5}$-Alkyl, -S-$CH_2F$, -S-$CHF_2$, -$SCF_3$, -$CF_3$, -NH-(C=O)-$CH_3$, -$SO_2$-$NH_2$, -$CHF_2$, -$CH_2F$, Phenyl und Phenoxy, wobei die letztgenannten Phenyl- und Phenoxy Substituenten selbst einfach oder mehrfach, gleich oder verschieden, mit einem Substituenten ausgewählt aus der Gruppe bestehend aus F, Cl, Br, -S-$CHF_2$, -$CF_3$, -$CHF_2$, -$CH_2F$, CN, $N0_2$, $C_{1-5}$-Alkyl und $C_{1-5}$-Alkoxy substituiert sein können,
für einen ggf. wenigstens ein Heteroatom als Ringglied aufweisenden, ggf. über eine -($CH_2$)-, -$(CH_2)_2$- oder -$(CH_2)_3$-Brükke gebundenen 3-, 4-, 5-, 6-, 7- oder 8-gliedrigen cycloaliphatischen Rest, der wenigstens einfach mit einer -(C $(CH_3)_2$)-oder -($CH_2$)-Gruppe überbrückt und/oder unsubstituiert oder einfach oder mehrfach, gleich oder verschieden, mit einem Substituenten ausgewählt aus der Gruppe bestehend aus $C_{1-5}$-Alkyl, $C_{1-5}$-Alkoxy, -$CF_3$, $CHF_2$, $CH_2F$ und Oxo substituiert sein kann,
oder für einen über eine lineare oder verzweigte, unsubstituierte oder wenigstens einfach substituierte $C_{1-5}$-Alkylen-Gruppe gebundenen -C(=O)-$OR^7$-Rest, für einen über eine lineare oder verzweigte, unsubstituierte oder wenigstens einfach substituierte $C_{1-5}$-Alkylen-Gruppe gebundenen -O-(C=O)-$R^8$-Rest, oder für einen über eine lineare oder verzweigte, unsubstituierte oder wenigstens einfach substituierte $C_{1-5}$-Alkylen-Gruppe gebundenen -(C=O)-$R^9$-Rest steht,
besonders bevorzugt für einen linearen oder verzweigten, ggf. eines oder mehrere Sauerstoffatome und/oder eines oder mehrere Schwefelatome als Kettenglied aufweisenden $C_{1-8}$-Alkyl-Rest, der unsubstituiert oder wenigstens einfach, gleich oder verschieden, mit einem Substituenten ausgewählt aus der Gruppe bestehend aus unsubstituiertem Phenyl, Dimethylamino, Carboxy und -NH-(C=O)-O-$C(CH_3)_3$ substituiert sein kann, für einen linearen oder verzweigten, unsubstituierten $C_{2-4}$-Alkenyl-Rest, für einen Phenyl-Rest, Naphthyl-Rest, Furyl- (Furanyl-)-Rest, Thienyl-(Thiophenyl-)-Rest, [1,2,3]-Triazolyl-Rest, Chromanyl-Rest, Benzo[1.3]dioxolyl-Rest, Benzo[1,2,5]oxadiazolyl-Rest, Pyrimidinyl-Rest, Pyrazolyl-Rest, Pyridinyl-Rest, Isoxazolyl-Rest oder [1,2,3]-Thiadiazolyl-Rest, wobei der zyklische Rest jeweils über eine -($CH_2$)-, -($CH_2$)-O-, -CH=CH-, -$(CH_2)_2$-, -$(CH_2)_2$-O-, oder -$(CH_2)_3$-Brücke gebunden sein kann und/oder einfach oder mehrfach, gleich oder verschieden, mit einem Substituenten ausgewählt aus der Gruppe bestehend aus F, Cl, Br, I, Methyl, Ethyl, iso-Propyl, n-Propyl, sec-Butyl, tert.-Butyl, n-Butyl, Methoxy, Ethoxy, -$CF_3$,-$OCF_3$, -$CH_2$-O-C(=O)-Phenyl, $NO_2$, -S-$CF_3$, -S-$CHF_2$, -$N(CH_3)_2$, -NH-CO-$CH_3$, CN, $SO_2$-$NH_2$, Phenyl und Phenoxy, wobei die letztgenannten Phenyl- und Phenoxy Substituenten selbst einfach oder mehrfach, gleich oder verschieden, mit einem Substituenten ausgewählt aus der Gruppe bestehend aus F, Cl, Br, $CH_3$ und $OCH_3$ substituiert sein können,
für einen ggf. über eine -($CH_2$)-, -$(CH_2)_2$- oder -$(CH_2)_3$-Brücke gebundenen Cyclopropyl, Cyclobutyl, Cyclopentyl, Cyclohexyl, Imidazolidin, 4,7,7-Trimethyl-3-oxo-2-oxa-bicyclo-[2.2.1]-heptyl-Rest oder Adamantyl-Rest, der unsubstituiert

oder einfach oder mehrfach, gleich oder verschieden, mit einem Substituenten ausgewählt aus der Gruppe bestehend aus $C_{1-5}$-Alkyl, $C_{1-5}$-Alkoxy, $-CF_3$, $CHF_2$, $CH_2F$ und Oxo substituiert ist,
für einen über eine $-(CH_2)-$, $-(CH_2)_2-$, -C(H)(Phenyl)-, oder $-C(CH_3)_2$-Gruppe gebundenen $-C(=O)-OR^7$-Rest, für einen über eine $-(CH_2)-$, $-(CH_2)_2-$, -C(H)(Phenyl)-, oder $-C(CH_3)_2$-Gruppe gebundenen $-O-(C=O)-R^8$-Rest, oder für einen über eine $-(CH_2)-$ oder $-(CH_2)_2$-Gruppe gebundenen $-(C=O)-R^9$-Rest steht,
ganz besonders bevorzugt für einen linearen oder verzweigten, ggf. eines oder mehrere Sauerstoffatome und/oder eines oder mehrere Schwefelatome als Kettenglied aufweisenden $C_{6-8}$-Alkyl-Rest, der unsubstituiert oder wenigstens einfach, gleich oder verschieden, mit einem Substituenten ausgewählt aus der Gruppe bestehend aus unsubstituiertem Phenyl, Dimethylamino, Carboxy und $-NH-(C=O)-O-C(CH_3)_3$ substituiert sein kann, für einen linearen oder verzweigten, unsubstituierten $C_{2-4}$-Alkenyl-Rest, für einen Naphthyl-Rest, Furyl- (Furanyl-)-Rest, Thienyl-(Thiophenyl-)-Rest, [1,2,3]-Triazolyl-Rest, Chromanyl-Rest, Benzo[1.3]dioxolyl-Rest, Benzo[1,2,5]oxadiazolyl-Rest, Pyrimidinyl-Rest, Pyrazolyl-Rest, Pyridinyl-Rest, Isoxazolyl-Rest oder [1,2,3]-Thiadiazolyl-Rest, wobei der zyklische Rest jeweils über eine $-(CH_2)-$, $-(CH_2)-O-$, -CH=CH-, $-(CH_2)_2-$, $-(CH_2)_2-O-$, oder $-(CH_2)_3$-Brücke gebunden sein kann und/oder einfach oder mehrfach, gleich oder verschieden, mit einem Substituenten ausgewählt aus der Gruppe bestehend aus F, Cl, Br, I, Methyl, Ethyl, iso-Propyl, n-Propyl, sec-Butyl, tert.-Butyl, n-Butyl, Methoxy, Ethoxy, $-CF_3$, $-OCF_3$, $-CH_2-O-C(=O)$-Phenyl, $-NO_2$, $-S-CF_3$, $-S-CHF_2$,$-N(CH_3)_2$, $-NH-CO-CH_3$, -CN, $-SO_2-NH_2$, Phenyl und Phenoxy, wobei die letztgenannten Phenyl- und Phenoxy Substituenten selbst einfach oder mehrfach, gleich oder verschieden, mit einem Substituenten ausgewählt aus der Gruppe bestehend aus F, Cl, Br, $CH_3$ und $OCH_3$ substituiert sein können,
für 2-Trifluoromethoxy-phenyl, 3- Trifluoromethoxy-phenyl, 4-Trifluoromethoxyphenyl, $2-CF_3$-S-phenyl, $3-CF_3$-S-phenyl, $4-CF_3$-S-phenyl, 2-Cyano-phenyl, 3-Cyanophenyl, 4-Cyano-phenyl, 2-Nitro-phenyl, 3-Nitro-phenyl, 4-Nitro-phenyl, $2-CHF_2$-S-Phenyl, $3-CHF_2$-S-Phenyl, $4-CHF_2$-S-Phenyl, 2-Dimethylamino-phenyl, 3-Dimethylamino-phenyl, 4-Dimethylamino-phenyl, 2-Phenyl-(C=O)-O-$CH_2$-phenyl, 3-Phenyl-(C=O)-O-$CH_2$-phenyl, 4-Phenyl-(C=O)-O-$CH_2$-phenyl, $2-CH_3$-(C=O)-NH-Phenyl, $3-CH_3$-(C=O)-NH-Phenyl, $4-CH_3$-(C=O)-NH-Phenyl, 2-Phenyl-phenyl, 3-Phenyl-phenyl, 4-Phenyl-phenyl, $2-NH_2-SO_2$-Phenyl, $3-NH_2-SO_2$-Pheny, $4-NH_2-SO_2$-Phenyl, Pentafluor-phenyl, 4-Methyl-3-nitro-phenyl, 2-Fluor-benzyl, 3-Fluor-benzyl, 4-Fluor-benzyl, 2-Chlor-benzyl, 3-Chlor-benzyl, 4-Chlor-benzyl, 2-Brom-benzyl, 3-Brom-benzyl, 4-Brom-benzyl, 2-Methyl-benzyl, 3-Methyl-benzyl, 4-Methyl-benzyl, 2-Methoxy-benzyl, 3-Methoxy-benzyl, 4-Methoxy-benzyl, 3,4-Dimethoxy-benzyl, 2,5-Dimethoxy-benzyl, -CH=CH-Phenyl, wobei der Phenyl-Rest in 2-, 3- oder 4- Position einfach oder mehrfach, gleich oder verschieden, mit einem Rest ausgewählt aus der Gruppe bestehend aus F, Cl, Br und $CF_3$ substituiert sein kann, $-CH_2$-O-Phenyl, wobei der Phenyl-Rest in 2-, 3- oder 4- Position einfach oder mehrfach, gleich oder verschieden, mit einem Rest ausgewählt aus der Gruppe bestehend aus F, Cl, Br und $CF_3$ substituiert sein kann
für einen ggf. über eine $-(CH_2)-$, $-(CH_2)_2-$ oder $-(CH_2)_3$-Brücke gebundenen Cyclopropyl, Cyclobutyl, Cyclopentyl, Cyclohexyl, Imidazolidin, , 4,7,7-Trimethyl-3-oxo-2-oxa-bicyclo-[2.2.1]-heptyl-Rest oder Adamantyl-Rest, der unsubstituiert oder einfach oder mehrfach, gleich oder verschieden, mit einem Substituenten ausgewählt aus der Gruppe bestehend aus $C_{1-5}$-Alkyl, $C_{1-5}$-Alkoxy, $-CF_3$, $CHF_2$, $CH_2F$ und Oxo substituiert ist,
für einen über eine $-(CH_2)-$, $-(CH_2)_2-$, -C(H)(Phenyl)-, oder $-C(CH_3)_2$-Gruppe gebundenen $-C(=O)-OR^7$-Rest, für einen über eine $-(CH_2)-$, $-(CH_2)_2-$, -C(H)(Phenyl)-, oder $-C(CH_3)_2$-Gruppe gebundenen $-O-(C=O)-R^8$-Rest, oder für einen über eine $-(CH_2)-$ oder $-(CH_2)_2$-Gruppe gebundenen $-(C=O)-R^9$-Rest steht, und die übrigen Reste $R^1$-$R^3$ und $R^5$ bis $R^9$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 1,4,8-Triazaspiro[4.5]decan-2-on-Verbindungen der vorstehend angegebenen allgemeinen Formel I, in denen die Reste $R^5$ und $R^6$, jeweils unabhängig voneinander, für einen linearen oder verzweigten $C_{1-5}$-Alkyl-Rest, einen linearen oder verzweigten $C_{2-5}$-Alkenyl-Rest oder für einen linearen oder verzweigten $C_{2-5}$-Alkinyl-Rest, vorzugsweise für einen Methyl-, Ethyl-, n-Propyl oder iso-Propyl-Rest stehen, und die jeweils die übrigen Reste $R^1$-$R^4$ und $R^7$-$R^9$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Bevorzugt sind auch substituierte 1,4,8-Triazaspiro[4.5]decan-2-on-Verbindungen der vorstehend angegebenen allgemeinen Formel I, worin die Reste $R^7$ und $R^8$, jeweils unabhängig voneinander, für einen Wasserstoff-Rest, für einen linearen oder verzweigten $C_{1-5}$-Alkyl-Rest, einen linearen oder verzweigten $C_{2-5}$-Alkenyl-Rest, einen linearen oder verzweigten $C_{2-5}$-Alkinyl-Rest, oder für einen ggf. wenigstens einfach substituierten Phenyl- oder Naphthyl-Rest stehen, vorzugsweise für einen Methyl-, Ethyl-, n-Propyl, iso-Propyl-Rest oder unsubstituierten Phenyl-Rest stehen, und jeweils die übrigen Reste $R^1$-$R^6$ und $R^9$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis,

oder jeweils in Form entsprechender Salze, oder jeweils in Form entsprechender Solvate.

Bevorzugt sind weiterhin substituierte 1,4,8-Triazaspiro[4.5]decan-2-on-Verbindungen der vorstehend angegebenen allgemeinen Formel I, worin der Rest $R^9$ für einen linearen oder verzweigten $C_{1-5}$-Alkyl-Rest, einen linearen oder verzweigten $C_{2-5}$-Alkenyl-Rest, einen linearen oder verzweigten $C_{2-5}$-Alkinyl-Rest, vorzugsweise für einen Methyl-, Ethyl-, n-Propyl oder iso-Propyl-Rest steht, und jeweils die übrigen Reste $R^1$-$R^8$ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte 1,4,8-Triazaspiro[4.5]decan-2-on-Verbindungen der vorstehend angegebenen allgemeinen Formel I

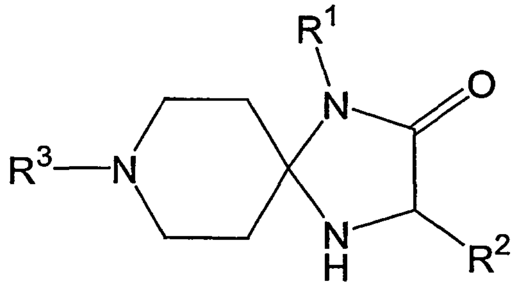

I

worin

$R^1$ für einen Wasserstoff-Rest, für einen linearen oder verzweigten, unsubstituierten $C_{1-4}$-Alkyl-Rest, für einen linearen oder verzweigten, unsubstituierten $C_{2-5}$-Alkenyl-Rest, für einen linearen oder verzweigten, unsubstituierten $C_{2-3}$-Alkinyl-Rest, für einen Phenyl- oder Naphthyl-Rest, der unsubstituiert oder einfach oder mehrfach, gleich oder verschieden, mit einem Substituenten ausgewählt aus der Gruppe bestehend aus F, Cl, Br, -CN, $-NO_2$, $C_{1-5}$-Alkyl, $C_{1-5}$-Alkoxy, -C(=O)-$C_{1-5}$-Alkyl, -C(=O)-O-$C_{1-5}$-Alkyl, $-S(=O)_2$-$C_{1-6}$-alkyl, -C(=O)-$C_{1-5}$-Perfluoroalkyl, $-CF_3$, $-CHF_2$ und $-CH_2F$ substituiert und/oder über eine $-(CH_2)$-, - $(CH_2)_2$-, $-(CH_2)_3$- oder $-(CH_2)_2$-O-Brücke gebunden ist, für einen über eine $-(CH_2)$-Gruppe gebundenen -C(=O)-$OR^5$-Rest oder für einen über eine $-(CH_2)$-, $-(CH_2)_2$-, - $(CH_2)_3$- oder $-(CH_2)_4$-Gruppe gebundenen -O-C(=O)-$R^6$-Rest steht,

$R^2$ für einen Wasserstoff-Rest, für einen linearen oder verzweigten, unsubstituierten, ggf. eines oder mehrere Schwefelatome als Kettenglied aufweisenden $C_{1-5}$-Alkyl-Rest oder für einen Phenyl-Rest steht, wobei der Phenyl-Rest einfach oder mehrfach, gleich oder verschieden, mit einem Substituenten ausgewählt aus der Gruppe bestehend aus F, Cl, Br, -CN, $-NO_2$, $C_{1-5}$-Alkyl, $C_{1-5}$-Alkoxy, -C(=O)-$C_{1-5}$-Alkyl, -C(=O)-O-$C_{1-5}$-Alkyl, $-S(=O)_2$-$C_{1-6}$-alkyl, -C(=O)- $C_{1-5}$-Perfluoroalkyl, $-CF_3$, $CHF_2$ und $CH_2F$ substituiert und/oder über eine $-(CH_2)$-Brücke gebunden sein kann,

$R^3$ für einen Wasserstoff-Rest, für einen Biphenyl-Rest, der über eine $-(CH_2)$-, - $(CH_2)_2$- oder $-(CH_2)_3$-Brücke gebunden ist, oder für eine C(=O)-$R^4$-Gruppe steht,

$R^4$ für einen Rest steht, der ausgewählt ist aus der Gruppe bestehend aus Methyl, Ethyl, iso-Propyl, n-Propyl, sec-Butyl, Isobutyl, tert-Butyl, n-Butyl, n-Pentyl, 1-Methylbutyl, 2-Dimethyl-propyl, 1-Ethyl-propyl, 1-Propyl-butyl, 1-Ethyl-pentyl, Dimethylaminomethyl, $-CH_2-CH_2-CH=CH_2$, $-CH_2-O-CH_3$, $-CH_2-O-CH_2-CH_2-O-CH_3$, - $CH_2-CH_2$-Phenyl, $-CH_2$-O-Phenyl, $-CH_2-O-CH_2$-Phenyl, $-CH_2-CH_2-CH_2$-O-Phenyl, - C(H)(Phenyl)($C_2H_5$), -C(H)($CH_3$)-O-Phenyl, $-CH_2-CH_2-(C=O)-CH_3$, $-CH_2-O-(C=O)-CH_3$, $-CH_2-CH_2-(C=O)-O-C_2H_5$, $-CH_2$-O-(C=O)-Phenyl, $-CH_2-(C=O)-O-CH_2-CH_3$, -C(H)(Phenyl)-(C=O)-$CH_3$, -C(H)(Phenyl)-O-(C=O)-$CH_3$, - $C(CH_3)_2-O-(C=O)-CH_3$, -C(H)(NH-(C=O)-O-$(CH_3)_3$)($CH_2$-O-$CH_2$-Phenyl), $-C(CH_3)_2-CH_2$-COOH, unsubstituiertem Phenyl, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl, 3-Brom-phenyl, 4-Brom-phenyl, 2-Iod-phenyl, 3-Iod-phenyl, 4-Iod-phenyl, 2-Methyl-phenyl, 3-Methyl-phenyl, 4-Methyl-phenyl, 2-Ethyl-phenyl, 3-Ethyl-phenyl, 4-Ethyl-phenyl, 2-n-Propyl-phenyl, 3-n-Proyl-phenyl, 4-n-Propyl-phenyl, 2-tert.-Butyl-phenyl, 3-tert.-Butylphenyl, 4-tert.-Butyl-phenyl, 2-Methoxy-phenyl, 3-Methoxy-phenyl, 4-Methoxy-phenyl, 2-Ethoxy-phenyl, 3-Ethoxy-phenyl, 4-Ethoxy-phenyl, 2-Trifluoromethyl-phenyl, 3-Trifluoromethyl-phenyl, 4-Trifluoromethyl-phenyl, 2-Trifluoromethoxy-phenyl, 3-Trifluoromethoxy-phenyl, 4-Trifluoromethoxy-phenyl, 2-$CF_3$-S-phenyl, 3-$CF_3$-S-phenyl, 4-$CF_3$-S-phenyl, 2-Cyano-phenyl, 3-Cyano-phenyl, 4-Cyano-phenyl, 2-Nitrophenyl, 3-Nitro-phenyl, 4-Nitro-phenyl, 2-$CHF_2$-S-Phenyl, 3-$CHF_2$-S-Phenyl, 4-$CHF_2$-S-Phenyl, 2-Dimethylamino-phenyl, 3-Dimethylamino-phenyl, 4-Dimethylaminophenyl, 2-Phenyl-(C=O)-O-CH2-phenyl, 3-Phe-

nyl-(C=O)-O-$CH_2$-phenyl, 4-Phenyl-(C=O)-O-$CH_2$-phenyl, 2-$CH_3$-(C=O)-NH-Phenyl, 3-$CH_3$-(C=O)-NH-Phenyl, 4-$CH_3$-(C=O)-NH-Phenyl, 2-Phenyl-phenyl, 3-Phenyl-phenyl, 4-Phenyl-phenyl, 2-$NH_2$-$SO_2$-Phenyl, 3-$NH_2$-$SO_2$-Pheny, 4-$NH_2$-$SO_2$-Phenyl, 2,4-Dimethoxy-phenyl, 2,5-Dimethoxy-phenyl, 3,5-Dimethoxy-phenyl, 3,4-Dimethoxy-phenyl, 2,3-Dimethyl-phenyl, 2,3-Dichlorphenyl, 3,4-Dichlorphenyl, 2,6-Dichlorphenyl, 2,3-Difluorphenyl, 3,4-Difluorphenyl, 3,5-Difluor-phenyl, Pentafluor-phenyl, 2-Chlor-6-Fluor-phenyl, 4-Brom-3-methyl-phenyl, 3-Fluor-4-methyl-phenyl, 3-Chlor-2-Fluor-phenyl, 2-Chlor-4-nitro-phenyl, 5-Fluor-2-trifluoromethyl-phenyl, 3-Fluor-4-trifluoromethyl, 4-Methyl-3-nitro-phenyl, 2-Chlor-5-trifluoro-methyl, 2,5-Bis-trifluoromethyl-phenyl, 3,5-Bis-trifluoromethyl-phenyl, 2-Chlor-6-fluor-3-methyl-phenyl, 6-Chlor-2-fluor-3-methylphenyl, 2,6-Difluor-4-methyl-phenyl, 2,6-Difluor-3-methyl-phenyl, 3,4,5-Trimethoxy-phenyl, 2,3-Difluor-4-methyl-phenyl, 2-Fluor-benzyl, 3-Fluor-benzyl, 4-Fluor-benzyl, 2-Chlor-benzyl, 3-Chlor-benzyl, 4-Chlor-benzyl, 2-Brom-benzyl, 3-Brom-benzyl, 4-Brom-benzyl, 2-Methyl-benzyl, 3-Methyl-benzyl, 4-Methyl-benzyl, 2-Methoxy-benzyl, 3-Methoxy-benzyl, 4-Methoxy-benzyl, 3,4-Dimethoxy-benzyl, 2,5-Dimethoxy-benzyl, - CH=CH-Phenyl, wobei der Phenyl-Rest in 2-, 3- oder 4- Position einfach oder mehrfach, gleich oder verschieden, mit einem Rest ausgewählt aus der Gruppe bestehend aus F, Cl, Br und $CF_3$ substituiert sein kann, -$CH_2$-O-Phenyl, wobei der Phenyl-Rest in 2-, 3- oder 4- Position einfach oder mehrfach, gleich oder verschieden, mit einem Rest ausgewählt aus der Gruppe bestehend aus F, Cl, Br und $CF_3$ substituiert sein kann, 1-Naphthyl, 2-Naphthyl, 2-Thienyl, 3-Thienyl, 3-Chlorthien-2-yl, 2-Furanyl, 3-Furanyl, 2,5-Dimethyl-Furan-3-yl, 5-tert.-Butyl-2-methyl-furan-3-yl, Pyridin-2-yl, Pyridin-3-yl, Pyridin-4-yl, 2-Chlor-pyridin-4-yl , 6-Chlor-pyridin-3-yl, 2-Chlor-pyridin-3-yl, 2-Ethylsulfanyl-pyridin-3-yl, 2-Phenoxy-pyridin-3-yl, 2-Methylsulfanyl-pyridin-3-yl, 2-Methyl-6-trifluoromethyl-pyridin-3-yl, Isoxazol-5-yl, 5-Methyl-isoxazol-3-yl, 3-(2-Chlor-6-Fluor-phenyl)-5-methyl-isoxazol-4-yl, 3-(2-Chlor-phenyl)-5-methyl-isoxazol-4-yl, 3-(2,6-Dichlor-phenyl)-5-methyl-isoxazol-4-yl, 5-tert.-Butyl-2-methyl-2H-pyrazol-3-yl, 1-Phenyl-5-n-Propyl-1H-pyrazol-4-yl, 1-(4-Chlor-phenyl)-5-trifluoromethyl-1H-pyrazol-4-yl, 2-tert-Butyl-5-methyl-2H-pyrazol-3-yl, 5-Methyl-2-Phenyl-2H-[1,2,3]triazol-4-yl, 4-Methyl-[1,2,3]thiadiazol-5-yl, 2-Chlor-4-trifluormethyl-pyrimidin-5-yl, 2-Chlor-4-trifluoromethyl-pyrimidin-5-yl, Benzo[1,2,5]oxadiazol-5-yl, Benzo[1.3]dioxol-5-yl, 6-Chlor-2H-chroman-3-yl, Imidazolidin-2,4-dion-5-yl-methyl, Cyclopropyl, Cyclobutyl, ggf. über eine -($CH_2$)- oder -$(CH_2)_2$-Brücke gebundenem Cyclopentyl, Cyclohexyl, 4,7,7-Trimethyl-3-oxo-2-oxabicyclo[2.2.1]heptyl und Adamantyl.

Vorzugsweise sind Verbindungen der vorstehend angegebenen Formel I, in denen

A)

$R^1$ für einen Wasserstoff-Rest, einen $C_{1-6}$-Alkyl-Rest, einen $C_{1-6}$-Alkoxy-Rest, einen $C_{2-6}$-Hydroxyalkyl-Rest, einen $C_{2-6}$Alkenyl-Rest, einen $C_{2-6}$-Alkinyl-Rest, einen Phenyl-Rest oder für einen einfach, zweifach oder dreifach mit einem Phenyl-Rest substituierten $C_{1-6}$-Alkyl-Rest steht,

$R^2$ für einen Wasserstoff-Rest, einen $C_{1-6}$-Alkyl-Rest, einen $C_{1-6}$-Alkoxy-Rest, einen $C_{2-6}$-Hydroxyalkyl-Rest, einen $C_{2-5}$Alkenyl-Rest, einen $C_{2-6}$-Alkinyl-Rest, einen Phenyl-Rest, einen einfach, zweifach oder dreifach mit einem Phenyl-Rest substituierten $C_{1-6}$-Alkyl-Rest, eine -O-$C_{1-6}$-Alkanoyl-Rest, einen OH-Rest, einen -0-$C_{1-6}$-Alkyl-Rest, einen -O-$C_{1-6}$-Alkoxy-Rest, einen -O-$C_{2-6}$-Hydroxyalkyl-Rest, einen - O-$C_{2-6}$Alkenyl-Rest, einen -O-$C_{2-6}$-Alkinyl-Rest, einen -O-Phenyl-Rest oder für einen einfach, zweifach oder dreifach mit einem Phenyl-Rest substituierten -O-$C_{1-6}$-Alkyl-Rest,

$R^3$ für einen Wasserstoff-Rest, einen $C_{1-6}$-Alkyl-Rest, einen $C_{2-6}$-Alkenyl-Rest, einen $C_{2-6}$-Alkinyl-Rest, einen $C_{3-7}$-Cycloalkyl-Rest, einen mit 1-6 Halogenatomen substituierten $C_{1-6}$-Alkyl-Rest, einen Hydroxy-$C_{1-6}$-Alkyl-Rest, einen $C_{1-6}$-Alkoxy-Rest, einen $C_{1-6}$-Alkylthio-Rest, einen $C_{1-6}$-Alkoxy-$C_{1-6}$-Alkyl-Rest, einen Carboxy-$C_{1-6}$-Alkyl-Rest, einen ($C_{1-6}$-Alkoxy)carbonyl-$C_{1-6}$-Alkyl-Rest, einen Amino-$C_{1-6}$-alkyl-Rest, einen mono-($C_{1-6}$-Alkyl)-amino-Rest, einen di-($C_{1-6}$-Alkyl)-amino-Rest, einen 2-oxopyrrolidin-1-yl-methyl-Rest, einen Aryl-Rest, einen Diarylmethylol-Rest, einen mit ein oder zwei Aryl-Resten substituierter $C_{1-6}$-Alkyl-Rest, einen $C_{1-6}$-Alkanoyl-Rest oder einen Arylcarbonyl-Rest steht, wobei Aryl jeweils für einen unsubstituierten Phenyl-Rest oder für einen Phenyl-Rest steht, der mit 1-3-Substituenten ausgewählt aus der Gruppe bestehend aus Halogen, $C_{1-6}$-Alkyl, $C_{1-6}$-Alkoxy und $CF_3$ substituiert ist, sowie deren physiologische verträgliche Salze, Enantiomeren und Racemate,
und/oder

B)

die Reste $R^1$, $R^2$ und $R^3$ jeweils für einen Wasserstoff-Rest oder einen Kohlenwasserstoff-Rest stehen sowie jeweils deren Salzen
ausgenommen.

Ganz besonders bevorzugt sind substituierte 1,4,8-Triazaspiro[4.5]decan-2-on-Verbindungen der vorstehend angegebenen allgemeinen Formel I ausgewählt aus der Gruppe bestehend aus

(1) 3-(*S*)-Benzyl-8-(2-ethyl-butyryl)-1,4,8-triaza-spiro[4.5]decan-2-on,
(2) 8-(2,4-Dimethoxy-benzoyl)-3-(*S*)-(2-methylsulfanyl-ethyl)-1,4,8-triazaspiro[4.5]decan-2-on,
(3) 3-(*S*,*R*)-Benzyl-8-(2-ethyl-butyryl)-1,4,8-triaza-spiro[4.5]decan-2-on,

(4) 8-Acetyl-3-(*S*)-benzyl-1,4,8-triaza-spiro[4.5]decan-2-on,
(5) 3-(*S,R*)-Benzyl-8-(4-chlor-benzoyl)-1,4,8-triaza-spiro[4.5]decan-2-on,
(6) 3-(*S*)-Benzyl-8-(4-chlor-benzoyl)-1,4,8-triaza-spiro[4.5]decan-2-on,
(7) 8-(2-ethyl-butyryl)-3-(*S*)-(2-methylsulfanyl-ethyl)-1,4,8-triazaspiro[4.5]decan-2-on,
(8) 1,3-(*S*)-Dibenzyl-8-(2-ethyl-butyryl)-1,4,8-triaza-spiro[4.5]decan-2-on,
(9) 8-Acetyl-1,3-(*S*)-dibenzyl-1,4,8-triaza-spiro[4.5]decan-2-on,
(10) 3-(*S,R*)-Benzyl-8-(4-chlor-benzoyl)-1-(4-methoxy-benzyl)-1,4,8-triazaspiro[4.5]decan-2-on,
(11) 3-(*S,R*)-Benzyl-8-(4-chlor-benzoyl)-1-(4-fluor-benzyl)-1,4,8-triazaspiro[4.5]decan-2-on,
(12) 1,3-(*S,R*)-Dibenzyl-8-(4-chlor-benzoyl)-1,4,8-triaza-spiro[4.5]decan-2on,
(13) 3-(*S,R*)-Benzyl-8-(2-ethyl-butyryl)-1-(4-methoxy-benzyl)-1,4,8-triazaspiro[4.5]decan-2-on,
(14) 3-(*S,R*)-Benzyl-8-(2-ethyl-butyryl)-1-(4-fluor-benzyl)-1,4,8-triazaspiro[4.5]decan-2-on,
(15) 1,3-(*S,R*)-Dibenzyl-8-(2-ethyl-butyryl)-1,4,8-triaza-spiro[4.5]decan-2-on,
(16) 1-Benzyl-8-(2-ethyl-butyryl)-3-(*S*)-(2-methylsulfanyl-ethyl)-1,4,8-triazaspiro[4.5]decan-2-on,
(17) 1,3-(*S*)-Dibenzyl-8-(4-chlor-benzoyl)-1,4,8-triaza-spiro[4.5]decan-2-on,
(18) 1,3-(*S*)-Dibenzyl-8-(2-ethyl-butyryl)-1,4,8-triaza-spiro[4.5]decan-2-on,
(19) 8-Acetyl-1,3-(*S*)-dibenzyl-1,4,8-triaza-spiro[4.5]decan-2-on,
(20) 3-(*S,R*)-Benzyl-8-(4-chlor-benzoyl)-1-(4-methoxy-benzyl)-1,4,8-triazaspiro[4.5]decan-2-on,
(21) 3-(*S,R*)-Benzyl-8-(4-chlor-benzoyl)-1-(4-fluor-benzyl)-1,4,8-triazaspiro[4.5]decan-2-on,
(22) 1,3-(*S,R*)-Dibenzyl-8-(4-chlor-benzoyl)-1,4,8-triaza-spiro[4.5]decan-2-on,
(23) 3-(*S,R*)-Benzyl-8-(2-ethyl-butyryl)-1-(4-methoxy-benzyl)-1,4,8-triazaspiro[4.5]decan-2-on,
(24) 3-(*S,R*)-Benzyl-8-(2-ethyl-butyryl)-1-(4-fluor-benzyl)-1,4,8-triazaspiro[4.5]decan-2-on,
(25) 1,3-(*S,R*)-Dibenzyl-8-(2-ethyl-butyryl)-1,4,8-triaza-spiro[4.5]decan-2-on,
(26) 1-Benzyl-8-(2-ethyl-butyryl)-3-(*S*)-(2-methylsulfanyl-ethyl)-1,4,8-triazaspiro[4.5]decan-2-on,
(27) 1,3-(*S*)-Dibenzyl-8-(4-chlor-benzoyl)-1,4,8-triaza-spiro[4.5]decan-2-on,
(28) 1-Benzyl-8-(2,4-dimethoxy-benzoyl)-3-(*S*)-(2-methylsulfanyl-ethyl)-1,4,8-triaza-spiro[4.5]decan-2-on,
(29) 1,3-(*S*)-Dibenzyl-1,4,8-triaza-spiro[4.5]decan-2-on,
(30) 1-Benzyl-3-(*S*)-(2-methylsulfanyl-ethyl)-1,4,8-triaza-spiro[4.5]decan-2-on,
(31) 1,3-(*S*)-Dibenzyl-8-butyryl-1,4,8-triaza-spiro[4.5]decan-2-on,
(32) 1,3(*S*)-Dibenzyl-8-(3-fluor-4-trifluormethyl-benzoyl)-1,4,8-triazaspiro[4.5]decan-2-on,
(33) 1,3-(*S*)-Dibenzyl-8-(2,3-difluor-benzoyl)-1,4,8-triaza-spiro[4.5]decan-2-on,
(34) 1,3-(*S*)-Dibenzyl-8-[2-(4-chlor-phenoxy)-acetyl]-1,4,8-triazaspiro[4.5]decan-2-on,
(35) 1,3-(*S*)-Dibenzyl-8-diphenylacetyl-1,4,8-triaza-spiro[4.5]decan-2-on,
(36) 1,3-(*S*)-Dibenzyl-8-(2-phenoxy-acetyl)-1,4,8-triaza-spiro[4.5]decan-2-on,
(37) 1,3-(*S*)-Dibenzyl-8-(3-phenyl-propionyl)-1,4,8-triaza-spiro[4.5]decan-2-on,
(38) 1,3-(*S*)-Dibenzyl-8-(naphthalin-2-carbonyl)-1,4,8-triaza-spiro[4.5]decan-2-on,
(39) 1,3-(*S*)-Dibenzyl-8-(furan-2-carbonyl)-1,4,8-triaza-spiro[4.5]decan-2-on,
(40) 1,3-(*S*)-Dibenzyl-8-(3-methoxy-benzoyl)-1,4,8-triaza-spiro[4.5]decan-2-on,
(41) 1,3-(*S*)-Dibenzyl-8-(4-fluor-benzoyl)-1,4,8-triaza-spiro[4.5]decan-2-on,
(42) 1-Benzyl-8-(4-fluor-benzoyl)-3-(*S*)-(2-methylsulfanyl-ethyl)-1,4,8-triaza-spiro[4.5]decan-2-on,
(43) 1,3-(*S*)-Dibenzyl-8-butyryl-1,4,8-triaza-spiro[4.5]decan-2-on,
(44) 1,3-(*S*)-Dibenzyl-8-(3-fluor-4-trifluormethyl-benzoyl)-1,4,8-triaza-spiro[4.5]decan-2-on,
(45) 1,3-(*S*)-Dibenzyl-8-(2,3-difluor-benzoyl)-1,4,8-triaza-spiro[4.5]decan-2-on,
(46) 1,3-(*S*)-Dibenzyl-8-[2-(4-chlor-phenoxy)-acetyl]-1,4,8-triazaspiro[4.5]decan-2-on,
(47) 1,3-(*S*)-Dibenzyl-8-diphenylacetyl-1,4,8-triaza-spiro[4.5]decan-2-on,
(48) 1,3-(*S*)-Dibenzyl-8-(2-phenoxy-acetyl)-1,4,8-triaza-spiro[4.5]decan-2-on,
(49) 1,3-(*S*)-Dibenzyl-8-(3-phenyl-propionyl)-1,4,8-triaza-spiro[4.5]decan-2-on,
(50) 1,3-(*S*)-Dibenzyl-8-(naphthalin-2-carbonyl)-1,4,8-triaza-spiro[4.5]decan-2-on,
(51) 1,3-(*S*)-Dibenzyl-8-(furan-2-carbonyl)-1,4,8-triaza-spiro[4.5]decan-2-on,
(52) 1,3-(*S*)-Dibenzyl-8-(3-methoxy-benzoyl)-1,4,8-triaza-spiro[4.5]decan-2-on,
(53) 1,3-(*S*)-Dibenzyl-8-(4-fluor-benzoyl)-1,4,8-triaza-spiro[4.5]decan-2-on,
(54) 1-Benzyl-8-(4-fluor-benzoyl)-3-(*S*)-(2-methylsulfanyl-ethyl)-1,4,8-triaza-spiro[4.5]decan-2-on,
(55) N-[4-(3-Isobutyl-2-oxo-1-prop-2-ynyl-1,4,8-triaza-spiro[4.5]decan-8-carbonyl)-phenyl]-acetamid,
(56) 1-(2-Phenoxy-ethyl)-8-(thiophen-2-carbonyl)-1,4,8-triaza-spiro[4.5]decan-2-on,
(57) 2-(2-Oxo-8-pent-4-enoyl-1,4,8-triaza-spiro[4.5]dec-1-ylmethyl)-benzonitril,
(58) 8-(2,4-Dimethoxy-benzoyl)-1-methyl-3-(2-methylsulfanyl-ethyl)-1,4,8-triaza-spiro[4.5]decan-2-on,
(59) 2-[8-(2-Ethyl-butyryl)-2-oxo-1,4,8-triaza-spiro[4.5]dec-1-ylmethyl]-benzonitril,
(60) 4-(2-Isobutyl-3-oxo-1,4,8-triaza-spiro[4.5]decan-8-carbonyl)-benzonitril,
(61) 8-(6-Chlor-pyridin-3-carbonyl)-3-isobutyl-1,4,8-triaza-spiro[4.5]decan-2-on,

(62) 2-[8-(2-Methyl-pentanoyl)-2-oxo-1,4,8-triaza-spiro[4.5]dec-1-ylmethyl]-benzonitril,
(63) 1-Benzyl-8-(biphenyl-4-carbonyl)-1,4,8-triaza-spiro[4.5]decan-2-on,
(64) 3-Isobutyl-8-(5-methyl-isoxazol-3-carbonyl)-1,4,8-triaza-spiro[4.5]decan-2-on,
(65) 3-Oxo-3-[2-oxo-1-(2-phenoxy-ethyl)-1,4,8-triaza-spiro[4.5]dec-8-yl]-propionsäureethylester,
(66) 8-(2-Chlor-benzoyl)-3-isopropyl-1,4,8-triaza-spiro[4.5]decan-2-on,
(67) 8-Cyclopentancarbonyl-3-isopropyl-1,4,8-triaza-spiro[4.5]decan-2-on,
(68) 8-(Furan-2-carbonyl)-1-methyl-3-(2-methylsulfanyl-ethyl)-1,4,8-triaza-spiro[4.5]decan-2-on,
(69) 3-Benzyl-8-(2-ethylsulfanyl-pyridin-3-carbonyl)-1-methyl-1,4,8-triaza-spiro[4.5]decan-2-on,
(70) 3-Benzyl-8-(4-chlor-benzoyl)-1,4,8-triaza-spiro[4.5]decan-2-on,
(71) 8-(2-Benzyloxy-acetyl)-3-isobutyl-1,4,8-triaza-spiro[4.5]decan-2-on,
(72) 3-Benzyl-8-(2-methoxy-acetyl)-1-prop-2-ynyl-1,4,8-triazaspiro[4.5]decan-2-on,
(73) 8-(5-tert-Butyl-2-methyl-2H-pyrazol-3-carbonyl)-3-isobutyl-1,4,8-triazaspiro[4.5]decan-2-on,
(74) 2-{8-[3-(2-chlor-6-fluor-phenyl)-5-methyl-isoxazol-4-carbonyl]-2-oxo-1,4,8-triaza-spiro[4.5]dec-1-ylmethyl}-benzonitril,
(75) 3-Benzyl-8-(2-chlor-benzoyl)-1,4,8-triaza-spiro[4.5]decan-2-on,
(76) 3-Benzyl-8-(3-dimethylamino-benzoyl)-1-methyl-1,4,8-triazaspiro[4.5]decan-2-on,
(77) 8-(3-Methyl-benzoyl)-1-(2-phenoxy-ethyl)-1,4,8-triaza-spiro[4.5]decan-2-on,
(78) 3-Isopropyl-1-(3-methyl-but-2-enyl)-8-(pyridin-4-carbonyl)-1,4,8-triazaspiro[4.5]decan-2-on,
(79) 1-Benzyl-8-(4-methyl-[1,2,3]thiadiazol-5-carbonyl)-1,4,8-triazaspiro[4.5]decan-2-on,
(80) 2-{8-[3-(2-chlor-phenyl)-acryloyl]-2-oxo-1,4,8-triaza-spiro[4.5]dec-1-ylmethyl}-benzonitril,
(81) 8-(3-Chlor-benzoyl)-3-isobutyl-1,4,8-triaza-spiro[4.5]decan-2-on,
(82) Essigsäure 2-(2-benzyl-3-oxo-1,4,8-triaza-spiro[4.5]dec-8-yl)-2-oxo-1-phenyl-ethylester,
(83) 8-(3,5-Dimethoxy-benzoyl)-3-isobutyl-1,4,8-triaza-spiro[4.5]decan-2-on,
(84) 3-Benzyl-8-(isoxazol-5-carbonyl)-1,4,8-triaza-spiro[4.5]decan-2-on,
(85) 8-(3-Chlor-thiophen-2-carbonyl)-3-isobutyl-1,4,8-triaza-spiro[4.5]decan-2-on,
(86) 3-Isopropyl-8-pentafluorbenzoyl-1,4,8-triaza-spiro[4.5]decan-2-on,
(87) 8-(2,5-Dimethyl-furan-3-carbonyl)-3-isobutyl-1,4,8-triazaspiro[4.5]decan-2-on,
(88) 1-Butyl-8-[2-(3,4-dimethoxy-phenyl)-acetyl]-3-(2-methylsulfanyl-ethyl)-1,4,8-triaza-spiro[4.5]decan-2-on,
(89) 1-Benzyl-3-isopropyl-8-(pyridin-4-carbonyl)-1,4,8-triaza-spiro[4.5]decan-2-on,
(90) 1,3-Dibenzyl-8-(3-dimethylamino-benzoyl)-1,4,8-triaza-spiro[4.5]decan-2-on,
(91) 5-{2-[1-(4-Fluor-benzyl)-3-(2-methylsulfanyl-ethyl)-2-oxo-1,4,8-triaza-spiro[4.5]dec-8-yl]-2-oxo-ethyl}-imidazolidin-2,4-dion,
(92) 8-(Biphenyl-4-carbonyl)-1-(4-fluor-benzyl)-3-isobutyl-1,4,8-triaza-spiro[4.5]decan-2-on,
(93) 2-[2-Oxo-8-(2-propyl-pentanoyl)-1,4,8-triaza-spiro[4.5]dec-1-ylmethyl]-benzonitril,
(94) 2-[8-(Furan-2-carbonyl)-3-isopropyl-2-oxo-1,4,8-triaza-spiro[4.5]dec-1-ylmethyl]-benzonitril,
(95) 8-[2-(4-Chlor-phenoxy)-acetyl]-3-isobutyl-1-prop-2-ynyl-1,4,8-triaza-spiro[4.5]decan-2-on,
(96) 1,3-Dibenzyl-8-(4-brom-benzoyl)-1,4,8-triaza-spiro[4.5]decan-2-on,
(97) 8-(3-Difluormethylsulfanyl-benzoyl)-3-isobutyl-1,4,8-triaza-spiro[4.5]decan-2-on,
(98) 8-(2,3-Dimethyl-benzoyl)-3-isopropyl-1,4,8-triaza-spiro[4.5]decan-2-on,
(99) 3-Benzyl-8-(2,3-dimethyl-benzoyl)-1,4,8-triaza-spiro[4.5]decan-2-on,
(100) 1-(4-Fluor-benzyl)-3-(2-methylsulfanyl-ethyl)-8-(pyridin-2-carbonyl)-1,4,8-triaza-spiro[4.5]decan-2-on,
(101) 3-Benzyl-8-(3,3-dimethyl-butyryl)-1,4,8-triaza-spiro[4.5]decan-2-on,
(102) 2-[8-(3-Dimethylamino-benzoyl)-3-isobutyl-2-oxo-1,4,8-triaza-spiro[4.5]dec-1-ylmethyl]-benzonitril,
(103) 3-[8-(2-Methoxy-acetyl)-3-(2-methylsulfanyl-ethyl)-2-oxo-1,4,8-triaza-spiro[4.5]dec-1-ylmethyl]-benzonitril,
(104) Essigsäure 2-(3-benzyl-1-methyl-2-oxo-1,4,8-triaza-spiro[4.5]dec-8-yl)-2-oxo-1-phenyl-ethylester,
(105) 2-[8-[2-(2-Methoxy-ethoxy)-acetyl]-3-(2-methylsulfanyl-ethyl)-2-oxo-1,4,8-triaza-spiro[4.5]dec-1-ylmethyl]-benzonitril,
(106) 3-Benzyl-8-[3-(2-chlor-6-fluor-phenyl)-5-methyl-isoxazol-4-carbonyl]-1,4,8-triaza-spiro[4.5]decan-2-on,
(107) 8-(2-Chlor-6-fluor-benzoyl)-3-isopropyl-1,4,8-triaza-spiro[4.5]decan-2-on,
(108) 1-Methyl-3-(2-methylsulfanyl-ethyl)-8-(2-phenoxy-acetyl)-1,4,8-triaza-spiro[4.5]decan-2-on,
(109) 8-(2-Chlor-pyridin-3-carbonyl)-1-(4-fluor-benzyl)-3-isobutyl-1,4,8-triaza-spiro[4.5]decan-2-on,
(110) 8-(2-Ethyl-butyryl)-1-methyl-3-(2-methylsulfanyl-ethyl)-1,4,8-triazaspiro[4.5]decan-2-on,
(111) 1-Methyl-3-(2-methylsulfanyl-ethyl)-8-(2-phenoxy-pyridin-3-carbonyl)-1,4,8-triaza-spiro[4.5]decan-2-on,
(112) 8-(3-Fluor-benzoyl)-1-methyl-3-(2-methylsulfanyl-ethyl)-1,4,8-triazaspiro[4.5]decan-2-on,
(113) 3-Benzyl-8-(naphthalin-2-carbonyl)-1,4,8-triaza-spiro[4.5]decan-2-on,
(114) 8-Cyclohexancarbonyl-1-(2-phenoxy-ethyl)-1,4,8-triaza-spiro[4.5]decan-2-on,
(115) 8-(2-Phenoxy-acetyl)-1-(2-phenoxy-ethyl)-1,4,8-triaza-spiro[4.5]decan-2-on,

(116) 4-[1-Butyl-3-(2-methylsulfanyl-ethyl)-2-oxo-1,4,8-triaza-spiro[4.5]decan-8-carbonyl]-benzonitril,
(117) 3-Benzyl-8-(3,3-dimethyl-butyryl)-1-methyl-1,4,8-triaza-spiro[4.5]decan-2-on,
(118) 3-Benzyl-8-(4-propyl-benzoyl)-1,4,8-triaza-spiro[4.5]decan-2-on,
(119) 3-Isopropyl-8-(2-phenoxy-propionyl)-1,4,8-triaza-spiro[4.5]decan-2-on,
(120) 1-Butyl-8-hexanoyl-3-Isopropyl-1,4,8-triaza-spiro[4.5]decan-2-on,
(121) 8-(4-Brom-3-methyl-benzoyl)-3-isopropyl-1,4,8-triaza-spiro[4.5]decan-2-on,
(122) 8-[3-(2-Chlor-6-fluor-phenyl)-5-methyl-isoxazol-4-carbonyl]-3-isopropyl-1-(2-phenoxy-ethyl)-1,4,8-triaza-spiro[4.5]decan-2-on,
(123) 1-(2-Fluor-benzyl)-3-isobutyl-8-(3-methyl-benzoyl)-1,4,8-triazaspiro[4.5]decan-2-on,
(124) 8-(2-Ethyl-hexanoyl)-1-(2-phenoxy-ethyl)-1,4,8-triaza-spiro[4.5]decan-2-on,
(125) 3-Benzyl-8-(3,4-difluor-benzoyl)-1,4,8-triaza-spiro[4.5]decan-2-on,
(126) 3-Benzyl-8-(4-ethoxy-benzoyl)-1-methyl-1,4,8-triaza-spiro[4.5]decan-2-on,
(127) 1-Benzyl-8-(6-chlor-pyridin-3-carbonyl)-1,4,8-triaza-spiro[4.5]decan-2-on,
(128) 8-(3-Dimethylamino-benzoyl)-1-(3,5-dimethyl-benzyl)-3-(2-methylsulfanyl-ethyl)-1,4,8-triaza-spiro[4.5]decan-2-on,
(129) 3-[8-(Benzo[1,3]dioxol-5-carbonyl)-3-(2-methylsulfanyl-ethyl)-2-oxo-1,4,8-triaza-spiro[4.5]dec-1-ylmethyl]-benzonitril,
(130) 3-Benzyl-1-methyl-8-pent-4-enoyl-1,4,8-triaza-spiro[4.5]decan-2-on,
(131) 8-(4-Ethoxy-benzoyl)-1-methyl-3-(2-methylsulfanyl-ethyl)-1,4,8-triazaspiro[4.5]decan-2-on,
(132) 3-Benzyl-8-(2-benzyloxy-acetyl)-1-methyl-1,4,8-triaza-spiro[4.5]decan2-on,
(133) 8-(3,4-Difluor-benzoyl)-1-(2-phenoxy-ethyl)-1,4,8-triaza-spiro[4.5]decan-2-on,
(134) 3-Benzyl-1-methyl-8-(2-methylsulfanyl-pyridin-3-carbonyl)-1,4,8-triazaspiro[4.5]decan-2-on,
(135) 1-Butyl-8-(4-methoxy-benzoyl)-3-(2-methylsulfanyl-ethyl)-1,4,8-triazaspiro[4.5]decan-2-on,
(136) 1,3-Dibenzyl-8-(2-ethylsulfanyl-pyridin-3-carbonyl)-1,4,8-triazaspiro[4.5]decan-2-on,
(137) 3-Benzyl-8-(3-difluormethylsulfanyl-benzoyl)-1,4,8-triazaspiro[4.5]decan-2-on,
(138) 1-(4-Fluor-benzyl)-8-(furan-2-carbonyl)-3-(2-methylsulfanyl-ethyl)-1,4,8-triaza-spiro[4.5]decan-2-on,
(139) 3-Benzyl-8-(3-fluor-4-methyl-benzoyl)-1-methyl-1,4,8-triazaspiro[4.5]decan-2-on,
(140) 8-[3-(2-Chlor-6-fluor-phenyl)-5-methyl-isoxazol-4-carbonyl]-3-isopropyl-1,4,8-triaza-spiro[4.5]decan-2-on,
(141) 1-Butyl-8-(6-Chlor-2H-chroman-3-carbonyl)-3-(2-methylsulfanyl-ethyl)-1,4,8-triaza-spiro[4.5]decan-2-on,
(142) 3-[8-(3-Methoxy-benzoyl)-3-(2-methylsulfanyl-ethyl)-2-oxo-1,4,8-triazaspiro[4.5]dec-1-ylmethyl]-benzonitril,
(143) 8-Cyclobutancarbonyl-3-(2-methylsulfanyl-ethyl)-1-prop-2-ynyl-1,4,8-triaza-spiro[4.5]decan-2-on,
(144) 3-Benzyl-1-butyl-8-(furan-2-carbonyl)-1,4,8-triaza-spiro[4.5]decan-2-on,
(145) 1-Benzyl-8-(3-chlor-thiophen-2-carbonyl)-3-isobutyl-1,4,8-triazaspiro[4.5]decan-2-on,
(146) 3-Benzyl-8-(2,5-bis-trifluormethyl-benzoyl)-1-butyl-1,4,8-triazaspiro[4.5]decan-2-on,
(147) 8-(3-Chlor-2-fluor-benzoyl)-1-(3,5-dimethyl-benzyl)-3-(2-methylsulfanyl-ethyl)-1,4,8-triaza-spiro[4.5]decan-2-on,
(148) 1-Benzyl-8-(2-chlor-pyridin-3-carbonyl)-3-isopropyl-1,4,8-triaza-spiro[4.5]decan-2-on,
(149) 3-Isobutyl-8-pentafluorbenzoyl-1-prop-2-ynyl-1,4,8-triaza-spiro[4.5]decan-2-on,
(150) 8-(2-Benzyloxy-acetyl)-1-butyl-3-(2-methylsulfanyl-ethyl)-1,4,8-triaza-spiro[4.5]decan-2-on,
(151) 8-(Furan-2-carbonyl)-3-isobutyl-1-prop-2-ynyl-1,4,8-triaza-spiro[4.5]decan-2-on,
(152) 1-Butyl-3-(2-methylsulfanyl-ethyl)-8-(4-phenoxy-butyryl)-1,4,8-triaza-spiro[4.5]decan-2-on,
(153) 3-Benzyl-8-(6-chlor-pyridin-3-carbonyl)-1-methyl-1,4,8-triaza-spiro[4.5]decan-2-on,
(154) 1-(2-Fluor-benzyl)-3-(2-methylsulfanyl-ethyl)-8-(2-phenoxy-pyridin-3-carbonyl)-1,4,8-triaza-spiro[4.5]decan-2-on,
(155) 1-(2-Fluor-benzyl)-3-isobutyl-8-(2-methyl-6-trifluormethyl-pyridin-3-carbonyl)-1,4,8-triaza-spiro[4.5]decan-2-on,
(156) 8-[2-(3-Chlor-phenoxy)-acetyl]-3-isopropyl-1-(2-phenoxy-ethyl)-1,4,8-triaza-spiro[4.5]decan-2-on,
(157) 8-(2,3-Dimethyl-benzoyl)-1-methyl-3-(2-methylsulfanyl-ethyl)-1,4,8-triaza-spiro[4.5]decan-2-on,
(158) 3-Isopropyl-8-(2-methyl-6-trifluormethyl-pyridin-3-carbonyl)-1-(2-phenoxy-ethyl)-1,4,8-triaza-spiro[4.5]decan-2-on,
(159) 3-Benzyl-1-methyl-8-(5-methyl-2-phenyl-2H-[1,2,3]triazole-4-carbonyl)-1,4,8-triaza-spiro[4.5]decan-2-on,
(160) 8-[3-(2-Chlor-phenyl)-5-methyl-isoxazol-4-carbonyl]-1-(3,5-dimethyl-benzyl)-3-(2-methylsulfanyl-ethyl)-1,4,8-triaza-spiro[4.5]decan-2-on,
(161) 1-(2-Fluor-benzyl)-3-(2-methylsulfanyl-ethyl)-8-(pyridin-2-carbonyl)-1,4,8-triaza-spiro[4.5]decan-2-on,
(162) 1-Benzyl-3-(2-methylsulfanyl-ethyl)-8-(2-phenoxy-pyridin-3-carbonyl)-1,4,8-triaza-spiro[4.5]decan-2-on,
(163) 1,3-Dibenzyl-8-(3-chlor-thiophen-2-carbonyl)-1,4,8-triaza-spiro[4.5]decan-2-on,
(164) 3-Benzyl-8-(4-tert-butyl-benzoyl)-1-methyl-1,4,8-triaza-spiro[4.5]decan-2-on,

(165) 2-[3-Isobutyl-8-(2-methoxy-acetyl)-2-oxo-1,4,8-triaza-spiro[4.5]dec-1-ylmethyl]-benzonitril,
(166) 3-Benzyl-1-butyl-8-(5-fluor-2-trifluormethyl-benzoyl)-1,4,8-triaza-spiro[4.5]decan-2-on,
(167) 3-Benzyl-8-[2-(4-methoxy-phenyl)-acetyl]-1-methyl-1,4,8-triaza-spiro[4.5]decan-2-on,
(168) 1,3-Dibenzyl-8-(4-methyl-[1,2,3]thiadiazol-5-carbonyl)-1,4,8-triaza-spiro[4.5]decan-2-on,
(169) 1-Benzyl-3-isopropyl-8-(2-methyl-6-trifluormethyl-pyridin-3-carbonyl)-1,4,8-triaza-spiro[4.5]decan-2-on,
(170) 1-Benzyl-8-(4-ethoxy-benzoyl)-3-isobutyl-1,4,8-triaza-spiro[4.5]decan-2-on,
(171) 3-Benzyl-1-butyl-8-cyclohexancarbonyl-1,4,8-triaza-spiro[4.5]decan-2-on,
(172) 3-[8-[3-(2-Chlor-6-fluor-phenyl)-5-methyl-isoxazol-4-carbonyl]-3-(2-methylsulfanyl-ethyl)-2-oxo-1,4,8-triaza-spiro[4.5]dec-1-ylmethyl]-benzonitril,
(173) 1-(2-Fluor-benzyl)-3-isobutyl-8-pent-4-enoyl-1,4,8-triaza-spiro[4.5]decan-2-on,
(174) 1-Methyl-3-(2-methylsulfanyl-ethyl)-8-(2-propyl-pentanoyl)-1,4,8-triaza-spiro[4.5]decan-2-on,
(175) 3-Benzyl-1-methyl-8-(2-phenoxy-acetyl)-1,4,8-triaza-spiro[4.5]decan-2-on,
(176) 3-Isobutyl-1-prop-2-ynyl-8-(3-trifluormethyl-benzoyl)-1,4,8-triaza-spiro[4.5]decan-2-on,
(177) 3-Benzyl-8-(furan-2-carbonyl)-1-prop-2-ynyl-1,4,8-triaza-spiro[4.5]decan-2-on,
(178) 1-Methyl-3-(2-methylsulfanyl-ethyl)-8-(naphthalin-1-carbonyl)-1,4,8-triaza-spiro[4.5]decan-2-on,
(179) 3-Benzyl-1-butyl-8-(3-cyclopentyl-propionyl)-1,4,8-triaza-spiro[4.5]decan-2-on,
(180) 1-(3,5-Dimethyl-benzyl)-3-(2-methylsulfanyl-ethyl)-8-pentanoyl-1,4,8-triaza-spiro[4.5]decan-2-on,
(181) 3-Benzyl-1-butyl-8-(2-methoxy-acetyl)-1,4,8-triaza-spiro[4.5]decan-2-on,
(182) 3-Benzyl-8-(3-fluor-4-trifluormethyl-benzoyl)-1-methyl-1,4,8-triaza-spiro[4.5]decan-2-on,
(183) 1-Benzyl-8-(3-difluormethylsulfanyl-benzoyl)-3-isopropyl-1,4,8-triaza-spiro[4.5]decan-2-on,
(184) 8-(2-Chlor-6-fiuor-3-methyl-benzoyl)-1-methyl-3-(2-methylsulfanyl-ethyl)-1,4,8-triaza-spiro[4.5]decan-2-on,
(185) 4-[3-Isopropyl-8-(2-methylsulfanyl-pyridin-3-carbonyl)-2-oxo-1,4,8-triaza-spiro[4.5]dec-1-ylmethyl]-benzoesäuremethylester,
(186) 8-[2-(2,5-Dimethoxy-phenyl)-acetyl]-1-(2-fluor-benzyl)-3-(2-methylsulfanyl-ethyl)-1,4,8-triaza-spiro[4.5] decan-2-on,
(187) 8-(5-tert-Butyl-2-methyl-furan-3-carbonyl)-3-isopropyl-1-(2-phenoxy-ethyl)-1,4,8-triaza-spiro[4.5]decan-2-on,
(188) 8-(2-Cyclopentyl-acetyl)-1-(4-fluor-benzyl)-3-isobutyl-1,4,8-triaza-spiro[4.5]decan-2-on,
(189) 4-[8-(3,3-Dimethyl-butyryl)-3-isopropyl-2-oxo-1,4,8-triaza-spiro[4.5]dec-1-ylmethyl]-benzoesäuremethylester,
(190) 3-[8-Cyclopropancarbonyl-3-(2-methylsulfanyl-ethyl)-2-oxo-1,4,8-triaza-spiro[4.5]dec-1-ylmethyl]-benzonitril,
(191) 3-[3-(2-Methylsulfanyl-ethyl)-2-oxo-8-(3-trifluormethoxy-benzoyl)-1,4,8-triaza-spiro[4.5]dec-1-ylmethyl]-benzonitril,
(192) 1-Butyl-8-(2-cyclopentyl-acetyl)-3-isopropyl-1,4,8-triaza-spiro[4.5]decan-2-on,
(193) 3-Benzyl-1-butyl-8-(pyridin-2-carbonyl)-1,4,8-triaza-spiro[4.5]decan-2-on,
(194) 3-Benzyl-8-[3-(2-chlor-phenyl)-acryloyl]-1-methyl-1,4,8-triaza-spiro[4.5]decan-2-on,
(195) 4-[8-(3-fluor-4-trifluormethyl-benzoyl)-3-isopropyl-2-oxo-1,4,8-triaza-spiro[4.5]dec-1-ylmethyl]-benzoesäuremethylester,
(196) 8-[3-(2,6-Dichlor-phenyl)-5-methyl-isoxazol-4-carbonyl]-1-methyl-3-(2-methylsulfanyl-ethyl)-1,4,8-triaza-spiro[4.5]decan-2-on,
(197) 1-Butyl-8-cyclohexancarbonyl-3-(2-methylsulfanyl-ethyl)-1,4,8-triaza-spiro[4.5]decan-2-on,
(198) 3-Benzyl-1-butyl-8-(4-iod-benzoyl)-1,4,8-triaza-spiro[4.5]decan-2-on
(199) 1-Methyl-3-(2-methylsulfanyl-ethyl)-8-[3-(3-trifluormethyl-phenyl)-acryloyl]-1,4,8-triaza-spiro[4.5]decan-2-on,
(200) 1,3-Dibenzyl-8-(4-phenoxy-butyryl)-1,4,8-triaza-spiro[4.5]decan-2-on
(201) 3-Benzyl-8-(2-chlor-6-fluor-benzoyl)-1-prop-2-ynyl-1,4,8-triaza-spiro[4.5]decan-2-on,
(202) 4-[8-(2-Chlor-pyridin-3-carbonyl)-3-isopropyl-2-oxo-1,4,8-triaza-spiro[4.5]dec-1-ylmethyl]-benzoesäuremethylester,
(203) 8-(2,5-Dimethyl-furan-3-carbonyl)-3-isopropyl-1-(2-phenoxy-ethyl)-1,4,8-triaza-spiro[4.5]decan-2-on,
(204) 8-(Biphenyl-4-carbonyl)-3-(2-methylsulfanyl-ethyl)-1-prop-2-ynyl-1,4,8-triaza-spiro[4.5]decan-2-on,
(205) 8-(3-Chlor-thiophen-2-carbonyl)-3-(2-methylsulfanyl-ethyl)-1-prop-2-ynyl-1,4,8-triaza-spiro[4.5]decan-2-on,
(206) 1-(4-Fluor-benzyl)-8-[2-(4-methoxy-phenyl)-acetyl]-3-(2-methylsulfanyl-ethyl)-1,4,8-triaza-spiro[4.5]decan-2-on,
(207) 1-Benzyl-3-isopropyl-8-(4-methyl-[1,2,3]thiadiazol-5-carbonyl)-1,4,8-triaza-spiro[4.5]decan-2-on,
(208) 2-[3-Isopropyl-8-(2-methylsulfanyl-pyridin-3-carbonyl)-2-oxo-1,4,8-triaza-spiro[4.5]dec-1-ylmethyl]-benzonitril,

(209) 3-[8-(5-tert-Butyl-2-methyl-2H-pyrazol-3-carbonyl)-3-isobutyl-2-oxo-1,4,8-triaza-spiro[4.5]dec-1-ylmethyl]-benzonitril,

(210) 1-Butyl-8-(2,5-dimethyl-furan-3-carbonyl)-3-(2-methylsulfanyl-ethyl)-1,4,8-triaza-spiro[4.5]decan-2-on,

(211) 8-(3-Cyclopentyl-propionyl)-1-(2-fluor-benzyl)-3-isobutyl-1,4,8-triaza-spiro[4.5]decan-2-on,

(212) 1-Benzyl-8-(3-cyclopentyl-propionyl)-3-(2-methylsulfanyl-ethyl)-1,4,8-triaza-spiro[4.5]decan-2-on,

(213) 3-(2-Methylsulfanyl-ethyl)-8-(4-phenoxy-butyryl)-1-prop-2-ynyl-1,4,8-triaza-spiro[4.5]decan-2-on,

(214) 1,3-Dibenzyl-8-(3-phenyl-acryloyl)-1,4,8-triaza-spiro[4.5]decan-2-on,

(215) 3-Benzyl-8-(6-chlor-2-fluor-3-methyl-benzoyl)-1-methyl-1,4,8-triazaspiro[4.5]decan-2-on,

(216) 8-[3-(2-Chlor-phenyl)-acryloyl]-3-isopropyl-1-(2-phenoxy-ethyl)-1,4,8-triaza-spiro[4.5]decan-2-on,

(217) 2-[3-Isopropyl-2-oxo-8-(3-phenyl-acryloyl)-1,4,8-triaza-spiro[4.5]dec-1-ylmethyl]-benzonitril,

(218) 3-Benzyl-1-methyl-8-(4-methyl-3-nitro-benzoyl)-1,4,8-triazaspiro[4.5]decan-2-on,

(219) 1-Benzyl-8-(furan-2-carbonyl)-3-isopropyl-1,4,8-triaza-spiro[4.5]decan-2-on,

(220) 1-Butyl-8-(3,5-dimethoxy-benzoyl)-3-(2-methylsulfanyl-ethyl)-1,4,8-triaza-spiro[4.5]decan-2-on,

(221) 1,3-Dibenzyl-8-(3,3-dimethyl-butyryl)-1,4,8-triaza-spiro[4.5]decan-2-on,

(222) 8-(2,6-Difluor-3-methyl-benzoyl)-1-methyl-3-(2-methylsulfanyl-ethyl)-1,4,8-triaza-spiro[4.5]decan-2-on,

(223) 2-[8-(2-Chlor-6-fluor-benzoyl)-3-(2-methylsulfanyl-ethyl)-2-oxo-1,4,8-triaza-spiro[4.5]dec-1-ylmethyl]-benzonitril,

(224) 8-[3-(2-Chlor-6-fluor-phenyl)-5-methyl-isoxazol-4-carbonyl]-3-isopropyl-1-(3-methyl-but-2-enyl)-1,4,8-triaza-spiro[4.5]decan-2-on,

(225) 3-Isobutyl-1-prop-2-ynyl-8-(4-trifluormethoxy-benzoyl)-1,4,8-triazaspiro[4.5]decan-2-on,

(226) 1-Benzyl-8-(2-chlor-6-fluor-3-methyl-benzoyl)-1,4,8-triazaspiro[4.5]decan-2-on,

(227) Benzoesäure 2-(1-benzyl-3-isopropyl-2-oxo-1,4,8-triazaspiro[4.5]decan-8-carbonyl)-benzylester,

(228) 1,3-Dibenzyl-8-(2-phenyl-butyryl)-1,4,8-triaza-spiro[4.5]decan-2-on,

(229) 3-Benzyl-1-methyl-8-(4-nitro-benzoyl)-1,4,8-triaza-spiro[4.5]decan-2-on,

(230) 3-Benzyl-1-methyl-8-(5-methyl-isoxazol-3-carbonyl)-1,4,8-triazaspiro[4.5]decan-2-on,

(231) 1-Benzyl-8-(6-chlor-2-fluor-3-methyl-benzoyl)-3-(2-methylsulfanyl-ethyl)-1,4,8-triaza-spiro[4.5]decan-2-on,

(232) 1-Benzyl-8-(3-fluor-4-trifluormethyl-benzoyl)-3-isobutyl-1,4,8-triazaspiro[4.5]decan-2-on,

(233) 3-Benzyl-8-(6-chlor-2H-chroman-3-carbonyl)-1-methyl-1,4,8-triaza-spiro[4.5]decan-2-on,

(234) 3-Benzyl-1-butyl-8-(4-methyl-[1,2,3]thiadiazol-5-carbonyl)-1,4,8-triaza-spiro[4.5]decan-2-on,

(235) 3-Benzyl-8-(4-methyl-[1,2,3]thiadiazol-5-carbonyl)-1-prop-2-ynyl-1,4,8-triaza-spiro[4.5]decan-2-on,

(236) 2-[8-(6-Chlor-2H-chroman-3-carbonyl)-3-isopropyl-2-oxo-1,4,8-triaza-spiro[4.5]dec-1-ylmethyl]-benzonitril,

(237) 2-[8-(5-Methyl-isoxazol-3-carbonyl)-3-(2-methylsulfanyl-ethyl)-2-oxo-1,4,8-triaza-spiro[4.5]dec-1-ylmethyl]-benzonitril,

(238) 2-[8-(3-Chlor-2-fluor-benzoyl)-3-isopropyl-2-oxo-1,4,8-triaza-spiro[4.5]dec-1-ylmethyl]-benzonitril,

(239) 4-[8-(5-tert-Butyl-2-methyl-2H-pyrazol-3-carbonyl)-3-isopropyl-2-oxo-1,4,8-triaza-spiro[4.5]dec-1-ylmethyl]-benzoesäuremethylester,

(240) 3-Benzyl-1-butyl-8-(5-tert-butyl-2-methyl-2H-pyrazol-3-carbonyl)-1,4,8-triaza-spiro[4.5]decan-2-on,

(241) 3-Benzyl-1-butyl-8-(2-chlor-4-trifluormethyl-pyrimidin-5-carbonyl)-1,4,8-triaza-spiro[4.5]decan-2-on,

(242) 3-Benzyl-8-(5-methyl-isoxazol-3-carbonyl)-1-prop-2-ynyl-1,4,8-triaza-spiro[4.5]decan-2-on,

(243) 4-[8-(2-Chlor-pyridin-3-carbonyl)-2-oxo-1,4,8-triaza-spiro[4.5]dec-1-ylmethyl]-benzoesäuremethylester,

(244) 8-(2-tert-Butyl-5-methyl-2H-pyrazol-3-carbonyl)-1-(2-fluor-benzyl)-1,4,8-triaza-spiro[4.5]decan-2-on,

(245) 4-[8-(2-Methylsulfanyl-pyridin-3-carbonyl)-2-oxo-1,4,8-triaza-spiro[4.5]dec-1-ylmethyl]-benzoesäuremethylester,

(246) Essigsäure 4-[8-(4-acetylamino-benzoyl)-3-isobutyl-2-oxo-1,4,8-triaza-spiro[4.5]dec-1-yl]-butylester,

(247) [8-(4-Acetylamino-benzoyl)-3-benzyl-2-oxo-1,4,8-triaza-spiro[4.5]dec-1-yl]-essigsäureethylester,

(248) Essigsäure 4-[8-(2-ethylsulfanyl-pyridin-3-carbonyl)-3-isobutyl-2-oxo-1,4,8-triaza-spiro[4.5]dec-1-yl]-butylester,

(249) 4-[8-(4-Methyl-[1,2,3]thiadiazol-5-carbonyl)-2-oxo-1,4,8-triaza-spiro[4.5]dec-1-ylmethyl]-benzoesäuremethylester,

(250) 4-[1-Allyl-3-(2-methylsulfanyl-ethyl)-2-oxo-1,4,8-triaza-spiro[4.5]decan-8-carbonyl]-benzolsulfonamid,

(251) 4-(8-Cyclobutancarbonyl-2-oxo-1,4,8-triaza-spiro[4.5]dec-1-ylmethyl)-benzoesäuremethylester,

(252) Essigsäure 2-[1-allyl-3-(2-methylsulfanyl-ethyl)-2-oxo-1,4,8-triazaspiro[4.5]dec-8-yl]-2-oxo-ethylester,

(253) 8-(Biphenyl-4-carbonyl)-1-(2-fluor-benzyl)-1,4,8-triaza-spiro[4.5]decan-2-on,

(254) [3-(2-Methylsulfanyl-ethyl)-2-oxo-8-propionyl-1,4,8-triaza-spiro[4.5]dec-1-yl]-essigsäureethylester,

(255) 8-(Benzo[1,3]dioxole-5-carbonyl)-1-(2,6-dichlor-benzyl)-3-isobutyl-1,4,8-triaza-spiro[4.5]decan-2-on,

(256) 1-Allyl-8-(biphenyl-4-carbonyl)-3-isopropyl-1,4,8-triaza-spiro[4.5]decan-2-on,
(257) [3-Benzyl-8-(biphenyl-4-carbonyl)-2-oxo-1,4,8-triaza-spiro[4.5]dec-1-yl]-essigsäureethylester,
(258) [8-(3-Dimethylamino-benzoyl)-3-(2-methylsulfanyl-ethyl)-2-oxo-1,4,8-triaza-spiro[4.5]dec-1-yl]-essigsäureethylester,
(259) 3-(2-Oxo-8-pentanoyl-1,4,8-triaza-spiro[4.5]dec-1-ylmethyl)-benzonitril,
(260) 4-(8-Cyclopentancarbonyl-2-oxo-1,4,8-triaza-spiro[4.5]dec-1-ylmethyl)-benzoesäuremethylester,
(261) 4-[1-(2-Fluor-benzyl)-2-oxo-1,4,8-triaza-spiro[4.5]dec-8-yl]-4-oxo-butansäurethylester,
(262) 1-(2-Fluor-benzyl)-8-(3,4,5-trimethoxy-benzoyl)-1,4,8-triazaspiro[4.5]decan-2-on,
(263) 4-[8-(2-Chlor-pyridin-4-carbonyl)-2-oxo-1,4,8-triaza-spiro[4.5]dec-1-ylmethyl]-benzoesäuremethylester,
(264) 3-[8-(3,5-Bis-trifluormethyl-benzoyl)-2-oxo-1,4,8-triaza-spiro[4.5]dec-1-ylmethyl]-benzonitril,
(265) 3-Benzyl-1-(2-fluor-benzyl)-8-(2-methoxy-benzoyl)-1,4,8-triazaspiro[4.5]decan-2-on,
(266) 8-(Benzo[1,2,5]oxadiazol-5-carbonyl)-1-(2,6-dichlor-benzyl)-3-isobutyl-1,4,8-triaza-spiro[4.5]decan-2-on,
(267) [3-Benzyl-2-oxo-8-(4-sulfamoyl-benzoyl)-1,4,8-triaza-spiro[4.5]dec-1-yl]-essigsäureethylester,
(268) 3-[2-Oxo-8-(3,4,5-trimethoxy-benzoyl)1,4,8-triaza-spiro[4.5]dec-1-ylmethyl]-benzonitril,
(269) Essigsäure 2-[1-(4-acetoxy-butyl)-3-isobutyl-2-oxo-1,4,8-triaza-spiro[4.5]dec-8-yl]-1,1-dimethyl-2-oxo-ethylester,
(270) 8-(6-Chlor-pyridin-3-carbonyl)-1-(2,6-dichlor-benzyl)-3-isobutyl-1,4,8-triaza-spiro[4.5]decan-2-on,
(271) 8-(2-Ethoxy-benzoyl)-1-(2-fluor-benzyl)-1,4,8-triaza-spiro[4.5]decan-2-on,
(272) 1-Allyl-8-cyclopropancarbonyl-3-(2-methylsulfanyl-ethyl)-1,4,8-triaza-spiro[4.5]decan-2-on,
(273) 3-[3-Isopropyl-8-(2-methoxy-acetyl)-2-oxo-1,4,8-triaza-spiro[4.5]dec-1-ylmethyl]-benzonitril,
(274) 4-(2-Oxo-8-phenylacetyl-1,4,8-triaza-spiro[4.5]dec-1-ylmethyl)-benzoesäuremethylester,
(275) Essigsäure 2-[1-(3-cyano-benzyl)-2-oxo-1,4,8-triaza-spiro[4.5]dec-8-yl]-2-oxo-1-phenyl-ethylester,
(276) 1-(2-Fluor-benzyl)-8-(4-methoxy-benzoyl)-1,4,8-triaza-spiro[4.5]decan-2-on,
(277) 4-(8-Cyclohexancarbonyl-2-oxo-1,4,8-triaza-spiro[4.5]dec-1-ylmethyl)-benzoesäuremethylester,
(278) 1-(2-Fluor-benzyl)-8-pent-4-enoyl-1,4,8-triaza-spiro[4.5]decan-2-on,
(279) [3-Isobutyl-8-(3-methyl-butyryl)-2-oxo-1,4,8-triaza-spiro[4.5]dec-1-yl]-essigsäureethylester,
(280) 1-Allyl-8-(3,3-dimethyl-butyryl)-3-(2-methylsulfanyl-ethyl)-1,4,8-triaza-spiro[4.5]decan-2-on,
(281) 1-Allyl-3-(2-methylsulfanyl-ethyl)-8-(2-methylsulfanyl-pyridin-3-carbonyl)-1,4,8-triaza-spiro[4.5]decan-2-on,
(282) [3-Isobutyl-8-(2-methyl-pentanoyl)-2-oxo-1,4,8-triaza-spiro[4.5]dec-1-yl]-essigsäureethylester,
(283) 4-[8-(5-tert-Butyl-2-methyl-furan-3-carbonyl)-2-oxo-1,4,8-triaza-spiro[4.5]dec-1-ylmethyl]-benzoesäuremethylester,
(284) (3-Benzyl-8-cyclopropancarbonyl-2-oxo-1,4,8-triaza-spiro[4.5]dec-1-yl)-essigsäureethylester,
(285) [3-Benzyl-8-(3-methyl-butyryl)-2-oxo-1,4,8-triaza-spiro[4.5]dec-1-yl]-essigsäureethylester,
(286) 1-(2,6-Dichlor-benzyl)-8-(2,5-dimethyl-furan-3-carbonyl)-3-isobutyl-1,4,8-triaza-spiro[4.5]decan-2-on,
(287) 1-Allyl-3-isopropyl-8-[2-(3-methoxy-phenyl)-acetyl]-1,4,8-triazaspiro[4.5]decan-2-on,
(288) [8-(4-tert-Butyl-benzoyl)-3-(2-methylsulfanyl-ethyl)-2-oxo-1,4,8-triazaspiro[4.5]dec-1-yl]-essigsäureethylester,
(289) 3-(2-Methylsulfanyl-ethyl)-1-(2-nitro-benzyl)-8-(2-phenoxy-pyridin-3-carbonyl)-1,4,8-triaza-spiro[4.5]decan-2-on,
(290) (3-Benzyl-2-oxo-8-pentanoyl-1,4,8-triaza-spiro[4.5]dec-1-yl)-essigsäureethylester,
(291) 8-(2-Chlor-pyridin-4-carbonyl)-1-(2-fluor-benzyl)-1,4,8-triaza-spiro[4.5]decan-2-on,
(292) [8-(3-Methyl-butyryl)-3-(2-methylsulfanyl-ethyl)-2-oxo-1,4,8-triaza-spiro[4.5]dec-1-yl]-essigsäureethylester,
(293) 1-Allyl-3-(2-methylsulfanyl-ethyl)-8-pentafluorbenzoyl-1,4,8-triaza-spiro[4.5]decan-2-on,
(294) 1-(2-Fluor-benzyl)-8-(2-trifluormethyl-benzoyl)-1,4,8-triaza-spiro[4.5]decan-2-on,
(295) 8-(Benzo[1,2,5]oxadiazol-5-carbonyl)-1-(2-fluor-benzyl)-1,4,8-triaza-spiro[4.5]decan-2-on,
(296) 4-[8-(4-tert-Butyl-benzoyl)-2-oxo-1,4,8-triaza-spiro[4.5]dec-1-ylmethyl]-benzoesäuremethylester,
(297) [3-Benzyl-2-oxo-8-(1-phenyl-5-propyl-1H-pyrazol-4-carbonyl)-1,4,8-triaza-spiro[4.5]dec-1-yl]-essigsäureethylester,
(298) 1-Allyl-3-isopropyl-8-pent-4-enoyl-1,4,8-triaza-spiro[4.5]decan-2-on,
(299) 3-[2-Oxo-8-(2-trifluormethyl-benzoyl)-1,4,8-triaza-spiro[4.5]dec-1-ylmethyl]-benzonitril,
(300) 8-(2-Dimethylamino-acetyl)-1-(3,5-dimethyl-benzyl)-3-isobutyl-1,4,8-triaza-spiro[4.5]decan-2-on,
(301) Essigsäure 4-[8-(2-ethoxy-benzoyl)-3-isobutyl-2-oxo-1,4,8-triaza-spiro[4.5]dec-1-yl]-butylester,
(302) 1-(2-Fluor-benzyl)-8-(3-methoxy-benzoyl)-1,4,8-triaza-spiro[4.5]decan-2-on,
(303) [8-(Furan-2-carbonyl)-3-isobutyl-2-oxo-1,4,8-triaza-spiro[4.5]dec-1-yl]-essigsäureethylester,
(304) 8-(5-tert-Butyl-2-methyl-2H-pyrazol-3-carbonyl)-1-(2-fluor-benzyl)-1,4,8-triaza-spiro[4.5]decan-2-on,
(305) 1-(2-Fluor-benzyl)-8-(thiophen-2-carbonyl)-1,4,8-triaza-spiro[4.5]decan-2-on,

(306) [3-Benzyl-8-(2-fluor-benzoyl)-2-oxo-1,4,8-triaza-spiro[4.5]dec-1-yl]-essigsäureethylester,
(307) [8-(Isoxazol-5-carbonyl)-3-(2-methylsulfanyl-ethyl)-2-oxo-1,4,8-triaza-spiro[4.5]dec-1-yl]-essigsäureethylester,
(308) 1-(2,6-Dichlor-benzyl)-3-isobutyl-8-(4-propyl-benzoyl)-1,4,8-triaza-spiro[4.5]decan-2-on,
(309) [8-[2-(2,5-Dimethoxy-phenyl)-acetyl]-3-(2-methylsulfanyl-ethyl)-2-oxo-1,4,8-triaza-spiro[4.5]dec-1-yl]-essigsäureethylester,
(310) (3-Benzyl-8-cyclobutancarbonyl-2-oxo-1,4,8-triaza-spiro[4.5]dec-1-yl)-essigsäureethylester,
(311) 1-(2-Fluor-benzyl)-8-[2-(4-methoxy-phenyl)-acetyl]-1,4,8-triaza-spiro[4.5]decan-2-on,
(312) 1-Allyl-3-(2-methylsulfanyl-ethyl)-8-(2-methyl-6-trifluormethyl-pyridin-3-carbonyl)-1,4,8-triaza-spiro[4.5]decan-2-on,
(313) [3-(2-Methylsulfanyl-ethyl)-2-oxo-8-(4-trifluormethyl-benzoyl)-1,4,8-triaza-spiro[4.5]dec-1-yl]-essigsäureethylester,
(314) 1-Allyl-3-(2-methylsulfanyl-ethyl)-8-(1-phenyl-5-propyl-1H-pyrazol-4-carbonyl)-1,4,8-triaza-spiro[4.5]decan-2-on,
(315) 1-(2,6-Dichlor-benzyl)-8-(2,3-difluor-4-methyl-benzoyl)-3-isobutyl-1,4,8-triaza-spiro[4.5]decan-2-on,
(316) 1-Allyl-3-isopropyl-8-(2-methyl-benzoyl)-1,4,8-triaza-spiro[4.5]decan-2-on,
(317) 8-(2-Chlor-5-trifluormethyl-benzoyl)-1-(2,6-dichlor-benzyl)-3-isobutyl-1,4,8-triaza-spiro[4.5]decan-2-on,
(318) 1-Allyl-8-[2-(3-methoxy-phenyl)-acetyl]-3-(2-methylsulfanyl-ethyl)-1,4,8-triaza-spiro[4.5]decan-2-on,
(319) 1-Allyl-3-(2-methylsulfanyl-ethyl)-8-(3-phenyl-propionyl)-1,4,8-triaza-spiro[4.5]decan-2-on,
(320) [3-Benzyl-8-(2-benzyloxy-acetyl)-2-oxo-1,4,8-triaza-spiro[4.5]dec-1-yl]-essigsäureethylester,
(321) 1-Allyl-8-(5-tert-butyl-2-methyl-2H-pyrazol-3-carbonyl)-3-(2-methylsulfanyl-ethyl)-1,4,8-triaza-spiro[4.5]decan-2-on,
(322) Essigsäure 1,1-dimethyl-2-[3-(2-methylsulfanyl-ethyl)-1-(2-nitro-benzyl)-2-oxo-1,4,8-triaza-spiro[4.5]dec-8-yl]-2-oxo-ethylester,
(323) Benzoesäure 2-[1-ethoxycarbonylmethyl-3-(2-methylsulfanyl-ethyl)-2-oxo-1,4,8-triaza-spiro[4.5]decan-8-carbonyl]-benzylester,
(324) 1-Allyl-3-isopropyl-8-(4-methyl-[1,2,3]thiadiazol-5-carbonyl)-1,4,8-triaza-spiro[4.5]decan-2-on,
(325) 1-Allyl-8-(2,5-dimethyl-furan-3-carbonyl)-3-(2-methylsulfanyl-ethyl)-1,4,8-triaza-spiro[4.5]decan-2-on,
(326) 1-Allyl-8-(benzo[1,2,5]oxadiazol-5-carbonyl)-3-(2-methylsulfanyl-ethyl)-1,4,8-triaza-spiro[4.5]decan-2-on,
(327) 1-(2-Fluor-benzyl)-8-(4-phenoxy-butyryl)-1,4,8-triaza-spiro[4.5]decan-2-on,
(328) 1-Allyl-8-(2-cyclopentyl-acetyl)-3-(2-methylsulfanyl-ethyl)-1,4,8-triaza-spiro[4.5]decan-2-on,
(329) [3-Benzyl-8-(naphthalin-2-carbonyl)-2-oxo-1,4,8-triaza-spiro[4.5]dec-1-yl]-essigsäureethylester,
(330) 8-[3-(2-Chlor-6-fluor-phenyl)-5-methyl-isoxazol-4-carbonyl]-1-(2-fluor-benzyl)-1,4,8-triaza-spiro[4.5]decan-2-on,
(331) 3-[8-(3,5-Dimethoxy-benzoyl)-3-isopropyl-2-oxo-1,4,8-triaza-spiro[4.5]dec-1-ylmethyl]-benzonitril,
(332) 3-(2-Methylsulfanyl-ethyl)-1-(2-nitro-benzyl)-8-(1-phenyl-5-propyl-1H-pyrazol-4-carbonyl)-1,4,8-triaza-spiro[4.5]decan-2-on,
(333) {3-Benzyl-8-[3-(2-chlor-6-fluor-phenyl)-5-methyl-isoxazol-4-carbonyl]-2-oxo-1,4,8-triaza-spiro[4.5]dec-1-yl}-essigsäureethylester,
(334) 1-(2-Fluor-benzyl)-8-(3-fluor-4-trifluormethyl-benzoyl)-1,4,8-triaza-spiro[4.5]decan-2-on,
(335) [8-[1-(4-Chlor-phenyl)-5-trifluormethyl-1H-pyrazol-4-carbonyl]-3-(2-methylsulfanyl-ethyl)-2-oxo-1,4,8-triaza-spiro[4.5]dec-1-yl]-essigsäureethylester,
(336) 3-[8-(Naphthalin-1-carbonyl)-2-oxo-1,4,8-triaza-spiro[4.5]dec-1-ylmethyl]-benzonitril,
(337) [8-(3,3-Dimethyl-butyryl)-3-(2-methylsulfanyl-ethyl)-2-oxo-1,4,8-triaza-spiro[4.5]dec-1-yl]-essigsäureethylester,
(338) 8-Acetyl-3-(2-methylsulfanyl-ethyl)-1-naphthalin-2-ylmethyl-1,4,8-triaza-spiro[4.5]decan-2-on,
(339) [3-Benzyl-8-(3-cyano-benzoyl)-2-oxo-1,4,8-triaza-spiro[4.5]dec-1-yl]-essigsäureethylester,
(340) 8-(5-tert-Butyl-2-methyl-2H-pyrazol-3-carbonyl)-1-(2,6-dichlor-benzyl)-3-isobutyl-1,4,8-triaza-spiro[4.5]decan-2-on,
(341) 4-[3-Benzyl-1-(2-fluor-benzyl)-2-oxo-1,4,8-triaza-spiro[4.5]decan-8-carbonyl]-benzonitril,
(342) 4-[3-Benzyl-1-(2-fluor-benzyl)-2-oxo-1,4,8-triaza-spiro[4.5]dec-8-yl]-4-oxo-butyrsäure methylester,
(343) 3-(2-Methylsulfanyl-ethyl)-1-naphthalin-2-ylmethyl-8-(3,4,5-trimethoxy-benzoyl)-1,4,8-triaza-spiro[4.5]decan-2-on,
(344) [3-Benzyl-8-(isoxazol-5-carbonyl)-2-oxo-1,4,8-triaza-spiro[4.5]dec-1-yl]-essigsäureethylester,
(345) 8-(3-Difluormethylsulfanyl-benzoyl)-1-(2-fluor-benzyl)-1,4,8-triaza-spiro[4.5]decan-2-on,
(346) [3-Benzyl-8-(2,3-dimethyl-benzoyl)-2-oxo-1,4,8-triaza-spiro[4.5]dec-1-yl]-essigsäureethylester,
(347) 1-(2-Fluor-benzyl)-8-(4-propyl-benzoyl)-1,4,8-triaza-spiro[4.5]decan-2-on,
(348) 3-[8-(4-Iod-benzoyl)-2-oxo-1,4,8-triaza-spiro[4.5]dec-1-ylmethyl]-benzonitril,

(349) 1-(2-Fluor-benzyl)-8-(2-propyl-pentanoyl)-1,4,8-triaza-spiro[4.5]decan-2-on,
(350) 3-[8-(2,6-Difluor-3-methyl-benzoyl)-3-isopropyl-2-oxo-1,4,8-triaza-spiro[4.5]dec-1-ylmethyl]-benzonitril,
(351) 3-(2-Methylsulfanyl-ethyl)-8-(4-methyl-[1,2,3]thiadiazol-5-carbonyl)-1-(2-nitro-benzyl)-1,4,8-triaza-spiro[4.5]decan-2-on,
(352) Essigsäure 4-[3-isobutyl-8-(4-methyl-[1,2,3]thiadiazol-5-carbonyl)-2-oxo-1,4,8-triaza-spiro[4.5]dec-1-yl]-butylester,
(353) 8-[2-(4-Chlor-phenoxy)-acetyl]-3-(2-methylsulfanyl-ethyl)-1-naphthalin-2-ylmethyl-1,4,8-triaza-spiro[4.5]decan-2-on,
(354) [3-Benzyl-2-oxo-8-(3-trifluormethyl-benzoyl)-1,4,8-triaza-spiro[4.5]dec-1-yl]-essigsäureethylester,
(355) 8-(4-Brom-benzoyl)-3-(2-methylsulfanyl-ethyl)-1-naphthalin-2-ylmethyl-1,4,8-triaza-spiro[4.5]decan-2-on,
(356) [8-(2-Chlor-pyridin-4-carbonyl)-3-(2-methylsulfanyl-ethyl)-2-oxo-1,4,8-triaza-spiro[4.5]dec-1-yl]-essigsäureethylester,
(357) 1-Allyl-8-(3,5-dimethoxy-benzoyl)-3-(2-methylsulfanyl-ethyl)-1,4,8-triaza-spiro[4.5]decan-2-on,
(358) 3-[8-(4-Methyl-3-nitro-benzoyl)-2-oxo-1,4,8-triaza-spiro[4.5]dec-1-ylmethyl]-benzonitril,
(359) 8-(4-tert-Butyl-benzoyl)-1-(2-fluor-benzyl)-1,4,8-triaza-spiro[4.5]decan-2-on,
(360) 1-Allyl-3-isopropyl-8-(5-methyl-isoxazol-3-carbonyl)-1,4,8-triaza-spiro[4.5]decan-2-on,
(361) 1-Allyl-3-(2-methylsulfanyl-ethyl)-8-(2-trifluormethyl-benzoyl)-1,4,8-triaza-spiro[4.5]decan-2-on,
(362) [3-Benzyl-8-(3-cyclopentyl-propionyl)-2-oxo-1,4,8-triaza-spiro[4.5]dec-1-yl]-essigsäureethylester,
(363) 3-Benzyl-8-(3,5-difluor-benzoyl)-1-(2-fluor-benzyl)-1,4,8-triaza-spiro[4.5]decan-2-on,
(364) [3-Benzyl-8-(5-fluor-2-trifluormethyl-benzoyl)-2-oxo-1,4,8-triaza-spiro[4.5]dec-1-yl]-essigsäureethylester,
(365) [3-Benzyl-2-oxo-8-(3,4,5-trimethoxy-benzoyl)-1,4,8-triaza-spiro[4.5]dec-1-yl]-essigsäureethylester,
(366) Benzoesäure 2-[1-(2-fluor-benzyl)-2-oxo-1,4,8-triaza-spiro[4.5]decan-8-carbonyl]-benzylester,
(367) 1-(2-Fluor-benzyl)-8-(2-methyl-benzoyl)-1,4,8-triaza-spiro[4.5]decan-2-on,
(368) 1-(2-Fluor-benzyl)-8-(2-phenyl-butyryl)-1,4,8-triaza-spiro[4.5]decan-2-on,
(369) 1-Allyl-8-(6-chlor-pyridin-3-carbonyl)-3-isopropyl-1,4,8-triaza-spiro[4.5]decan-2-on,
(370) [8-(2,5-Dimethyl-furan-3-carbonyl)-3-(2-methylsulfanyl-ethyl)-2-oxo-1,4,8-triaza-spiro[4.5]dec-1-yl]-essigsäureethylester,
(371) [8-(5-tert-Butyl-2-methyl-2H-pyrazol-3-carbonyl)-3-(2-methylsulfanyl-ethyl)-2-oxo-1,4,8-triaza-spiro[4.5]dec-1-yl]-essigsäureethylester,
(372) Benzoesäure 2-[1-allyl-3-(2-methylsulfanyl-ethyl)-2-oxo-1,4,8-triaza-spiro[4.5]decan-8-carbonyl]-benzylester,
(373) Essigsäure 2-[1-allyl-3-(2-methylsulfanyl-ethyl)-2-oxo-1,4,8-triaza-spiro[4.5]dec-8-yl]-2-oxo-1-phenyl-ethylester,
(374) 3-[8-(2-Chlor-6-fluor-benzoyl)-3-isopropyl-2-oxo-1,4,8-triaza-spiro[4.5]dec-1-ylmethyl]-benzonitril,
(375) 3-(2-Methylsulfanyl-ethyl)-1-(2-nitro-benzyl)-8-(3-phenyl-propionyl)-1,4,8-triaza-spiro[4.5]decan-2-on,
(376) 3-[8-(2,3-Dimethyl-benzoyl)-3-isopropyl-2-oxo-1,4,8-triaza-spiro[4.5]dec-1-ylmethyl]-benzonitril,
(377) [8-(5-Fluor-2-trifluormethyl-benzoyl)-3-(2-methylsulfanyl-ethyl)-2-oxo-1,4,8-triaza-spiro[4.5]dec-1-yl]-essigsäureethylester,
(378) [8-Cyclopentancarbonyl-3-(2-methylsulfanyl-ethyl)-2-oxo-1,4,8-triaza-spiro[4.5]dec-1-yl]-essigsäureethylester,
(379) [3-(2-Methylsulfanyl-ethyl)-2-oxo-8-(thiophen-2-carbonyl)-1,4,8-triaza-spiro[4.5]dec-1-yl]-essigsäureethylester,
(380) 1-Allyl-8-[1-(4-chlor-phenyl)-5-trifluormethyl-1H-pyrazol-4-carbonyl]-3-(2-methylsulfanyl-ethyl)-1,4,8-triaza-spiro[4.5]decan-2-on,
(381) 1-Allyl-3-(2-methylsulfanyl-ethyl)-8-(naphthalin-1-carbonyl)-1,4,8-triaza-spiro[4.5]decan-2-on,
(382) 1-(2-fluor-benzyl)-8-(4-trifluormethoxy-benzoyl)-1,4,8-triaza-spiro[4.5]decan-2-on,
(383) 3-Benzyl-8-(2-benzyloxy-acetyl)-1-(2-fluor-benzyl)-1,4,8-triaza-spiro[4.5]decan-2-on,
(384) 3-[8-(2-chlor-6-fluor-3-methyl-benzoyl)-3-isopropyl-2-oxo-1,4,8-triaza-spiro[4.5]dec-1-ylmethyl]-benzonitril,
(385) 1-Allyl-8-(2-chlor-5-trifluormethyl-benzoyl)-3-isopropyl-1,4,8-triaza-spiro[4.5]decan-2-on,
(386) 8-(3-Methyl-benzoyl)-3-(2-methylsulfanyl-ethyl)-1-naphthalin-2-ylmethyl-1,4,8-triaza-spiro[4.5]decan-2-on,
(387) 1-(3,5-Dimethyl-benzyl)-8-(2-ethyl-butyryl)-3-isobutyl-1,4,8-triaza-spiro[4.5]decan-2-on,
(388) 8-(5-tert-Butyl-2-methyl-2H-pyrazol-3-carbonyl)-3-(2-methylsulfanyl-ethyl)-1-(2-nitro-benzyl)-1,4,8-triaza-spiro[4.5]decan-2-on,
(389) Benzoesäure 2-[1-(3-cyano-benzyl)-3-isopropyl-2-oxo-1,4,8-triaza-spiro[4.5]decan-8-carbonyl]-benzylester,
(390) 8-(4-Methyl-3-nitro-benzoyl)-3-(2-methylsulfanyl-ethyl)-1-(2-nitro-benzyl)-1,4,8-triaza-spiro[4.5]decan-2-on,

(391) 1-(2,6-Dichlor-benzyl)-3-isobutyl-8-(3,4,5-trimethoxy-benzoyl)-1,4,8-triaza-spiro[4.5]decan-2-on,

(392) 3-{8-[2-(2-Brom-phenyl)-acetyl]-3-isopropyl-2-oxo-1,4,8-triaza-spiro[4.5]dec-1-ylmethyl}-benzonitril,

(393) 3-[8-(2,3-Dichlor-benzoyl)-3-isopropyl-2-oxo-1,4,8-triaza-spiro[4.5]dec-1-ylmethyl]-benzonitril,

(394) 1-Allyl-8-(6-chlor-2H-chroman-3-carbonyl)-3-isopropyl-1,4,8-triaza-spiro[4.5]decan-2-on,

(395) {3-Benzyl-8-[2-(4-methoxy-phenyl)-acetyl]-2-oxo-1,4,8-triaza-spiro[4.5]dec-1-yl}-essigsäureethylester,

(396) 3-[3-Isopropyl-2-oxo-8-(1-phenyl-5-propyl-1H-pyrazol-4-carbonyl)-1,4,8-triaza-spiro[4.5]dec-1-ylmethyl]-benzonitril,

(397) 8-(3-Cyclopentyl-propionyl)-1-(2-fluor-benzyl)-1,4,8-triaza-spiro[4.5]decan-2-on,

(398) 1-Allyl-8-(3-difluormethylsulfanyl-benzoyl)-3-isopropyl-1,4,8-triaza-spiro[4.5]decan-2-on,

(399) 3-[8-(2-chlor-4-nitro-benzoyl)-3-isopropyl-2-oxo-1,4,8-triaza-spiro[4.5]dec-1-ylmethyl]-benzonitril,

(400) [8-(2-Ethoxy-benzoyl)-3-(2-methylsulfanyl-ethyl)-2-oxo-1,4,8-triaza-spiro[4.5]dec-1-yl]-essigsäureethylester,

(401) 8-(2,5-Bis-trifluormethyl-benzoyl)-1-(2-fluor-benzyl)-1,4,8-triaza-spiro[4.5]decan-2-on,

(402) 8-(2-Chlor-pyridin-4-carbonyl)-3-(2-methylsulfanyl-ethyl)-1-(2-nitro-benzyl)-1,4,8-triaza-spiro[4.5]decan-2-on,

(403) {3-Benzyl-8-[3-(2,6-dichlor-phenyl)-5-methyl-isoxazol-4-carbonyl]-2-oxo-1,4,8-triaza-spiro[4.5]dec-1-yl}-essigsäureethylester,

(404) 1-(3,5-Dimethyl-benzyl)-3-isobutyl-8-(2-trifluormethyl-benzoyl)-1,4,8-triaza-spiro[4.5]decan-2-on,

(405) 8-(3,5-Dimethoxy-benzoyl)-3-(2-methylsulfanyl-ethyl)-1-(2-nitro-benzyl)-1,4,8-triaza-spiro[4.5]decan-2-on,

(406) Benzoesäure 2-[3-benzyl-1-(2-fluor-benzyl)-2-oxo-1,4,8-triaza-spiro[4.5]decan-8-carbonyl]-benzylester,

(407) 3-[8-(Benzo[1,2,5]oxadiazol-5-carbonyl)-3-isopropyl-2-oxo-1,4,8-triaza-spiro[4.5]dec-1-ylmethyl]-benzonitril,

(408) [3-(2-Methylsulfanyl-ethyl)-2-oxo-8-(4-trifluormethoxy-benzoyl)-1,4,8-triaza-spiro[4.5]dec-1-yl]-essigsäureethylester,

(409) 1-Allyl-3-isopropyl-8-[2-(4-methoxy-phenyl)-acetyl]-1,4,8-triaza-spiro[4.5]decan-2-on,

(410) 3-(2-Methylsulfanyl-ethyl)-1-(2-nitro-benzyl)-8-(4-propyl-benzoyl)-1,4,8-triaza-spiro[4.5]decan-2-on,

(411) 3-Benzyl-8-(4-tert-butyl-benzoyl)-1-(2-fluor-benzyl)-1,4,8-triaza-spiro[4.5]decan-2-on,

(412) 1-Allyl-8-(2,6-difluor-3-methyl-benzoyl)-3-(2-methylsulfanyl-ethyl)-1,4,8-triaza-spiro[4.5]decan-2-on,

(413) 8-(3,5-Bis-trifluormethyl-benzoyl)-1-(2-fluor-benzyl)-1,4,8-triaza-spiro[4.5]decan-2-on,

(414) 3-[8-(4-Iod-benzoyl)-3-isopropyl-2-oxo-1,4,8-triaza-spiro[4.5]dec-1-ylmethyl]-benzonitril,

(415) 3-(2-Methylsulfanyl-ethyl)-1-naphthalin-2-ylmethyl-8-(thiophen-2-carbonyl)-1,4,8-triaza-spiro[4.5]decan-2-on,

(416) [3-Benzyl-8-(2-chlor-pyridin-4-carbonyl)-2-oxo-1,4,8-triaza-spiro[4.5]dec-1-yl]-essigsäureethylester,

(417) [3-(2-Methylsulfanyl-ethyl)-8-(4-nitro-benzoyl)-2-oxo-1,4,8-triaza-spiro[4.5]dec-1-yl]-essigsäureethylester,

(418) 8-(2-Ethyl-hexanoyl)-3-(2-methylsulfanyl-ethyl)-1-(2-nitro-benzyl)-1,4,8-triaza-spiro[4.5]decan-2-on,

(419) 1-(2,6-Dichlor-benzyl)-3-isobutyl-8-(thiophen-2-carbonyl)-1,4,8-triaza-spiro[4.5]decan-2-on,

(420) [3-Benzyl-8-(2-chlor-4-nitro-benzoyl)-2-oxo-1,4,8-triaza-spiro[4.5]dec-1-yl]-essigsäureethylester,

(421) 3-Benzyl-8-(3,5-bis-trifluormethyl-benzoyl)-1-(2-fluor-benzyl)-1,4,8-triaza-spiro[4.5]decan-2-on,

(422) [3-Benzyl-8-(4-brom-3-methyl-benzoyl)-2-oxo-1,4,8-triaza-spiro[4.5]dec-1-yl]-essigsäureethylester,

(423) 8-Cyclohexancarbonyl-3-(2-methylsulfanyl-ethyl)-1-(2-nitro-benzyl)-1,4,8-triaza-spiro[4.5]decan-2-on,

(424) 8-(2,5-Dimethyl-furan-3-carbonyl)-3-(2-methylsulfanyl-ethyl)-1-(2-nitro-benzyl)-1,4,8-triaza-spiro[4.5]decan-2-on,

(425) 8-(3-Difluormethylsulfanyl-benzoyl)-3-(2-methylsulfanyl-ethyl)-1-(2-nitro-benzyl)-1,4,8-triaza-spiro[4.5]decan-2-on,

(426) 8-(Furan-2-carbonyl)-3-(2-methylsulfanyl-ethyl)-1-(2-nitro-benzyl)-1,4,8-triaza-spiro[4.5]decan-2-on,

(427) [3-Benzyl-8-(2,3-difluor-benzoyl)-2-oxo-1,4,8-triaza-spiro[4.5]dec-1-yl]-essigsäureethylester,

(428) 3-[3-Isopropyl-8-(4-methyl-[1,2,3]thiadiazol-5-carbonyl)-2-oxo-1,4,8-triaza-spiro[4.5]dec-1-ylmethyl]-benzonitril,

(429) 3-[8-(3-Chlor-2-fluor-benzoyl)-3-isopropyl-2-oxo-1,4,8-triaza-spiro[4.5]dec-1-ylmethyl]-benzonitril,

(430) [3-Benzyl-8-(naphthalin-1-carbonyl)-2-oxo-1,4,8-triaza-spiro[4.5]dec-1-yl]-essigsäureethylester,

(431) 8-(4-tert-Butyl-benzoyl)-3-(2-methylsulfanyl-ethyl)-1-(2-nitro-benzyl)-1,4,8-triaza-spiro[4.5]decan-2-on,

(432) 3-[3-Benzyl-1-(2-fluor-benzyl)-2-oxo-1,4,8-triaza-spiro[4.5]decan-8-carbonyl]-benzonitril,

(433) 1-(2,6-Dichlor-benzyl)-8-(furan-2-carbonyl)-3-isobutyl-1,4,8-triaza-spiro[4.5]decan-2-on,

(434) 8-(2,6-Dichlor-benzoyl)-1-(2-fluor-benzyl)-1,4,8-triaza-spiro[4.5]decan-2-on,

(435) 1-(3,5-Dimethyl-benzyl)-8-(3-fluor-4-trifluormethyl-benzoyl)-3-isobutyl-1,4,8-triaza-spiro[4.5]decan-2-on,

(436) 1-(2-Fluor-benzyl)-8-(3-fluor-4-methyl-benzoyl)-1,4,8-triaza-spiro[4.5]decan-2-on,

(437) 1-Allyl-8-(3-cyclopentyl-propionyl)-3-(2-methylsulfanyl-ethyl)-1,4,8-triaza-spiro[4.5]decan-2-on,
(438) 8-[2-(3-Chlor-phenoxy)-acetyl]-3-(2-methylsulfanyl-ethyl)-1-(2-nitro-benzyl)-1,4,8-triaza-spiro[4.5]decan-2-on,
(439) 1-(3,5-Dimethyl-benzyl)-3-isobutyl-8-(2-methylsulfanyl-pyridin-3-carbonyl)-1,4,8-triaza-spiro[4.5]decan-2-on,
(440) 3-(2-Methylsulfanyl-ethyl)-1-(2-nitro-benzyl)-8-propionyl-1,4,8-triaza-spiro[4.5]decan-2-on,
(441) 3-[8-(3,4-Dimethoxy-benzoyl)-3-isopropyl-2-oxo-1,4,8-triaza-spiro[4.5]dec-1-ylmethyl]-benzonitril,
(442) 3-(2-Methylsulfanyl-ethyl)-8-(naphthalin-2-carbonyl)-1-naphthalin-2-ylmethyl-1,4,8-triaza-spiro[4.5]decan-2-on,
(443) 8-(6-Chlor-2H-chroman-3-carbonyl)-3-(2-methylsulfanyl-ethyl)-1-(2-nitro-benzyl)-1,4,8-triaza-spiro[4.5]decan-2-on,
(444) {3-Benzyl-2-oxo-8-[3-(3-trifluormethyl-phenyl)-acryloyl]-1,4,8-triaza-spiro[4.5]dec-1-yl}-essigsäureethylester,
(445) 1-(3,5-Dimethyl-benzyl)-3-isobutyl-8-(3-phenyl-acryloyl)-1,4,8-triaza-spiro[4.5]decan-2-on,
(446) 3-[3-Isopropyl-2-oxo-8-(2-phenoxy-propionyl)-1,4,8-triaza-spiro[4.5]dec-1-ylmethyl]-benzonitril,
(447) 3-[3-Isopropyl-2-oxo-8-(4-trifluormethoxy-benzoyl)-1,4,8-triaza-spiro[4.5]dec-1-ylmethyl]-benzonitril,
(448) 1-Allyl-3-(2-methylsulfanyl-ethyl)-8-(3-nitro-benzoyl)-1,4,8-triaza-spiro[4.5]decan-2-on,
(449) [3-Benzyl-8-(3,4-dichlor-benzoyl)-2-oxo-1,4,8-triaza-spiro[4.5]dec-1-yl]-essigsäureethylester,
(450) 1-(2-Fluor-benzyl)-8-[3-(3-trifluormethyl-phenyl)-acryloyl]-1,4,8-triaza-spiro[4.5]decan-2-on,
(451) 1-Allyl-8-(3,5-bis-trifluormethyl-benzoyl)-3-(2-methylsulfanyl-ethyl)-1,4,8-triaza-spiro[4.5]decan-2-on,
(452) 8-(3-Cyclopentyl-propionyl)-3-(2-methylsulfanyl-ethyl)-1-(2-nitro-benzyl)-1,4,8-triaza-spiro[4.5]decan-2-on,
(453) 1-(3,5-Dimethyl-benzyl)-3-isobutyl-8-(4-nitro-benzoyl)-1,4,8-triaza-spiro[4.5]decan-2-on,
(454) 8-(3-Cyclopentyl-propionyl)-1-(3,5-dimethyl-benzyl)-3-isobutyl-1,4,8-triaza-spiro[4.5]decan-2-on,
(455) 3-[3-Isopropyl-8-(isoxazol-5-carbonyl)-2-oxo-1,4,8-triaza-spiro[4.5]dec-1-ylmethyl]-benzonitril und
(456) 3-Benzyl-1-(2-fluor-benzyl)-8-(2-methyl-6-trifluormethyl-pyridin-3-carbonyl)-1,4,8-triaza-spiro[4.5]decan-2-on,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, vorzugsweise Hydrochloride, oder jeweils in Form entsprechender Solvate.

Ein weiterer Gegenstand der vorliegenden Erfindung ist ein Verfahren zur Herstellung der erfindungsgemäßen substituierten 1,4,8-Triazaspiro[4.5]decan-2-on-Verbindungen, gemäß dem ein geschütztes Piperidin-4-on der allgemeinen Formel 11,

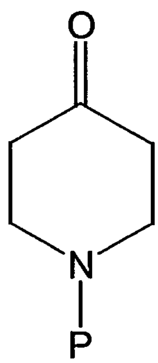

II

worin P für eine Schutzgruppe steht, durch Umsetzung mit wenigstens einer Verbindung der allgemeinen Formel III,

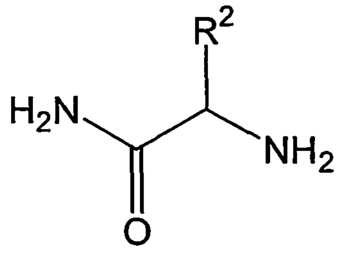

III

worin $R^2$ die vorstehend genannte Bedeutung, in wenigstens eine Verbindung der allgemeinen Formel IV,

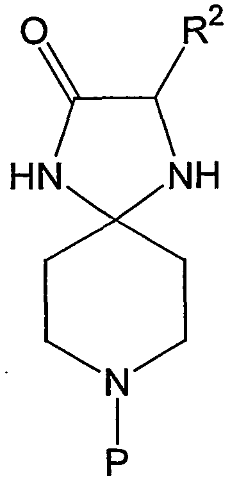

IV

worin P und $R^2$ die vorstehend genannte Bedeutung haben, überführt wird, welche ggf. gereinigt und/oder ggf. isoliert wird, und gegebenenfalls durch Umsetzung mit wenigstens einer Verbindung der allgemeinen Formel $R^1$-$X^1$, worin $R^1$ die vorstehend genannte Bedeutung hat und $X^1$ für eine geeignete Austrittsgruppe, vorzugsweise für einen Halogen-Rest, steht, ggf. in Gegenwart wenigstens einer Base, in wenigstens eine Verbindung der allgemeinen Formel V,

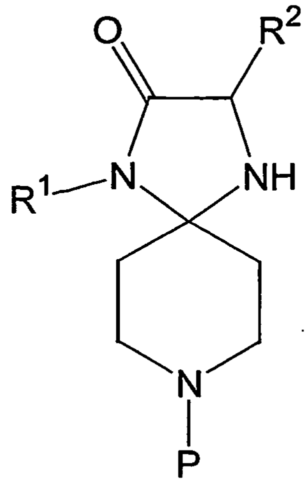

V

worin $R^1$, $R^2$ und P die vorstehend genannte Bedeutung haben, überführt wird, und diese ggf. gereinigt und/oder ggf. isoliert wird, und ggf. durch Abspaltung der Schutzgruppe P in wenigstens eine Verbindung der allgemeinen Formel VI,

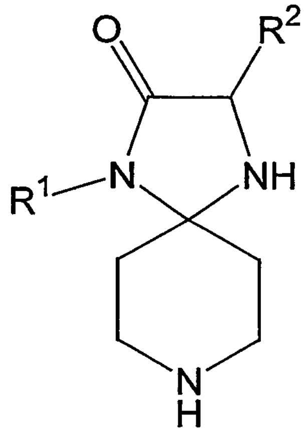

VI

überführt wird, diese ggf. gereinigt und/oder ggf. isoliert wird, und ggf. wenigstens eine Verbindung der allgemeinen Formel IV, V oder VI durch Umsetzung mit einem Carbonsäurederivat der allgemeinen Formel $R^4$-(C=O)-$X^2$, einem Carbonsäureanhydrid der allgemeinen Formel $(R^4\text{-(C=O)})_2O$ oder einer Verbindung der allgemeinen Formel $R^3$-$X^3$, worin der Rest $R^3$ die vorstehend genannte Bedeutung mit Ausnahme von Wasserstoff und -(C=O)-$R^4$ hat, $R^4$ jeweils die vorstehend genannte Bedeutung hat und $X^2$ und $X^3$ jeweils für eine geeignete Austrittsgruppe, vorzugsweise für einen Halogen-Rest, stehen, in wenigstens eine erfindungsgemäße substituierte 1,4,8-Triazaspiro[4.5]decan-2-on-Verbindung überführt wird, und diese Verbindung ggf. gereinigt und/oder ggf. isoliert wird,
oder
4-Piperidon der Formel VII, ggf. in Form eines entsprechenden Salzes,

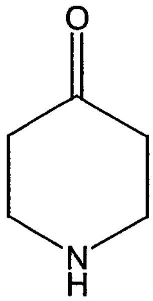

VII

durch Umsetzung mit einem Carbonsäurederivat der allgemeinen Formel $R^4$-(C=O)-$X^2$, einem Carbonsäureanhydrid der allgemeinen Formel $(R^4\text{-(C=O)})_2O$ oder einer Verbindung der allgemeinen Formel $R^3$-$X^3$, worin $R^3$ die vorstehend genannte Bedeutung mit Ausnahme von Wasserstoff und -(C=O)-$R^4$ hat, der Rest $R^4$ jeweils die vorstehend genannte Bedeutung hat und $X^2$ und $X^3$ jeweils für eine geeignete Austrittsgruppe, vorzugsweise für einen Halogen-Rest, stehen, in eine Verbindung der allgemeinen Formel VIII,

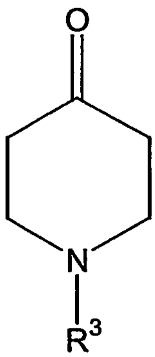

VIII

überführt wird, worin $R^3$ die vorstehend genannte Bedeutung hat, diese ggf. gereinigt und/oder isoliert wird und durch Umsetzung mit wenigstens einer Verbindung der allgemeinen Formel III

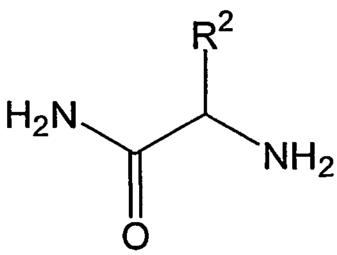

III

worin $R^2$ die vorstehend genannte Bedeutung hat, in wenigstens eine Verbindung der allgemeinen Formel IX,

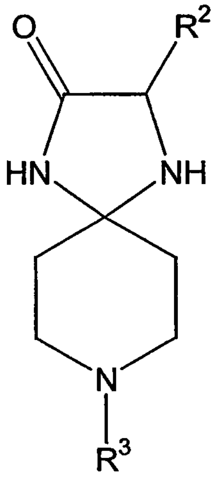

IX

überführt wird, worin $R^2$ und $R^3$ jeweils die vorstehend genannte Bedeutung haben, diese ggf. gereinigt und/oder isoliert wird und durch Umsetzung mit wenigstens einer Verbindung der allgemeinen Formel $R^1$-$X^1$, worin $R^1$ und $X^1$ die vorstehend genannte Bedeutung haben, ggf. in Gegenwart wenigstens einer Base, in wenigstens eine erfindungsgemäße substituierte 1,4,8-Triazaspiro[4.5]decan-2-on-Verbindung überführt wird und diese ggf. gereinigt und/oder isoliert wird.

Die N-geschützten Piperidin-4-on-Verbindungen der allgemeinen Formel II sind - ebenso wie ungeschütztes Piperidin-2-on und entsprechende Salze wie beispielsweise dessen Hydrochlorid - käuflich am Markt erhältlich oder können nach üblichen, dem Fachmann bekannten Methoden hergestellt werden. Geeignete Schutzgruppen sind beispielsweise Trifluoracetamid, Methoxycarbonyl, Ethoxycarbonyl, tert-Butoxycarbonyl, Benzyloxycarbonyl, Allyloxycarbonyl oder 9-Fluorenylmethoxycarbonyl,

Die Aminosäureamide der allgemeinen Formel III, die in dem erfindungsgemäßen Verfahren auch in Form ihres entsprechenden Salzes eingesetzt werden können, sind ebenfalls käuflich am Markt erhältlich oder können nach üblichen, dem Fachmann bekannten Methoden hergestellt werden. Die jeweiligen Aminosäureamide können in dem erfindungsgemäßen Verfahren sowohl in enantiomerenreiner Form, d.h. in (*S*)- oder (*R*)-Konfiguration, oder in Form eines vorzugsweise racemischen Gemisches mit (*S,R*)-Konfiguration eingesetzt werden.

Die Umsetzung von Verbindungen der allgemeinen Formel II mit Verbindungen der allgemeinen Formel III zu N-geschützten, ggf. in 3-Position substituierten 1,4,8-triaza-spiro[4.5]decan-2-on-Verbindungen der allgemeinen Formel IV kann unter üblichen, dem Fachmann bekannten Bedingungen erfolgen. Vorzugsweise erfolgt die Umsetzung in einem geeigneten Reaktionsmedium, beispielsweise in einem oder mehreren trockenen organischen Lösungsmitteln. Geeignete Lösungsmittel sind beispielsweise Alkohole wie Ethanol oder chlorierte Kohlenwasserstoffe wie Methylenchlorid oder Chloroform. Die Temperatur während der Vereingung und Umsetzung der Reaktionskomponenten kann über einen weiten Bereich variieren.

Die Umsetzung einer N-geschützten, ggf. in 3-Position substituierten 1,4,8-triaza-spiro[4.5]decan-2-on-Verbindung der allgemeinen Formel IV mit einer Verbindung der allgemeinen Formel $R^1$-$X^1$ zu Verbindungen der allgemeinen Formel V erfolgt bevorzugt in einem Reaktionsmedium in Gegenwart wenigstens einer organischen Base und/oder in Gegenwart wenigstens einer anorganischen Base unter üblichen, dem Fachmann bekannten Bedingungen. Die Umsetzung kann vorteilhafterweise auch in der Mikrowelle durchgeführt werden.

Geeignete anorganische Basen sind beispielsweise Metallalkoholate wie Natriummethanolat, Natriumethanolat, Kalium-tert-butylat, Lithium- oder Natriumbasen wie Lithiumdiisopropylamid, Buthyllithium, tert-Butyllithium, Natriummethylat oder Metallhydride wie Kaliumhydrid, Lithiumhydrid, Natriumhydrid. Als geeignete organische Basen kommen beispielsweise Diisopropylethylamin oder Triethylamin in Betracht. Geeignete Reaktionsmedien sind organische Lösungsmittel wie beispielsweise Tetrahydrofuran.

Die Abspaltung der Schutzgruppe (P) zur Herstellung von N-ungeschützten, ggf. in 1-und/oder 3-Position substituierten 1,4,8-triaza-spiro[4.5]decan-2-on-Verbindungen der allgemeinen Formel VI kann ebenfalls unter üblichen, dem Fachmann bekannten Bedingungen erfolgen, die jeweils in Abhängigkeit von der verwendeten Schutzgruppe variieren. Beispielhaft seien die Abspaltung in Gegenwart einer anorganischen Base, Säure oder Lewis-Säure, wie Kaliumcarbonat, Lithiumhydroxid, Kaliumhydroxid, Schwefelsäure, Bromwasserstoffsäure, Flusssäure, Salzsäure, Bortrifluoridetherat, Bortrichlorid, einer organischen Säure wie Trifluoressigsäure, Trifluormethansulfonsäure, Essigsäure, die Abspaltung in Gegenwart einer organischen Base, wie Morpholin, Triethylamin, Diethylamin, Diisopropylethylamin, Pyridin oder die Hydrierung genannt.

Die jeweilige Verbindungen der allgemeinen Formel IV, V oder VI, insbesondere die jeweilige Verbindung der allgemeinen Formel VI, kann anschließend durch Umsetzung mit einem Carbonsäurederivat der allgemeinen Formel $R^4$-(C=O)-$X^2$ oder einem Carbonsäureanhydrid der allgemeinen Formel ($R^4$-(C=O))$_2$O, worin der Rest $R^4$ jeweils die vorstehend genannte Bedeutung hat und $X^2$ für eine geeignete Austrittsgruppe, vorzugsweise für einen Halogen-Rest, steht vorzugsweise in Gegenwart wenigstens einer organischen Base und/oder wenigstens einer anorganischen Base wie beispielsweise Diisopropylethylamin, Triethylamin, Pyridin oder Diethylamin, ggf. in Gegenwart eines geeigneten Katalysators wie beispielsweise DMAP (Dimethylaminopyridin) unter üblichen, dem Fachmann bekannten Methoden in die jeweilige erfindungsgemäße substituierte 1,4,8-triaza-spiro[4.5]decan-2-on-Verbindung einschließlich entsprechender Stereoisomeren übergeführt werden.

Die jeweilige Verbindungen der allgemeinen Formel IV, V oder VI, insbesondere die jeweilige Verbindung der allgemeinen Formel VI, kann anschließend auch durch Umsetzung mit einer Verbindung der allgemeinen Formel $R^3$-$X^3$, worin $R^3$ die vorstehend genannte Bedeutung mit Ausnahme von Wasserstoff und -(C=O)-$R^4$ hat und $X^2$ für eine geeignete Austrittsgruppe, vorzugsweise für einen Halogen-Rest wie beispielsweise Chlor, steht vorzugsweise in Gegenwart wenigstens einer organischen Base und/oder wenigstens einer anorganischen Base wie beispielsweise Diisopropylethylamin, Triethylamin, Pyridin oder Diethylamin nach üblichen, dem Fachmann bekannten Methoden in die jeweilige erfindungsgemäße substituierte 1,4,8-triaza-spiro[4.5]decan-2-on-Verbindung einschließlich entsprechender Stereoisomeren übergeführt werden.

Die Umsetzung von Verbindungen der allgemeinen Formel IV, V oder VI, insbesondere von Verbindungen der allgemeinen Formel VI, zu einer erfindungsgemäßen substituierten 1,4,8-triaza-spiro[4.5]decan-2-on-Verbindung der allgemeinen Formel I erfolgt bevorzugt in einem geeigneten Reaktionsmedium, beispielsweise in einem oder mehreren trockenen organischen Lösungsmitteln. Geeignete Lösungsmittel sind beispielsweise ggf. chlorierte, ggf. aromatische Kohlenwasserstoffe wie Toluol, Methylenchlorid oder Chloroform. Die Temperatur während der Vereingung und Umsetzung der Reaktionskomponenten kann über einen weiten Bereich variieren.

Die Umsetzung von 4-Piperidon, vorzugsweise in Form des Hydrochlorid-Salzes, mit einem Carbonsäurederivat $R^4$-(C=O)-$X^2$ oder einem Carbonsäurenahydrid ($R^4$-(C=O))$_2$O erfolgt bevorzugt in Gegenwart wenigstens einer organischen Base und/oder in Gegenwart wenigstens einer anorganischen Base. Als geeignete Basen seien Diisopropylethylamin, Triethylamin, Pyridin oder Diethylamin genannt.

Die Umsetzung von 4-Piperidon, vorzugsweise in Form des Hydrochlorid-Salzes, mit einer Verbindung $R^3$-$X^3$, worin $R^3$ die vorstehend genannte Bedeutung mit Ausnahme von Wasserstoff und -(C=O)-$R^4$ hat und $X^3$ für eine geeignete Austrittsgruppe, vorzugsweise für einen Halogenrest wie beispielsweise Chlor, steht, erfolgt bevorzugt in Gegenwart wenigstens einer organischen Base und/oder in Gegenwart wenigstens einer anorganischen Base. Als geeignete organische Basen seien Diisopropylethylamin, Triethylamin, Pyridin oder Diethylamin genannt. Als Reaktionsmedium kommen bevorzugt getrockente organische Lösungsmittel wie beispielsweise trochenes Tetrahydrofuran in Betracht.

Die Umsetzung von Verbindungen der allgemeinen Formel VIII mit Verbindungen der allgemeinen Formel III zu in 3-Position substituierten 1,4,8-triaza-spiro[4.5]decan-2-on-Verbindungen der allgemeinen Formel IX kann unter üblichen, dem Fachmann bekannten Bedingungen erfolgen. Vorzugsweise erfolgt die Umsetzung in einem geeigneten Reaktionsmedium, beispielsweise in einem oder mehreren trockenen organischen Lösungsmitteln. Geeignete Lösungsmittel sind beispielsweise Alkohole wie Ethanol oder chlorierte Kohlenwasserstoffe wie Methylenchlorid oder Chloroform. Die Temperatur während der Vereingung und Umsetzung der Reaktionskomponenten kann über einen weiten Bereich variieren.

Die Umsetzung einer in 3-Position substituierten 1,4,8-triaza-spiro[4.5]decan-2-on-Verbindung der allgemeinen Formel IX mit einer Verbindung der allgemeinen Formel $R^1$-$X^1$ zu einer erfindungsgemäßen 1,4,8-triaza-spiro[4.5]decan-2-on-Verbindung erfolgt bevorzugt in einem Reaktionsmedium in Gegenwart wenigstens einer organischen Base und/ oder in Gegenwart wenigstens einer anorganischen Base unter üblichen, dem Fachmann bekannten Bedingungen. Die Umsetzung kann vorteilhafterweise auch in der Mikrowelle durchgeführt werden.

Geeignete anorganische Basen sind beispielsweise Metallalkoholate wie Natriummethanolat, Natriumethanolat, Kalium-tert-butylat, Lithium- oder Natriumbasen wie Lithiumdiisopropylamid, Buthyllithium, tert-Butyllithium, Natriummethylat oder Metallhydride wie Kaliumhydrid, Lithiumhydrid, Natriumhydrid. Als geeignete organische Basen kommen beispielsweise Diisopropylethylamin oder Triethylamin in Betracht. Geeignete Reaktionsmedien sind organische Lösungsmittel wie beispielsweise Tetrahydrofuran.

Die Verbindungen der allgemeinen Formeln $R^1$-$X^1$, $R^4$-(C=O)-$X^2$ und ($R^4$-(C=O))$_2$O, sind jeweils am Markt käuflich erhältlich und/oder können nach üblichen, dem Fachmann bekannten Methoden hergestellt werden. $X^1$ und $X^2$

sind übliche, dem Fachmann bekannte Austrittsgruppen, vorzugsweise Halogen-Reste, besonders bevorzugt Chlor-Reste.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reingungsverfahren sind beispielsweise Extraktionsverfahren und chromatographische Verfahren wie Säulenchromatographie oder präparative Chromatographie.

Die erfindungsgemäßen substituierten 1,4,8-triaza-spiro[4.5]decan-2-on-Verbindungen der vorstehenden allgemeinen Formeln I sowie ggf. jeweils entsprechende Stereoisomere können nach üblichen, dem Fachmann bekannten Verfahren in Form entsprechender Salze, insbesondere in Form entsprechender physiologisch verträglicher Salze erhalten werden, erhalten werden, wobei das erfindungsgemäße Arzneimittel eines oder mehrere Salze einer oder mehrerer dieser Verbindungen aufweisen kann.

Die jeweiligen Salze der erfindungsgemäßen substituierten 1,4,8-triaza-spiro[4.5]decan-2-on-Verbindungen der vorstehenden allgemeinen Formel I können beispielsweise durch Umsetzung mit einer oder mehreren anorganischen Säuren und/oder einer oder mehreren organischen Säuren erhalten werden. Geeignete Säuren können bevorzugt ausgewählt werden aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, $\alpha$-Liponsäure, Acetylglycin, Hippursäure, Phosphorsäure, Maleinsäure, Malonsäure und Asparaginsäure. Die entsprechenden Hydrochloridsalze werden bevorzugt durch Umsetzung mit Trimethylsilylchlorid in Ethylmethylketon erhalten.

Die erfindungsgemäßen substituierten 1,4,8-triaza-spiro[4.5]decan-2-on-Verbindungen der vorstehenden allgemeinen Formel I sowie ggf. entsprechende Stereoisomere und jeweils deren physiologisch verträgliche Salze können auch nach üblichen, dem Fachmann bekannten Verfahren in Form ihrer Solvate, insbesondere in Form ihrer Hydrate erhalten werden.

Sofern die erfindungsgemäßen substituierten 1,4,8-triaza-spiro[4.5]decan-2-on-Verbindungen der vorstehenden allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Es wurde überraschenderweise gefunden, daß die 1,4,8-Triazaspiro[4.5]decan-2-on-Verbindungen der vorstehend angegebenen allgemeinen Formel I sich nicht nur zur Regulation, vorzugsweise zur Inhibierung, der Noradrenalin-Wiederaufnahme und/oder zur Regulation, vorzugsweise zur Inhibierung, der 5-Hydroxy-Tryptophan-Wiederaufnahme eignen, sondern darüber hinaus auch eine hohe Affinität für Opioid-Rezeptoren, insbesondere für $\mu$-Opioid-Rezeptoren und für Batrachotoxin-(BTX)-Rezeptoren aufweisen und sich daher insbesondere als pharmazeutische Wirkstoffe in Arzneimitteln zur Prophylaxe und/oder Behandlung von mit diesen Rezeptoren bzw. Prozessen in Verbindung stehenden Erkrankungen eignen.

Die 1,4,8-Triazaspiro[4.5]decan-2-on-Verbindungen der vorstehend angegebenen allgemeinen Formel I und ggf. entsprechende Stereoisomere sowie jeweils die entsprechenden Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße 1,4,8-Triazaspiro[4.5]decan-2-on-Verbindung der vorstehend angegebenen allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates, sowie ggf. wenigstens einen pharmazeutisch verträglichen Hilfsstoff.

Bevorzugt sind Arzneimittel, die wenigstens eine 1,4,8-Triazaspiro[4.5]decan-2-on-Verbindung der vorstehend angegebenen allgemeinen Formel I mit Ausnahme der Verbindungen gemäß A) und/oder mit Ausnahme der Verbindungen gemäß B) enthalten, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates, sowie ggf. wenigstens einen pharmazeutisch verträglichen Hilfsstoff enthalten.

Vorzugsweise eignet sich das erfindungsgemäße Arzneimittel zur Regulation, insbesondere zur Hemmung,

der Noradrenalin-Wiederaufnahme (Noradrenalin-Uptake), zur Regulation, insbesondere zur Hemmung, der 5-Hydroxy-Tryptophan-Wiederaufnahme (5-HT-Uptake), zur Opioid-Rezeptor-Regulation, insbesondere zur μ-Opioid-Rezeptor-Regulation und/oder zur Batrachotoxin-(BTX)-Rezeptor-Regulation.

Ebenfalls bevorzugt eignet sich das erfindungsgemäße Arzneimittel zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, besonders bevorzugt zur Prophylaxe und/oder Behandlung von Fettsucht.

Ebenfalls bevorzugt eignet sich das erfindungsgemäße Arzneimittel zur Prophylaxe und/oder Behandlung von Schmerzen, vorzugsweise zur Behandlung und/oder Prophylaxe von akuten Schmerzen, chronischen Schmerzen, neuropathischen Schmerzen und/oder Clusterkopfschmerzen, oder zur Prophylaxe und/oder Behandlung von Depressionen.

Besonders bevorzugt eignet sich das erfindungsgemäße Arzneimittel zur kombinierten Prophylaxe und/oder Behandlung von Depressionen und Schmerzen, vorzugsweise zur kombinierten Behandlung von Depressionen und Schmerzen ausgewählt aus der Gruppe bestehend aus akuten Schmerzen, chronischen Schmerzen, neuropathischen Schmerzen und Clusterkopfschmerzen.

Weiterhin bevorzugt eignet sich das erfindungsgemäße Arzneimittel zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Katalepsie, zur Vigilanzsteigerung, zur Libidosteigerung, zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose, zur Prophylaxe und/oder Behandlung von Ischämien und/oder zur Lokalanästhesie.

Ein weiterer Gegenstand der vorliegenden Erfindung ist die Verwendung wenigstens einer erfindungsgemäßen substituierten 1,4,8-Triazaspiro[4.5]decan-2-on-Verbindung der allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates, zur Herstellung eines Arzneimittels zur Regulation der Noradrenalin-Wiederaufnahme (Noradrenalin-Uptake), vorzugsweise zur Hemmung der Noradrenalin-Wiederaufnahme (Noradrenalin-Uptake), zur Regulation der 5-Hydroxy-Tryptophan-Wiederaufnahme (5-HT-Uptake), vorzugsweise zur Hemmung der 5-Hydroxy-Tryptophan-Wiederaufnahme (5-HT-Uptake), zur Opioid-Rezeptor-Regulation, vorzugsweise zur μ-Opioid-Rezeptor-Regulation und/oder zur zur Batrachotoxin-(BTX)-Rezeptor-Regulation.

Bevorzugt ist die Verwendung wenigstens einer erfindungsgemäßen substituierten 1,4,8-Triazaspiro[4.5]decan-2-on-Verbindung der allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates zur Herstellung eines Arzneimittels zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, besonders bevorzugt Fettsucht.

Bevorzugt ist die Verwendung wenigstens einer erfindungsgemäßen substituierten 1,4,8-Triazaspiro[4.5]decan-2-on-Verbindung der allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates zur Herstellung eines Arzneimittels zur Prophylaxe und/oder Behandlung von Schmerzen, vorzugsweise von akuten Schmerzen, chronischen Schmerzen, neuropathischen Schmerzen und/oder Clusterkopfschmerzen.

Bevorzugt ist ferner die Verwendung wenigstens einer erfindungsgemäßen substituierten 1,4,8-Triazaspiro[4.5]decan-2-on-Verbindung der allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates zur Herstellung eines Arzneimittels zur Prophylaxe und/oder Behandlung von Depressionen.

Bevorzugt ist weiterhin die Verwendung wenigstens einer erfindungsgemäßen substituierten 1,4,8-Triazaspiro[4.5]decan-2-on-Verbindung der allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates zur Herstellung eines Arzneimittels zur kombinierten Prophylaxe und/oder Behandlung von Depressionen und Schmerzen, vorzugsweise

zur kombinierten Behandlung von Depressionen und Schmerzen ausgewählt aus der Gruppe bestehend aus akuten Schmerzen, chronischen Schmerzen, neuropathischen Schmerzen und Clusterkopfschmerzen.

Bevorzugt ist auch die Verwendung wenigstens einer erfindungsgemäßen substituierten 1,4,8-Triazaspiro[4.5]decan-2-on-Verbindung der allgemeinen Formel I, ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates, zur Herstellung eines Arzneimittels zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder, zur Prophylaxe und/oder Behandlung von Katalepsie, zur Vigilanzsteigerung, zur Libidosteigerung, zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose, zur Prophylaxe und/oder Behandlung von Ischämien und/oder zur Lokalanästhesie.

Besonders bevorzugt kann auch jeweils die Verwendung wenigstens einer erfindungsgemäßen substituierten 1,4,8-Triazaspiro[4.5]decan-2-on-Verbindung der allgemeinen Formel I mit Ausnahme der Verbindungen gemäß A) und/oder mit Ausnahme der Verbindungen gemäß B), jeweils ggf. in Form eines ihrer reinen Stereoisomeren, insbesondere Enantiomeren oder Diastereomeren, ihres Racemates oder in Form einer Mischung von Stereoisomeren, insbesondere der Enantiomeren und/oder Diastereomeren, in einem beliebigen Mischungsverhältnis, oder jeweils in Form eines entsprechenden Salzes, vorzugsweise Hydrochlorids, oder jeweils in Form eines entsprechenden Solvates, zur Herstellung eines Arzneimittels zur Prophylaxe und/oder Behandlung der vorstehend genannten Krankheiten bzw. Störungen erfolgen.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren der in dem erfindungsgemäßen Arzneimittel zum Einsatz kommenden substituierten 1,4,8-Triazaspiro[4.5]decan-2-on-Verbindungen der vorstehenden allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten 1,4,8-Triazaspiro[4.5]decan-2-on-Verbindungen in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten 1,4,8-Triazaspiro[4.5]decan-2-on-Verbindungen auch verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen substituierten 1,4,8-Triazaspiro[4.5]decan-2-on-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 50 mg/kg, vorzugsweise 0,05 bis 50 mg/kg Körpergewicht des Patienten wenigstens einer erfindungsgemäßen substituierten 1,4,8-Triazaspiro[4.5]decan-2-on-Verbindung appliziert.

**Pharmakologischen Methoden:**

**I. Analgesieprüfung im Writhing-Test an der Maus**

Die Untersuchung auf analgetische Wirksamkeit wird im Phenylchinon-induzierten Writhing an der Maus (modifiziert nach I.C. Hendershot und J. Forsaith (1959) J. Pharmacol. Exp. Ther. 125, 237-240) durchgeführt. Die entsprechende Beschreibung wird hiermit als Referenz eingeführt und gilt als Teil der vorliegenden Offenbarung.

Dazu werden männliche NMRI-Mäuse im Gewicht von 25 bis 30 g verwendet. Gruppen von 10 Tieren pro Substanzdosis erhalten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45°C) intraperitoneal appliziert. Die Tiere werden einzeln in Beobachtungskäfige gesetzt. Mittels eines Drucktastenzähler wird die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle werden Tiere mitgeführt, die nur physiologische Kochsalzlösung erhalten. Alle Substanzen werden in der Standarddosierung von 10 mg/kg getestet. Die prozentuale Hemmung (%Hemmung) der Writhingreaktion durch eine Substanz wird nach folgender Formel berechnet:

$$\% \text{ Hemmung} = 100 - \frac{\text{Writhingreaktionen der behandelten Tiere} * 100}{\text{Writhingreaktionen der Kontrolltiere}}$$

Für einige Substanzen wird aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.

**II. Methode zur Bestimmung der Affinität zum humanen μ-Opioidrezeptor:**

Die Rezeptoraffinität zum humanen μ-Opioidrezeptor wird in einem homogenen Ansatz in Mikrotiterplatten bestimmt. Hierzu werden Verdünnungsreihen der zu prüfenden Substanzen mit einer Rezeptormembranpräparation (15-40 μg Protein /250 μl Inkubationsansatz) von CHO-K1-Zellen, welche den humanen μ-Opioidrezeptor exprimieren (RB-HOM-Rezeptormembran-Präparation von der Firma NEN, Zaventem, Belgien) in Gegenwart von 1 nmol/l des radioaktiven Liganden [$^3$H]-Naloxon (NET 719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 μl für 90 Minuten bei Raumtemperatur inkubiert. Als Inkubationspuffer wird 50 mmol/l Tris-HCl supplementiert mit 0,05 % (Gewicht/Volumen) Natriumazid und mit 0,06 % (Gewicht/Volumen) bovinem Serumalbumin verwendet. Zur Bestimmung der unspezifischen Bindung wird zusätzlich 25 μmol/l Naloxon zugegeben. Nach Beendigung der neunzigminütigen Inkubationszeit werden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wird die prozentuale Verdrängung des radioaktiven Liganden aus seiner Bindung zum humanen μ-Opioidrezeptor bei einer Konzentration der Prüfsubstanzen von 1 μmol/l bestimmt und als Prozent Hemmung der spezifischen Bindung angegeben.

Ausgehend von der prozentualen Verdrängung durch unterschiedliche Konzentrationen der Prüfsubstanzen werden $IC_{50}$ Hemmkonzentrationen berechnet, die eine 50-prozentige Verdrängung des radioaktiven Liganden bewirken. Durch Umrechnung mittels der Cheng-Prusoff-Beziehung können die Ki-Werte für die Prüfsubstanzen erhalten werden, wie in der Veröffentlichung von Y. Cheng, W.H. Prusoff, 1973, Biochem. Pharmacol., 22, Seiten 3099-3108 beschrieben. Die entsprechende Beschreibung wird hiermit als Referenz eingeführt und gilt als Teil der vorliegenden Offenbarung.

**III. Methode zur Bestimmung der Noradrenalin- und der 5HT-Uptake-Inhibierung:**

Für in vitro Studien werden Synaptosomen aus Rattenhimarealen frisch isoliert, wie in der Veröffentlichung "The isolation of nerve endings from brain" von E.G. Gray und V.P. Whittaker, J. Anatomy 96, Seiten 79-88, 1962,

beschrieben. Die entsprechende Literaturbeschreibung wird hiermit als Referenz eingeführt und gilt als Teil der Offenbarung.

Das Gewebe (Hypothalamus für die Bestimmung der Noradrenalin-Uptake-Inhibierung und Mark und Pons für die Bestimmung der 5HT-Uptake-Inhibierung) wird in eisgekühlter 0,32 M Sucrose (100 mg Gewebe / 1 mL) in einem Glas-Homogenisierer mit Teflonstößel homogenisiert, indem fünf volle Auf- und Abschläge bei 840 Umdrehungen /Minute benutzt werden.

Das Homogenat wird bei 4°C für 10 Minuten bei 1000 g zentrifugiert. Nach anschließender Zentrifugierung bei 17000 g für 55 Minuten erhält man die Synaptosomen ($P_2$-Fraktion), die in 0,32 M Glucose (0,5 mU 100 mg des ursprünglichen Gewichts) noch einmal suspendiert werden.

Der jeweilige Uptake wird in einer 96-well Mikrotiterplatte gemessen. Das Volumen beträgt 250 µl und die Inkubation erfolgt bei Raumtemperatur (ca. 20-25 °C) unter $O_2$ Atmosphäre.

Die Inkubationszeit beträgt 7,5 Minuten für [$^3$H]-NA und 5 Minuten für [$^3$H]-5-HT. Anschließend werden die 96 Proben durch eine Unifilter GF/B® Mikrotiterplatte (Packard) filtriert und mit 200 mL inkubierten Puffer mit Hilfe eines "Brabdel Cell-Harvester MPXRI-96T" gewaschen. Die Unifilter GF/B Platte wird bei 55°C 1 Stunde getrocknet. Im Anschluß wird die Platte mit einem Back seal® (Packard) verschlossen und mit 35 µl Szintilationsflüssigkeit pro Well (Ultima Gold®, Packard) versetzt. Nach dem Verschließen mit einem top seal® (Packard) wird, nach der Einstellung des Gleichgewichts (etwa 5 Stunden), die Radioaktivität in einem "Trilux 1450 Microbeta" (Wallac, Freiburg, Deutschland) bestimmt.

Die Menge des bei der vorstehenden Bestimmung eingesetzten Proteins entspricht den aus der Literatur bekannten Werten, wie z.B. in "Protein measurement with the folin phenol reagent", Lowry et al., J. Biol. Chem., 193, 265-275, 1951 beschrieben.

Eine detaillierte Methodenbeschreibung kann ferner auch der Literatur, beispielsweise aus M.Ch. Frink, H.-H. Hennies, W. Engelberger, M. Haurand und B. Wilffert (1996) Arzneim.-Forsch./Drug Res. 46 (III), 11, 1029-1036, entnommen werden. Folgende Kenndaten wurden für den NA- bzw. 5-HT-Transporter ermittelt:

$$\text{NA-Uptake}: \ Km = 0,32 \pm 0,11\ \mu M$$

$$\text{5HT-Uptake}: \ Km = 0,084 \pm 0,011\ \mu M$$

**IV. Methode zur Bestimmung der Affinität zur Batrachotoxin-(BTX)-Bindungsstelle des Natriumkanals:**

Die Bindungsstelle 2 des Natriumkanals ist die sogenannte Batrachotoxin-(BTX) Bindungsstelle. Als Ligand wird [$^3$H]-Batrachotoxinin A20 α-Benzoat (10 nM im Ansatz) eingesetzt. Die Ionenkanal-Partikel (Synaptosomen) werden aus dem Ratten Cerebrocortex angereichert, wie in der Veröffentlichung von Gray und Whittaker, 1962, J. Anat. 76, 79-88 beschrieben. Die entsprechende Beschreibung wird hiermit als Referenz eingeführt und gilt als Teil der vorliegenden Offenbarung. Als unspezifische Bindung ist die Radioaktivität definiert, die in Gegenwart von Veratridin ($3 \times 10^{-4}$ M im Ansatz) gemessen wird.

Die Assaybedingungen werden entsprechend der Veröffentlichung von Pauwels, Leysen und Laduron durchgeführt, wie in Eur. J. Pharmacol. 124, 291-298 beschrieben. Die entsprechende Beschreibung wird hiermit als Referenz eingeführt und gilt als Teil der vorliegenden Offenbarung.

Abweichend von dieser Vorschrift wird der Gesamtansatz auf 250 µl verkleinert, so daß der Assay auf 96-well Mikrotiterplatten durchgeführt werden kann. Die Inkubationszeit in diesen Mikrotiterplatten beträgt zwei Stunden bei Raumtemperatur (ca. 20-25°C).

Folgende Kenndaten wurden für den $K_D$-Wert der Bindungsstelle ermittelt:

$K_D$: 24,63 ± 1,56 nM.

Im folgenden wird die Erfindung anhand von Beispielen erläutert. Diese Erläuterungen sind lediglich beispielhaft und schränken den allgemeinen Erfindungsgedanken nicht ein.

**Beispiele:**

Die Ausbeuten der hergestellten Verbindungen sind nicht optimiert.

Alle Temperaturen sind unkorrigiert.

Die Angabe "Ether" bedeutet Diethylether, "EE" Ethylacetat, "DCM" Dichlormethan, "DMF" Dimethylformamid, "DME" Dimethoxyethan"DMSO" Dimethylsulfoxid und "THF" Tetrahydrofuran. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch, "konz." konzentriert, "min" Minuten, "h" Stunden, "d" Tage, "Vol.%" Volumenprozent, "m%" Massenprozent und "M" ist eine Konzentrationsangabe in mol/l.

Die eingesetzten Chemikalien und Lösungsmittel wurden kommerziell bei den herkömmlichen Anbietern (beispielsweise Acros, Acocado, Aldrich, Bachem, Fluka, Lancaster, Maybridge, Merck, Sigma, Tci etc) erworben oder nach üblichen, dem Fachmann bekannten Methoden synthetisiert.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in Volumen/Volumen angegeben.

Die Analytik erfolgte mittels HPLC-MS oder/und NMR.

Die Numerierung der nachfolgenden Beispielverbindungen stimmt nicht mit der Numerierung der vorstehend explizit angegebenen substituierten 1,4,8-Triazaspiro[4.5]decan-2-on-Verbindungen überein.

Die Herstellung der substituierten 1,4,8-Triazaspiro[4.5]decan-2-on-Verbindungen gemäß den Beispielen 1-36 erfolgte gemäß dem allgemeinen Herstellungsverfahren 1, welches nachstehend schematisch wiedergegeben ist:

**Allgemeines Verfahren I:**

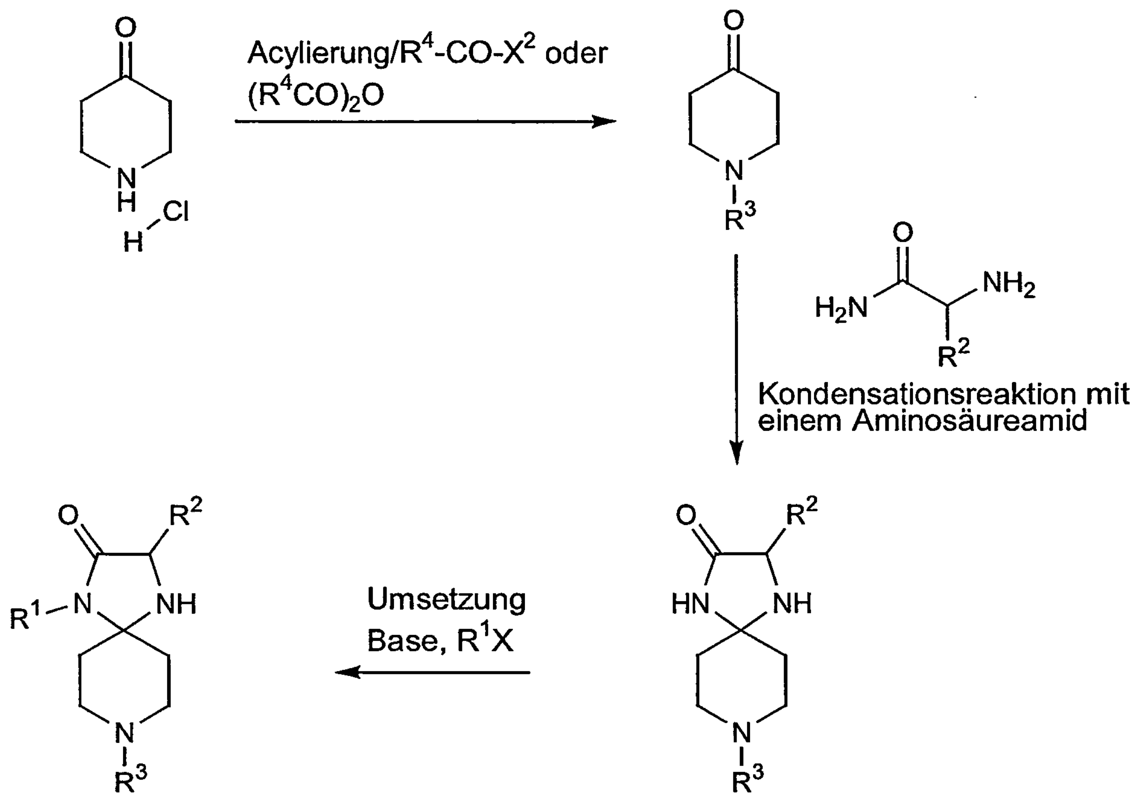


Im folgenden ist die Herstellung einzelner Reaktanden beispielhaft wiedergegeben:

**a) Herstellung von 1-(2-Ethyl-butyryl)-piperidin-4-on**

Zu einer Lösung von Piperidon Hydrochlorid (4.0 g, 26 mmol) in $CH_2Cl_2$ (20 ml) wurde Triethylamin (5.8 g, 57.3 mmol) und eine katalytische Menge DMAP gegeben. Nach Abkühlung auf -10°C wurde 2-Ethylbutyrchlorid (3.9 g, 28.6 mmol) gelöst in $CH_2Cl_2$ (10 ml) langsam zugegeben und für 16 h bei RT gerührt. Zur Suspension gab man 5M Kalilauge (25 ml), die organische Phase wurde abgetrennt und die wässerige Phase mit $CH_2Cl_2$ (3 x 10 ml) extrahiert. Nach Trocknung der vereinigten organischen Phasen über $Na_2SO_4$, Filtration und Entfernung des Lösungsmittels erhielt man das Produkt 1-(2-Ethyl-butyryl)-piperidin-4-on mit einer Ausbeute von 4.3 g (84 % der Theorie).

**b) Herstellung von 1-(4-Chlor-benzoyl)-piperidin-4-on**

Zu einer Lösung von Piperidon Hydrochlorid (3.0 g, 19.5 mmol) in $CH_2Cl_2$ (20 ml) wurde Triethylamin (4.4 g, 43.0 mmol) und eine katalytische Menge DMAP gegeben. Nach Abkühlung auf - 10°C wurde 4-Chlorbenzoylchlorid (3.8 g, 21.5 mmol)) gelöst in $CH_2Cl_2$ (10 ml) langsam zugegeben und für 16 h bei RT gerührt. Zur Suspension gab man 5M Kalilauge (20 ml), die organische Phase wurde abgetrennt und die wässerige Phase mit $CH_2Cl_2$ (3 x 10 ml) extrahiert. Nach Trocknung der vereinigten organischen Phasen über $Na_2SO_4$, Filtration und Entfernung des Lösungsmittels erhielt man das Produkt 1-(4-Chlor-benzoyl)-piperidin-4-on mit einer Ausbeute von 4.4 g (95 %).

**c) Herstellung von 1-(2,4-Dimethoxy-benzoyl)-piperidin-4-on**

Zu einer Lösung von von 2,4-Dimethoxybenzoesäure (11.8 g, 65.1 mmol) in DMF (25 ml) gab man bei 0°C Piperidon Hydrochlorid (3.0 g, 19.5 mmol) gelöst in DMF (25 ml), N,N-Diisopropylcarbodiimid (8.2 g, 65.1 mmol) und 1-Hydroxybenzotriazol (8.8 g, 65.1 mmol) und ließ 3 h bei 0°C und anschließend 2 d bei RT rühren. Zur Reaktionsmischung wurde wässerige 1 M $Na_2CO_3$-Lösung (20 ml) gegeben und mit EE (3 x 20 ml) extrahiert. Nach Trocknung der vereinigten organischen Phasen wurde das Lösungsmittel entfernt und der Rückstand mittels Säulenchromathographie gereinigt. Man erhielt das Produkt 1-(2,4-Dimethoxybenzoyl)-piperidin-4-on mit einer Ausbeute von 2.7 g (39 %).

**Beispiel 1:** 3-(*S*)-Benzyl-8-(2-ethyl-butyryl)-1,4,8-triaza-spiro[4.5]decan-2-on

Eine Lösung von 1-(2-Ethyl-butyryl)-piperidin-4-on (1.8 g, 9.1 mmol) und (*S*)-Phenylalaninamid (1.5 g, 9.1 mmol) in EtOH (40 ml) wurde für 2.5 h unter Rückfluß erhitzt und für weitere 16 h bei RT gerührt. Nach Entfernung des Lösungsmittels und Trocknung im Vakuum erhielt man das Produkt 3-(*S*)-Benzyl-8-(2-ethyl-butyryl)-1,4,8-triaza-spiro [4.5]decan-2-on mit einer Ausbeute von 3.1 g (99 %).

**Beispiel 2:** 3-(*S*)-Benzyl-8-(2-ethyl-butyryl)-1,4,8-triaza-spiro[4.5]decan-2-on Hydrochlorid

3-(*S*)-Benzyl-8-(2-ethyl-butyryl)-1,4,8-triaza-spiro[4.5]decan-2-on (610 mg, 1.8 mmol) wurde in Ethylmethylketon (5 ml) gelöst, mit Wasser (18 µl) und TMSCI (247 µl) versetzt und über Nacht gerührt. Der dabei ausgefallene Feststoff wurde abfiltriert, mit Ether gewaschen und im Vakuum getrocknet. Man erhielt das Produkt 3-(*S*)-Benzyl-8-(2-ethyl-butyryl)-1,4,8-triaza-spiro[4.5]decan-2-on Hydrochlorid mit einer Ausbeute von 590 mg (87 %).

**Beispiel 3:** 8-(2,4-Dimethoxy-benzoyl)-3-(*S*)-(2-methylsulfanyl-ethyl)-1,4,8-triaza-spiro[4.5]decan-2-on

Eine Lösung von 1-(2,4-Dimethoxy-benzoyl)-piperidin-4-on (1.4 g, 5.1 mmol), (*S*)-Methioninamid Hydrochlorid (950 mg, 5.1 mmol) und Triethylamin (520 mg, 5.1 mmol) in EtOH (40 ml) wurde für 2.5 h unter Rückfluß erhitzt und für weitere 16 h bei RT gerührt. Nach Entfernung des Lösungsmittels wurde zum Rückstand Wasser (25 ml) und $CH_2Cl_2$ (25 ml) zugegeben und mit konz. Natronlauge basisch gestellt. Die wässerige Phase wurde mit $CH_2Cl_2$ (3 x 20 ml) extrahiert und die vereinigten organischen Phasen über $Na_2SO_4$ getrocknet. Nach Entfernung des Lösungsmittels und Trocknung im Vakuum erhielt man das Produkt 8-(2,4-Dimethoxy-benzoyl)-3-(*S*)-(2-methylsulfanyl-ethyl)-1,4,8-triaza-spiro[4.5]decan-2-on mit einer Ausbeute von 1.9 g (95%).

**Beispiel 4:** 8-(2,4-Dimethoxy-benzoyl)-3-(*S*)-(2-methylsulfanyl-ethyl)-1,4,8-triaza-spiro[4.5]decan-2-on Hydrochlorid

8-(2,4-Dimethoxy-benzoyl)-3-(*S*)-(2-methylsulfanyl-ethyl)-1,4,8-triaza-spiro[4.5]decan-2-on (300 mg, 0.8 mmol) wurde in Ethylmethylketon (2 ml) gelöst, mit Wasser (8 µl) und TMSCI (106 µl) versetzt und über Nacht gerührt. Der Feststoff wurde abfiltriert und im Vakuum getrocknet. Man erhielt das Produkt 8-(2,4- Dimethoxy-benzoyl)-3-(*S*)-(2-methylsulfanyl-ethyl)-1,4,8-triaza-spiro[4.5]decan-2-on Hydrochlorid mit einer Ausbeute von 320 mg (97 %).

**Beispiel 5:** 3-(*S,R*)-Benzyl-8-(2-ethyl-butyryl)-1,4,8-triaza-spiro[4.5]decan-2-on

Eine Lösung von 1-(2-Ethyl-butyryl)-piperidin-4-on (1.8 g, 9.0 mmol), (*S,R*)-Phenylalaninamid Hydrochlorid (1.8 g, 9.0 mmol) und Triethylamin (910 mg, 9.0 mmol) in EtOH (40 ml) wurde für 2.5 h unter Rückfluß erhitzt und für weitere 16 h bei RT gerührt. Nach Entfernung des Lösunsmittels wurde zum Rückstand Wasser (25 ml) und $CH_2Cl_2$ (25 ml) zugegeben und mit konz. Natronlauge basisch gestellt. Die wässerige Phase wurde mit $CH_2Cl_2$ (3 x 20 ml) extrahiert und die vereinigten organischen Phasen über $Na_2SO_4$ getrocknet. Nach Entfernung des Lösungsmittels und Trocknung im Vakuum erhielt man das Produkt 3-(*S*,*R*)-Benzyl-8-(2-ethyl-butyryl)-1,4,8-triaza-spiro[4.5]decan-2-on mit einer Ausbeute von 2.9 g (96 %).

**Beispiel 6:** 3-(*S*,*R*)-Benzyl-8-(2-ethyl-butyryl)-1,4,8-triaza-spiro[4.5]decan-2-on Hydrochlorid

3-(*S*,*R*)-Benzyl-8-(2-ethyl-butyryl)-1,4,8-triaza-spiro[4.5]decan-2-on (300 mg, 0.9 mmol) wurde in Ethylmethylketon (2 ml) gelöst, mit Wasser (9 μl) und TMSCI (122 μl) versetzt und über Nacht gerührt. Der Feststoff wurde abfiltriert, mit Ether gewaschen und im Vakuum getrocknet. Man erhielt das Produkt 3-(*S,R*)-Benzyl-8-(2-ethyl-butyryl)-1,4,8-triaza-spiro[4.5]decan-2-on Hydrochlorid mit einer Ausbeute von 300 mg (91 %).

**Beispiel 7:** 8-Acetyl-3-(*S*)-benzyl-1,4,8-triaza-spiro[4.5]decan-2-on

Eine Lösung von 1-Acetyl-piperidin-4-on (1.5 g, 10.8 mmol) und (*S*)-Phenylalaninamid (1.8 g, 10.8 mmol) in EtOH (30 ml) wurde für 2.5 h unter Rückfluß erhitzt und für weitere 16 h bei RT gerührt. Nach Entfernung des Lösungsmittels und Trocknung im Vakuum erhielt man das Produkt 8-Acetyl-3-(*S*)-benzyl-1,4,8-triaza-spiro[4.5]decan-2-on mit einer Ausbeute von 3.0 g (99 %).

**Beispiel 8:** 8-Acetyl-3-(*S*)-benzyl-1,4,8-triaza-spiro[4.5]decan-2-on Hydrochlorid

8-Acetyl-3-(*S*)-benzyl-1,4,8-triaza-spiro[4.5]decan-2-on (300 mg, 1.0 mmol) wurde in Ethylmethylketon (2 ml) gelöst, mit Wasser (21 μl) und TMSCI (290 μl) versetzt und über Nacht gerührt. Der dabei ausgefallene Feststoff wurde abfiltriert, mit Ether gewaschen und im Vakuum getrocknet. Man erhielt das Produkt 8-Acetyl-3-(*S*)-benzyl-1,4,8-triaza-spiro[4.5]decan-2-on Hydrochlorid mit einer Ausbeute von 330 mg (96 %).

**Beispiel 9:** 3-(*S*,*R*)-Benzyl-8-(4-chlor-benzoyl)-1,4,8-triaza-spiro[4.5]decan-2-on

Eine Lösung von 1-(4-Chlor-benzoyl)-piperidin-4-on (2.1 g, 8.7 mmol), (*S*,*R*)-Phenylalaninamid Hydrochlorid (1.8 g, 8.7 mmol) und Triethylamin (880 mg, 8.7 mmol) in EtOH (40 ml) wurde für 2.5 h unter Rückfluß erhitzt und für weitere 16 h bei RT gerührt. Nach Entfernung des Lösunsmittels wurde zum Rückstand Wasser (25 ml) und $CH_2Cl_2$ (25 ml) zugegeben und mit konz. Natronlauge basisch gestellt. Die Wässerige Phase wurde mit. $CH_2Cl_2$ (3 x 20 ml) extrahiert und die vereinigten organischen Phasen über $Na_2SO_4$ getrocknet. Nach Entfernung des Lösnungsmittels und Trocknung im Vakuum erhielt man das Produkt 3-(*S*,*R*)-Benzyl-8-(4-chlorbenzoyl)-1,4,8-triaza-spiro[4.5]decan-2-on mit einer Ausbeute von 3.3 g (98 %).

**Beispiel 10:** 3-(*S*,*R*)-Benzyl-8-(4-chlor-benzoyl)-1,4,8-triaza-spiro[4.5]decan-2-on Hydrochlorid

3-(*S*,*R*)-Benzyl-8-(4-chlor-benzoyl)-1,4,8-triaza-spiro[4.5]decan-2-on (300 mg, 0.8 mmol) wurde in Ethylmethylketon (2 ml) gelöst, mit Wasser (8 μl) und TMSCI (109 μl) versetzt und über Nacht gerührt. Nach Zugabe von Hexan (10 ml) wurde die wässerige Phase abgetrennt und der Rückstand im Vakuum getrocknet. Man erhielt das Produkt 3-(*S*, *R*)-Benzyl-8-(4-chlor-benzoyl)-1,4,8-triaza-spiro[4.5]decan-2-on Hydrochlorid mit einer Ausbeute von 260 mg (77 %).

**Beispiel 11:** 3-(*S*)-Benzyl-8-(4-chlor-benzoyl)-1,4,8-triaza-spiro[4.5]decan-2-on

Eine Lösung von 1-(4-Chlor-benzoyl)-piperidin-4-on (2.2 g, 9.1 mmol) und (*S*)-Phenylalaninamid (1.5 g, 9.1 mmol) in EtOH (40 ml) wurde für 2.5 h unter Rückfluß erhitzt und für weitere 16 h bei RT gerührt. Nach Entfernung des Lösungsmittels gab man EE (60 ml) zu und rührte für 1 h bei 40°C. Nach Filtration und Trocknung im Vakuum erhielt man das Produkt 3-(*S*)-Benzyl-8-(4-chlor-benzoyl)-1,4,8-triaza-spiro[4.5]decan-2-on mit einer Ausbeute von 2.6 g (76 %).

**Beispiel 12:** 3-(*S*)-Benzyl-8-(4-chlor-benzoyl)-1,4,8-triaza-spiro[4.5]decan-2-on Hydrochlorid

3-(*S*)-Benzyl-8-(4-chlor-benzoyl)-1,4,8-triaza-spiro[4.5]decan-2-on (500 mg, 1.3 mmol) wurde in Ethylmethylketon (4 ml) gelöst, mit Wasser (13 µl) und TMSCl (181 µl) versetzt und über Nacht gerührt. Der dabei ausgefallene Feststoff wurde abfiltriert, mit Ether gewaschen und im Vakuum getrocknet. Man erhielt das Produkt 3-(*S*)-Benzyl-8-(4-chlor-benzoyl)-1,4,8-triaza-spiro[4.5]decan-2-on Hydrochlorid mit einer Ausbeute von 390 mg (72 %).

**Beispiel 13:** 8-(2-ethyl-butyryl)-3-(*S*)-(2-methylsulfanyl-ethyl)-1,4,8-triazaspiro[4.5]decan-2-on

Eine Lösung von 1-(2-Ethyl-butyryl)-piperidin-4-on (1.6 g, 8.1 mmol), (*S*)-Methioninamid Hydrochlorid (1.5 g, 8.1 mmol) und Triethylamin (820 mg, 8.1 mmol) in EtOH (66 ml) wurde für 16 h unter Rückfluß erhitzt. Nach Entfernung des Lösungsmittels wurde zum Rückstand Wasser (50 ml) und $CH_2Cl_2$ (50 ml) zugegeben und mit konz. Natronlauge basisch gestellt. Die wässerige Phase wurde mit $CH_2Cl_2$ (3 x 20 ml) extrahiert und die vereinigten organischen Phasen über $Na_2SO_4$ getrocknet. Nach Entfernung des Lösungsmittels und Trocknung im Vakuum erhielt man das Produkt 8-(2-ethyl-butyryl)-3-(*S*)-(2-methylsulfanyl-ethyl)-1,4,8-triazaspiro[4.5]decan-2-on mit einer Ausbeute von 1.8 g (69 %).

**Beispiel 14:** 8-(2-ethyl-butyryl)-3-(*S*)-(2-methylsulfanyl-ethyl)-1,4,8-triazaspiro[4.5]decan-2-on Hydrochlorid

8-(2-ethyl-butyryl)-3-(*S*)-(2-methylsulfanyl-ethyl)-1,4,8-triazaspiro[4.5]decan-2-on (140 mg, 0.4 mmol) wurde in Ethylmethylketon (1 ml) gelöst, mit Wasser (4 µl) und TMSCl (59 µl) versetzt und über Nacht gerührt. Der Feststoff wurde abfiltriert und im Vakuum getrocknet. Man erhielt das Produkt 8-(2-ethyl-butyryl)-3-(*S*)-(2-methylsulfanyl-ethyl)-1,4,8-triazaspiro[4.5]decan-2-on Hydrochlorid mit einer Ausbeute von 117 mg (75 %).

**Beispiel 15:** 1,3-(*S*)-Dibenzyl-8-(2-ethyl-butyryl)-1,4,8-triaza-spiro[4.5]decan-2-on

Zu einer Lösung von 3-(*S*)-Benzyl-8-(2-ethyl-butyryl)-1,4,8-triaza-spiro[4.5]decan-2-on (1.0 g, 2.9 mmol) in 40 ml trockenem THF gab man bei RT und unter Stickstoff Natriumhydrid (90 mg, 3.8 mmol) hinzu. Nach Rühren für 1 h bei RT wurde langsam Benzylchlorid (1.47 g, 11.6 mmol) zugetropft und erhitzte anschließend 12 h unter Rückfluß. Nach Zugabe einer wässerigen gesättigten $NH_4Cl$-Lösung (20 ml) wurde die Reaktionsmischung abfiltriert, das Filtrat mit EE (3 x 15 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 1,3-(*S*)-Dibenzyl-8-(2-ethyl-butyryl)-1,4,8-triaza-spiro[4.5]decan-2-on mit einer Ausbeute von 1.1 g (52 %).

**Beispiel 25:** 1,3-(*S*)-Dibenzyl-8-(2-ethyl-butyryl)-1,4,8-triaza-spiro[4.5]decan-2-on Hydrochlorid

1,3-(*S*)-Dibenzyl-8-(2-ethyl-butyryl)-1,4,8-triaza-spiro[4.5]decan-2-on (654 mg, 1.5 mmol) wurde in Ethylmethylketon (5 ml) gelöst, mit Wasser (15 µl) und TMSCl (210 µl) versetzt und über Nacht gerührt. Anschließend wurde Hexan (10 ml) zugegeben. Der dabei ausgefallene Feststoff wurde abfiltriert und im Vakuum getrocknet. Man erhielt das Produkt 1,3-(*S*)-Dibenzyl-8-(2-ethyl-butyryl)-1,4,8-triaza-spiro[4.5]decan-2-on Hydrochlorid mit einer Ausbeute von 671 mg (95 %).

**Beispiel 16:** 8-Acetyl-1,3-(*S*)-dibenzyl-1,4,8-triaza-spiro[4.5]decan-2-on

Zu einer Lösung von 8-Acetyl-3-(*S*)-benzyl-1,4,8-triaza-spiro[4.5]decan-2-on (2.8 g, 9.7 mmol) in 20 ml trockenem THF gab man bei 0°C und unter Stickstoff Natriumhydrid (350 mg, 14.6 mmol) hinzu. Nach Rühren für 1 h bei 0°C wurde langsam Benzylchlorid (1.36 g, 10.7 mmol) zugetropft, ließ auf RT erwärmen und erhitzte anschließend 24 h unter Rückfluß. Nach Zugabe einer wässerigen gesättigten $NH_4Cl$-Lösung (25 ml) wurde die Reaktionsmischung abfiltriert, das Filtrat mit EE (3 x 20 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 8-Acetyl-1,3-(*S*)-dibenzyl-1,4,8-triaza-spiro[4.5]decan-2-on mit einer Ausbeute von 1.25 g (34 %).

**Beispiel 26:** 8-Acetyl-1,3-(*S*)-dibenzyl-1,4,8-triaza-spiro[4.5]decan-2-on Hydrochlorid

8-Acetyl-1,3(*S*)-dibenzyl-1,4,8-triaza-spiro[4.5]decan-2-on (1.25 g, 3.3 mmol) wurde in Ethylmethylketon (10 ml) gelöst, mit Wasser (33 µl) und TMSCl (461 µl) versetzt und über Nacht gerührt. Der dabei ausgefallene Feststoff wurde abfiltriert, mit Diethylether gewaschen und im Vakuum getrocknet. Man erhielt das Produkt 8-Acetyl-1,3-(*S*)-dibenzyl-1,4,8-triaza-spiro[4.5]decan-2-on Hydrochlorid mit einer Ausbeute von 939 mg (69 %).

**Beispiel 17:** 3-(*S,R*)-Benzyl-8-(4-chlor-benzoyl)-1-(4-methoxy-benzyl)-1,4,8-triaza-spiro[4.5]decan-2-on

Zu einer Lösung von 3-(*S,R*)-Benzyl-8-(4-chlor-benzoyl)-1,4,8-triaza-spiro[4.5]decan-2-on (920 mg, 2.4 mmol) in 20 ml trockenem THF gab man bei RT und unter Stickstoff Natriumhydrid (90 mg, 3.6 mmol) hinzu. Nach Rühren für 1 h bei RT wurde langsam 4-Methoxybenzylchlorid (410 mg, 2.6 mmol) zugetropft und erhitzte anschließend 48 h unter Rückfluß. Nach Zugabe einer wässerigen gesättigten $NH_4Cl$-Lösung (15 ml) wurde die Reaktionsmischung abfiltriert, das Filtrat mit EE (3 x 10 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 3-(*S,R*)-Benzyl-8-(4-chlor-benzoyl)-1-(4-methoxy-benzyl)-1,4,8-triaza-spiro [4.5]decan-2-on mit einer Ausbeute von 500 mg (41 %).

**Beispiel 27:** 3-(*S,R*)-Benzyl-8-(4-chlor-benzoyl)-1-(4-methoxy-benzyl)-1,4,8-triaza-spiro[4.5]decan-2-on Hydrochlorid

3-(*S,R*)-Benzyl-8-(4-chlor-benzoyl)-1-(4-methoxy-benzyl)-1,4,8-triaza-spiro[4.5]decan-2-on (500 mg, 1.0 mmol) wurde in Ethylmethylketon (4 ml) gelöst, mit Wasser (10 μl) und TMSCl (138 μl) versetzt und über Nacht gerührt. Anschließend wurde Hexan (10 ml) zugegeben, die wässerige Phase abgetrennt und der Rückstand im Vakuum getrocknet. Man erhielt das Produkt 3-(*S,R*)-Benzyl-8-(4-chlor-benzoyl)-1-(4-methoxy-benzyl)-1,4,8-triaza-spiro[4.5]decan-2-on Hydrochlorid mit einer Ausbeute von 507 mg (92 %).

**Beispiel 18:** 3-(*S,R*)-Benzyl-8-(4-chlor-benzoyl)-1-(4-fluor-benzyl)-1,4,8-triaza-spiro[4.5]decan-2-on

Zu einer Lösung von 3-(*S,R*)-Benzyl-8-(4-chlor-benzoyl)-1,4,8-triaza-spiro[4.5]decan-2-on (920 mg, 2.4 mmol) in 20 ml trockenem THF gab man bei RT und unter Stickstoff Natriumhydrid (90 mg, 3.6 mmol) hinzu. Nach Rühren für 1 h bei RT wurde langsam 4-Fluorbenzylchlorid (380 mg, 2.6 mmol) zugetropft und erhitzte anschließend 48 h unter Rückfluß. Nach Zugabe einer wässerigen gesättigten $NH_4Cl$-Lösung (15 ml) wurde die Reaktionsmischung abfiltriert, das Filtrat mit EE (3 x 15 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 3-(*S,R*)-Benzyl-8-(4-chlor-benzoyl)-1-(4-fluor-benzyl)-1,4,8-triaza-spiro[4.5] decan-2-on mit einer Ausbeute von 566 mg (48 %).

**Beispiel 28:** 3-(*S,R*)-Benzyl-8-(4-chlor-benzoyl)-1-(4-fluor-benzyl)-1,4,8-triaza-spiro[4.5]decan-2-on Hydrochlorid

3-(*S,R*)-Benzyl-8-(4-chlor-benzoyl)-1-(4-fluor-benzyl)-1,4,8-triaza-spiro[4.5]decan-2-on (560 mg, 1.1 mmol) wurde in Ethylmethylketon (4 ml) gelöst, mit Wasser (11 μl) und TMSCl (158 μl) versetzt und über Nacht gerührt. Der dabei ausgefallene Feststoff wurde abfiltriert und im Vakuum getrocknet. Man erhielt das Produkt 3-(*S,R*)-Benzyl-8-(4-chlor-benzoyl)-1-(4-fluor-benzyl)-1,4,8-triaza-spiro[4.5]decan-2-on Hydrochlorid mit einer Ausbeute von 450 mg (75 %).

**Beispiel 19:** 1,3-(*S,R*)-Dibenzyl-8-(4-chlor-benzoyl)-1,4,8-triaza-spiro[4.5]decan-2-on

Zu einer Lösung von 3-(*S,R*)-Benzyl-8-(4-chlor-benzoyl)-1,4,8-triaza-spiro[4.5]decan-2-on (920 mg, 2.4 mmol) in 20 ml trockenem THF gab man bei RT und unter Stickstoff Natriumhydrid (90 mg, 3.6 mmol) hinzu. Nach Rühren für 1 h bei RT wurde langsam Benzylchlorid (330 mg, 2.6 mmol) zugetropft und erhitzte anschließend 48 h unter Rückfluß. Nach Zugabe einer wässerigen gesättigten $NH_4Cl$-Lösung (15 ml) wurde die Reaktionsmischung abfiltriert, das Filtrat mit EE (3 x 10 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 1,3-(*S,R*)-Dibenzyl-8-(4-chlor-benzoyl)-1,4,8-triaza-spiro[4.5]decan-2-on mit einer Ausbeute von 375 mg (33%).

**Beispiel 29:** 1,3-(*S,R*)-Dibenzyl-8-(4-chlor-benzoyl)-1,4,8-triaza-spiro[4.5]decan-2-on Hydrochlorid

1,3-(*S,R*)-Dibenzyl-8-(4-chlor-benzoyl)-1,4,8-triaza-spiro[4.5]decan-2-on (370 mg, 0.8 mmol) wurde in Ethylmethylketon (3 ml) gelöst, mit Wasser (8 μl) und TMSCl (109 μl) versetzt und über Nacht gerührt. Der dabei ausgefallene Feststoff wurde abfiltriert und im Vakuum getrocknet. Man erhielt das Produkt 1,3-(*S,R*)-Dibenzyl-8-(4-chlorbenzoyl)-1,4,8-triaza-spiro[4.5]decan-2-on Hydrochlorid mit einer Ausbeute von 355 mg (89 %).

**Beispiel 20:** 3-(*S,R*)-Benzyl-8-(2-ethyl-butyryl)-1-(4-methoxy-benzyl)-1,4,8-triaza-spiro[4.5]decan-2-on

Zu einer Lösung von 3-(*S,R*)-Benzyl-8-(2-ethyl-butyryl)-1,4,8-triaza-spiro[4.5]decan-2-on (850 mg, 2.5 mmol)

in 20 ml trockenem THF gab man bei RT und unter Stickstoff Natriumhydrid (90 mg, 3.6 mmol) hinzu. Nach Rühren für 1 h bei RT wurde langsam 4-Methoxybenzylchlorid (430 mg, 2.7 mmol) zugetropft und erhitzte anschließend 48 h unter Rückfluß. Nach Zugabe einer wässerigen gesättigten $NH_4Cl$-Lösung (15 ml) wurde die Reaktionsmischung abfiltriert, das Filtrat mit EE (3 x 10 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 3-(*S,R*)-Benzyl-8-(2-ethyl-butyryl)-1-(4-methoxy-benzyl)-1,4,8-triaza-spiro [4.5]decan-2-on mit einer Ausbeute von 563 mg (51 %).

**Beispiel 30:** 3-(*S,R*)-Benzyl-8-(2-ethyl-butyryl)-1-(4-methoxy-benzyl)-1,4,8-triaza-spiro[4.5]decan-2-on Hydrochlorid

3-(*S,R*)-Benzyl-8-(2-ethyl-butyryl)-1-(4-methoxy-benzyl)-1,4,8-triaza-spiro[4.5]decan-2-on (550 mg, 1.2 mmol) wurde in Ethylmethylketon (4 ml) gelöst, mit Wasser (12 µl) und TMSCl (169 µl) versetzt und über Nacht gerührt. Anschließend wurde Hexan (10 ml) zugegeben, die wässerige Phase abgetrennt und der Rückstand im Vakuum getrocknet. Man erhielt das Produkt 3-(*S,R*)-Benzyl-8-(2-ethyl-butyryl)-1-(4-methoxybenzyl)-1,4,8-triaza-spiro[4.5]decan-2-on Hydrochlorid mit einer Ausbeute von 530 mg (90%).

**Beispiel 21:** 3-(*S,R*)-Benzyl-8-(2-ethyl-butyryl)-1-(4-fluor-benzyl)-1,4,8-triaza-spiro[4.5]decan-2-on

Zu einer Lösung von 3-(*S,R*)-Benzyl-8-(2-ethyl-butyryl)-1,4,8-triaza-spiro[4.5]decan-2-on (850 mg, 2.5 mmol) in 20 ml trockenem THF gab man bei RT und unter Stickstoff Natriumhydrid (90 mg, 3.6 mmol) hinzu. Nach Rühren für 1 h bei RT wurde langsam 4-Fluorbenzylchlorid (390 mg, 2.7 mmol) zugetropft und erhitzte anschließend 48 h unter Rückfluß. Nach Zugabe einer wässerigen gesättigten $NH_4Cl$-Lösung (15 ml) wurde die Reaktionsmischung abfiltriert, das Filtrat mit EE (3 x 10 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 3-(*S,R*)-Benzyl-8-(2-ethyl-butyryl)-1-(4-fluor-benzyl)-1,4,8-triaza-spiro[4.5] decan-2-on mit einer Ausbeute von 861 mg (75 %).

**Beispiel 31:** 3-(*S,R*)-Benzyl-8-(2-ethyl-butyryl)-1-(4-fluor-benzyl)-1,4,8-triaza-spiro[4.5]decan-2-on Hydrochlorid

3-(*S,R*)-Benzyl-8-(2-ethyl-butyryl)-1-(4-fluor-benzyl)-1,4,8-triaza-spiro[4.5]decan-2-on (850 mg, 1.2 mmol) wurde in Ethylmethylketon (7 ml) gelöst, mit Wasser (18 µl) und TMSCl (255 µl) versetzt und über Nacht gerührt. Anschließend wurde Hexan (10 ml) zugegeben, die wässerige Phase abgetrennt und der Rückstand im Vakuum getrocknet. Man erhielt das Produkt 3-(*S,R*)-Benzyl-8-(2-ethyl-butyryl)-1-(4-fluor-benzyl)-1,4,8-triaza-spiro[4.5]decan-2-on Hydrochlorid mit einer Ausbeute von 853 mg (95 %).

**Beispiel 22:** 1,3-(*S,R*)-Dibenzyl-8-(2-ethyl-butyryl)-1,4,8-triaza-spiro[4.5]decan-2-on

Zu einer Lösung von 3-(*S,R*)-Benzyl-8-(2-ethyl-butyryl)-1,4,8-triaza-spiro[4.5]decan-2-on (850 mg, 2.5 mmol) in 20 ml trockenem THF gab man bei RT und unter Stickstoff Natriumhydrid (90 mg, 3.6 mmol) hinzu. Nach Rühren für 1 h bei RT wurde langsam Benzylchlorid (340 mg, 2.7 mmol) zugetropft und erhitzte anschließend 48 h unter Rückfluß. Es wurde Benzylchlorid (170 mg, 1.4 mmol) zugegeben und für weitere 16 h unter Rückfluß erhitzt. Nach Zugabe einer wässerigen gesättigten $NH_4Cl$-Lösung (15 ml) wurde die Reaktionsmischung abfiltriert, das Filtrat mit EE (3 x 10 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 1,3-(*S,R*)-Dibenzyl-8-(2-ethyl-butyryl)-1,4,8-triaza-spiro[4.5]decan-2-on mit einer Ausbeute von 826 mg (77 %).

**Beispiel 32:** 1,3-(*S,R*)-Dibenzyl-8-(2-ethyl-butyryl)-1,4,8-triaza-spiro[4.5]decan-2-on Hydrochlorid

1,3-(*S,R*)-Dibenzyl-8-(2-ethyl-butyryl)-1,4,8-triaza-spiro[4.5]decan-2-on (810 mg, 1.9 mmol) wurde in Ethylmethylketon (6 ml) gelöst, mit Wasser (18 µl) und TMSCl (260 µl) versetzt und über Nacht gerührt. Der dabei ausgefallene Feststoff wurde abfiltriert und im Vakuum getrocknet. Man erhielt das Produkt 1,3-(*S,R*)-Dibenzyl-8-(2-ethylbutyryl)-1,4,8-triaza-spiro[4.5]decan-2-on Hydrochlorid mit einer Ausbeute von 685 (78 %).

**Beispiel 23:** 1-Benzyl-8-(2-ethyl-butyryl)-3-(*S*)-(2-methylsulfanyl-ethyl)-1,4,8-triaza-spiro[4.5]decan-2-on

Zu einer Lösung von 8-(2-Ethyl-butyryl)-3-(*S*)-(2-methylsulfanyl-ethyl)-1,4,8-triaza-spiro[4.5]decan-2-on (420 mg, 1.3 mmol) in 20 ml trockenem THF gab man bei 0°C und unter Stickstoff Natriumhydrid (50 mg, 1.9 mmol) hinzu.

Nach Rühren für 1 h bei 0°C wurde langsam Benzylchlorid (140 mg, 1.4 mmol) zugetropft und erhitzte anschließend 24 h unter Rückfluß. Nach Zugabe einer wässerigen gesättigten $NH_4Cl$-Lösung (15 ml) wurde die Reaktionsmischung abfiltriert, das Filtrat mit EE (3 x 10 ml) extrahiert und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 1-Benzyl-8-(2-ethyl-butyryl)-3-(*S*)-(2-methylsulfanyl-ethyl)-1,4,8-triaza-spiro[4.5]decan-2-on mit einer Ausbeute von 239 mg (45 %).

**Beispiel 33:** 1-Benzyl-8-(2-ethyl-butyryl)-3-(2-methylsulfanyl-ethyl)-1,4,8-triaza-spiro[4.5]decan-2-on Hydrochlorid

1-Benzyl-8-(2-ethyl-butyryl)-3-(*S*)-(2-methylsulfanyl-ethyl)-1,4,8-triaza-spiro[4.5]decan-2-on (230 mg, 0.6 mmol) wurde in Ethylmethylketon (2 ml) gelöst, mit Wasser (5 µl) und TMSCl (77 µl) versetzt und über Nacht gerührt. Anschließend wurde Hexan (8 ml) zugegeben, die wässerige Phase abgetrennt und der Rückstand im Vakuum getrocknet. Man erhielt das Produkt 1-Benzyl-8-(2-ethyl-butyryl)-3-(*S*)-(2-methylsulfanyl-ethyl)-1,4,8-triaza-spiro[4.5]decan-2-on Hydrochlorid mit einer Ausbeute von 246 mg (90 %).

**Beispiel 24:** 1,3-(*S*)-Dibenzyl-8-(4-chlor-benzoyl)-1,4,8-triaza-spiro[4.5]decan-2-on

Zu einer Lösung von 3-(*S*)-Benzyl-8-(4-chlor-benzoyl)-1,4,8-triaza-spiro[4.5]decan-2-on (2.1 g, 5.5 mmol) in 20 ml trockenem THF gab man bei 0°C und unter Stickstoff Natriumhydrid (130 mg, 5.5 mmol) hinzu. Nach Rühren für 1 h bei 0°C wurde langsam Benzylchlorid (760 mg, 6.0 mmol) zugetropft, ließ bis auf RT erwärmen und erhitzte anschließend 4 d unter Rückfluß. Nach Zugabe einer wässerigen gesättigten $NH_4Cl$-Lösung (30 ml) wurde die Reaktionsmischung abfiltriert, das Filtrat mit EE (3 x 20 ml) extrahiert, die vereinigten organischen Phasen über $Na_2SO_4$ getrocknet und das Lösungsmittels entfernt. Weitere Aufreinigung erfolgte durch Säulenchromathographie. Man erhielt das Produkt 1,3-(*S*)-Dibenzyl-8-(4-chlorbenzoyl)-1,4,8-triaza-spiro[4.5]decan-2-on als gelbes Öl mit einer Ausbeute von 2.62 g (99 %).

**Beispiel 34:** 1,3-(*S*)-Dibenzyl-8-(4-chlor-benzoyl)-1,4,8-triaza-spiro[4.5]decan-2-on Hydrochlorid

1,3-(*S*)-Dibenzyl-8-(4-chlor-benzoyl)-1,4,8-triaza-spiro[4.5]decan-2-on (2.6 g, 5.5 mmol) wurde in Ethylmethylketon (21 ml) gelöst, mit Wasser (54 µl) und TMSCl (763 µl) versetzt und über Nacht gerührt. Anschließend wurde Hexan (10 ml) zugegeben, der Feststoff abfiltriert und im Vakuum getrocknet. Der dabei ausgefallene Feststoff wurde abfiltriert und im Vakuum getrocknet. Man erhielt das Produkt 1,3-(*S*)-Dibenzyl-8-(4-chlor-benzoyl)-1,4,8-triaza-spiro [4.5]decan-2-on Hydrochlorid mit einer Ausbeute von 2.53 g (90 %).

**Die Beispiele 35 und 36 wurden unter Einstrahlung von Mikrowellen hergestellt:**

**Beispiel 35:** 1-Benzyl-8-(2,4-dimethoxy-benzoyl)-3-(*S*)-(2-methylsulfanyl-ethyl)-1,4,8-triaza-spiro[4.5]decan-2-on

Zu einer Lösung von 8-(2,4-Dimethoxy-benzoyl)-3-(*S*)-(2-methylsulfanyl-ethyl)-1,4,8-triaza-spiro[4.5]decan-2-on (400 mg, 1.0 mmol) in 10 ml DMF gab man bei RT und unter Stickstoff Natriumhydrid (40 mg, 1.5 mmol) hinzu. Nach Rühren für 1 h bei RT wurde langsam 4-Fluorbenzylchlorid (160 mg, 1.1 mmol) zugetropft und erhitzte anschließend für 30 min in der Mikrowelle bei 100°C. Nach Zugabe einer wässerigen gesättigten $NH_4Cl$-Lösung (15 ml) wurde mit Ether (3 x 10 ml) extrahiert, die etherische Phase mit wässeriger gesättigter $NaHCO_3$-Lösung (2 x 10 ml) und die vereinigten organischen Phasen über $Na_2SO_4$ getrocknet. Nach Abdestillation des Lösungsmittels erhielt man das Rohprodukt als gelbes Öl. Weitere Aufreinigung erfolgte durch Säulenchromathographie. Man erhielt das Produkt 1-Benzyl-8-(2,4-dimethoxy-benzoyl)-3-(*S*)-(2-methylsulfanyl-ethyl)-1,4,8-triaza-spiro[4.5]decan-2-on als mit einer Ausbeute von 296 mg (58 %).

**Beispiel 36:** 1-Benzyl-8-(2,4-dimethoxy-benzoyl)-3-(*S*)-(2-methylsulfanyl-ethyl)-1,4,8-triaza-spiro[4.5]decan-2-on Hydrochlorid

1-Benzyl-8-(2,4-dimethoxy-benzoyl)-3-(*S*)-(2-methylsulfanyl-ethyl)-1,4,8-triaza-spiro[4.5]decan-2-on (290 mg, 0.6 mmol) wurde in Ethylmethylketon (2 ml) gelöst, mit Wasser (6 µl) und TMSCl (80 µl) versetzt und über Nacht gerührt. Der dabei ausgefallene Feststoff wurde abfiltriert und im Vakuum getrocknet. Man erhielt das Produkt 1-Benzyl-8-(2,4-dimethoxy-benzoyl)-3-(*S*)-(2-methylsulfanyl-ethyl)-1,4,8-triaza-spiro[4.5]decan-2-on Hydrochlorid mit einer Ausbeute von 105 mg (95 %).

Die Herstellung der substituierten 1,4,8-Triazaspiro[4.5]decan-2-on-Verbindungen gemäß den nachfolgenden Beispielen erfolgte nach dem allgemeinen Herstellungsverfahren II, welches nachstehend schematisch wiedergegeben

ist:

**Allgemeines Verfahren II:**

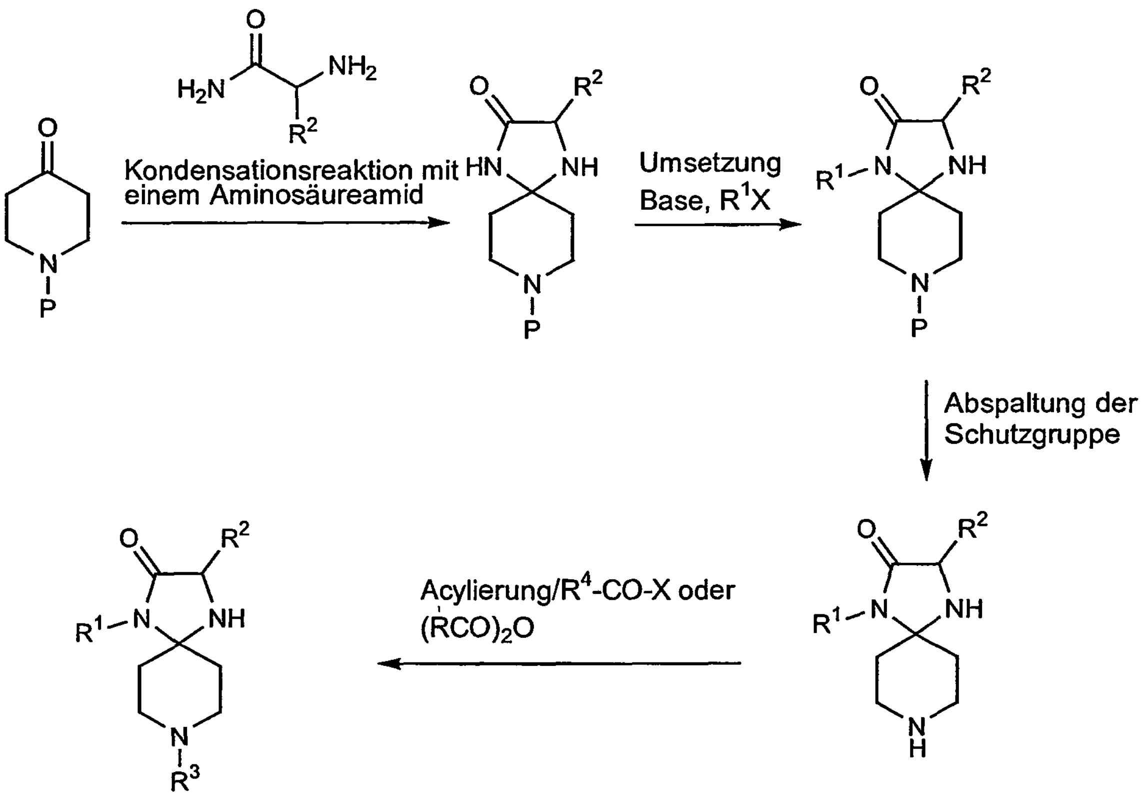


Im folgenden ist die Herstellung einiger Edukte beispielhaft wiedergegeben:

**a) Herstellung von 3-(*S*)-Benzyl-2-oxo-1,4,8-triaza-spiro[4.5]decan-8-carbonsäure tert-butylester**

Zu einer Lösung von (*S*)-Phenylalaninamid (4.9 g, 29.8 mmol) in trockenem EtOH (20 ml) gab man N-tert-Butyloxycarbonyl-piperidon (6.0 g, 29.8 mmol) und erhitzte für 2.5 h unter Rückfluß. Das Lösungsmittel wurde abdestilliert und das 3-(*S*)-Benzyl-2-oxo-1,4,8-triaza-spiro[4.5]decan-8-carbonsäure tert-butylester (10.8 g) wurde ohne weitere Aufarbeitung verwendet.

**b) Herstellung von 3-(*S*)-(2-methylsulfanyl-ethyl)-2-oxo-1,4,8-triaza-spiro[4.5]decan-8-carbonsäure tert-butylester**

Zu einer Lösung von (*S*)-Methioninamid Hydrochlorid (5.5 g, 29.8 mmol) in trockenem EtOH (66 ml) gab man N-tert-Butyloxycarbonyl-piperidon (5.9 g, 29.8 mmol), Triethylamin (3.02 g, 29.8 mmol) und erhitzte für 2.5 h unter Rückfluß. Nach Zugabe von Wasser (50 ml) und $CH_2Cl_2$ (200 ml) wurde die organische Phase abgetrennt und die wässerige Phase mit $CH_2Cl_2$ (3 x 20 ml) extrahiert. Die vereinigten organischen Phasen wurden über $Na_2SO_4$ getrocknet. Nach Filtration wurde das Lösungsmittel abdestilliert. Man erhielt 3-(*S*)-(2-methylsulfanyl-ethyl)-2-oxo-1,4,8-triaza-spiro [4.5]decan-8-carbonsäure tert-butylester mit einer Ausbeute von 8.0 g (82 %).

**c) Herstellung von 1,3-(*S*)-Dibenzyl-2-oxo-1,4,8-triaza-spiro[4.5]decan-8-carbonsäure tert-butylester**

Zu einer Lösung von 3-(*S*)-Benzyl-2-oxo-1,4,8-triaza-spiro[4.5]decan-8-carbonsäure tert-butylester (12.1 g, 35 mmol) in THF (250 ml) gab man bei RT NaH (1,26 g, 52,5 mmol) hinzu. Nach Rühren für 1 h bei 0°C wurde Benzylchlorid

(4.9 g, 38.5 mmol) zugetropft, ließ auf RT erwärmen und erhitzte für 60 h unter Rückfluß. Anschließend wurde erneut Benzylchlorid (2.4 g, 19 mmol) zugegeben und für weitere 16 h unter Rückfluß erhitzt. Die Reaktionsmischung wurde mit wässeriger gesättigter $NH_4Cl$-Lösung (20 ml) versetzt und mit EE (3 x 50 ml) extrahiert. Die vereinigte organische Phase wurde über $Na_2SO_4$ getrocknet, filtriert und das Lösungsmittel abdestilliert. Die Aufarbeitung erfolgte über Säulenchromathographie und ergab 1,3-(*S*)-Dibenzyl-2-oxo-1,4,8-triaza-spiro[4.5]decan-8-carbonsäure tert-butylester mit einer Ausbeute von 13.5 g (88 %).

**d) Herstellung von 1-Benzyl-3-(*S*)-(2-methylsulfanyl-ethyl)-2-oxo-1,4,8-triaza-spiro[4.5]decan-8-carbonsäure tert-butylester**

Zu einer Suspension von NaH (385 mg, 16,0 mmol) in THF (60 ml) tropfte man bei 0°C eine Lösung von 3-(*S*)-(2-methylsulfanyl-ethyl)-2-oxo-1,4,8-triaza-spiro[4.5]decan-8-carbonsäure tert-butylester (4.4 g, 13.4 mmol) in THF (72 ml). gab Nach Rühren für 1 h bei 0°C wurde Benzylchlorid (2.0 g, 16.0 mmol) zugetropft, ließ auf RT erwärmen und erhitzte für 68 h unter Rückfluß. Die Reaktionsmischung wurde mit wässeriger gesättigter $NH_4Cl$-Lösung (20 ml) versetzt, die organische Phase abgetrennt und die wässerige Phase mit EE (3 x 50 ml) extrahiert. Die vereinigte organische Phase wurde über $Na_2SO_4$ getrocknet, filtriert und das Lösungsmittel abdestilliert. Die Aufarbeitung erfolgte über Säulenchromathographie und ergab 1-Benzyl-3-(*S*)-(2-methylsulfanyl-ethyl)-2-oxo-1,4,8-triaza-spiro[4.5]decan-8-carbonsäure tert-butylester mit einer Ausbeute von 1.6 g (30 %).

**Beispiel 37:** 1,3-(*S*)-Dibenzyl-1,4,8-triaza-spiro[4.5]decan-2-on

Zu einer Lösung von 1,3-(*S*)-Dibenzyl-2-oxo-1,4,8-triaza-spiro[4.5]decan-8-carbonsäure tert-butylester (13.5 g, 31.0 mmol) in $CH_2Cl_2$ (93 ml) wurde TFA (68.5 g, 601 mmol) bei 0°C zugetropft und 15 min bei dieser Temperatur gerührt. Nach Erwärmen auf RT wurde die Reaktionslösung für weitere 2.5 h gerührt. Anschließend wurde die Reaktionslösung mit wässeriger gesättigter $NaHCO_3$-Lösung auf den pH-Wert 7-8 eingestellt, die organische Phase abgetrennt und die wässerige Phase mit $CH_2Cl_2$ (2 x 30 ml) extrahiert. Die vereinigte organische Phase wurde über $Na_2SO_4$ getrocknet, filtriert und das Lösungsmittel abdestilliert. Die Aufarbeitung erfolgte über Säulenchromathographie und ergab 1,3-(*S*)-Dibenzyl-1,4,8-triaza-spiro[4.5]decan-2-on mit einer Ausbeute von 7.8 g (75 %).

**Beispiel 38:** 1,3-(*S*)-Dibenzyl-1,4,8-triaza-spiro[4.5]decan-2-on Hydrochlorid

1,3-(*S*)-Dibenzyl-1,4,8-triaza-spiro[4.5]decan-2-on (400 mg, 1.2 mmol) wurde in Ethylmethylketon (3 ml) gelöst, mit Wasser (12 µl) und mit TMSCl (166 µl) versetzt und über Nacht gerührt. Der Feststoff wurde abfiltriert, mit Ether gewaschen und im Vakuum getrocknet. Man erhielt das Produkt 1,3-(*S*)-Dibenzyl-1,4,8-triaza-spiro[4.5]decan-2-on Hydrochlorid mit einer Ausbeute von 315 mg (71 %).

**Beispiel 39:** 1-Benzyl-3-(*S*)-(2-methylsulfanyl-ethyl)-1,4,8-triaza-spiro[4.5]decan-2-on

Zu einer Lösung von 1-Benzyl-3-(*S*)-(2-methylsulfanyl-ethyl)-2-oxo-1,4,8-triaza-spiro[4.5]decan-8-carbonsäure tert-butylester (1.5 g, 3.5 mmol) in $CH_2Cl_2$ (10 ml) wurde TFA (7.8 g, 68.0 mmol) bei 0°C zugetropft und 15 min bei dieser Temperatur gerührt. Nach Erwärmen auf RT wurde die Reaktionslösung für weitere 2.5 h gerührt. Anschließend wurde die Reaktionslösung mit wässeriger gesättigter $NaHCO_3$-Lösung auf den pH-Wert 7-8 eingestellt, die organische Phase abgetrennt und die wässerige Phase mit $CH_2Cl_2$ (2 x 10 ml) extrahiert. Die vereinigte organische Phase wurde über $Na_2SO_4$ getrocknet, filtriert und das Lösungsmittel abdestilliert. Die Aufarbeitung erfolgte über Säulenchromathographie und ergab 1-Benzyl-3-(*S*)-(2-methylsulfanylethyl)-1,4,8-triaza-spiro[4.5]decan-2-on mit einer Ausbeute von 655 mg (58 %).

**Die Verbindungen gemäß den nachstehenden Beispielen 40-51 wurden gemäß folgender allgemeiner Arbeitsvorschrift erhalten:**

Zu einer Lösung von 1 äq. 1,3-(*S*)-Dibenzyl-1,4,8-triaza-spiro[4.5]decan-2-on oder 1 äq. 1-Benzyl-3-(*S*)-(2-methylsulfanyl-ethyl)-1,4,8-triaza-spiro[4.5]decan-2-on in trockenem $CH_2Cl_2$ (5ml pro mmol Amin) gab man 2 äq. Triethylamin, eine katalytische Menge DMAP, kühlte die Reaktionslösung auf 0°C ab und gab dann tropfenweise 1 äq. Säurechlorid gelöst in trockenem $CH_2Cl_2$ (6ml pro mmol Säurechlorid) hinzu. Es wurde für 18 h gerührt wobei sich die Reaktionslösung bis auf RT erwärmte. Es wurde wässerige 5M Kalilauge zugegeben (4ml pro 1.2 mmol Amin), die wässerige Phase mit Dichlormethan extrahiert (2 x 5 ml), die vereinigten organischen Phasen mit wässeriger gesättigter NaCl-Lösung (10 ml) gewaschen und die gesammelten organischen Phasen über $Na_2SO_4$. Nach Abdestillation des Lösungsmittels erfolgte die weitere Aufreinigung über Säulenchromathographie.

**Beispiel 40:** 1,3-(*S*)-Dibenzyl-8-butyryl-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| Buttersäurechlorid: | 127 mg (1.19 mmol) |
| Triethylamin: | 241 mg (2.39 mmol) |
| Ausbeute nach Säulenchromathographie: | 263 mg (55 %) |

**Beispiel 41:** 1,3-(*S*)-Dibenzyl-8-(3-fluor-4-trifluormethyl-benzoyl)-1,4,8-triaza-spiro[4.5]decan-2-on

| | |
|---|---|
| 1,3-(*S*)-Dibenzy!-1,4,8-triaza-spiro[4.5]decan-2-on: | 400 mg (1.19 mmol) |
| 3-Fluor-4-(trifluormethylbenzoylchlorid): | 270 mg (1.19 mmol) |
| Triethylamin: | 241 mg (2.39 mmol) |
| Ausbeute nach Säulenchromathographie: | 409 mg (65 %) |

**Beispiel 42:** 1,3-(*S*)-Dibenzyl-8-(2,3-difluor-benzoyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 2,3-Difluorbenzoylchlorid: | 211 mg (1.19 mmol) |
| Triethylamin: | 241 mg (2.39 mmol) |
| Ausbeute nach Säulenchromathographie: | 383 mg (68 %) |

**Beispiel 43:** 1,3-(*S*)-Dibenzyl-8-[2-(4-chlor-phenoxy)-acetyl]-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 4-Chlorphenoxyacetylchlorid: | 245 mg (1.19 mmol) |
| Triethylamin: | 241 mg (2.39 mmol) |
| Ausbeute nach Säulenchromathographie: | 417 mg (69 %) |

**Beispiel 44:** 1,3-(*S*)-Dibenzyl-8-diphenylacetyl-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| Diphenylacetylchlorid: | 275 mg (1.19 mmol) |
| Triethylamin: | 241 mg (2.39 mmol) |
| Ausbeute nach Säulenchromathographie: | 460 mg (73 %) |

**Beispiel 45:** 1,3-(*S*)-Dibenzyl-8-(2-phenoxy-acetyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| Phenoxyacetylchlorid: | 203 mg (1.19 mmol) |
| Triethylamin: | 241 mg (2.39 mmol) |
| Ausbeute nach Säulenchromathographie: | 419 mg (75 %) |

**Beispiel 46:** 1,3-(*S*)-Dibenzyl-8-(3-phenyl-propionyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 3-Phenylpropionylchlorid: | 201 mg (1.19 mmol) |
| Triethylamin: | 241 mg (2.39 mmol) |
| Ausbeute nach Säulenchromathographie: | 417 mg (75 %) |

**Beispiel 47:** 1,3-(*S*)-Dibenzyl-8-(naphthalin-2-carbonyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 2-Naphthoylchlorid: | 227 mg (1.19 mmol) |
| Triethylamin: | 241 mg (2.39 mmol) |
| Ausbeute nach Säulenchromathographie: | 411 mg (70 %) |

**Beispiel 48:** 1,3-(*S*)-Dibenzyl-8-(furan-2-carbonyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 2-Furoylchlorid: | 156 mg (1.19 mmol) |
| Triethylamin: | 241 mg (2.39 mmol) |
| Ausbeute nach Säulenchromathographie: | 365 mg (71 %) |

**Beispiel 49:** 1,3-(*S*)-Dibenzyl-8-(3-methoxy-benzoyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 3-Methoxybenzoylchlorid: | 203 mg (1.19 mmol) |
| Triethylamin: | 241 mg (2.39 mmol) |
| Ausbeute nach Säulenchromathographie: | 404 mg (72 %) |

**Beispiel 50:** 1,3-(*S*)-Dibenzyl-8-(4-fluor-benzoyl)-1,4,8-triaza-spiro[4.5]decan-2-on

| | |
|---|---|
| 1,3-(*S*)-Dibenzyl-1,4,8-triaza-spiro[4.5]decan-2-on: | 400 mg (1.19 mmol) |
| 3-Methoxybenzoylchlorid: | 189 mg (1.19 mmol) |
| Triethylamin: | 241 mg (2.39 mmol) |
| Ausbeute nach Säulenchromathographie: | 377 mg (69 %) |

**Beispiel 51:** 1-Benzyl-8-(4-fluor-benzoyl)-3-(*S*)-(2-methylsulfanyl-ethyl)-1,4,8-triaza-spiro[4.5]decan-2-on

| | |
|---|---|
| 1-Benzyl-3-(*S*)-(2-methylsulfanyl-ethyl)- 1,4,8-triaza-spiro[4.5]decan-2-on: | 200 mg (1.19 mmol) |
| 3-Methoxybenzoylchlorid: | 99 mg (1.19 mmol) |
| Triethylamin: | 127 mg (2.39 mmol) |
| Ausbeute nach Säulenchromathographie: | 246 mg (89 %) |

**Beispiel 52:** 1,3-(*S*)-Dibenzyl-8-butyryl-1,4,8-triaza-spiro[4.5]decan-2-on Hydrochlorid

1,3-(*S*)-Dibenzyl-8-butyryl-1,4,8-triaza-spiro[4.5]decan-2-on (272 mg, 0.7 mmol) wurde in Ethylmethylketon (2.2 ml) gelöst, mit Wasser (7 μl) und TMSCI (93 μl) versetzt und über Nacht gerührt. Der Feststoff wurde abfiltriert, mit Ether gewaschen und im Vakuum getrocknet. Man erhielt das Produkt 1,3-(*S*)-Dibenzyl-8-butyryl-1,4,8-triaza-spiro[4.5]decan-2-on Hydrochlorid mit einer Ausbeute von 281 mg (95 %).

**Beispiel 53:** 1,3-(*S*)-Dibenzyl-8-(3-fluor-4-trifluormethyl-benzoyl)-1,4,8-triaza-spiro[4.5]decan-2-on Hydrochlorid

1,3-(*S*)-Dibenzyl-8-(3-fluor-4-trifluormethyl-benzoyl)-1,4,8-triaza-spiro[4.5]decan-2-on (397 mg, 0.8 mmol) wurde in Ethylmethylketon (3.2 ml) gelöst, mit Wasser (7 μl), Ether (30 ml) und mit TMSCI (105 μl) versetzt und über Nacht gerührt. Der Feststoff wurde abfiltriert, mit Ether gewaschen und im Vakuum getrocknet. Man erhielt das Produkt 11,3-(*S*)-Dibenzyl-8-(3-fluor-4-trifluormethyl-benzoyl)-1,4,8-triaza-spiro[4.5]decan-2-on Hydrochlorid mit einer Ausbeute von 396 mg (93 %).

**Beispiel 54:** 1,3-(*S*)-Dibenzyl-8-(2,3-difiuor-benzoyl)-1,4,8-triaza-spiro[4.5]decan-2-on Hydrochlorid

1,3-(*S*)-Dibenzyl-8-(2,3-difluor-benzoyl)-1,4,8-triaza-spiro[4.5]decan-2-on (370 mg, 0.8 mmol) wurde in Ethylmethylketon (2.9 ml) gelöst, mit Wasser (8 μl), Ether (30 ml) und mit TMSCI (108 μl) versetzt und über Nacht gerührt. Der Feststoff wurde abfiltriert, mit Ether gewaschen und im Vakuum getrocknet. Man erhielt das Produkt 1,3-(*S*)-Dibenzyl-8-(2,3-difluor-benzoyl)-1,4,8-triaza-spiro[4.5]decan-2-on Hydrochlorid mit einer Ausbeute von 317 mg (80 %).

**Beispiel 55:** 1,3-(*S*)-Dibenzyl-8-[2-(4-chlor-phenoxy)-acetyl]-1,4,8-triaza-spiro[4.5]decan-2-on Hydrochlorid

1,3-(*S*)-Dibenzyl-8-[2-(4-chlor-phenoxy)-acetyl]-1,4,8-triaza-spiro[4.5]decan-2-on (415 mg, 0.8 mmol) wurde in Ethylmethylketon (3.3 ml) gelöst, mit Wasser (8 μl), und mit TMSCI (115 μl) versetzt und über Nacht gerührt. Der Feststoff wurde abfiltriert, mit Ether gewaschen und im Vakuum getrocknet. Man erhielt das Produkt 1,3-(*S*)-Dibenzyl-8-[2-(4-chlor-phenoxy)-acetyl]-1,4,8-triaza-spiro[4.5]decan-2-on Hydrochlorid mit einer Ausbeute von 385 mg (87 %).

**Beispiel 56:** 1,3-(*S*)-Dibenzyl-8-diphenylacetyl-1,4,8-triaza-spiro[4.5]decan-2-on Hydrochlorid

1,3-(*S*)-Dibenzyl-8-diphenylacetyl-1,4,8-triaza-spiro[4.5]decan-2-on (450 mg, 0.8 mmol) wurde in Ethylmethylketon (3.6 ml) gelöst, mit Wasser (8 μl), mit TMSCI (118 μl) und mit Ether (10 ml) versetzt und über Nacht gerührt. Der Feststoff wurde abfiltriert, mit Ether gewaschen und im Vakuum getrocknet. Man erhielt das Produkt 1,3-( *S*)-Dibenzyl-8-diphenylacetyl-1,4,8-triaza-spiro[4.5]decan-2-on Hydrochlorid mit einer Ausbeute von 402 mg (84 %).

**Beispiel 57:** 1,3-(*S*)-Dibenzyl-8-(2-phenoxy-acetyl)-1,4,8-triaza-spiro[4.5]decan-2-on Hydrochlorid

1,3-(*S*)-Dibenzyl-8-(2-phenoxy-acetyl)-1,4,8-triaza-spiro[4.5]decan-2-on (410 mg, 0.9 mmol) wurde in Ethylmethylketon (3.3 ml) gelöst, mit Wasser (9 μl), mit TMSCI (121 μl) und mit Ether (30 ml) versetzt und über Nacht gerührt. Der Feststoff wurde abfiltriert, mit Ether gewaschen und im Vakuum getrocknet. Man erhielt das Produkt 1,3-(*S*)-Dibenzyl-8-(2-phenoxy-acetyl)-1,4,8-triaza-spiro[4.5]decan-2-on Hydrochlorid mit einer Ausbeute von 347 mg (79 %).

**Beispiel 58:** 1,3-(*S*)-Dibenzyl-8-(3-phenyl-propionyl)-1,4,8-triaza-spiro[4.5]decan-2-on Hydrochlorid

1,3-(*S*)-Dibenzyl-8-(3-phenyl-propionyl)-1,4,8-triaza-spiro[4.5]decan-2-on (360 mg, 0.8 mmol) wurde in Ethylmethylketon (2.9 ml) gelöst, mit Wasser (8 μl), TMSCI (107 μl) und mit Ether (10 ml) versetzt und über Nacht gerührt. Der Feststoff wurde abfiltriert, mit Ether gewaschen und im Vakuum getrocknet. Man erhielt das Produkt 1,3-( *S*)-Dibenzyl-8-(3-phenyl-propionyl)-1,4,8-triaza-spiro[4.5]decan-2-on Hydrochlorid mit einer Ausbeute von 323 mg (83 %).

**Beispiel 59:** 1,3-(*S*)-Dibenzyl-8-(naphthalin-2-carbonyl)-1,4,8-triaza-spiro[4.5]decan-2-on Hydrochlorid

1,3-(*S*)-Dibenzyl-8-(naphthalin-2-carbonyl)-1,4,8-triaza-spiro[4.5]decan-2-on (400 mg, 0.8 mmol) wurde in Ethylmethylketon (3.2 ml) gelöst, mit Wasser (8 μl), TMSCI (114 μl) und mit Ether (20 ml) versetzt und über Nacht gerührt. Der Feststoff wurde abfiltriert, mit Ether gewaschen und im Vakuum getrocknet. Man erhielt das Produkt 1,3-(*S*)-Dibenzyl-8-(naphthalin-2-carbonyl)-1,4,8-triaza-spiro[4.5]decan-2-on Hydrochlorid mit einer Ausbeute von 407 mg (95 %).

**Beispiel 60:** 1,3-(*S*)-Dibenzyl-8-(furan-2-carbonyl)-1,4,8-triaza-spiro[4.5]decan-2-on Hydrochlorid

1,3-(*S*)-Dibenzyl-8-(furan-2-carbonyl)-1,4,8-triaza-spiro[4.5]decan-2-on (365 mg, 0.9 mmol) wurde in Ethylmethylketon (2.9 ml) gelöst, mit Wasser (8 μl), TMSCI (118 μl) und mit Ether (20 ml) versetzt und über Nacht gerührt. Der Feststoff wurde abfiltriert, mit Ether gewaschen und im Vakuum getrocknet. Man erhielt das Produkt 1,3-(S)-Dibenzyl-8-(furan-2-carbonyl)-1,4,8-triaza-spiro[4.5]decan-2-on Hydrochlorid mit einer Ausbeute von 338 mg (85 %).

**Beispiel 61:** 1,3-(*S*)-Dibenzyl-8-(3-methoxy-benzoyl)-1,4,8-triaza-spiro[4.5]decan-2-on Hydrochlorid

1,3-(*S*)-Dibenzyl-8-(3-methoxy-benzoyl)-1,4,8-triaza-spiro[4.5]decan-2-on (404 mg, 0.9 mmol) wurde in Ethylmethylketon (3.2 ml) gelöst, mit Wasser (9 μl) und TMSCI (120 μl) versetzt und über Nacht gerührt. Der Feststoff wurde abfiltriert, mit Ether gewaschen und im Vakuum getrocknet. Man erhielt das Produkt 1,3-(*S*)-Dibenzyl-8-(3-methoxy-benzoyl)-1,4,8-triaza-spiro[4.5]decan-2-on Hydrochlorid mit einer Ausbeute von 358 mg (82 %).

**Beispiel 62:** 1,3-(*S*)-Dibenzyl-8-(4-fluor-benzoyl)-1,4,8-triaza-spiro[4.5]decan-2-on Hydrochlorid

1,3-(*S*)-Dibenzyl-8-(4-fluor-benzoyl)-1,4,8-triaza-spiro[4.5]decan-2-on (254 mg, 0.9 mmol) wurde in Ethylmethylketon (2.0 ml) gelöst, mit Wasser (5 μl), mit TMSCI (77 μl) und mit Ether (20 ml) versetzt und über Nacht gerührt. Der Feststoff wurde abfiltriert, mit Ether gewaschen und im Vakuum getrocknet. Man erhielt das Produkt 1,3-(*S*)-Dibenzyl-8-(4-fluor-benzoyl)-1,4,8-triaza-spiro[4.5]decan-2-on Hydrochlorid mit einer Ausbeute von 242 mg (88 %).

**Beispiel 63:** 1-Benzyl-8-(4-fluor-benzoyl)-3-(*S*)-(2-methylsulfanyl-ethyl)-1,4,8-triaza-spiro[4.5]decan-2-on Hydrochlorid

1-Benzyl-8-(4-fluor-benzoyl)-3-(*S*)-(2-methylsulfanyl-ethyl)-1,4,8-triaza-spiro[4.5]decan-2-on (223 mg, 0.5 mmol) wurde in Ethylmethylketon (1.8 ml) gelöst, mit Wasser (5 μl) und mit TMSCI (70 μl) versetzt und über Nacht gerührt. Der Feststoff wurde abfiltriert, mit Ether gewaschen und im Vakuum getrocknet. Man erhielt das Produkt 1-Benzyl-8-(4-fluor-benzoyl)-3-(*S*)-(2-methylsulfanyl-ethyl)-1,4,8-triaza-spiro[4.5]decan-2-on Hydrochlorid mit einer Ausbeute von 227 mg (94 %).

Die Verbindungen gemäß den nachfolgenden Beispielen wurden analog nach dem vorstehend beschriebenen allgemeinen Herstellungsverfahren II erhalten:

| Bsp. | Verbindung |
|---|---|
| **64** | N-[4-(3-Isobutyl-2-oxo-1-prop-2-ynyl-1,4,8-triaza-spiro[4.5]decan-8-carbonyl)-phenyl]-acetamid |
| **65** | 1-(2-Phenoxy-ethyl)-8-(thiophen-2-carbonyl)-1,4,8-triaza-spiro[4.5]decan-2-on |
| **66** | 2-(2-Oxo-8-pent-4-enoyl-1,4,8-triaza-spiro[4.5]dec-1-ylmethyl)-benzonitril |
| **67** | 8-(2,4-Dimethoxy-benzoyl)-1-methyl-3-(2-methylsulfanyl-ethyl)-1,4,8-triaza-spiro[4.5]decan-2-on |
| **68** | 2-[8-(2-Ethyl-butyryl)-2-oxo-1,4,8-triaza-spiro[4.5]dec-1-ylmethyl]-benzonitril |
| **69** | 4-(2-Isobutyl-3-oxo-1,4,8-triaza-spiro[4.5]decan-8-carbonyl)-benzonitril |
| **70** | 8-(6-Chlor-pyridin-3-carbonyl)-3-isobutyl-1,4,8-triaza-spiro[4.5]decan-2-on |
| **71** | 2-[8-(2-Methyl-pentanoyl)-2-oxo-1,4,8-triaza-spiro[4.5]dec-1-ylmethyl]-benzonitril |
| **72** | 1-Benzyl-8-(biphenyl-4-carbonyl)-1,4,8-triaza-spiro[4.5]decan-2-on |
| **73** | 3-Isobutyl-8-(5-methyl-isoxazol-3-carbonyl)-1,4,8-triaza-spiro[4.5]decan-2-on |
| **74** | 3-Oxo-3-[2-oxo-1-(2-phenoxy-ethyl)-1,4,8-triaza-spiro[4.5]dec-8-yl]-propionsäureethylester |
| **75** | 8-(2-Chlor-benzoyl)-3-isopropyl-1,4,8-triaza-spiro[4.5]decan-2-on |
| **76** | 8-Cyclopentancarbonyl-3-isopropyl-1,4,8-triaza-spiro[4.5]decan-2-on |
| **77** | 8-(Furan-2-carbonyl)-1-methyl-3-(2-methylsulfanyl-ethyl)-1,4,8-triaza-spiro[4.5]decan-2-on |
| **78** | 3-Benzyl-8-(2-ethylsulfanyl-pyridin-3-carbonyl)-1-methyl-1,4,8-triaza-spiro[4.5]decan-2-on |
| **79** | 3-Benzyl-8-(4-chlor-benzoyl)-1,4,8-triaza-spiro[4.5]decan-2-on |
| **80** | 8-(2-Benzyloxy-acetyl)-3-isobutyl-1,4,8-triaza-spiro[4.5]decan-2-on |

(fortgesetzt)

| Bsp. | Verbindung |
|---|---|
| 81 | 3-Benzyl-8-(2-methoxy-acetyl)-1-prop-2-ynyl-1,4,8-triaza-spiro[4.5]decan-2-on |
| 82 | 8-(5-tert-Butyl-2-methyl-2H-pyrazol-3-carbonyl)-3-isobutyl-1,4,8-triaza-spiro[4.5]decan-2-on |
| 83 | 2-{8-[3-(2-chlor-6-fluor-phenyl)-5-methyl-isoxazol-4-carbonyl]-2-oxo-1,4,8-triaza-spiro[4.5]dec-1-ylmethyl}-benzonitril |
| 84 | 3-Benzyl-8-(2-chlor-benzoyl)-1,4,8-triaza-spiro[4.5]decan-2-on |
| 85 | 3-Benzyl-8-(3-dimethylamino-benzoyl)-1-methyl-1,4,8-triaza-spiro[4.5]decan-2-on |
| 86 | 8-(3-Methyl-benzoyl)-1-(2-phenoxy-ethyl)-1,4,8-triaza-spiro[4.5]decan-2-on |
| 87 | 3-Isopropyl-1-(3-methyl-but-2-enyl)-8-(pyridin-4-carbonyl)-1,4,8-triaza-spiro[4.5]decan-2-on |
| 88 | 1-Benzyl-8-(4-methyl-[1,2,3]thiadiazol-5-carbonyl)-1,4,8-triaza-spiro[4.5]decan-2-on |
| 89 | 2-{8-[3-(2-chlor-phenyl)-acryloyl]-2-oxo-1,4,8-triaza-spiro[4.5]dec-1-ylmethyl}-benzonitril |
| 90 | 8-(3-Chlor-benzoyl)-3-isobutyl-1,4,8-triaza-spiro[4.5]decan-2-on |
| 91 | Essigsäure 2-(2-benzyl-3-oxo-1,4,8-triaza-spiro[4.5]dec-8-yl)-2-oxo-1-phenyl-ethylester |
| 92 | 8-(3,5-Dimethoxy-benzoyl)-3-isobutyl-1,4,8-triaza-spiro[4.5]decan-2-on |
| 93 | 3-Benzyl-8-(isoxazol-5-carbonyl)-1,4,8-triaza-spiro[4.5]decan-2-on |
| 94 | 8-(3-Chlor-thiophen-2-carbonyl)-3-isobutyl-1,4,8-triaza-spiro[4.5]decan-2-on |
| 95 | 3-Isopropyl-8-pentafluorbenzoyl-1,4,8-triaza-spiro[4.5]decan-2-on |
| 96 | 8-(2,5-Dimethyl-furan-3-carbonyl)-3-isobutyl-1,4,8-triaza-spiro[4.5]decan-2-on |
| 97 | 1-Butyl-8-[2-(3,4-dimethoxy-phenyl)-acetyl]-3-(2-methylsulfanyl-ethyl)-1,4,8-triaza-spiro[4.5]decan-2-on |
| 98 | 1-Benzyl-3-isopropyl-8-(pyridin-4-carbonyl)-1,4,8-triaza-spiro[4.5]decan-2-on |
| 99 | 1,3-Dibenzyl-8-(3-dimethylamino-benzoyl)-1,4,8-triaza-spiro[4.5]decan-2-on |
| 100 | 5-{2-[1-(4-Fluor-benzyl)-3-(2-methylsulfanyl-ethyl)-2-oxo-1,4,8-triaza-spiro[4.5]dec-8-yl]-2-oxo-ethyl}-imidazolidin-2,4-dion |
| 101 | 8-(Biphenyl-4-carbonyl)-1-(4-fluor-benzyl)-3-isobutyl-1,4,8-triaza-spiro[4.5]decan-2-on |
| 102 | 2-[2-Oxo-8-(2-propyl-pentanoyl)-1,4,8-triaza-spiro[4.5]dec-1-ylmethyl]-benzonitril |
| 103 | 2-[8-(Furan-2-carbonyl)-3-isopropyl-2-oxo-1,4,8-triaza-spiro[4.5]dec-1-ylmethyl]-benzonitril |
| 104 | 8-[2-(4-Chlor-phenoxy)-acetyl]-3-isobutyl-1-prop-2-ynyl-1,4,8-triaza-spiro[4.5]decan-2-on |
| 105 | 1,3-Dibenzyl-8-(4-brom-benzoyl)-1,4,8-triaza-spiro[4.5]decan-2-on |
| 106 | 8-(3-Difluormethylsulfanyl-benzoyl)-3-isobutyl-1,4,8-triaza-spiro[4.5]decan-2-on |
| 107 | 8-(2,3-Dimethyl-benzoyl)-3-isopropyl-1,4,8-triaza-spiro[4.5]decan-2-on |
| 108 | 3-Benzyl-8-(2,3-dimethyl-benzoyl)-1,4,8-triaza-spiro[4.5]decan-2-on |
| 109 | 1-(4-Fluor-benzyl)-3-(2-methylsulfanyl-ethyl)-8-(pyridin-2-carbonyl)-1,4,8-triaza-spiro[4.5]decan-2-on |
| 110 | 3-Benzyl-8-(3,3-dimethyl-butyryl)-1,4,8-triaza-spiro[4.5]decan-2-on |
| 111 | 2-[8-(3-Dimethylamino-benzoyl)-3-Isobutyl-2-oxo-1,4,8-triaza-spiro[4.5]dec-1-ylmethyl]-benzonitril |
| 112 | 3-[8-(2-Methoxy-acetyl)-3-(2-methylsulfanyl-ethyl)-2-oxo-1,4,8-triaza-spiro[4.5]dec-1-ylmethyl]-benzonitril |
| 113 | Essigsäure 2-(3-benzyl-1-methyl-2-oxo-1,4,8-triaza-spiro[4.5]dec-8-yl)-2-oxo-1-phenyl-ethylester |
| 114 | 2-[8-[2-(2-Methoxy-ethoxy)-acetyl]-3-(2-methylsulfanyl-ethyl)-2-oxo-1,4,8-triaza-spiro[4.5]dec-1-ylmethyl]-benzonitril |
| 115 | 3-Benzyl-8-[3-(2-chlor-6-fluor-phenyl)-5-methyl-isoxazol-4-carbonyl]-1,4,8-triaza-spiro[4.5]decan-2-on |

(fortgesetzt)

| Bsp. | Verbindung |
|---|---|
| 116 | 8-(2-Chlor-6-fluor-benzoyl)-3-isopropyl-1,4,8-triaza-spiro[4.5]decan-2-on |
| 117 | 1-Methyl-3-(2-methylsulfanyl-ethyl)-8-(2-phenoxy-acetyl)-1,4,8-triaza-spiro[4.5]decan-2-on |
| 118 | 8-(2-Chlor-pyridin-3-carbonyl)-1-(4-fluor-benzyl)-3-isobutyl-1,4,8-triaza-spiro[4.5]decan-2-on |
| 119 | 8-(2-Ethyl-butyryl)-1-methyl-3-(2-methylsulfanyl-ethyl)-1,4,8-triaza-spiro[4.5]decan-2-on |
| 120 | 1-Methyl-3-(2-methylsulfanyl-ethyl)-8-(2-phenoxy-pyridin-3-carbonyl)-1,4,8-triaza-spiro[4.5]decan-2-on |
| 121 | 8-(3-Fluor-benzoyl)-1-methyl-3-(2-methylsulfanyl-ethyl)-1,4,8-triaza-spiro[4.5]decan-2-on |
| 122 | 3-Benzyl-8-(naphthalin-2-carbonyl)-1,4,8-triaza-spiro[4.5]decan-2-on |
| 123 | 8-Cyclohexancarbonyl-1-(2-phenoxy-ethyl)-1,4,8-triaza-spiro[4.5]decan-2-on |
| 124 | 8-(2-Phenoxy-acetyl)-1-(2-phenoxy-ethyl)-1,4,8-triaza-spiro[4.5]decan-2-on |
| 125 | 4-[1-Butyl-3-(2-methylsulfanyl-ethyl)-2-oxo-1,4,8-triaza-spiro[4.5]decan-8-carbonyl]-benzonitril |
| 126 | 3-Benzyl-8-(3,3-dimethyl-butyryl)-1-methyl-1,4,8-triaza-spiro[4.5]decan-2-on |
| 127 | 3-Benzyl-8-(4-propyl-benzoyl)-1,4,8-triaza-spiro[4.5]decan-2-on |
| 128 | 3-Isopropyl-8-(2-phenoxy-propionyl)-1,4,8-triaza-spiro[4.5]decan-2-on |
| 129 | 1-Butyl-8-hexanoyl-3-isopropyl-1,4,8-triaza-spiro[4.5]decan-2-on |
| 130 | 8-(4-Brom-3-methyl-benzoyl)-3-isopropyl-1,4,8-triaza-spiro[4.5]decan-2-on |
| 131 | 8-[3-(2-Chlor-6-fluor-phenyl)-5-methyl-isoxazol-4-carbonyl]-3-isopropyl-1-(2-phenoxy-ethyl)-1,4,8-triaza-spiro[4.5]decan-2-on |
| 132 | 1-(2-Fluor-benzyl)-3-isobutyl-8-(3-methyl-benzoyl)-1,4,8-triaza-spiro[4.5]decan-2-on |
| 133 | 8-(2-Ethyl-hexanoyl)-1-(2-phenoxy-ethyl)-1,4,8-triaza-spiro[4.5]decan-2-on |
| 134 | 3-Benzyl-8-(3,4-difluor-benzoyl)-1,4,8-triaza-spiro[4.5]decan-2-on |
| 135 | 3-Benzyl-8-(4-ethoxy-benzoyl)-1-methyl-1,4,8-triaza-spiro[4.5]decan-2-on |
| 136 | 1-Benzyl-8-(6-chlor-pyridin-3-carbonyl)-1,4,8-triaza-spiro[4.5]decan-2-on |
| 137 | 8-(3-Dimethylamino-benzoyl)-1-(3,5-dimethyl-benzyl)-3-(2-methylsulfanyl-ethyl)-1,4,8-triaza-spiro[4.5] decan-2-on |
| 138 | 3-[8-(Benzo[1,3]dioxol-5-carbonyl)-3-(2-methylsulfanyl-ethyl)-2-oxo-1,4,8-triaza-spiro[4.5]dec-1-ylmethyl]-benzonitril |
| 139 | 3-Benzyl-1-methyl-8-pent-4-enoyl-1,4,8-triaza-spiro[4.5]decan-2-on |
| 140 | 8-(4-Ethoxy-benzoyl)-1-methyl-3-(2-methylsulfanyl-ethyl)-1,4,8-triaza-spiro[4.5]decan-2-on |
| 141 | 3-Benzyl-8-(2-benzyloxy-acetyl)-1-methyl-1,4,8-triaza-spiro[4.5]decan-2-on |
| 142 | 8-(3,4-Difluor-benzoyl)-1-(2-phenoxy-ethyl)-1,4,8-triaza-spiro[4.5]decan-2-on |
| 143 | 3-Benzyl-1-methyl-8-(2-methylsulfanyl-pyridin-3-carbonyl)-1,4,8-triaza-spiro[4.5]decan-2-on |
| 144 | 1-Butyl-8-(4-methoxy-benzoyl)-3-(2-methylsulfanyl-ethyl)-1,4,8-triaza-spiro[4.5]decan-2-on |
| 145 | 1,3-Dibenzyl-8-(2-ethylsulfanyl-pyridin-3-carbonyl)-1,4,8-triaza-spiro[4.5]decan-2-on |
| 146 | 3-Benzyl-8-(3-difluormethylsulfanyl-benzoyl)-1,4,8-triaza-spiro[4.5]decan-2-on |
| 147 | 1-(4-Fluor-benzyl)-8-(furan-2-carbonyl)-3-(2-methylsulfanyl-ethyl)-1,4,8-triaza-spiro[4.5]decan-2-on |
| 148 | 3-Benzyl-8-(3-fluor-4-methyl-benzoyl)-1-methyl-1,4,8-triaza-spiro[4.5]decan-2-on |
| 149 | 8-[3-(2-Chlor-6-fluor-phenyl)-5-methyl-isoxazol-4-carbonyl]-3-isopropyl-1,4,8-triaza-spiro[4.5]decan-2-on |
| 150 | 1-Butyl-8-(6-Chlor-2H-chroman-3-carbonyl)-3-(2-methylsulfanyl-ethyl)-1,4,8-triaza-spiro[4.5]decan-2-on |

(fortgesetzt)

| Bsp. | Verbindung |
|---|---|
| 151 | 3-[8-(3-Methoxy-benzoyl)-3-(2-methylsulfanyl-ethyl)-2-oxo-1,4,8-triaza-spiro[4.5]dec-1-ylmethyl]-benzonitril |
| 152 | 8-Cyclobutancarbonyl-3-(2-methylsulfanyl-ethyl)-1-prop-2-ynyl-1,4,8-triaza-spiro[4.5]decan-2-on |
| 153 | 3-Benzyl-1-butyl-8-(furan-2-carbonyl)-1,4,8-triaza-spiro[4.5]decan-2-on |
| 154 | 1-Benzyl-8-(3-chlor-thiophen-2-carbonyl)-3-isobutyl-1,4,8-triaza-spiro[4.5]decan-2-on |
| 155 | 3-Benzyl-8-(2,5-bis-trifluormethyl-benzoyl)-1-butyl-1,4,8-triaza-spiro[4.5]decan-2-on |
| 156 | 8-(3-Chlor-2-fluor-benzoyl)-1-(3,5-dimethyl-benzyl)-3-(2-methylsulfanyl-ethyl)-1,4,8-triaza-spiro[4.5]decan-2-on |
| 157 | 1-Benzyl-8-(2-chlor-pyridin-3-carbonyl)-3-isopropyl-1,4,8-triaza-spiro[4.5]decan-2-on |
| 158 | 3-Isobutyl-8-pentafluorbenzoyl-1-prop-2-ynyl-1,4,8-triaza-spiro[4.5]decan-2-on |
| 159 | 8-(2-Benzyloxy-acetyl)-1-butyl-3-(2-methylsulfanyl-ethyl)-1,4,8-triaza-spiro[4.5]decan-2-on |
| 160 | 8-(Furan-2-carbonyl)-3-isobutyl-1-prop-2-ynyl-1,4,8-triaza-spiro[4.5]decan-2-on |
| 161 | 1-Butyl-3-(2-methylsulfanyl-ethyl)-8-(4-phenoxy-butyryl)-1,4,8-triaza-spiro[4.5]decan-2-on |
| 162 | 3-Benzyl-8-(6-chlor-pyridin-3-carbonyl)-1-methyl-1,4,8-triaza-spiro[4.5]decan-2-on |
| 163 | 1-(2-Fluor-benzyl)-3-(2-methylsulfanyl-ethyl)-8-(2-phenoxy-pyridin-3-carbonyl)-1,4,8-triaza-spiro[4.5] decan-2-on |
| 164 | 1-(2-Fluor-benzyl)-3-isobutyl-8-(2-methyl-6-trifluormethyl-pyridin-3-carbonyl)-1,4,8-triaza-spiro[4.5]decan-2-on |
| 165 | 8-[2-(3-Chlor-phenoxy)-acetyl]-3-isopropyl-1-(2-phenoxy-ethyl)-1,4,8-triaza-spiro[4.5]decan-2-on |
| 166 | 8-(2,3-Dimethyl-benzoyl)-1-methyl-3-(2-methylsulfanyl-ethyl)-1,4,8-triaza-spiro[4.5]decan-2-on |
| 167 | 3-Isopropyl-8-(2-methyl-6-trifluormethyl-pyridin-3-carbonyl)-1-(2-phenoxy-ethyl)-1,4,8-triaza-spiro[4.5] decan-2-on |
| 168 | 3-Benzyl-1-methyl-8-(5-methyl-2-phenyl-2H-[1,2,3]triazole-4-carbonyl)-1,4,8-triaza-spiro[4.5]decan-2-on |
| 169 | 8-[3-(2-Chlor-phenyl)-5-methyl-isoxazol-4-carbonyl]-1-(3,5-dimethyl-benzyl)-3-(2-methylsulfanyl-ethyl)-1,4,8-triaza-spiro[4.5]decan-2-on |
| 170 | 1-(2-Fluor-benzyl)-3-(2-methylsulfanyl-ethyl)-8-(pyridin-2-carbonyl)-1,4,8-triaza-spiro[4.5]decan-2-on |
| 171 | 1-Benzyl-3-(2-methylsulfanyl-ethyl)-8-(2-phenoxy-pyridin-3-carbonyl)-1,4,8-triaza-spiro[4.5]decan-2-on |
| 172 | 1,3-Dibenzyl-8-(3-chlor-thiophen-2-carbonyl)-1,4,8-triaza-spiro[4.5]decan-2-on |
| 173 | 3-Benzyl-8-(4-tert-butyl-benzoyl)-1-methyl-1,4,8-triaza-spiro[4.5]decan-2-on |
| 174 | 2-[3-Isobutyl-8-(2-methoxy-acetyl)-2-oxo-1,4,8-triaza-spiro[4.5]dec-1-ylmethyl]-benzonitril |
| 175 | 3-Benzyl-1-butyl-8-(5-fluor-2-trifluormethyl-benzoyl)-1,4,8-triaza-spiro[4.5]decan-2-on |
| 176 | 3-Benzyl-8-[2-(4-methoxy-phenyl)-acetyl]-1-methyl-1,4,8-triaza-spiro[4.5]decan-2-on |
| 177 | 1,3-Dibenzyl-8-(4-methyl-[1,2,3]thiadiazol-5-carbonyl)-1,4,8-triaza-spiro[4.5]decan-2-on |
| 178 | 1-Benzyl-3-isopropyl-8-(2-methyl-6-trifluorm ethyl-pyrid in-3-carbonyl)-1,4,8-triaza-spiro[4.5]decan-2-on |
| 179 | 1-Benzyl-8-(4-ethoxy-benzoyl)-3-isobutyl-1,4,8-triaza-spiro[4.5]decan-2-on |
| 180 | 3-Benzyl-1-butyl-8-cyclohexancarbonyl-1,4,8-triaza-spiro[4.5]decan-2-on |
| 181 | 3-[8-[3-(2-Chlor-6-fluor-phenyl)-5-methyl-isoxazol-4-carbonyl]-3-(2-methylsulfanyl-ethyl)-2-oxo-1,4,8-triaza-spiro[4.5]dec-1-ylmethyl]-benzonitril |
| 182 | 1-(2-Fluor-benzyl)-3-isobutyl-8-pent-4-enoyl-1,4,8-triaza-spiro[4.5]decan-2-on |
| 183 | 1-Methyl-3-(2-methylsulfanyl-ethyl)-8-(2-propyl-pentanoyl)-1,4,8-triaza-spiro[4.5]decan-2-on |

(fortgesetzt)

| Bsp. | Verbindung |
|---|---|
| 184 | 3-Benzyl-1-methyl-8-(2-phenoxy-acetyl)-1,4,8-triaza-spiro[4.5]decan-2-on |
| 185 | 3-Isobutyl-1-prop-2-ynyl-8-(3-trifluormethyl-benzoyl)-1,4,8-triaza-spiro[4.5]decan-2-on |
| 186 | 3-Benzyl-8-(furan-2-carbonyl)-1-prop-2-ynyl-1,4,8-triaza-spiro[4.5]decan-2-on |
| 187 | 1-Methyl-3-(2-methylsulfanyl-ethyl)-8-(naphthalin-1-carbonyl)-1,4,8-triaza-spiro[4.5]decan-2-on |
| 188 | 3-Benzyl-1-butyl-8-(3-cyclopentyl-propionyl)-1,4,8-triaza-spiro[4.5]decan-2-on |
| 189 | 1-(3,5-Dimethyl-benzyl)-3-(2-methylsulfanyl-ethyl)-8-pentanoyl-1,4,8-triaza-spiro[4.5]decan-2-on |
| 190 | 3-Benzyl-1-butyl-8-(2-methoxy-acetyl)-1,4,8-triaza-spiro[4.5]decan-2-on |
| 191 | 3-Benzyl-8-(3-fluor-4-trifluormethyl-benzoyl)-1-methyl-1,4,8-triaza-spiro[4.5]decan-2-on |
| 192 | 1-Benzyl-8-(3-difluormethylsulfanyl-benzoyl)-3-isopropyl-1,4,8-triaza-spiro[4.5]decan-2-on |
| 193 | 8-(2-Chlor-6-fluor-3-methyl-benzoyl)-1-methyl-3-(2-methylsulfanyl-ethyl)-1,4,8-triaza-spiro[4.5]decan-2-on |
| 194 | 4-[3-Isopropyl-8-(2-methylsulfanyl-pyridin-3-carbonyl)-2-oxo-1,4,8-triaza-spiro[4.5]dec-1-ylmethyl]-benzoesäuremethylester |
| 195 | 8-[2-(2,5-Dimethoxy-phenyl)-acetyl]-1-(2-fluor-benzyl)-3-(2-methylsulfanyl-ethyl)-1,4,8-triaza-spiro[4.5] decan-2-on |
| 196 | 8-(5-tert-Butyl-2-methyl-furan-3-carbonyl)-3-isopropyl-1-(2-phenoxy-ethyl)-1,4,8-triaza-spiro[4.5]decan-2-on |
| 197 | 8-(2-Cyclopentyl-acetyl)-1-(4-fluor-benzyl)-3-isobutyl-1,4,8-triaza-spiro[4.5]decan-2-on |
| 198 | 4-[8-(3,3-Dimethyl-butyryl)-3-isopropyl-2-oxo-1,4,8-triaza-spiro[4.5]dec-1-ylmethyl]-benzoesäuremethylester |
| 199 | 3-[8-Cyclopropancarbonyl-3-(2-methylsulfanyl-ethyl)-2-oxo-1,4,8-triaza-spiro[4.5]dec-1-ylmethyl]-benzonitril |
| 200 | 3-(3-(2-Methylsulfanyl-ethyl)-2-oxo-8-(3-trifluormethoxy-benzoyl)-1,4,8-triaza-spiro[4.5]dec-1-ylmethyl]-benzonitril |
| 201 | 1-Butyl-8-(2-cyclopentyl-acetyl)-3-isopropyl-1,4,8-triaza-spiro[4.5]decan-2-on |
| 202 | 3-Benzyl-1-butyl-8-(pyridin-2-carbonyl)-1,4,8-triaza-spiro[4.5]decan-2-on |
| 203 | 3-Benzyl-8-[3-(2-chlor-phenyl)-acryloyl]-1-methyl-1,4,8-triaza-spiro[4.5]decan-2-on |
| 204 | 4-[8-(3-fluor-4-trifluormethyl-benzoyl)-3-isopropyl-2-oxo-1,4,8-triaza-spiro[4.5]dec-1-ylmethyl]-benzoesäuremethylester |
| 205 | 8-[3-(2,6-Dichlor-phenyl)-5-methyl-isoxazol-4-carbonyl]-1-methyl-3-(2-methylsulfanyl-ethyl)-1,4,8-triaza-spiro[4.5]decan-2-on |
| 206 | 1-Butyl-8-cyclohexancarbonyl-3-(2-methylsulfanyl-ethyl)-1,4,8-triaza-spiro[4.5]decan-2-on |
| 207 | 3-Benzyl-1-butyl-8-(4-iod-benzoyl)-1,4,8-triaza-spiro[4.5]decan-2-on |
| 208 | 1-Methyl-3-(2-methylsulfanyl-ethyl)-8-[3-(3-trifluormethyl-phenyl)-acryloyl]-1,4,8-triaza-spiro[4.5]decan-2-on |
| 209 | 1,3-Dibenzyl-8-(4-phenoxy-butyryl)-1,4,8-triaza-spiro[4.5]decan-2-on |
| 210 | 3-Benzyl-8-(2-chlor-6-fluor-benzoyl)-1-prop-2-ynyl-1,4,8-triaza-spiro[4.5]decan-2-on |
| 211 | 4-[8-(2-Chlor-pyridin-3-carbonyl)-3-isopropyl-2-oxo-1,4,8-triaza-spiro[4.5]dec-1-ylmethyl]-benzoesäuremethylester |
| 212 | 8-(2,5-Dimethyl-furan-3-carbonyl)-3-isopropyl-1-(2-phenoxy-ethyl)-1,4,8-triaza-spiro[4.5]decan-2-on |
| 213 | 8-(Biphenyl-4-carbonyl)-3-(2-methylsulfanyl-ethyl)-1-prop-2-ynyl-1,4,8-triaza-spiro[4.5]decan-2-on |
| 214 | 8-(3-Chlor-thiophen-2-carbonyl)-3-(2-methylsulfanyl-ethyl)-1-prop-2-ynyl-1,4,8-triaza-spiro[4.5]decan-2-on |

(fortgesetzt)

| Bsp. | Verbindung |
|---|---|
| **215** | 1-(4-Fluor-benzyl)-8-[2-(4-methoxy-phenyl)-acetyl]-3-(2-methylsulfanyl-ethyl)-1,4,8-triaza-spiro[4.5]decan-2-on |
| **216** | 1-Benzyl-3-isopropyl-8-(4-methyl-[1,2,3]thiadiazol-5-carbonyl)-1,4,8-triaza-spiro[4.5]decan-2-on |
| **217** | 2-[3-Isopropyl-8-(2-methylsulfanyl-pyridin-3-carbonyl)-2-oxo-1,4,8-triaza-spiro[4.5]dec-1-ylmethyl]-benzonitril |
| **218** | 3-[8-(5-tert-Butyl-2-methyl-2H-pyrazol-3-carbonyl)-3-isobutyl-2-oxo-1,4,8-triaza-spiro[4.5]dec-1-ylmethyl]-benzonitril |
| **219** | 1-Butyl-8-(2, 5-dimethyl-furan-3-carbonyl)-3-(2-methylsulfanyl-ethyl)-1,4,8-triaza-spiro[4.5]decan-2-on |
| **220** | 8-(3-Cyclopentyl-propionyl)-1-(2-fluor-benzyl)-3-isobutyl-1,4,8-triaza-spiro[4.5]decan-2-on |
| **221** | 1-Benzyl-8-(3-cyclopentyl-propionyl)-3-(2-methylsulfanyl-ethyl)-1,4,8-triaza-spiro[4.5]decan-2-on |
| **222** | 3-(2-Methylsulfanyl-ethyl)-8-(4-phenoxy-butyryl)-1-prop-2-ynyl-1,4,8-triaza-spiro[4.5]decan-2-on |
| **223** | 1,3-Dibenzyl-8-(3-phenyl-acryloyl)-1,4,8-triaza-spiro[4.5]decan-2-on |
| **224** | 3-Benzyl-8-(6-chlor-2-fluor-3-methyl-benzoyl)-1-methyl-1,4,8-triaza-spiro[4.5]decan-2-on |
| **225** | 8-[3-(2-Chlor-phenyl)-acryloyl]-3-isopropyl-1-(2-phenoxy-ethyl)-1,4,8-triaza-spiro[4.5]decan-2-on |
| **226** | 2-[3-Isopropyl-2-oxo-8-(3-phenyl-acryloyl)-1,4,8-triaza-spiro[4.5]dec-1-ylmethyl]-benzonitril |
| **227** | 3-Benzyl-1-methyl-8-(4-methyl-3-nitro-benzoyl)-1,4,8-triaza-spiro[4.5]decan-2-on |
| **228** | 1-Benzyl-8-(furan-2-carbonyl)-3-isopropyl-1,4,8-triaza-spiro[4.5]decan-2-on |
| **229** | 1-Butyl-8-(3,5-dimethoxy-benzoyl)-3-(2-methylsulfanyl-ethyl)-1,4,8-triaza-spiro[4.5]decan-2-on |
| **230** | 1,3-Dibenzyl-8-(3,3-dimethyl-butyryl)-1,4,8-triaza-spiro[4.5]decan-2-on |
| **231** | 8-(2,6-Difluor-3-methyl-benzoyl)-1-methyl-3-(2-methylsulfanyl-ethyl)-1,4,8-triaza-spiro[4.5]decan-2-on |
| **232** | 2-[8-(2-Chlor-6-fluor-benzoyl)-3-(2-methylsulfanyl-ethyl)-2-oxo-1,4,8-triaza-spiro[4.5]dec-1-ylmethyl]-benzonitril |
| **233** | 8-[3-(2-Chlor-6-fluor-phenyl)-5-methyl-isoxazol-4-carbonyl]-3-isopropyl-1-(3-methyl-but-2-enyl)-1,4,8-triaza-spiro[4.5]decan-2-on |
| **234** | 3-Isobutyl-1-prop-2-ynyl-8-(4-trifluormethoxy-benzoyl)-1,4,8-triaza-spiro[4.5]decan-2-on |
| **235** | 1-Benzyl-8-(2-chlor-6-fluor-3-methyl-benzoyl)-1,4,8-triaza-spiro[4.5]decan-2-on |
| **236** | Benzoesäure 2-(1-benzyl-3-isopropyl-2-oxo-1,4,8-triaza-spiro[4.5]decan-8-carbonyl)-benzylester |
| **237** | 1,3-Dibenzyl-8-(2-phenyl-butyryl)-1,4,8-triaza-spiro[4.5]decan-2-on |
| **238** | 3-Benzyl-1-methyl-8-(4-nitro-benzoyl)-1,4,8-triaza-spiro[4.5]decan-2-on |
| **239** | 3-Benzyl-1-methyl-8-(5-methyl-isoxazol-3-carbonyl)-1,4,8-triaza-spiro[4.5]decan-2-on |
| **240** | 1-Benzyl-8-(6-chlor-2-fluor-3-methyl-benzoyl)-3-(2-methylsulfanyl-ethyl)-1,4,8-triaza-spiro[4.5]decan-2-on |
| **241** | 1-Benryl-8-(3-fluor-4-trifluormethyl-benzoyl)-3-isobutyl-1,4,8-triaza-spiro[4.5]decan-2-on |
| **242** | 3-Benzyl-8-(6-chlor-2H-chroman-3-carbonyl)-1-methyl-1,4,8-triaza-spiro[4.5]decan-2-on |
| **243** | 3-Benzyl-1-butyl-8-(4-methyl-[1,2,3]thiadiazol-5-carbonyl)-1,4,8-triaza-spiro[4.5]decan-2-on |
| **244** | 3-Benzyl-8-(4-methyl-[1,2,3]thiadiazol-5-carbonyl)-1-prop-2-ynyl-1,4,8-triaza-spiro[4.5]decan-2-on |
| **245** | 2-[8-(6-Chlor-2H-chroman-3-carbonyl)-3-isopropyl-2-oxo-1,4,8-triaza-spiro[4.5]dec-1-ylmethyl]-benzonitril |
| **246** | 2-[8-(5-Methyl-isoxazol-3-carbonyl)-3-(2-methylsulfanyl-ethyl)-2-oxo-1,4,8-triaza-spiro[4.5]dec-1-ylmethyl]-benzonitril |
| **247** | 2-[8-(3-Chlor-2-fluor-benzoyl)-3-isopropyl-2-oxo-1,4,8-triaza-spiro[4.5]dec-1-ylmethyl]-benzonitril |

(fortgesetzt)

| Bsp. | Verbindung |
|---|---|
| **248** | 4-[8-(5-tert-Butyl-2-methyl-2H-pyrazol-3-carbonyl)-3-isopropyl-2-oxo-1,4,8-triaza-spiro[4.5]dec-1-ylmethyl]-benzoesäuremethylester |
| **249** | 3-Benzyl-1-butyl-8-(5-tert-butyl-2-methyl-2H-pyrazol-3-carbonyl)-1,4,8-triaza-spiro[4.5]decan-2-on |
| **250** | 3-Benzyl-1-butyl-8-(2-chlor-4-trifluormethyl-pyrimidin-5-carbonyl)-1,4,8-triaza-spiro[4.5]decan-2-on |
| **251** | 3-Benzyl-8-(5-methyl-isoxazol-3-carbonyl)-1-prop-2-ynyl-1,4,8-triaza-spiro[4.5]decan-2-on |
| **252** | 4-[8-(2-Chlor-pyridin-3-carbonyl)-2-oxo-1,4,8-triaza-spiro[4.5]dec-1-ylmethyl]-benzoesäuremethylester |
| **253** | 8-(2-tert-Butyl-5-methyl-2H-pyrazol-3-carbonyl)-1-(2-fluor-benzyl)-1,4,8-triaza-spiro[4.5]decan-2-on |
| **254** | 4-[8-(2-Methylsulfanyl-pyridin-3-carbonyl)-2-oxo-1,4,8-triaza-spiro[4.5]dec-1-ylmethyl]-benzoesäuremethylester |
| **255** | Essigsäure 4-[8-(4-acetylamino-benzoyl)-3-isobutyl-2-oxo-1,4,8-triaza-spiro[4.5]dec-1-yl]-butylester |
| **256** | [8-(4-Acetylamino-benzoyl)-3-benzyl-2-oxo-1,4,8-triaza-spiro[4.5]dec-1-yl]-essigsäureethylester |
| **257** | Essigsäure 4-[8-(2-ethylsulfanyl-pyridin-3-carbonyl)-3-isobutyl-2-oxo-1,4,8-triaza-spiro[4.5]dec-1-yl]-butylester |
| **258** | 4-[8-(4-Methyl-[1,2,3]thiadiazol-5-carbonyl)-2-oxo-1,4,8-triaza-spiro[4.5]dec-1-ylmethyl]-benzoesäuremethylester |
| **259** | 4-[1-Allyl-3-(2-methylsulfanyl-ethyl)-2-oxo-1,4,8-triaza-spiro[4.5]decan-8-carbonyl]-benzolsulfonamid |
| **260** | 4-(8-Cyclobutancarbonyl-2-oxo-1,4,8-triaza-spiro[4.5]dec-1-ylmethyl)-benzoesäuremethylester |
| **261** | Essigsäure 2-[1-allyl-3-(2-methylsulfanyl-ethyl)-2-oxo-1,4,8-triaza-spiro[4.5]dec-8-yl]-2-oxo-ethylester |
| **262** | 8-(Biphenyl-4-carbonyl)-1-(2-fluor-benzyl)-1,4,8-triaza-spiro[4.5]decan-2-on |
| **263** | [3-(2-Methylsulfanyl-ethyl)-2-oxo-8-propionyl-1,4,8-triaza-spiro[4.5]dec-1-yl]-essigsäureethylester |
| **264** | 8-(Benzo[1,3]dioxole-5-carbonyl)-1-(2,6-dichlor-benzyl)-3-isobutyl-1,4,8-triaza-spiro[4.5]decan-2-on |
| **265** | 1-Allyl-8-(biphenyl-4-carbonyl)-3-isopropyl-1,4,8-triaza-spiro[4.5]decan-2-on |
| **266** | [3-Benzyl-8-(biphenyl-4-carbonyl)-2-oxo-1,4,8-triaza-spiro[4.5]dec-1-yl]-essigsäureethylester |
| **267** | [8-(3-Dimethylamino-benzoyl)-3-(2-methylsulfanyl-ethyl)-2-oxo-1,4,8-triaza-spiro[4.5]dec-1-yl]-essigsäureethylester |
| **268** | 3-(2-Oxo-8-pentanoyl-1,4,8-triaza-spiro[4.5]dec-1-ylmethyl)-benzonitril |
| **269** | 4-(8-Cyclopentancarbonyl-2-oxo-1,4,8-triaza-spiro[4.5]dec-1-ylmethyl)-benzoesäuremethylester |
| **270** | 4-[1-(2-Fluor-benzyl)-2-oxo-1,4,8-triaza-spiro[4.5]dec-8-yl]-4-oxo-butansäurethylester |
| **271** | 1-(2-Fluor-benzyl)-8-(3,4,5-trimethoxy-benzoyl)-1,4,8-triaza-spiro[4.5]decan-2-on |
| **272** | 4-[8-(2-Chlor-pyridin-4-carbonyl)-2-oxo-1,4,8-triaza-spiro[4.5]dec-1-ylmethyl]-benzoesäuremethylester |
| **273** | 3-[8-(3,5-Bis-trifluormethyl-benzoyl)-2-oxo-1,4,8-triaza-spiro[4.5]dec-1-ylmethyl]-benzonitril |
| **274** | 3-Benzyl-1-(2-fluor-benzyl)-8-(2-methoxy-benzoyl)-1,4,8-triaza-spiro[4.5]decan-2-on |
| **275** | 8-(Benzo[1,2,5]oxadiazol-5-carbonyl)-1-(2,6-dichlor-benzyl)-3-isobutyl-1,4,8-triaza-spiro[4.5]decan-2-on |
| **276** | [3-Benzyl-2-oxo-8-(4-sulfamoyl-benzoyl)-1,4,8-triaza-spiro[4.5]dec-1-yl]-essigsäureethylester |
| **277** | 3-[2-Oxo-8-(3,4,5-trimethoxy-benzoyl)-1,4,8-triaza-spiro[4.5]dec-1-ylmethyl]-benzonitril |
| **278** | Essigsäure 2-[1-(4-acetoxy-butyl)-3-isobutyl-2-oxo-1,4,8-triaza-spiro[4.5]dec-8-yl]-1,1-dimethyl-2-oxo-ethylester |
| **279** | 8-(6-Chlor-pyridin-3-carbonyl)-1-(2,6-dichlor-benzyl)-3-isobutyl-1,4,8-triaza-spiro[4.5]decan-2-on |
| **280** | 8-(2-Ethoxy-benzoyl)-1-(2-fluor-benzyl)-1,4,8-triaza-spiro[4.5]decan-2-on |

(fortgesetzt)

| Bsp. | Verbindung |
|---|---|
| 281 | 1-Allyl-8-cyclopropancarbonyl-3-(2-methylsulfanyl-ethyl)-1,4,8-triaza-spiro[4.5]decan-2-on |
| 282 | 3-[3-Isopropyl-8-(2-methoxy-acetyl)-2-oxo-1,4,8-triaza-spiro[4.5]dec-1-ylmethyl]-benzonitril |
| 283 | 4-(2-Oxo-8-phenylacetyl-1,4,8-triaza-spiro[4.5]dec-1-ylmethyl)-benzoesäuremethylester |
| 284 | Essigsäure 2-[1-(3-cyano-benzyl)-2-oxo-1,4,8-triaza-spiro[4.5]dec-8-yl]-2-oxo-1-phenyl-ethylester |
| 285 | 1-(2-Fluor-benzyl)-8-(4-methoxy-benzoyl)-1,4,8-triaza-spiro[4.5]decan-2-on |
| 286 | 4-(8-Cyclohexancarbonyl-2-oxo-1,4,8-triaza-spiro[4.5]dec-1-ylmethyl)-benzoesäuremethylester |
| 287 | 1-(2-Fluor-benzyl)-8-pent-4-enoyl-1,4,8-triaza-spiro[4.5]decan-2-on |
| 288 | [3-Isobutyl-8-(3-methyl-butyryl)-2-oxo-1,4,8-triaza-spiro[4.5]dec-1-yl]-essigsäureethylester |
| 289 | 1-Allyl-8-(3,3-dimethyl-butyryl)-3-(2-methylsulfanyl-ethyl)-1,4,8-triaza-spiro[4.5]decan-2-on |
| 290 | 1-Allyl-3-(2-methylsulfanyl-ethyl)-8-(2-methylsulfanyl-pyridin-3-carbonyl)-1,4,8-triaza-spiro[4.5]decan-2-on |
| 291 | [3-Isobutyl-8-(2-methyl-pentanoyl)-2-oxo-1,4,8-triaza-spiro[4.5]dec-1-yl]-essigsäureethylester |
| 292 | 4-[8-(5-tert-Butyl-2-methyl-furan-3-carbonyl)-2-oxo-1,4,8-triaza-spiro[4.5]dec-1-ylmethyl]-benzoesäuremethylester |
| 293 | (3-Benzyl-8-cyclopropancarbonyl-2-oxo-1,4,8-triaza-spiro[4.5]dec-1-yl)-essigsäureethylester |
| 294 | [3-Benzyl-8-(3-methyl-butyryl)-2-oxo-1,4,8-triaza-spiro[4.5]dec-1-yl]-essigsäureethylester |
| 295 | 1-(2,6-Dichlor-benzyl)-8-(2,5-dimethyl-furan-3-carbonyl)-3-isobutyl-1,4,8-triaza-spiro[4.5]decan-2-on |
| 296 | 1-Allyl-3-isopropyl-8-[2-(3-methoxy-phenyl)-acetyl]-1,4,8-triaza-spiro[4.5]decan-2-on |
| 297 | [8-(4-tert-Butyl-benzoyl)-3-(2-methylsulfanyl-ethyl)-2-oxo-1,4,8-triaza-spiro[4.5]dec-1-yl]-essigsäureethylester |
| 298 | 3-(2-Methylsulfanyl-ethyl)-1-(2-nitro-benzyl)-8-(2-phenoxy-pyridin-3-carbonyl)-1,4,8-triaza-spiro[4.5]decan-2-on |
| 299 | (3-Benzyl-2-oxo-8-pentanoyl-1,4,8-triaza-spiro[4.5]dec-1-yl)-essigsäureethylester |
| 300 | 8-(2-Chlor-pyridin-4-carbonyl)-1-(2-fluor-benzyl)-1,4,8-triaza-spiro(4.5]decan-2-on |
| 301 | [8-(3-Methyl-butyryl)-3-(2-methylsulfanyl-ethyl)-2-oxo-1,4,8-triaza-spiro[4.5]dec-1-yl]-essigsäureethylester |
| 302 | 1-Allyl-3-(2-methylsulfanyl-ethyl)-8-pentafluorbenzoyl-1,4,8-triaza-spiro[4.5]decan-2-on |
| 303 | 1-(2-Fluor-benzyl)-8-(2-trifluormethyl-benzoyl)-1,4,8-triaza-spiro[4.5]decan-2-on |
| 304 | 8-(Benzo[1,2,5]oxadiazol-5-carbonyl)-1-(2-fluor-benzyl)-1,4,8-triaza-spiro[4.5]decan-2-on |
| 305 | 4-[8-(4-tert-Butyl-benzoyl)-2-oxo-1,4,8-triaza-spiro[4.5]dec-1-ylmethyl]-benzoesäuremethylester |
| 306 | [3-Benzyl-2-oxo-8-(1-phenyl-5-propyl-1H-pyrazol-4-carbonyl)-1,4,8-triaza-spiro[4.5]dec-1-yl]-essigsäureethylester |
| 307 | 1-Allyl-3-isopropyl-8-pent-4-enoyl-1,4,8-triaza-spiro[4.5]decan-2-on |
| 308 | 3-[2-Oxo-8-(2-trifluormethyl-benzoyl)-1,4,8-triaza-spiro[4.5]dec-1-ylmethyl]-benzonitril |
| 309 | 8-(2-Dimethylamino-acetyl)-1-(3,5-dimethyl-benzyl)-3-isobutyl-1,4,8-triaza-spiro[4.5]decan-2-on |
| 310 | Essigsäure 4-[8-(2-ethoxy-benzoyl)-3-isobutyl-2-oxo-1,4,8-triaza-spiro[4.5]dec-1-yl]-butylester |
| 311 | 1-(2-Fluor-benzyl)-8-(3-methoxy-benzoyl)-1,4,8-triaza-spiro[4.5]decan-2-on |
| 312 | [8-(Furan-2-carbonyl)-3-isobutyl-2-oxo-1,4,8-triaza-spiro[4.5]dec-1-yl]-essigsäureethylester |
| 313 | 8-(5-tert-Butyl-2-methyl-2H-pyrazol-3-carbonyl)-1-(2-fluor-benzyl)-1,4,8-triaza-spiro[4.5]decan-2-on |
| 314 | 1-(2-Fluor-benzyl)-8-(thiophen-2-carbonyl)-1,4,8-triaza-spiro[4.5]decan-2-on |
| 315 | [3-Benzyl-8-(2-fluor-benzoyl)-2-oxo-1,4,8-triaza-spiro[4.5]dec-1-yl]-essigsäureethylester |

(fortgesetzt)

| Bsp. | Verbindung |
|---|---|
| 316 | [8-(Isoxazol-5-carbonyl)-3-(2-methylsulfanyl-ethyl)-2-oxo-1,4,8-triaza-spiro[4.5]dec-1-yl]-essigsäureethylester |
| 317 | 1-(2,6-Dichlor-benzyl)-3-isobutyl-8-(4-propyl-benzoyl)-1,4,8-triaza-spiro[4.5]decan-2-on |
| 318 | [8-[2-(2,5-Dimethoxy-phenyl)-acetyl]-3-(2-methylsulfanyl-ethyl)-2-oxo-1,4,8-triaza-spiro[4.5]dec-1-yl]-essigsäureethylester |
| 319 | (3-Benzyl-8-cyclobutancarbonyl-2-oxo-1,4,8-triaza-spiro[4.5]dec-1-yl)-essigsäureethylester |
| 320 | 1-(2-Fluor-benzyl)-8-[2-(4-methoxy-phenyl)-acetyl]-1,4,8-triaza-spiro[4.5]decan-2-on |
| 321 | 1-Allyl-3-(2-methylsulfanyl-ethyl)-8-(2-methyl-6-trifluormethyl-pyridin-3-carbonyl)-1,4,8-triaza-spiro[4.5]decan-2-on |
| 322 | [3-(2-Methylsulfanyl-ethyl)-2-oxo-8-(4-trifluormethyl-benzoyl)-1,4,8-triaza-spiro[4.5]dec-1-yl]-essigsäureethylester |
| 323 | 1-Allyl-3-(2-methylsulfanyl-ethyl)-8-(1-phenyl-5-propyl-1H-pyrazol-4-carbonyl)-1,4,8-triaza-spiro[4.5]decan-2-on |
| 324 | 1-(2,6-Dichlor-benzyl)-8-(2,3-difluor-4-methyl-benzoyl)-3-isobutyl-1,4,8-triaza-spiro[4.5]decan-2-on |
| 325 | 1-Allyl-3-isopropyl-8-(2-methyl-benzoyl)-1,4,8-triaza-spiro[4.5]decan-2-on |
| 326 | 8-(2-Chlor-5-trifluormethyl-benzoyl)-1-(2,6-dichlor-benzyl)-3-isobutyl-1,4,8-triaza-spiro[4.5]decan-2-on |
| 327 | 1-Allyl-8-[2-(3-methoxy-phenyl)-acetyl]-3-(2-methylsulfanyl-ethyl)-1,4,8-triaza-spiro[4.5]decan-2-on |
| 328 | 1-Allyl-3-(2-methylsulfanyl-ethyl)-8-(3-phenyl-propionyl)-1,4,8-triaza-spiro[4.5]decan-2-on |
| 329 | [3-Benzyl-8-(2-benzyloxy-acetyl)-2-oxo-1,4,8-triaza-spiro[4.5]dec-1-yl]-essigsäureethylester |
| 330 | 1-Allyl-8-(5-tert-butyl-2-methyl-2H-pyrazol-3-carbonyl)-3-(2-methylsulfanyl-ethyl)-1,4,8-triaza-spiro[4.5]decan-2-on |
| 331 | Essigsäure 1,1-dimethyl-2-[3-(2-methylsulfanyl-ethyl)-1-(2-nitro-benzyl)-2-oxo-1,4,8-triaza-spiro[4.5]dec-8-yl]-2-oxo-ethylester |
| 332 | Benzoesäure 2-[1-ethoxycarbonylmethyl-3-(2-methylsulfanyl-ethyl)-2-oxo-1,4,8-triaza-spiro[4.5]decan-8-carbonyl]-benzylester |
| 333 | 1-Allyl-3-isopropyl-8-(4-methyl-[1,2,3]thiadiazol-5-carbonyl)-1,4,8-triaza-spiro[4.5]decan-2-on |
| 334 | 1-Allyl-8-(2, 5-dimethyl-furan-3-carbonyl)-3-(2-methylsulfanyl-ethyl)-1,4,8-triaza-spiro[4.5]decan-2-on |
| 335 | 1-Allyl-8-(benzo[1,2,5]oxadiazol-5-carbonyl)-3-(2-methylsulfanyl-ethyl)-1,4,8-triaza-spiro[4.5]decan-2-on |
| 336 | 1-(2-Fluor-benzyl)-8-(4-phenoxy-butyryl)-1,4,8-triaza-spiro[4.5]decan-2-on |
| 337 | 1-Allyl-8-(2-cyclopentyl-acetyl)-3-(2-methylsulfanyl-ethyl)-1,4,8-triaza-spiro[4.5]decan-2-on |
| 338 | [3-Benzyl-8-(naphthalin-2-carbonyl)-2-oxo-1,4,8-triaza-spiro[4.5]dec-1-yl]-essigsäureethylester |
| 339 | 8-[3-(2-Chlor-6-fluor-phenyl)-5-methyl-isoxazol-4-carbonyl]-1-(2-fluor-benzyl)-1,4,8-triaza-spiro[4.5]decan-2-on |
| 340 | 3-[8-(3,5-Dimethoxy-benzoyl)-3-isopropyl-2-oxo-1,4,8-triaza-spiro[4.5]dec-1-ylmethyl]-benzonitril |
| 341 | 3-(2-Methylsulfanyl-ethyl)-1-(2-nitro-benzyl)-8-(1-phenyl-5-propyl-1H-pyrazol-4-carbonyl)-1,4,8-triaza-spiro[4.5]decan-2-on |
| 342 | {3-Benzyl-8-[3-(2-chlor-6-fluor-phenyl)-5-methyl-isoxazol-4-carbonyl]-2-oxo-1,4,8-triaza-spiro[4.5]dec-1-yl}-essigsäureethylester |
| 343 | 1-(2-Fluor-benzyl)-8-(3-fluor-4-trifluormethyl-benzoyl)-1,4,8-triaza-spiro[4.5]decan-2-on |
| 344 | [8-(1-(4-Chlor-phenyl)-5-trifluormethyl-1H-pyrazol-4-carbonyl]-3-(2-methylsulfanyl-ethyl)-2-oxo-1,4,8-triaza-spiro[4.5]dec-1-yl]-essigsäureethylester |

(fortgesetzt)

| Bsp. | Verbindung |
|---|---|
| 345 | 3-[8-(Naphthalin-1-carbonyl)-2-oxo-1,4,8-triaza-spiro[4.5]dec-1-yimethyl]-benzonitril |
| 346 | [8-(3,3-Dimethyl-butyryl)-3-(2-methylsulfanyl-ethyl)-2-oxo-1,4,8-triaza-spiro[4.5]dec-1-yl]-essigsäureethylester |
| 347 | 8-Acetyl-3-(2-methylsulfanyl-ethyl)-1-naphthalin-2-ylmethyl-1,4,8-triaza-spiro[4.5]decan-2-on |
| 348 | [3-Benzyl-8-(3-cyano-benzoyl)-2-oxo-1,4,8-triaza-spiro[4.5]dec-1-yl]-essigsäureethylester |
| 349 | 8-(5-tert-Butyl-2-methyl-2H-pyrazol-3-carbonyl)-1-(2,6-dichlor-benzyl)-3-isobutyl-1,4,8-triaza-spiro[4.5] decan-2-on |
| 350 | 4-[3-Benzyl-1-(2-fluor-benzyl)-2-oxo-1,4,8-triaza-spiro[4.5]decan-8-carbonyl]-benzonitril |
| 351 | 4-[3-Benzyl-1-(2-fluor-benzyl)-2-oxo-1,4,8-triaza-spiro[4.5]dec-8-yl]-4-oxo-butyric acid methylester |
| 352 | 3-(2-Methylsulfanyl-ethyl)-1-naphthalin-2-ylmethyl-8-(3,4,5-trimethoxy-benzoyl)-1,4,8-triaza-spiro[4.5] decan-2-on |
| 353 | [3-Benzyl-8-(isoxazol-5-carbonyl)-2-oxo-1,4,8-triaza-spiro[4.5]dec-1-yl]-essigsäureethylester |
| 354 | 8-(3-Difluormethylsulfanyl-benzoyl)-1-(2-fluor-benzyl)-1,4,8-triaza-spiro[4.5]decan-2-on |
| 355 | [3-Benzyl-8-(2,3-dimethyl-benzoyl)-2-oxo-1,4,8-triaza-spiro[4.5]dec-1-yl]-essigsäureethylester |
| 356 | 1-(2-Fluor-benzyl)-8-(4-propyl-benzoyl)-1,4,8-triaza-spiro[4.5]decan-2-on |
| 357 | 3-[8-(4-Iod-benzoyl)-2-oxo-1,4,8-triaza-spiro[4.5]dec-1-ylmethyl]-benzonitril |
| 358 | 1-(2-Fluor-benzyl)-8-(2-propyl-pentanoyl)-1,4,8-triaza-spiro[4.5]decan-2-on |
| 359 | 3-[8-(2,6-Difluor-3-methyl-benzoyl)-3-isopropyl-2-oxo-1,4,8-triaza-spiro[4.5]dec-1-ylmethyl]-benzonitril |
| 360 | 3-(2-Methylsulfanyl-ethyl)-8-(4-methyl-[1,2,3]thiadiazol-5-carbonyl)-1-(2-nitro-benzyl)-1,4,8-triaza-spiro [4.5]decan-2-on |
| 361 | Essigsäure 4-[3-isobutyl-8-(4-methyl-[1,2,3]thiadiazol-5-carbonyl)-2-oxo-1,4,8-triaza-spiro[4.5]dec-1-yl]-butylester |
| 362 | 8-[2-(4-Chlor-phenoxy)-acetyl]-3-(2-methylsulfanyl-ethyl)-1-naphthalin-2-ylmethyl-1,4,8-triaza-spiro[4.5] decan-2-on |
| 363 | [3-Benzyl-2-oxo-8-(3-trifluormethyl-benzoyl)-1,4,8-triaza-spiro[4.5]dec-1-yl]-essigsäureethylester |
| 364 | 8-(4-Brom-benzoyl)-3-(2-methylsulfanyl-ethyl)-1-naphthalin-2-ylmethyl-1,4,8-triaza-spiro[4.5]decan-2-on |
| 365 | [8-(2-Chlor-pyridin-4-carbonyl)-3-(2-methylsulfanyl-ethyl)-2-oxo-1,4,8-triaza-spiro[4.5]dec-1-yl]-essigsäureethylester |
| 366 | 1-Allyl-8-(3,5-dimethoxy-benzoyl)-3-(2-methylsulfanyl-ethyl)-1,4,8-triaza-spiro[4.5]decan-2-on |
| 367 | 3-[8-(4-Methyl-3-nitro-benzoyl)-2-oxo-1,4,8-triaza-spiro[4.5]dec-1-ylmethyl]-benzonitril |
| 368 | 8-(4-tert-Butyl-benzoyl)-1-(2-fluor-benzyl)-1,4,8-triaza-spiro[4.5]decan-2-on |
| 369 | 1-Allyl-3-isopropyl-8-(5-methyl-isoxazol-3-carbonyl)-1,4,8-triaza-spiro[4.5]decan-2-on |
| 370 | 1-Allyl-3-(2-methylsulfanyl-ethyl)-8-(2-trifluormethyl-benzoyl)-1,4,8-triaza-spiro[4.5]decan-2-on |
| 371 | [3-Benzyl-8-(3-cyclopentyl-propionyl)-2-oxo-1,4,8-triaza-spiro[4.5]dec-1-yl]-essigsäureethylester |
| 372 | 3-Benzyl-8-(3,5-difluor-benzoyl)-1-(2-fluor-benzyl)-1,4,8-triaza-spiro[4.5]decan-2-on |
| 373 | [3-Benzyl-8-(5-fluor-2-trifluormethyl-benzoyl)-2-oxo-1,4,8-triaza-spiro[4.5]dec-1-yl]-essigsäureethylester |
| 374 | [3-Benzyl-2-oxo-8-(3,4,5-trimethoxy-benzoyl)-1,4,8-triaza-spiro[4.5]dec-1-yl]-essigsäureethylester |
| 375 | Benzoesäure 2-[1-(2-fluor-benzyl)-2-oxo-1,4,8-triaza-spiro[4.5]decan-8-carbonyl]-benzylester |
| 376 | 1-(2-Fluor-benzyl)-8-(2-methyl-benzoyl)-1,4,8-triaza-spiro[4.5]decan-2-on |
| 377 | 1-(2-Fluor-benzyl)-8-(2-phenyl-butyryl)-1,4,8-triaza-spiro[4.5]decan-2-on |

(fortgesetzt)

| Bsp. | Verbindung |
|---|---|
| 378 | 1-Allyl-8-(6-chlor-pyridin-3-carbonyl)-3-isopropyl-1,4,8-triaza-spiro[4.5]decan-2-on |
| 379 | [8-(2,5-Dimethyl-furan-3-carbonyl)-3-(2-methylsulfanyl-ethyl)-2-oxo-1,4,8-triaza-spiro[4.5]dec-1-yl]-essigsäureethylester |
| 380 | [8-(5-tert-Butyl-2-methyl-2H-pyrazol-3-carbonyl)-3-(2-methylsulfanyl-ethyl)-2-oxo-1,4,8-triaza-spiro[4.5] dec-1-yl]-essigsäureethylester |
| 381 | Benzoesäure 2-[1-allyl-3-(2-methylsulfanyl-ethyl)-2-oxo-1,4,8-triaza-spiro[4.5]decan-8-carbonyl]-benzylester |
| 382 | Essigsäure 2-[1-allyl-3-(2-methylsulfanyl-ethyl)-2-oxo-1,4,8-triaza-spiro[4.5]dec-8-yl]-2-oxo-1-phenyl-ethylester |
| 383 | 3-[8-(2-Chlor-6-fluor-benzoyl)-3-isopropyl-2-oxo-1,4,8-triaza-spiro[4.5]dec-1-ylmethyl]-benzonitril |
| 384 | 3-(2-Methylsulfanyl-ethyl)-1-(2-nitro-benzyl)-8-(3-phenyl-propionyl)-1,4,8-triaza-spiro[4.5]decan-2-on |
| 385 | 3-[8-(2,3-Dimethyl-benzoyl)-3-isopropyl-2-oxo-1,4,8-triaza-spiro[4.5]dec-1-ylmethyl]-benzonitril |
| 386 | [8-(5-Fluor-2-trifluormethyl-benzoyl)-3-(2-methylsulfanyl-ethyl)-2-oxo-1,4,8-triaza-spiro[4.5]dec-1-yl]-essigsäureethylester |
| 387 | [8-Cyclopentancarbonyl-3-(2-methylsulfanyl-ethyl)-2-oxo-1,4,8-triaza-spiro[4.5]dec-1-yl]-essigsäureethylester |
| 388 | [3-(2-Methylsulfanyl-ethyl)-2-oxo-8-(thiophen-2-carbonyl)-1,4,8-triaza-spiro[4.5]dec-1-yl]-essigsäureethylester |
| 389 | 1-Allyl-8-[1-(4-chlor-phenyl)-5-trifluormethyl-1H-pyrazol-4-carbonyl]-3-(2-methylsulfanyl-ethyl)-1,4,8-triaza-spiro[4.5]decan-2-on |
| 390 | 1-Allyl-3-(2-methylsulfanyl-ethyl)-8-(naphthalin-1-carbonyl)-1,4,8-triaza-spiro[4.5]decan-2-on |
| 391 | 1-(2-fluor-benzyl)-8-(4-trifluormethoxy-benzoyl)-1,4,8-triaza-spiro[4.5]decan-2-on |
| 392 | 3-Benzyl-8-(2-benzyloxy-acetyl)-1-(2-fluor-benzyl)-1,4,8-triaza-spiro[4.5]decan-2-on |
| 393 | 3-[8-(2-chlor-6-fluor-3-methyl-benzoyl)-3-isopropyl-2-oxo-1,4,8-triaza-spiro[4.5]dec-1-ylmethyl]-benzonitril |
| 394 | 1-Aliyl-8-(2-chlor-5-trifluormethyl-benzoyl)-3-isopropyl-1,4,8-triaza-spiro[4.5]decan-2-on |
| 395 | 8-(3-Methyl-benzoyl)-3-(2-methylsulfanyl-ethyl)-1-naphthalin-2-ylmethyl-1,4,8-triaza-spiro[4.5]decan-2-on |
| 396 | 1-(3,5-Dimethyl-benzyl)-8-(2-ethyl-butyryl)-3-isobutyl-1,4,8-triaza-spiro[4.5]decan-2-on |
| 397 | 8-(5-tert-Butyl-2-methyl-2H-pyrazol-3-carbonyl)-3-(2-methylsulfanyl-ethyl)-1-(2-nitro-benzyl)-1,4,8-triaza-spiro[4.5]decan-2-on |
| 398 | Benzoesäure 2-[1-(3-cyano-benzyl)-3-isopropyl-2-oxo-1,4,8-triaza-spiro[4.5]decan-8-carbonyl]-benzylester |
| 399 | 8-(4-Methyl-3-nitro-benzoyl)-3-(2-methylsulfanyl-ethyl)-1-(2-nitro-benzyl)-1,4,8-triaza-spiro[4.5]decan-2-on |
| 400 | 1-(2,6-Dichlor-benzyl)-3-isobutyl-8-(3,4,5-trimethoxy-benzoyl)-1,4,8-triaza-spiro[4.5]decan-2-on |
| 401 | 3-{8-[2-(2-Brom-phenyl)-acetyl]-3-isopropyl-2-oxo-1,4,8-triaza-spiro[4.5]dec-1-ylmethyl}-benzonitril |
| 402 | 3-[8-(2,3-Dichlor-benzoyl)-3-isopropyl-2-oxo-1,4,8-triaza-spiro[4.5]dec-1-ylmethyl]-benzonitril |
| 403 | 1-Allyl-8-(6-chlor-2H-chroman-3-carbonyl)-3-isopropyl-1,4,8-triaza-spiro[4.5]decan-2-on |
| 404 | {3-Benzyl-8-[2-(4-methoxy-phenyl)-acetyl]-2-oxo-1,4,8-triaza-spiro[4.5]dec-1-yl}-essigsäureethylester |
| 405 | 3-[3-Isopropyl-2-oxo-8-(1-phenyl-5-propyl-1H-pyrazol-4-carbonyl)-1,4,8-triaza-spiro[4.5]dec-1-ylmethyl]-benzonitril |
| 406 | 8-(3-Cyclopentyl-propionyl)-1-(2-fluor-benzyl)-1,4,8-triaza-spiro[4.5]decan-2-on |
| 407 | 1-Allyl-8-(3-difluormethylsulfanyl-benzoyl)-3-isopropyl-1,4,8-triaza-spiro[4.5]decan-2-on |
| 408 | 3-[8-(2-chlor-4-nitro-benzoyl)-3-isopropyl-2-oxo-1,4,8-triaza-spiro[4.5]dec-1-ylmethyl]-benzonitril |

(fortgesetzt)

| Bsp. | Verbindung |
|---|---|
| 409 | [8-(2-Ethoxy-benzoyl)-3-(2-methylsulfanyl-ethyl)-2-oxo-1,4,8-triaza-spiro[4.5]dec-1-yl]-essigsäureethylester |
| 410 | 8-(2,5-Bis-trifluormethyl-benzoyl)-1-(2-fluor-benzyl)-1,4,8-triaza-spiro[4.5]decan-2-on |
| 411 | 8-(2-Chlor-pyridin-4-carbonyl)-3-(2-methylsulfanyl-ethyl)-1-(2-nitro-benzyl)-1,4,8-triaza-spiro[4.5]decan-2-on |
| 412 | {3-Benzyi-8-[3-(2,6-dichlor-phenyl)-5-methyl-isoxazol-4-carbonyl]-2-oxo-1,4,8-triaza-spiro[4.5]dec-1-yl}-essigsäureethylester |
| 413 | 1-(3,5-Dimethyl-benzyl)-3-isobutyl-8-(2-trifluormethyl-benzoyl)-1,4,8-triaza-spiro[4.5]decan-2-on |
| 414 | 8-(3,5-Dimethoxy-benzoyl)-3-(2-methylsuifanyl-ethyl)-1-(2-nitro-benzyl)-1,4,8-triaza-spiro[4.5]decan-2-on |
| 415 | Benzoesäure 2-[3-benzyl-1-(2-fluor-benzyl)-2-oxo-1,4,8-triaza-spiro[4.5]decan-8-carbonyl]-benzylester |
| 416 | 3-[8-(Benzo[1,2,5]oxadiazol-5-carbonyl)-3-isopropyl-2-oxo-1,4,8-triaza-spiro[4.5]dec-1-ylmethyl]-benzonitril |
| 417 | [3-(2-Methylsulfanyl-ethyl)-2-oxo-8-(4-trifluormethoxy-benzoyl)-1,4,8-triaza-spiro[4.5]dec-1-yl]-essigsäureethylester |
| 418 | 1-Allyl-3-isopropyl-8-[2-(4-methoxy-phenyl)-acetyl]-1,4,8-triaza-spiro[4.5]decan-2-on |
| 419 | 3-(2-Methylsulfanyl-ethyl)-1-(2-nitro-benzyl)-8-(4-propyl-benzoyl)-1,4,8-triaza-spiro[4.5]decan-2-on |
| 420 | 3-Benzyl-8-(4-tert-butyl-benzoyl)-1-(2-fluor-benzyl)-1,4,8-triaza-spiro[4.5]decan-2-on |
| 421 | 1-Allyl-8-(2,6-difluor-3-methyl-benzoyl)-3-(2-methylsulfanyl-ethyl)-1,4,8-triaza-spiro[4.5]decan-2-on |
| 422 | 8-(3,5-Bis-trifluormethyl-benzoyl)-1-(2-fluor-benzyl)-1,4,8-triaza-spiro[4.5]decan-2-on |
| 423 | 3-[8-(4-Iod-benzoyl)-3-isopropyl-2-oxo-1,4,8-triaza-spiro[4.5]dec-1-ylmethyl]-benzonitril |
| 424 | 3-(2-Methylsulfanyl-ethyl)-1-naphthalin-2-ylmethyl-8-(thiophen-2-carbonyl)-1,4,8-triaza-spiro[4.5]decan-2-on |
| 425 | [3-Benzyl-8-(2-chlor-pyridin-4-carbonyl)-2-oxo-1,4,8-triaza-spiro[4.5]dec-1-yl]-essigsäureethylester |
| 426 | [3-(2-Methylsulfanyl-ethyl)-8-(4-nitro-benzoyl)-2-oxo-1,4,8-triaza-spiro[4.5]dec-1-yl]-essigsäureethylester |
| 427 | 8-(2-Ethyl-hexanoyl)-3-(2-methylsulfanyl-ethyl)-1-(2-nitro-benzyl)-1,4,8-triaza-spiro[4.5]decan-2-on |
| 428 | 1-(2,6-Dichlor-benzyl)-3-isobutyl-8-(thiophen-2-carbonyl)-1,4,8-triaza-spiro[4.5]decan-2-on |
| 429 | [3-Benzyl-8-(2-chlor-4-nitro-benzoyl)-2-oxo-1,4,8-triaza-spiro[4.5]dec-1-yl]-essigsäureethylester |
| 430 | 3-Benzyl-8-(3,5-bis-trifluormethyl-benzoyl)-1-(2-fluor-benzyl)-1,4,8-triaza-spiro[4.5]decan-2-on |
| 431 | [3-Benzyl-8-(4-brom-3-methyl-benzoyl)-2-oxo-1,4,8-triaza-spiro[4.5]dec-1-yl]-essigsäureethylester |
| 432 | 8-Cyclohexancarbonyl-3-(2-methylsulfanyl-ethyl)-1-(2-nitro-benzyl)-1,4,8-triaza-spiro[4.5]decan-2-on |
| 433 | 8-(2,5-Dimethyl-furan-3-carbonyl)-3-(2-methylsulfanyl-ethyl)-1-(2-nitro-benzyl)-1,4,8-triaza-spiro[4.5] decan-2-on |
| 434 | 8-(3-Difluormethylsulfanyl-benzoyl)-3-(2-methylsulfanyl-ethyl)-1-(2-nitro-benzyl)-1,4,8-triaza-spiro[4.5] decan-2-on |
| 435 | 8-(Furan-2-carbonyl)-3-(2-methylsulfanyl-ethyl)-1-(2-nitro-benzyl)-1,4,8-triaza-spiro[4.5]decan-2-on |
| 436 | [3-Benzyl-8-(2,3-difluor-benzoyl)-2-oxo-1,4,8-triaza-spiro[4.5]dec-1-yl]-essigsäureethylester |
| 438 | 3-[3-Isopropyl-8-(4-methyl-[1,2,3]thiadiazol-5-carbonyl)-2-oxo-1,4,8-triaza-spiro[4.5]dec-1-ylmethyl]-benzonitril |
| 439 | 3-[8-(3-Chlor-2-fluor-benzoyl)-3-isopropyl-2-oxo-1,4,8-triaza-spiro[4.5]dec-1-ylmethyl]-benzonitril |
| 440 | [3-Benzyl-8-(naphthalin-1-carbonyl)-2-oxo-1,4,8-triaza-spiro[4.5]dec-1-yl]-essigsäureethylester |

(fortgesetzt)

| Bsp. | Verbindung |
|---|---|
| **441** | 8-(4-tert-Butyl-benzoyl)-3-(2-methylsulfanyl-ethyl)-1-(2-nitro-benzyl)-1,4,8-triaza-spiro[4.5]decan-2-on |
| **442** | 3-[3-Benzyl-1-(2-fluor-benzyl)-2-oxo-1,4,8-triaza-spiro[4.5]decan-8-carbonyl]-benzonitril |
| **443** | 1-(2,6-Dichlor-benzyl)-8-(furan-2-carbonyl)-3-isobutyl-1,4,8-triaza-spiro[4.5]decan-2-on |
| **444** | 8-(2,6-Dichlor-benzoyl)-1-(2-fluor-benzyl)-1,4,8-triaza-spiro[4.5]decan-2-on |
| **445** | 1-(3,5-Dimethyl-benzyl)-8-(3-fluor-4-trifluormethyl-benzoyl)-3-isobutyl-1,4,8-triaza-spiro[4.5]decan-2-on |
| **446** | 1-(2-Fluor-benzyl)-8-(3-fluor-4-methyl-benzoyl)-1,4,8-triaza-spiro[4.5]decan-2-on |
| **447** | 1-Allyl-8-(3-cyclopentyl-propionyl)-3-(2-methylsulfanyl-ethyl)-1,4,8-triaza-spiro[4.5]decan-2-on |
| **448** | 8-[2-(3-Chlor-phenoxy)-acetyl]-3-(2-methylsulfanyl-ethyl)-1-(2-nitro-benzyl)-1,4, 8-triaza-spiro[4.5]decan-2-on |
| **449** | 1-(3,5-Dimethyl-benzyl)-3-isobutyl-8-(2-methylsulfanyl-pyridin-3-carbonyl)-1,4,8-triaza-spiro[4.5]decan-2-on |
| **450** | 3-(2-Methylsulfanyl-ethyl)-1-(2-nitro-benzyl)-8-propionyl-1,4,8-triaza-spiro[4.5]decan-2-on |
| **451** | 3-[8-(3,4-Dimethoxy-benzoyl)-3-isopropyl-2-oxo-1,4,8-triaza-spiro[4.5]dec-1-ylmethyl]-benzonitril |
| **452** | 3-(2-Methylsulfanyl-ethyl)-8-(naphthalin-2-carbonyl)-1-naphthalin-2-ylmethyl-1,4,8-triaza-spiro[4.5]decan-2-on |
| **453** | 8-(6-Chlor-2H-chroman-3-carbonyl)-3-(2-methylsulfanyl-ethyl)-1-(2-nitro-benzyl)-1,4,8-triaza-spiro[4.5] decan-2-on |
| **454** | {3-Benzyl-2-oxo-8-[3-(3-trifluormethyl-phenyl)-acryloyl]-l,4,8-triaza-spiro[4.5]dec-1-yl}-essigsäureethylester |
| **455** | 1-(3,5-Dimethyl-benzyl)-3-isobutyl-8-(3-phenyl-acryloyl)-1,4,8-triaza-spiro[4.5]decan-2-on |
| **456** | 3-[3-Isopropyl-2-oxo-8-(2-phenoxy-propionyl)-1,4,8-triaza-spiro[4.5]dec-1-ylmethyl]-benzonitril |
| **457** | 3-[3-Isopropyl-2-oxo-8-(4-trifluormethoxy-benzoyl)-1,4,8-triaza-spiro[4.5]dec-1-ylmethyl]-benzonitril |
| **458** | 1-Allyl-3-(2-methylsulfanyl-ethyl)-8-(3-nitro-benzoyl)-1,4,8-triaza-spiro[4.5]decan-2-on |
| **459** | [3-Benzyl-8-(3,4-dichlor-benzoyl)-2-oxo-1,4,8-triaza-spiro[4.5]dec-1-yl]-essigsäureethylester |
| **460** | 1-(2-Fluor-benzyl)-8-[3-(3-trifluormethyl-phenyl)-acryloyl]-1,4,8-triaza-spiro[4.5]decan-2-on |
| **461** | 1-Allyl-8-(3,5-bis-trifluormethyl-benzoyl)-3-(2-methylsulfanyl-ethyl)-1,4,8-triaza-spiro[4.5]decan-2-on |
| **462** | 8-(3-Cyclopentyl-propionyl)-3-(2-methylsulfanyl-ethyl)-1-(2-nitro-benzyl)-1,4,8-triaza-spiro[4.5]decan-2-on |
| **465** | 1-(3,5-Dimethyl-benzyl)-3-isobutyl-8-(4-nitro-benzoyl)-1,4,8-triaza-spiro[4.5]decan-2-on |
| **466** | 8-(3-Cyclopentyl-propionyl)-1-(3,5-dimethyl-benzyl)-3-isobutyl-1,4,8-triaza-spiro[4.5]decan-2-on |
| **467** | 3-[3-Isopropyl-8-(isoxazol-5-carbonyl)-2-oxo-1,4,8-triaza-spiro[4.5]dec-1-ylmethyl]-benzonitril |
| **468** | 3-Benzyl-1-(2-fluor-benzyl)-8-(2-methyl-6-trifluormethyl-pyridin-3-carbonyl)-1,4,8-triaza-spiro[4.5]decan-2-on |

**Pharmakologische Daten:**

Die Affinität der erfindungsgemäßen substituierten 1,4,8-Triazaspiro[4.5]decan-2-on-Verbindungen der allgemeinen Formel I zum humanen μ-Opioidrezeptor, deren Affinität zur Batrachotoxin-(BTX)-Bindungsstelle des Natriumkanals sowie die Hemmung der Noradrenalin- bzw. 5-HT-Wiederaufnahme, wurde wie vorstehend beschrieben bestimmt. Des weiteren wurde - ebenfalls wie vorstehend beschrieben - die analgetische Wirksamkeit der erfindungsgemäßen substituierten 1,4,8-Triazaspiro[4.5]decan-2-on-Verbindungen der allgemeinen Formel I im Writhing-Test an der Maus bestimmt.

Die erfindungsgemäßen substituierten 1,4,8-Triazaspiro[4.5]decan-2-on-Verbindungen der vorstehend angegebenen allgemeinen Formel I zeigen eine gute bis sehr gute Inhibierung der Noradrenalin-Wiederaufnahme sowie eine gute bis sehr gute Inhibierung der 5-Hydroxy-Tryptophan-Wiederaufnahme. Im Writhing-Test an der Maus zeigen die

erfindungsgemäßen substituierten 1,4,8-Triazaspiro[4.5]decan-2-on-Verbindungen der allgemeinen Formel I eine ausgeprägte analgetische Wirksamkeit.

Darüber hinaus zeigen diese erfindungsgemäßen Verbindungen auch ausgezeichnete Affinitäten für Opioid-Rezeptoren, insbesondere für den μ-Opioid-Rezeptor und/oder für die Batrachotoxin-(BTX)-Bindungsstelle des Natriumkanals.

In den nachfolgenden Tabellen I und II sind die jeweiligen pharmakologischen Daten für die beispielgemäßen substituierten 1,4,8-Triazaspiro[4.5]decan-2-on-Verbindungen wiedergegeben.

**Tabelle I:**

| **Beispiel** | **%Hemmung der Writhingreaktion** |
|---|---|
| Nr. | (Dosierung in mg/kg intravenös) |
| 25 | 42 (10) |
| 31 | 36 (10) |
| 55 | 45 (10) |
| 53 | 30 (10) |
| 2 | 51 (10) |

**Tabelle II:**

| **Beispiel** | BTX Inhibition | μ (Naloxon), 1μM, % Hemmung | Uptake 5-HT, Ratte, 1μM,% Hemmung |
|---|---|---|---|
| **25** | 47 | 66 | 66 |
| **29** | 60 | 38 | 45 |
| **31** | 61 | | 34 |
| **30** | 35 | | 42 |
| **29** | 65 | | 80 |
| **28** | 47 | | |
| **27** | 79 | | 78 |
| **38** | 87 | | 94 |
| **33** | | | 49 |
| **10** | 53 | 94 | |
| **62** | 48 | | 87 |
| **61** | 41 | | |
| **60** | 39 | | 80 |
| **59** | 49 | | 53 |
| **58** | 46 | | 46 |
| **57** | 44 | 60 | 72 |
| **56** | 67 | | |
| **55** | 64 | | |
| **54** | 37 | | 81 |
| **53** | 48 | | |

**Tabelle III:**

| Beispiel | BTX Inhibierung | Uptake_5-HT, Ratte, 1μM, % Hemmung | Uptake (NA), Ratte, 10μM,% Hemmung |
|---|---|---|---|
| **67** | | 46 | |
| **68** | | 75 | |
| **71** | | 53 | 52 |
| **72** | 39 | | |
| **74** | | 47 | |
| **78** | | 56 | 33 |
| **80** | | | 30 |
| **81** | | 43 | |
| **84** | | 64 | |
| **86** | | 50 | 38 |
| **87** | | 56 | |
| **88** | | 34 | 32 |
| **89** | | 43 | 41 |
| **90** | | 41 | 35 |
| **92** | | 30 | 30 |
| **94** | | | 38 |
| **95** | | | 31 |
| **97** | | 67 | 30 |
| **98** | | 57 | 36 |
| **99** | 66 | 79 | 42 |
| **100** | | 42 | 32 |
| **101** | 78 | 33 | |
| **103** | | 34 | 30 |
| **104** | 41 | 48 | 36 |
| **105** | 66 | 68 | |
| **108** | | | 37 |
| **109** | | 50 | 32 |
| **110** | | 33 | |
| **111** | 42 | 58 | 38 |
| **112** | | 43 | 42 |
| **113** | | 35 | 46 |
| **114** | | 53 | |
| **115** | | 30 | 34 |
| **116** | | 37 | 34 |
| **118** | 44 | 38 | 30 |
| **120** | | | 32 |
| **121** | | 41 | 37 |
| **122** | | 34 | 38 |

(fortgesetzt)

| Beispiel | BTX Inhibierung | Uptake_5-HT, Ratte, 1µM, % Hemmung | Uptake (NA), Ratte, 10µM,% Hemmung |
|---|---|---|---|
| **123** | | | 36 |
| **125** | | 44 | 36 |
| **126** | 34 | | 44 |
| **129** | 30 | 47 | |
| **130** | | 32 | |
| **131** | 37 | | |
| **132** | 54 | | |
| **133** | 35 | | |
| **135** | 31 | | |
| **137** | 59 | | |
| **138** | | | 30 |
| **139** | | | 31 |
| **141** | 45 | | 36 |
| **142** | 32 | | |
| **143** | | 42 | |
| **144** | | 51 | |
| **145** | 64 | 89 | |
| **147** | | 45 | |
| **148** | | 43 | 32 |
| **149** | 33 | 33 | |
| **150** | 56 | 72 | |
| **152** | | 56 | |
| **153** | | 63 | |
| **155** | | 74 | |
| **156** | 60 | | |
| **157** | | 64 | |
| **159** | | 40 | |
| **161** | | 39 | |
| **163** | | 71 | |
| **165** | 63 | | |
| **166** | 47 | | |
| **167** | 38 | 52 | |
| **168** | 44 | 46 | |
| **169** | 51 | | |
| **170** | | 46 | |
| **171** | | 62 | |
| **172** | 49 | 76 | |
| **175** | 38 | 69 | |

(fortgesetzt)

| **Beispiel** | BTX Inhibierung | Uptake_5-HT, Ratte, 1μM, % Hemmung | Uptake (NA), Ratte, 10μM,% Hemmung |
|---|---|---|---|
| **176** | 30 | 37 | |
| **177** | 30 | 79 | |
| **178** | | 49 | |
| **179** | 46 | | |
| **180** | 42 | 63 | |
| **181** | 43 | | |
| **184** | 62 | | |
| **186** | | 32 | |
| **187** | | 47 | |
| **188** | 69 | 70 | |
| **189** | 40 | | |
| **190** | | 43 | |
| **191** | | 30 | |
| **192** | | 53 | |
| **194** | | 32 | |
| **195** | 34 | 50 | |
| **196** | 52 | | |
| **197** | 41 | | |
| **198** | 30 | | |
| **200** | 44 | 45 | |
| **201** | 42 | | |
| **202** | | 56 | |
| **203** | 48 | 34 | |
| **204** | 44 | 42 | |
| **206** | | 55 | |
| **207** | 57 | 76 | |
| **208** | 33 | | |
| **209** | 67 | 69 | |
| **210** | | 51 | 31 |
| **213** | 43 | | |
| **215** | 32 | | |
| **216** | | 55 | |
| **218** | 39 | 57 | |
| **219** | | 30 | |
| **220** | 62 | | |
| **221** | 36 | 79 | |
| **222** | | 60 | |
| **223** | 62 | 49 | |

(fortgesetzt)

| Beispiel | BTX Inhibierung | Uptake_5-HT, Ratte, 1μM, % Hemmung | Uptake (NA), Ratte, 10μM,% Hemmung |
|---|---|---|---|
| **224** | | 30 | |
| **225** | 55 | 32 | |
| **228** | | 32 | |
| **229** | | 36 | |
| **230** | 30 | 72 | |
| **231** | | 31 | |
| **233** | | 30 | |
| **234** | | 45 | |
| **236** | 50 | 48 | |
| **237** | 42 | 63 | |
| **238** | | | 31 |
| **239** | | 36 | |
| **240** | 30 | 54 | |
| **241** | 58 | | |
| **242** | 42 | | |
| **243** | | 46 | |
| **244** | | 51 | |
| **245** | 60 | | |
| **247** | 32 | | |
| **248** | | | |
| **249** | 34 | 55 | |
| **251** | | 50 | |
| **252** | | 73 | |
| **253** | | 82 | |
| **254** | | 34 | |
| **255** | | 44 | |
| **256** | 48 | 94 | 57 |
| **257** | 35 | 59 | |
| **258** | 32 | | |
| **259** | | 50 | |
| **261** | | 39 | |
| **262** | 70 | 60 | |
| **263** | | 50 | |
| **264** | 63 | | 36 |
| **265** | 34 | | 32 |
| **266** | 57 | 94 | 38 |
| **268** | 31 | | |
| **269** | | 40 | |

(fortgesetzt)

| **Beispiel** | BTX Inhibierung | Uptake_5-HT, Ratte, 1μM, % Hemmung | Uptake (NA), Ratte, 10μM,% Hemmung |
|---|---|---|---|
| **270** | 33 | 31 | 37 |
| **271** | | 44 | 31 |
| **272** | | | 31 |
| **273** | | 51 | 38 |
| **274** | 85 | 97 | 64 |
| **275** | 61 | 33 | |
| **276** | | 95 | 46 |
| **277** | | 43 | |
| **278** | | 39 | 56 |
| **279** | 60 | 48 | |
| **280** | 30 | 60 | 35 |
| **281** | | 56 | 30 |
| **282** | | 49 | |
| **283** | | 51 | |
| **284** | | 51 | |
| **285** | 32 | 65 | |
| **286** | | 47 | |
| **287** | 32 | 41 | 41 |
| **288** | 32 | 45 | |
| **290** | 30 | | 31 |
| **291** | 33 | 38 | |
| **292** | 40 | 40 | |
| **293** | 31 | 55 | |
| **294** | 33 | 53 | |
| **295** | 63 | | 36 |
| **296** | 36 | 30 | |
| **297** | 36 | | 32 |
| **298** | 48 | 34 | 30 |
| **299** | 36 | 66 | |
| **300** | 33 | | |
| **302** | 39 | | |
| **303** | 30 | | |
| **304** | 33 | 34 | 37 |
| **305** | 51 | 37 | |
| **306** | 68 | 85 | |
| **307** | 31 | | |
| **308** | | 59 | |
| **309** | 82 | 43 | |

(fortgesetzt)

| Beispiel | BTX Inhibierung | Uptake_5-HT, Ratte, 1µM, % Hemmung | Uptake (NA), Ratte, 10µM,% Hemmung |
|---|---|---|---|
| **310** | | 48 | |
| **311** | | 49 | |
| **313** | | 60 | |
| **315** | | 61 | |
| **317** | 62 | 33 | |
| **319** | | 70 | |
| **320** | | 38 | |
| **322** | 36 | | |
| **323** | 43 | 33 | |
| **324** | 62 | 31 | |
| **325** | | 32 | |
| **326** | 68 | | |
| **328** | | 32 | 33 |
| **329** | 43 | 55 | |
| **330** | 31 | 45 | |
| **331** | 46 | 58 | 38 |
| **332** | | | 36 |

**Patentansprüche**

**1.** Substituierte 1,4,8-Triazaspiro[4.5]decan-2-on-Verbindungen der allgemeinen Fornnel I,

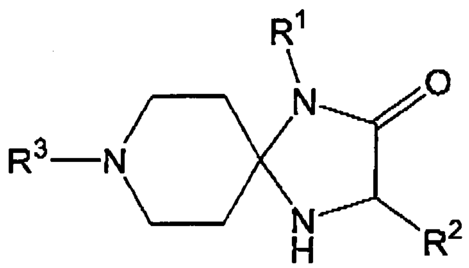

I

worin
$R^1$ für einen Wasserstoff-Rest, für einen linearen oder verzweigten, ggf. wenigstens ein Heteroatom als Kettenglied aufweisenden unsubstituierten Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, ggf. wenigstens ein Heteroatom als Kettenglied aufweisende Alkylen-Gruppe gebunden ist, für einen über eine lineare oder verzweigte Alkylen-Gruppe gebundenen -C(=O)-$OR^5$-Rest oder für einen über eine lineare oder verzweigte Alkylen-Gruppe gebundenen -O-(C=O)-$R^6$-Rest steht,

$R^2$ für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, oder für einen unsubstituierten oder wenigstens einfach substituierten Aryl- oder Heteroaryl-Rest steht, der über eine lineare oder verzweigte, ggf. wenigstens ein Heteroatom als Kettenglied aufweisende Alkylen-Gruppe gebunden sein kann,

$R^3$ für einen Wasserstoff-Rest, für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest, für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-Gruppe gebunden und gegebenenfalls mit einem unsubstituierten oder wenigstens einfach substituierten monozyklischen Ringsystem kondensiert ist, für einen unsubstituierten oder wenigstens einfach substituierten, ggf. wenigstens ein Heteroatom als Ringglied aufweisenden, ggf. über eine lineare oder verzweigte, unsubstituierte oder wenigstens einfach substituierte, ggf. wenigstens ein Heteroatom als Kettenglied aufweisende Alkylen-Gruppe gebundenen cycloaliphatischen Rest, der mit einer linearen oder verzweigten, unsubstituierten oder wenigstens einfach substituierten, ggf. wenigstens ein Heteroatom als Kettenglied aufweisenden Alkylen-Gruppe überbrückt und/oder mit einem unsubstituierten oder wenigstens einfach substituierten monozyklischen Ringsystem kondensiert sein kann, oder für eine - C(=O)-$R^4$-Gruppe steht,

$R^4$ für einen linearen oder verzweigten, unsubstituierten oder wenigstens einfach substituierten, ggf. wenigstens ein Heteroatom als Kettenglied aufweisenden Alkyl-Rest, für einen linearen oder verzweigten, unsubstituierten oder wenigstens einfach substituierten, ggf. wenigstens ein Heteroatom als Kettenglied aufweisenden Alkenyl-Rest, für einen linearen oder verzweigten, unsubstituierten oder wenigstens einfach substituierten, ggf. wenigstens ein Heteroatom als Kettenglied aufweisenden Alkinyl-Rest,

für einen unsubstituierten oder wenigstens einfach substituierten Aryl- oder Heteroaryl-Rest, der über eine lineare oder verzweigte, unsubstituierte oder wenigstens einfach substituierte, ggf. wenigstens ein Heteroatom als Kettenglied aufweisende Alkylen-, Alkenylen- oder Alkinylen-Gruppe gebunden und/oder mit einem unsubstituierten oder wenigstens einfach substituierten monozyklischen Ringsystem kondensiert sein kann,

für einen unsubstituierten oder wenigstens einfach substituierten, ggf. wenigstens ein Heteroatom als Ringglied aufweisenden, ggf. über eine lineare oder verzweigte, unsubstituierte oder wenigstens einfach substituierte, ggf. wenigstens ein Heteroatom als Kettenglied aufweisende Alkylen-Gruppe gebundenen cycloaliphatischen Rest, der mit einer linearen oder verzweigten, unsubstituierten oder wenigstens einfach substituierten Alkylen-Gruppe wenigstens einfach überbrückt und/oder mit einem unsubstituierten oder wenigstens einfach substituierten monozyklischen Ringsystem kondensiert sein kann,

für einen über eine lineare oder verzweigte, unsubstituierte oder wenigstens einfach substituierte Alkylen-Gruppe gebundenen -C(=O)-O$R^7$-Rest, für einen über eine lineare oder verzweigte, unsubstituierte oder wenigstens einfach substituierte Alkylen-Gruppe gebundenen -O-(C=O)$R^8$-Rest oder für einen über eine lineare oder verzweigte, unsubstituierte oder wenigstens einfach substituierte Alkylen-Gruppe gebundenen -(C=O)-$R^9$-Rest steht,

$R^5$ und $R^6$, unabhängig voneinander, jeweils für einen linearen oder verzweigten Alkyl-Rest, für einen linearen oder verzweigten Alkenyl-Rest oder für einen linearen oder verzweigten Alkinyl-Rest stehen,

$R^7$ und $R^8$, unabhängig voneinander, jeweils für einen Wasserstoff-Rest, für einen linearen oder verzweigten Alkyl-Rest, für einen linearen oder verzweigten Alkenyl-Rest, für einen linearen oder verzweigten Alkinyl-Rest, oder für einen unsubstituierten oder wenigstens einfach substituierten Aryl- oder Heteroaryl-Rest stehen,

$R^9$ für einen linearen oder verzweigten Alkyl-Rest, für einen linearen oder verzweigten Alkenyl-Rest oder für einen linearen oder verzweigten Alkinyl-Rest steht,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate,

worin "wenigstens einfach substituiert" im Zusammenhang mit einem Alkyl-, Alkenyl- oder Alkinyl-Rest für einen einfach oder mehrfach substituierten Alkyl-, Alkenyl- oder Alkinyl-Rest steht, wobei die Substituenten jeweils unabhängig voneinander ausgewählt sind aus der Gruppe bestehend aus F, Cl, Br, I, $C_{1-6}$-Alkoxy, -$NO_2$, -OH,-SH, -(C=O)-OH, -(C=O)-O-$C_{1-4}$-Alkyl, -O-(C=O)-$C_{1-4}$-Alkyl, -$CH_2$-O-$CH_2$-Phenyl, -CN, -$CF_3$, -$CHF_2$, -$CH_2F$, unsubstituiertem Phenyl und -$NR^aR^b$, worin $R^a$ und $R^b$ jeweils unabhängig voneinander ausgewählt werden können aus der Gruppe bestehend aus H, Methyl, Ethyl, n-Propyl, iso-Propyl, n-Butyl, sec-Butyl, tert.-Butyl, -(C=O)-O-$C_{1-4}$-Alkyl

und unsubstituiertem Phenyl;

worin "wenigstens einfach substituiert" im Zusammenhang mit einem cycloaliphatischen Rest für einen einfach oder mehrfach substituierten cycloaliphatischen Rest steht, wobei die Substituenten jeweils unabhängig voneinander ausgewählt sind aus der Gruppe bestehend aus F, Cl, Br, $C_{1-6}$-Alkoxy, Oxo, $C_{1-6}$-Alkyl, Hydroxy, -CN, $-CF_3$, $-CHF_2$, $CH_2F$, unsubstituiertem Phenyl, $-NR^aR^b$, worin $R^a$ und $R^b$ jeweils unabhängig voneinander ausgewählt werden können aus der Gruppe bestehend aus H, Methyl, Ethyl, n-Propyl, iso-Propyl und unsubstituiertem Phenyl, Thioxo (=S), I, $-SF_5$, $-NO_2$, $-O-CF_3$, $-S-CF_3$, -SH, $-S-C_{1-5}$-Alkyl, -C(=O)-H,$-C(=O)-C_{1-5}$-Alkyl, -C(=O)-OH, $-C(=O)-O-C_{1-5}$-Alkyl, $-(CH_2)$-C(=O)-OH, $-(CH_2)-C(=O)-O-C_{1-5}$-Alkyl, $-(CH_2)$-Benzo[b]furanyl, -O-Phenyl, -O-Benzyl, Phenyl, Benzyl, Naphthyl und $-(CH_2)$-Naphthyl, wobei jeweils der zyklische Teil der Reste -O-Phenyl, -0-Benzyl, Phenyl, $-(CH_2)$-Benzo[b]furanyl. Benzyl, Naphthyl und $-(CH_2)$-Naphthyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-C_{1-5}$-Alkyl, $-C_{1-5}$-Alkyl, $-0-CF_3$, $-S-CF_3$, Phenyl und -O-Benzyl substituiert sein kann;

worin "wenigstens einfach substituiert" im Zusammenhang mit einem Aryl- oder Heteroaryl-Rest für einen einfach oder mehrfach substituierten Aryl- oder Heteroaryl-Rest steht, wobei die Substituenten jeweils unabhängig voneinander vorzugsweise ausgewählt sind aus der Gruppe bestehend aus F, Cl, Br, I, -CN, $-NO_2$, $C_{1-5}$-Alkyl, OH, SH. $C_{1-5}$-Alkoxy, $C_{1-5}$-Perfluoroalkoxy, $-C(=O)-C_{1-5}$-Alkyl, $-C(=O)-O-C_{1-5}$-Alkyl,$-(CH_2)_{1-3}$-O-(C=O)-Phenyl, $-S-C_{1-5}$-Alkyl, $-S(=O)_2-C_{1-6}$-alkyl, $-S(=O)_2-NH_2$. $-NH-(C=O)-CH_3$, $-S-CHF_2$, $-S-CH_2F$, $-C(=O)-C_{1-5}$-Perfluoroalkyl, $-CF_3$, $CHF_2$, $CH_2F$, Phenyl, Phenoxy und $-NR^aR^b$, worin $R^a$ und $R^b$ jeweils unabhängig voneinander ausgewählt werden können aus der Gruppe bestehend aus H, Methyl, Ethyl, n-Propyl, iso-Propyl und unsubstituiertem Phenyl, $-SF_5$, $-O-C_{2-5}$-Alkenyl, $-O-CF_3$, $-O-CHF_2$, $-O-CH_2F$, - C(=O)-OH, $-O-C(=O)-C_{1-5}$-Alkyl, -O-C(=O)-Phenyl, $-(CH_2)-O-C(-O)-C_{1-5}$-Alkyl, $-NH-C_{1-5}$-Alkyl, $-N(C_{1-5}\text{-Alkyl})_2$, $-NH-C(=O)-O-C_{1-5}$-Alkyl, -C(=O)-H, $-C(=O)-C_{1-5}$-Alkyl, - $C(=O)-NH_2$, $-C(=O)-NH-C_{1-5}$-Alkyl, $C(=O)-N-(C_{1-5}\text{-Alkyl})_2$, $-S(=O)_2-NH-C_{1-5}$-Alkyl, - $S(=O)_2$-NH-Phenyl, $-(CH_2)$-Benzo[b]furanyl, -O-Phenyl, -S-Phenyl, -S-Benzyl, -O-Benzyl, Phenyl und Benzyl, wobei der zyklische Teil der Reste $-S(=O)_2$-NH-Phenyl, - O-Phenyl, -S-phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-C_{1-5}$-Alkyl, $-C_{1-5}$-Alkyl, $-O-CF_3$, $-S-CF_3$, Phenyl und -O-Benzyl substituiert sein kann und die Phenyl- und Phenoxy-Substituenten selbst einfach oder mehrfach, gleich oder verschieden, mit einem Substituenten ausgewählt aus der Gruppe bestehend aus F, Cl, Br, $-S-CHF_2$,- $CF_3$, $-CHF_2$, $-CH_2F$, -CN, $-NO_2$, $C_{1-5}$-Alkyl und $C_{1-5}$-Alkoxy substituiert sein können;

worin "wenigstens einfach substituiert" im Zusammenhang mit einem monozyklischen Ringsystem für ein einfach oder mehrfach substituiertes monozyklisches Ringsystem steht, wobei die Substituenten jeweils unabhängig voneinander vorzugsweise ausgewählt sind aus der Gruppe bestehend aus F, Cl, Br, I, -CN, $-NO_2$, Oxo, $C_{1-5}$-Alkyl, $C_{1-5}$-Alkoxy, $C_{1-5}$-Perfluoralkoxy, $-C(=O)C_{1-5}$-Alkyl, - $C(=O)-O-C_{1-5}$-Alkyl, $-S(=O)_2-C_{1-6}$-alkyl, -C(=O)- $C_{1-5}$-Pertluoroalkyl, $-CF_3$, $CHF_2$, $CH_2F$ und $-NR^aR^b$, worin $R^a$ und $R^b$ jeweils unabhängig voneinander ausgewählt werden können aus der Gruppe bestehend aus H, Methyl, Ethyl, n-Propyl, iso-Propyl und unsubstituiertem Phenyl, Thioxo (=S), $-SF_5$, -OH, $-O-C_{2-5}$-Alkanyl, $-NH_2$, $-NO_2$, $-O-GF_3$, $-O-CHF_2$, $-O-CH_2F$, $-S-CF_3$, $-S-CHF_2$, $-S-CH_2F$, -SH, $-S-C_{1-5}$-Alkyl, -C(=O)-OH, $-O-C(=O)-C_{1-5}$-Alkyl, -O-C(=O)-Phenyl, $-(CH_2)-O-C(=O)-C_{1-5}$-Alkyl, $-(CH_2)$-O-C(=O)-Phenyl, $-NH-C_{1-5}$-Alkyl, $-N(C_{1-5}\text{-Alkyl})_2$, $-NH-C(=O)-O-C_{1-5}$-Alkyl, $-NH-C(=O)-C_{1-5}$-Alkyl. -C(=O)-H, $-C(=O)-NH_2$, $-C(=O)-NH-C_{1-5}$-Alkyl, $C(=O)-N-(C_{1-5}\text{-Alkyl})_2$, $-S(=O)_2-NH_2$, - $S(=O)_2-NH-C_{1-5}$-Alkyl, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl, wobei jeweils der zyklische Teil der Reste -O-C(=O)-Phenyl, $-(CH_2)$-O-C(=O)-Phenyl, $-S(=O)_2$-NH-Phenyl. -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-C_{1-5}$-Alkyl, $-C_{1-5}$-Alkyl, $-O-CF_3$, $-S-CF_3$, Phenyl und -O-Benzyl substituiert sein kann;

worin "wenigstens einfach substituiert" im Zusammenhang mit einer linearen oder verzweigten Alkylen-, Alkenylen- oder Alkinylen-Gruppe für eine einfach oder mehrfach substituierte lineare oder verzweigte Alkylen-, Alkenylen- oder Alkinylen-Gruppe steht, wobei die Substituenten jeweils unabhängig voneinander vorzugsweise ausgewählt sind aus der Gruppe bestehend aus F, Cl, Br, $C_{1-5}$-Alkoxy, Hydroxy, CN, $CF_3$, $CHF_2$, $CH_2F$, unsubstituiertem Phenyl und $-NR^aR^b$, worin $R^a$ und $R^b$ jeweils unabhängig voneinander ausgewählt werden können aus der Gruppe bestehend aus H, $C_{1-3}$-Alkyl und unsubstituiertem Phenyl;

wobei Verbindungen der allgemeinen Formel I, in denen

A)

$R^1$ für einen Wasserstoff-Rest, einen $C_{1-6}$-Alkyl-Rest, einen $C_{1-6}$-Alkoxy-Rest, einen $C_{2-6}$-Alkenyl-Rest, einen $C_{2-6}$-Alkinyl-Rest oder für einen einfach mit einem Phenyl-Rest substituierten $C_{1-6}$-Alkyl-Rest steht,

$R^2$ für einen Wasserstoff-Rest, einen $C_{1-6}$-Alkyl-Rest, einen $C_{1-6}$-Alkoxy-Rest, einen $C_{2-6}$-Hydroxyalkyl-Rest, einen $C_{2-6}$-Afkenyl-Rest, einen $C_{2-6}$-Alkinyl-Rest, einen Phenyl-Rest, einen einfach, zweifach oder dreifach mit einem Phenyl-Rest substituierten $C_{1-6}$-Alkyl-Rest, eine $-O-C_{1-6}$-Alkanoyl-Rest, einen $-O-C_{1-6}$-Alkyl-Rest, einen

$-O-C_{2-6}$-Hydroxyalkyl-Rest, einen $-O-C_{2-6}$-Alkenyl-Rest, einen- $O-C_{2-6}$-Alkinyl-Rest oder für einen einfach, zweifach oder dreifach mit einem Phenyl-Rest substituierten $-O-C_{1-6}$-Alkyl-Rest steht,
$R^3$ für einen Wasserstoff-Rest, einen $C_{1-6}$-Alkyl-Rest, einen $C_{2-6}$-Alkenyl-Rest, einen $C_{2-6}$-Alkinyl-Rest, einen $C_{3-7}$-Cycloalkyl-Rest, einen mit 1-6 Halogenatomen substituierten $C_{1-6}$-Alkyl-Rest, einen Hydroxy-$C_{1-6}$-Alkyl-Rest, einen $C_{1-6}$-Alkoxy-Rest, einen $C_{1-6}$-Alkylthio-Rest, einen $C_{1-6}$-Alkoxy-$C_{1-6}$-Alkyl-Rest, einen Carboxy-$C_{1-6}$-Alkyl-Rest, einen ($C_{1-6}$-Alkoxy)carbonyl-$C_{1-6}$-Alkyl-Rest, einen Amino-$C_{1-6}$-alkyl-Rest, einen mono-($C_{1-6}$-Alkyl)-amino-$C_{1-6}$-Alkyl-Rest, einen di-($C_{1-6}$-Alkyl)-amino-$C_{1-6}$-Alkyl-Rest, einen 2-oxo-pyrrlidin-1-ylmethyl-Rest, einen Aryl-Rest, einen Diarylmethylol-Rest, einen mit ein oder zwei Aryl-Resten substituierten $C_{1-6}$-Alkyl-Rest, einen $C_{1-6}$-Alkanoyl-Rest oder einen Arylcarbonyl-Rest steht, wobei Aryl jeweils für einen unsubstituierten Phenyl-Rest oder für einen mit 1-3-Substituenten ausgewählt aus der Gruppe bestehend aus Halogen, $C_{1-6}$-Alkyl, $C_{1-6}$-Alkoxy und $CF_3$ substituierten Phenyl-Rest steht
sowie deren physiologische verträgliche Salze, Enantiomeren und Racemate,
und
B)
die Reste $R^1$, $R^2$ und $R^3$ jeweils für einen Wasserstoff-Rest oder einem Kohlenwasserstoff-Rest stehen sowie jeweils deren Salze ausgenommen sind;

2. Verbindungen gemäß Anspruch 1, **dadurch gekennzeichnet, daß** $R^1$ für einen Wasserstoff-Rest, für einen linearen oder verzweigten, unsubstituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest, für einen unsubstituierten oder wenigstens einfach substituierten, 5- bis 14-gliedrigen Aryl- oder Heteroaryl-Rest, der über eine lineare oder verzweigte, ggf. wenigstens ein Heteroatom als Kettenglied aufweisende $d_{1-5}$-Alkylen-Gruppe gebunden ist, für einen über eine lineare oder verzweigte $C_{1-5}$-Alkylen-Gruppe gebundenen -C (=O)-$OR^5$-Rest oder für einen über eine lineare oder verzweigte $C_{1-5}$-Alkylen-Gruppe gebundenen -O-C (=O)-$R^6$-Rest steht,
vorzugsweise für einen Wasserstoff-Rest, für einen linearen oder verzweigten, unsubstituierten $C_{1-5}$-Alkyl-Rest, für einen linearen oder verzweigten, unsubstituierten $C_{1-5}$-Alkenyl-Rest, für einen linearen oder verzweigten, unsubstituierten $C_{2-5}$-Alkinyl-Rest, für einen unsubstituierten oder wenigstens einfach substituierten Phenyl- oder Naphthyl-Rest, der über eine lineare oder verzweigte, ggf. eines oder mehrere Sauerstoffatome als Kettenglied aufweisende $C_{1-5}$-Alkylen-Gruppe gebunden ist, für einen über eine lineare oder verzweigte $C_{1-4}$-Alkylen-Gruppe gebundenen -C(=O)-$OR^5$-Rest oder für einen über eine lineare oder verzweigte $C_{1-4}$-Alkylen-Gruppe gebundenen -O-C (=O)-$R^6$-Rest steht,
besonders bevorzugt für einen Wasserstoff-Rest, für einen linearen oder verzweigten, unsubstituierten $C_{1-4}$-Alkyl-Rest, für einen linearen oder verzweigten, unsubstituierten $C_{2-5}$-Alkenyl-Rest, für einen linearen oder verzweigten, unsubstituierten $C_{2-3}$-Alkinyl-Rest, für einen Phenyl- oder Naphthyl-Rest, der über eine $-(CH_2)-$, $-(CH_2)_2-$, $-(CH_2)_3-$ oder $-(CH_2)_2-O-$Brücke gebunden und ggf. einfach oder mehrfach, gleich oder verschieden, mit einem Substituenten ausgewählt aus der Gruppe bestehend aus F, Cl, Br, -CN, $-NO_2$, $C_{1-5}$-Alkyl, $C_{1-5}$-Alkoxy, -C(=O)-$C_{1-5}$-Alkyl, -C (=O)-O-$C_{1-5}$-Alkyl, $-S(=O)_2-C_{1-6}$-alkyl, -C(=O)- $C_{1-5}$-Perfluoroalkyl, $-CF_3$, $-CHF_2$ und $-CH_2F$ substituiert ist, für einen über eine $-(CH_2)-$Gruppe gebundenen -C(=O)-$OR^5$-Rest oder für einen über eine $-(CH_2)-$, $-(CH_2)_2-$, $-(CH_2)_3-$ oder $-(CH_2)_4-$Gruppe gebundenen -0-C(=O)-$R^6$-Rest steht.

3. Verbindungen gemäß Anspruch 1 oder 2, **dadurch gekennzeichnet, daß** der Rest $R^2$ für einen Wasserstoff-Rest, für einen linearen oder verzweigten, unsubstituierten oder wenigstens einfach substituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest, oder für einen unsubstituierten oder wenigstens einfach substituierten, 5- bis 14-gliedrigen Aryl- oder Heteroaryl-Rest steht, der über eine lineare oder verzweigte, ggf. wenigstens ein Heteroatom als Kettenglied aufweisende $C_{1-5}$-Alkylen-Gruppe gebunden sein kann,
vorzugsweise für einen Wasserstoff-Rest, für einen linearen oder verzweigten, unsubstituierten, ggf. eines oder mehrere Sauerstoffatome und/oder eines oder mehrere Schwefelatome als Kettenglied aufweisenden $C_{1-5}$-Alkyl-Rest oder für einen Phenyl- oder Naphthyl-Rest steht, der einfach oder mehrfach, gleich oder verschieden, mit einem Substituenten ausgewählt aus der Gruppe bestehend aus F, Cl, Br, -CN, $-NO_2$, $C_{1-5}$-Alkyl, $C_{1-5}$-Alkoxy, -C (=O)-$C_{1-5}$-Alkyl, -C(=O)-O-$C_{1-5}$-Alkyl, $-S(=O)_2-C_{1-6}$-alkyl, -C(=O)- $C_{1-5}$-Perfluoroalkyl, $-CF_3$, $-CHF_2$ und $-CH_2F$ substituiert und/oder über eine $-(CH_2)-$, $-(CH_2)_2-$, $-(CH_2)_3-$ oder $-(CH_2)_2-O-$Brücke gebunden sein kann,
besonders bevorzugt für einen Wasserstoff-Rest, für einen linearen oder verzweigten, unsubstituierten, ggf. eines

oder mehrere Schwefelatome als Kettenglied aufweisenden $C_{1-5}$-Alkyl-Rest oder für einen Phenyl-Rest steht, wobei der Phenyl-Rest einfach oder mehrfach, gleich oder verschieden, mit einem Substituenten ausgewählt aus der Gruppe bestehend aus F, Cl, Br, -CN,- $NO_2$, $C_{1-5}$-Alkyl, $C_{1-5}$-Alkoxy, -C(=O)-$C_{1-5}$-Alkyl, -C(=O)-O-$C_{1-5}$-Alkyl, -S(=O)$_2$-$C_{1-6}$-alkyl, -C(=O)- $C_{1-5}$-Perfluoroalkyl, -$CF_3$, -$CHF_2$ und -$CH_2$F substituiert und/oder über eine -($CH_2$)-Brücke gebunden sein kann.

**4.** Verbindungen gemäß einem oder mehreren der Ansprüche 1-3, **dadurch gekennzeichnet, daß** $R^3$ für einen Wasserstoff-Rest, für einen linearen oder verzweigten, unsubstituierten oder wenigstens einfach substituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest, für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende $C_{1-5}$-Alkylen-Gruppe gebunden und gegebenenfalls mit einem unsubstituierten oder wenigstens einfach substituierten monozyklischen Ringsystem kondensiert ist, für einen unsubstituierten oder wenigstens einfach substituierten, ggf. wenigstens ein Heteroatom als Ringglied aufweisenden $C_{3-8}$-cycloaliphatischen Rest steht, der über eine lineare oder verzweigte, unsubstituierte oder wenigstens einfach substituierte, ggf. wenigstens ein Heteroatom als Kettenglied aufweisende $C_{1-5}$-Alkylen-Gruppe gebunden und/oder mit einem unsubstituierten oder wenigstens einfach substituierten monozyklischen Ringsystem kondensiert sein kann, oder für eine -C(=O)-$R^4$-Gruppe steht,
vorzugsweise für einen Wasserstoff-Rest, für einen Biphenyl-Rest, der über eine -($CH_2$)-, -($CH_2$)$_2$- oder -($CH_2$)$_3$-Brükke gebunden ist, oder für eine -C(=O)-$R^4$-Gruppe steht,
besonders bevorzugt für eine -C(=O)-$R^4$-Gruppe steht.

**5.** Verbindungen gemäß einem oder mehreren der Ansprüche 1-4, **dadurch gekennzeichnet, daß** der Rest $R^4$ für einen linearen oder verzweigten, unsubstituierten oder wenigstens einfach substituierten, ggf. wenigstens ein Heteroatom als Kettenglied aufweisenden $C_{1-10}$-Alkyl-Rest, für einen linearen oder verzweigten, unsubstituierten oder wenigstens einfach substituierten, ggf. wenigstens ein Heteroatom als Kettenglied aufweisenden $C_{2-10}$-Alkenyl-Rest, für einen linearen oder verzweigten, unsubstituierten oder wenigstens einfach substituierten, ggf. wenigstens ein Heteroatom als Kettenglied aufweisenden $C_{2-10}$-Alkinyl-Rest,
für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte, ggf. wenigstens ein Heteroatom als Kettenglied aufweisende $C_{1-5}$-Alkylen-, $C_{2-5}$-Alkenylen oder $C_{2-5}$-Alkinylen-Gruppe gebunden und/oder mit einem unsubstituierten oder wenigstens einfach substituierten monozyklischen Ringsystem kondensiert sein kann,
für einen unsubstituierten oder wenigstens einfach substituierten, ggf. wenigstens ein Heteroatom als Ringglied aufweisenden, ggf. über eine lineare oder verzweigte, unsubstituierte oder wenigstens einfach substituierte, ggf. wenigstens ein Heteroatom als Kettenglied aufweisende $C_{1-6}$-Alkylen-Gruppe gebundenen $C_{3-8}$ cycloaliphatischen Rest, der mit einer linearen oder verzweigten, unsubstituierten oder wenigstens einfach substituierten $C_{1-6}$-Alkylen-Gruppe wenigstens einfach überbrückt und/oder mit einem unsubstituierten oder wenigstens einfach substituierten monozyklischen Ringsystem kondensiert sein kann,
für einen über eine lineare oder verzweigte, unsubstituierte oder wenigstens einfach substituierte $C_{1-5}$-Alkylen-Gruppe gebundenen -C(=O)-O$R^7$-Rest, für einen über eine lineare oder verzweigte, unsubstituierte oder wenigstens einfach substituierte $C_{1-5}$-Alkylen-Gruppe gebundenen -O-(C=O)-$R^8$-Rest oder für einen über eine lineare oder verzweigte, unsubstituierte oder wenigstens einfach substituierte $C_{1-5}$-Alkylen-Gruppe gebundenen -(C=O)-$R^9$-Rest steht,
vorzugsweise für einen linearen oder verzweigten, ggf. eines oder mehrere Sauerstoffatome und/oder eines oder mehrere Schwefelatome als Kettenglied aufweisenden $C_{1-10}$-Alkyl-Rest, der unsubstituiert oder wenigstens einfach, gleich oder verschieden, mit einem Substituenten ausgewählt aus der Gruppe bestehend aus Phenyl, Di-($C_{1-5}$-Alkylamino), Carboxy und -NH-(C=O)-O-$C_{1-5}$-Alkyl substituiert sein kann, für einen linearen oder verzweigten, unsubstituierten $C_{2-5}$-Alkenyl-Rest, für einen Phenyl-Rest, Naphthyl-Rest, Furyl-(Furanyl-)-Rest, Thienyl-(Thiophenyl-)-Rest oder [1,2,3]-Triazolyl-Rest, Benzo[1.3]dioxolyl-Rest, Benzo[1,2,5]oxadiazolyl-Rest, Chromanyl-Rest, Pyrimidinyl-Rest, Pyrazolyl-Rest, Pyridinyl-Rest, Isoxazolyl-Rest, oder [1,2,3]-Thiadiazolyl-Rest, wobei der zyklische Rest jeweils über eine-($CH_2$)-, -($CH_2$)-O-, -CH=CH-, -($CH_2$)$_2$-, -($CH_2$)$_2$-O-, oder -($CH_2$)$_3$-Brücke gebunden sein kann und/oder einfach oder mehrfach, gleich oder verschieden, mit einem Substituenten ausgewählt aus der Gruppe bestehend aus F, Cl, Br, I, -CN,-$NO_2$, $C_{1-5}$-Alkyl, $C_{1-5}$-Alkoxy, $C_{1-5}$-Perfluoralkoxy, -C(=O)-$C_{1-5}$-Alkyl, -C(=O)-O-$C_{1-5}$-Alkyl, -($CH_2$)$_{1-3}$-O-C(=O)-Phenyl, -N($CH_3$)$_2$, -S(=O)$_2$-$C_{1-6}$-alkyl, -S(=O)$_2$-$NH_2$, -C(=O)-$C_{1-5}$-Perfluoroalkyl, -S-

$C_{1-5}$-Alkyl, -S-$CH_2$F, -S-$CHF_2$, -$SCF_3$, -$CF_3$, -NH-(C=O)-$CH_3$, -$CHF_2$, -$CH_2$F, Phenyl und Phenoxy, wobei die letztgenannten Phenyl- und Phenoxy Substituenten selbst einfach oder mehrfach, gleich oder verschieden, mit einem Substituenten ausgewählt aus der Gruppe bestehend aus F, Cl, Br, -S-$CHF_2$, -$CF_3$, -$CHF_2$, -$CH_2$F, -CN, -$NO_2$, $C_{1-5}$-Alkyl und $C_{1-5}$-Alkoxy substituiert sein können,
für einen ggf. wenigstens ein Heteroatom als Ringglied aufweisenden, ggf. über eine -($CH_2$)-, -$(CH_2)_2$- oder -$(CH_2)_3$-Brücke gebundenen 3-, 4-, 5-, 6-, 7- oder 8-gliedrigen cycloaliphatischen Rest, der wenigstens einfach mit einer-(C$(CH_3)_2$)- oder -($CH_2$)-Gruppe überbrückt und/oder unsubstituiert oder einfach oder mehrfach, gleich oder verschieden, mit einem Substituenten ausgewählt aus der Gruppe bestehend aus $C_{1-5}$-Alkyl, $C_{1-5}$-Alkoxy, -$CF_3$, -$CHF_2$, -$CH_2$F und Oxo substituiert sein kann,
oder für einen über eine lineare oder verzweigte, unsubstituierte oder wenigstens einfach substituierte $C_{1-5}$-Alkylen-Gruppe gebundenen -C(=O)-$OR^7$-Rest, für einen über eine lineare oder verzweigte, unsubstituierte oder wenigstens einfach substituierte $C_{1-5}$-Alkylen-Gruppe gebundenen -O-(C=O)-$R^8$-Rest, oder für einen über eine lineare oder verzweigte, unsubstituierte oder wenigstens einfach substituierte $C_{1-5}$-Alkylen-Gruppe gebundenen -(C=O)-$R^9$-Rest steht,
besonders bevorzugt für einen linearen oder verzweigten, ggf. eines oder mehrere Sauerstoffatome und/oder eines oder mehrere Schwefelatome als Kettenglied aufweisenden $C_{1-8}$-Alkyl-Rest, der unsubstituiert oder wenigstens einfach, gleich oder verschieden, mit einem Substituenten ausgewählt aus der Gruppe bestehend aus unsubstituiertem Phenyl, Dimethylamino, Carboxy und - NH-(C=O)-O-C$(CH_3)_3$ substituiert sein kann, für einen linearen oder verzweigten, unsubstituierten $C_{2-4}$-Alkenyl-Rest, für einen Phenyl-Rest, Naphthyl-Rest, Furyl- (Furanyl-)-Rest, Thienyl-(Thiophenyl-)-Rest, [1,2,3]-Triazolyl-Rest, Chromanyl-Rest, Benzo[1.3]dioxolyl-Rest, Benzo[1,2,5]oxadiazolyl-Rest, Pyrimidinyl-Rest, Pyrazolyl-Rest, Pyridinyl-Rest, Isoxazolyl-Rest oder [1,2,3]-Thiadiazolyl-Rest, wobei der zyklische Rest jeweils über eine -($CH_2$)-, -($CH_2$)-O-, -CH=CH-, -$(CH_2)_2$-, -$(CH_2)_2$-O-, oder -$(CH_2)_3$-Brücke gebunden sein kann und/oder einfach oder mehrfach, gleich oder verschieden, mit einem Substituenten ausgewählt aus der Gruppe bestehend aus F, Cl, Br, I, Methyl, Ethyl, iso-Propyl, n-Propyl, sec-Butyl, tert.-Butyl, n-Butyl, Methoxy, Ethoxy, -$CF_3$, -$OCF_3$, -$CH_2$-O-C(=O)-Phenyl, -$NO_2$, -S-$CF_3$, -S-$CHF_2$, -N$(CH_3)_2$, -NH-CO-$CH_3$, -CN, -$SO_2$-$NH_2$, Phenyl und Phenoxy, wobei die letztgenannten Phenyl- und Phenoxy Substituenten selbst einfach oder mehrfach, gleich oder verschieden, mit einem Substituenten ausgewählt aus der Gruppe bestehend aus F, Cl, Br, -$CH_3$ und -$OCH_3$ substituiert sein können,
für einen ggf. über eine -($CH_2$)-, -$(CH_2)_2$- oder -$(CH_2)_3$-Brücke gebundenen Cyclopropyl, Cyclobutyl, Cyclopentyl, Cyclohexyl, Imidazolidin, , 4,7,7-Trimethyl-3-oxo-2-oxa-bicyclo-[2.2.1]-heptyl-Rest oder Adamantyl-Rest, der unsubstituiert oder einfach oder mehrfach, gleich oder verschieden, mit einem Substituenten ausgewählt aus der Gruppe bestehend aus $C_{1-5}$-Alkyl, $C_{1-5}$-Alkoxy, -$CF_3$, -$CHF_2$, -$CH_2$F und Oxo substituiert ist,
für einen über eine -($CH_2$)-, -$(CH_2)_2$-, -C(H)(Phenyl)-, oder -C$(CH_3)_2$-Gruppe gebundenen -C(=O)-$OR^7$-Rest, für einen über eine -($CH_2$)-, -$(CH_2)_2$-, -C(H)(Phenyl)-, oder -C$(CH_3)_2$-Gruppe gebundenen -O-(C=O)-$R^8$-Rest, oder für einen über eine -($CH_2$)- oder -$(CH_2)_2$-Gruppe gebundenen -(C=O)-$R^9$-Rest steht,
ganz besonders bevorzugt für einen linearen oder verzweigten, ggf. eines oder mehrere Sauerstoffatome und/oder eines oder mehrere Schwefelatome als Kettenglied aufweisenden $C_{6-8}$-Alkyl-Rest, der unsubstituiert oder wenigstens einfach, gleich oder verschieden, mit einem Substituenten ausgewählt aus der Gruppe bestehend aus unsubstituiertem Phenyl, Dimethylamino, Carboxy und - NH-(C=O)-O-C$(CH_3)_3$ substituiert sein kann, für einen linearen oder verzweigten, unsubstituierten $C_{2-4}$-Alkenyl-Rest, für einen Naphthyl-Rest, Furyl-(Furanyl-)-Rest, Thienyl-(Thiophenyl-)-Rest, [1,2,3]-Triazolyl-Rest, Chromanyl-Rest, Benzo[1.3]dioxolyl-Rest, Benzo[1,2,5]oxadiazolyl-Rest, Pyrimidinyl-Rest, Pyrazolyl-Rest, Pyridinyl-Rest, Isoxazolyl-Rest oder [1,2,3]-Thiadiazolyl-Rest, wobei der zyklische Rest jeweils über eine -($CH_2$)-, -($CH_2$)-O-, -CH=CH-, - $(CH_2)_2$-, -$(CH_2)_2$-O-, oder -$(CH_2)_3$-Brücke gebunden sein kann und/oder einfach oder mehrfach, gleich oder verschieden, mit einem Substituenten ausgewählt aus der Gruppe bestehend aus F, Cl, Br, I, Methyl, Ethyl, iso-Propyl, n-Propyl, sec-Butyl, tert.-Butyl, n-Butyl, Methoxy, Ethoxy, -$CF_3$, -$OCF_3$, -$CH_2$-O-C(=O)-Phenyl, -$NO_2$, -S-$CF_3$, -S-$CHF_2$, -N$(CH_3)_2$, -NH-CO-$CH_3$, -CN, -$SO_2$-$NH_2$, Phenyl und Phenoxy, wobei die letztgenannten Phenyl- und Phenoxy Substituenten selbst einfach oder mehrfach, gleich oder verschieden, mit einem Substituenten ausgewählt aus der Gruppe bestehend aus F, Cl, Br, -$CH_3$ und -$OCH_3$ substituiert sein können, für 2-Trifluoromethoxy-phenyl, 3- Trifluoromethoxyphenyl, 4-Trifluoromethoxy-phenyl, 2-$CF_3$-S-phenyl, 3-$CF_3$-S-phenyl, 4-$CF_3$-S-phenyl, 2-Cyano-phenyl, 3-Cyano-phenyl, 4-Cyano-phenyl, 2-Nitro-phenyl, 3-Nitro-phenyl, 4-Nitro-phenyl, 2-$CHF_2$-S-Phenyl, 3-$CHF_2$-S-Phenyl, 4-$CHF_2$-S-Phenyl, 2-Dimethylamino-phenyl, 3-Dimethylamino-phenyl, 4-Dimethylaminophenyl, 2-Phenyl-(C=O)-O-$CH_2$-phenyl, 3-Phenyl-(C=O)-O-$CH_2$-phenyl, 4-Phenyl-(C=O)-O-$CH_2$-phenyl, 2-$CH_3$-(C=O)-NH-Phenyl, 3-$CH_3$-(C=O)-NH-Phenyl, 4-$CH_3$-(C=O)-NH-Phenyl, 2-Phenyl-phenyl, 3-Phenyl-phenyl, 4-Phenylphenyl, 2-$NH_2$-$SO_2$-Phenyl, 3-$NH_2$-$SO_2$-Pheny, 4-$NH_2$-$SO_2$-Phenyl, Pentafluorphenyl, 4-Methyl-3-nitro-phenyl, 2-Fluor-benzyl, 3-Fluor-benzyl, 4-Fluor-benzyl, 2-Chlor-benzyl, 3-Chlor-benzyl, 4-Chlor-benzyl, 2-Brom-benzyl, 3-Brom-benzyl, 4-Brom-benzyl, 2-Methyl-benzyl, 3-Methyl-benzyl, 4-Methyl-benzyl, 2-Methoxybenzyl, 3-Methoxy-benzyl, 4-Methoxy-benzyl, 3,4-Dimethoxy-benzyl, 2,5-Dimethoxy-benzyl, -CH=CH-Phenyl, wobei der Phenyl-Rest in 2-, 3- oder 4-Position einfach oder mehrfach, gleich oder verschieden, mit einem

Rest ausgewählt aus der Gruppe bestehend aus F, Cl, Br und $CF_3$ substituiert sein kann, $-CH_2$-O-Phenyl, wobei der Phenyl-Rest in 2-, 3- oder 4- Position einfach oder mehrfach, gleich oder verschieden, mit einem Rest ausgewählt aus der Gruppe bestehend aus F, Cl, Br und $CF_3$ substituiert sein kann, für einen ggf. über eine $-(CH_2)-$, $-(CH_2)_2-$ oder $-(CH_2)_3$-Brücke gebundenen Cyclopropyl, Cyclobutyl, Cyclopentyl, Cyclohexyl, Imidazolidin, 4,7,7-Trimethyl-3-oxo-2-oxabicyclo-[2.2.1]-heptyl-Rest oder Adamantyl-Rest, wobei die zyklischen Reste jeweils unsubstituiert oder einfach oder mehrfach, gleich oder verschieden, mit einem Substituenten ausgewählt aus der Gruppe bestehend aus $C_{1-5}$-Alkyl, $C_{1-5}$-$_5$-Alkoxy, $-CF_3$, $CHF_2$, $CH_2F$ und Oxo substituiert sein können, für einen über eine $-(CH_2)-$, $-(CH_2)_2-$, -C(H)(Phenyl)-, oder $-C(CH_3)_2$-Gruppe gebundenen - C(=O)-$OR^7$-Rest, für einen über eine $-(CH_2)-$, $-(CH_2)_2-$, -C(H)(Phenyl)-, oder-$C(CH_3)_2$-Gruppe gebundenen -O-(C=O)-$R^8$-Rest, oder für einen über eine - $(CH_2)$- oder $-(CH_2)_2$-Gruppe gebundenen -(C=O)-$R^9$-Rest steht.

**6.** Verbindungen gemäß einem oder mehreren der Ansprüche 1-5, **dadurch gekennzeichnet, daß** die Reste $R^5$ und $R^6$, jeweils unabhängig voneinander, für einen linearen oder verzweigten $C_{1-5}$-Alkyl-Rest, einen linearen oder verzweigten $C_{2-5}$-Alkenyl-Rest oder für einen linearen oder verzweigten $C_{2-5}$-Alkinyl-Rest, vorzugsweise für einen Methyl-, Ethyl-, n-Propyl oder iso-Propyl-Rest stehen.

**7.** Verbindungen gemäß einem oder mehreren der Ansprüche 1-6, **dadurch gekennzeichnet, daß** die Reste $R^7$ und $R^8$, jeweils unabhängig voneinander, für einen Wasserstoff-Rest, für einen linearen oder verzweigten $C_{1-5}$-Alkyl-Rest, einen linearen oder verzweigten $C_{2-5}$-Alkenyl-Rest, einen linearen oder verzweigten $C_{2-5}$-Alkinyl-Rest, oder für einen ggf. wenigstens einfach substituierten Phenyl- oder Naphthyl-Rest stehen, vorzugsweise für einen Methyl-, Ethyl-, n-Propyl, iso-Propyl-Rest oder unsubstituierten Phenyl-Rest stehen.

**8.** Verbindungen gemäß einem oder mehreren der Ansprüche 1-7, **dadurch gekennzeichnet, daß** der Rest $R^9$ für einen linearen oder verzweigten $C_{1-5}$-Alkyl-Rest, einen linearen oder verzweigten $C_{2-5}$-Alkenyl-Rest, einen linearen oder verzweigten $C_{2-5}$-Alkinyl-Rest, vorzugsweise für einen Methyl-, Ethyl-, n-Propyl oder iso-Propyl-Rest steht.

**9.** Verbindungen gemäß einem oder mehreren der Ansprüche 1-8, **dadurch gekennzeichnet, daß**
$R^1$ für einen Wasserstoff-Rest, für einen linearen oder verzweigten, unsubstituierten $C_{1-4}$-Alkyl-Rest, für einen linearen oder verzweigten, unsubstituierten $C_{2-5}$-Alkenyl-Rest, für einen linearen oder verzweigten, unsubstituierten $C_{2-3}$-Alkinyl-Rest, für einen Phenyl- oder Naphthyl-Rest, der unsubstituiert oder einfach oder mehrfach, gleich oder verschieden, mit einem Substituenten ausgewählt aus der Gruppe bestehend aus F, Cl, Br, -CN, $-NO_2$, $C_{1-5}$-Alkyl, $C_{1-5}$-Alkoxy, -C(=O)-$C_{1-5}$-Alkyl, -C(=O)-O-$C_{1-5}$-Alkyl, $-S(=O)_2$-$C_{1-6}$-alkyl, -C(=O)-$C_{1-5}$-Perfluoroalkyl, $-CF_3$, $-CHF_2$ und $-CH_2F$ substituiert und/oder über eine $-(CH_2)-$, $-(CH_2)_2-$, $-(CH_2)_3-$ oder $-(CH_2)_2$-O-Brücke gebunden ist, für einen über eine $-(CH_2)$-Gruppe gebundenen -C(=O)-$OR^5$-Rest oder für einen über eine $-(CH_2)-$, $-(CH_2)_2-$, $-(CH_2)_3-$ oder $-(CH_2)_4$-Gruppe gebundenen -O-C(=O)-$R^6$-Rest steht,
$R^2$ für einen Wasserstoff-Rest, für einen linearen oder verzweigten, unsubstituierten, ggf. eines oder mehrere Schwefelatome als Kettenglied aufweisenden $C_{1-5}$-Alkyl-Rest oder für einen Phenyl-Rest steht, wobei der Phenyl-Rest einfach oder mehrfach, gleich oder verschieden, mit einem Substituenten ausgewählt aus der Gruppe bestehend aus F, Cl, Br, -CN, $-NO_2$, $C_{1-5}$-Alkyl, $C_{1-5}$-Alkoxy, -C(=O)-$C_{1-5}$-Alkyl, -C(=O)-O-$C_{1-5}$-Alkyl, $-S(=O)_2$-$C_{1-6}$-alkyl, -C (=O)-$C_{1-5}$-Perfluoroalkyl, $-CF_3$, $-CHF_2$ und $-CH_2F$ substituiert und/oder über eine $-(CH_2)$-Brücke gebunden sein kann,
$R^3$ für einen Wasserstoff-Rest, für einen Biphenyl-Rest, der über eine $-(CH_2)-$,$-(CH_2)_2-$ oder $-(CH_2)_3$-Brücke gebunden ist, oder für eine C(=O)-$R^4$-Gruppe steht,
$R^4$ für einen Rest steht ausgewählt aus der Gruppe bestehend aus Methyl, Ethyl, iso-Propyl, n-Propyl, sec-Butyl, Isobutyl, tert-Butyl, n-Butyl, n-Pentyl, 1-Methyl-butyl, 2-Dimethyl-propyl, 1-Ethyl-propyl, 1-Propyl-butyl, 1-Ethyl-pentyl, Dimethylaminomethyl, $-CH_2-CH_2-CH=CH_2$, $-CH_2-O-CH_3$, $-CH_2-O-CH_2-CH_2-O-CH_3$, $-CH_2-CH_2$-Phenyl, $-CH_2$-O-Phenyl, $-CH_2-O-CH_2$-Phenyl, $-CH_2-CH_2-CH_2$-O-Phenyl, -C(H)(Phenul)($C_2H_5$), -C(H)($CH_3$)-O-Phenyl, $-CH_2-CH_2-(C=O)-CH_3$, $-CH_2-O-(C=O)-CH_3$, $-CH_2-CH_2-(C=O)-O-C_2H_5$, $-CH_2$-O-(C=O)-Phenyl, $-CH_2-(C=O)-O-CH_2-CH_3$, -C(H)(Phenyl)-(C=O)-$CH_3$, -C(H)(Phenyl)-O-(C=O)-$CH_3$, $-C(CH_3)_2-O-(C=O)-CH_3$, -C(H)(NH-(C=O)-O-$(CH_3)_3$)($CH_2-O-CH_2$-Phenyl), - $C(CH_3)_2-CH_2$-COOH, unsubstituiertem Phenyl, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl, 3-Brom-phenyl, 4-Brom-phenyl, 2-lod-phenyl, 3-lod-phenyl, 4-lod-phenyl, 2-Methyl-phenyl, 3-Methyl-phenyl, 4-Methyl-phenyl, 2-Ethyl-phenyl, 3-Ethyl-phenyl, 4-Ethyl-phenyl, 2-n-Propyl-phenyl, 3-n-Proyl-phenyl, 4-n-Propylphenyl, 2-tert.-Butyl-phenyl, 3-tert.-Butyl-phenyl, 4-tert.-Butyl-phenyl, 2-Methoxy-phenyl, 3-Methoxy-phenyl, 4-Methoxy-phenyl, 2-Ethoxy-phenyl, 3-Ethoxy-phenyl, 4-Ethoxy-phenyl, 2-Trifluoromethyl-phenyl, 3- Trifluoromethylphenyl, 4-Trifluoromethyl-phenyl, 2-Trifluoromethoxy-phenyl, 3-Trifluoromethoxy-phenyl, 4-Trifluoromethoxy-phenyl, 2-$CF_3$-S-phenyl, 3-$CF_3$-S-phenyl, 4-$CF_3$-S-phenyl, 2-Cyano-phenyl, 3-Cyano-phenyl, 4-Cyano-phenyl, 2-Nitro-phenyl, 3-Nitro-phenyl, 4-Nitro-phenyl, 2-$CHF_2$-S-Phenyl, 3-$CHF_2$-S-Phenyl, 4-$CHF_2$-S-Phenyl, 2-Dimethylamino-phenyl, 3-Dimethylamino-phenyl, 4-Dimethylamino-phenyl, 2-Phenyl-(C=O)-O-$CH_2$-phenyl, 3-Phenyl-(C=O)-O-$CH_2$-phenyl, 4-Phe-

nyl-(C=O)-O-$CH_2$-phenyl, 2-$CH_3$-(C=O)-NH-Phenyl, 3-$CH_3$-(C=O)-NH-Phenyl, 4-$CH_3$-(C=O)-NH-Phenyl, 2-Phenyl-phenyl, 3-Phenyl-phenyl, 4-Phenyl-phenyl, 2-$NH_2$-$SO_2$-Phenyl, 3-$NH_2$-$SO_2$-Pheny, 4-$NH_2$-$SO_2$-Phenyl, 2,4-Dimethoxy-phenyl, 2,5-Dimethoxy-phenyl, 3,5-Dimethoxy-phenyl, 3,4-Dimethoxy-phenyl, 2,3-Dimethyl-phenyl, 2,3-Dichlorphenyl, 3,4-Dichlorphenyl, 2,6-Dichlorphenyl, 2,3-Difluorphenyl, 3,4-Difluorphenyl, 3,5-Difluor-phenyl, Pentafluorphenyl, 2-Chlor-6-Fluor-phenyl, 4-Brom-3-methyl-phenyl, 3-Fluor-4-methyl-phenyl, 3-Chlor-2-Fluor-phenyl, 2-Chlor-4-nitro-phenyl, 5-Fluor-2-trifluoromethyl-phenyl, 3-Fluor-4-trifluoromethyl, 4-Methyl-3-nitro-phenyl, 2-Chlor-5-trifluoro-methyl, 2,5-Bis-trifluoromethyl-phenyl, 3,5-Bis-trifluoromethylphenyl, 2-Chlor-6-fluor-3-methyl-phenyl, 6-Chlor-2-fluor-3-methyl-phenyl, 2,6-Difluor-4-methyl-phenyl, 2,6-Difluor-3-methyl-phenyl, 3,4,5-Trimethoxy-phenyl, 2,3-Difluor-4-methyl-phenyl, 2-Fluor-benzyl, 3-Fluor-benzyl, 4-Fluor-benzyl, 2-Chlor-benzyl, 3-Chlor-benzyl, 4-Chlor-benzyl, 2-Brom-benzyl, 3-Brom-benzyl, 4-Brom-benzyl, 2-Methyl-benzyl, 3-Methyl-benzyl, 4-Methyl-benzyl, 2-Methoxybenzyl, 3-Methoxy-benzyl, 4-Methoxy-benzyl, 3,4-Dimethoxy-benzyl, 2,5-Dimethoxy-benzyl, -CH=CH-Phenyl, wobei der Phenyl-Rest in 2-, 3- oder 4-Position einfach oder mehrfach, gleich oder verschieden, mit einem Rest ausgewählt aus der Gruppe bestehend aus F, Cl, Br und $CF_3$ substituiert sein kann, -$CH_2$-O-Phenyl, wobei der Phenyl-Rest in 2-, 3- oder 4- Position einfach oder mehrfach, gleich oder verschieden, mit einem Rest ausgewählt aus der Gruppe bestehend aus F, Cl, Br und $CF_3$ substituiert sein kann, 1-Naphthyl, 2-Naphthyl, 2-Thienyl, 3-Thienyl, 3-Chlor-thien-2-yl, 2-Furanyl, 3-Furanyl, 2,5-Dimethyl-Furan-3-yl, 5-tert.-Butyl-2-methyl-furan-3-yl, Pyridin-2-yl, Pyridin-3-yl, Pyridin-4-yl, 2-Chlor-pyridin-4-yl , 6-Chlor-pyridin-3-yl, 2-Chlor-pyridin-3-yl, 2-Ethylsulfanyl-pyridin-3-yl, 2-Phenoxy-pyridin-3-yl, 2-Methylsulfanyl-pyridin-3-yl, 2-Methyl-6-trifluoromethyl-pyridin-3-yl, Isoxazol-5-yl, 5-Methyl-isoxazol-3-yl, 3-(2-Chlor-6-Fluor-phenyl)-5-methyl-isoxazol-4-yl, 3-(2-Chlor-phenyl)-5-methylisoxazol-4-yl, 3-(2,6-Dichlor-phenyl)-5-methyl-isoxazol-4-yl, 5-tert.-Buiyl-2-methyl-2H-pyrazol-3-yl, 1-Phenyl-5-n-Propyl-1H-pyrazol-4-yl, 1-(4-Chlor-phenyl)-5-trifluoromethyl-1H-pyrazol-4-yl, 2-tert.-Butyl-5-methyl-2H-pyrazol-3-yl, 5-Methyl-2-Phenyl-2H-[1,2,3]triazol-4-yl, 4-Methyl-[1,2,3]thiadiazol-5-yl, 2-Chlor-4-trifluormethyl-pyrimidin-5-yl, 2-Chlor-4-trifluoromethyl-pyrimidin-5-yl, Benzo[1,2,5]oxadiazol-5-yl, Benzo[1.3]dioxol-5-yl, 6-Chlor-2H-chroman-3-yl, Imidazolidin-2,4-dion-5-yl-methyl, Cyclopropyl, Cyclobutyl, ggf. über eine-($CH_2$)- oder -$(CH_2)_2$-Brücke gebundenem Cyclopentyl, Cyclohexyl, 4,7,7-Trimethyl-3-oxo-2-oxa-bicyclo[2.2.1]heptyl und Adamantyl.

**10.** Verbindungen gemäß einem oder mehreren der Ansprüche 1 bis 9, **dadurch gekennzeichnet, dass**

I.)

$R^1$ für einen substituierten 6- oder 10-gliedrigen Aryl-Rest steht;
für einen ggf. substituierten 5- bis 14-gliedrigen Heteroaryl-Rest steht;
für -$(CH_2)_m$-O-C(=O)-$R^5$ mit m = 1, 2, 3, 4 oder 5 steht;
für -$(CH_2)_n$-C(=O)-O-$R^6$ mit n = 1, 2, 3, 4 oder 5 steht;
für -($CH_2$)-$R^{22}$ steht;
oder für -($CH_2$)-$(CH_2)_c$-U-$(CH_2)_d$-$R^{23}$ mit c = 0 oder 1 und d = 0 oder 1 steht, worin U für O, S, NH, N($CH_3$) oder N($C_2H_5$) steht;
$R^2$ für einen Wasserstoff-Rest steht;
für -($CH_2$)-$(CH_2)_e$-$V_f$-$(CH_2)_g$-$R^{24}$ mit e = 0 oder 1, f = 0 oder 1 und g = 0 oder 1, worin V für O, S, NH, N ($CH_3$) oder N($C_2H_5$) steht;
für einen jeweils linearen oder verzweigten, jeweils ggf.substituierten $C_{1-10}$ Alkyl-Rest, $C_{2-10}$ Alkenyl-Rest oder $C_{2-10}$ Alkinyl-Rest steht;
oder für einen ggf. substituierten 5- bis 14-gliedrigen Aryl- oder Heteroaryl-Rest steht;
$R^3$ für eine -C(=O)-$R^4$-Gruppe steht;

und

$R^4$ für -($CHR^{10}$)-$P_s$-$(CH_2)_t$$(CH_2)_u$-$Q_v$-$R^{11}$ mit s = 0 oder 1, t = 0 oder 1, u = 0 oder 1 und v = 0 oder 1 steht, worin P und Q unabhängig voneinander jeweils für O, S, NH, N($CH_3$) oder N($C_2H_5$) stehen und die Summe von s und v gleich 1 ist;
für -($CR^{12}R^{13}$)-$(CH_2)_w$$(CH_2)_x$-C(=O)-O-$R^7$ mit w = 0 oder 1 und x = 0 oder 1 steht;
für -($CR^{14}R^{15}$)-$(CH_2)_y$-O-C(=O)-$R^8$ mit y = 0 oder 1 steht;
für -($CHR^{16}$)-$(CH_2)_z$-C(=O)-$R^9$ mit z = 0 oder 1 steht;
für -CH[($CH_2$)-$T_a$-$(CH_2)_b$-$R^{17}$][NH-C(=O)-O-$R^{18}$] mit a = 0 oder 1 und b = 0 oder 1 steht, worin T für O, S, NH, N($CH_3$) oder N($C_2H_5$) steht;
für -$CHR^{19}R^{20}$ oder -($CH_2$)-$CHR^{19}R^{20}$ steht;
für -(CH=CH)-$R^{21}$ steht;
für einen jeweils linearen oder verzweigten, jeweils ggf.substituierten $C_{1-10}$ Alkyl-Rest, $C_{2-10}$ Alkenyl-Rest

oder $C_{2-10}$ Alkinyl-Rest steht;
für einen ungesättigten oder gesättigten, ggf. substituierten 3-, 4-, 5-, 6-, 7-, 8- oder 9-gliedrigen cycloaliphatischen Rest steht, der mit einem gesättigten oder ungesättigten, ggf. aromatischen, ggf. substituierten monozyklischen Ringsystem kondensiert und/oder mit einer oder zwei linearen oder verzweigten, ggf. substituierten $C_{1-5}$-Alkylen- Gruppe überbrückt sein kann,
oder für einen ggf. substituierten 5- bis 14- gliedrigen Aryl- oder Heteroaryl- Rest steht, der mit einem gesättigten oder ungesättigten, ggf. substituierten monozyklischen Ringsystem kondensiert sein kann;

oder II.)

$R^1$ für einen Wasserstoff-Rest steht;
für einen jeweils linearen oder verzweigten, jeweils unsubstituierten $C_{1-10}$ Alkyl-Rest, $C_{2-10}$ Alkenyl-Rest oder $C_{2-10}$ Alkinyl-Rest steht;
oder für einen unsubstituierten Phenyl- oder Benzyl-Rest steht;
$R^2$ für einen Wasserstoff-Rest steht;
für $-(CH_2)-(CH_2)_e-V_f-(CH_2)_g-R^{24}$ mit e = 0 oder 1, f = 0 oder 1 und g = 0 oder 1, worin V für O, S, NH, N $(CH_3)$ oder $N(C_2H_5)$ steht;
für einen jeweils linearen oder verzweigten, jeweils ggf.substituierten $C_{1-10}$ Alkyl-Rest, $C_{2-10}$ Alkenyl-Rest oder $C_{2-10}$ Alkinyl-Rest steht;
oder für einen ggf. substituierten 5- bis 14-gliedrigen Aryl- oder Heteroaryl-Rest steht;
$R^3$ für eine $-C(=O)-R^4$-Gruppe steht;

und

$R^4$ für $-(CHR^{10})-P_s-(CH_2)_t-(CH_2)_u-Q_v-R^{11}$ mit s = 0 oder 1, t = 0 oder 1, u = 0 oder 1 und v = 0 oder 1 steht, worin P und Q unabhängig voneinander jeweils für O, S, NH, $N(CH_3)$ oder $N(C_2H_5)$ stehen, und die Summe von s und v gleich 1 ist;
für $-(CR^{12}R^{13})-(CH_2)_w-(CH_2)_x-C(=O)-O-R^7$ mit w = 0 oder 1 und x = 0 oder 1 steht;
für $-(CR^{14}R^{15})-(CH_2)_y-O-C(=O)-R^8$ mit y = 0 oder 1 steht;
für $-(CHR^{16})-(CH_2)_z-C(=O)-R^9$ mit z = 0 oder 1 steht;
für $-CH[(CH_2)-T_a-(CH_2)_b-R^{17}][NH-C(=O)-O-R^{18}]$ mit a = 0 oder 1 und b = 0 oder 1 steht, worin T für O, S, NH, $N(CH_3)$ oder $N(C_2H_5)$ steht;
für $-CHR^{19}R^{20}$ oder $-(CH_2)-CHR^{19}R^{20}$ steht;
für $-(CH=CH)-R^{21}$ steht;
für einen linearen oder verzweigten, substituierten $C_{1-10}$ Alkyl-Rest steht;
für einen linearen oder verzweigten, ggf. substituierten $C_{2-10}$ Alkenyl-Rest steht;
für einen linearen oder verzweigten, ggf. substituierten $C_{2-10}$ Alkinyl Rest steht;
für einen ungesättigten oder gesättigten, ggf. substituierten 3-, 4-, 5-, 6-, 7-, 8- oder 9-gliedrigen cycloaliphatischen Rest steht, der mit einem gesättigten oder ungesättigten, ggf. aromatischen, ggf. substituierten monozyklischen Ringsystem kondensiert und/oder mit einer oder zwei linearen oder verzweigten, ggf. substituierten $C_{1-5}$-Alkylen-Gruppe überbrückt sein kann;
für einen Phenyl-Rest, der mit 1, 2, 3, 4 oder 5 Substituenten unabhängig voneinander ausgewählt aus der Gruppe bestehend aus-CN, $-SF_5$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-C_{1-5}$-Alkyl, -C(=O)-OH, $-C(=O)-O-C_{1-5}$-Alkyl, $-O-C(=O)-C_{1-5}$-Alkyl, -O-C(=O)-Phenyl, $-(CH_2)-O-C(=O)-C_{1-5}$-Alkyl, $-(CH_2)$-O-C(=O)-Phenyl, $-NH-C_{1-5}$-Alkyl, $-N(C_{1-5}\text{-Alkyl})_2$, $-NH-C(=O)-O-C_{1-5}$-Alkyl, $-NH-C(=O)-C_{1-5}$-Alkyl, -C(=O)-H, $-C(=O)-C_{1-5}$-Alkyl, $-C(=O)-NH_2$, $-C(=O)-NH-C_{1-5}$-Alkyl, $C(=O)-N(C_{1-5}\text{-Alkyl})_2$, $-S(=O)_2-NH_2$, $-S(=O)_2-NH-C_{1-5}$-Alkyl,$-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert ist, wobei jeweils der zyklische Teil der Reste -O-C(=O)-Phenyl, $-(CH_2)$-O-C(=O)-Phenyl,$-S(=O)_2$-NH-Phenyl, -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-C_{1-5}$-Alkyl, $-C_{1-5}$-Alkyl, $-O-CF_3$, $-S-CF_3$, Phenyl und -O-Benzyl substituiert sein kann,
für einen Rest ausgewählt aus der Gruppe bestehend aus 1,2,3,4,5-Pentafluorophenyl, 1,2,3,4-Tetrafluorophenyl, 4-Methyl-3-nitro-phenyl, 5-Methyl-3-nitro-phenyl, 6-Methyl-3-nitro-phenyl und 2-Methyl-3-nitrophenyl steht;
für einen unsubstituierten Naphthyl-Rest steht;
für einen ggf. substituierten 6- oder 1 0-gliedrigen Aryl-Rest steht, der mit einem gesättigten oder ungesättigten, ggf. substituierten monozyklischen Ringsystem kondensiert ist;

oder für einen ggf. substituierten 5- bis 14-gliedrigen Heteroaryl-Rest steht, der mit einem gesättigten oder ungesättigten, ggf. substituierten monozyklischen Ringsystem kondensiert sein kann;

oder III.)

$R^1$ für einen Wasserstoff-Rest steht;
für einen jeweils linearen oder verzweigten, jeweils unsubstituierten $C_{1-10}$Alkyl-Rest, $C_{2-10}$ Alkenyl-Rest oder $C_{2-10}$Alkinyl-Rest steht;
für einen ggf. substituierten 5- bis 14-gliedrigen Aryl- oder Heteroaryl-Rest steht;
für $-(CH_2)_m-O-C(=O)-R^5$ mit m = 1, 2, 3, 4 oder 5 steht;
für $-(CH_2)_n-C(=O)-O-R^6$ mit n = 1, 2, 3, 4 oder 5 steht;
für einen unsubstituierten Benzyl-Rest steht;
für $-(CH_2)-R^{22}$ steht;
oder für-$(CH_2)-(CH_2)_c-U-(CH_2)_d-R^{23}$ mit c = 0 oder 1 und d = 0 oder 1 steht, worin U für O, S, NH, $N(CH_3)$ oder $N(C_2H_5)$ steht;
$R^2$ für $-(CH_2)-(CH_2)_e-V-(CH_2)_9-R^{24}$ mit e = 0 oder 1 und g = 0 oder 1, worin V für S, NH, $N(CH_3)$ oder N $(C_2H_5)$ steht;
$R^3$ für eine $-C(=O)-R^4$-Gruppe steht;

und

$R^4$ für $-(CHR^{10})-P_s-(CH_2)_t-(CH_2)_u-Q_v-R^{11}$ mit s = 0 oder 1, t = 0 oder 1, u = 0 oder 1 und v = 0 oder 1 steht, worin P und Q unabhängig voneinander jeweils für O, S, NH, $N(CH_3)$ oder $N(C_2H_5)$ stehen und die Summe von s und v gleich 1 ist;
für $-(CR^{12}R^{13})-(CH_2)_w-(CH_2)_x-C(=O)-O-R^7$ mit w = 0 oder 1 und x = 0 oder 1 steht;
für $-(CR^{14}R^{15})-(CH_2)_y-O-C(=O)-R^8$ mit y = 0 oder 1 steht;
für $-(CHR^{16})-(CH_2)_z-C(=O)-R^9$ mit z = 0 oder 1 steht;
für $-CH[(CH_2)-T_a-(CH_2)_b-R^{17}][NH-C(=O)-O-R^{18}]$ mit a = 0 oder 1 und b = 0 oder 1 steht, worin T für O, S, NH, $N(CH_3)$ oder $N(C_2H_5)$ steht;
für $-CHR^{19}R^{20}$ oder $-(CH_2)-CHR^{19}R^{20}$ steht;
für $-(CH=CH)-R^{21}$ steht;
für einen jeweils linearen oder verzweigten, jeweils ggf. substituierten $C_{1-10}$Alkyl-Rest, $C_{2-10}$Alkenyl-Rest oder $C_{2-10}$ Alkinyl-Rest steht;
für einen ungesättigten oder gesättigten, ggf. substituierten 3-, 4-, 5-, 6-, 7-, 8- oder 9-gliedrigen cycloaliphatischen Rest steht, der mit einem gesättigten oder ungesättigten, ggf. aromatischen, ggf. substituierten monozyklischen Ringsystem kondensiert und/oder mit einer oder zwei linearen oder verzweigten, ggf. substituierten $C_{1-5}$-Alkylen- Gruppe überbrückt sein kann,
oder für einen ggf. substituierten 5- bis 14- gliedrigen Aryl- oder Heteroaryl- Rest steht, der mit einem gesättigten oder ungesättigten, ggf. substituierten monozyklischen Ringsystem kondensiert sein kann;

und jeweils - sofern vorhanden - in den vorstehend genannten Gruppen I.) II.) und III.) die Reste

$R^5$, $R^6$, $R^9$ und $R^{18}$ unabhängig voneinander jeweils
für einen jeweils linearen oder verzweigten, jeweils ggf. substituierten $C_{1-10}$ Alkyl-Rest, $C_{2-10}$ Alkenyl-Rest oder $C_{2-10}$ Alkinyl-Rest stehen;
$R^7$ für einen Wasserstoff-Rest steht;
für einen jeweils linearen oder verzweigten, jeweils ggf. substituierten $C_{1-10}$ Alkyl-Rest, $C_{2-10}$ Alkenyl-Rest oder $C_{2-10}$ Alkinyl-Rest steht;
oder für einen ggf. substituierten 5- bis 14-gliedrigen Aryl- oder Heteroaryl-Rest steht;
$R^8$, $R^{16}$ und $R^{24}$ unabhängig voneinander jeweils
für einen jeweils linearen oder verzweigten, jeweils ggf.substituierten $C_{1-10}$Alkyl-Rest, $C_{2-10}$Alkenyl-Rest oder $C_{2-10}$Alkinyl-Rest stehen;
oder für einen ggf. substituierten 5- bis 14-gliedrigen Aryl- oder Heteroaryl-Rest stehen;
$R^{10}$, $R^{12}$ und $R^{13}$ unabhängig voneinander jeweils
für einen Wasserstoff-Rest stehen;
für einen jeweils linearen oder verzweigten, jeweils ggf. substituierten $C_{1-10}$ Alkyl-Rest, $C_{2-10}$ Alkenyl-Rest oder $C_{2-10}$ Alkinyl-Rest stehen;
$R^{11}$ für einen jeweils linearen oder verzweigten, jeweils ggf. substituierten $C_{1-10}$ Alkyl-Rest, $C_{2-10}$ Alkenyl-

Rest oder $C_{2-10}$ Alkinyl-Rest steht;
für einen ungesättigten oder gesättigten, ggf. substituierten 3-, 4-, 5-, 6-, 7-, 8- oder 9-gliedrigen cycloaliphatischen Rest steht;
oder für einen ggf. substituierten 6- oder 10-gliedrigen Aryl-Rest steht;
$R^{14}$, $R^{15}$ und $R^{19}$ unabhängig voneinander jeweils
für einen Wasserstoff-Rest stehen;
für einen jeweils linearen oder verzweigten, jeweils ggf.substituierten $C_{1-10}$ Alkyl-Rest, $C_{2-10}$ Alkenyl-Rest oder $C_{2-10}$ Alkinyl-Rest stehen;
oder für einen ggf. substituierten 6- oder 1 0-gliedrigen Aryl-Rest stehen;
$R^{17}$ und $R^{21}$ unabhängig voneinander jeweils
für einen ggf. substituierten 6- oder 10-gliedrigen Aryl-Rest stehen;
$R^{20}$ für einen ungesättigten oder gesättigten, ggf. substituierten 3-, 4-, 5-, 6-, 7-, 8- oder 9-gliedrigen cycloaliphatischen Rest steht
oder für einen ggf. substituierten 6- oder 10-gliedrigen Aryl-Rest steht;
$R^{22}$ für einen substituierten 6-gliedrigen Aryl-Rest steht;
für einen ggf. substituierten 1 0-gliedrigen Aryl-Rest steht
oder für einen ggf. substituierten Heteroaryl-Rest steht;

und

$R^{23}$ für einen ggf. substituierten 5- bis 14-gliedrigen Aryl- oder Heteroaryl-Rest steht;

wobei jeweils
die vorstehend genannten $C_{1-10}$ Alkyl-Reste unsubstituiert oder jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$ und -N($C_{1-5}$-Alkyl)($C_{1-5}$-Alkyl) substituiert sein können;
die vorstehend genannten $C_{2-10}$ Alkenyl-Reste unsubstituiert oder jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$ und -N($C_{1-5}$-Alkyl)($C_{1-5}$-Alkyl) substituiert sein können;
die vorstehend genannten $C_{2-10}$ Alkinyl-Reste unsubstituiert oder jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$ und -N($C_{1-5}$-Alkyl)($C_{1-5}$-Alkyl) substituiert sein können;
die vorstehend genannten $C_{1-5}$-Alkylen-Gruppen unsubstituiert oder jeweils mit 1, 2, 3, 4 oder 5 Substituenten unabhängig voneinander ausgewählt aus der Gruppe bestehend aus F, Cl, Br, -OH, -SH, $-NH_2$, -CN, $NO_2$ und Phenyl substituiert sein können,
die vorstehend genannten cycloaliphatischen Reste unsubstituiert oder jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, -O-$C_{1-5}$-Alkyl, $-NH_2$, $-NO_2$, $-O-CF_3$, $-S-CF_3$, -SH, -S-$C_{1-5}$-Alkyl, -$C_{1-5}$-Alkyl, -C(=O)-H, -C(=O)-$C_{1-5}$-Alkyl, -C(=O)-OH, -C(=O)-O-$C_{1-5}$-Alkyl, -($CH_2$)C(=O)-OH, -($CH_2$)-C(=O)-O-$C_{1-5}$-Alkyl, -NH-$C_{1-5}$-Alkyl, -N($C_{1-5}$-Alkyl)$_2$, -($CH_2$)-Benzo[b]furanyl, -O-Phenyl, -O-Benzyl, Phenyl, Benzyl, Naphthyl und -($CH_2$)-Naphthyl substituiert sein können, wobei jeweils der zyklische Teil der Reste -O-Phenyl, -O-Benzyl, Phenyl, -($CH_2$)-Benzo[b]furanyl, Benzyl, Naphthyl und -($CH_2$)-Naphthyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -O-$C_{1-5}$-Alkyl, -$C_{1-5}$-Alkyl, $-O-CF_3$, $-S-CF_3$, Phenyl und -O-Benzyl substituiert sein kann,
und die vorstehend genannten cycloaliphatischen Reste jeweils 1, 2, 3, 4 oder 5 Heteroatom(e) unabhängig voneinander ausgewählt aus der Gruppe bestehend aus Sauerstoff, Stickstoff und Schwefel aufweisen können;
der Ring der vorstehend genannten monozyklischen Ringsysteme ggf. unsubstituiert oder mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, -O-$C_{1-5}$-Alkyl, -O-$C_{2-5}$-Alkenyl, $-NH_2$,$-NO_2$, $-O-CF_3$, $-O-CHF_2$, $-O-CH_2F$, $-S-CF_3$, $-S-CHF_2$, $-S-CH_2F$, -SH, -S-$C_{1-5}$-Alkyl, -$C_{1-5}$-Alkyl, -C(=O)-OH, -C(=O)-O-$C_{1-5}$-Alkyl, -O-C(=O)-$C_{1-5}$-Alkyl, -0-C(=O)-Phenyl, -($CH_2$)-O-C(=O)-$C_{1-5}$-Alkyl, -($CH_2$)-O-C(=O)-Phenyl, -NH-$C_{1-5}$-Alkyl, -N($C_{1-5}$-Alkyl)$_2$, -NH-C(=O)-O-$C_{1-5}$-Alkyl, -NH-C(=O)-$C_{1-5}$-Alkyl, -C(=O)-H, -C(=O)-$C_{1-5}$-Alkyl, -C(=O)-$NH_2$, -C(=O)-NH-$C_{1-5}$-Alkyl, C(=O)-N($C_{1-5}$-Alkyl)$_2$, -S(=O)$_2$-$NH_2$, -S(=O)$_2$-NH-$C_{1-5}$-Alkyl, -S(=O)$_2$-NH-Phenyl, -($CH_2$)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -0-C(=O)-Phenyl, -($CH_2$)-O-C(=O)-Phenyl, -S(=O)$_2$-NH-Phenyl, -O-Phenyl, -S-Phenyl, -S-Benzyl, -O-Benzyl, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, -O-$C_{1-5}$-Alkyl,-$C_{1-5}$-Alkyl, $-O-CF_3$, $-S-CF_3$, Phenyl und -O-Benzyl substituiert sein kann,

und der Ring der vorstehend genannten monozyklischen Ringsysteme jeweils 5-, 6- oder 7-gliedrig ist und jeweils 1, 2, 3, 4 oder 5 Heteroatom(e) als Ringglied(er) aufweisen kann, die unabhängig voneinander aus der Gruppe bestehend aus Sauerstoff, Stickstoff und Schwefel ausgewählt sind;
und, sofern nicht anders angegeben, die vorstehend genannten Aryl- oder Heteroaryl-Reste unsubstituiert oder jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, -O-$C_{1-5}$-Alkyl, -O-$C_{2-5}$-Alkenyl, $-NH_2$, $-NO_2$, $-O-CF_3$, $-O-CHF_2$, $-O-CH_2F$, $-S-CF_3$, $-S-CHF_2$, $-S-CH_2F$, -SH, -S-$C_{1-5}$-Alkyl, -$C_{1-5}$-Alkyl, -C(=O)-OH, -C(=O)-O-$C_{1-5}$-Alkyl, -O-C(=O)-$C_{1-5}$-Alkyl, -O-C (=O)-Phenyl, -($CH_2$)-O-C(=O)-$C_{1-5}$-Alkyl, -($CH_2$)-O-C(=O)-Phenyl, -NH-$C_{1-5}$-Alkyl, -N($C_{1-5}$-Alkyl)$_2$, -NH-C(=O)-O-$C_{1-5}$-Alkyl, -NH-C(=O)-$C_{1-5}$-Alkyl, -C(=O)-H, -C(=O)-$C_{1-5}$-Alkyl, -C(=O)-$NH_2$, -C(=O)-NH-$C_{1-5}$-Alkyl, C (=O)-N-($C_{1-5}$-Alkyl)$_2$, -S(=O)$_2$-$NH_2$, -S(=O)$_2$-NH-$C_{1-5}$-Alkyl, -S(=O)$_2$-NH-Phenyl, - ($CH_2$)-Benzo[b]furanyl, -O-Phenyl, -S-Phenyl, -S-Benzyl, -O-Benzyl, Phenyl und Benzyl substituiert sein können, wobei jeweils der zyklische Teil der Reste -O-C(=O)-Phenyl, -($CH_2$)-O-C(=O)-Phenyl, -S(=O)$_2$-NH-Phenyl, -O-Phenyl, -S-Phenyl, -S-Benzyl, -O-Benzyl, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN,$-NO_2$, -O-$C_{1-5}$-Alkyl, -$C_{1-5}$-Alkyl, $-O-CF_3$, $-S-CF_3$, Phenyl und -O-Benzyl substituiert sein kann,
und
die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**11.** Verbindungen gemäß Anspruch 10, **dadurch gekennzeichnet, dass**

I.)

$R^1$ für einen Phenyl- oder Naphthyl-Rest steht, der jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, -C(=O)-O-$CH_3$, -C(=O)-O-$C_2H_5$, -C(=O)-O-C($CH_3$)$_3$, -O-C(=O)-$CH_3$, -O-C(=O)-$C_2H_5$, -O-C(=O)-Phenyl, -($CH_2$)-O-C(=O)-$CH_3$,- ($CH_2$)-O-C(=O)-$C_2H_5$, -($CH_2$)-O-C(=O)-Phenyl, $-N(CH_3)_2$,$-N(C_2H_5)_2$, -N(H)($CH_3$), -N(H)($C_2H_5$), -NH-C(=O)-O-$CH_3$, -NH-C(=O)-O-$C_2H_5$, -NH-C(=O)-$CH_3$, -NH-C(=O)-$C_2H_5$, -C(=O)-H, -C (=O)$CH_3$,-C(=O)-$C_2H_5$, -C(=O)-$NH_2$, -C(=O)-NH-$CH_3$, -C(=O)-NH-$C_2H_5$, -S(=O)$_2$-$NH_2$, -S(=O)$_2$-NH-$CH_3$, -S(=O)$_2$-NH-$C_2H_5$, -S(=O)$_2$-NH-Phenyl, -($CH_2$)-Benzo[b]furanyl, -O-Phenyl, -S-Phenyl, -S-Benzyl, -O-Benzyl, Phenyl und Benzyl substituiert ist, wobei jeweils der zyklische Teil der Reste-O-C(=O)-Phenyl, -($CH_2$)-O-C(=O)-Phenyl, -S(=O)$_2$-NH-Phenyl, -O-Phenyl, -S-Phenyl, -S-Benzyl, -O-Benzyl, Phenyl, -($CH_2$)-Benzo[b] furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,
für einen Heteroaryl-Rest ausgewählt aus der Gruppe bestehend aus Thiophenyl, Furanyl, Pyrrolyl, Pyrazolyl, Pyrazinyl, Pyranyl, Triazolyl, Pyridinyl, Imidazolyl, Indolyl, Isoindolyl, Benzo [b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl steht, wobei der Heteroaryl-Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$,$-O-CHF_2$, $-O-CH_2F$, $-S-CF_3$, $-S-CHF_2$, $-S-CH_2F$, -SH, $-S-CH_3$, $-S-C_2H_5$,$-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, -C(=O)-O-$CH_3$, -C(=O)-O-$C_2H_5$, -C(=O)-O-C($CH_3$)$_3$, -O-C(=O)-$CH_3$, -O-C(=O)-$C_2H_5$, -O-C(=O)-Phenyl, -($CH_2$)-O-C(=O)-$CH_3$, -($CH_2$)-O-C(=O)-$C_2H_5$,-($CH_2$)-O-C(=O)-Phenyl, $-N(CH_3)_2$, $-N(C_2Hs)_2$, -N(H)($CH_3$), -N(H)($C_2H_5$),-NH-C (=O)-O-$CH_3$, -NH-C(=O)-O-$C_2H_5$, -NH-C(=O)-$CH_3$, -NH-C(=O)-$C_2H_5$, -C(=O)-H, -C(=O)-$CH_3$, -C (=O)-$C_2H_5$, -C(=O)-$NH_2$, -C(=O)-NH-$CH_3$, -C(=O)-NH-$C_2H_5$, -S(=O)$_2$-$NH_2$, -S(=O)$_2$-NH-$CH_3$, -S(=O)$_2$-NH-$C_2H_5$, -S(=O)$_2$-NH-Phenyl, -($CH_2$)-Berlzo[b]furanyl, -O-Phenyl, -S-Phenyl,- S-Benzyl, -O-Benzyl, Phenyl und Benzyl substituiert sein kann, wobei jeweils der zyklische Teil der Reste -O-C(=O)-Phenyl, -($CH_2$)-O-C(=O)-Phenyl, -S(=O)$_2$-NH-Phenyl, -O-Phenyl, -S-Phenyl, -S-Benzyl,-O-Benzyl, Phenyl, -($CH_2$)-Benzo[b]

furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

für $-(CH_2)_m-O-C(=O)-R^5$ mit m = 1, 2, 3, 4 oder 5 steht;

für $-(CH_2)_n-C(=O)-O-R^6$ mit n = 1, 2, 3, 4 oder 5 steht;

für $-(CH_2)-R^{22}$ steht;

oder für $-(CH_2)-O-R^{23}$, $-(CH_2)-S-R^{23}$, $-(CH_2)-N(CH_3)-R^{23}$, $-(CH_2)-(CH_2)-O-R^{23}$, $-(CH_2)-(CH_2)-S-R^{23}$, $-(CH_2)-(CH_2)-NH-R^{23}$, $-(CH_2)-(CH_2)-N(CH_3)-R^{23}$, $-(CH_2)-(CH_2)-O-(CH_2)-R^{23}$, $-(CH_2)-(CH_2)-S-(CH_2)-R^{23}$ oder $-(CH_2)-(CH_2)-N(CH_3)-R^{23}$ steht;

$R^2$ für einen Wasserstoff-Rest steht;

für $-(CH_2)-R^{24}$, $-(CH_2)-O-R^{24}$, $-(CH_2)-S-R^{24}$, $-(CH_2)-N(CH_3)-R^{24}$, $-(CH_2)-(CH_2)-R^{24}$, $-(CH_2)-(CH_2)-O-R^{24}$, $-(CH_2)-(CH_2)-S-R^{24}$, $-(CH_2)-(CH_2)-NH-R^{24}$, $-(CH_2)-(CH_2)-N(CH_3)-R^{24}$, $-(CH_2)-(CH_2)-(CH_2)-R^{24}$, $-(CH_2)-(CH_2)-O-(CH_2)-R^{24}$, $-(CH_2)-(CH_2)-S-(CH_2)-R^{24}$ oder $-(CH_2)-(CH_2)-N(CH_3)-R^{24}$ steht;

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, $-(CH_2)-(CH_2)-(C(CH_3)_3)$, n-Hexyl, n-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl, 3-Butenyl, 2-Methyl-2-butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, $-N(CH_3)_2$, $-N(C_2H_5)_2$ und $-N(CH_3)(C_2H_5)$ substituiert sein kann;

für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl steht, wobei der Rest jeweils mit 1, 2, 3, 4 oder 5 Substituenten unabhängig voneinander ausgewählt aus der Gruppe bestehend aus F, Cl, Br, I,-CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$,$-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-Phenyl, $-(CH_2)-O-C(=O)-CH_3$, $-(CH_2)-O-C(=O)-C_2H_5$, $-(CH_2)$-O-C(=O)-Phenyl, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, $-(CH_2)$-O-C(=O)-Phenyl,$-S(=O)_2$-NH-Phenyl, -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

$R^3$ für eine $-C(=O)-R^4$-Gruppe steht;

und

$R^4$ für $-(CHR^{10})-O-R^{11}$, $-(CHR^{10})-S-R^{11}$, $-(CHR^{10})-NH-R^{11}$, $-(CHR^{10})-O-(CH_2)-R^{11}$, $-(CHR^{10})-S-(CH_2)-R^{11}$, $-(CHR^{10})-NH-(CH_2)-R^{11}$, $-(CHR^{10})-N(CH_3)-(CH_2)-R_{11}$, $-(CHR^{10})-(CH_2)-(CH_2)-O-R^{11}$, $-(CHR^{10})-(CH_2)-(CH_2)-S-R^{11}$, $-(CHR^{10})-(CH_2)-(CH_2)-NH-R^{11}$ $-(CHR^{10})-(CH_2)-(CH_2)-N(C_2H_5)-R^{11}$, $-(CHR^{10})-O-(CH_2)-(CH_2)-O-R^{11}$, $-(CHR^{10})-S-(CH_2)-(CH_2)-S-R^{11}$,$-(CHR^{10})-O-(CH_2)-(CH_2)-S-R^{11}$ oder $-(CHR^{10})-S-(CH_2)-(CH_2)-O-R^{11}$ steht,

für $-(CR^{12}R^{13})-C(=O)-O-R^7$, $-(CR^{12}R^{13})-(CH_2)-C(=O)-O-R^7$ oder-$(CR^{12}R^{13})-(CH_2)-(CH_2)-C(=O)-O-R^7$ steht;

für $-(CR^{14}R^{15})-O-C(=O)-R^8$ oder $-(CR^{14}R^{15})-(CH_2)-O-C(=O)-R^8$ steht;

für $-(CHR^{16})-C(=O)-R^9$ oder $-(CHR^{16})-(CH_2)-C(=O)-R^9$ steht;

für $-CH[(CH_2)-O-(CH_2)-R^{17}][NH-C(=O)-O-R^{18}]$ oder $-CH[(CH_2)-S-(CH_2)-R^{17}][NH-C(=O)-O-R^{18}]$ steht;

für $-CHR^{19}R^{20}$ oder $-(CH_2)-CHR^{19}R^{20}$ steht;

für $-(CH=CH)-R^{21}$ steht;

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Pro-

penyl, 2-Propenyl, 1-Butenyl, 2-Butenyl und 3-Butenyl steht, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$,- $N(C_2H_5)_2$ und $-N(CH_3)(C_2H_5)$ substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und 7,7-Dimethyl-2-oxa-bicyclo [2.2.1]heptyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$,-OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, Methyl, Ethyl, n-Propyl, Isopropyl, n-Butyl, sec-Butyl, Isobutyl, tert-Butyl, -C(=O)-H, -C (=O)-$CH_3$, -C(=O)-$C_2H_5$,-C(=O)-OH, -C(=O)-O-$CH_3$, -C(=O)-O-$C_2H_5$, -C(=O)-O-$C(CH_3)_3$, -($CH_2$)-C (=O)-OH, -($CH_2$)-C(=O)-O-$CH_3$, -($CH_2$)-C(=O)-O-$C_2H_5$, $-N(CH_3)_2$,$-N(C_2H_5)_2$, -N(H)($CH_3$), -N(H)($C_2H_5$), -($CH_2$)-Benzo[b]furanyl, -O-Phenyl,-O-Benzyl, Phenyl, Benzyl, Naphthyl und -($CH_2$)-Naphthyl substituiert sein kann, wobei jeweils der zyklische Teil der Reste -O-Phenyl, -O-Benzyl, Phenyl, -($CH_2$)-Benzo[b]furanyl, Benzyl, Naphthyl und -($CH_2$)-Naphthyl mit 1, 2, 3, 4 oder 5 Substituenten unabhängig voneinander ausgewählt aus der Gruppe bestehend aus F, Cl, Br, -OH, $-CF_3$, $-SF_5$,-CN, $-NO_2$, $-O-CH_3$, $-O-C_2H_5$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$,$-O-CHF_2$, $-O-CH_2F$, $-S-CF_3$, $-S-CHF_2$, $-S-CH_2F$, -SH, $-S-CH_3$, $-S-C_2H_5$,$-S-CH(CH_3)_2$, -S-C ($CH_3$)$_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, -C(=O)-O-$CH_3$, -C(=O)-O-$C_2H_5$, -C(=O)-O-$C(CH_3)_3$, -O-C(=O)-$CH_3$, -O-C(=O)-$C_2H_5$, -O-C (=O)-Phenyl, -($CH_2$)-O-C(=O)-$CH_3$, -($CH_2$)-O-C(=O)-$C_2H_5$,-($CH_2$)-O-C(=O)-Phenyl, $-N(CH_3)_2$, $-N(C_2H_5)_2$, -N(H)($CH_3$), -N(H)($C_2H_5$),-NH-C(=O)-O-$CH_3$, -NH-C(=O)-O-$C_2H_5$, -NH-C(=O)-$CH_3$, -NH-C(=O)-$C_2H_5$, -C (=O)-H, -C(=O)-$CH_3$, -C(=O)-$C_2H_5$, -C(=O)-$NH_2$, -C(=O)-NH-$CH_3$, -C(=O)-NH-$C_2H_5$, -S(=O)$_2$-$NH_2$, -S (=O)$_2$-NH-$CH_3$, -S(=O)$_2$-NH-$C_2H_5$, -S(=O)$_2$-NH-Phenyl, -($CH_2$)-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, -($CH_2$)-O-C(=O)-Phenyl, -S(=O)$_2$-NH-Phenyl, -O-Phenyl, -S-Phenyl, -S-Benzyl,-O-Benzyl, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, -O-$CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

oder II.)

$R^1$ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -($CH_2$)-($CH_2$)-($C(CH_3)_3$), n-Hexyl, n-Heptyl, n-Octyl, -($CH_2$)-(CH)($C_2H_5$)-($CH_2$)-($CH_2$)-($CH_2$)-($CH_3$), Vinyl, 1-Propenyl, 2-Propenyl, 1-Butenyl, 2-Butenyl, 3-Butenyl, 2-Methyl-2-butenyl, 1-Propinyl, 2-Propinyl, 1-Butinyl, 2-Butinyl und 3-Butinyl steht;
$R^2$ oder für einen unsubstituierten Phenyl- oder Benzyl-Rest steht;
für einen Wasserstoff-Rest steht;
für -($CH_2$)-$R^{24}$, -($CH_2$)-O-$R^{24}$, -($CH_2$)-S-$R^{24}$, -($CH_2$)-N($CH_3$)-$R^{24}$, -($CH_2$)-($CH_2$)-$R^{24}$, -($CH_2$)-($CH_2$)-O-$R^{24}$, -($CH_2$)-($CH_2$)-S-$R^{24}$, -($CH_2$)-($CH_2$)-NH-$R^{24}$, -($CH_2$)-($CH_2$)-N($CH_3$)-$R^{24}$, -($CH_2$)-($CH_2$)-($CH_2$)-$R^{24}$, -($CH_2$)-($CH_2$)-O-($CH_2$)-$R^{24}$, -($CH_2$)-($CH_2$)-S-($CH_2$)-$R^{24}$ oder -($CH_2$)-($CH_2$)-N($CH_3$)-$R^{24}$ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, -($CH_2$)-($CH_2$)-($C(CH_3)_3$), n-Hexyl, n-Heptyl, n-Octyl, -($CH_2$)-(CH)($C_2H_5$)-($CH_2$)-($CH_2$)-($CH_2$)-($CH_3$), Vinyl, 1-Propenyl, 2-Propenyl, 1-Butenyl, 2-Butenyl, 3-Butenyl, 2-Methyl-2-butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I,-CN, $-NO_2$, -OH, -SH, $-NH_2$, $-N(CH_3)_2$, $-N(C_2H_5)_2$ und $-N(CH_3)(C_2H_5)$ substituiert sein kann;

oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und Isochinolinyl steht, wobei der Rest jeweils mit 1, 2, 3, 4 oder 5 Substituenten unabhängig voneinander ausgewählt aus der Gruppe bestehend aus F, Cl, Br, I,-CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$,$-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$,- $C(=O)-O-C(CH_3)_3$, $-O-C(=O)-CH_3$, $-O-C(=O)-C_2H_5$, -O-C(=O)-Phenyl, $-(CH_2)-O-C(=O)-CH_3$, $-(CH_2)-O-C(=O)-C_2H_5$, $-(CH_2)$-O-C(=O)-Phenyl, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, $-(CH_2)$-O-C(=O)-Phenyl,$-S(=O)_2$-NH-Phenyl, -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

$R^3$ für eine $-C(=O)-R^4$-Gruppe steht;

und

$R^4$ für $-(CHR^{10})-O-R^{11}$, $-(CHR^{10})-S-R^{11}$, $-(CHR^{10})-NH-R^{11}$, $-(CHR^{10})-O-(CH_2)-R^{11}$, $-(CHR^{10})-S-(CH_2)-R^{11}$, $-(CHR^{10})-NH-(CH_2)-R^{11}$, $-(CHR^{10})-N(CH_3)-(CH_2)-R^{11}$, $-(CHR^{10})-(CH_2)-(CH_2)-O-R^{11}$, $-(CHR^{10})-(CH_2)-(CH_2)-S-R^{11}$, $-(CHR^{10})-(CH_2)-(CH_2)-NH-R^{11}$ $-(CHR^{10})-(CH_2)-(CH_2)-N(C_2H_5)-R^{11}$, $-(CHR^{10})-O-(CH_2)-(CH_2)-O-R^{11}$, $-(CHR^{10})-S-(CH_2)-(CH_2)-S-R^{11}$,$-(CHR^{10})-O-(CH_2)-(CH_2)-S-R^{11}$ oder $-(CHR^{10})-S-(CH_2)-(CH_2)-O-R^{11}$ steht,

für $-(CR^{12}R^{13})-C(=O)-O-R^7$, $-(CR^{12}R^{13})-(CH_2)-C(=O)-O-R^7$ oder-$(CR^{12}R^{13})-(CH_2)-(CH_2)-C(=O)-O-R^7$ steht;

für $-(CR^{14}R^{15})-O-C(=O)-R^8$ oder $-(CR^{14}R^{15})-(CH_2)$ $-O-C(=O)-R^8$ steht;

für $-(CHR^{16})-C(=O)-R^9$ oder $-(CHR^{16})-(CH_2)-C(=O)-R^9$ steht;

für $-CH[(CH_2)-O-(CH_2)-R^{17}][NH-C(=O)-O-R^{18}]$ oder $-CH[(CH_2)-S-(CH_2)-R^{17}][NH-C(=O)-O-R^{18}]$ steht;

für $-CHR^{19}R^{20}$ oder $-(CH_2)-CHR^{19}R^{20}$ steht;

für $-(CH=CH)-R^{21}$ steht;

für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl und $-(CH_2)-(CH)(C_2H_5)-(CH_2)-(CH_2)-(CH_2)-(CH_3)$, wobei der Alkyl-Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert ist;

für einen Rest ausgewählt aus der Gruppe bestehend aus Vinyl, 1-Propenyl, 2-Propenyl, 1-Butenyl, 2-Butenyl, 3-Butenyl, 1-Propinyl, 2-Propinyl, 1-Butinyl, 2-Butinyl und 3-Butinyl steht, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, $-N(CH_3)_2$, $-N(C_2H_5)_2$ und $-N(CH_3)(C_2H_5)$ substituiert sein kann;

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und 7,7-Dimethyl-2-oxa-bicyclo[2.2.1]heptyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$,-OH,- $O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, Methyl, Ethyl, n-Propyl, Isopropyl, n-Butyl, sec-Butyl, Isobutyl, tert-Butyl, -C(=O)-H, $-C(=O)-CH_3$, $-C(=O)-C_2H_5$,-C(=O)-OH, $-C(=O)-O-CH_3$, $-C(=O)-O-C_2H_5$, $-C(=O)-O-C(CH_3)_3$, $-(CH_2)-C(=O)-OH$, $-(CH_2)-C(=O)-O-CH_3$, $-(CH_2)-C(=O)-O-C_2H_5$, $-N(CH_3)_2$,$-N(C_2H_5)_2$, $-N(H)(CH_3)$, $-N(H)(C_2H_5)$, $-(CH_2)$-Benzo[b]furanyl, -O-Phenyl,-O-Benzyl, Phenyl, Benzyl, Naphthyl und $-(CH_2)$-Naphthyl substituiert sein kann, wobei jeweils der zyklische Teil der Reste -O-Phenyl, -O-Benzyl, Phenyl, $-(CH_2)$-Benzo[b]furanyl, Benzyl, Naphthyl und $-(CH_2)$-Naphthyl mit 1, 2, 3, 4 oder 5 Substituenten unabhängig voneinander ausgewählt aus der Gruppe bestehend aus F, Cl, Br, -OH, $-CF_3$, $-SF_5$,-CN, $-NO_2$, $-O-CH_3$, $-O-C_2H_5$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

für einen Phenyl-Rest steht, wobei der Phenyl-Rest jeweils mit 1, 2, 3, 4 oder 5 Substituenten unabhängig voneinander ausgewählt aus der Gruppe bestehend aus -CN, $-SF_5$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$,$-S-C(CH_3)_3$, $-S-CH_2-CH_2-CH_2-CH_3$, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-Phenyl, $-(CH_2)-O-C(=O)-CH_3$, $-(CH_2)-O-C(=O)-C_2H_5$, $-(CH_2)$-O-C(=O)-Phenyl, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert ist, wobei jeweils der zyklische Teil der Reste -O-C(=O)-Phenyl, $-(CH_2)$-O-C(=O)-Phenyl, $-S(=O)_2$-NH-Phenyl, -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

für einen Rest ausgewählt aus der Gruppe bestehend aus 1,2,3,4,5-Pentafluorophenyl, 1,2,3,4-Tetrafluorophenyl, 4-Methyl-3-nitro-phenyl, 5-Methyl-3-nitro-phenyl, 6-Methyl-3-nitro-phenyl und 2-Methyl-3-nitrophenyl steht;

für einen unsubstituierten Naphthyl-Rest steht;

für einen Rest ausgewählt aus der Gruppe bestehend aus (1,3)-Benzodioxolyl und (1,4)-Benzodioxanyl steht, wobei der Rest jeweils mit 1, 2, 3, 4 oder 5 Substituenten ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$,$-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl substituiert sein kann;

oder für einen Heteroaryl-Rest ausgewählt aus der Gruppe bestehend aus Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Heteroaryl-Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$,$-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl,-C(=O)-OH, $-C(=O)-O-CH_3$, $-C(=O)-O-C_2H_5$, $-C(=O)-O-C(CH_3)_3$, $-O-C(=O)-CH_3$,- $O-C(=O)-C_2H_5$, -O-C(=O)-Phenyl, $-(CH_2)-O-C(=O)-CH_3$,$-(CH_2)-O-C(=O)-C_2H_5$, $-(CH_2)$-O-C(=O)-Phenyl, $-N(CH_3)_2$, $-N(C_2H_5)_2$,$-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$,$-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl, substituiert sein kann, wobei jeweils der zyklische Teil der Reste -O-C(=O)-Phenyl, $-(CH_2)$-O-C(=O)-Phenyl, $-S(=O)_2$-NH-Phenyl, -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$,-CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

oder III.)

$R^1$ für einen Wasserstoff-Rest steht;

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, $-(CH_2)-(CH_2)-(C(CH_3)_3)$, n-Hexyl, n-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl, 3-Butenyl, 2-Methyl-2-butenyl, 1-Propinyl, 2-Propinyl, 1-Butinyl, 2-Butinyl und 3-Butinyl steht;

für einen Rest ausgewählt aus der Gruppe bestehend aus Phenyl, Napthtyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrimidinyl, Indazolyl, Chinazolinyl, Chinolinyl und

Isochinolinyl steht, wobei der Heteroaryl-Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$,$-O-CHF_2$, $-O-CH_2F$, $-S-CF_3$, $-S-CHF_2$, $-S-CH_2F$, -SH, $-S-CH_3$, $-S-C_2H_5$,$-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-Phenyl, $-(CH_2)-O-C(=O)-CH_3$, $-(CH_2)-O-C(=O)-C_2H_5$,$-(CH_2)$-O-C(=O)-Phenyl, $-N(CH_3)_2$, $-N(C_2H_5)_2$, $-N(H)(CH_3)$, $-N(H)(C_2H_5)$,$-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, $-(CH_2)$-O-C(=O)-Phenyl, $-S(=O)_2$-NH-Phenyl, -O-Phenyl, -S-Phenyl, -S-Benzyl,-O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

für $-(CH_2)_m-O-C(=O)-R^5$ mit m = 1, 2, 3, 4 oder 5 steht;

für $-(CH_2)_n-C(=O)-O-R^6$ mit n = 1, 2, 3, 4 oder 5 steht;

für einen unsubsituierten Benzyl-Rest steht;

für $-(CH_2)-R^{22}$ steht;

oder für $-(CH_2)-O-R^{23}$, $-(CH_2)-S-R^{23}$, $-(CH_2)-N(CH_3)-R^{23}$, $-(CH_2)-(CH_2)-O-R^{23}$, $-(CH_2)-(CH_2)-S-R^{23}$, $-(CH_2)-(CH_2)-NH-R^{23}$, $-(CH_2)-(CH_2)-N(CH_3)-R^{23}$, $-(CH_2)-(CH_2)-O-(CH_2)-R^{23}$, $-(CH_2)-(CH_2)-S-(CH_2)-R^{23}$ oder $-(CH_2)-(CH_2)-N(CH_3)-R^{23}$ steht;

$R^2$ für $-(CH_2)-S-R^{24}$, $-(CH_2)-N(CH_3)-R^{24}$, $-(CH_2)-(CH_2)-S-R^{24}$, $-(CH_2)-(CH_2)-NH-R^{24}$, $-(CH_2)-(CH_2)-N(CH_3)-R^{24}$, $-(CH_2)-(CH_2)-S-(CH_2)-R^{24}$ oder$-(CH_2)-(CH_2)-N(CH_3)-R^{24}$ steht;

$R^3$ für eine $-C(=O)-R^4$-Gruppe steht;

und

$R^4$ für $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$, $-(CHR^{10})-(CH_2)-(CH_2)-O-R^{11}$, $-(CHR^{10})-(CH_2)-(CH_2)-S-R^{11}$ oder $-(CHR^{10})-O-(CH_2)-(CH_2)-O-R^{11}$ steht,

für $-(CR^{12}R^{13})-C(=O)-O-R^7$ $ode_r$ $-(CR^{12}R^{13})-(CH_2)-C(=O)-O-R^7$ steht;

für $-(CR^{14}R^{15})-O-C(=O)-R^8$ oder $-(CR^{14}R^{15})-(CH_2)$ $-O-C(=O)-R^8$ steht;

für $-(CHR^{16})-C(=O)-R^9$ steht;

für $-CH[(CH_2)-O-(CH_2)-R^{17}][NH-C(=O)-O-R^{18}]$ steht;

für $-CHR^{19}R^{20}$ oder $-(CH_2)-CHR^{19}R^{20}$ steht;

für $-(CH=CH)-R^{21}$ steht;

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl steht, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert sein kann;

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Adamantyl und 7,7-Dimethyl-2-oxa-bicyclo[2.2.1]heptyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$,-OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, Methyl, Ethyl, n-Propyl, Isopropyl, n-Butyl, sec-Butyl, Isobutyl, tert-Butyl, -C(=O)-H, $-C(=O)-CH_3$, $-C(=O)-C_2H_5$,-C(=O)-OH, $-C(=O)-O-CH_3$, $-C(=O)-O-C_2H_5$, $-C(=O)-O-C(CH_3)_3$, $-(CH_2)-C(=O)-OH$, $-(CH_2)-C(=O)-O-CH_3$, $-(CH_2)-C(=O)-O-C_2H_5$, $-N(CH_3)_2$,$-N(C_2H_5)_2$, $-N(H)(CH_3)$, $-N(H)(C_2H_5)$, $-(CH_2)$-Benzo[b]furanyl, -O-Phenyl,-O-Benzyl, Phenyl, Benzyl, Naphthyl und $-(CH_2)$-Naphthyl substituiert sein kann, wobei jeweils der zyklische Teil der Reste -O-Phenyl, -O-Benzyl, Phenyl, $-(CH_2)$-Benzo[b]furanyl, Benzyl, Naphthyl und $-(CH_2)$-Naphthyl mit 1, 2, 3, 4 oder 5 Substituenten unabhängig voneinander ausgewählt aus der Gruppe bestehend aus F, Cl, Br, -OH, $-CF_3$, $-SF_5$,-CN, $-NO_2$, $-O-CH_3$, $-O-C_2H_5$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$,$-O-CHF_2$, $-O-CH_2F$, $-S-CF_3$, $-S-CHF_2$, $-S-CH_2F$, -SH, $-S-CH_3$, $-S-C_2H_5$,$-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-Phenyl, $-(CH_2)-O-C(=O)-CH_3$, $-(CH_2)-O-C(=O)-C_2H_5$,$-(CH_2)-O-C(=O)$-Phenyl, $-N(CH_3)_2$, $-N(C_2H_5)_2$, $-N(H)(CH_3)$, $-N(H)(C_2H_5)$,$-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, $-(CH_2)-O-C(=O)$-Phenyl, $-S(=O)_2$-NH-Phenyl, -O-Phenyl, -S-Phenyl, -S-Benzyl,-O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

und jeweils - sofern vorhanden - in den vorstehend genannten Gruppen I.) II.) und III.) die Reste

$R^5$, $R^6$, $R^9$ und $R^{18}$ unabhängig voneinander jeweils

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl stehen, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert sein kann;

$R^7$ für einen Wasserstoff-Rest steht;

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl steht, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$, $-N(CH_3)_2$,$-N(C_2H_5)_2$ und $-N(CH_3)(C_2H_5)$ substituiert sein kann;

oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$,$-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl,-C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-Phenyl, $-(CH_2)-O-C(=O)-CH_3$,$-(CH_2)-O-C(=O)-C_2H_5$, $-(CH_2)-O-C(=O)$-Phenyl, $-N(CH_3)_2$, $-N(C_2H_5)_2$,$-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$,$-C(=O)-NH_2$,- $C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-Phenyl, $-(CH_2)$-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, $-(CH_2)-O-C(=O)$-Phenyl, $-S(=O)_2$-NH-Phenyl,-O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

$R^8$, $R^{16}$ und $R^{24}$ unabhängig voneinander jeweils

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl stehen, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert sein kann;

oder für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl, Chinolinyl, Isochinolinyl, Phenazinyl, Phenothiazinyl, Oxadiazolyl, Benzo[1,2,5]oxadiazolyl und Chromanyl stehen, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$,- $NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$,$-C(=O)-O-C(CH_3)_3$, $-O-C(=O)-CH_3$, $-O-C(=O)-C_2H_5$, -O-C(=O)-Phenyl,$-(CH_2)-O-C(=O)-CH_3$, $-(CH_2)-O-C(=O)-C_2H_5$, $-(CH_2)$-O-C(=O)-Phenyl,$-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=0)-NH_2$, $-C(=O)-NH-CH_3$, $-C(=O)-NH-C_2H_5$,$-S(=O)_2-NH_2$, $-S(=O)_2-NH-CH_3$, $-S(=O)_2-NH-C_2H_5$, $-S(=O)_2$-NH-Phenyl,$-(CH_2)$-Benzo[b]furanyl, -O-Phenyl, -S-Phenyl, -S-Benzyl, -O-Benzyl, Phenyl und Benzyl substituiert sein kann, wobei jeweils der zyklische Teil der Reste -O-C(=O)-Phenyl, $-(CH_2)$-O-C(=O)-Phenyl, $-S(=O)_2$-NH-Phenyl, -O-Phenyl, -S-Phenyl, -S-Benzyl, -O-Benzyl, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, Methyl, Ethyl, n-Propyl, Isopropyl, n-Butyl, sec-Butyl, Isobutyl, tert-Butyl, $-O-CF_3$, $-S-CF_3$, Phenyl und -O-Benzyl substituiert sein kann,

$R^{10}$, $R^{12}$ und $R^{13}$ unabhängig voneinander jeweils

für einen Wasserstoff-Rest stehen

oder für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl stehen, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert sein kann;

$R^{11}$ für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl steht, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert sein kann;

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Pyrrolidinyl, Piperidinyl, Morpholinyl, Piperazinyl, Azepanyl und Diazepanyl steht, wobei der (hetero)cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, Methyl, Ethyl, n-Propyl, Isopropyl, n-Butyl, sec-Butyl, Isobutyl, tert-Butyl, -C(=O)-H, $-C(=O)-CH_3$, $-C(=O)-C_2H_5$, -C(=O)-OH, $-C(=O)-O-CH_3$,$-C(=O)-O-C_2H_5$ und $-C(=O)-O-C(CH_3)_3$ substituiert sein kann;

oder für einen Phenyl- oder Naphthyl-Rest steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl und Phenyl substituiert sein kann;

$R^{14}$, $R^{15}$ und $R^{19}$ unabhängig voneinander jeweils

für einen Wasserstoff-Rest stehen;

für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, n-Octyl, $-(CH_2)-(CH)(C_2H_5)-(CH_2)-(CH_2)-(CH_2)-(CH_3)$, Vinyl, 1-Propenyl, 2-Propenyl, 1-Butenyl, 2-Butenyl und 3-Butenyl stehen, wobei der Rest jeweils mit 1, 2, 3, 4 oder 5 Substituenten unabhängig voneinander ausgewählt aus der Gruppe bestehend aus F, Cl, Br, I, -CN, $-NO_2$, -OH, -SH, $-NH_2$,$-N(CH_3)_2$, $-N(C_2H_5)_2$ und $-N(CH_3)(C_2H_5)$ substituiert sein kann;

oder für einen Phenyl- oder Naphthyl-Rest stehen, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl und tert-Butyl substituiert sein kann;

$R^{20}$ für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Pyrrolidinyl, Piperidinyl, Morpholinyl, Piperazinyl, Azepanyl und Diazepanyl steht, wobei der (hetero) cycloaliphatische Rest jeweils mit 1, 2, 3, 4 oder 5 Substituenten unabhängig voneinander ausgewählt aus der Gruppe bestehend aus Oxo (=O), Thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, Methyl, Ethyl, n-Propyl, Isopropyl, n-Butyl, sec-Butyl, Isobutyl, tert-Butyl, -C(=O)-H, $-C(=O)-CH_3$, $-C(=O)-C_2H_5$, -C(=O)-OH, $-C(=O)-O-CH_3$,$-C(=O)-O-C_2H_5$ und $-C(=O)-O-C(CH_3)_3$ substituiert sein kann;

oder für einen Phenyl- oder Naphthyl-Rest steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl und Phenyl substituiert sein kann;

$R^{17}$ und $R^{21}$ unabhängig voneinander jeweils

für einen Phenyl- oder Naphthyl-Rest stehen, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl und tert-Butyl substituiert sein kann;

$R^{22}$ für einen Phenyl-Rest steht, wobei der Phenyl-Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, $-N(CH_3)_2$, $-N(C_2H_5)_2$,$-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$,$-C(=O)-NH_2$,- $C(=O)-NH-CH_3$, $-C(=O)-NH-C_2H_5$, $-S(=O)_2-NH_2$, $-S(=O)_2-NH-CH_3$ und $-S(=O)_2-NH-C_2H_5$ substituiert ist,

für einen Naphtyl-Rest steht, wobei der Naphtyl-Rest mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$,$-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl und tert-Butyl substituiert sein kann;

oder für einen Heteroaryl-Rest ausgewählt aus der Gruppe bestehend aus Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl und Chinolinyl steht, wobei der Heteroaryl-Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$,$-N0_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl,-C(=O)-OH, $-C(=O)-O-CH_3$, $-C(=O)-O-C_2H_5$ und $-C(=O)-O-C(CH_3)_3$ substituiert sein kann,

und

$R^{23}$ für einen Rest ausgewählt aus der Gruppe bestehend aus Phenyl, Naphthyl, Thiophenyl, Furanyl, Pyrrolyl, Pyrazolyl, Pyranyl, Triazolyl, Pyridinyl, Imidazolyl, Indolyl, Isoindolyl, Benzo[b]furanyl, Benzo[b]thiophenyl, Thiazolyl, Thiadiazolyl, Oxazolyl, Isoxazolyl, Pyridazinyl, Pyrazinyl, Pyrimidinyl, Indazolyl, Chinazolinyl und Chinolinyl steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$,$-O-CHF_2$, $-O-CH_2F$, $-S-CF_3$, $-S-CHF_2$, $-S-CH_2F$, -SH, $-S-CH_3$, $-S-C_2H_5$,$-S-CH(CH_3)_2$, $-S-C(CH_3)_3$, $-S-CH_2-CH_2-CH_2-CH_3$, Methyl, Ethyl, n-Propyl, Isopropyl, n-Butyl, sec-Butyl, Isobutyl, tert-Butyl, -C(=O)-OH, $-C(=O)-O-CH_3$, $-C(=O)-O-C_2H_5$ und $-C(=O)-O-C(CH_3)_3$ substituiert sein kann.

**12.** Verbindungen gemäß Anspruch 10 oder 11, **dadurch gekennzeichnet, dass**

I.)

$R^1$ für $-(CH_2)_m-O-C(=O)-R^5$ mit m = 1, 2, 3 oder 4 steht;
für $-(CH_2)_n-C(=O)-O-R^6$ mit n = 1, 2 oder 3 steht;
für $-(CH_2)-R^{22}$ steht;
oder für $-(CH_2)-(CH_2)-O-R^{23}$, $-(CH_2)-(CH_2)-S-R^{23}$, $-(CH_2)-(CH_2)-NH-R^{23}$ oder $-(CH_2)-(CH_2)-N(CH_3)-R^{23}$ steht;
$R^2$ für einen Wasserstoff-Rest steht;
für$-(CH_2)-R^{24}$, $-(CH_2)-(CH_2)-R^{24}$, $-(CH_2)-(CH_2)-O-R^{24}$, $-(CH_2)-(CH_2)-S-R^{24}$ oder $-(CH_2)-(CH_2)-(CH_2)-R^{24}$ steht;
für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl und sec-Pentyl steht;
$R^3$ für eine $-C(=O)-R^4$-Gruppe steht;

und

$R^4$ für $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$, $-(CHR^{10})-(CH_2)-(CH_2)-O-R^{11}$, $-(CHR^{10})-(CH_2)-(CH_2)-S-R^{11}$ oder $-(CHR^{10})-O-(CH_2)-(CH_2)-O-R^{11}$ steht,
für $-(CR^{12}R^{13})-C(=O)-O-R^7$ oder$-(CR^{12}R^{13})-(CH_2)-C(=O)-O-R^7$ steht;
für $-(CR^{14}R^{15})-O-C(=O)-R^8$ oder $-(CR^{14}R^{15})-(CH_2)$ $-O-C(=O)-R^8$ steht;
für $-(CHR^{16})-C(=O)-R^9$ steht;
für $-CH[(CH_2)-O-(CH_2)-R^{17}][NH-C(=O)-O-R^{18}]$ steht;
für $-CHR^{19}R^{20}$ oder $-(CH_2)-CHR^{19}R^{20}$ steht;
für $-(CH=CH)-R^{21}$ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, $-(CH_2)-(C(CH_3)_3)$, n-Hexyl, 3-Hexyl, $-(CH_2)-(CH_2)-(C(CH_3)_3)$, n-Heptyl, 3-Heptyl, 4-Heptyl, $-(CH_2)-N(CH_3)_2$, $-(CH_2)-(CH_2)-N(CH_3)_2$, 1-Butenyl, 2-Butenyl und 3-Butenyl steht;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Adamantyl und 7,7-Dimethyl-3-oxo-2-oxa-bicyclo[2.2.1]heptyl steht, wobei der cycloaliphatische Rest jeweils mit 1, 2, 3, 4 oder 5 Substituenten Methyl, Ethyl und n-Propyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrazolyl, Triazolyl, Pyridinyl, Imidazolyl, Indolyl, Thiazolyl, Thiadiazolyl, Oxazolyl, Isoxazolyl, Pyrimidinyl, Oxadiazolyl, Benzo[1,2,5]oxadiazolyl und Chromanyl steht, wobei der Rest jeweils mit 1, 2, 3, 4 oder 5 Substituenten unabhängig voneinander ausgewählt aus der Gruppe bestehend aus F, Cl, Br, I, -CN, $-CF_3$, $-O-CH_3$, $-O-C_2H_5$, $-NO_2$, $-O-CF_3$, $-O-CHF_2$, $-O-CH_2F$, $-S-CF_3$, $-S-CHF_2$, $-S-CH_2F$, $-S-CH_3$,$-S-C_2H_5$, $-S-CH(CH_3)_2$, $-S-C(CH_3)_3$, Methyl, Ethyl, n-Propyl, Isopropyl, n-Butyl, sec-Butyl, Isobutyl, tert-Butyl, $-(CH_2)-O-C(=O)-CH_3$, $-(CH_2)-O-C(=O)-C_2H_5$, $-(CH_2)-O-C(=O)$-Phenyl, $-N(CH_3)_2$, $-N(C_2H_5)_2$, $-NH-C(=O)-CH_3$, $-NH-C(=O)-C_2H_5$, $-S(=O)_2-NH_2$, -O-Phenyl und Phenyl substituiert sein kann, wobei jeweils der zyklische Teil der Reste -O-Phenyl und Phenyl mit 1, 2, 3, 4 oder 5 Substituenten unabhängig voneinander ausgewählt aus der Gruppe bestehend aus F, Cl, Br, Methyl, Ethyl, n-Propyl und Isopropyl substituiert sein kann.

oder II.)

$R^1$ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, 1-Propenyl, 2-Propenyl, 1-Butenyl, 2-Butenyl, 3-Butenyl, 2-Methyl-2-butenyl, 1-Propinyl und 2-Propinyl steht;
oder für einen unsubstituierten Benzyl-Rest steht;
$R^2$ für einen Wasserstoff-Rest steht;
für $-(CH_2)-R^{24}$, $-(CH_2)-(CH_2)-R^{24}$, $-(CH_2)-(CH_2)-O-R^{24}$, $-(CH_2)-(CH_2)-S-R^{24}$ oder $-(CH_2)-(CH_2)-(CH_2)-R^{24}$ steht;
oder für einen Alkyl-Rest ausgewählt aus der Gruppe bestehend aus Methyl, Ethyl, n-Propyl, Isopropyl, n-Butyl, sec-Butyl, Isobutyl, tert-Butyl, n-Pentyl und sec-Pentyl steht;
$R^3$ für eine $-C(=O)-R^4$-Gruppe steht;

und

$R^4$ für $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$, $-(CHR^{10})-(CH_2)-(CH_2)-O-R^{11}$, $-(CHR^{10})-(CH_2)-(CH_2)-S-R^{11}$ oder $-(CHR^{10})-O-(CH_2)-(CH_2)-O-R^{11}$ steht,
für $-(CR^{12}R^{13})-C(=O)-O-R^7$ oder $-(CR^{12}R^{13})-(CH_2)-C(=O)-O-R^7$ steht;
für $-(CR^{14}R^{15})-O-C(=O)-R^8$ oder $-(CR^{14}R^{15})-(CH_2)$ $-O-C(=O)-R^8$ steht;
für $-(CHR^{16})-C(=O)-R^9$ steht;
für $-CH[(CH_2)-O-(CH_2)-R^{17}][NH-C(=O)-O-R^{18}]$ steht;
für-$CHR^{19}R^{20}$ oder $-(CH_2)-CHR^{19}R^{20}$ steht;
für $-(CH=CH)-R^{21}$ steht;
für einen Rest ausgewählt aus der Gruppe bestehend aus $-(CH_2)-N(CH_3)_2$, $-(CH_2)-(CH_2)-N(CH_3)_2$, 1-Butenyl, 2-Butenyl und 3-Butenyl steht;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Adamantyl und 7,7-Dimethyl-3-oxo-2-oxa-bicyclo[2.2.1]heptyl steht, wobei der cycloaliphatische Rest jeweils mit 1, 2, 3, 4 oder 5 Substituenten Methyl, Ethyl und n-Propyl substituiert sein kann;
für einen Phenyl-Rest steht, wobei der Phenyl-Rest jeweils mit 1, 2, 3, 4 oder 5 Substituenten unabhängig voneinander ausgewählt aus der Gruppe bestehend aus -CN, $-NO_2$, $-O-CF_3$, $-O-CHF_2$, $-O-CH_2F$, $-S-CF_3$, $-S-CHF_2$, $-S-CH_2F$, $-(CH_2)-O-C(=O)-CH_3$, $-(CH_2)-O-C(=O)-C_2H_5$, $-(CH_2)-O-C(=O)$-Phenyl, $-N(CH_3)_2$, $-N(C_2H_5)_2$, $-N(H)(CH_3)$, $-N(H)(C_2H_5)$, $-NH-C(=O)-CH_3$, $-NH-C(=O)-C_2H_5$, $-S(=O)_2-NH_2$, $-S(=O)_2-NH-CH_3$, $-S(=O)_2-NH-C_2H_5$ und Phenyl substituiert ist, wobei jeweils der zyklische Teil der Reste $-(CH_2)-O-C(=O)$-Phenyl und Phenyl mit 1, 2, 3, 4 oder 5 Substituenten unabhängig voneinander ausgewählt aus der Gruppe bestehend aus F, Cl, Br, Methyl, Ethyl, n-Propyl, Isopropyl, n-Butyl, sec-Butyl, Isobutyl und tert-Butyl substituiert sein kann,
für einen Rest ausgewählt aus der Gruppe bestehend aus 1,2,3,4,5-Pentafluorophenyl, 1,2,3,4-Tetrafluorophenyl, 4-Methyl-3-nitro-phenyl, 5-Methyl-3-nitro-phenyl, 6-Methyl-3-nitro-phenyl und 2-Methyl-3-nitro-phenyl steht;
für einen unsubstituierten Naphthyl-Rest steht;
für einen unsubstituierten Rest ausgewählt aus der Gruppe bestehend aus (1,3)-Benzodioxolyl und (1,4)-Benzodioxanyl steht,
oder für einen Heteroaryl-Rest ausgewählt aus der Gruppe bestehend aus Thiophenyl, Furanyl, Pyrazolyl, Triazolyl, Pyridinyl, Imidazolyl, Indolyl, Thiazolyl, Thiadiazolyl, Oxazolyl, Isoxazolyl, Pyrimidinyl, Oxadiazolyl, Benzo[1,2,5]oxadiazolyl und Chromanyl steht, wobei der Heteroaryl-Rest jeweils mit 1, 2, 3, 4 oder 5 Substituenten unabhängig voneinander ausgewählt aus der Gruppe bestehend aus F, Cl, Br, I,-CN, $-CF_3$, $-O-CH_3$, $-O-C_2H_5$, $-NO_2$, $-O-CF_3$, $-O-CHF_2$, $-O-CH_2F$, $-S-CF_3$,$-S-CHF_2$, $-S-CH_2F$, $-S-CH_3$, $-S-C_2H_5$, $-S-CH(CH_3)_2$, $-S-C(CH_3)_3$, Methyl, Ethyl, n-Propyl, Isopropyl, n-Butyl, sec-Butyl, Isobutyl, tert-Butyl, -O-Phenyl und Phenyl substituiert sein kann, wobei jeweils der zyklische Teil der Reste -O-Phenyl und Phenyl mit 1, 2, 3, 4 oder 5 Substituenten unabhängig voneinander ausgewählt aus der Gruppe bestehend aus F, Cl, Br, Methyl, Ethyl, n-Propyl und Isopropyl substituiert sein kann;

oder III.)

$R^1$ für einen Wasserstoff-Rest steht;
für einen Rest ausgewählt aus der Gruppe bestehend aus Methyl, Ethyl, n-Propyl, Isopropyl, n-Butyl, sec-Butyl, Isobutyl, tert-Butyl, 1-Propenyl, 2-Propenyl, 1-Butenyl, 2-Butenyl, 3-Butenyl, 2-Methyl-2-butenyl, 1-Propinyl und 2-Propinyl steht;

für-$(CH_2)_m$-O-C(=O)-$R^5$ mit m = 1, 2, 3 oder 4 steht;
für -$(CH_2)_n$-C(=O)-O-$R^6$ mit n = 1, 2 oder 3 steht;
für -$(CH_2)_n$-C(=O)-O-$R^6$ mit n = 1, 2 oder 3 steht;
für einen unsubstituierten Benzyl-Rest steht;
für -$(CH_2)$-$R^{22}$ steht;
oder für -$(CH_2)$-$(CH_2)$-O-$R^{23}$, -$(CH_2)$-$(CH_2)$-S-$R^{23}$, -$(CH_2)$-$(CH_2)$-NH-$R^{23}$ oder -$(CH_2)$-$(CH_2)$-N$(CH_3)$-$R^{23}$ steht;
$R^2$ für -$(CH_2)$-$(CH_2)$-S-$R^{24}$ steht;
$R^3$ für eine -C(=O)-$R^4$-Gruppe steht;

und

$R^4$ für -$(CHR^{10})$-O-$R^{11}$, -$(CHR^{10})$-S-$R^{11}$, -$(CHR^{10})$-O-$(CH_2)$-$R^{11}$, -$(CHR^{10})$-S-$(CH_2)$-$R^{11}$, -$(CHR^{10})$-$(CH_2)$-$(CH_2)$-O-$R^{11}$, -$(CHR^{10})$-$(CH_2)$-$(CH_2)$-S-$R^{11}$ oder -$(CHR^{10})$-O-$(CH_2)$-$(CH_2)$-O-$R^{11}$ steht,
für -$(CR^{12}R^{13})$-C(=O)-O-$R^7$ oder -$(CR^{12}R^{13})$-$(CH_2)$-C(=O)-O-$R^7$ steht;
für -$(CR^{14}R^{15})$-O-C(=O)-$R^8$ oder -$(CR^{14}R^{15})$-$(CH_2)$ -O-C(=O)-$R^8$ steht;
für -$(CHR^{16})$-C(=O)-$R^9$ steht;
für -CH[$(CH_2)$-O-$(CH_2)$-$R^{17}$][NH-C(=O)-O-$R^{18}$] steht;
für -$CHR^{19}R^{20}$ oder -$(CH_2)$-$CHR^{19}R^{20}$ steht;
für -(CH=CH)-$R^{21}$ steht;
für einen Rest ausgewählt aus der Gruppe bestehend aus Methyl, Ethyl, n-Propyl, Isopropyl, n-Butyl, sec-Butyl, Isobutyl, tert-Butyl, n-Pentyl, 2-Pentyl, 3-Pentyl, -$(CH_2)$-$(C(CH_3)_3)$, n-Hexyl, 3-Hexyl, -$(CH_2)$-$(CH_2)$-$(C(CH_3)_3)$, n-Heptyl, 3-Heptyl, 4-Heptyl, -$(CH_2)$-$N(CH_3)_2$, -$(CH_2)$-$(CH_2)$-$N(CH_3)_2$, 1-Butenyl, 2-Butenyl und 3-Butenyl steht;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Adamantyl und 7,7-Dimethyl-3-oxo-2-oxa-bicyclo[2.2.1]heptyl steht, wobei der cycloaliphatische Rest jeweils mit 1, 2, 3, 4 oder 5 Substituenten Methyl, Ethyl und n-Propyl substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrazolyl, Triazolyl, Pyridinyl, Imidazolyl, Indolyl, Thiazolyl, Thiadiazolyl, Oxazolyl, Isoxazolyl, Pyrimidinyl, Oxadiazolyl, Benzo[1,2,5]oxadiazoly! und Chromanyl steht, wobei der Rest jeweils mit 1, 2, 3, 4 oder 5 Substituenten unabhängig voneinander ausgewählt aus der Gruppe bestehend aus F, Cl, Br, I, -CN, -$CF_3$, -O-$CH_3$, -O-$C_2H_5$, -$NO_2$, -O-$CF_3$, -O-$CHF_2$, -O-$CH_2F$, -S-$CF_3$, -S-$CHF_2$, -S-$CH_2F$, -S-$CH_3$,-S-$C_2H_5$, -S-$CH(CH_3)_2$, -S-$C(CH_3)_3$, Methyl, Ethyl, n-Propyl, Isopropyl, n-Butyl, sec-Butyl, Isobutyl, tert-Butyl, -$(CH_2)$-O-C(=O)-$CH_3$, -$(CH_2)$-O-C(=O)-$C_2H_5$, -$(CH_2)$-O-C(=O)-Phenyl, -$N(CH_3)_2$, -$N(C_2H_5)_2$, -NH-C(=O)-$CH_3$, -NH-C(=O)-$C_2H_5$, -$S(=O)_2$-$NH_2$, -O-Phenyl und Phenyl substituiert sein kann, wobei jeweils der zyklische Teil der Reste -O-Phenyl und Phenyl mit 1, 2, 3, 4 oder 5 Substituenten unabhängig voneinander ausgewählt aus der Gruppe bestehend aus F, Cl, Br, Methyl, Ethyl, n-Propyl und Isopropyl substituiert sein kann;

und jeweils - sofern vorhanden - in den vorstehend genannten Gruppen I.) II.) und III.) die Reste

$R^5$ für einen Methyl- oder Ethyl-Rest steht;
$R^6$ für einen Methyl- oder Ethyl-Rest steht;
$R^7$ für einen Wasserstoff-Rest
oder für einen Methyl- oder Ethyl-Rest steht;
$R^8$ für einen Methyl- oder Ethyl-Rest steht
oder für einen unsubstituierten Phenyl-Rest steht;
$R^9$ für einen Methyl- oder Ethyl-Rest steht;
$R^{10}$ für einen Wasserstoff-Rest steht
oder für einen Methyl- oder Ethyl-Rest steht;
$R^{11}$ für einen Methyl- oder Ethyl-Rest steht;
oder für einen Phenyl-Rest steht, der mit 1, 2, 3, 4 oder 5 Substituenten unabhängig voneinander ausgewählt aus der Gruppe bestehend aus F, Cl, Br, -O-$CH_3$, -O-$C_2H_5$, -O-$CH(CH_3)_2$, -O-$C(CH_3)_3$ und Phenyl substituiert sein kann;
$R^{12}$ für einen Wasserstoff-Rest oder für einen Methyl-Rest steht;
$R^{13}$ für einen Wasserstoff-Rest oder für einen Methyl-Rest steht;
$R^{14}$ für einen Wasserstoff-Rest steht;

für einen Methyl- oder Ethyl-Rest steht
oder für einen unsubstituierten Phenyl-Rest steht;
$R^{15}$ für einen Wasserstoff-Rest oder für einen Methyl-Rest steht.
$R^{16}$ für einen Methyl- oder Ethyl-Rest steht
oder für einen unsubstituierten Phenyl-Rest steht;
$R^{17}$ für einen unsubstituierten Phenyl-Rest steht;
$R^{18}$ für einen Alkyl-Rest ausgewählt aus der Gruppe bestehend aus Methyl, Ethyl, n-Propyl, Isopropyl, n-Butyl, Isobutyl und tert-Butyl steht;
$R^{19}$ für einen Wasserstoff-Rest steht;
für einen Methyl- oder Ethyl-Rest steht
oder für einen unsubstituierten Phenyl-Rest steht;
$R^{20}$ für einen Rest ausgewählt aus der Gruppe bestehend aus Cyclopentyl, Cyclohexyl und Hydantoin steht; oder für einen Phenyl-Rest steht, der mit 1, 2, 3, 4 oder 5 Substituenten unabhängig voneinander ausgewählt aus der Gruppe bestehend aus F, Cl, Br, $-O-CH_3$, $-O-C_2H_5$, $-O-CH(CH_3)_2$, $-O-C(CH_3)_3$ und Phenyl substituiert sein kann;
$R^{21}$ für einen Phenyl-Rest steht, der mit 1, 2, 3, 4 oder 5 Substituenten unabhängig voneinander ausgewählt aus der Gruppe bestehend aus F, Cl, Br und $-CF_3$ substituiert sein kann;
$R^{22}$ für einen Phenyl-Rest steht, wobei der Phenyl-Rest jeweils mit 1, 2, 3, 4 oder 5 Substituenten unabhängig voneinander ausgewählt aus der Gruppe bestehend aus F, Cl, Br, -CN, $-CF_3$, $-NO_2$, $-O-CF_3$, $-S-CF_3$, Methyl, Ethyl, n-Propyl, Isopropyl, n-Butyl, sec-Butyl, Isobutyl, tert-Butyl, -C(=O)-OH, $-C(=O)-O-CH_3$ und $-C(=O)-O-C_2H_5$ substituiert ist;
oder für einen unsubstituierten Naphthyl-Rest steht;
$R^{23}$ für einen unsubstituierten Phenyl-Rest steht

und

$R^{24}$ für einen Methyl- oder Ethyl-Rest steht
oder für einen unsubstituierten Phenyl-Rest steht.

**13.** Substituierte 1,4,8-Triazaspiro[4.5]decan-2-on-Verbindungen der allgemeinen Formel I gemäß einem oder mehreren der Ansprüche 1 bis 12 ausgewählt aus der Gruppe bestehend aus

(1) 3-(*S*)-Benzyl-8-(2-ethyl-butyryl)-1,4,8-triaza-spiro[4.5]decan-2-on,
(2) 8-(2,4-Dimethoxy-benzoyl)-3-(*S*)-(2-methylsulfanyl-ethyl)-1,4,8-triaza-spiro[4.5]decan-2-on,
(3) 3-(*S*,*R*)-Benzyl-8-(2-ethyl-butyryl)-1,4,8-triaza-spiro[4.5]decan-2-on,
(4) 8-Acetyl-3-(*S*)-benzyl-1,4,8-triaza-spiro[4.5]decan-2-on,
(5) 3-(*S*,*R*)-Benzyl-8-(4-chlor-benzoyl)-1,4,8-triaza-spiro[4.5]decan-2-on,
(6) 3-(*S*)-Benzyl-8-(4-chlor-benzoyl)-1,4,8-triaza-spiro[4.5]decan-2-on,
(7) 8-(2-ethyl-butyryl)-3-(*S*)-(2-methylsulfanyl-ethyl)-1,4,8-triaza-spiro[4.5]decan-2-on,
(8) 1,3-(*S*)-Dibenzyl-8-(2-ethyl-butyryl)-1,4,8-triaza-spiro[4.5]decan-2-on,
(9) 8-Acetyl-1,3-(*S*)-dibenzyl-1,4,8-triaza-spiro[4.5]decan-2-on,
(10) 3-(*S*,*R*)-Benzyl-8-(4-chlor-benzoyl)-1-(4-methoxy-benzyl)-1,4,8-triaza-spiro[4.5]decan-2-on,
(11) 3-(*S*,*R*)-Benzyl-8-(4-chlorbenzoyl)-1-(4-fluor-benzyl)-1,4,8-triaza-spiro[4.5]decan-2-on,
(12) 1,3-(*S*,*R*)-Dibenzyl-8-(4-chlor-benzoyl)-1,4,8-triaza-spiro[4.5]decan-2on,
(13) 3-(*S*,*R*)-Benzyl-8-(2-ethyl-butyryl)-1-(4-methoxy-benzyl)-1,4,8-triaza-spiro[4.5]decan-2-on,
(14) 3-(*S*,*R*)-Benzyl-8-(2-ethyl-butyryl)-1-(4-fluor-benzyl)-1,4,8-triaza-spiro[4.5]decan-2-on,
(15) 1,3-(*S*,*R*)-Dibenzyl-8-(2-ethyl-butyryl)-1,4,8-triaza-spiro[4.5]decan-2-on,
(16) 1-Benzyl-8-(2-ethyl-butyryl)-3-(*S*)-(2-methylsulfanyl-ethyl)-1,4,8-triaza-spiro[4.5]decan-2-on,
(17) 1,3-(*S*)-Dibenzyl-8-(4-chlor-benzoyl)-1,4,8-triaza-spiro[4.5]decan-2-on,
(18) 1,3-(*S*)-Dibenzyl-8-(2-ethyl-butyryl)-1,4,8-triaza-spiro[4.5]decan-2-on,
(19) 8-Acetyl-1,3-(*S*)-dibenzyl-1,4,8-triaza-spiro[4.5]decan-2-on,
(20) 3-(*S*,*R*)-Benzyl-8-(4-chlor-benzoyl)-1-(4-methoxy-benzyl)-1,4,8-triaza-spiro[4.5]decan-2-on,
(21) 3-(*S*,*R*)-Benzyl-8-(4-chlor-benzoyl)-1-(4-fluor-benzyl)-1,4,8-triaza-spiro[4.5]decan-2-on,
(22) 1,3-(*S*,*R*)-Dibenzyl-8-(4-chlor-benzoyl)-1,4,8-triaza-spiro[4.5]decan-2-on,
(23) 3-(*S*,*R*)-Benzyl-8-(2-ethyl-butyryl)-1-(4-methoxy-benzyl)-1,4,8-triaza-spiro[4.5]decan-2-on,
(24) 3-(*S*,*R*)-Benzyl-8-(2-ethyl-butyryl)-1-(4-fluor-benzyl)-1,4,8-triaza-spiro[4.5]decan-2-on,
(25) 1,3-(*S*,*R*)-Dibenzyl-8-(2-ethyl-butyryl)-1,4,8-triaza-spiro[4.5]decan-2-on,
(26) 1-Benzyl-8-(2-ethyl-butyryl)-3-(*S*)-(2-methylsulfanyl-ethyl)-1,4,8-triaza-spiro[4.5]decan-2-on,

(27) 1,3-(*S*)-Dibenzyl-8-(4-chlor-benzoyl)-1,4,8-triaza-spiro[4.5]decan-2-on,
(28) 1-Benzyl-8-(2,4-dimethoxy-benzoyl)-3-(*S*)-(2-methylsulfanyl-ethyl)-1,4,8-triaza-spiro[4.5]decan-2-on,
(29) 1,3-(*S*)-Dibenzyl-1,4,8-triaza-spiro[4.5]decan-2-on,
(30) 1-Benzyl-3-(S)-(2-methylsulfanyl-ethyl)-1,4,8-triaza-spiro[4.5]decan-2-on,
(31) 1,3-(*S*)-Dibenzyl-8-butyryl-1,4,8-triaza-spiro[4.5]decan-2-on,
(32) 1,3-(*S*)-Dibenzyl-8-(3-fluor-4-trifluormethyl-benzoyl)-1,4,8-triaza-spiro[4.5]decan-2-on,
(33) 1,3-(*S*)-Dibenzyl-8-(2,3-difluor-benzoyl)-1,4,8-triaza-spiro[4.5]decan-2-on,
(34) 1,3-(*S*)-Dibenzyl-8-[2-(4-chlor-phenoxy)-acetyl]-1,4,8-triaza-spiro[4.5]decan-2-on,
(35) 1,3-(*S*)-Dibenzyl-8-diphenylacetyl-1,4,8-triaza-spiro[4.5]decan-2-on,
(36) 1,3-(*S*)-Dibenzyl-8-(2-phenoxy-acetyl)-1,4,8-triaza-spiro[4.5]decan-2-on,
(37) 1,3-(*S*)-Dibenzyl-8-(3-phenyl-propionyl)-1,4,8-triaza-spiro[4.5]decan-2-on,
(38) 1,3-(*S*)-Dibenzyl-8-(naphthalin-2-carbonyl)-1,4,8-triaza-spiro[4.5]decan-2-on,
(39) 1,3-(*S*)-Dibenzyl-8-(furan-2-carbonyl)-1,4,8-triaza-spiro[4.5]decan-2-on,
(40) 1,3-(*S*)-Dibenzyl-8-(3-methoxy-benzoyl)-1,4,8-triaza-spiro[4.5]decan-2-on,
(41) 1,3-(*S*)-Dibenzyl-8-(4-fluor-benzoyl)-1,4,8-triaza-spiro[4.5]decan-2-on,
(42) 1-Benzyl-8-(4-fluor-benzoyl)-3-(*S*)-(2-methylsulfanyl-ethyl)-1,4,8-triaza-spiro[4.5]decan-2-on,
(43) 1,3-(*S*)-Dibenzyl-8-butyryl-1,4,8-triaza-spiro[4.5]decan-2-on,
(44) 1,3-(*S*)-Dibenzyl-8-(3-fluor-4-trifluormethyl-benzoyl)-1,4,8-triaza-spiro[4.5]decan-2-on,
(45) 1,3-(*S*)-Dibenzyl-8-(2,3-difluor-benzoyl)-1,4,8-triaza-spiro[4.5]decan-2-on,
(46) 1,3-(*S*)-Dibenzyl-8-[2-(4-chlor-phenoxy)-acetyl]-1,4,8-triaza-spiro[4.5]decan-2-on,
(47) 1,3-(*S*)-Dibenzyl-8-diphenylacetyl-1,4,8-triaza-spiro[4.5]decan-2-on,
(48) 1,3-(*S*)-Dibenzyl-8-(2-phenoxy-acetyl)-1,4,8-triaza-spiro[4.5]decan-2-on,
(49) 1,3-(*S*)-Dibenzyl-8-(3-phenyl-propionyl)-1,4,8-triaza-spiro[4.5]decan-2-on,
(50) 1,3-(*S*)-Dibenzyl-8-(naphthalin-2-carbonyl)-1,4,8-triaza-spiro[4.5]decan-2-on,
(51) 1,3-(*S*)-Dibenzyl-8-(furan-2-carbonyl)-1,4,8-triaza-spiro[4.5]decan-2-on,
(52) 1,3-(*S*)-Dibenzyl-8-(3-methoxy-benzoyl)-1,4,8-triaza-spiro[4.5]decan-2-on,
(53) 1,3-(*S*)-Dibenzyl-8-(4-fluor-benzoyl)-1,4,8-triaza-spiro[4.5]decan-2-on,
(54) 1-Benzyl-8-(4-fluor-benzoyl)-3-(*S*)-(2-methylsulfanyl-ethyl)-1,4,8-triaza-spiro[4.5]decan-2-on,
(55) N-[4-(3-Isobutyl-2-oxo-1-prop-2-ynyl-1,4,8-triaza-spiro[4.5]decan-8-carbonyl)-phenyl]-acetamid,
(56) 1-(2-Phenoxy-ethyl)-8-(thiophen-2-carbonyl)-1,4,8-triaza-spiro[4.5]decan-2-on,
(57) 2-(2-Oxo-8-pent-4-enoyl-1,4,8-triaza-spiro[4.5]dec-1-ylmethyl)-benzonitril,
(58) 8-(2,4-Dimethoxy-benzoyl)-1-methyl-3-(2-methylsulfanyl-ethyl)-1,4,8-triaza-spiro[4.5]decan-2-on,
(59) 2-[8-(2-Ethyl-butyryl)-2-oxo-1,4,8-triaza-spiro[4.5]dec-1-ylmethyl]-benzonitril,
(60) 4-(2-Isobutyl-3-oxo-1,4,8-triaza-spiro[4.5]decan-8-carbonyl)-benzonitril,
(61) 8-(6-Chlor-pyridin-3-carbonyl)-3-isobutyl-1,4,8-triaza-spiro[4.5]decan-2-on,
(62) 2-[8-(2-Methyl-pentanoyl)-2-oxo-1,4,8-triaza-spiro[4.5]dec-1-ylmethyl]-benzonitril,
(63) 1-Benzyl-8-(biphenyl-4-carbonyl)-1,4,8-triaza-spiro[4.5]decan-2-on,
(64) 3-Isobutyl-8-(5-methyl-isoxazol-3-carbonyl)-1,4,8-triaza-spiro[4.5]decan-2-on,
(65) 3-Oxo-3-[2-oxo-1-(2-phenoxy-ethyl)-1,4,8-triaza-spiro[4.5]dec-8-yl]-propionsäureethylester,
(66) 8-(2-Chlor-benzoyl)-3-isopropyl-1,4,8-triaza-spiro[4.5]decan-2-on,
(67) 8-Cyclopentancarbonyl-3-isopropyl-1,4,8-triaza-spiro[4.5]decan-2-on,
(68) 8-(Furan-2-carbonyl)-1-methyl-3-(2-methylsulfanyl-ethyl)-1,4,8-triaza-spiro[4.5]decan-2-on,
(69) 3-Benzyl-8-(2-ethylsulfanyl-pyridin-3-carbonyl)-1-methyl-1,4,8-triaza-spiro[4.5]decan-2-on,
(70) 3-Benzyl-8-(4-chlor-benzoyl)-1,4,8-triaza-spiro[4.5]decan-2-on,
(71) 8-(2-Benzyloxy-acetyl)-3-isobutyl-1,4,8-triaza-spiro[4.5]decan-2-on,
(72) 3-Benzyl-8-(2-methoxy-acetyl)-1-prop-2-ynyl-1,4,8-triaza-spiro[4.5]decan-2-on,
(73) 8-(5-tert-Butyl-2-methyl-2H-pyrazol-3-carbonyl)-3-isobutyl-1,4,8-triaza-spiro[4.5]decan-2-on,
(74) 2-{8-[3-(2-chlor-6-fluor-phenyl)-5-methyl-isoxazol-4-carbonyl]-2-oxo-1,4,8-triaza-spiro[4.5]dec-1-ylmethyl}-benzonitril,
(75) 3-Benzyl-8-(2-chlor-benzoyl)-1,4,8-triaza-spiro[4.5]decan-2-on,
(76) 3-Benzyl-8-(3-dimethylamino-benzoyl)-1-methyl-1,4,8-triaza-spiro[4.5]decan-2-on,
(77) 8-(3-Methyl-benzoyl)-1-(2-phenoxy-ethyl)-1,4,8-triaza-spiro[4.5]decan-2-on,
(78) 3-Isopropyl-1-(3-methyl-but-2-enyl)-8-(pyridin-4-carbonyl)-1,4,8-triaza-spiro[4.5]decan-2-on,
(79) 1-Benzyl-8-(4-methyl-[1,2,3]thiadiazol-5-carbonyl)-1,4,8-triaza-spiro[4.5]decan-2-on,
(80) 2-{8-[3-(2-chlor-phenyl)-acryloyl]-2-oxo-1,4,8-triaza-spiro[4.5]dec-1-ylmethyl}-benzonitril,
(81) 8-(3-Chlor-benzoyl)-3-isobutyl-1,4,8-triaza-spiro[4.5]decan-2-on,
(82) Essigsäure 2-(2-benzyl-3-oxo-1,4,8-triaza-spiro[4.5]dec-8-yl)-2-oxo-1-phenyl-ethylester,
(83) 8-(3,5-Dimethoxy-benzoyl)-3-isobutyl-1,4,8-triaza-spiro[4.5]decan-2-on,

(84) 3-Benzyl-8-(isoxazol-5-carbonyl)-1,4,8-triaza-spiro[4.5]decan-2-on,
(85) 8-(3-Chlor-thiophen-2-carbonyl)-3-isobutyl-1,4,8-triaza-spiro[4.5]decan-2-on,
(86) 3-Isopropyl-8-pentafluorbenzoyl-1,4,8-triaza-spiro[4.5]decan-2-on,
(87) 8-(2,5-Dimethyl-furan-3-carbonyl)-3-isobutyl-1,4,8-triaza-spiro[4.5]decan-2-on,
(88) 1-Butyl-8-[2-(3,4-dimethoxy-phenyl)-acetyl]-3-(2-methylsulfanyl-ethyl)-1,4,8-triaza-spiro[4.5]decan-2-on,
(89) 1-Benzyl-3-isopropyl-8-(pyridin-4-carbonyl)-1,4,8-triaza-spiro[4.5]decan-2-on,
(90) 1,3-Dibenzyl-8-(3-dimethylamino-benzoyl)-1,4,8-triaza-spiro[4.5]decan-2-on,
(91) 5-{2-[1-(4-Fluor-benzyl)-3-(2-methylsulfanyl-ethyl)-2-oxo-1,4,8-triaza-spiro[4.5]dec-8-yl]-2-oxo-ethyl}-imidazolidin-2,4-dion,
(92) 8-(Biphenyl-4-carbonyl)-1-(4-fluor-benzyl)-3-isobutyl-1,4,8-triaza-spiro[4.5]decan-2-on,
(93) 2-[2-Oxo-8-(2-propyl-pentanoyl)-1,4,8-triaza-spiro[4.5]dec-1-ylmethyl]-benzonitril,
(94) 2-[8-(Furan-2-carbonyl)-3-isopropyl-2-oxo-1,4,8-triaza-spiro[4.5]dec-1-ylmethyl]-benzonitril,
(95) 8-[2-(4-Chlor-phenoxy)-acetyl]-3-isobutyl-1-prop-2-ynyl-1,4,8-triaza-spiro[4.5]decan-2-on,
(96) 1,3-Dibenzyl-8-(4-brom-benzoyl)-1,4,8-triaza-spiro[4.5]decan-2-on,
(97) 8-(3-Difluormethylsulfanyl-benzoyl)-3-isobutyl-1,4,8-triaza-spiro[4.5]decan-2-on,
(98) 8-(2,3-Dimethyl-benzoyl)-3-isopropyl-1,4,8-triaza-spiro[4.5]decan-2-on,
(99) 3-Benzyl-8-(2,3-dimethyl-benzoyl)-1,4,8-triaza-spiro[4.5]decan-2-on,
(100) 1-(4-Fluor-benzyl)-3-(2-methylsulfanyl-ethyl)-8-(pyridin-2-carbonyl)-1,4,8-triaza-spiro[4.5]decan-2-on,
(101) 3-Benzyl-8-(3,3-dimethyl-butyryl)-1,4,8-triaza-spiro[4.5]decan-2-on,
(102) 2-[8-(3-Dimethylamino-benzoyl)-3-isobutyl-2-oxo-1,4,8-triaza-spiro[4.5]dec-1-ylmethyl]-benzonitril,
(103) 3-[8-(2-Methoxy-acetyl)-3-(2-methylsulfanyl-ethyl)-2-oxo-1,4,8-triaza-spiro[4.5]dec-1-ylmethyl]-benzonitril,
(104) Essigsäure 2-(3-benzyl-1-methyl-2-oxo-1,4,8-triaza-spiro[4.5]dec-8-yl)-2-oxo-1-phenyl-ethylester,
(105) 2-[8-[2-(2-Methoxy-ethoxy)-acetyl]-3-(2-methylsulfanyl-ethyl)-2-oxo-1,4,8-triaza-spiro[4.5]dec-1-ylmethyl]-benzonitril,
(106) 3-Benzyl-8-[3-(2-chlor-6-fluor-phenyl)-5-methyl-isoxazol-4-carbonyl]-1,4,8-triaza-spiro[4.5]decan-2-on,
(107) 8-(2-Chlor-6-fluor-benzoyl)-3-isopropyl-1,4,8-triaza-spiro[4.5]decan-2-on,
(108) 1-Methyl-3-(2-methylsulfanyl-ethyl)-8-(2-phenoxy-acetyl)-1,4,8-triaza-spiro[4.5]decan-2-on,
(109) 8-(2-Chlor-pyridin-3-carbonyl)-1-(4-fluor-benzyl)-3-isobutyl-1,4,8-triaza-spiro[4.5]decan-2-on,
(110) 8-(2-Ethyl-butyryl)-1-methyl-3-(2-methylsulfanyl-ethyl)-1,4,8-triaza-spiro[4.5]decan-2-on,
(111) 1-Methyl-3-(2-methylsulfanyl-ethyl)-8-(2-phenoxy-pyridin-3-carbonyl)-1,4,8-triaza-spiro[4.5]decan-2-on,
(112) 8-(3-Fluor-benzoyl)-1-methyl-3-(2-methylsulfanyl-ethyl)-1,4,8-triaza-spiro[4.5]decan-2-on,
(113) 3-Benzyl-8-(naphthalin-2-carbonyl)-1,4,8-triaza-spiro[4.5]decan-2-on,
(114) 8-Cyclohexancarbonyl-1-(2-phenoxy-ethyl)-1,4,8-triaza-spiro[4.5]decan-2-on,
(115) 8-(2-Phenoxy-acetyl)-1-(2-phenoxy-ethyl)-1,4,8-triaza-spiro[4.5]decan-2-on,
(116) 4-[1-Butyl-3-(2-methylsulfanyl-ethyl)-2-oxo-1,4,8-triaza-spiro[4.5]decan-8-carbonyl]-benzonitril,
(117) 3-Benzyl-8-(3,3-dimethyl-butyryl)-1-methyl-1,4,8-triaza-spiro[4.5]decan-2-on,
(118) 3-Benzyl-8-(4-propyl-benzoyl)-1,4,8-triaza-spiro[4.5]decan-2-on,
(119) 3-Isopropyl-8-(2-phenoxy-propionyl)-1,4,8-triaza-spiro[4.5]decan-2-on,
(120) 1-Butyl-8-hexanoyl-3-isopropyl-1,4,8-triaza-spiro[4.5]decan-2-on,
(121) 8-(4-Brom-3-methyl-benzoyl)-3-isopropyl-1,4,8-triaza-spiro[4.5]decan-2-on,
(122) 8-[3-(2-Chlor-6-fluor-phenyl)-5-methyl-isoxazol-4-carbonyl]-3-isopropyl-1-(2-phenoxy-ethyl)-1,4,8-triaza-spiro[4.5]decan-2-on,
(123) 1-(2-Fluor-benzyl)-3-isobutyl-8-(3-methyl-benzoyl)-1,4,8-triaza-spiro[4.5]decan-2-on,
(124) 8-(2-Ethyl-hexanoyl)-1-(2-phenoxy-ethyl)-1,4,8-triaza-spiro[4.5]decan-2-on,
(125) 3-Benzyl-8-(3,4-difluor-benzoyl)-1,4,8-triaza-spiro[4.5]decan-2-on,
(126) 3-Benzyl-8-(4-ethoxy-benzoyl)-1-methyl-1,4,8-triaza-spiro[4.5]decan-2-on,
(127) 1-Benzyl-8-(6-chlor-pyridin-3-carbonyl)-1,4,8-triaza-spiro[4.5]decan-2-on,
(128) 8-(3-Dimethylamino-benzoyl)-1-(3,5-dimethyl-benzyl)-3-(2-methylsulfanyl-ethyl)-1,4,8-triaza-spiro[4.5]decan-2-on,
(129) 3-[8-(Benzo[1,3]dioxol-5-carbonyl)-3-(2-methylsulfanyl-ethyl)-2-oxo-1,4,8-triaza-spiro[4.5]dec-1-ylmethyl]-benzonitril,
(130) 3-Benzyl-1-methyl-8-pent-4-enoyl-1,4,8-triaza-spiro[4.5]decan-2-on,
(131) 8-(4-Ethoxy-benzoyl)-1-methyl-3-(2-methylsulfanyl-ethyl)-1,4,8-triaza-spiro[4.5]decan-2-on,
(132) 3-Benzyl-8-(2-benzyloxy-acetyl)-1-methyl-1,4,8-triaza-spiro[4.5]decan-2-on,
(133) 8-(3,4-Difluor-benzoyl)-1-(2-phenoxy-ethyl)-1,4,8-triaza-spiro[4.5]decan-2-on,
(134) 3-Benzyl-1-methyl-8-(2-methylsulfanyl-pyridin-3-carbonyl)-1,4,8-triaza-spiro[4.5]decan-2-on,
(135) 1-Butyl-8-(4-methoxy-benzoyl)-3-(2-methylsulfanyl-ethyl)-1,4,8-triaza-spiro[4.5]decan-2-on,

(136) 1,3-Dibenzyl-8-(2-ethylsulfanyl-pyridin-3-carbonyl)-1,4,8-triaza-spiro[4.5]decan-2-on,
(137) 3-Benzyl-8-(3-difluormethylsulfanyl-benzoyl)-1,4,8-triaza-spiro[4.5]decan-2-on,
(138) 1-(4-Fluor-benzyl)-8-(furan-2-carbonyl)-3-(2-methylsulfanyl-ethyl)-1,4,8-triaza-spiro[4.5]decan-2-on,
(139) 3-Benzyl-8-(3-tluor-4-methyl-benzoyl)-1-methyl-1,4,8-triaza-spiro[4.5]decan-2-on,
(140) 8-[3-(2-Chlor-6-fluor-phenyl)-5-methyl-isoxazol-4-carbonyl]-3-isopropyl-1,4,8-triaza-spiro[4.5]decan-2-on,
(141) 1-Butyl-8-(6-Chlor-2H-chroman-3-carbonyl)-3-(2-methylsulfanyl-ethyl)-1,4,8-triaza-spiro[4.5]decan-2-on,
(142) 3-[8-(3-Methoxy-benzoyl)-3-(2-methylsulfanyl-ethyl)-2-oxo-1,4,8-triaza-spiro[4.5]dec-1-ylmethyl]-benzonitril,
(143) 8-Cyclobutancarbonyl-3-(2-methylsulfanyl-ethyl)-1-prop-2-ynyl-1,4,8-triaza-spiro[4.5]decan-2-on,
(144) 3-Benzyl-1-butyl-8-(furan-2-carbonyl)-1,4,8-triaza-spiro[4.5]decan-2-on,
(145) 1-Benzyl-8-(3-chlor-thiophen-2-carbonyl)-3-isobutyl-1,4,8-triaza-spiro[4.5]decan-2-on,
(146) 3-Benzyl-8-(2,5-bis-trifluormethyl-benzoyl)-1-butyl-1,4,8-triaza-spiro[4.5]decan-2-on,
(147) 8-(3-Chlor-2-fluor-benzoyl)-1-(3,5-dimethyl-benzyl)-3-(2-methylsulfanylethyl)-1,4,8-triaza-spiro[4.5]decan-2-on,
(148) 1-Benzyl-8-(2-chlor-pyridin-3-carbonyl)-3-isopropyl-1,4,8-triaza-spiro[4.5]decan-2-on,
(149) 3-Isobutyl-8-pentafluorbenzoyl-1-prop-2-ynyl-1,4,8-triaza-spiro[4.5]decan-2-on,
(150) 8-(2-Benzyloxy-acetyl)-1-butyl-3-(2-methylsulfanyl-ethyl)-1,4,8-triaza-spiro[4.5]decan-2-on,
(151) 8-(Furan-2-carbonyl)-3-isobutyl-1-prop-2-ynyl-1,4,8-triaza-spiro[4.5]decan-2-on,
(152) 1-Butyl-3-(2-methylsulfanyl-ethyl)-8-(4-phenoxy-butyryl)-1,4,8-triaza-spiro[4.5]decan-2-on,
(153) 3-Benzyl-8-(6-chlor-pyridin-3-carbonyl)-1-methyl-1,4,8-triaza-spiro[4.5]decan-2-on,
(154) 1-(2-Fluor-benzyl)-3-(2-methylsulfanyl-ethyl)-8-(2-phenoxy-pyridin-3-carbonyl)-1,4,8-triaza-spiro[4.5]decan-2-on,
(155) 1-(2-Fluor-benzyl)-3-isobutyl-8-(2-methyl-6-trifluormethyl-pyridin-3-carbonyl)-1,4,8-triaza-spiro[4.5]decan-2-on,
(156) 8-[2-(3-Chlor-phenoxy)-acetyl]-3-Isopropyl-1-(2-phenoxy-ethyl)-1,4,8-triaza-spiro[4.5]decan-2-on,
(157) 8-(2,3-Dimethyl-benzoyl)-1-methyl-3-(2-methylsulfanyl-ethyl)-1,4,8-triaza-spiro[4.5]decan-2-on,
(158) 3-Isopropyl-8-(2-methyl-6-trifluormethyl-pyridin-3-carbonyl)-1-(2-phenoxy-ethyl)-1,4,8-triaza-spiro[4.5] decan-2-on,
(159) 3-Benzyl-1-methyl-8-(5-methyl-2-phenyl-2H-[1,2,3]triazole-4-carbonyl)-1,4,8-triaza-spiro[4.5]decan-2-on,
(160) 8-[3-(2-Chlor-phenyl)-5-methyl-isoxazol-4-carbonyl]-1-(3,5-dimethyl-benzyl)-3-(2-methylsulfanyl-ethyl)-1,4,8-triaza-spiro[4.5]decan-2-on,
(161) 1-(2-Fluor-benzyl)-3-(2-methylsulfanyl-ethyl)-8-(pyridin-2-carbonyl)-1,4,8-triaza-spiro[4.5]decan-2-on,
(162) 1-Benzyl-3-(2-methylsulfanyl-ethyl)-8-(2-phenoxy-pyridin-3-carbonyl)-1,4,8-triaza-spiro[4.5]decan-2-on,
(163) 1,3-Dibenzyl-8-(3-chlor-thiophen-2-carbonyl)-1,4,8-triaza-spiro[4.5]decan-2-on,
(164) 3-Benzyl-8-(4-tert-butyl-benzoyl)-1-methyl-1,4,8-triaza-spiro[4.5]decan-2-on,
(165) 2-[3-Isobutyl-8-(2-methoxy-acetyl)-2-oxo-1,4,8-triaza-spiro[4.5]dec-1-ylmethyl]-benzonitril,
(166) 3-Benzyl-1-butyl-8-(5-fluor-2-trifluormethyl-benzoyl)-1,4,8-triaza-spiro[4.5]decan-2-on,
(167) 3-Benzyl-8-[2-(4-methoxy-phenyl)-acetyl]-1-methyl-1,4,8-triaza-spiro[4.5]decan-2-on,
(168) 1,3-Dibenzyi-8-(4-methyl-[1,2,3]thiadiazol-5-carbonyl)-1,4,8-triaza-spiro[4.5]decan-2-on,
(169) 1-Benzyl-3-isopropyl-8-(2-methyl-6-trifluormethyl-pyridin-3-carbonyl)-1,4,8-triaza-spiro[4.5]decan-2-on,
(170) 1-Benzyl-8-(4-ethoxy-benzoyl)-3-isobutyl-1,4,8-triaza-spiro[4.5]decan-2-on,
(171) 3-Benzyl-1-butyl-8-cyclohexancarbonyl-1,4,8-triaza-spiro[4.5]decan-2-on,
(172) 3-[8-[3-(2-Chlor-6-fluor-phenyl)-5-methyl-isoxazol-4-carbonyl]-3-(2-methylsulfanyl-ethyl)-2-oxo-1,4,8-triaza-spiro[4.5]dec-1-ylmethyl]-benzonitril,
(173) 1-(2-Fluor-benzyl)-3-isobutyl-8-pent-4-enoyl-1,4,8-triaza-spiro[4.5]decan-2-on,
(174) 1-Methyl-3-(2-methylsulfanyl-ethyl)-8-(2-propyl-pentanoyl)-1,4,8-triaza-spiro[4.5]decan-2-on,
(175) 3-Benzyl-1-methyl-8-(2-phenoxy-acetyl)-1,4,8-triaza-spiro[4.5]decan-2-on,
(176) 3-Isobutyl-1-prop-2-ynyl-8-(3-trifluormethyl-benzoyl)-1,4,8-triaza-spiro[4.5]decan-2-on,
(177) 3-Benzyl-8-(furan-2-carbonyl)-1-prop-2-ynyl-1,4,8-triaza-spiro[4.5]decan-2-on,
(178) 1-Methyl-3-(2-methylsulfanyl-ethyl)-8-(naphthalin-1-carbonyl)-1,4,8-triaza-spiro[4.5]decan-2-on,
(179) 3-Benzyl-1-butyl-8-(3-cyclopentyl-propionyl)-1,4,8-triaza-spiro[4.5]decan-2-on,
(180) 1-(3,5-Dimethyl-benzyl)-3-(2-methylsulfanyl-ethyl)-8-pentanoyl-1,4,8-triaza-spiro[4.5]decan-2-on,
(181) 3-Benzyl-1-butyl-8-(2-methoxy-acetyl)-1,4,8-triaza-spiro[4.5]decan-2-on,
(182) 3-Benzyl-8-(3-fluor-4-trifluormethyl-benzoyl)-1-methyl-1,4,8-triaza-spiro[4.5]decan-2-on,
(183) 1-Benzyl-8-(3-difluormethylsulfanyl-benzoyl)-3-isopropyl-1,4,8-triaza-spiro[4.5]decan-2-on,

(184) 8-(2-Chlor-6-fluor-3-methyl-benzoyl)-1-methyl-3-(2-methylsulfanyl-ethyl)-1,4,8-triaza-spiro[4.5]decan-2-on,
(185) 4-[3-Isopropyl-8-(2-methylsulfanyl-pyridin-3-carbonyl)-2-oxo-1,4,8-triaza-spiro[4.5]dec-1-ylmethyl]-benzoesäuremethylester,
(186) 8-[2-(2,5-Dimethoxy-phenyl)-acetyl]-1-(2-fluor-benzyl)-3-(2-methylsulfanyl-ethyl)-1,4,8-triaza-spiro[4.5]decan-2-on,
(187) 8-(5-tert-Butyl-2-methyl-furan-3-carbonyl)-3-isopropyl-1-(2-phenoxy-ethyl)-1,4,8-triaza-spiro[4.5]decan-2-on,
(188) 8-(2-Cyclopentyl-acetyl)-1-(4-fluor-benzyl)-3-isobutyl-1,4,8-triaza-spiro[4.5]decan-2-on,
(189) 4-[8-(3,3-Dimethyl-butyryl)-3-isopropyl-2-oxo-1,4,8-triaza-spiro[4.5]dec-1-ylmethyl]-benzoesäuremethylester,
(190) 3-[8-Cyclopropancarbonyl-3-(2-methylsulfanyl-ethyl)-2-oxo-1,4,8-triaza-spiro[4.5]dec-1-ylmethyl]-benzonitril,
(191) 3-[3-(2-Methylsulfanyl-ethyl)-2-oxo-8-(3-trifluormethoxy-benzoyl)-1,4,8-triaza-spiro[4.5]dec-1-ylmethyl]-benzonitril,
(192) 1-Butyl-8-(2-cyclopentyl-acetyl)-3-isopropyl-1,4,8-triaza-spiro[4.5]decan-2-on,
(193) 3-Benzyl-1-butyl-8-(pyridin-2-carbonyl)-1,4,8-triaza-spiro[4.5]decan-2-on,
(194) 3-Benzyl-8-[3-(2-chlor-phenyl)-acryloyl]-1-methyl-1,4,8-triaza-spiro[4.5]decan-2-on,
(195) 4-[8-(3-fluor-4-trifluormethyl-benzoyl)-3-isopropyl-2-oxo-1,4,8-triaza-spiro[4.5]dec-1-ylmethyl]-benzoesäuremethylester,
(196) 8-[3-(2,6-Dichlor-phenyl)-5-methyl-isoxazol-4-carbonyl]-1-methyl-3-(2-methylsulfanyl-ethyl)-1,4,8-triaza-spiro[4.5]decan-2-on,
(197) 1-Butyl-8-cyclohexancarbonyl-3-(2-methylsulfanyl-ethyl)-1,4,8-triaza-spiro[4.5]decan-2-on,
(198) 3-Benzyl-1-butyl-8-(4-iod-benzoyl)-1,4,8-triaza-spiro[4.5]decan-2-on
(199) 1-Methyl-3-(2-methylsulfanyl-ethyl)-8-[3-(3-trifluormethyl-phenyl)-acryloyl]-1,4,8-triaza-spiro[4.5]decan-2-on,
(200) 1,3-Dibenzyl-8-(4-phenoxy-butyryl)-1,4,8-triaza-spiro[4.5]decan-2-on
(201) 3-Benzyl-8-(2-chlor-6-fluor-benzoyl)-1-prop-2-ynyl-1,4,8-triaza-spiro[4.5]decan-2-on,
(202) 4-[8-(2-Chlor-pyridin-3-carbonyl)-3-isopropyl-2-oxo-1,4,8-triaza-spiro[4.5]dec-1-ylmethyl]-benzoesäuremethylester,
(203) 8-(2,5-Dimethyl-furan-3-carbonyl)-3-isopropyl-1-(2-phenoxy-ethyl)-1,4,8-triaza-spiro[4.5]decan-2-on,
(204) 8-(Biphenyl-4-carbonyl)-3-(2-methylsulfanyl-ethyl)-1-prop-2-ynyl-1,4,8-triaza-spiro[4.5]decan-2-on,
(205) 8-(3-Chlor-thiophen-2-carbonyl)-3-(2-methylsulfanyl-ethyl)-1-prop-2-ynyl-1,4,8-triaza-spiro[4.5]decan-2-on,
(206) 1-(4-Fluor-benzyl)-8-[2-(4-methoxy-phenyl)-acetyl]-3-(2-methylsulfanyl-ethyl)-1,4,8-triaza-spiro[4.5]decan-2-on,
(207) 1-Benzyl-3-isopropyl-8-(4-methyl-[1,2,3]thiadiazol-5-carbonyl)-1,4,8-triaza-spiro[4.5]decan-2-on,
(208) 2-[3-Isopropyl-8-(2-methylsulfanyl-pyridin-3-carbonyl)-2-oxo-1,4,8-triaza-spiro[4.5]dec-1-ylmethyl]-benzonitril,
(209) 3-[8-(5-tert-Butyl-2-methyl-2H-pyrazol-3-carbonyl)-3-isobutyl-2-oxo-1,4,8-triaza-spiro[4.5]dec-1-ylmethyl]-benzonitril,
(210) 1-Butyl-8-(2,5-dimethyl-furan-3-carbonyl)-3-(2-methylsulfanyl-ethyl)-1,4,8-triaza-spiro[4.5]decan-2-on,
(211) 8-(3-Cyclopentyl-propionyl)-1-(2-fluor-benzyl)-3-isobutyl-1,4,8-triaza-spiro[4.5]decan-2-on,
(212) 1-Benzyl-8-(3-cyclopentyl-propionyl)-3-(2-methylsulfanyl-ethyl)-1,4,8-triaza-spiro[4.5]decan-2-on,
(213) 3-(2-Methylsulfanyl-ethyl)-8-(4-phenoxy-butyryl)-1-prop-2-ynyl-1,4,8-triaza-spiro[4.5]decan-2-on,
(214) 1,3-Dibenzyl-8-(3-phenyl-acryloyl)-1,4,8-triaza-spiro[4.5]decan-2-on,
(215) 3-Benzyl-8-(6-chlor-2-fluor-3-methyl-benzoyl)-1-methyl-1,4,8-triaza-spiro[4.5]decan-2-on,
(216) 8-[3-(2-Chlor-phenyl)-acryloyl]-3-isopropyl-1-(2-phenoxy-ethyl)-1,4,8-triaza-spiro[4.5]decan-2-on,
(217) 2-[3-Isopropyl-2-oxo-8-(3-phenyl-acryloyl)-1,4,8-triaza-spiro[4.5]dec-1-ylmethyl]-benzonitril,
(218) 3-Benzyl-1-methyl-8-(4-methyl-3-nitro-benzoyl)-1,4,8-triaza-spiro[4.5]decan-2-on,
(219) 1-Benzyl-8-(furan-2-carbonyl)-3-isopropyl-1,4,8-triaza-spiro[4.5]decan-2-on,
(220) 1-Butyl-8-(3,5-dimethoxy-benzoyl)-3-(2-methylsulfanyl-ethyl)-1,4,8-triaza-spiro[4.5]decan-2-on,
(221) 1,3-Dibenzyl-8-(3,3-dimethyl-butyryl)-1,4,8-triaza-spiro[4.5]decan-2-on,
(222) 8-(2,6-Difluor-3-methyl-benzoyl)-1-methyl-3-(2-methylsulfanyl-ethyl)-1,4,8-triaza-spiro[4.5]decan-2-on,
(223) 2-[8-(2-Chlor-6-fluor-benzoyl)-3-(2-methylsulfanyl-ethyl)-2-oxo-1,4,8-triaza-spiro[4.5]dec-1-ylmethyl]-benzonitril,
(224) 8-[3-(2-Chlor-6-fluor-phenyl)-5-methyl-isoxazol-4-carbonyl]-3-isopropyl-1-(3-methyl-but-2-enyl)-1,4,8-triaza-spiro[4.5]decan-2-on,

(225) 3-Isobutyl-1-prop-2-ynyl-8-(4-trifluormethoxy-benzoyl)-1,4,8-triaza-spiro[4.5]decan-2-on,
(226) 1-Benzyl-8-(2-chlor-6-fluor-3-methyl-benzoyl)-1,4,8-triaza-spiro[4.5]decan-2-on,
(227) Benzoesäure 2-(1-benzyl-3-isopropyl-2-oxo-1,4,8-triaza-spiro[4.5]decan-8-carbonyl)-benzylester,
(228) 1,3-Dibenzyl-8-(2-phenyl-butyryl)-1,4,8-triaza-spiro[4.5]decan-2-on,
(229) 3-Benzyl-1-methyl-8-(4-nitro-benzoyl)-1,4,8-triaza-spiro[4.5]decan-2-on,
(230) 3-Benzyl-1-methyl-8-(5-methyl-isoxazol-3-carbonyl)-1,4,8-triaza-spiro[4.5]decan-2-on,
(231) 1-Benzyl-8-(6-chlor-2-fluor-3-methyl-benzoyl)-3-(2-methylsulfanyl-ethyl)-1,4,8-triaza-spiro[4.5]decan-2-on,
(232) 1-Benzyl-8-(3-fluor-4-trifluormethyl-benzoyl)-3-isobutyl-1,4,8-triaza-spiro[4.5]decan-2-on,
(233) 3-Benzyl-8-(6-chlor-2H-chroman-3-carbonyl)-1-methyl-1,4,8-triaza-spiro[4.5]decan-2-on,
(234) 3-Benzyl-1-butyl-8-(4-methyl-[1,2,3]thiadiazol-5-carbonyl)-1,4,8-triaza-spiro[4.5]decan-2-on,
(235) 3-Benzyl-8-(4-methyl-[1,2,3]thiadiazol-5-carbonyl)-1-prop-2-ynyl-1,4,8-triaza-spiro[4.5]decan-2-on,
(236) 2-[8-(6-Chlor-2H-chroman-3-carbonyl)-3-isopropyl-2-oxo-1,4,8-triaza-spiro[4.5]dec-1-ylmethyl]-benzonitril,
(237) 2-[8-(5-Methyl-isoxazol-3-carbonyl)-3-(2-methylsulfanyl-ethyl)-2-oxo-1,4,8-triaza-spiro[4.5]dec-1-ylmethyl]-benzonitril,
(238) 2-[8-(3-Chlor-2-fluor-benzoyl)-3-isopropyl-2-oxo-1,4,8-triaza-spiro[4.5]dec-1-ylmethyl]-benzonitril,
(239) 4-[8-(5-tert-Butyl-2-methyl-2H-pyrazol-3-carbonyl)-3-isopropyl-2-oxo-1,4,8-triaza-spiro[4.5]dec-1-ylmethyl]-benzoesäuremethylester,
(240) 3-Benzyl-1-butyl-8-(5-tert-butyl-2-methyl-2H-pyrazol-3-carbonyl)-1,4,8-triaza-spiro[4.5]decan-2-on,
(241) 3-Benzyl-1-butyl-8-(2-chlor-4-trifluormethyl-pyrimidin-5-carbonyl)-1,4,8-triaza-spiro[4.5]decan-2-on,
(242) 3-Benzyl-8-(5-methyl-isoxazol-3-carbonyl)-1-prop-2-ynyl-1,4,8-triaza-spiro[4.5]decan-2-on,
(243) 4-[8-(2-Chlor-pyridin-3-carbonyl)-2-oxo-1,4,8-triaza-spiro[4.5]dec-1-ylmethyl]-benzoesäuremethylester,
(244) 8-(2-tert-Butyl-5-methyl-2H-pyrazol-3-carbonyl)-1-(2-fluor-benzyl)-1,4,8-triaza-spiro[4.5]decan-2-on,
(245) 4-[8-(2-Methylsulfanyl-pyridin-3-carbonyl)-2-oxo-1,4,8-triaza-spiro[4.5]dec-1-ylmethyl]-benzoesäuremethylester,
(246) Essigsäure 4-[8-(4-acetylamino-benzoyl)-3-isobutyl-2-oxo-1,4,8-triaza-spiro[4.5]dec-1-yl]-butylester,
(247) [8-(4-Acetylamino-benzoyl)-3-benzyl-2-oxo-1,4,8-triaza-spiro[4.5]dec-1-yl]-essigsäureethylester,
(248) Essigsäure 4-[8-(2-ethylsulfanyl-pyridin-3-carbonyl)-3-isobutyl-2-oxo-1,4,8-triaza-spiro[4.5]dec-1-yl]-butylester,
(249) 4-[8-(4-Methyl-[1,2,3]thiadiazol-5-carbonyl)-2-oxo-1,4,8-triaza-spiro[4.5]dec-1-ylmethyl]-benzoesäuremethylester,
(250) 4-[1-Allyl-3-(2-methylsulfanyl-ethyl)-2-oxo-1,4,8-triaza-spiro[4.5]decan-8-carbonyl]-benzolsulfonamid,
(251) 4-(8-Cyclobutancarbonyl-2-oxo-1,4,8-triaza-spiro[4.5]dec-1-ylmethyl)-benzoesäuremethylester,
(252) Essigsäure 2-[1-allyl-3-(2-methylsulfanyl-ethyl)-2-oxo-1,4,8-triaza-spiro[4.5]dec-8-yl]-2-oxo-ethylester,
(253) 8-(Biphenyl-4-carbonyl)-1-(2-fluor-benzyl)-1,4,8-triaza-spiro[4.5]decan-2-on,
(254) [3-(2-Methylsulfanyl-ethyl)-2-oxo-8-propionyl-1,4,8-triaza-spiro[4.5]dec-1-yl]-essigsäureethylester,
(255) 8-(Benzo[1,3]dioxole-5-carbonyl)-1-(2,6-dichlor-benzyl)-3-isobutyl-1,4,8-triaza-spiro[4.5]decan-2-on,
(256) 1-Allyl-8-(biphenyl-4-carbonyl)-3-isopropyl-1,4,8-triaza-spiro[4.5]decan-2-on,
(257) [3-Benzyl-8-(biphenyl-4-carbonyl)-2-oxo-1,4,8-triaza-spiro[4.5]dec-1-yl]-essigsäureethylester,
(258) [8-(3-Dimethylamino-benzoyl)-3-(2-methylsulfanyl-ethyl)-2-oxo-1,4,8-triaza-spiro[4.5]dec-1-yl]-essigsäureethylester,
(259) 3-(2-Oxo-8-pentanoyl-1,4,8-triaza-spiro[4.5]dec-1-ylmethyl)-benzonitril,
(260) 4-(8-Cyclopentancarbonyl-2-oxo-1,4,8-triaza-spiro[4.5]dec-1-ylmethyl)-benzoesäuremethylester,
(261) 4-[1-(2-Fluor-benzyl)-2-oxo-1,4,8-triaza-spiro[4.5]dec-8-yl]-4-oxo-butansäurethylester,
(262) 1-(2-Fluor-benzyl)-8-(3,4,5-trimethoxy-benzoyl)-1,4,8-triaza-spiro[4.5]decan-2-on,
(263) 4-[8-(2-Chlor-pyridin-4-carbonyl)-2-oxo-1,4,8-triaza-spiro[4.5]dec-1-ylmethyl]-benzoesäuremethylester,
(264) 3-[8-(3,5-Bis-trifluormethyl-benzoyl)-2-oxo-1,4,8-triaza-spiro[4.5]dec-1-ylmethyl]-benzonitril,
(265) 3-Benzyl-1-(2-fluor-benzyl)-8-(2-methoxy-benzoyl)-1,4,8-triaza-spiro[4.5]decan-2-on,
(266) 8-(Benzo[1,2,5]oxadiazol-5-carbonyl)-1-(2,6-dichlor-benzyl)-3-isobutyl-1,4,8-triaza-spiro[4.5]decan-2-on,
(267) [3-Benzyl-2-oxo-8-(4-sulfamoyl-benzoyl)-1,4,8-triaza-spiro[4.5]dec-1-yl]-essigsäureethylester,
(268) 3-[2-Oxo-8-(3,4,5-trimethoxy-benzoyl)-1,4,8-triaza-spiro[4.5]dec-1-ylmethyl]-benzonitril,
(269) Essigsäure 2-[1-(4-acetoxy-butyl)-3-isobutyl-2-oxo-1,4,8-triaza-spiro[4.5]dec-8-yl]-1,1-dimethyl-2-oxo-ethylester,
(270) 8-(6-Chlor-pyridin-3-carbonyl)-1-(2,6-dichlor-benzyl)-3-isobutyl-1,4,8-triaza-spiro[4.5]decan-2-on,
(271) 8-(2-Ethoxy-benzoyl)-1-(2-fluor-benzyl)-1,4,8-triaza-spiro[4.5]decan-2-on,
(272) 1-Allyl-8-cyclopropancarbonyl-3-(2-methylsulfanyl-ethyl)-1,4,8-triaza-spiro[4.5]decan-2-on,

(273) 3-[3-Isopropyl-8-(2-methoxy-acetyl)-2-oxo-1,4,8-triaza-spiro[4.5]dec-1-ylmethyl]-benzonitril,
(274) 4-(2-Oxo-8-phenylacetyl-1,4,8-triaza-spiro[4.5]dec-1-ylmethyl)-benzoesäuremethylester,
(275) Essigsäure 2-[1-(3-cyano-benzyl)-2-oxo-1,4,8-triaza-spiro[4.5]dec-8-yl]-2-oxo-1-phenyl-ethylester,
(276) 1-(2-Fluor-benzyl)-8-(4-methoxy-benzoyl)-1,4,8-triaza-spiro[4.5]decan-2-on,
(277) 4-(8-Cyclohexancarbonyl-2-oxo-1,4,8-triaza-spiro[4.5]dec-1-ylmethyl)-benzoesäuremethylester,
(278) 1-(2-Fluor-benzyl)-8-pent-4-enoyl-1,4,8-triaza-spiro[4.5]decan-2-on,
(279) [3-Isobutyl-8-(3-methyl-butyryl)-2-oxo-1,4,8-triaza-spiro[4.5]dec-1-yl]-essigsäureethylester,
(280) 1-Allyl-8-(3,3-dimethyl-butyryl)-3-(2-methylsulfanyl-ethyl)-1,4,8-triaza-spiro[4.5]decan-2-on,
(281) 1-Allyl-3-(2-methylsulfanyl-ethyl)-8-(2-methylsulfanyl-pyridin-3-carbonyl)-1,4,8-triaza-spiro[4.5]decan-2-on,
(282) [3-Isobutyl-8-(2-methyl-pentanoyl)-2-oxo-1,4,8-triaza-spiro[4.5]dec-1-yl]-essigsäureethylester,
(283) 4-[8-(5-tert-Butyl-2-methyl-furan-3-carbonyl)-2-oxo-1,4,8-triaza-spiro[4.5]dec-1-ylmethyl]-benzoesäuremethylester,
(284) (3-Benzyl-8-Cyclopropancarbonyl-2-oxo-1,4,8-triaza-spiro[4.5]dec-1-yl)-essigsäureethylester,
(285) [3-Benzyl-8-(3-methyl-butyryl)-2-oxo-1,4,8-triaza-spiro[4.5]dec-1-yl]-essigsäureethylester,
(286) 1-(2,6-Dichlor-benzyl)-8-(2,5-dimethyl-furan-3-carbonyl)-3-isobutyl-1,4,8-triaza-spiro[4.5]decan-2-on,
(287) 1-Allyl-3-isopropyl-8-[2-(3-methoxy-phenyl)-acetyl]-1,4,8-triaza-spiro[4.5]decan-2-on,
(288) [8-(4-tert-Butyl-benzoyl)-3-(2-methylsulfanyl-ethyl)-2-oxo-1,4,8-triaza-spiro[4.5]dec-1-yl]-essigsäureethylester,
(289) 3-(2-Methylsulfanyl-ethyl)-1-(2-nitro-benzyl)-8-(2-phenoxy-pyridin-3-carbonyl)-1,4,8-triaza-spiro[4.5]decan-2-on,
(290) (3-Benzyl-2-oxo-8-pentanoyl-1,4,8-triaza-spiro[4.5]dec-1-yl)-essigsäureethylester,
(291) 8-(2-Chlor-pyridin-4-carbonyl)-1-(2-fluor-benzyl)-1,4,8-triaza-spiro[4.5]decan-2-on,
(292) [8-(3-Methyl-butyryl)-3-(2-methylsulfanyl-ethyl)-2-oxo-1,4,8-triaza-spiro[4.5]dec-1-yl]-essigsäureethylester,
(293) 1-Allyl-3-(2-methylsulfanyl-ethyl)-8-pentafluorbenzoyl-1,4,8-triaza-spiro[4.5]decan-2-on,
(294) 1-(2-Fluor-benzyl)-8-(2-trifluormethyl-benzoyl)-1,4,8-triaza-spiro[4.5]decan-2-on,
(295) 8-(Benzo[1,2,5]oxadiazol-5-carbonyl)-1-(2-fluor-benzyl)-1,4,8-triaza-spiro[4.5]decan-2-on,
(296) 4-[8-(4-tert-Butyl-benzoyl)-2-oxo-1,4,8-triaza-spiro[4.5]dec-1-ylmethyl]-benzoesäuremethylester,
(297) [3-Benzyl-2-oxo-8-(1-phenyl-5-propyl-1H-pyrazol-4-carbonyl)-1,4,8-triaza-spiro[4.5]dec-1-yl]-essigsäureethylester,
(298) 1-Allyl-3-isopropyl-8-pent-4-enoyl-1,4,8-triaza-spiro[4.5]decan-2-on,
(299) 3-[2-Oxo-8-(2-trifluormethyl-benzoyl)-1,4,8-triaza-spiro[4.5]dec-1-ylmethyl]-benzonitril,
(300) 8-(2-Dimethylamino-acetyl)-1-(3,5-dimethyl-benzyl)-3-isobutyl-1,4,8-triaza-spiro[4.5]decan-2-on,
(301) Essigsäure 4-[8-(2-ethoxy-benzoyl)-3-isobutyl-2-oxo-1,4,8-triaza-spiro[4.5]dec-1-yl]-butylester,
(302) 1-(2-Fluor-benzyl)-8-(3-methoxy-benzoyl)-1,4,8-triaza-spiro[4.5]decan-2-on,
(303) [8-(Furan-2-carbonyl)-3-isobutyl-2-oxo-1,4,8-triaza-spiro[4.5]dec-1-yl]-essigsäureethylester,
(304) 8-(5-tert-Butyl-2-methyl-2H-pyrazol-3-carbonyl)-1-(2-fluor-benzyl)-1,4,8-triaza-spiro[4.5]decan-2-on,
(305) 1-(2-Fluor-benzyl)-8-(thiophen-2-carbonyl)-1,4,8-triaza-spiro[4.5]decan-2-on,
(306) [3-Benzyl-8-(2-fluor-benzoyl)-2-oxo-1,4,8-triaza-spiro[4.5]dec-1-yl]-essigsäureethylester,
(307) [8-(Isoxazol-5-carbonyl)-3-(2-methylsulfanyl-ethyl)-2-oxo-1,4,8-triaza-spiro[4.5]dec-1-yl]-essigsäureethylester,
(308) 1-(2,6-Dichlor-benzyl)-3-isobutyl-8-(4-propyl-benzoyl)-1,4,8-triaza-spiro[4.5]decan-2-on,
(309) [8-[2-(2,5-Dimethoxy-phenyl)-acetyl]-3-(2-methylsulfanyl-ethyl)-2-oxo-1,4,8-triaza-spiro[4.5]dec-1-yl]-essigsäureethylester,
(310) (3-Benzyl-8-cyclobutancarbonyl-2-oxo-1,4,8-triaza-spiro[4.5]dec-1-yl)-essigsäureethylester,
(311) 1-(2-Fluor-benzyl)-8-[2-(4-methoxy-phenyl)-acetyl]-1,4,8-triaza-spiro[4.5]decan-2-on,
(312) 1-Allyl-3-(2-methylsulfanyl-ethyl)-8-(2-methyl-6-trifluormethyl-pyridin-3-carbonyl)-1,4,8-triaza-spiro[4.5]decan-2-on,
(313) [3-(2-Methylsulfanyl-ethyl)-2-oxo-8-(4-trifluormethyl-benzoyl)-1,4,8-triaza-spiro[4.5]dec-1-yl]-essigsäureethylester,
(314) 1-Allyl-3-(2-methylsulfanyl-ethyl)-8-(1-phenyl-5-propyl-1H-pyrazol-4-carbonyl)-1,4,8-triaza-spiro[4.5]decan-2-on,
(315) 1-(2,6-Dichlor-benzyl)-8-(2,3-difluor-4-methyl-benzoyl)-3-isobutyl-1,4,8-triaza-spiro[4.5]decan-2-on,
(316) 1-Allyl-3-isopropyl-8-(2-methyl-benzoyl)-1,4,8-triaza-spiro[4.5]decan-2-on,
(317) 8-(2-Chlor-5-trifluormethyl-benzoyl)-1-(2,6-dichlor-benzyl)-3-isobutyl-1,4,8-triaza-spiro[4.5]decan-2-on,
(318) 1-Allyl-8-[2-(3-methoxy-phenyl)-acetyl]-3-(2-methylsulfanyl-ethyl)-1,4,8-triaza-spiro[4.5]decan-2-on,
(319) 1-Allyl-3-(2-methylsulfanyl-ethyl)-8-(3-phenyl-propionyl)-1,4,8-triaza-spiro[4.5]decan-2-on,

(320) [3-Benzyl-8-(2-benzyloxy-acetyl)-2-oxo-1,4,8-triaza-spiro[4.5]dec-1-yl]-essigsäureethylester,
(321) 1-Allyl-8-(5-tert-butyl-2-methyl-2H-pyrazol-3-carbonyl)-3-(2-methylsulfanyl-ethyl)-1,4,8-triaza-spiro[4.5]decan-2-on,
(322) Essigsäure 1,1-dimethyl-2-[3-(2-methylsulfanyl-ethyl)-1-(2-nitro-benzyl)-2-oxo-1,4,8-triaza-spiro[4.5]dec-8-yl]-2-oxo-ethylester,
(323) Benzoesäure 2-[1-ethoxycarbonylmethyl-3-(2-methylsulfanyl-ethyl)-2-oxo-1,4,8-triaza-spiro[4.5]decan-8-carbonyl]-benzylester,
(324) 1-Allyl-3-isopropyl-8-(4-methyl-[1,2,3]thiadiazol-5-carbonyl)-1,4,8-triaza-spiro[4.5]decan-2-on,
(325) 1-Allyl-8-(2,5-dimethyl-furan-3-carbonyl)-3-(2-methylsulfanyl-ethyl)-1,4,8-triaza-spiro[4.5]decan-2-on,
(326) 1-Allyl-8-(benzo[1,2,5]oxadiazol-5-carbonyl)-3-(2-methylsulfanyl-ethyl)-1,4,8-triaza-spiro[4.5]decan-2-on,
(327) 1-(2-Fluor-benzyl)-8-(4-phenoxy-butyryl)-1,4,8-triaza-spiro[4.5]decan-2-on,
(328) 1-Allyl-8-(2-cyclopentyl-acetyl)-3-(2-methylsulfanyl-ethyl)-1,4,8-triaza-spiro[4.5]decan-2-on,
(329) [3-Benzyl-8-(naphthalin-2-carbonyl)-2-oxo-1,4,8-triaza-spiro[4.5]dec-1-yl]-essigsäureethylester,
(330) 8-[3-(2-Chlor-6-fluor-phenyl)-5-methyl-isoxazol-4-carbonyl]-1-(2-fluor-benzyl)-1,4,8-triaza-spiro[4.5]decan-2-on,
(331) 3-[8-(3,5-Dimethoxy-benzoyl)-3-isopropyl-2-oxo-1,4,8-triaza-spiro[4.5]dec-1-ylmethyl]-benzonitril,
(332) 3-(2-Methylsulfanyl-ethyl)-1-(2-nitro-benzyl)-8-(1-phenyl-5-propyl-1H-pyrazol-4-carbonyl)-1,4,8-triaza-spiro[4.5]decan-2-on,
(333) {3-Benzyl-8-[3-(2-chlor-6-fluor-phenyl)-5-methyl-isoxazol-4-carbonyl]-2-oxo-1,4,8-triaza-spiro[4.5]dec-1-yl}-essigsäureethylester,
(334) 1-(2-Fluor-benzyl)-8-(3-fluor-4-trifluormethyl-benzoyl)-1,4,8-triaza-spiro[4.5]decan-2-on,
(335) [8-[1-(4-Chlor-phenyl)-5-trifluormethyl-1H-pyrazol-4-carbonyl]-3-(2-methylsulfanyl-ethyl)-2-oxo-1,4,8-triaza-spiro[4.5]dec-1-yl]-essigsäureethylester,
(336) 3-[8-(Naphthalin-1-carbonyl)-2-oxo-1,4,8-triaza-spiro[4.5]dec-1-ylmethyl]-benzonitril,
(337) [8-(3,3-Dimethyl-butyryl)-3-(2-methylsulfanyl-ethyl)-2-oxo-1,4,8-triaza-spiro[4.5]dec-1-yl]-essigsäureethylester,
(338) 8-Acetyl-3-(2-methylsulfanyl-ethyl)-1-naphthalin-2-ylmethyl-1,4,8-triaza-spiro[4.5]decan-2-on,
(339) [3-Benzyl-8-(3-cyano-benzoyl)-2-oxo-1,4,8-triaza-spiro[4.5]dec-1-yl]-essigsäureethylester,
(340) 8-(5-tert-Butyl-2-methyl-2H-pyrazol-3-carbonyl)-1-(2,6-dichlor-benzyl)-3-isobutyl-1,4,8-triaza-spiro[4.5]decan-2-on,
(341) 4-[3-Benzyl-1-(2-fluor-benzyl)-2-oxo-1,4,8-triaza-spiro[4.5]decan-8-carbonyl]-benzonitril,
(342) 4-[3-Benzyl-1-(2-fluor-benzyl)-2-oxo-1,4,8-triaza-spiro[4.5]dec-8-yl]-4-oxo-butyrsäure methylester,
(343) 3-(2-Methylsulfanyl-ethyl)-1-naphthalin-2-ylmethyl-8-(3,4,5-trimethoxy-benzoyl)-1,4,8-triaza-spiro[4.5]decan-2-on,
(344) [3-Benzyl-8-(isoxazol-5-carbonyl)-2-oxo-1,4,8-triaza-spiro[4.5]dec-1-yl]-essigsäureethylester,
(345) 8-(3-Difluormethylsulfanyl-benzoyl)-1-(2-fluor-benzyl)-1,4,8-triaza-spiro[4.5]decan-2-on,
(346) [3-Benzyl-8-(2,3-dimethyl-benzoyl)-2-oxo-1,4,8-triaza-spiro[4.5]dec-1-yl]-essigsäureethylester,
(347) 1-(2-Fluor-benzyl)-8-(4-propyl-benzoyl)-1,4,8-triaza-spiro[4.5]decan-2-on,
(348) 3-[8-(4-Iod-benzoyl)-2-oxo-1,4,8-triaza-spiro[4.5]dec-1-ylmethyl]-benzonitril,
(349) 1-(2-Ftuor-benzyl)-8-(2-propyl-pentanoyl)-1,4,8-triaza-spiro[4.5]decan-2-on,
(350) 3-[8-(2,6-Difluor-3-methyl-benzoyl)-3-isopropyl-2-oxo-1,4,8-triaza-spiro[4.5]dec-1-ylmethyl]-benzonitril,
(351) 3-(2-Methylsulfanyl-ethyl)-8-(4-methyl-[1,2,3]thiadiazol-5-carbonyl)-1-(2-nitro-benzyl)-1,4,8-triaza-spiro[4.5]decan-2-on,
(352) Essigsäure 4-[3-isobutyl-8-(4-methyl-[1,2,3]thiadiazol-5-carbonyl)-2-oxo-1,4,8-triaza-spiro[4.5]dec-1-yl]-butylester,
(353) 8-[2-(4-Chlor-phenoxy)-acetyl]-3-(2-methylsulfanyl-ethyl)-1-naphthalin-2-ylmethyl-1,4,8-triaza-spiro[4.5]decan-2-on,
(354) [3-Benzyl-2-oxo-8-(3-trifluormethyl-benzoyl)-1,4,8-triaza-spiro[4.5]dec-1-yl]-essigsäureethylester,
(355) 8-(4-Brom-benzoyl)-3-(2-methylsulfanyl-ethyl)-1-naphthalin-2-ylmethyl-1,4,8-triaza-spiro[4.5]decan-2-on,
(356) [8-(2-Chlor-pyridin-4-carbonyl)-3-(2-methylsulfanyl-ethyl)-2-oxo-1,4,8-triaza-spiro[4.5]dec-1-yl]-essigsäureethylester,
(357) 1-Allyl-8-(3,5-dimethoxy-benzoyl)-3-(2-methylsulfanyl-ethyl)-1,4,8-triaza-spiro[4.5]decan-2-on,
(358) 3-[8-(4-Methyl-3-nitro-benzoyl)-2-oxo-1,4,8-triaza-spiro[4.5]dec-1-ylmethyl]-benzonitril,
(359) 8-(4-tert-Butyl-benzoyl)-1-(2-fluor-benzyl)-1,4,8-triaza-spiro[4.5]decan-2-on,
(360) 1-Allyl-3-isopropyl-8-(5-methyl-isoxazol-3-carbonyl)-1,4,8-triaza-spiro[4.5]decan-2-on,
(361) 1-Allyl-3-(2-methylsulfanyl-ethyl)-8-(2-trifluormethyl-benzoyl)-1,4,8-triaza-spiro[4.5]decan-2-on,

(362) [3-Benzyl-8-(3-cyclopentyl-propionyl)-2-oxo-1,4,8-triaza-spiro[4.5]dec-1-yl]-essigsäureethylester,
(363) 3-Benzyl-8-(3,5-difluor-benzoyl)-1-(2-fluor-benzyl)-1,4,8-triaza-spiro[4.5]decan-2-on,
(364) [3-Benzyl-8-(5-fluor-2-trifluormethyl-benzoyl)-2-oxo-1,4,8-triaza-spiro[4.5]dec-1-yl]-essigsäureethylester,
(365) [3-Benzyl-2-oxo-8-(3,4,5-trimethoxy-benzoyl)-1,4,8-triaza-spiro[4.5]dec-1-yl]-essigsäureethylester,
(366) Benzoesäure 2-[1-(2-fluor-benzyl)-2-oxo-1,4,8-triaza-spiro[4.5]decan-8-carbonyl]-benzylester,
(367) 1-(2-Fluor-benzyl)-8-(2-methyl-benzoyl)-1,4,8-triaza-spiro[4.5]decan-2-on,
(368) 1-(2-Fluor-benzyl)-8-(2-phenyl-butyryl)-1,4,8-triaza-spiro[4.5]decan-2-on,
(369) 1-Allyl-8-(6-chlor-pyridin-3-carbonyl)-3-isopropyl-1,4,8-triaza-spiro[4.5]decan-2-on,
(370) [8-(2,5-Dimethyl-furan-3-carbonyl)-3-(2-methylsulfanyl-ethyl)-2-oxo-1,4,8-triaza-spiro[4.5]dec-1-yl]-essigsäureethylester,
(371) [8-(5-tert-Butyl-2-methyl-2H-pyrazol-3-carbonyl)-3-(2-methylsulfanyl-ethyl)-2-oxo-1,4,8-triaza-spiro[4.5]dec-1-yl]-essigsäureethylester,
(372) Benzoesäure 2-[1-allyl-3-(2-methylsulfanyl-ethyl)-2-oxo-1,4,8-triaza-spiro[4.5]decan-8-carbonyl]-benzylester,
(373) Essigsäure 2-[1-allyl-3-(2-methylsulfanyl-ethyl)-2-oxo-1,4,8-triaza-spiro[4.5]dec-8-yl]-2-oxo-1-phenylethylester,
(374) 3-[8-(2-Chlor-6-fluor-benzoyl)-3-isopropyl-2-oxo-1,4,8-triaza-spiro[4.5]dec-1-ylmethyl]-benzonitril,
(375) 3-(2-Methylsulfanyl-ethyl)-1-(2-nitro-benzyl)-8-(3-phenyl-propionyl)-1,4,8-triaza-spiro[4.5]decan-2-on,
(376) 3-[8-(2,3-Dimethyl-benzoyl)-3-isopropyl-2-oxo-1,4,8-triaza-spiro[4.5]dec-1-ylmethyl]-benzonitril,
(377) [8-(5-Fluor-2-trifluormethyl-benzoyl)-3-(2-methylsulfanyl-ethyl)-2-oxo-1,4,8-triaza-spiro[4.5]dec-1-yl]-essigsäureethylester,
(378) [8-Cyclopentancarbonyl-3-(2-methylsulfanyl-ethyl)-2-oxo-1,4,8-triaza-spiro[4.5]dec-1-yl]-essigsäureethylester,
(379) [3-(2-Methylsulfanyl-ethyl)-2-oxo-8-(thiophen-2-carbonyl)-1,4,8-triaza-spiro[4.5]dec-1-yl]-essigsäureethylester,
(380) 1-Allyl-8-[1-(4-chlor-phenyl)-5-trifluormethyl-1H-pyrazol-4-carbonyl]-3-(2-methylsulfanyl-ethyl)-1,4,8-triaza-spiro[4.5]decan-2-on,
(381) 1-Allyl-3-(2-methylsulfanyl-ethyl)-8-(naphthalin-1-carbonyl)-1,4,8-triaza-spiro[4.5]decan-2-on,
(382) 1-(2-fluor-benzyl)-8-(4-trifluormethoxy-benzoyl)-1,4,8-triaza-spiro[4.5]decan-2-on,
(383) 3-Benzyl-8-(2-benzyloxy-acetyl)-1-(2-fluor-benzyl)-1,4,8-triaza-spiro[4.5]decan-2-on,
(384) 3-[8-(2-chlor-6-fluor-3-methyl-benzoyl)-3-isopropyl-2-oxo-1,4,8-triaza-spiro[4.5]dec-1-ylmethyl]-benzonitril,
(385) 1-Allyl-8-(2-chlor-5-trifluormethyl-benzoyl)-3-isopropyl-1,4,8-triaza-spiro[4.5]decan-2-on,
(386) 8-(3-Methyl-benzoyl)-3-(2-methylsulfanyl-ethyl)-1-naphthalin-2-ylmethyl-1,4,8-triaza-spiro[4.5]decan-2-on,
(387) 1-(3,5-Dimethyl-benzyl)-8-(2-ethyl-butyryl)-3-isobutyl-1,4,8-triaza-spiro[4.5]decan-2-on,
(388) 8-(5-tert-Butyl-2-methyl-2H-pyrazol-3-carbonyl)-3-(2-methylsulfanyl-ethyl)-1-(2-nitro-benzyl)-1,4,8-triaza-spiro[4.5]decan-2-on,
(389) Benzoesäure 2-[1-(3-cyano-benzyl)-3-isopropyl-2-oxo-1,4,8-triaza-spiro[4.5]decan-8-carbonyl]-benzylester,
(390) 8-(4-Methyl-3-nitro-benzoyl)-3-(2-methylsulfanyl-ethyl)-1-(2-nitro-benzyl)-1,4,8-triaza-spiro[4.5]decan-2-on,
(391) 1-(2,6-Dichlor-benzyl)-3-isobutyl-8-(3,4,5-trimethoxy-benzoyl)-1,4,8-triaza-spiro[4.5]decan-2-on,
(392) 3-{8-[2-(2-Brom-phenyl)-acetyl]-3-isopropyl-2-oxo-1,4,8-triaza-spiro[4.5]dec-1-ylmethyl}-benzonitril,
(393) 3-[8-(2,3-Dichlor-benzoyl)-3-isopropyl-2-oxo-1,4,8-triaza-spiro[4.5]dec-1-ylmethyl]-benzonitril,
(394) 1-Allyl-8-(6-chlor-2H-chroman-3-carbonyl)-3-isopropyl-1,4,8-triaza-spiro[4.5]decan-2-on,
(395) {3-Benzyl-8-[2-(4-methoxy-phenyl)-acetyl]-2-oxo-1,4,8-triaza-spiro[4.5]dec-1-yl}-essigsäureethylester,
(396) 3-[3-Isopropyl-2-oxo-8-(1-phenyl-5-propyl-1H-pyrazol-4-carbonyl)-1,4,8-triaza-spiro[4.5]dec-1-ylmethyl]-benzonitril,
(397) 8-(3-Cyclopentyl-propionyl)-1-(2-fluor-benzyl)-1,4,8-triaza-spiro[4.5]decan-2-on,
(398) 1-Allyl-8-(3-difluormethylsulfanyl-benzoyl)-3-isopropyl-1,4,8-triaza-spiro[4.5]decan-2-on,
(399) 3-[8-(2-chlor-4-nitro-benzoyl)-3-isopropyl-2-oxo-1,4,8-triaza-spiro[4.5]dec-1-ylmethyl]-benzonitril,
(400) [8-(2-Ethoxy-benzoyl)-3-(2-methylsulfanyl-ethyl)-2-oxo-1,4,8-triaza-spiro[4.5]dec-1-yl]-essigsäureethylester,
(401) 8-(2,5-Bis-trifluormethyl-benzoyl)-1-(2-fluor-benzyl)-1,4,8-triaza-spiro[4.5]decan-2-on,
(402) 8-(2-Chlor-pyridin-4-carbonyl)-3-(2-methylsulfanyl-ethyl)-1-(2-nitro-benzyl)-1,4,8-triaza-spiro[4.5]decan-2-on,

(403) {3-Benzyl-8-[3-(2,6-dichlor-phenyl)-5-methyl-isoxazol-4-carbonyl]-2-oxo-1,4,8-triaza-spiro[4.5]dec-1-yl}-essigsäureethylester,
(404) 1-(3,5-Dimethyl-benzyl)-3-isobutyl-8-(2-trifluormethyl-benzoyl)-1,4,8-triaza-spiro[4.5]decan-2-on,
(405) 8-(3,5-Dimethoxy-benzoyl)-3-(2-methylsulfanyl-ethyl)-1-(2-nitro-benzyl)-1,4,8-triaza-spiro[4.5]decan-2-on,
(406) Benzoesäure 2-[3-benzyl-1-(2-fluor-benzyl)-2-oxo-1,4,8-triaza-spiro[4.5]decan-8-carbonyl]-benzylester,
(407) 3-[8-(Benzo[1,2,5]oxadiazol-5-carbonyl)-3-isopropyl-2-oxo-1,4,8-triaza-spiro[4.5]dec-1-ylmethyl]-benzonitril,
(408) [3-(2-Methylsulfanyl-ethyl)-2-oxo-8-(4-trifluormethoxy-benzoyl)-1,4,8-triaza-spiro[4.5]dec-1-yl]-essigsäureethylester,
(409) 1-Allyl-3-isopropyl-8-[2-(4-methoxy-phenyl)-acetyl]-1,4,8-triaza-spiro[4.5]decan-2-on,
(410) 3-(2-Methylsulfanyl-ethyl)-1-(2-nitro-benzyl)-8-(4-propyl-benzoyl)-1,4,8-triaza-spiro[4.5]decan-2-on,
(411) 3-Benzyl-8-(4-tert-butyl-benzoyl)-1-(2-fluor-benzyl)-1,4,8-triaza-spiro[4.5]decan-2-on,
(412) 1-Allyl-8-(2,6-difluor-3-methyl-benzoyl)-3-(2-methylsulfanyl-ethyl)-1,4,8-triaza-spiro[4.5]decan-2-on,
(413) 8-(3,5-Bis-trifluormethyl-benzoyl)-1-(2-fluor-benzyl)-1,4,8-triaza-spiro[4.5]decan-2-on,
(414) 3-[8-(4-Iod-benzoyl)-3-isopropyl-2-oxo-1,4,8-triaza-spiro[4.5]dec-1-ylmethyl]-benzonitril,
(415) 3-(2-Methylsulfanyl-ethyl)-1-naphthalin-2-ylmethyl-8-(thiophen-2-carbonyl)-1,4,8-triaza-spiro[4.5]decan-2-on,
(416) [3-Benzyl-8-(2-chlor-pyridin-4-carbonyl)-2-oxo-1,4,8-triaza-spiro[4.5]dec-1-yl]-essigsäureethylester,
(417) [3-(2-Methylsulfanyl-ethyl)-8-(4-nitro-benzoyl)-2-oxo-1,4,8-triaza-spiro[4.5]dec-1-yl]-essigsäureethylester,
(418) 8-(2-Ethyl-hexanoyl)-3-(2-methylsulfanyl-ethyl)-1-(2-nitro-benzyl)-1,4,8-triaza-spiro[4.5]decan-2-on,
(419) 1-(2,6-Dichlor-benzyl)-3-isobutyl-8-(thiophen-2-carbonyl)-1,4,8-triaza-spiro[4.5]decan-2-on,
(420) [3-Benzyl-8-(2-chlor-4-nitro-benzoyl)-2-oxo-1,4,8-triaza-spiro[4.5]dec-1-yl]-essigsäureethylester,
(421) 3-Benzyl-8-(3,5-bis-trifluormethyl-benzoyl)-1-(2-fluor-benzyl)-1,4,8-triaza-spiro[4.5]decan-2-on,
(422) [3-Benzyl-8-(4-brom-3-methyl-benzoyl)-2-oxo-1,4,8-triaza-spiro[4.5]dec-1-yl]-essigsäureethylester,
(423) 8-Cyclohexancarbonyl-3-(2-methylsulfanyl-ethyl)-1-(2-nitro-benzyl)-1,4,8-triaza-spiro[4.5]decan-2-on,
(424) 8-(2,5-Dimethyl-furan-3-carbonyl)-3-(2-methylsulfanyl-ethyl)-1-(2-nitro-benzyl)-1,4,8-triaza-spiro[4.5]decan-2-on,
(425) 8-(3-Difluormethylsulfanyl-benzoyl)-3-(2-methylsulfanyl-ethyl)-1-(2-nitro-benzyl)-1,4,8-triaza-spiro[4.5]decan-2-on,
(426) 8-(Furan-2-carbonyl)-3-(2-methylsulfanyl-ethyl)-1-(2-nitro-benzyl)-1,4,8-triaza-spiro[4.5]decan-2-on,
(427) [3-Benzyl-8-(2,3-difluor-benzoyl)-2-oxo-1,4,8-triaza-spiro[4.5]dec-1-yl]-essigsäureethylester,
(428) 3-[3-Isopropyl-8-(4-methyl-[1,2,3]thiadiazol-5-carbonyl)-2-oxo-1,4,8-triaza-spiro[4.5]dec-1-ylmethyl]-benzonitril,
(429) 3-[8-(3-Chlor-2-fluor-benzoyl)-3-isopropyl-2-oxo-1,4,8-triaza-spiro[4.5]dec-1-ylmethyl]-benzonitril,
(430) [3-Benzyl-8-(naphthalin-1-carbonyl)-2-oxo-1,4,8-triaza-spiro[4.5]dec-1-yl]-essigsäureethylester,
(431) 8-(4-tert-Butyl-benzoyl)-3-(2-methylsulfanyl-ethyl)-1-(2-nitro-benzyl)-1,4,8-triaza-spiro[4.5]decan-2-on,
(432) 3-[3-Benzyl-1-(2-fluor-benzyl)-2-oxo-1,4,8-triaza-spiro[4.5]decan-8-carbonyl]-benzonitril,
(433) 1-(2,6-Dichlor-benzyl)-8-(furan-2-carbonyl)-3-isobutyl-1,4,8-triaza-spiro[4.5]decan-2-on,
(434) 8-(2,6-Dichlor-benzoyl)-1-(2-fluor-benzyl)-1,4,8-triaza-spiro[4.5]decan-2-on,
(435) 1-(3,5-Dimethyl-benzyl)-8-(3-fluor-4-trifluormethyl-benzoyl)-3-isobutyl-1,4,8-triaza-spiro[4.5]decan-2-on,
(436) 1-(2-Fluor-benzyl)-8-(3-fluor-4-methyl-benzoyl)-1,4,8-triaza-spiro[4.5]decan-2-on,
(437) 1-Allyl-8-(3-cyclopentyl-propionyl)-3-(2-methylsulfanyl-ethyl)-1,4,8-triaza-spiro[4.5]decan-2-on,
(438) 8-[2-(3-Chlor-phenoxy)-acetyl]-3-(2-methylsulfanyl-ethyl)-1-(2-nitro-benzyl)-1,4,8-triaza-spiro[4.5]decan-2-on,
(439) 1-(3,5-Dimethyl-benzyl)-3-isobutyl-8-(2-methylsulfanyl-pyridin-3-carbonyl)-1,4,8-triaza-spiro[4.5]decan-2-on,
(440) 3-(2-Methylsulfanyl-ethyl)-1-(2-nitro-benzyl)-8-propionyl-1,4,8-triaza-spiro[4.5]decan-2-on,
(441) 3-[8-(3,4-Dimethoxy-benzoyl)-3-isopropyl-2-oxo-1,4,8-triaza-spiro[4.5]dec-1-ylmethyl]-benzonitril,
(442) 3-(2-Methylsulfanyl-ethyl)-8-(naphthalin-2-carbonyl)-1-naphthalin-2-ylmethyl-1,4,8-triaza-spiro[4.5]decan-2-on,
(443) 8-(6-Chlor-2H-chroman-3-carbonyl)-3-(2-methylsulfanyl-ethyl)-1-(2-nitro-benzyl)-1,4,8-triaza-spiro[4.5]decan-2-on,
(444) {3-Benzyl-2-oxo-8-[3-(3-trifluormethyl-phenyl)-acryloyl]-1,4,8-triaza-spiro[4.5]dec-1-yl}-essigsäureethylester,
(445) 1-(3,5-Dimethyl-benzyl)-3-isobutyl-8-(3-phenyl-acryloyl)-1,4,8-triaza-spiro[4.5]decan-2-on,
(446) 3-[3-Isopropyl-2-oxo-8-(2-phenoxy-propionyl)-1,4,8-triaza-spiro[4.5]dec-1-ylmethyl]-benzonitril,

(447) 3-[3-Isopropyl-2-oxo-8-(4-trifluormethoxy-benzoyl)-1,4,8-triaza-spiro[4.5]dec-1-ylmethyl]-benzonitril,
(448) 1-Allyl-3-(2-methylsulfanyl-ethyl)-8-(3-nitro-benzoyl)-1,4,8-triaza-spiro[4.5]decan-2-on,
(449) [3-Benzyl-8-(3,4-dichlor-benzoyl)-2-oxo-1,4,8-triaza-spiro[4.5]dec-1-yl]-essigsäureethylester,
(450) 1-(2-Fluor-benzyl)-8-[3-(3-trifluormethyl-phenyl)-acryloyl]-1,4,8-triaza-spiro[4.5]decan-2-on,
(451) 1-Allyl-8-(3,5-bis-trifluormethyl-benzoyl)-3-(2-methylsulfanyl-ethyl)-1,4,8-triaza-spiro[4.5]decan-2-on,
(452) 8-(3-Cyclopentyl-propionyl)-3-(2-methylsulfanyl-ethyl)-1-(2-nitro-benzyl)-1,4,8-triaza-spiro[4.5]decan-2-on,
(453) 1-(3,5-Dimethyl-benzyl)-3-isobutyl-8-(4-nitro-benzoyl)-1,4,8-triaza-spiro[4.5]decan-2-on,
(454) 8-(3-Cyclopentyl-propionyl)-1-(3,5-dimethyl-benzyl)-3-isobutyl-1,4,8-triaza-spiro[4.5]decan-2-on,
(455) 3-[3-Isopropyl-8-(isoxazol-5-carbonyl)-2-oxo-1,4,8-triaza-spiro[4.5]dec-1-ylmethyl]-benzonitril und
(456) 3-Benzyl-1-(2-fluor-benzyl)-8-(2-methyl-6-trifluormethyl-pyridin-3-carbonyl)-1,4,8-triaza-spiro[4.5]decan-2-on,

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, vorzugsweise Hydrochloride, oder jeweils in Form entsprechender Solvate.

**14.** Verfahren zur Herstellung substituierter 1,4,8-Triazaspiro[4.5]decan-2-on-Verbindungen gemäß einem oder mehreren der Ansprüche 1 bis 13, **dadurch gekennzeichnet, daß** ein geschütztes Piperidin-4-on der allgemeinen Formel II,

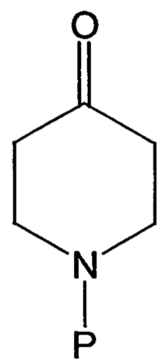

II

worin P für eine Schutzgruppe steht, durch Umsetzung mit wenigstens einer Verbindung der allgemeinen Formel III,

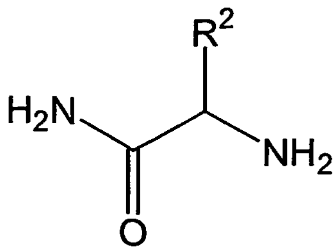

III

worin $R^2$ die Bedeutung gemäß einem oder mehreren der Ansprüche 1 bis 13 hat, in wenigstens eine Verbindung der allgemeinen Formel IV,

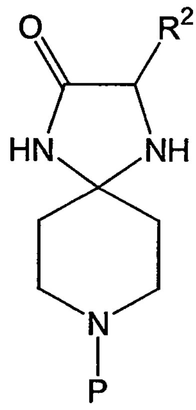

IV

worin P und $R^2$ die vorstehend genannte Bedeutung haben, überführt wird, welche ggf. gereinigt und/oder ggf. isoliert wird, und gegebenenfalls durch Umsetzung mit wenigstens einer Verbindung der allgemeinen Formel $R^1$-$X^1$, worin $R^1$ die Bedeutung gemäß einem oder mehrerer der Ansprüche 1 bis 13 hat und $X^1$ für eine geeignete Austrittsgruppe, vorzugsweise für einen Halogen-Rest, steht, ggf. in Gegenwart wenigstens einer Base, in wenigstens eine Verbindung der allgemeinen Formel V,

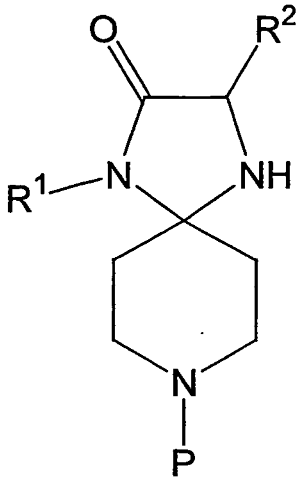

V

worin $R^1$, $R^2$ und P die vorstehend genannte Bedeutung haben, überführt wird, und diese ggf. gereinigt und/oder ggf. isoliert wird, und ggf. durch Abspaltung der Schutzgruppe P in wenigstens eine Verbindung der allgemeinen Formel VI,

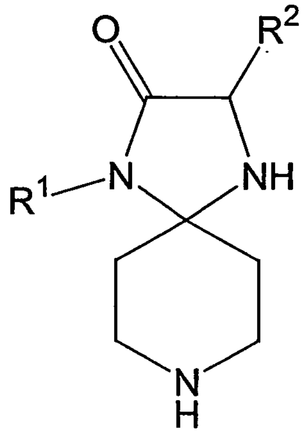

VI

überführt wird, diese ggf. gereinigt und/oder ggf. isoliert wird, und ggf. wenigstens eine Verbindung der allgemeinen Formel IV, V oder VI durch Umsetzung mit einem Carbonsäurederivat der allgemeinen Formel $R^4$-(C=O)-$X^2$, einem Carbonsäureanhydrid der allgemeinen Formel ($R^4$-(C=O))$_2$O oder einer Verbindung der allgemeinen Formel $R^3$-$X^3$, worin der Rest $R^3$ die Bedeutung gemäß einem der Ansprüche 1 bis 13 mit Ausnahme von Wasserstoff und -(C=O)-$R^4$ hat, $R^4$ jeweils die Bedeutung gemäß einem oder mehreren der Ansprüche 1 bis 13 hat und $X^2$ und $X^3$ jeweils für eine geeignete Austrittsgruppe, vorzugsweise für einen Halogen-Rest, stehen, in wenigstens eine Verbindung gemäß einem oder mehreren der Ansprüche 1 bis 13 überführt wird, und diese Verbindung ggf. gereinigt und/oder ggf. isoliert wird,

oder

4-Piperidon der Formel VII, ggf. in Form eines entsprechenden Salzes,

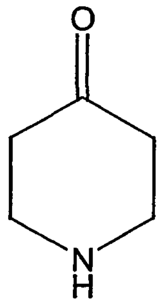

VII

durch Umsetzung mit einem Carbonsäurederivat der allgemeinen Formel $R^4$-(C=O)-$X^2$, einem Carbonsäureanhydrid der allgemeinen Formel ($R^4$-(C=O))$_2$O oder einer Verbindung der allgemeinen Formel $R^3$-$X^3$, worin der Rest $R^3$ die Bedeutung gemäß einem oder mehreren der Ansprüche 1 bis 13 mit Ausnahme von Wasserstoff und -(C=O)-$R^4$ hat, der Rest $R^4$ jeweils die vorstehend genannte Bedeutung hat und $X^2$ und $X^3$ für eine geeignete Austrittsgruppe, vorzugsweise für einen Halogen-Rest, stehen, in eine Verbindung der allgemeinen Formel VIII,

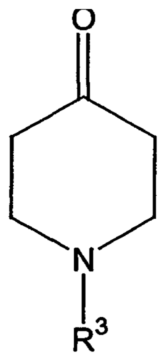

VIII

überführt wird, worin $R^3$ die Bedeutung gemäß einem oder mehreren der Ansprüche 1 bis 13 hat, diese ggf. gereinigt und/oder isoliert wird und durch Umsetzung mit wenigstens einer Verbindung der allgemeinen Formel III

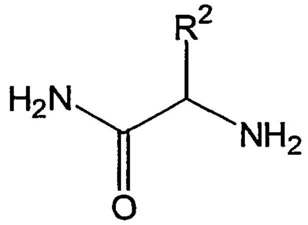

III

worin $R^2$ die vorstehend genannte Bedeutung hat, in wenigstens eine Verbindung der allgemeinen Formel X,

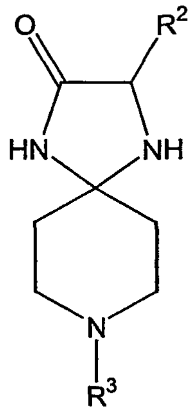

X

überführt wird, worin $R^2$ und $R^3$ jeweils die vorstehend genannte Bedeutung haben, diese ggf. gereinigt und/oder isoliert wird und ggf. durch Umsetzung mit wenigstens einer Verbindung der allgemeinen Formel $R^1$-$X^1$, worin $R^1$ und $X^1$ die vorstehend genannte Bedeutung haben, ggf. in Gegenwart wenigstens einer Base, in wenigstens eine Verbindung gemäß einem oder mehreren der Ansprüche 1 bis 13 überführt und diese ggf. gereinigt und/oder isoliert wird.

**15.** Arzneimittel enthaltend wenigstens eine substituierte 1,4,8-Triazaspiro[4.5]decan-2-on-Verbindung gemäß einem oder mehreren der Ansprüche 1 bis 13 und ggf. einen oder mehrere pharmazeutisch verträgliche Hilfsstoffe.

**16.** Arzneimittel gemäß Anspruch 15 zur Regulation, insbesondere zur Hemmung, der Noradrenalin-Wiederaufnahme (Noradrenalin-Uptake), zur Regulation, insbesondere zur Hemmung, der 5-Hydroxy-Tryptophan-Wiederaufnahme (5-HT-Uptake), zur Opioid-Rezeptor-Regulation, insbesondere zur μ-Opioid-Rezeptor-Regulation und/oder zur Batrachotoxin-(BTX)-Rezeptor-Regulation.

**17.** Arzneimittel gemäß Anspruch 15 oder 16 zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, vorzugsweise Fettsucht.

**18.** Arzneimittel gemäß Anspruch 15 oder 16 zur Prophylaxe und/oder Behandlung von Schmerzen, vorzugsweise zur Behandlung und/oder Prophylaxe von akuten Schmerzen, chronischen Schmerzen, neuropathischen Schmerzen und/oder Clusterkopfschmerzen.

**19.** Arzneimittel gemäß Anspruch 15 oder 16 zur Prophylaxe und/oder Behandlung von Depressionen.

**20.** Arzneimittel gemäß Anspruch 15 oder 16 zur kombinierten Prophylaxe und/oder Behandlung von Depressionen und Schmerzen, vorzugsweise zur kombinierten Behandlung von Depressionen und Schmerzen ausgewählt aus der Gruppe bestehend aus akuten Schmerzen, chronischen Schmerzen, neuropathischen Schmerzen und Clusterkopfschmerzen.

**21.** Arzneimittel gemäß Anspruch 15 oder 16 zur Prophylaxe und/oder Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Ischämien, zur Prophylaxe und/oder Behandlung von Katalepsie, zur Vigilanzsteigerung, zur Libidosteigerung, zur Anxiolyse, zur Prophylaxe und/oder Behandlung von neurodegenerativen Erkrankungen, vorzugsweise einer oder mehrerer neurodegenerativer Erkrankungen ausgewählt aus der Gruppe bestehend aus Morbus Parkinson, Morbus Huntington, Morbus Alzheimer und Multipler Sklerose und/oder zur Lokalanästhesie.

**22.** Verwendung wenigstens einer substituierten 1,4,8-Triazaspiro[4.5]decan-2-on-Verbindung gemäß einem der Ansprüche 1 bis 13 einschließlich der ausgenommenen Verbindungen zur Herstellung eines Arzneimittels zur Regulation der Noradrenalin-Wiederaufnahme (Noradrenalin-Uptake), vorzugsweise zur Hemmung der Noradrenalin-Wiederaufnahme (Noradrenalin-Uptake).

**23.** Verwendung wenigstens einer substituierten 1,4,8-Triazaspiro[4.5]decan-2-on-Verbindung gemäß einem der Ansprüche 1 bis 13 einschließlich der ausgenommenen Verbindungen zur Herstellung eines Arzneimittels zur Regulation der 5-Hydroxy-Tryptophan-Wiederaufnahme (5-HT-Uptake), vorzugsweise zur Hemmung der 5-Hydroxy-Tryptophan-Wiederaufnahme (5-HT-Uptake).

**24.** Verwendung wenigstens einer substituierten 1,4,8-Triazaspiro[4.5]decan-2-on-Verbindung gemäß einem der Ansprüche 1 bis 13 einschließlich der ausgenommenen Verbindungen zur Herstellung eines Arzneimittels zur Opioid-Rezeptor-Regulation, vorzugsweise zur μ-Opioid-Rezeptor-Regulation.

**25.** Verwendung wenigstens einer substituierten 1,4,8-Triazaspiro[4.5]decan-2-on-Verbindung gemäß einem der Ansprüche 1 bis 13 einschließlich der ausgenommenen Verbindungen zur Herstellung eines Arzneimittels zur Batrachotoxin-(BTX)-Rezeptor-Regulation.

**26.** Verwendung wenigstens einer substituierten 1,4,8-Triazaspiro[4.5]decan-2-on-Verbindung der allgemeinen Formel I gemäß einem der Ansprüche 1 bis 13 einschließlich der ausgenommenen Verbindungen zur Herstellung eines

Arzneimittels zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, besonders bevorzugt Fettsucht.

**27.** Verwendung wenigstens einer substituierten 1,4,8-Triazaspiro[4.5]decan-2-on-Verbindung der allgemeinen Formel I gemäß einem der Ansprüche 1 bis 13 einschließlich der gemäß B) ausgenommenen Verbindungen aber mit Ausnahme der Verbindungen gemäß A) zur Herstellung eines Arzneimittels zur Prophylaxe und/oder Behandlung von Schmerzen.

**28.** Verwendung wenigstens einer substituierten 1,4,8-Triazaspiro[4.5]decan-2-on-Verbindung gemäß einem der Ansprüche 1 bis 13 einschließlich der ausgenommenen Verbindungen zur Herstellung eines Arzneimittels zur Prophylaxe und/oder Behandlung von akuten Schmerzen, chronischen Schmerzen, neuropathischen Schmerzen und/oder Clusterkopfschmerzen.

**29.** Verwendung wenigstens einer substituierten 1,4,8-Triazaspiro[4.5]decan-2-on-Verbindung gemäß einem der Ansprüche 1 bis 13 einschließlich der gemäß B) ausgenommenen Verbindungen aber mit Ausnahme der Verbindungen gemäß A) zur Herstellung eines Arzneimittels zur Prophylaxe und/oder Behandlung von Depressionen.

**30.** Verwendung wenigstens einer substituierten 1,4,8-Triazaspiro[4.5]decan-2-on-Verbindung gemäß einem der Ansprüche 1 bis 13 einschließlich der ausgenommenen Verbindungen zur Prophylaxe und/oder Behandlung von Drogen- und/oder Mediamentenmißbrauch, zur Prophylaxe und/oder Behandlung von Drogen- und/oder Medikamentenabhängigkeit, zur Prophylaxe und/oder Behandlung von Multipler Sklerose, zur Prophylaxe und/oder Behandlung von Antriebslosigkeit, zur Prophylaxe und/oder Behandlung von Katalepsie, zur Vigilanzsteigerung, zur Libidosteigerung, zur Anxiolyse, zur Prophylaxe und/oder Behandlung von Ischämien und/oder zur Lokalanästhesie.

**31.** Verwendung wenigstens einer substituierten 1,4,8-Triazaspiro[4.5]decan-2-on-Verbindung gemäß einem der Ansprüche 1 bis 13 einschließlich der gemäß B) ausgenommenen Verbindungen aber mit Ausnahme der Verbindungen gemäß A) zur Herstellung eines Arzneimittels zur Prophylaxe und/oder Behandlung von Akoholmißbrauch, zur Prophylaxe und/oder Behandlung von Akoholabhängigkeit und/oder zur Prophylaxe und/oder Behandlung von Entzündungen.

**32.** Verwendung wenigstens einer substituierten 1,4,8-Triazaspiro[4.5]decan-2-on-Verbindung gemäß einem der Ansprüche 1 bis 13 zur Herstellung eines Arzneimittels zur Prophylaxe und/oder Behandlung von neurodegenerativen Erkrankungen, vorzugsweise einer oder mehrerer Erkrankungen ausgewählt aus der Gruppe bestehend aus Morbus Huntington, Morbus Alzheimer und Morbus Parkinson.

**Claims**

**1.** Substituted 1,4,8-triazaspiro[4.5]decan-2-one compounds of the general formula I,

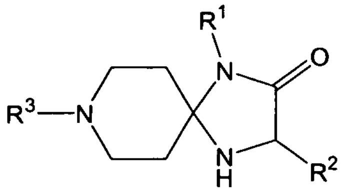

**I**

in which
$R^1$ stands for a hydrogen residue, or for a linear or branched unsubstituted alkyl residue optionally comprising at least one heteroatom as link, or for a linear or branched, unsubstituted or at least monosubstituted alkenyl residue optionally comprising at least one heteroatom as link, or for a linear or branched, unsubstituted or at least monosubstituted alkynyl residue optionally comprising at least one heteroatom as link, or for an unsubstituted or at least monosubstituted aryl residue or heteroaryl residue, which is attached via a linear or branched alkylene group optionally comprising at least one heteroatom as link, or for a -C(=O)-$OR^5$ residue attached via a linear or branched

alkylene group or for an -O-(C=O)-$R^6$ residue attached via a linear or branched alkylene group,

$R^2$ stands for a hydrogen residue, or for a linear or branched, unsubstituted or at least monosubstituted alkyl residue optionally comprising at least one heteroatom as link, or for a linear or branched, unsubstituted or at least monosubstituted alkenyl residue optionally comprising at least one heteroatom as link, or for a linear or branched, unsubstituted or at least monosubstituted alkynyl residue optionally comprising at least one heteroatom as link, or for an unsubstituted or at least monosubstituted aryl residue or heteroaryl residue, which can be attached via a linear or branched alkylene group optionally comprising at least one heteroatom as link,

$R^3$ stands for a hydrogen residue, or for a linear or branched unsubstituted or at least monosubstituted alkyl residue optionally comprising at least one heteroatom as link, or for a linear or branched unsubstituted or at least monosubstituted alkenyl residue optionally comprising at least one heteroatom as link, or for a linear or branched unsubstituted or at least monosubstituted alkynyl residue optionally comprising at least one heteroatom as link, or for an unsubstituted or at least monosubstituted aryl residue or heteroaryl residue, which is attached via a linear or branched unsubstituted or at least monosubstituted alkylene group optionally comprising at least one heteroatom as link and optionally fused with an unsubstituted or at least monosubstituted monocyclic ring system, or for an unsubstituted or at least monosubstituted cycloaliphatic residue optionally comprising at least one heteroatom as ring member and optionally attached via a linear or branched unsubstituted or at least monosubstituted alkylene group optionally comprising at least one heteroatom as link, which can be bridged by a linear or branched unsubstituted or at least monosubstituted alkylene group optionally comprising at least one heteroatom as link and/or fused with an unsubstituted or at least monosubstituted monocyclic ring system, or for a -C(=O)-$R^4$ group,

$R^4$ stands for a linear or branched unsubstituted or at least monosubstituted alkyl residue optionally comprising at least one heteroatom as link, or for a linear or branched unsubstituted or at least monosubstituted alkenyl residue optionally comprising at least one heteroatom as link, or for a linear or branched unsubstituted or at least monosubstituted alkynyl residue optionally comprising at least one heteroatom as link,

or for an unsubstituted or at least monosubstituted aryl residue or heteroaryl residue, which residue can be attached via a linear or branched unsubstituted or at least monosubstituted alkylene group, alkenylene group, or alkynylene group, which groups optionally comprise at least one heteroatom as link, and/or can be fused with an unsubstituted or at least monosubstituted monocyclic ring system,

or for an unsubstituted or at least monosubstituted cycloaliphatic residue optionally comprising at least one heteroatom as ring member and optionally attached via a linear or branched unsubstituted or at least monosubstituted alkylene group optionally comprising at least one heteroatom as link, which cycloaliphatic residue can be at least singly bridged by a linear or branched unsubstituted or at least monosubstituted alkylene group and/or fused with an unsubstituted or at least monosubstituted monocyclic ring system,

or for a -C(=O)-$OR^7$ residue attached via a linear or branched unsubstituted or at least monosubstituted alkylene group, or for an -O-(C=O)-$R^8$ residue attached via a linear or branched unsubstituted or at least monosubstituted alkylene group or for a -(C=O)-$R^9$ residue attached via a linear or branched unsubstituted or at least monosubstituted alkylene group,

$R^5$ and $R^6$ each independently stand for a linear or branched alkyl residue, or for a linear or branched alkenyl residue or for a linear or branched alkynyl residue,

$R^7$ and $R^8$ each independently stand for a hydrogen residue, or for a linear or branched alkyl residue, or for a linear or branched alkenyl residue, or for a linear or branched alkynyl residue, or for an unsubstituted or at least monosubstituted aryl residue or heteroaryl residue,

$R^9$ stands for a linear or branched alkyl residue, or for a linear or branched alkenyl residue or for a linear or branched alkynyl residue,

in each case optionally in the form of one of the pure stereoisomers thereof, particularly enantiomers or diastereoisomers thereof, the racemates thereof or in the form of a mixture of stereoisomers thereof, particularly the enantiomers and/or diastereoisomers, in any desired mixing ratio, or in each case in the form of corresponding salts, or in each case in the form of corresponding solvates.

in which "at least monosubstituted" in connection with an alkyl, alkenyl or alkynyl residue stands for a mono- or polysubstituted alkyl, alkenyl or alkynyl residue, the substituents being in each case mutually independently selected from the group consisting of F, Cl, Br, I, $C_{1-6}$-alkoxy, -$NO_2$, -OH, -SH, -(C=O)-OH, -(C=O)-O-$C_{1-4}$-alkyl, -O-(C=O)-$C_{1-4}$-alkyl, -$CH_2$-O-$CH_2$-phenyl, -CN, -$CF_3$, -$CHF_2$, -$CH_2F$, unsubstituted phenyl and -$NR^aR^b$, in which $R^a$ and $R^b$ may in each case be mutually independently selected from the group consisting of H, methyl, ethyl, n-propyl, isopropyl, n-butyl, sec-butyl, tert.-butyl, -(C=O)-O-$C_{1-4}$-alkyl, and unsubstituted phenyl;

in which "at least monosubstituted" in connection with a cycloaliphatic residue stands for a mono- or polysubstituted cycloaliphatic residue, the substituents being in each case mutually independently selected from the group consisting of F, Cl, Br, $C_{1-6}$-alkoxy, oxo, $C_{1-6}$-alkyl, hydroxy, -CN, -$CF_3$, -$CHF_2$, -$CH_2F$, unsubstituted phenyl, -$NR^aR^b$, in which $R^a$ and $R^b$ may in each case be mutually independently selected from the group consisting of H, methyl, ethyl, n-propyl, isopropyl and unsubstituted phenyl, thioxo (=S), I, -$SF_5$, -$NO_2$, -O-$CF_3$, -S-$CF_3$, -SH, -S-$C_{1-5}$-alkyl, -C(=O)-H,

-C(=O)-$C_{1-5}$-alkyl, -C(=O)-OH, -C(=O)-O-$C_{1-5}$-alkyl, -($CH_2$)-C(=O)-OH, -$CH_2$-C(=O)-O-$C_{1-5}$-alkyl, -($CH_2$)-benzo[b]furanyl, -O-phenyl, - O-benzyl, phenyl, benzyl, naphthyl and -($CH_2$)-naphthyl, wherein the cyclic moiety of the residues -0-phenyl, -O-benzyl, phenyl, -($CH_2$)-benzo[b]furanyl, benzyl, naphthyl and -($CH_2$)-naphthyl can in each case be substituted with 1, 2, 3, 4 or 5 substituents mutually independently selected from the group consisting of F, Cl, Br, -OH, -$CF_3$, -$SF_5$, -CN, -$NO_2$, -O-$C_{1-5}$-alkyl, -$C_{1-5}$-alkyl, -O-$CF_3$, -S-$CF_3$, phenyl and -O-benzyl;
in which "at least monosubstituted" in connection with an aryl or heteroaryl residue stands for a mono- or polysubstituted aryl or heteroaryl residue, the substituents preferably being in each case mutually independently selected from the group consisting of F, Cl, Br, I, -CN, -$NO_2$, $C_{1-5}$-alkyl, OH, SH, $C_{1-5}$-alkoxy, $C_{1-5}$-perfluoroalkoxy, -C(=O)-$C_{1-5}$-alkyl, -C(=O)-O-$C_{1-5}$-alkyl, -($CH_2$)$_{1-3}$-O-(C=O)-phenyl, -S-$C_{1-5}$-alkyl, -S(=O)$_2$-$C_{1-6}$-alkyl, -S(=O)$_2$-$NH_2$, -NH,-(C=O)-$CH_3$, -S-$CHF_2$, -S-$CH_2$F, -C(=O)-$C_{1-5}$-perfluoroalkyl, -$CF_3$, -$CHF_2$, -$CH_2$F, phenyl, phenoxy and -$NR^aR^b$, in which $R^a$ and $R^b$ may be in each case mutually independently selected from the group consisting of H, methyl, ethyl, n-propyl, isopropyl and unsubstituted phenyl, -$SF_5$, -O-$C_{2-5}$-Alkenyl, -O-$CF_3$, -O-$CHF_2$, -O-$CH_2$F, -C(=O)-OH, -O-C(=O)-$C_{1-5}$-alkyl, -O-C(=O)-phenyl, -($CH_2$)-O-C(=O)-$C_{1-5}$-alkyl, -NH-$C_{1-5}$ alkyl, -N($C_{1-5}$-alkyl)$_2$, -NH-C(=O)-O-$C_{1-5}$-alkyl, -C(=O)-H, -C(=O)-$C_{1-5}$-alkyl, -C(=O)-$NH_2$, -C(=O)-NH-$C_{1-5}$-Alkyl, C(=O)-N-($C_{1-5}$-alkyl)$_2$, -S(=O)$_2$-NH-$C_{1-5}$ alkyl, -S(=O)$_2$-NH-phenyl, -($CH_2$)-benzo[b]furanyl, -O-phenyl, -S-phenyl, -S-benzyl, -O-benzyl, phenyl and benzyl, wherein the cyclic moiety of the residues -S(=O)$_2$-NH-phenyl, -O-phenyl, -S-phenyl, -S-benzyl, -O-benzyl, phenyl, -($CH_2$)-benzo[b]furanyl and benzyl can be substituted with 1, 2, 3, 4 or 5 substituents mutually independently selected from the group consisting of F, Cl, Br, -OH, - $CF_3$, -$SF_5$, -CN, -$NO_2$, -O-$C_{1-5}$-alkyl, -$C_{1-5}$-alkyl, -O-$CF_3$, -S-$CF_3$, phenyl and -O-benzyl and the phenyl and phenoxy substituents can themselves be mono- or polysubstituted, identically or differently, with a substituent selected from the group consisting of F, Cl, Br, -S-$CHF_2$, -$CF_3$, -$CHF_2$, -$CH_2$F, -CN, -$NO_2$, $C_{1-5}$-alkyl and $C_{1-5}$-alkoxy;
in which "at least monosubstituted" in connection with a monocyclic ring system stands for a mono- or polysubstituted monocyclic ring system, the substituents preferably being in each case mutually independently selected from the group consisting of F, Cl, Br, I, -CN, -$NO_2$, oxo, $C_{1-5}$-alkyl, $C_{1-5}$-alkoxy, $C_{1-5}$-perfluoroalkoxy, -C(=O)-$C_{1-5}$-alkyl, -C(=O)-O-$C_{1-5}$-alkyl, -S(=O)$_2$-$C_{1-6}$-alkyl -C(=O)- $C_{1-5}$-perfluoroalkyl, -$CF_3$, $CHF_2$, $CH_2$F, and -$NR^aR^b$, in which $R^a$ and $R^b$ may be in each case mutually independently selected from the group consisting of H, methyl, ethyl, n-propyl, isopropyl and unsubstituted phenyl, thioxo (=S), -$SF_5$, -OH, -O-$C_{2-5}$-alkenyl, -$NH_2$, -$NO_2$, -O-$CF_3$, -O-$CHF_2$, -O-$CH_2$F, -S-$CF_3$, -S-$CHF_2$, -S-$CH_2$F, -SH, -S-$C_{1-5}$-alkyl, -C(-O)-OH, -O-C(=O)-$C_{1-5}$-alkyl, -O-C(=O)-phenyl,-($CH_2$)-O-C(=O)-$C_{1-5}$-alkyl, -($CH_2$)-O-C(=O)-phenyl, -NH-$C_{1-5}$ alkyl, -N($C_{1-5}$-alkyl)$_2$, -NH-C(=O)-O-$C_{1-5}$-alkyl, -NH-C(=O)-$C_{1-5}$-alkyl, -C(=O)-H, -C(=O)-$NH_2$, -C(=O)-NH-$C_{1-5}$-alkyl, C(=O)-N-($C_{1-5}$-alkyl)$_2$, - S(=O)$_2$-$NH_2$, -S(=O)$_2$-NH-$C_{1-5}$-alkyl, -S(=O)$_2$-NH-phenyl, -($CH_2$)-benzo[b]furanyl, -O-phenyl, -S-phenyl, -S-benzyl, -O-benzyl, phenyl and benzyl, wherein the cyclic moiety of the residues -O-C(=O)-phenyl, - ($CH_2$)-O-C(=O)-phenyl, -S(=O)$_2$-NH-phenyl, -O-phenyl, -S-phenyl, -S-benzyl, -O-benzyl, phenyl, - (CH2)-benzo[b]furanyl and benzyl can in each case be substituted with 1, 2, 3, 4 or 5 substituents mutually independently selected from the group consisting of F, Cl, Br, -OH, -$CF_3$, -$SF_5$, -CN, -$NO_2$, - O-$C_{1-5}$-alkyl, -$C_{1-5}$-alkyl, -O-$CF_3$, -S-$CF_3$, phenyl and -O-benzyl;
in which "at least monosubstituted" in connection with a linear or branched alkylene, alkenylene or alkynylene group stands for a mono- or polysubstituted linear or branched, alkylene, alkenylene or alkynylene group, the substituents preferably being in each case mutually independently selected from the group consisting of F, Cl, Br, $C_{1-6}$-alkoxy, hydroxy, CN, $CF_3$, $CHF_2$, $CH_2$F, unsubstituted phenyl and -$N^aR^b$, in which $R^a$ and $R^b$ may be in each case mutually independently selected from the group consisting of H, $C_{1-3}$-alkyl and unsubstituted phenyl;
excluding those compounds of the general formula I in which

A)
$R^1$ stands for a hydrogen residue, a $C_{1-6}$-alkyl residue, a $C_{1-6}$-alkoxy residue, a $C_{2-6}$-alkenyl residue, a $C_{2-6}$-alkynyl residue or for a $C_{1-6}$-alkyl residue that is monosubstituted with a phenyl residue,
$R^2$ stands for a hydrogen residue, a $C_{1-6}$-alkyl residue, a $C_{1-6}$-alkoxy residue, a $C_{2-6}$-hydroxyalkyl residue, a $C_{2-6}$-alkenyl residue, a $C_{2-6}$-alkynyl residue, a phenyl residue, a $C_{1-6}$-alkyl residue monosubstituted, disubstituted, or trisubstituted with a phenyl residue, an -O-$C_{1-6}$-alkanoyl residue, an -O-$C_{1-6}$-alkyl residue, an -O-$C_{2-6}$-hydroxyalkyl residue, a -O-$C_{2-6}$-alkenyl residue, an -O-$C_{2-6}$-alkynyl residue or for an -O-$C_{1-6}$-alkyl residue that is monosubstituted, disubstituted, or trisubstituted with a phenyl residue,
$R^3$ stands for a hydrogen residue, a $C_{1-6}$-alkyl residue, a $C_{2-6}$-alkenyl residue, a $C_{2-6}$-alkynyl residue, a $C_{3-7}$-cycloalkyl residue, a $C_{1-6}$-alkyl residue that is substituted with 1 to 6 halogen atoms, a hydroxy-$C_{1-6}$-alkyl residue, a $C_{1-6}$-alkoxy residue, a $C_{1-6}$-alkylthio residue, a $C_{1-6}$-alkoxy-$C_{1-6}$-alkyl residue, a carboxy-$C_{1-6}$-alkyl residue, a ($C_{1-6}$-alkoxy)carbonyl-$C_{1-6}$-alkyl residue, an amino-$C_{1-6}$-alkyl residue, a mono-($C_{1-6}$-alkyl)- amino-$C_{1-6}$-alkyl residue, a di($C_{1-6}$-alkyl)-amino-$C_{1-6}$-alkyl residue, a 2-oxo-pyrrolidin-1-ylmethyl residue, an aryl residue, a diarylmethylol residue, a $C_{1-6}$-alkyl residue that is substituted with one or two aryl residues, a $C_{1-6}$-alkanoyl residue, or an arylcarbonyl residue, aryl in each case standing for an unsubstituted phenyl residue or for a phenyl residue which is substituted with 1 to 3 substituents selected from the group consisting of halogen, $C_{1-6}$-alkyl, $C_{1-6}$-alkoxy,

and $CF_3$,
and the physiologically compatible salts thereof, enantiomers thereof and racemates thereof,
and
B)
the residues $R^1$, $R^2$, and $R^3$ in each case stand for a hydrogen residue or a hydrocarbon residue and in each case the salts thereof.

**2.** Compounds according to claim 1, **characterized in that** $R^1$ stands for a hydrogen residue, or for a linear or branched unsubstituted, $C_{1-10}$-alkyl residue optionally comprising at least one heteroatom as link, or for a linear or branched unsubstituted or at least monosubstituted $C_{2-10}$-alkenyl residue optionally comprising at least one heteroatom as link, or for a linear or branched unsubstituted or at least monosubstituted $C_{2-10}$-alkynyl residue optionally comprising at least one heteroatom as link, or for an unsubstituted or at least monosubstituted five-membered to fourteen-membered aryl residue or heteroaryl residue, which is attached via a linear or branched $C_{1-5}$-alkylene group optionally comprising at least one heteroatom as link, or for a -C(=O)-$OR^5$ residue attached via a linear or branched $C_{1-5}$ alkylene group, or for a -O-C(=O)-$R^6$ residue attached via a linear or branched $C_{1-5}$ alkylene group,
preferably for a hydrogen residue, or for a linear or branched, unsubstituted $C_{1-5}$-alkyl residue, or for a linear or branched, unsubstituted $C_{2-5}$-alkenyl residue, or for a linear or branched, unsubstituted $C_{2-5}$-alkynyl residue, or for an unsubstituted or at least monosubstituted phenyl or naphthyl residue attached via a linear or branched $C_{1-5}$-alkylene group, optionally comprising one or more oxygen atoms as links, or for a -C(=O)-$OR^5$ residue attached via a linear or branched $C_{1-4}$-alkylene group or for a -O-C(=O)-$R^6$ residue attached via a linear or branched $C_{1-4}$-alkylene group,
particularly preferably for a hydrogen residue, or for a linear or branched unsubstituted $C_{1-4}$-alkyl residue, or for a linear or branched unsubstituted $C_{2-5}$-alkenyl residue, or for a linear or branched unsubstituted $C_{2-3}$-alkynyl residue, or for a phenyl or naphthyl residue, which is attached via a -($CH_2$) bridge, -$(CH_2)_2$ bridge, -$(CH_2)_3$ bridge or -$(CH_2)_2$-O-bridge and is optionally monosubstituted or polysubstituted, identically or differently, with a substituent selected from the group consisting of F, Cl, Br, -CN, -$NO_2$, $C_{1-5}$-alkyl, $C_{1-5}$-alkoxy, -C(=O)-$C_{1-5}$-alkyl, -C(=O)-O-$C_{1-5}$-alkyl, -S(=O)$_2$C-$_{1-6}$-alkyl, - C(=O)-$C_{1-5}$ perfluoroalkyl, -$CF_3$, $CHF_2$, and $CH_2F$, or for a -C(=O)-$OR^5$ residue attached via a -($CH_2$) group or for a -O-C(=O)-$R^6$ residue attached via a -($CH_2$) group, -$(CH_2)_2$ group, -$(CH_2)_3$ group or - $(CH_2)_4$ group.

**3.** Compounds according to claim 1 or claim 2, **characterized in that** $R^2$ stands for a hydrogen residue, or for a linear or branched unsubstituted or at least monosubstituted $C_{1-10}$-alkyl residue optionally comprising at least one heteroatom as link, or for a linear or branched unsubstituted or at least monosubstituted $C_{2-10}$-alkenyl residue optionally comprising at least one heteroatom as link, or for a linear or branched unsubstituted or at least monosubstituted $C_{2-10}$-alkynyl residue optionally comprising at least one heteroatom as link, or for an unsubstituted or at least monosubstituted, five- to fourteen-membered aryl residue or heteroaryl residue, which can be attached via a linear or branched $C_{1-5}$-alkylene group optionally comprising at least one heteroatom as link,
preferably for a hydrogen residue, or for a linear or branched, unsubstituted $C_{1-5}$ alkyl residue, optionally comprising one or more oxygen atoms and/or one or more sulfur atoms as link(s) or for a phenyl or naphthyl residue which is monosubstituted or polysubstituted, identically or differently, with a substituent selected from the group consisting of F, Cl, Br, -CN, -$NO_2$, $C_{1-5}$-alkyl, $C_{1-5}$-alkoxy, -C(=O)-$C_{1-5}$-alkyl, -C(=O)-O-$C_{1-5}$-alkyl, -S(=O)$_2$-$C_{1-6}$-alkyl, -C(=O)-$C_{1-5}$-perfluoroalkyl, -$CF_3$, -$CHF_2$, and -$CH_2F$ and/or can be attached via a -(-$CH_2$)- bridge, a -$(CH_2)_2$- bridge, a -$(CH_2)_3$- bridge, or a -$(CH_2)_2$-O-bridge,
particularly preferably for a hydrogen residue, or for a linear or branched unsubstituted $C_{1-5}$ alkyl residue optionally comprising one or more sulfur atoms as links or for a phenyl residue, wherein the phenyl residue can be monosubstituted or polysubstituted, identically or differently, with a substituent selected from the group consisting of F, Cl, Br, -CN, -$NO_2$, $C_{1-5}$-alkyl, $C_{1-5}$-alkoxy, -C(=O)-$C_{1-5}$-alkyl, - C(=O)-O-$C_{1-5}$-alkyl, -S(=O)$_2$-$C_{1-6}$-alkyl, -C(=O)- $C_{1-5}$-perfluoroalkyl, -$CF_3$, -$CHF_2$, and -$CH_2F$ and/or can be attached via a -($CH_2$)- bridge.

**4.** Compounds according to one or more of claims 1-3, **characterized in that** $R^3$ stands for a hydrogen residue, or for a linear or branched unsubstituted or at least monosubstituted $C_{1-10}$-alkyl residue optionally comprising at least one heteroatom as link, or for a linear or branched unsubstituted or at least monosubstituted $C_{2-10}$-alkenyl residue optionally comprising at least one heteroatom as link, or for a linear or branched unsubstituted or at least monosubstituted $C_{2-10}$alkynyl residue optionally comprising at least one heteroatom as link, or for an unsubstituted or at least monosubstituted five-membered to fourteen-membered aryl residue or heteroaryl residue, which is attached via a linear or branched unsubstituted or at least monosubstituted $C_{1-5}$-alkylene group optionally comprising at least one heteroatom as link and is optionally fused with an unsubstituted or at least monosubstituted monocyclic ring system, or for an unsubstituted or at least monosubstituted $C_{3-8}$-cycloaliphatic residue, optionally comprising at least one heteroatom as ring member and which can be attached via a linear or branched unsubstituted or at least monosub-

stituted $C_{1-5}$ alkylene group optionally comprising at least one heteroatom as link and/or can be fused with an unsubstituted or at least monosubstituted monocyclic ring system, or for a -C(=O)-$R^4$ group,
preferably for a hydrogen residue, or for a biphenyl residue, which is attached via a -($CH_2$)- bridge, a - ($CH_2$)$_2$- bridge or a -($CH_2$)$_3$- bridge, or for a C(=O)-$R^4$ group,
particularly preferably for a -C(=O)-$R^4$ group,

**5.** Compounds according to one or more of claims 1 to 4, **characterized in that** $R^4$
stands for a linear or branched unsubstituted or at least monosubstituted $C_{1-10}$-alkyl residue optionally comprising at least one heteroatom as link, or for a linear or branched unsubstituted or at least monosubstituted $C_{2-10}$-alkenyl residue optionally comprising at least one heteroatom as link, or for a linear or branched unsubstituted or at least monosubstituted $C_{2-10}$-alkynyl residue optionally comprising at least one heteroatom as link,
or for an unsubstituted or at least monosubstituted five-membered to fourteen-membered aryl residue or heteroaryl residue, which can be attached via a linear or branched unsubstituted or at least monosubstituted $C_{1-5}$-alkylene group, $C_{2-5}$-alkenylene group, or $C_{2-5}$-alkynylene group optionally comprising at least one heteroatom as link and/or can be fused with an unsubstituted or at least monosubstituted monocyclic ring system,
or for an unsubstituted or at least monosubstituted $C_{3-8}$ cycloaliphatic residue optionally comprising at least one heteroatom as ring member and optionally attached via a linear or branched unsubstituted or at least monosubstituted $C_{1-6}$ alkylene group optionally comprising at least one heteroatom as link, which cycloaliphatic residue can be at least singly bridged by a linear or branched unsubstituted or at least monosubstituted $C_{1-6}$-alkylene group and/or fused with an unsubstituted or at least monosubstituted monocyclic ring system,
or for a -C(=O)-$OR^7$ residue attached via a linear or branched unsubstituted or at least monosubstituted $C_{1-5}$-alkylene group, or for an -O-(C=O)-$R^8$ residue attached via a linear or branched unsubstituted or at least monosubstituted $C_{1-5}$-alkylene group or for a -(C=O)-$R^9$ residue attached via a linear or branched unsubstituted or at least monosubstituted $C_{1-5}$ alkylene group,
preferably for a linear or branched $C_{1-10}$-alkyl residue optionally comprising one or more oxygen atoms and/or one or more sulfur atoms as link(s), which residue can be unsubstituted or at least monosubstituted, identically or differently, with a substituent selected from the group consisting of phenyl, di-($C_{1-5}$ alkylamino), carboxy, and -NH-(C=O)-O-$C_{1-5}$-alkyl, or for a linear or branched $C_{2-5}$-alkenyl residue, or for a phenyl residue, naphthyl residue, furyl (furanyl) residue, thienyl (thiophenyl) residue or [1,2,3]-triazolyl residue, benzo[1.3]dioxolyl residue, benzo [1,2,5]oxadiazolyl residue, chromanyl residue, pyrimidinyl residue, pyrazolyl residue, pyridinyl residue, isoxazolyl residue, or [1,2,3]-thiadiazolyl residue, wherein the cyclic residue may in each case be attached via over a-($CH_2$)-, -($CH_2$)-O- bridge, a -CH=CH- bridge, a -($CH_2$)$_2$- bridge, a -($CH_2$)$_2$-O- bridge or a -($CH_2$)$_3$-bridge and/or can be mono- or polysubstituted, identically or differently, with a substituent selected from the group consisting of F, Cl, Br, I, -CN, -$NO_2$, $C_{1-5}$-alkyl, $C_{1-5}$-alkoxy, $C_{1-5}$-perfluoroalkoxy, - C(=O)-$C_{1-5}$-alkyl, -C(=O)-O-$C_{1-5}$-alkyl, -($CH_2$)$_{1-3}$-O-C(=O)-phenyl, -N($CH_3$)$_2$, -S(=O)$_2$-$C_{1-6}$-alkyl, -S(=O)$_2$-$NH_2$, -C(=O)-$C_{1-5}$-perfluoroalkyl, -S-$C_{1-5}$-alkyl, -S-$CH_2F$, -S-$CHF_2$, -$SCF_3$, -$CF_3$, -NH-(C=O)-$CH_3$, -$CHF_2$, -$CH_2F$, phenyl, and phenoxy, wherein the latter phenyl and phenoxy substituents can themselves be mono- or polysubstituted, identically or differently, with a substituent selected from the group consisting of F, Cl, Br, -S-$CHF_2$, -$CF_3$, -$CHF_2$, -$CH_2F$, CN, $NO_2$, $C_{1-5}$-alkyl, and $C_{1-5}$-alkoxy,
or for a three-membered, four-membered, five-membered, six-membered, seven-membered, or eight-membered cycloaliphatic residue optionally comprising at least one heteroatom as ring member and optionally attached via a -($CH_2$)- bridge, a -($CH_2$)$_2$- bridge, or a -($CH_2$)$_3$- bridge, which cycloaliphatic residue is at least singly bridged at least by one -(C($CH_3$)$_2$)- or -($CH_2$) group and/or unsubstituted or monosubstituted or polysubstituted, identically or differently, with a substituent selected from the group consisting of $C_{1-5}$-alkyl, $C_{1-5}$-alkoxy, -$CF_3$, -$CHF_2$, -$CH_2F$, and oxo, or for a -C(=O)-$OR^7$ residue attached via a linear or branched unsubstituted or at least monosubstituted $C_{1-5}$ alkylene group, or for a -O-(C=O)-$R^8$ residue attached via a linear or branched unsubstituted or at least monosubstituted $C_{1-5}$ alkylene group, or for a -(C=O)-$R^9$ residue attached via a linear or branched unsubstituted or at least monosubstituted $C_{1-5}$ alkylene group,
particularly preferably for a linear or branched $C_{1-8}$-alkyl residue optionally comprising one or more oxygen atoms and/or one or more sulfur atoms as links, which residue can be unsubstituted or at least monosubstituted, identically or differently, with a substituent selected from the group consisting of unsubstituted phenyl, dimethylamino, carboxy, and -NH-(C=O)-O-C($CH_3$)$_3$, or for a linear or branched unsubstituted $C_{2-4}$-alkenyl residue, or for a phenyl residue, naphthyl residue, furyl-(furanyl) residue, thienyl-(thiophenyl) residue, [1,2,3]-triazolyl residue, chromanyl residue, benzo[1.3]dioxolyl residue, benzo[1,2,5]oxadiazoleyl residue, pyrimidinyl residue, pyrazolyl residue, pyridinyl residue, isoxazolyl residue, or [1,2,3]-thiadiazolyl residue, wherein the cyclic residue can in each case be attached via a - ($CH_2$)- bridge, a -($CH_2$)-O- bridge, a -CH=CH- bridge, a -($CH_2$)$_2$- bridge, a -($CH_2$)$_2$-O- bridge, or a-($CH_2$)$_3$-bridge and/or can be mono- or polysubstituted, identically or differently, with a substituent selected from the group consisting of F, Cl, Br, I, methyl, ethyl, isopropyl, n-propyl, sec-butyl, tert.-butyl, n-butyl, methoxy, ethoxy, -$CF_3$, -$OCF_3$, -$CH_2$-O-C(=O)-phenyl, -$NO_2$, -S-$CF_3$, -S-$CHF_2$, -N($CH_3$)$_2$, -NH-CO-$CH_3$, -CN, -$SO_2$-$NH_2$, phenyl, and phenoxy, wherein the

latter phenyl and phenoxy substituents can themselves be mono- or polysubstituted, identically or differently, with a substituent selected from the group consisting of F, Cl, Br, $-CH_3$, and $-OCH_3$,
or for a cyclopropyl, cyclobutyl, cyclopentyl, cyclohexyl, imidazolidine, 4,7,7-trimethyl-3-oxo-2-oxa-bicyclo[2.2.1] heptyl residue or adamantyl residue optionally attached via a $-(CH_2)-$ bridge, a $-(CH_2)_2$-bridge, or a $-(CH_2)_3-$ bridge, and which is unsubstituted or monosubstituted or polysubstituted, identically or differently, with a substituent selected from the group consisting of $C_{1-5}$-alkyl, $C_{1-5}$-alkoxy, $-CF_3$, $-CHF_2$, $-CH_2F$, and oxo,
or for a $-C(=O)-OR^7$ residue attached via a $-(CH_2)-$ group, a $-(CH_2)_2$ group, a -C(H)-phenyl group, or a $-C(CH_3)_2$ group, or for a $-O-(C=O)-R^8$ residue attached via a $-(CH_2)-$ group, a $-(CH_2)_2$ group, a -C(H)-phenyl group, or a -C $(CH_3)_2$ group, or for a $-(C=O)-R^9$ residue attached via a $-(CH_2)-$ group or a - $(CH_2)_2$ group,
very particularly preferably for a linear or branched $C_{6-8}$-alkyl residue optionally comprising one or more oxygen atoms and/or one or more sulfur atoms as links, which can be unsubstituted or at least monosubstituted, identically or differently, with a substituent selected from the group consisting of unsubstituted phenyl, dimethylamino, carboxy, and $-NH-(C=O)-O-C(CH_3)_3$, or for a linear or branched unsubstituted $C_{2-4}$ alkenyl residue, or for a naphthyl residue, furyl-(furanyl) residue, thienyl-(thiophenyl) residue, [1,2,3]-triazolyl residue, chromanyl residue, benzo[1.3]dioxolyl residue, benzo[1,2,5]oxadiazolyl residue, pyrimidinyl residue, pyrazolyl residue, pyridinyl residue, isoxazolyl residue, or [1,2,3]-thiadiazolyl residue, wherein the cyclic residue can in each case be attached via a - $(CH_2)-$ bridge, a $-(CH_2)-O-$ bridge, a -CH=CH- bridge, a $-(CH_2)_2-$ bridge, a $-(CH_2)_2-O-$ bridge, or a - $(CH_2)_3$ bridge and/or can be mono- or polysubstituted, identically or differently, with a substituent selected from the group consisting of F, Cl, Br, I, methyl, ethyl, isopropyl, n-propyl, sec-butyl, tert.-butyl, n-butyl, methoxy, ethoxy, $-CF_3$, $-OCF_3$, $-CH_2-O-C(=O)$-phenyl, $-NO_2$, $-S-CF_3$, $-S-CHF_2$, $-N(CH_3)_2$, $-NH-CO-CH_3$, -CN, $-SO_2-NH_2$, phenyl, and phenoxy, wherein the latter phenyl and phenoxy substituents can themselves be mono- or polysubstituted, identically or differently, with a substituent selected from the group consisting of F, Cl, Br, $CH_3$, and $OCH_3$, or for 2-trifluoromethoxyphenyl, 3-trifluoromethoxyphenyl, 4-trifluoromethoxyphenyl, $2-CF_3$-S-phenyl, $3-CF_3$-S-phenyl, $4-CF_3$-S-phenyl, 2-cyanophenyl, 3-cyanophenyl, 4-cyanophenyl, 2-nitrophenyl, 3-nitrophenyl, 4-nitrophenyl, $2-CHF_2$-S-phenyl, $3-CHF_2$-S-phenyl, $4-CHF_2$-S-phenyl, 2-dimethylaminophenyl, 3-dimethylaminophenyl, 4-dimethylaminophenyl, $2$-phenyl-$(C=O)-O-CH_2$-phenyl, 3-phenyl-$(C=O)-O-CH_2$-phenyl, 4-phenyl-$(C=O)-O-CH_2$-phenyl, $2-CH_3-(C=O)$-NH-phenyl, $3-CH_3-(C=O)$-NH-phenyl, $4-CH_3-(C=O)$-NH-phenyl, 2-phenylphenyl, 3-phenylphenyl, 4-phenylphenyl, $2-NH_2-SO_2$-phenyl, $3-NH_2-SO_2$-phenyl, $4-NH_2-SO_2$-phenyl, pentafluorophenyl, 4-methyl-3-nitrophenyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 2-methylbenzyl, 3-methylbenzyl, 4-methylbenzyl, 2-methoxybenzyl, 3-methoxybenzyl, 4-methoxybenzyl, 3,4-dimethoxybenzyl, 2,5-dimethoxybenzyl, -CH=CH-phenyl, wherein the phenyl residue can be mono- or polysubstituted in the 2, 3 or 4 position, identically or differently, with a residue selected from the group consisting of F, Cl, Br, and $CF_3$, $-CH_2-O$-phenyl, wherein the phenyl residue can be mono- or polysubstituted in 2, 3 or 4 position, identically or differently, with a residue selected from the group consisting of F, Cl, Br, and $CF_3$, for a cyclopropyl, cyclobutyl, cyclopentyl, cyclohexyl, imidazolidine, 4,7,7-trimethyl-3-oxo-2-oxa-bicyclo[2.2.1]heptyl residue or adamantyl residue, which residues are optionally attached via a $-(CH_2)-$ bridge, a $-(CHF_2)_2-$ bridge, or a $-(CH_2)_3-$ bridge, wherein the cyclic residues can in each case be unsubstituted or monosubstituted or polysubstituted, identically or differently, with a substituent selected from the group consisting of $C_{1-5}$ alkyl, $C_{1-5}$ alkoxy, $-CF_3$, $CHF_2$, $CH_2F$, and oxo, for a $-C(=O)-OR^7$ residue attached via a $-(CH_2)$ group, $-(CH_2)_2$ group, -C(H)(phenyl) group, or $-C(CH_3)_2$ group, for a $-O-(C=O)-R^8$ residue attached via a $-(CH_2)$ group, $-(CH_2)_2$ group, - C(H)(phenyl) group, or $-C(CH_3)_2$ group, or for a $-(C=O)-R^9$ residue attached via a $-(CH_2)$ group or a - $(CH_2)_2$ group.

**6.** Compounds according to one or more of claims 1 to 5, **characterized in that** the residues $R^5$ and $R^6$ in each case mutually independently stand for a linear or branched $C_{1-5}$-alkyl residue, a linear or branched $C_{2-5}$-alkenyl residue or a linear or branched $C_{2-5}$-alkynyl residue and preferably for a methyl residue, ethyl residue, n-propyl residue, or isopropyl residue.

**7.** Compounds according to one or more of claims 1 to 6, **characterized in that** the residues $R^7$ and $R^8$ in each case mutually independently stand for a hydrogen residue, or for a linear or branched $C_{1-5}$-alkyl residue, a linear or branched $C_{2-5}$-alkenyl residue, a linear or branched $C_{2-5}$-alkynyl residue, or for an optionally at least monosubstituted phenyl or naphthyl residue, preferably for a methyl residue, ethyl residue, n-propyl residue, or isopropyl residue, or for an unsubstituted phenyl residue.

**8.** Compounds according to one or more of claims 1 to 7, **characterized in that** the residue $R^9$ stands for a linear or branched $C_{1-5}$-alkyl residue, a linear or branched $C_{2-5}$-alkenyl residue, a linear or branched $C_{2-5}$-alkynyl residue, preferably for a methyl residue, ethyl residue, n-propyl residue or isopropyl residue.

**9.** Compounds according to one or more of claims 1 to 8, **characterized in that**

$R^1$ stands for a hydrogen residue, or for a linear or branched unsubstituted $C_{1-4}$-alkyl residue, or for a linear or branched unsubstituted $C_{2-5}$-alkenyl residue, or for a linear or branched unsubstituted $C_{2-3}$-alkynyl residue, or for a phenyl or naphthyl residue, which is unsubstituted or monosubstituted or polysubstituted, identically or differently, with a substituent selected from the group consisting of F, Cl, Br, -CN, $-NO_2$, $C_{1-5}$-alkyl, $C_{1-5}$-alkoxy, -C(=O)-$C_{1-5}$-alkyl, -C(=O)-O-$C_{1-5}$-alkyl, $-S(=O)_2-C_{12}$-alkyl,-C(=O)-$C_{1-5}$-perfluoroalkyl, $-CF_3$, $-CHF_2$, and $-CH_2F$ and/or is attached via a $-(CH_2)-$ bridge, a $-(CH_2)_2$-bridge, a $-(CH_2)_3-$ bridge or a $-(CHF_2)_2$-O- bridge, or for a -C(=O)-$OR^5$ residue attached via a $-(CH_2)$ group or for a -O-C(=O)-$R^6$ residue attached via a $-(CH_2)$ group, a $-(CH_2)_2$ group, a $-(CH_2)_3$ group, or $-(CH_2)_4$ group,

$R^2$ stands for a hydrogen residue, or for a linear or branched unsubstituted $C_{1-5}$-alkyl residue optionally comprising one or more sulfur atoms as link(s) or for a phenyl residue, wherein the phenyl residue can be monosubstituted or polysubstituted, identically or differently, with a substituent selected from the group consisting of F, Cl, Br, -CN, $-NO_2$, $C_{1-5}$-alkyl, $C_{1-5}$-alkoxy, -C(=O)-$C_{1-5}$-alkyl, -C(=O)-O-$C_{1-5}$-alkyl, - $S(=O)_2-C_{1-6}$-alkyl, -C(=O)- $C_{1-5}$-perfluoroalkyl, $-CF_3$, $-CHF_2$, and $-CH_2F$, and/or can be attached via a - $(CH_2)$- bridge,

$R^3$ stands for a hydrogen residue, or for a biphenyl residue, which is attached via a $-(CH_2)-$ bridge, a - $(CH_2)_2-$ bridge, or a $-(CH_2)_3-$ bridge, or for a C(=O)-$R^4$ group,

$R^4$ stands for a residue, which is selected from the group consisting of methyl, ethyl, isopropyl, n-propyl, sec-butyl, isobutyl, tert.-butyl, n-butyl, n-pentyl, 1-methylbutyl, 2-dimethylpropyl, 1-ethylpropyl, 1-propylbutyl, 1-ethylpentyl, dimethylaminomethyl, $-CH_2-CH_2-CH=CH_2$, $-CH_2-O-CH_3$, $-CH_2-O-CH_2$-CH2-O-$CH_3$, $-CH_2-CH_2$-phenyl, $-CH_2$-O-phenyl, $-CH_2-O-CH_2$-phenyl, $-CH_2-CH_2-CH_2$-O-phenyl, - C(H)(phenyl)-$(C_2H_5)$, -C(H)$(CH_3)$-O-phenyl, $-CH_2-CH_2-(C=O)-CH_3$, $-CH_2-O-(C=O)-CH_3$, $-CH_2-CH_2-(C=O)-O-C_2H_5$, $-CH_2$-O-(C=O)-phenyl, $-CH_2-(C=O)-O-CH_2-CH_3$, -C(H)(phenyl)-(C=O)-$CH_3$, - C(H)(phenyl)-O-(C=O)-$CH_3$, $-C(CH_3)_2-O-(C=O)-CH_3$, -C(H)(NH-(C=O)-O-$(CH_3)_3$)-$(CH_2-O-CH_2$-phenyl), $-C(CH_3)_2-CH_2$-COOH, unsubstituted phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 2-iodophenyl, 3-iodophenyl, 4-iodophenyl, 2-methylphenyl, 3-methylphenyl, 4-methylphenyl, 2-ethylphenyl, 3-ethylphenyl, 4-ethylphenyl, 2-n-propylphenyl, 3-n-propylphenyl, 4-n-propylphenyl, 2-tert.-butylphenyl, 3-tert.-butylphenyl, 4-tert.-butylphenyl, 2-methoxyphenyl, 3-methoxyphenyl, 4-methoxyphenyl, 2-ethoxyphenyl, 3-ethoxyphenyl, 4-ethoxyphenyl, 2-trifluoromethylphenyl, 3 trifluoromethylphenyl, 4-trifluoromethylphenyl, 2-trifluoromethoxyphenyl, 3-trifluoromethoxyphenyl, 4-trifluoromethoxyphenyl, 2-$CF_3$-S-phenyl, 3-$CF_3$-S-phenyl, 4-$CF_3$-S-phenyl, 2-cyanophenyl, 3-cyanophenyl, 4-cyanophenyl, 2-nitrophenyl, 3-nitrophenyl, 4-nitrophenyl, 2-$CHF_2$-S-phenyl, 3-$CHF_2$-S-phenyl, 4-$CHF_2$-S-phenyl, 2-dimethylaminophenyl, 3-dimethylaminophenyl, 4-dimethylaminophenyl, 2-phenyl-(C=O)-O-$CH_2$-phenyl, 3-phenyl-(C=O)-O-$CH_2$-phenyl, 4-phenyl-(C=O)-O-$CH_2$-phenyl, 2-$CH_3$-(C=O)-NH-phenyl, 3-$CH_3$-(C=O)-NH-phenyl, 4-$CH_3$-(C=O)-NH-phenyl, 2-phenylphenyl, 3-phenylphenyl, 4-phenylphenyl, 2-$NH_2-SO_2$-phenyl, 3-$NH_2-SO_2$-phenyl, 4-$NH_2-SO_2$-phenyl, 2,4-dimethoxyphenyl, 2,5-dimethoxyphenyl, 3,5-dimethoxyphenyl, 3,4-dimethoxyphenyl, 2,3-dimethylphenyl, 2,3-dichlorophenyl, 3,4-dichlorophenyl, 2,6-dichlorophenyl, 2,3-difluorophenyl, 3,4-difluorophenyl, 3,5-difluorophenyl, pentafluorophenyl, 2-chloro-6-fluorophenyl, 4-bromo-3-methylphenyl, 3-fluoro-4-methylphenyl, 3-chloro-2-fluorophenyl, 2-chloro-4-nitrophenyl, 5-fluoro-2-trifluoromethylphenyl, 3-fluoro-4-trifluoromethyl, 4-methyl-3-nitrophenyl, 2-chloro-5-trifluoromethyl, 2,5-bis-trifluoromethylphenyl, 3,5-bis-trifluoromethylphenyl, 2-chloro-6-fluoro-3-methylphenyl, 6-chloro-2-fluoro-3-methylphenyl, 2,6-difluoro-4-methylphenyl, 2,6-difluoro-3-methylphenyl, 3,4,5-trimethoxyphenyl, 2,3-difluoro-4-methylphenyl, 2-fluorobenzyl, 3-fluorobenzyl, 4-fluorobenzyl, 2-chlorobenzyl, 3-chlorobenzyl, 4-chlorobenzyl, 2-bromobenzyl, 3-bromobenzyl, 4-bromobenzyl, 2-methylbenzyl, 3-methylbenzyl, 4-methylbenzyl, 2-methoxybenzyl, 3-methoxybenzyl, 4-methoxybenzyl, 3,4-dimethoxybenzyl, 2,5-dimethoxybenzyl, -CH=CH-phenyl, wherein the phenyl residue can be monosubstituted or polysubstituted in the 2, 3, or 4 position, identically or differently, with a residue selected from the group consisting of F, Cl, Br, and $CF_3$, $-CH_2$-O-phenyl, wherein the phenyl residue can be monosubstituted or polysubstituted in the 2, 3 or 4 position, identically or differently with a residue selected from the group consisting of F, Cl, Br, and $CF_3$, 1-naphthyl, 2-naphthyl, 2-thienyl, 3-thienyl, 3-chlorothien-2-yl, 2-furanyl, 3-furanyl, 2,5-dimethylfuran-3-yl, 5-tert.-butyl-2-methylfuran-3-yl, pyridin-2-yl, pyridin-3-yl, pyridin-4-yl, 2-chloropyridin-4-yl, 6-chloropyridin-3-yl, 2-chloropyridin-3-yl, 2-ethylsulfanylpyridin-3-yl, 2-phenoxypyridin-3-yl, 2-methylsulfanylpyridin-3-yl, 2-methyl-6-trifluoromethylpyridin-3-yl, isoxazol-5-yl, 5-methylisoxazol-3-yl, 3-(2-chloro-6-fluorophenyl)-5-methylisoxazol-4-yl, 3-(2-chlorophenyl)-5-methylisoxazol-4-yl, 3-(2,6-dichlorophenyl)-5-methylisoxazol-4-yl, 5-tert.-butyl-2-methyl-2H-pyrazol-3-yl, 1-phenyl-5-n-propyl-1H-pyrazol-4-yl, 1-(4-chlorophenyl)-5-trifluoromethyl-1H-pyrazol-4-yl, 2-tert.-butyl-5-methyl-2H-pyrazol-3-yl, 5-methyl-2-phenyl-2H-[1,2,3]triazol-4-yl, 4-methyl[1,2,3]thiadiazol-5-yl, 2-chloro-4-trifluoromethylpyrimidin-5-yl, 2-chloro-4-trifluoromethylpyrimidin-5-yl, benzo[1,2,5]oxadiazol-5-yl, benzo[1.3]dioxol-5-yl, 6-chloro-2H-chroman-3-yl, imidazolidin-2,4-dion-5-ylmethyl, cyclopropyl, cyclobutyl, cyclopentyl optionally attached via a - $(CH_2)$- bridge or a $-(CH_2)_2$ bridge, cyclohexyl, 4,7,7-trimethyl-3-oxo-2-oxa-bicyclo[2.2.1]heptyl and adamantyl.

**10.** Compounds according to one or more of claims 1 to 9, **characterized in that**

I.)

$R^1$ stands for a substituted six-membered or ten-membered aryl residue

or for an optionally substituted five-membered to fourteen-membered heteroaryl residue;
or for $-(CH_2)_m-O-C(=O)-R^5$, where m is 1, 2, 3, 4, or 5;
or for $-(CH_2)_n-C(=O)-O-R^6$, where n is 1, 2, 3, 4, or 5;
or for $-(CH_2)-R^{22}$;
or for $-(CH_2)-(CH_2)_c-U-(CH_2)_d-R^{23}$, where c is 0 or 1 and d is 0 or 1 and in which U stands for O, S, NH, $N(CH_3)$, or $N(C_2H_5)$;

$R^2$ stands for a hydrogen residue;

or for $-(CH_2)-(CH_2)_e-V_f-(CH_2)_g-R^{24}$, where e is 0 or 1, f is 0 or 1, and g is 0 or 1 and in which V stands for O, S, NH, $N(CH_3)$, or $N(C_2H_5)$;
or for an in each case linear or branched, in each case optionally substituted $C_{1-10}$-alkyl residue, $C_{2-10}$-alkenyl residue or $C_{2-10}$-alkynyl residue;
or for an optionally substituted five-membered to fourteen-membered aryl residue or heteroaryl residue;

$R^3$ stands for a $-C(=O)-R^4$ group;

and

$R^4$ stands for $-(CHR^{10})-P_s-(CH_2)_t-(CH_2)_u-Q_v-R^{11}$, where s is 0 or 1, t is 0 or 1, u is 0 or 1, and v is 0 or 1 and in which P and Q each independently stand for O, S, NH, $N(CH_3)$, or $N(C_2H_5)$ and the sum of s and v is equal to 1;

or for $-(CR^{12}R^{13})-(CH_2)_w-(CH_2)_x-C(=O)-O-R^7$, where w is 0 or 1 and x is 0 or 1;
or for $-(CR^{14}R^{15})-(CH_2)_y-O-C(=O)-R^8$, where y is 0 or 1;
or for $-(CHR^{16})-(CH_2)_2-C(=O)-R^9$, where z is 0 or 1;
or for $-CH[(CH_2)-T_a-(CH_2)_b-R^{17}][NH-C(=O)-O-R^{18}]$, where a is 0 or 1 and b is 0 or 1 and in which T stands for O, S, NH, $N(CH_3)$, or $N(C_2H_5)$;
or for $-CHR^{19}R^{20}$ or $-(CH_2)-CHR^{19}R^{20}$;
or for $-(CH=CH)-R^{21}$;
or for an in each case linear or branched, in each case optionally substituted $C_{1-10}$-alkyl residue, $C_{2-10}$-alkenyl residue or $C_{2-10}$-alkynyl residue;
or for an unsaturated or saturated, optionally substituted three-membered, four-membered, five-membered, six-membered, seven-membered, eight-membered or nine-membered cycloaliphatic residue, which can be fused with a saturated or unsaturated, optionally aromatic, optionally substituted monocyclic ring system and/or bridged by one or two linear or branched, optionally substituted $C_{1-5}$-alkylene groups,
or for an optionally substituted five-membered to fourteen-membered aryl residue or heteroaryl residue, which can be fused with a saturated or unsaturated, optionally substituted monocyclic ring system;

or II.)

$R^1$ stands for a hydrogen residue

or for an in each case linear or branched, in each case unsubstituted $C_{1-10}$-alkyl residue, $C_{2-10}$-alkenyl residue or $C_{2-10}$-alkynyl residue;
or for an unsubstituted phenyl residue or an unsubstituted benzyl residue;

$R^2$ stands for a hydrogen residue;

or for $-(CH_2)-(CH_2)_e-V_f-(CH_2)_g-R^{24}$, where e is 0 or 1, f is 0 or 1, and g is 0 or 1 and in which V stands for O, S, NH, $N(CH_3)$, or $N(C_2H_5)$;
or for an in each case linear or branched, in each case optionally substituted $C_{1-10}$-alkyl residue, $C_{2-10}$-alkenyl residue or $C_{2-10}$-alkynyl residue;

or for an optionally substituted five-membered to fourteen-membered aryl or heteroaryl residue;

$R^3$ stands for a -C(=O)-$R^4$ group;

and

$R^4$ stands for $-(CHR^{10})-P_s-(CH_2)_t-(CH_2)_u-Q_v-R^{11}$, where s is 0 or 1, t is 0 or 1, u is 0 or 1 and v is 0 or 1 and in which P and Q each stand independently for O, S, NH, $N(CH_3)$, or $N(C_2H_5)$, and the sum of s and v is equal to 1;

or for $-(CR^{12}R^{13})-(CH_2)_w-(CH_2)_x-C(=O)-O-R^7$, where w is 0 or 1 and x is 0 or 1;
or for $-(CR^{14}R^{15})-(CH_2)_y-O-C(=O)-R^8$, where y is 0 or 1;
or for $-(CHR^{16})-(CH_2)_z-C(=O)-R^9$, where z is 0 or 1;
or for $-CH[(CH_2)-T_a-(CH_2)_b-R^{17}][NH-C(=O)-O-R^{18}]$, where a is 0 or 1 and b is 0 or 1 and in which T stands for O, S, NH, $N(CH_3)$, or $N(C_2H_5)$;
or for $-CHR^{19}R^{20}$ or $-(CH_2)-CHR^{19}R^{20}$;
or for $-(CH=CH)-R^{21}$;
or for a linear or branched, substituted $C_{1-10}$-alkyl residue;
or for a linear or branched, optionally substituted $C_{2-10}$-alkenyl residue;
or for a linear or branched, optionally substituted $C_{2-10}$-alkynyl residue;
or for an unsaturated or saturated, optionally substituted three-membered, four-membered, five-membered, six-membered, seven-membered, eight-membered or nine-membered cycloaliphatic residue, which can be fused with a saturated or unsaturated, optionally aromatic, optionally substituted monocyclic ring system and/or bridged by one or two linear or branched, optionally substituted $C_{1-5}$ alkylene groups;
or for a phenyl residue, which is substituted with 1, 2, 3, 4, or 5 substituents independently selected from the group consisting of -CN, $-SF_5$, $-NH_2$, $-NO_2$, $-O-CF_3$, $-O-CHF_2$, $-O-CH_2F$, $-S-CF_3$, $-S-CHF_2$, $-S-CH_2F$, -SH, -S-$C_{1-5}$-alkyl, -C(=O)-OH, -C(=O)-O-$C_{1-5}$-alkyl,-O-C(=O)-$C_{1-5}$-alkyl, -O-C(=O)-phenyl, $-(CH_2)$-O-C(=O)-$C_{1-5}$-alkyl, $-(CH_2)$-O-C(=O)-phenyl, -NH-$C_{1-5}$-alkyl, -N($C_{1-5}$-alkyl$)_2$, -NH-C(=O)-O-$C_{1-5}$-alkyl, -NH-C(=O)-$C_{1-5}$-alkyl,-C(=O)-H, -C(=O)-$C_{1-5}$-alkyl, $-C(=O)-NH_2$, -C(=O)-NH-$C_{1-5}$-alkyl, C (=O)-N($C_{1-5}$-alkyl$)_2$,$-S(=O)_2-NH_2$, $-S(=O)_2$-NH-$C_{1-5}$-alkyl, $-S(=O)_2$-NH-phenyl, $-(CH_2)$benzo[b]furanyl, -O-phenyl, -S-phenyl, -S-benzyl, -O-benzyl, phenyl, and benzyl, wherein in each case the cyclic moiety of the residues -O-C(=O)-phenyl, $-(CH_2)$-O-C(=O)-phenyl, $-S(=O)_2$-NH-phenyl, -O-phenyl, -S-phenyl, -S-benzyl, -O-benzyl, phenyl, $-(CH_2)$benzo[b]furanyl, and benzyl can be substituted with 1, 2, 3, 4, or 5 substituents independently selected from the group consisting of F, Cl, Br, -OH, $-CF_3$, $-SF_5$, -CN, $-NO_2$, -O-$C_{1-5}$-alkyl, -$C_{1-5}$-alkyl, $O-CF_3$, $-S-CF_3$, phenyl and O-benzyl,
or for a residue selected from the group consisting of 1,2,3,4,5-pentafluorophenyl, 1,2,3,4-tetrafluorophenyl, 4-methyl-3-nitrophenyl, 5-methyl-3-nitrophenyl, 6-methyl-3-nitrophenyl, and 2-methyl-3-nitrophenyl;
or for an unsubstituted naphthyl residue;
or for an optionally substituted six-membered or ten-membered aryl residue, which is fused with a saturated or unsaturated, optionally substituted monocyclic ring system;
or for an optionally substituted five-membered to fourteen-membered heteroaryl residue, which can be fused with a saturated or unsaturated, optionally substituted monocyclic ring system;

or III.)

$R^1$ stands for a hydrogen residue;

or for an in each case linear or branched, in each case unsubstituted $C_{1-10}$-alkyl residue, $C_{2-10}$-alkenyl residue or $C_{2-10}$-alkynyl residue;
or for an optionally substituted five-membered to fourteen-membered aryl or heteroaryl residue;
or for $-(CH_2)_m$-O- C (=O)-$R^5$ where m is 1, 2, 3, 4, or 5;
or for $-(CH_2)_n$-C (=O)-O- $R^6$, where n is 1, 2, 3, 4, or 5;
or for an unsubstituted benzyl residue;
or for $-(CH_2)-R^{22}$;
or for $-(CH_2)-(CH_2)_c-U-(CH_2)_d-R^{23}$, where c is 0 or 1 and d is 0 or 1 and in which U stands for O, S, NH, $N(CH_3)$, or $N(C_2H_5)$;

$R^2$ stands for $-(CH_2)-(CH_2)_e-V-(CH_2)_g-R^{24}$ where e is 0 or 1 and g is 0 or 1 and in which V stands for S, NH, $N(CH_3)$, or $N(C_2H_5)$;
$R^3$ stands for a $-C(=O)-R^4$ group;

and

$R^4$ stands for $-(CHR^{10})-P_s-(CH_2)_t-(CH_2)_uQ_v-R^{11}$ where s is 0 or 1, t is 0 or 1, u is 0 or 1 and v is 0 or 1 and in which P and Q each stand independently for O, S, NH, $N(CH_3)$, or $N(C_2H_5)$ and the sum of s and v is equal to 1;

or for $-(CR^{12}R^{13})-(CH_2)_w-(CH_2)_x-C\ (=O)-O-\ R^7$, where w is 0 or 1 and x is 0 or 1;
or for $-(CR^{14}R^{15})-(CH_2)_y-O-\ C\ (=O)-R^8$, where y is 0 or 1;
or for $-(CHR^{16})-(CH_2)_z-C\ (=O)-R^9$, where z is 0 or 1;
or for $CH[(CH_2)-T_a-(CH_2)_b-R^{17}][NH-C(=O)-O-R^{18}]$, where a is 0 or 1 and b is 0 or 1 and in which T stands for O, S, NH, $N(CH_3)$, or $N(C_2H_5)$;
or for $CHR^{19}R^{20}$ or $-(CH_2)-CHR^{19}R^{20}$;
or for $-(CH=CH)-R^{21}$;
or for an in each case linear or branched, in each case optionally substituted $C_{1-10}$-alkyl residue, $C_{2-10}$-alkenyl residue or $C_{2-10}$-alkynyl residue;
or for an unsaturated or saturated, optionally substituted three-membered, four-membered, five-membered, six-membered, seven-membered, eight-membered or nine-membered cycloaliphatic residue, which can be fused with a saturated or unsaturated, optionally aromatic, optionally substituted monocyclic ring system and/or bridged by one or two linear or branched, optionally substituted $C_{1-5}$-alkylene groups,
or for an optionally substituted five-membered to fourteen-membered aryl residue or heteroaryl residue, which can be fused with a saturated or unsaturated, optionally substituted monocyclic ring system;

and in each case, if present, in the aforementioned groups I.), II.) and III.) the residues

$R^5$, $R^6$, and $R^9$, and $R^{18}$ each independently

stand for an in each case linear or branched, in each case optionally substituted $C_{1-10}$-alkyl residue, $C_{2-10}$-alkenyl residue or $C_{2-10}$-alkynyl residue;

$R^7$ stands for a hydrogen residue

or for an in each case linear or branched, in each case optionally substituted $C_{1-10}$-alkyl residue, $C_{2-10}$-alkenyl residue or $C_{2-10}$-alkynyl residue;
or for an optionally substituted five-membered to fourteen-membered aryl residue or an optionally substituted five-membered to fourteen-membered heteroaryl residue;

$R^8$, $R^{16}$, and $R^{24}$ each independently

stand for an in each case linear or branched, in each case optionally substituted $C_{1-10}$-alkyl residue, $C_{2-10}$-alkenyl residue or $C_{2-10}$-alkynyl residue;
or for an optionally substituted five to fourteen-membered aryl residue or an optionally substituted five to fourteen-membered heteroaryl residue;

$R^{10}$, $R^{12}$, and $R^{13}$ each independently

stand for a hydrogen residue;
or for an in each case linear or branched, in each case optionally substituted $C_{1-10}$-alkyl residue, $C_{2-10}$-alkenyl residue or $C_{2-10}$-alkynyl residue;

$R^{11}$ stands for an in each case linear or branched, in each case optionally substituted $C_{1-10}$-alkyl residue, $C_{2-10}$-alkenyl residue or $C_{2-10}$-alkynyl residue;

or for an unsaturated or saturated, optionally substituted three-membered, four-membered, five-membered, six-membered, seven-membered, eight-membered, or nine-membered cycloaliphatic residue; or for an optionally substituted six-membered or ten-membered aryl residue;

$R^{14}$, $R^{15}$, and $R^{19}$ each independently

stand for a hydrogen residue;
or for an in each case linear or branched, in each case optionally substituted $C_{1-10}$-alkyl residue, $C_{2-10}$-alkenyl residue or $C_{2-10}$-alkynyl residue;
or for an optionally substituted six-membered or ten-membered aryl residue;

$R^{17}$ and $R^{21}$ each independently

stand for an optionally substituted six-membered or ten-membered aryl residue;

$R^{20}$ stands for an unsaturated or saturated, optionally substituted three-membered, four-membered, five-membered, six-membered, seven-membered, eight-membered or nine-membered cycloaliphatic residue

or for an optionally substituted six-membered or ten-membered aryl residue;

$R^{22}$ stands for a substituted six-membered aryl residue

or for an optionally substituted ten-membered aryl residue
or for an optionally substituted heteroaryl residue;

and

$R^{23}$ stands for an optionally substituted five to fourteen-membered aryl residue or an optionally substituted five to fourteen-membered heteroaryl residue;

wherein
the aforementioned $C_{1-10}$-alkyl residues can be unsubstituted or can in each case be substituted with 1, 2, 3, 4 or 5 substituents independently selected from the group consisting of F, Cl, Br, I, -CN, $-NO_2$, -OH, -SH, $-NH_2$, and -N($C_{1-5}$-alkyl)-($C_{1-5}$-alkyl);
the aforementioned $C_{2-10}$-alkenyl residues can be unsubstituted or can in each case be substituted with 1, 2, 3, 4 or 5 substituents independently selected from the group consisting of F, Cl, Br, I, -CN, - $NO_2$, -OH, -SH, $-NH_2$, and -N($C_1$-$_5$-alkyl)-($C_1$-$_5$-alkyl);
the aforementioned $C_{2-10}$-alkynyl residues can be unsubstituted or can in each case be substituted with 1, 2, 3, 4 or 5 substituents independently selected from the group consisting of F, Cl, Br, I, -CN, - $NO_2$, -OH, -SH, $-NH_2$, and -N($C_{1-5}$-alkyl)-($C_{1-5}$-alkyl);
the aforementioned $C_{1-5}$-alkylene groups can be unsubstituted or can in each case be substituted with 1, 2, 3, 4 or 5 substituents independently selected from the group consisting of F, Cl, Br, -OH, -SH, - $NH_2$, -CN, $NO_2$, and phenyl,
the aforementioned cycloaliphatic residues can be unsubstituted or in each case substituted with 1, 2, 3, 4, or 5 substituents independently selected from the group consisting of oxo (=O), thioxo (=S), F, Cl, Br, I, -CN, $-CF_3$, $-SF_5$, -OH, -O-$C_{1-5}$-alkyl, $-NH_2$, $-NO_2$, $-O-CF_3$, $-S-CF_3$, -SH, -S-$C_{1-5}$ alkyl, -$C_{1-5}$-alkyl,-C(=O)-H, -C(=O)-$C_{1-5}$-alkyl, -C (=O)-OH, -C(=O)-O-$C_{1-5}$-alkyl, -($CH_2$)-C(=O)-OH, -($CH_2$)-C(=O)-O-$C_{1-5}$-alkyl, -NH-$C_{1-5}$-alkyl, -N($C_{1-5}$-alkyl)$_2$, -($CH_2$) benzo[b]furanyl, -O-phenyl, -O-benzyl, phenyl, benzyl, naphthyl, and -($CH_2$)-naphthyl, wherein in each case the cyclic moiety of the residues -O-phenyl, -O-benzyl, phenyl, -($CH_2$)-benzo[b]furanyl, benzyl, naphthyl, and -($CH_2$)-naphthyl can be substituted with 1, 2, 3, 4, or 5 substituents independently selected from the group consisting of F, Cl, Br, -OH, $-CF_3$, - $SF_5$, -CN, $-NO_2$, -O-$C_{1-5}$-alkyl, -$C_{1-5}$-alkyl, $-O-CF_3$, $-S-CF_3$, phenyl, and -O-benzyl,
and the aforementioned cycloaliphatic residues can in each case comprise 1, 2, 3, 4, or 5 heteroatom(s) independently selected from the group consisting of oxygen, nitrogen, and sulfur;
the ring of the aforementioned monocyclic ring systems can be optionally unsubstituted or substituted with 1, 2, 3, 4, or 5 substituents independently selected from the group consisting of oxo (=O), thioxo (=S), F, Cl, Br, I, -CN, $-CF_3$, $-SF_5$, -OH, -O-$C_{1-5}$-alkyl, -O-$C_{2-5}$-alkenyl, $-NH_2$, $-NO_2$, $-O-CF_3$, $-O-CHF_2$, - O-$CH_2$F, $-S-CF_3$, $-S-CHF_2$, -S-$CH_2$F, -SH, -S-$C_{1-5}$-alkyl, -$C_{1-5}$-alkyl, -C(=O)-OH, -C(=O)-O-$C_{1-5}$-alkyl, -0-C(=O)-$C_{1-5}$-alkyl, -O-C(=O)-phenyl, -($CH_2$)-O-C(=O)-$C_{1-5}$-alkyl, -($CH_2$)-O-C(=O)-phenyl, -NH-$C_{1-5}$-alkyl, -N($C_{1-5}$-alkyl)$_2$, -NH-C(=O)-O-$C_{1-5}$-alkyl, -NH-C(=O)-$C_{1-5}$-alkyl, -C(=O)-H, -C(=O)-$C_{1-5}$-alkyl, -C(=O)-$NH_2$, -C(=O)-NH-$C_{1-5}$-alkyl, C(=O)-N-($C_{1-5}$-alkyl)$_2$, -S (=O)$_2$-$NH_2$, -S(=O)$_2$-NH-$C_{1-5}$-alkyl, -S(=O)$_2$-NH-phenyl,- ($CH_2$)benzo[b]furanyl, -O-phenyl, -S-phenyl, -S-benzyl,

-O-benzyl, phenyl, and benzyl, wherein in each case the cyclic moiety of the residues -O-C(=O)-phenyl, -($CH_2$)-O-C(=O)-phenyl, - S(=O)$_2$-NH-phenyl, -O-phenyl, -S-phenyl, -S-benzyl, -O-benzyl, phenyl, -($CH_2$)benzo[b]furanyl, and benzyl can be substituted with 1, 2, 3, 4 or 5 substituents independently selected from the group consisting of F, Cl, Br, -OH, -$CF_3$, -$SF_5$, -CN, -$NO_2$, -O-$C_{1-5}$-alkyl, -$C_{1-5}$-alkyl, -O-$CF_3$, -S-$CF_3$, phenyl und -O-benzyl,
and the ring of the aforementioned monocyclic ring systems is in each case five-membered, six-membered, or seven-membered and can comprise 1, 2, 3, 4, or 5 heteroatom(s) as ring member(s), which are independently selected from the group consisting of oxygen, nitrogen, and sulfur;
and, unless otherwise stated, the aforementioned aryl residues or heteroaryl residues can be unsubstituted or in each case substituted with 1, 2, 3, 4, or 5 substituents independently selected from the group consisting of F, Cl, Br, I, -CN, -$CF_3$, -$SF_5$, -OH, -O-$C_{1-5}$-alkyl, -O-$C_{2-5}$-alkenyl, -$NH_2$, -$NO_2$, -O-$CF_3$, -O-$CHF_2$, -O-$CH_2$F, -S-$CF_3$, -S-$CHF_2$, -S-$CH_2$F, -SH, -S-$C_{1-5}$-alkyl, -$C_{1-5}$-alkyl, -C(=O)-OH, -C(=O)-O-$C_{1-5}$-alkyl, -O-C(=O)-$C_{1-5}$-alkyl, -O-C(=O)-phenyl, -($CH_2$)-O-C(=O)-$C_{1-5}$-alkyl, -($CH_2$)-O-C(=O)-phenyl, -NH-$C_{1-5}$-alkyl, -N($C_{1-5}$-alkyl)$_2$, -NH-C(=O)-O-$C_{1-5}$-alkyl, -NH-C(=O)-$C_{1-5}$-alkyl, -C(=O)-H, -C(=O)-$C_{1-5}$-alkyl, -C(=O)-$NH_2$, -C(=O)-NH-$C_{1-5}$-alkyl, C(=O)-N-($C_{1-5}$-alkyl)$_2$, -S(=O)$_2$-$NH_2$, -S(=O)$_2$-NH-$C_{1-5}$-alkyl, - S(=O)$_2$-NH-phenyl, -($CH_2$)benzo[b]furanyl, -O-phenyl, -S-phenyl, -S-benzyl, -O-benzyl, phenyl, and benzyl, wherein in each case the cyclic moiety of the residues -O-C(=O)-phenyl, -($CH_2$)-O-C(=O)-phenyl, -S(=O)$_2$-NH-phenyl, -O-phenyl, -S-phenyl, -S-benzyl, -O-benzyl, phenyl, -($CH_2$)benzo[b]furanyl und benzyl can be substituted with 1, 2, 3, 4 or 5 substituents independently selected from the group consisting of F, Cl, Br, -OH, -$CF_3$, -$SF_5$, -CN, -$NO_2$, -O-$C_{1-5}$-alkyl, -$C_{1-5}$-alkyl, -O-$CF_3$, -S-$CF_3$, phenyl und -O-benzyl,
and
the aforementioned heteroaryl residues can in each case comprise 1, 2, 3, 4, or 5 heteroatom(s) independently selected from the group consisting of oxygen, nitrogen, and sulfur, as ring member(s);
in each case optionally in the form of one of the pure stereoisomers thereof, particularly enantiomers or diastereoisomers thereof, the racemates thereof or in the form of a mixture of stereoisomers, particularly the enantiomers and/or diastereoisomers, in any desired mixing ratio, or in each case in the form of corresponding salts, or in each case in the form of corresponding solvates.

**11.** Compounds according to claim 10, **characterized in that**

I.)

$R^1$ stands for a phenyl or naphthyl residue, which is in each case substituted with 1, 2, 3, 4, or 5 substituents independently selected from the group consisting of F, Cl, Br, I, -CN,-$CF_3$,- $SF_5$, -OH, -O-$CH_3$, -O-$C_2H_5$, -O-CH($CH_3$)$_2$, -O-C($CH_3$)$_3$, -O-$CH_2$-$CH_2$-$CH_2$-$CH_3$,-O-$CH_2$-CH=$CH_2$, -$NH_2$, -$NO_2$, -O-$CF_3$, -O-$CHF_2$, -O-$CH_2$F, -S-$CF_3$, -S-$CHF_2$, -S-$CH_2$F,-SH, -S-$CH_3$, -S-$C_2H_5$, -S-CH($CH_3$)$_2$, -S-C($CH_3$)$_3$, -S-$CH_2$-$CH_2$-$CH_2$-$CH_3$, methyl, ethyl, n-propyl, isopropyl, n-butyl, sec-butyl, isobutyl, tert.-butyl, -C(=O)-OH, -C(=O)-O-$CH_3$,-C(=O)-O-$C_2H_5$, -C(=O)-O-C($CH_3$)$_3$, -O-C(=O)-$CH_3$, -O-C(=O)-$C_2H_5$, -O-C(=O)-phenyl,-($CH_2$)-O-C(=O)-$CH_3$, -($CH_2$)-O-C(=O)-$C_2H_5$, -($CH_2$)-O-C(=O)-phenyl, -N($CH_3$)$_2$, N($C_2H_5$)$_2$, -N(H)($CH_3$), -N(H)$C_2H_5$), -NH-C(=O)-)-$CH_3$, NH-C(=O)-O-$C_2H_5$, NH-C(=O)-$CH_3$, -NH-C(=O)-$C_2H_5$, -C(=O)-$CH_3$, -C(=O)-$C_2H_5$, -C(=O)-$NH_2$, -C(=O)-NH-$CH_3$,-C(=O)-NH-$C_2H_5$, S(=O)$_2$-$NH_2$, S(=O)$_2$-NH-$CH_3$, -S(=O)$_2$NH-$C_2H_5$, S(=O)$_2$-NH=phenyl,-($CH_2$)-benzo[b]furanyl, -O-phenyl, -S-phenyl, -S-benzyl, -O-benzyl, phenyl and benzyl, wherein in each case the cyclic moiety of the residues O-C(=O)-phenyl, -($CH_2$)-O-C(=O)-phenyl, -S(=O)$_2$-NH-phenyl, -O-phenyl, -S-phenyl, -S-benzyl, -O-benzyl, phenyl, -($CH_2$)-benzo[b]furanyl, and benzyl can be substituted with 1, 2, 3, 4, or 5 substituents independently selected from the group consisting of F, Cl, Br, -OH, -$CF_3$, -$SF_5$, -CN,-$NO_2$, -O-$CH_3$, -O-$C_2H_5$, -O-CH($CH_3$)$_2$, -O-C($CH_3$)$_3$, methyl, ethyl, n-propyl, isopropyl, n-butyl, sec-butyl, isobutyl, tert.-butyl, -O-$CF_3$, -S-$CF_3$, phenyl, and -O-benzyl,

or for a heteroaryl residue selected from the group consisting of thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl, and isoquinolinyl, wherein the heteroaryl residue can in each case be substituted with 1, 2, 3, 4, or 5 substituents independently selected from the group consisting of F, Cl, Br, I, -CN, -$CF_3$, -$SF_5$, -OH, -O-$CH_3$, -O-$C_2H_5$, -O-CH($CH_3$)$_2$, -O-C($CH_3$)$_3$, -O-$CH_2$-$CH_2$-$CH_2$-$CH_3$, -O-$CH_2$-CH=$CH_2$, -$NH_2$, -$NO_2$, -O-$CF_3$, -O-$CHF_2$, -O-$CH_2$F, -S-$CF_3$, -S-$CHF_2$, -S-$CH_2$F, -SH, -S-$CH_3$, -S-$C_2H_5$, -S-CH($CH_3$)$_2$, -S-C($CH_3$)$_3$, -S-$CH_2$-$CH_2$-$CH_2$-$CH_3$, methyl, ethyl, n-propyl, isopropyl, n-butyl, sec-butyl, isobutyl, tert.-butyl, -C(=O)-OH, -C(=O)-O-$CH_3$, -C(=O)-O-$C_2H_5$,-C(=O)-O-C($CH_3$)$_3$, -O-C(=O)-$CH_3$, -O-C(=O)-$C_2H_5$, -O-C(=O)-phenyl, -($CH_2$)-O-C(=O)-$CH_3$, -($CH_2$)-O-C(=O)-$C_2H_5$, -($CH_2$)-O-C(=O)-phenyl, -N($CH_3$)$_2$, -N($C_2H_5$)$_2$, -N(H)($CH_3$), -N(H)($C_2H_5$), -NH-C(=O)-O-$CH_3$, -NH-C(=O)-O-$C_2H_5$, -NH-C(=O)-$CH_3$, -NH-C

(=O)-$C_2H_5$, -C(=O)-H, -C(=O)-$CH_3$, -C(=O)-$C_2H_5$, -C(=O)-$NH_2$, -C(=O)-NH-$CH_3$, -C(=O)-NH-$C_2H_5$, -S(=O)$_2$-$NH_2$, -S(=O)$_2$-NH-$CH_3$, -S(=O)$_2$-NH-$C_2H_5$, -S(=O)$_2$-NH-phenyl, -($CH_2$)-benzo[b]furanyl, -O-phenyl, -S-phenyl, -S-benzyl, -O-benzyl, phenyl and benzyl, and in each case the cyclic moiety of the residues -O-C(=O)-phenyl, -($CH_2$)-O-C(=O)-phenyl, -S(=O)$_2$-NH-phenyl, -O-phenyl, -S-phenyl, -S-benzyl, -O-benzyl, phenyl, -($CH_2$)-benzo[b]furanyl, and benzyl can be substituted with 1, 2, 3, 4, or 5 substituents independently selected from the group consisting of F, Cl, Br, -OH, -$CF_3$, -$SF_5$, -CN,-$NO_2$, -O-$CH_3$, -O-$C_2H_5$, -O-CH($CH_3$)$_2$, -O-C($CH_3$)$_3$, methyl, ethyl, n-propyl, isopropyl, n-butyl, sec-butyl, isobutyl, tert.-butyl, -O-$CF_3$, -S-$CF_3$, phenyl, and -O-benzyl,

or for -($CH_2$)$_m$-O-C(=O)-$R^5$, where m is 1, 2, 3, 4, or 5;

or for -($CH_2$)$_n$-C(=O)-O-$R^6$, where n is 1, 2, 3, 4, or 5;

or for -($CH_2$)-$R^{22}$;

or for -($CH_2$)-O-$R^{23}$, -($CH_2$)-S-$R^{23}$, -($CH_2$)-N($CH_3$)-$R^{23}$, -($CH_2$)-($CH_2$)-O-$R^{23}$, -($CH_2$)-($CH_2$)-S-$R^{23}$, -($CH_2$)-($CH_2$)-NH-$R^{23}$, -($CH_2$)-($CH_2$)-N($CH_3$)-$R^{23}$, -($CH_2$)-($CH_2$)-O-($CH_2$)-$R^{23}$, -($CH_2$)-($CH_2$)-S-($CH_2$)-$R^{23}$, or -($CH_2$)-($CH_2$)-N($CH_3$)-$R^{23}$;

$R^2$ stands for a hydrogen residue;

or for -($CH_2$)-$R^{24}$, -($CH_2$)-O-$R^{24}$, -($CH_2$)-S-$R^{24}$, -($CH_2$)-N($CH_3$)-$R^{24}$, -($CH_2$)-($CH_2$)-$R^{24}$, -($CH_2$)-($CH_2$)-O-$R^{24}$, -($CH_2$)-($CH_2$)-S-$R^{24}$, -($CH_2$)-($CH_2$)-NH-$R^{24}$, -($CH_2$)-($CH_2$)-N($CH_3$)-$R^{24}$, ($CH_2$)-($CH_2$)-($CH_2$)-$R^{24}$, -($CH_2$)-($CH_2$)-O-($CH_2$)-$R^{24}$, -($CH_2$)-($CH_2$)-S-($CH_2$)-$R^{24}$, or-($CH_2$)-($CH_2$)-N($CH_3$)-$R^{24}$;

or for a residue selected from the group consisting of methyl, ethyl, n-propyl, isopropyl, n-butyl, sec-butyl, isobutyl, tert.-butyl, n-pentyl, sec-pentyl, -($CH_2$)-($CH_2$)-(C($CH_3$)$_3$), n-hexyl, n-heptyl, n-octyl, -($CH_2$)-(CH)-($C_2H_5$)-($CH_2$)-($CH_2$)-($CH_2$)-($CH_3$), vinyl, 1-propenyl, 2-propenyl, 1-butenyl, 2-butenyl, 3-butenyl, 2-methyl-2-butenyl, 1-propynyl, 2-propynyl, 1-butynyl, 2-butynyl, and 3-butynyl, wherein the residue can in each case be substituted with 1, 2, 3, 4, or 5 substituents independently selected from the group consisting of F, Cl, Br, I, -CN, -$NO_2$, -OH, -SH, -$NH_2$, -N($CH_3$)$_2$, -N($C_2H_5$)$_2$, and -N-($CH_3$)-($C_2H_5$);

or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrimidinyl, indazolyl, quinazolinyl, quinolinyl, and isoquinolinyl, wherein the residue can in each case be substituted with 1, 2, 3, 4, or 5 substituents independently selected from the group consisting of F, Cl, Br, I, -CN, -$CF_3$, -$SF_5$, -OH, -O-$CH_3$, -O-$C_2H_5$, -O-CH($CH_3$)$_2$, -O-C($CH_3$)$_3$, -O-$CH_2$-$CH_2$-$CH_2$-$CH_3$, -O-$CH_2$-CH=$CH_2$, -$NH_2$, -$NO_2$, -O-$CF_3$, -O-$CHF_2$, -O-$CH_2$F, -S-$CF_3$, -S-$CHF_2$, -S-$CH_2$F, -SH, -S-$CH_3$, -S-$C_2H_5$, -S-CH($CH_3$)$_2$, -S-C($CH_3$)$_3$, -S-$CH_2$-$CH_2$-$CH_2$-$CH_3$, methyl, ethyl, n-propyl, isopropyl, n-butyl, sec-butyl, isobutyl, tert.-butyl, -C(=O)-OH, -C(=O)-O-$CH_3$, -C(=O)-O-$C_2H_5$,-C(=O)-O-C($CH_3$)$_3$, -O-C(=O)-$CH_3$, -O-C(=O)-$C_2H_5$, -O-C(=O)-phenyl, -($CH_2$)-O-C(=O)$CH_3$, -($CH_2$)-O-C(=O)-$C_2H_5$, -($CH_2$)-O-C(=O)-phenyl, -N($CH_3$)$_2$, -N($C_2H_5$)$_2$,-N(H)($CH_3$), -N(H)($C_2H_5$), -NH-C(=O)-O-$CH_3$, -NH-C(=O)-O-$C_2H_5$, -NH-C(=O)-$CH_3$,-NH-C(=O)-$C_2H_5$, -C(=O)-H, -C(=O)-$CH_3$, -C(=O)-$C_2H_5$, -C(=O)-$NH_2$, -C(=O)-NH-$CH_3$,-C(=O)-NH-$C_2H_5$, -S(=O)$_2$-$NH_2$, -S(=O)$_2$-NH-$CH_3$, -S(=O)$_2$-NH-$C_2H_5$, -S(=O)$_2$-NH-phenyl, -($CH_2$)-benzo[b]furanyl, -O-phenyl, -S-phenyl, -S-benzyl, -O-benzyl, phenyl and benzyl, wherein in each case the cyclic moiety of the residues -O-C(=O)-phenyl,-($CH_2$)-O-C(=O)-phenyl, -S(=O)$_2$-NH-phenyl, -O-phenyl, -S-phenyl, -S-benzyl, -O-benzyl, phenyl, -($CH_2$)-benzo[b]furanyl and benzyl can be substituted with 1, 2, 3, 4, or 5 substituents independently selected from the group consisting of F, Cl, Br, -OH,-$CF_3$, -$SF_5$, -CN, -$NO_2$, -O-$CH_3$, -O-$C_2H_5$, -O-CH($CH_3$)$_2$, -O-C($CH_3$)$_3$, methyl, ethyl, n-propyl, isopropyl, n-butyl, sec-butyl, isobutyl, tert.-butyl, -O-$CF_3$, -S-$CF_3$, phenyl and-O-benzyl,

$R^3$ stands for a -C(=O)-$R^4$ group;

and

$R^4$ stands for -($CHR^{10}$)-O-$R^{11}$, -($CHR^{10}$)-S-$R^{11}$, -($CHR^{10}$)-NH-$R^{11}$, -($CHR^{10}$)-O-($CH_2$)-$R^{11}$, -($CHR^{10}$)-S-($CH_2$)-$R^{11}$, -($CHR^{10}$)-NH-($CH_2$)-$R^{11}$, -($CHR^{10}$)-N($CH_3$)-($CH_2$)-$R^{11}$, -($CHR^{10}$)-($CH_2$)-($CH_2$)-O-$R^{11}$, -($CHR^{10}$)-($CH_2$)-($CH_2$)-S-$R^{11}$, -($CHR^{10}$)-($CH_2$)-($CH_2$)-NH-$R^{11}$-($CHR^{10}$)-($CH_2$)-($CH_2$)-N($C_2H_5$)-$R^{11}$, -($CHR^{10}$)-O-($CH_2$)-($CH_2$)-O-$R^{11}$, -($CHR^{10}$)-S-($CH_2$)-($CH_2$)-S-$R^{11}$,-($CHR^{10}$)-O-($CH_2$)-($CH_2$)-S-$R^{11}$ or -($CHR^{10}$)-S-($CH_2$)-($CH_2$)-O-$R^{11}$,

or for -($CR^{12}R^{13}$)-C(=O)-O-$R^7$, -($CR^{12}R^{13}$)-($CH_2$)-C(=O)-O-$R^7$, or -($CR^{12}R^{13}$)-($CH_2$)-($CH_2$)-C(=O)-O-

$R^7$;
or for $-(CR^{14}R^{15})-O-C(=O)-R^8$ or $-(CR^{14}R^5)-(CH_2)$ $-O-C(=O)-R^8$;
or for $-(CHR^{16})-C(=O)-R^9$ or $-(CHR^{16})-(CH_2)-C(=O)-R^9$;
or for $CH[(CH_2)-O-(CH_2)-R^{17}][NH-C(=O)-O-R^{18}]$ or $-CH[(CH_2)-S-(CH_2)-R^{17}][NH-C(=O)-O-R^{18}]$;
or for $CHR^{19}R^{20}$ or $-(CH_2)-CHR^{19}R^{20}$;
or for $-(CH=CH)-R^{21}$;
or for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, n-octyl, $-(CH_2)-(CH)-(C_2H_s)-(CH_2)-(CH_2)-(CH_2)-(CH_3)$, vinyl, 1-propenyl, 2-propenyl, 1-butenyl, 2-butenyl, and 3-butenyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and $N-(CH_3)-(C_2H_5)$;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl, and 7,7-dimethyl-2-oxa-bicyclo[2.2.1] heptyl, wherein the (hetero)cycloaliphatic residue can in each case be substituted with 1, 2, 3, 4, or 5 substituents independently selected from the group consisting of oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$,$-NO_2$, $-O-CF_3$, $-S-CF_3$, -SH, $S-CH_3$, $-S-C_2H_5$, $-S-C(CH_3)_3$, methyl, ethyl, n-propyl, isopropyl, n-butyl, sec-butyl, isobutyl, tert.-butyl, -C(=O)-H, $-C(=O)-CH_3$, $-C(=O)-C_2H_5$, -C(=O)-OH, $-C(=O)-O-CH_3$, $-C(=O)-O-C_2H_5$, $-C(=O)-O-C(CH_3)_3$, $-(CH_2)-C(=O)-OH$,$-(CH_2)-C(=O)-O-CH_3$, $-(CH_2)-C(=O)-O-C_2H_5$, $-N(CH_3)_2$, $-N(C_2H_5)_2$, $-N(H)(CH_3)$,$-N(H)(C_2H_5)$, $-(CH_2)$-benzo[b]furanyl, -O-phenyl, -O-benzyl, phenyl, benzyl, naphthyl, and $-(CH_2)$-naphthyl, wherein in each case the cyclic moiety of the residues -O-phenyl, -O-benzyl, phenyl, $-(CH_2)$-benzo[b]furanyl, benzyl, naphthyl, and $-(CH_2)$-naphthyl can be substituted with 1, 2, 3, 4, or 5 substituents independently selected from the group consisting of F, Cl, Br, -OH, $-CF_3$, $-SF_5$, -CN, $-NO_2$, $-O-CH_3$, $-O-C_2H_5$, methyl, ethyl, n-propyl, isopropyl, n-butyl, sec-butyl, isobutyl, tert.-butyl, $-O-CF_3$, $-S-CF_3$, phenyl and-O-benzyl,
or for a residue selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrimidinyl, indazolyl, quinazolinyl, quinolinyl, isoquinolinyl, phenazinyl, phenothiazinyl, oxadiazolyl, benzo[1,2,5]oxadiazolyl, and chromanyl, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$, $-C(=O)-O-C(CH_3)_3$, $-O-C(=O)-CH_3$,$-O-C(=O)-C_2H_5$, -O-C(=O)-phenyl, $-(CH_2)-O-C(=O)-CH_3$, $-(CH_2)-O-C(=O)-C_2H_5$, $-(CH_2)$-O-C(=O)-phenyl, $-N(CH_3)_2$, $-N(C_2H_5)_2$, $-N(H)(CH_3)$, $-N(H)(C_2H_5)$, $-NH-C(=O)-O-CH_3$,$-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$,$-S(=O)_2-NH-C_2H_5$, $-S(=O)_2$-NH-phenyl, $-(CH_2)$benzo[b]furanyl, -O-phenyl, -S-phenyl, -S-benzyl, -O-benzyl, phenyl and benzyl, wherein in each case the cyclic moiety of the residues -O-C(=O)-phenyl, $-(CH_2)$-O-C(=O)-phenyl, $-S(=O)_2$-NH-phenyl, -O-phenyl, -S-phenyl, -S-benzyl, -O-benzyl, phenyl, $-(CH_2)$-benzo[b]furanyl and benzyl can be substituted with 1, 2, 3, 4, or 5 substituents selected independently from the group consisting of F, Cl, Br, -OH, $-CF_3$, $-SF_5$, -CN, $-NO_2$. $-O-CH_3$, $-O-C_2H_5$, $-O-CH(CH_3)_2$,$-O-C(CH_3)_3$, methyl, ethyl, n-propyl, isopropyl, n-butyl, sec-butyl, isobutyl, tert.-butyl,$-O-CF_3$, $-S-CF_3$, phenyl, and -O-benzyl,

or II.)

$R^1$ stands for a hydrogen residue

or for a residue selected from the group consisting of methyl, ethyl, n-propyl, isopropyl, n-butyl, sec-butyl, isobutyl, tert.-butyl, n-pentyl, sec-pentyl, $-(CH_2)-(CH_2)-(C(CH_3)_3)$, n-hexyl, n-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, 3-butenyl, 2-methyl-2-butenyl, 1-propynyl, 2-propynyl, 1-butynyl, 2-butynyl, and 3-butynyl;
or for an unsubstituted phenyl residue or benzyl residue;

$R^2$ stands for a hydrogen residue;

or for $-(CH_2)-R^{24}$, $-(CH_2)-O-R^{24}$, $-(CH_2)-S-R^{24}$, $-(CH_2)-N(CH_3)-R^{24}$, $-(CH_2)-(CH_2)-R^{24}$, $-(CH_2)-(CH_2)-O-R^{24}$, $-(CH_2)-(CH_2)-S-R^{24}$, $-(CH_2)-(CH_2)-NH-R^{24}$, $-(CH_2)-(CH_2)-N(CH_3)-R^{24}$,$-(CH_2)-(CH_2)-(CH_2)-R^{24}$,$-(CH_2)-(CH_2)-O-(CH_2)-R^{24}$, $-(CH_2)-(CH_2)-S-(CH_2)-R^{24}$ or $-(CH_2)-(CH_2)-N(CH_3)-R^{24}$;
or for a residue selected from the group consisting of methyl, ethyl, n-propyl, isopropyl, n-butyl, sec-butyl, isobutyl, tert.-butyl, n-pentyl, sec-pentyl, $-(CH_2)-(CH_2)-(C(CH_3)_3)$, n-hexyl, n-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, 3-butenyl, 2-methyl-2-butenyl, 1-propynyl, 2-propynyl, 1-butynyl, 2-butynyl, and 3-butynyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$ and $-N-(CH_3)-(C_2H_5)$;
or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo [b]thiophenyl, thiazolyl, oxazolyl, isoxazolyl, pyridazinyl, pyrimidinyl, indazolyl, quinazolinyl, quinolinyl, and isoquinolinyl, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C (=O)-OH, $-C(=O)-O-CH_3$, $-C(=O)-O-C_2H_5$,$-C(=O)-O-C(CH_3)_3$, $-O-C(=O)-CH_3$, $-0-C(=O)-C_2H_5$, -O-C (=O)-phenyl, $-(CH_2)-O-C(=O)-CH_3$, $-(CH_2)-O-C(=O)-C_2H_5$, $-(CH_2)-O-C(=O)$-phenyl, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$ $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phenyl, $-(CH_2)$-benzo[b]furanyl, -O-phenyl, -S-phenyl, -S-benzyl, -O-benzyl, phenyl and benzyl, wherein in each case the cyclic moiety of the residues -O-C(=O)-phenyl, $-(CH_2)-O-C(=O)$-phenyl, $-S(=O)_2$-NH-phenyl, -O-phenyl, -S-phenyl, -S-benzyl, -O-benzyl, phenyl, $-(CH_2)$-benzo[b]furanyl and benzyl can be substituted with 1, 2, 3, 4, or 5 substituents independently selected from the group consisting of F, Cl, Br, -OH, $-CF_3$, $-SF_5$, -CN,$-NO_2$, $-O-CH_3$, $-O-C_2H_5$, $-O-CH(CH_3)_2$, $-O-C(CH_3)_3$, methyl, ethyl, n-propyl, isopropyl, n-butyl, sec-butyl, isobutyl, tert.-butyl, $-O-CF_3$, $-S-CF_3$, phenyl and -O-benzyl,

$R^3$ stands for a $-C(=O)-R^4$ group;

and

$R^4$ stands for $-(CHR^{10})-O-R^{11}$, $-(CHR^{10})-S-R^{11}$, $-(CHR^{10})-NH-R^{11}$, $-(CHR^{10})-O-(CH_2)-R^{11}$, $-(CHR^{10})-S-(CH_2)-R^{11}$, $-(CHR^{10})-NH-(CH_2)-R^{11}$, $-(CHR^{10})-N(CH_3)-(CH_2)-R^{11}$, $-(CHR^{10})-(CH_2)-(CH_2)-O-R^{11}$, $-(CHR^{10})-(CH_2)-(CH_2)-S-R^{11}$, $-(CHR^{10})-(CH_2)-(CH_2)-NH-R^{11}-(CHR^{10})-(CH_2)-(CH_2)-N(C_2H_5)-R^{11}$, $-(CHR^{10})-O-(CH_2)-(CH_2)-O-R^{11}$, $-(CHR^{10})-S-(CH_2)-(CH_2)-S-R^{11}$,$-(CHR^{10})-O-(CH_2)-(CH_2)-S-R^{11}$ or $-(CHR^{10}-S-(CH_2-(CH_2)-O-R^{11}$,

or for $-(CR^{12}R^{13})-C(=O)-O-R^7$, $-(CR^{12}R^{13})-(CH_2)-C(=O)-O-R^7$, or $-(CR^{12}R^{13})-(CH_2)-(CH_2)-C(=O)-O-R^7$;
or for $-(CR^{14}R^{15})-O-C(=O)-R^8$ or $-(CR^{14}R^{15})-(CH_2)$, and $-O-C(=O)-R^8$;
or for $-(CHR^{16})-C(=O)-R^9$ or $-(CHR^{16})-(CH_2)-C(=O)-R^9$;
or for $-CH[(CH_2)-O-(CH_2)-R^{11}][NH-C(=O)-O-R^{18}]$ or $-CH[(CH_2)-S-(CH_2)-R^{17}][NH-C(=O)-O-R^{18}]$;
or for $-CHR^{19}R^{20}$ or $-(CH_2)-CHR^{19}R^{20}$;
or for $-(CH=CH)-R^{21}$;
or for an alkyl residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, n-octyl, and $-(CH_2)-(CH)-(C_2H_5)-(CH_2)-(CH_2)-(CH_2)-(CH_3)$, wherein the alkyl residue is in each case substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and$-N-(CH_3)-(C_2H_5)$;
or for a residue selected from the group consisting of vinyl, 1-propenyl, 2-propenyl, 1-butenyl, 2-butenyl, 3-butenyl, 1-propynyl, 2-propynyl, 1-butynyl, 2-butynyl, and 3-butynyl, wherein residue can in each case be substituted with 1, 2, 3, 4, or 5 substituents independently selected from the group consisting

of F, Cl, Br, I, -CN,-$NO_2$, -OH, -SH, -$NH_2$, -N-$(CH_3)_2$, -$N(C_2H_5)_2$, and -N-$(CH_3)$-$(C_2H_5)$;

or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl, and 7,7-dimethyl-2-oxa-bicyclo[2.2.1] heptyl, wherein the (hetero)cycloaliphatic residue can in each case be substituted with 1, 2, 3, 4, or 5 substituents independently selected from the group consisting of oxo (=O), thioxo (=S), F, Cl, Br, I, -CN, -$CF_3$, -$SF_5$, -OH, -O-$CH_3$, -O-$C_2H_5$, -O-$C(CH_3)_3$, -$NH_2$,-$NO_2$, -O-$CF_3$, -S-$CF_3$, -SH, S-$CH_3$, -S-$C_2H_5$, -S-$C(CH_3)_3$, methyl, ethyl, n-propyl, isopropyl, n-butyl, sec-butyl, isobutyl, tert.-butyl, -C(=O)-H, -C(=O)-$CH_3$, -C(=O)-$C_2H_5$, -C(=O)-OH, -C(=O)-O-$CH_3$, -C(=O)-O-$C_2H_5$, -C(=O)-O-$C(CH_3)_3$, -$(CH_2)$-C(=O)-OH,-$(CH_2)$-C(=O)-O-$CH_3$, -$(CH_2)$-C(=O)-O-$C_2H_5$, -$N(CH_3)_2$, -$N(C_2H_5)_2$, -N(H)$(CH_3)$,-N(H)$(C_2H_5)$, -$(CH_2)$-benzo[b]furanyl, -O-phenyl, -O-benzyl, phenyl, benzyl, naphthyl, and -$(CH_2)$-naphthyl, wherein in each case the cyclic moiety of the residues -O-phenyl, -O-benzyl, phenyl, -$(CH_2)$-benzo[b]furanyl, benzyl, naphthyl, and -$(CH_2)$-naphthyl can be substituted with 1, 2, 3, 4, or 5 substituents independently selected from the group consisting of F, Cl, Br, -OH, -$CF_3$, -$SF_5$, -CN, -$NO_2$, -O-$CH_3$, -O-$C_2H_5$, methyl, ethyl, n-propyl, isopropyl, n-butyl, sec-butyl, isobutyl, tert.-butyl, -O-$CF_3$, -S-$CF_3$, phenyl, and -O-benzyl,

or for a phenyl residue, wherein the phenyl residue can be substituted in each case with 1, 2, 3, 4 or 5 substituents independently selected from the group consisting of -CN,-$SF_5$, -$NH_2$, -$NO_2$, -O-$CF_3$, -O-$CHF_2$, -O-$CH_2F$, -S-$CF_3$, -S-$CHF_2$, -S-$CH_2F$, -SH, -S-$CH_3$, -S-$C_2H_5$, -S-$CH(CH_3)_2$, -S-$C(CH_3)_3$, -S-$CH_2$-$CH_2$-$CH_2$-$CH_3$, -C(=O)-OH, -C(=O)-O-$CH_3$, -C(=O)-O-$C_2H_5$, -C(=O)-O-$C(CH_3)_3$, -O-C(=O)-$CH_3$, -O-C(=O)-$C_2H_5$, -O-C(=O)-phenyl, -$(CH_2)$-O-C(=O)-$CH_3$, -$(CH_2)$-O-C(=O)-$C_2H_5$, -$(CH_2)$-O-C(=O)-phenyl, -$N(CH_3)_2$,-$N(C_2H_5)_2$, -N(H)$(CH_3)$, -N(H)$(C_2H_5)$, -NH-C(=O)-O-$CH_3$, -NH-C(=O)-O-$C_2H_5$, -NH-C(=O)-$CH_3$, -NH-C(=O)-$C_2H_5$, -C(=O)-H, -C(=O)-$CH_3$, -C(=O)-$C_2H_5$, -C(=O)-$NH_2$,-C(=O)-NH-$CH_3$, -C(=O)-NH-$C_2H_5$, -$S(=O)_2$-$NH_2$, -$S(=O)_2$-NH-$CH_3$, -$S(=O)_2$-NH-$C_2H_5$,-$S(=O)_2$-NH-phenyl, -$(CH_2)$-benzo[b]furanyl, -O-phenyl, -S-phenyl, -S-benzyl, -O-benzyl, phenyl and benzyl, wherein in each case the cyclic moiety of the residues -O-C(=O)-phenyl, -$(CH_2)$-O-C(=O)-phenyl, -$S(=O)_2$-NH-phenyl, -O-phenyl, -S-phenyl, -S-benzyl, -O-benzyl, phenyl, -$(CH_2)$-benzo[b]furanyl and benzyl can be substituted with 1, 2, 3, 4 or 5 substituents independently selected from the group consisting of F, Cl, Br, -OH, -$CF_3$, -$SF_5$, -CN, -$NO_2$, -O-$CH_3$, -O-$C_2H_5$, -O-$CH(CH_3)_2$, -O-$C(CH_3)_3$, methyl, ethyl, n-propyl, isopropyl, n-butyl, sec-butyl, isobutyl, tert.-butyl, -O-$CF_3$, -S-$CF_3$, phenyl and -O-benzyl,

or for a residue selected from the group consisting of 1,2,3,4,5-pentafluorophenyl, 1,2,3,4-tetrafluorophenyl, 4-methyl-3-nitrophenyl, 5-methyl-3-nitrophenyl, 6-methyl-3-nitrophenyl, and 2-methyl-3-nitrophenyl;

or for an unsubstituted naphthyl residue;

or for a residue selected from the group consisting of (1,3)-benzodioxolyl and (1,4)-benzodioxanyl, wherein the residue can in each case be substituted with 1, 2, 3, 4, or 5 substituents selected from the group consisting of F, Cl, Br, I, -CN, -$CF_3$, -$SF_5$, -OH, -O-$CH_3$, -O-$C_2H_5$, -O-$CH(CH_3)_2$, -O-$C(CH_3)_3$, -O-$CH_2$-$CH_2$-$CH_2$-$CH_3$, -O-$CH_2$-CH=$CH_2$, -$NH_2$, -$NO_2$, -O-$CF_3$, -O-$CHF_2$, -O-$CH_2F$, -S-$CF_3$, -S-$CHF_2$, -S-$CH_2F$, -SH, -S-$CH_3$, -S-$C_2H_5$, -S-$CH(CH_3)_2$, -S-$C(CH_3)_3$, -S-$CH_2$-$CH_2$-$CH_2$-$CH_3$, methyl, ethyl, n-propyl, isopropyl, n-butyl, sec-butyl, isobutyl, tert.-butyl;

or for a heteroaryl residue selected from the group consisting of thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrimidinyl, indazolyl, quinazolinyl, quinolinyl, isoquinolinyl, phenazinyl, phenothiazinyl, oxadiazolyl, benzo[1,2,5]oxadiazolyl, and chromanyl, wherein the heteroaryl residue can in each case be substituted with 1, 2, 3, 4, or 5 substituents independently selected from the group consisting of F, Cl, Br, I, -CN, -$CF_3$, -$SF_5$, -OH, -O-$CH_3$, -O-$C_2H_5$, -O-$CH(CH_3)_2$, -O-$C(CH_3)_3$, -O-$CH_2$-$CH_2$-$CH_2$-$CH_3$, -O-$CH_2$-CH=$CH_2$, -$NH_2$, -$NO_2$, -O-$CF_3$, -O-$CHF_2$, -O-$CH_2F$, -S-$CF_3$, -S-$CHF_2$, -S-$CH_2F$, -SH, -S-$CH_3$, -S-$C_2H_5$, -S-$CH(CH_3)_2$, -S-$C(CH_3)_3$, -S-$CH_2$-$CH_2$-$CH_2$-$CH_3$, methyl, ethyl, n-propyl, isopropyl, n-butyl, sec-butyl, isobutyl, tert.-butyl, -C(=O)-OH, -C(=O)-O-$CH_3$, -C(=O)-O-$C_2H_5$, -C(=O)-O-$C(CH_3)_3$, -O-C(=O)-$CH_3$, -O-C(=O)-$C_2H_5$, -O-C(=O)-phenyl, -$(CH_2)$-O-C(=O)-$CH_3$, -$(CH_2)$-O-C(=O)-$C_2H_5$, -$(CH_2)$-O-C(=O)-phenyl, -$N(CH_3)_2$, -$N(C_2H_5)_2$,-N(H)$(CH_3)$,- N(H)$(C_2H_5)$, -NH-C(=O)-O-$CH_3$, -NH-C(=O)-O-$C_2H_5$, -NH-C(=O)-$CH_3$,-NH-C(=O)-$C_2H_5$, -C(=O)-H, -C(=O)-$CH_3$, -C(=O)-$C_2H_5$, -C(=O)-$NH_2$, -C(=O)-NH-$CH_3$,-C(=O)-NH-$C_2H_5$, -$S(=O)_2$-$NH_2$, -$S(=O)_2$-NH-$CH_3$, -$S(=O)_2$-NH-$C_2H_5$, -$S(=O)_2$-NH-phenyl, -$(CH_2)$-benzo[b]furanyl, -O-phenyl, -S-phenyl, -S-benzyl, -O-benzyl, phenyl, and benzyl, wherein in each case the cyclic moiety of the residues -O-C(=O)-phenyl,-$(CH_2)$-O-C(=O)-phenyl, -$S(=O)_2$-NH-phenyl, -O-phenyl, -S-phenyl, -S-benzyl, -O-benzyl, phenyl, -$(CH_2)$-ben-

zo[b]furanyl, and benzyl can be substituted with 1, 2, 3, 4, or 5 substituents independently selected from the group consisting of F, Cl, Br, -OH,-$CF_3$, -$SF_5$, -CN, -$NO_2$, -O-$CH_3$, -O-$C_2H_5$, -O-$CH(CH_3)_2$, -O-$C(CH_3)_3$, methyl, ethyl, n-propyl, isopropyl, n-butyl, sec-butyl, isobutyl, tert.-butyl, -O-$CF_3$, -S-$CF_3$, phenyl, and-O-benzyl,

or III.)

$R^1$ stands for a hydrogen residue;

or for a residue selected from the group consisting of methyl, ethyl, n-propyl, isopropyl, n-butyl, sec-butyl, isobutyl, tert.-butyl, n-pentyl, sec-pentyl, -($CH_2$)-($CH_2$)-($C(CH_3)_3$), n-hexyl, n-heptyl, n-octyl, -($CH_2$)-(CH)-($C_2H_5$)-($CH_2$)-($CH_2$)-($CH_2$)-($CH_3$), vinyl, 1-propenyl, 2-propenyl, 1-butenyl, 2-butenyl, 3-butenyl, 2-methyl-2-butenyl, 1-propynyl, 2-propynyl, 1-butynyl, 2-butynyl, and 3-butynyl;
or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo [b]thiophenyl, thiazolyl, oxazolyl, isoxazolyl, pyridazinyl, pyrimidinyl, indazolyl, quinazolinyl, quinolinyl, and isoquinolinyl, wherein the heteroaryl residue can in each case be substituted with 1, 2, 3, 4, or 5 substituents independently selected from the group consisting of F, Cl, Br, I, -CN, -$CF_3$, -$SF_5$, -OH, -O-$CH_3$, -O-$C_2H_5$, -O-$CH(CH_3)_2$, -O-$C(CH_3)_3$, -O-$CH_2$-$CH_2$-$CH_2$-$CH_3$, -O-$CH_2$-CH=$CH_2$, -$NH_2$, -$NO_2$, -O-$CF_3$, -O-$CHF_2$, -O-$CH_2F$, -S-$CF_3$, -S-$CHF_2$, -S-$CH_2F$, -SH, -S-$CH_3$, -S-$C_2H_5$, -S-$CH(CH_3)_2$, -S-$C(CH_3)_3$, -S-$CH_2$-$CH_2$-$CH_2$-$CH_3$, methyl, ethyl, n-propyl, isopropyl, n-butyl, sec-butyl, isobutyl, tert.-butyl, -C (=O)-OH, -C(=O)-O-$CH_3$, -C(=O)-O-$C_2H_5$,-C(=O)-O-$C(CH_3)_3$, -O-C(=O)-$CH_3$, -O-C(=O)-$C_2H_5$, -O-C (=O)-phenyl, -($CH_2$)-O-C(=O)-$CH_3$, -($CH_2$)-O-C(=O)-$C_2H_5$, -($CH_2$)-O-C(=O)-phenyl, -$N(CH_3)_2$, -N $(C_2H_5)_2$, -N(H)($CH_3$), -N(H)($C_2H_5$), -NH-C(=O)-O-$CH_3$, -NH-C(=O)-0-$C_2H_5$, -NH-C(=O)-$CH_3$, -NH-C (=O)-$C_2H_5$, -C(=O)-H, -C(=O)-$CH_3$, -C(=O)-$C_2H_5$, -C(=O)-$NH_2$, -C(=O)-NH-$CH_3$, -C(=O)-NH-$C_2H_5$, -S $(=O)_2$-$NH_2$, -S$(=O)_2$-NH-$CH_3$, -S$(=O)_2$-NH-$C_2H_5$, -S$(=O)_2$-NH-phenyl, -($CH_2$)-Benzo[b]furanyl, -O-phenyl, -S-phenyl, -S-benzyl, -O-benzyl, phenyl, and benzyl, wherein in each case the cyclic moiety of the residues -O-C(=O)-phenyl, -($CH_2$)-O-C(=O)-phenyl, -S$(=O)_2$-NH-phenyl, -O-phenyl, -S-phenyl, -S-benzyl, -O-benzyl, phenyl, -($CH_2$)-benzo[b]furanyl, and benzyl can be substituted with 1, 2, 3, 4, or 5 substituents independently selected from the group consisting of F, Cl, Br, -OH, -$CF_3$, -$SF_5$, -CN,-$NO_2$, - O-$CH_3$, -O-$C_2H_5$, -O-$CH(CH_3)_2$, -O-$C(CH_3)_3$, methyl, ethyl, n-propyl, isopropyl, n-butyl, sec-butyl, isobutyl, tert.-butyl, -O-$CF_3$, -S-$CF_3$, phenyl, and -O-benzyl,
or for -$(CH_2)_m$-O-C(=O)-$R^5$, where m is 1, 2, 3, 4, or 5;
or for -$(CH_2)_n$-C(=O)-O-$R^6$, where n is 1, 2, 3, 4, or 5;
or for an unsubstituted benzyl residue;
or for -($CH_2$)-$R^{22}$;
or for -($CH_2$)-O-$R^{23}$, -($CH_2$)-S-$R^{23}$, -($CH_2$)-N($CH_3$)-$R^{23}$, -($CH_2$)-($CH_2$)-O-$R^{23}$, -($CH_2$)-($CH_2$)-S-$R^{23}$, -($CH_2$)-($CH_2$)-NH-$R^{23}$, -($CH_2$)-($CH_2$)-N($CH_3$)-$R^{23}$, -($CH_2$)-($CH_2$)-O-($CH_2$)-$R^{23}$, -($CH_2$)-($CH_2$)-S-($CH_2$)-$R^{23}$, or -($CH_2$)-($CH_2$)-N($CH_3$)-$R^{23}$;

$R^2$ stands for -($CH_2$)-S-$R^{24}$, -($CH_2$)-N($CH_3$)-$R^{24}$, -($CH_2$)-($CH_2$)-S-$R^{24}$, -($CH_2$)-($CH_2$)-NH-$R^{24}$, -($CH_2$)-($CH_2$)-N($CH_3$)-$R^{24}$, -($CH_2$)-($CH_2$)-S-($CH_2$)-$R^{24}$, or -($CH_2$)-($CH_2$)-N($CH_3$)-$R^{24}$;
$R^3$ stands for a -C(=O)-$R^4$ group;

and

$R^4$ stands for -($CHR^{10}$)-O-$R^{11}$, -($CHR^{10}$)-S-$R^{11}$, -($CHR^{10}$)-O-($CH_2$)-$R^{11}$, -($CHR^{10}$)-S-($CH_2$)-$R^{11}$,-($CHR^{10}$)-($CH_2$)-($CH_2$)-O-$R^{11}$, -($CHR^{10}$)-($CH_2$)-($CH_2$)-S-$R^{11}$, or -($CHR^{10}$)-O-($CH_2$)-($CH_2$)-O-$R^{11}$,

or for -($CR^{12}R^{13}$)-C(=O)-O-$R^7$ or -($CR^{12}R^{13}$)-($CH_2$)-C(=O)-O-$R^7$;
or for -($CR^{14}R^{15}$)-O-C(=O)-$R^8$ or -($CR^{14}R^{15}$)-($CH_2$), and -O-C(=O)-$R^8$;
or for -($CHR^{16}$)-C(=O)-$R^9$;
or for CH[($CH_2$)-O-($CH_2$)-$R^{17}$][NH-C(=O)-O-$R^{18}$];
or for $CHR^{19}R^{20}$ or -($CH_2$)-$CHR^{19}R^{20}$;
or for -(CH=CH)-$R^{21}$;
or for a residue selected from the group consisting of methyl, ethyl, n-propyl, isopropyl, n-butyl, sec-butyl, isobutyl, tert.-butyl, n-pentyl, 2-pentyl, 3-pentyl, -($CH_2$)-($C(CH_3)_3$), n-hexyl, 3-hexyl, -($CH_2$)-($CH_2$)-($C(CH_3)_3$), n-heptyl, 3-heptyl, 4-heptyl, n-octyl, -($CH_2$)-(CH)-($C_2H_5$)-($CH_2$)-($CH_2$)-($CH_2$)-

$(CH_3)$, vinyl, 1-propenyl, 2-propenyl, 1-butenyl, 2-butenyl, and 3-butenyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and $N-(CH_3)-(C_2H_5)$;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, dithiolanyl, adamantyl, and 7,7-dimethyl-2-oxa-bicyclo[2.2.1] heptyl, wherein the (hetero)cycloaliphatic residue can in each case be substituted with 1, 2, 3, 4, or 5 substituents independently selected from the group consisting of oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$,$-NO_2$,- $O-CF_3$, $-S-CF_3$, -SH, $S-CH_3$, $-S-C_2H_5$, $-S-C(CH_3)_3$, methyl, ethyl, n-propyl, isopropyl, n-butyl, sec-butyl, isobutyl, tert.-butyl, -C(=O)-H, $-C(=O)-CH_3$, $-C(=O)-C_2H_5$, -C(=O)-OH, $-C(=O)-O-CH_3$, $-C(=O)-O-C_2H_5$, $-C(=O)-O-C(CH_3)_3$, $-(CH_2)-C(=O)-OH$,$-(CH_2)-C(=O)-O-CH_3$, $-(CH_2)-C(=O)-O-C_2H_5$, $-N(CH_3)_2$, $-N(C_2H_5)_2$, $-N(H)(CH_3)$,$-N(H)(C_2H_5)$, $-(CH_2)$-benzo[b]furanyl, -O-phenyl, -O-benzyl, phenyl, benzyl, naphthyl, and $-(CH_2)$-naphthyl, wherein in each case the cyclic moiety of the residues -O-phenyl, -O-benzyl, phenyl, $-(CH_2)$-benzo[b]furanyl, benzyl, naphthyl, and $-(CH_2)$-naphthyl can be substituted with 1, 2, 3, 4, or 5 substituents independently selected from the group consisting of F, Cl, Br, -OH, $-CF_3$, $-SF_5$, -CN, $-NO_2$, $-O-CH_3$, $-O-C_2H_5$, methyl, ethyl, n-propyl, isopropyl, n-butyl, sec-butyl, isobutyl, tert.-butyl, $-O-CF_3$, $-S-CF_3$, phenyl, and-O-benzyl,
or for a residue selected from the group consisting of phenyl, naphthyl, (1,3)-benzodioxolyl, (1,4)-benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrimidinyl, indazolyl, quinazolinyl, quinolinyl, isoquinolinyl, phenazinyl, phenothiazinyl, oxadiazolyl, benzo[1,2,5]oxadiazolyl und chromanyl, wherein the residue can in each case be substituted with 1, 2, 3, 4 or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$, $-C(=O)-O-C(CH_3)_3$, $-O-C(=O)-CH_3$,$-O-C(=O)-C_2H_5$, -O-C(=O)-phenyl, $-(CH_2)-O-C(=O)-CH_3$, $-(CH_2)-O-C(=O)-C_2H_5$, $-(CH_2)$-O-C(=O)-phenyl, $-N(CH_3)_2$, $-N(C_2H_5)_2$, $-N(H)(CH_3)$, $-N(H)(C_2H_5)$, $-NH-C(=O)-O-CH_3$,$-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$,$-S(=O)_2-NH-C_2H_5$, $-S(=O)_2$-NH-phenyl, $-(CH_2)$-benzo[b]furanyl, -O-phenyl, -S-phenyl,-S-benzyl, -O-benzyl, phenyl und benzyl, wherein in each case the cyclic moiety of the residues -O-C(=O)-phenyl, $-(CH_2)$-O-C(=O)-phenyl, $-S(=O)_2$-NH-phenyl, -O-phenyl, -S-phenyl, -S-benzyl, -O-benzyl, phenyl, $-(CH_2)$-benzo[b]furanyl, and benzyl can be substituted with 1, 2, 3, 4, or 5 substituents independently selected from the group consisting of F, Cl, Br, -OH, $-CF_3$, $-SF_5$, -CN, $-NO_2$, $-O-CH_3$, $-O-C_2H_5$, $-O-CH(CH_3)_2$,$-O-C(CH_3)_3$, methyl, ethyl, n-propyl, isopropyl, n-butyl, sec-butyl, isobutyl, tert.-butyl,$-O-CF_3$, $-S-CF_3$, phenyl, and -O-benzyl,

and in each case, if present, in the aforementioned groups I.) II.) and III.) the residues

$R^5$, $R^6$, $R^9$ and $R^{18}$ each independently

stand for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_Z)-(C(CH_3)_3)$, n-heptyl, 3-heptyl, 4-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, and 3-butenyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and $-N-(CH_3)-(C_2H_5)$;

$R^7$ stands for a hydrogen residue;

or for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, and 3-butenyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and

$N-(CH_3)-(C_2H_5)$;

or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo [b]thiophenyl, thiazolyl, thiadiazolyl, oxazolyl, isoxazolyl, pyridazinyl, pyrimidinyl, indazolyl, quinazolinyl, quinolinyl, isoquinolinyl, phenazinyl, phenothiazinyl, oxadiazolyl, benzo[1,2,5]oxadiazolyl, and chromanyl, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$,$-C(=O)-O-C(CH_3)_3$, $-O-C(=O)-CH_3$, $-O-C(=O)-C_2H_5$, -O-C(=O)-phenyl, $-(CH_2)-O-C(=O)-CH_3$, $-(CH_2)-O-C(=O)-C_2H_5$, $-(CH_2)-O-C(=O)$-phenyl, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phenyl, $-(CH_2)$-benzo[b]furanyl, -O-phenyl, -S-phenyl, -S-benzyl, -O-benzyl, phenyl, and benzyl, wherein in each case the cyclic moiety of the residues -O-C(=O)-phenyl, $-(CH_2)-O-C(=O)$-phenyl, $-S(=O)_2$-NH-phenyl, -O-phenyl, -S-phenyl, -S-benzyl, -O-benzyl, phenyl, $-(CH_2)$-benzo[b]furanyl, and benzyl can be substituted with 1, 2, 3, 4, or 5 substituents independently selected from the group consisting of F, Cl, Br, -OH, $-CF_3$, $-SF_5$, -CN,$-NO_2$, $-O-CH_3$, $-O-C_2H_5$, $-O-CH(CH_3)_2$, $-O-C(CH_3)_3$, methyl, ethyl, n-propyl, isopropyl, n-butyl, sec-butyl, isobutyl, tert.-butyl, $-O-CF_3$, $-S-CF_3$, phenyl, and -O-benzyl,

$R^8$, $R^{16}$, and $R^{24}$ each independently

stand for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, and 3-butenyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and $N-(CH_3)-(C_2H_5)$;

or for a residue selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, quinolinyl, isoquinolinyl, phenazinyl, phenothiazinyl, oxadiazolyl, benzo[1,2,5]oxadiazolyl, and chromanyl, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phenyl, $-(CH_2)-O-C(=O)-CH_3$, $-(CH_2)-O-C(=O)-C_2H_5$, $-(CH_2)-O-C(=O)$-phenyl, $-N(CH_3)_2$, $-N(C_2H_5)_2$,$-N(H)(CH_3)$, $-N(H)(C_2H_5)$, $-NH-C(=O)-O-CH_3$, $-NH-C(=O)-O-C_2H_5$, $-NH-C(=O)-CH_3$,$-NH-C(=O)-C_2H_5$, -C(=O)-H, $-C(=O)-CH_3$, $-C(=O)-C_2H_5$, $-C(=O)-NH_2$, $-C(=O)-NH-CH_3$,$-C(=O)-NH-C_2H_5$, $-S(=O)_2-NH_2$, $-S(=O)_2-NH-CH_3$, $-S(=O)_2-NH-C_2H_5$, $-S(=O)_2$-NH-phenyl, $-(CH_2)$-benzo[b]furanyl, -O-phenyl, -S-phenyl, -S-benzyl, -O-benzyl, phenyl, and benzyl, wherein in each case the cyclic moiety of the residues -O-C(=O)-phenyl,$-(CH_2)-O-C(=O)$-phenyl, $-S(=O)_2$-NH-phenyl, -O-phenyl, -S-phenyl, -S-benzyl, -O-benzyl, phenyl, $-(CH_2)$-benzo[b]furanyl, and benzyl can be substituted with 1, 2, 3, 4, or 5 substituents independently selected from the group consisting of F, Cl, Br, -OH,$-CF_3$, $-SF_5$, -CN, $-NO_2$, $-O-CH_3$, $-O-C_2H_5$, $-O-CH(CH_3)_2$, $-O-C(CH_3)_3$, methyl, ethyl, n-propyl, isopropyl, n-butyl, sec-butyl, isobutyl, tert.-butyl, $-O-CF_3$, $-S-CF_3$, phenyl, and-O-benzyl,

$R^{10}$, $R^{12}$, and $R^{13}$ each independently

stand for a hydrogen residue

or for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, and 3-butenyl, wherein the residue can in each case be

substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and $-N-(CH_3)-(C_2H_5)$;

$R^{11}$ stands for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-Heptyl, 3-heptyl, 4-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl und 3-butenyl, wherein the residue can be substituted in each case by 1, 2, 3, 4 or 5 substituents independently selected from the group consisting of F, Cl, Br, I,-CN, $-NO_2$, -OH, -SH, $-NH_2$, $-N(CH_3)_2$, $-N(C_2H_5)_2$ und $-N(CH_3)-(C_2H_5)$;

or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, pyrrolidinyl, piperidinyl, morpholinyl, piperazinyl, azepanyl, and diazepanyl, wherein the (hetero)cycloaliphatic residue can in each case be substituted with 1, 2, 3, 4, or 5 substituents independently selected from the group consisting of oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, methyl, ethyl, n-propyl, isopropyl, n-butyl, sec-butyl, isobutyl, tert.-butyl, -C(=O)-H,$-C(=O)-CH_3$, $-C(=O)-C_2H_5$, -C(=O)-OH, $-C(=O)-O-CH_3$, $-C(=O)-O-C_2H_5$, and $-C(=O)-O-C(CH_3)_3$,
or for a phenyl or naphthyl residue, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, and phenyl;

$R^{14}$, $R^{15}$, and $R^{19}$ each independently

stand for a hydrogen residue;
or for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, n-octyl, $-(CH_2)-(CH)-(C_2H_5)-(CH_2)-(CH_2)-(CH_2)-(CH_3)$, vinyl, 1-propenyl, 2-propenyl, 1-butenyl, 2-butenyl, and 3-butenyl, wherein the residue can in each case be substituted with 1, 2, 3, 4, or 5 substituents independently selected from the group consisting of F, Cl, Br, I, -CN, $-NO_2$, -OH, -SH, $-NH_2$, $-N(CH_3)_2$, $-N(C_2H_5)_2$, and $N-(CH_3)-(C_2H_5)$;
or for a phenyl residue or naphthyl residue, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, and tert.-butyl;

$R^{20}$ stands for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, pyrrolidinyl, piperidinyl, morpholinyl, piperazinyl, azepanyl, and diazepanyl, wherein the (hetero)cycloaliphatic residue can in each case be substituted with 1, 2, 3, 4, or 5 substituents independently selected from the group consisting of oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, methyl, ethyl, n-propyl, isopropyl, n-butyl, sec-butyl, isobutyl, tert.-butyl, -C(=O)-H,$-C(=O)-CH_3$, $-C(=O)-C_2H_5$, -C(=O)-OH, $-C(=O)-O-CH_3$, $-C(=O)-O-C_2H_5$, and $-C(=O)-O-C(CH_3)_3$;

or for a phenyl or naphthyl residue, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, and phenyl;

$R^{17}$ and $R^{21}$ each independently

stand for a phenyl or naphthyl residue, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, and tert.-butyl;

$R^{22}$ stands for a phenyl residue, wherein the phenyl residue is in each case substituted with 1, 2, 3, 4, or 5 substituents independently selected from the group consisting of F, Cl, Br, I,-CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, $-N(CH_3)_2$,$-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$,$-C(=O)-NH-CH_3$, $-C(=O)-NH-C_2H_5$, $-S(=O)_2-NH_2$, $-S(=O)_2-NH-CH_3$ and $-S(=O)_2-NH-C_2H_5$,

or for a naphthyl residue, wherein the naphthyl residue can be substituted with 1, 2, 3, 4, or 5 substituents independently selected from the group consisting of F, Cl, Br, I, -CN,$-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$,$-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$,-SH,- $S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, and tert.-butyl;
or for a heteroaryl residue selected from the group consisting of thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, and quinolinyl, wherein the heteroaryl residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$,$-O-CHF_2$, $-O-CH_2F$, $-S-CF_3$, $-S-CHF_2$, $-S-CH_2F$, -SH, $-S-CH_3$, -S-C2Hs, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$, and $-C(=O)-O-C(CH_3)_3$,

and

$R^{23}$ stands for a residue selected from the group consisting of phenyl, naphthyl, thiophenyl, furanyl, pyrrolyl, pyrazolyl, pyranyl, triazolyl, pyridinyl, imidazolyl, indolyl, isoindolyl, benzo[b]furanyl, benzo[b]thiophenyl, thiazolyl, thiadiazolyl, oxazolyl, isoxazolyl, pyridazinyl, pyrazinyl, pyrimidinyl, indazolyl, quinazolinyl, and quinolinyl, wherein the residue can in each case be substituted with 1, 2, 3, 4, or 5 substituents independently selected from the group consisting of F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, methyl, ethyl, n-propyl, isopropyl, n-butyl, sec-butyl, isobutyl, tert.-butyl, -C(=O)-OH, $-C(=O)-O-CH_3$, $-C(=O)-O-C_2H_5$, and-$C(=O)-O-C(CH_3)_3$.

**12.** Compounds according to claim 10 or claim 11, **characterized in that**

I.)

$R^1$ stands for $-(CH_2)_mO-C(=O)-R^5$ where m is 1, 2, 3, or 4;

or for $-(CH_2)_n-C(=O)-O-R^6$, where n is 1, 2 or 3;
or for $-(CH_2)-R^{22}$;
or for $-(CH_2)-(CH_2)-O-R^{23}$, $-(CH_2)-(CH_2)-S-R^{23}$, $-(CH_2)-(CH_2)-NH-R^{23}$, or $-(CH_2)-(CH_2)-N(CH_3)-R^{23}$;

$R^2$ stands for a hydrogen residue;

or for $-(CH_2)-R^{24}$, $-(CH_2)-(CH_2)-R^{24}$, $-(CH_2)-(CH_2)-O-R^{24}$, $-(CH_2)-(CH_2)-S-R^{24}$, or $-(CH_2)-(CH_2)-(CH_2)-R^{24}$;

or for an alkyl residue selected from the group consisting of methyl, ethyl, n-propyl, isopropyl, n-butyl, sec-butyl, isobutyl, tert.-butyl, n-pentyl, and sec-pentyl;

$R^3$ stands for a -C(=O)-$R^4$ group;

and

$R^4$ stands for -($CHR^{10}$)-O-$R^{11}$, -($CHR^{10}$)-S-$R^{11}$, -($CHR^{10}$)-O-($CH_2$)-$R^{11}$, -($CHR^{10}$)-S-($CH_2$)-$R^{11}$,-($CHR^{10}$)-($CH_2$)-($CH_2$)-O-$R^{11}$, -($CHR^{10}$)-($CH_2$)-($CH_2$)-S-$R^{11}$, or -($CHR^{10}$)-O-($CH_2$)-($CH_2$)-O-$R^{11}$,

or for -($CR^{12}R^{13}$)-C(=O)-O-$R^7$ or -($CR^{12}R^{13}$)-($CH_2$)-C(=O)-O-$R^7$;
or for -($CR^{14}R^{15}$)-O-C(=O)-$R^8$ or -($CR^{14}R^{15}$)-($CH_2$)-O-C(=O)-$R^8$;
or for -($CHR^{16}$)-C(=O)-$R^9$;
or for -CH[($CH_2$)-O-($CH_2$)-$R^{17}$][NH-C(=O)-O-$R^{18}$];
or for -$CHR^{19}R^{20}$ or -($CH_2$)-$CHR^{19}R^{20}$;
or for -(CH=CH)-$R^{21}$;
or for a residue selected from the group consisting of methyl, ethyl, n-propyl, isopropyl, n-butyl, sec-butyl, isobutyl, tert.-butyl, n-pentyl, 2-pentyl, 3-pentyl, -($CH_2$)-(C($CH_3$)$_3$), n-hexyl, 3-hexyl, -($CH_2$)-($CH_2$)-(C($CH_3$)$_3$), n-heptyl, 3-heptyl, 4-heptyl, -($CH_2$)-N($CH_3$)$_2$,-($CH_2$)-($CH_2$)-N($CH_3$)$_2$, 1-butenyl, 2-butenyl, and 3-butenyl;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, adamantyl, and 7,7-dimethyl-3-oxo-2-oxa-bicyclo[2.2.1]heptyl, and the cycloaliphatic residue can in each case be substituted with 1, 2, 3, 4, or 5 substituents methyl, ethyl, and n-propyl;
or for a residue selected from the group consisting of phenyl, naphthyl, (1,3)-benzodioxolyl, (1,4)-benzodioxanyl, thiophenyl, furanyl, pyrazolyl, triazolyl, pyridinyl, imidazolyl, indolyl, thiazolyl, thiadiazolyl, oxazolyl, isoxazolyl, pyrimidinyl, oxadiazolyl, benzo[1,2,5]oxadiazolyl, and chromanyl, wherein the residue can in each case be substituted with 1, 2, 3, 4, or 5 substituents independently selected from the group consisting of F, Cl, Br, I, -CN, -$CF_3$, -O-$CH_3$, -O-$C_2H_5$, -$NO_2$, -O-$CF_3$, -O-$CHF_2$, -O-$CH_2F$, -S-$CF_3$, -S-$CHF_2$, -S-$CH_2F$, -S-$CH_3$, -S-$C_2H_5$, -S-CH($CH_3$)$_2$, -S-C($CH_3$)$_3$, methyl, ethyl, n-propyl, isopropyl, n-butyl, sec-butyl, isobutyl, tert.-butyl, -($CH_2$)-O-C(=O)-$CH_3$,-($CH_2$)-O-C(=O)-$C_2H_5$, -($CH_2$)-O-C(=O)-phenyl, -N($CH_3$)$_2$, -N($C_2H_5$)$_2$, -NH-C(=O)-$CH_3$,-NH-C(=O)-$C_2H_5$, -S(=O)$_2$-$NH_2$, -O-phenyl, and phenyl, wherein the cyclic moiety of the residues -O-phenyl and phenyl can in each case be substituted with 1, 2, 3, 4, or 5 substituents independently selected from the group consisting of F, Cl, Br, methyl, ethyl, n-propyl, and isopropyl.

or II.)

$R^1$ stands for a hydrogen residue

or for a residue selected from the group consisting of methyl, ethyl, n-propyl, isopropyl, n-butyl, sec-butyl, isobutyl, tert.-butyl, 1-propenyl, 2-propenyl, 1-butenyl, 2-butenyl, 3-butenyl, 2-methyl-2-butenyl, 1-propynyl, and 2-propynyl;
or for an unsubstituted benzyl residue;

$R^2$ stands for a hydrogen residue;

or for -($CH_2$)-$R^{24}$, -($CH_2$)-($CH_2$)-$R^{24}$, -($CH_2$)-($CH_2$)-O-$R^{24}$, -($CH_2$)-($CH_2$)-S-$R^{24}$, or -($CH_2$)-($CH_2$)-($CH_2$)-$R^{24}$;
or for an alkyl residue selected from the group consisting of methyl, ethyl, n-propyl, isopropyl, n-butyl, sec-butyl, isobutyl, tert.-butyl, n-pentyl, and sec-pentyl;

$R^3$ stands for a -C(=O)-$R^4$ group;

and

$R^4$ stands for -($CHR^{10}$)-O-$R^{11}$, -($CHR^{10}$)-S-$R^{11}$, -($CHR^{10}$)-O-($CH_2$)-$R^{11}$, -($CHR^{10}$)-S-($CH_2$)-$R^{11}$,-($CHR^{10}$)-($CH_2$)-($CH_2$)-O-$R^{11}$, -($CHR^{10}$)-($CH_2$)-($CH_2$)-S-$R^{11}$, or -($CHR^{10}$)-O-($CH_2$)-($CH_2$)-O-$R^{11}$,

or for $-(CR^{12}R^{13})-C(=O)-O-R^7$ or $-(CR^{12}R^{13})-(CH_2)-C(=O)-O-R^7$;
or for $-(CR^{10}R^{15})-O-C(=O)-R^8$ or $-(CR^{14}R^{15})-(CH_2)-O-C(=O)-R^8$;
or for $-(CHR^{16})-C(=O)-R^9$;
or for $-CH[(CH_2)-O-(CH_2)-R^{17}][NH-C(=O)-O-R^{18}]$;
or for $-CHR^{19}R^{20}$ or $-(CH_2)-CHR^{19}R^{20}$;
or for $-(CH=CH)-R^{21}$;
or for a residue selected from the group consisting of $-(CH_2)-N(CH_3)_2$, $-(CH_2)-(CH_2)-N(CH_3)_2$, 1-butenyl, 2-butenyl, and 3-butenyl;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, adamantyl, and 7,7-dimethyl-3-oxo-2-oxa-bicyclo[2.2.1]heptyl, wherein the cycloaliphatic residue can in each case be substituted with 1, 2, 3, 4, or 5 substituents methyl, ethyl, and n-propyl;
or for a phenyl residue, wherein the phenyl residue is in each case substituted with 1, 2, 3, 4, or 5 substituents independently selected from the group consisting of -CN, $-NO_2$,$-O-CF_3$, $-O-CHF_2$, $-O-CH_2F$, $-S-CF_3$, $-S-CHF_2$, $-S-CH_2F$, $-(CH_2)-O-C(=O)-CH_3$, $-(CH_2)-O-C(=O)-C_2H_5$, $-(CH_2)-O-C(=O)$-phenyl, $-N(CH_3)_2$, $-N(C_2H_5)_2$, $-N(H)(CH_3)$, $-N(H)(C_2H_5)$, $-NH-C(=O)-CH_3$, $-NH-C(=O)-C_2H_5$, $-S(=O)_2-NH_2$, $-S(=O)_2-NH-CH_3$, $-S(=O)_2-NH-C_2H_5$, and phenyl, wherein the cyclic moiety of the residues $-(CH_2)-O-C(=O)$-phenyl and phenyl can in each case be substituted with 1, 2, 3, 4, or 5 substituents independently selected from the group consisting of F, Cl, Br, methyl, ethyl, n-propyl, isopropyl, n-butyl, sec-butyl, isobutyl, and tert.-butyl,
or for a residue selected from the group consisting of 1,2,3,4,5-pentafluorophenyl, 1,2,3,4-tetrafluorophenyl, 4-methyl-3-nitrophenyl, 5-methyl-3-nitrophenyl, 6-methyl-3-nitrophenyl, and 2-methyl-3-nitrophenyl;
or for an unsubstituted naphthyl residue;
or for an unsubstituted residue selected from the group consisting of (1,3)-benzodioxolyl and (1,4)-benzodioxanyl,
or for a heteroaryl residue selected from the group consisting of thiophenyl, furanyl, pyrazolyl, triazolyl, pyridinyl, imidazolyl, indolyl, thiazolyl, thiadiazolyl, oxazolyl, isoxazolyl, pyrimidinyl, oxadiazolyl, benzo [1,2,5]oxadiazolyl, and chromanyl, wherein the heteroaryl residue can in each case be substituted with 1, 2, 3, 4, or 5 substituents independently selected from the group consisting of F, Cl, Br, I, -CN, $-CF_3$, $-O-CH_3$,$-O-C_2H_5$, $-NO_2$, $-O-CF_3$, $-O-CHF_2$, $-O-CH_2F$, $-S-CF_3$, $-S-CHF_2$, $-S-CH_2F$, $-S-CH_3$, $-S-C_2H_5$, $-S-CH(CH_3)_2$, $-S-C(CH_3)_3$, methyl, ethyl, n-propyl, isopropyl, n-butyl, sec-butyl, isobutyl, tert.-butyl, -O-phenyl, and phenyl, wherein the cyclic moiety of the residues-O-phenyl and phenyl can in each case be substituted with 1, 2, 3, 4, or 5 substituents independently selected from the group consisting of F, Cl, Br, methyl, ethyl, n-propyl, and isopropyl;

or III.)

$R^1$ stands for a hydrogen residue

or for a residue selected from the group consisting of methyl, ethyl, n-propyl, isopropyl, n-butyl, sec-butyl, isobutyl, tert.-butyl, 1-propenyl, 2-propenyl, 1-butenyl, 2-butenyl, 3-butenyl, 2-methyl-2-butenyl, 1-propynyl, and 2-propynyl;
or for $-(CH_2)_m-O-C(=O)-R^5$, where m is 1, 2, 3, or 4;
or for $-(CH_2)_n-C(=O)-O-R^6$, where n is 1, 2 or 3;
or for $-(CH_2)_n-C(=O)-O-R^6$, where n is 1, 2 or 3;
or for an unsubstituted benzyl residue;
or for $-(CH_2)-R^{22}$;
or for $-(CH_2)-(CH_2)-O-R^{23}$, $-(CH_2)-(CH_2)-S-R^{23}$, $-(CH_2)-(CH_2)-NH-R^{23}$, or $-(CH_2)-(CH_2)-N(CH_3)-R^{23}$;

$R^2$ stands for $-(CH_2)-(CH_2)-S-R^{24}$;
$R^3$ stands for a $-C(=O)-R^4$ group;

and

$R^4$ stands for $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$,$-(CHR^{10})-(CH_2)-(CH_2)-O-R^{11}$, $-(CHR^{10})-(CH_2)-(CH_2)-S-R^{11}$, or $-(CHR^{10})-O-(CH_2)-(CH_2)-O-R^{11}$,

or for $-(CR^{12}R^{13})-C(=O)-O-R^7$ or $-(CR^{12}R^{13})-(CH_2)-C(=O)-O-R^7$;
or for $-(CR^{14}R^{15})-O-C(=O)-R^8$ or $-(CR^{14}R^{15})-(CH_2)$ $-O-C(=O)-R^8$;
or for $-(CHR^{16})-C(=O)-R^9$;
or for $CH[(CH_2)-O-(CH_2)-R^{17}][NH-C(=O)-O-R^{18}]$;
or for $CHR^{19}R^{20}$ or $-(CH_2)-CHR^{19}R^{20}$;
or for $-(CH=CH)-R^{21}$;
or for a residue selected from the group consisting of methyl, ethyl, n-propyl, isopropyl, n-butyl, sec-butyl, isobutyl, tert.-butyl, n-pentyl, 2-pentyl, 3-pentyl, $-(CH_2)-(C(CH_3)_3)$, n-hexyl, 3-hexyl, $-(CH_2)-(CH_2)-(C(CH_3)_3)$, n-heptyl, 3-heptyl, 4-heptyl, $-(CH_2)-N(CH_3)_2$,$-(CH_2)-(CH_2)-N(CH_3)_2$, 1-butenyl, 2-butenyl, and 3-butenyl;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, adamantyl, and 7,7-dimethyl-3-oxo-2-oxa-bicyclo[2.2.1]heptyl, wherein the cycloaliphatic residue can in each case be substituted with 1, 2, 3, 4, or 5 substituents methyl, ethyl, and n-propyl;
or for a residue selected from the group consisting of phenyl, naphthyl, (1,3)-benzodioxolyl, (1,4)-benzodioxanyl, thiophenyl, furanyl, pyrazolyl, triazolyl, pyridinyl, imidazolyl, indolyl, thiazolyl, thiadiazolyl, oxazolyl, isoxazolyl, pyrimidinyl, oxadiazolyl, benzo[1,2,5]oxadiazolyl, and chromanyl, wherein the residue can in each case be substituted with 1, 2, 3, 4, or 5 substituents independently selected from the group consisting of F, Cl, Br, I, -CN, $-CF_3$, $-O-CH_3$, $-O-C_2H_5$, $-NO_2$, $-O-CF_3$, $-O-CHF_2$, $-O-CH_2F$, $-S-CF_3$, $-S-CHF_2$, $-S-CH_2F$, $-S-CH_3$, $-S-C_2H_5$, $-S-CH(CH_3)_2$, $-S-C(CH_3)_3$, methyl, ethyl, n-propyl, isopropyl, n-butyl, sec-butyl, isobutyl, tert.-butyl, $-(CH_2)-O-C(=O)-CH_3$,$-(CH_2)-O-C(=O)-C_2H_5$, $-(CH_2)-O-C(=O)$-phenyl, $-N(CH_3)_2$, $-N(C_2H_5)_2$, $-NH-C(=O)-CH_3$,$-NH-C(=O)-C_2H_5$, $-S(=O)_2-NH_2$, -O-phenyl, and phenyl, wherein the cyclic moiety of the residues -O-phenyl and phenyl can in each case be substituted with 1, 2, 3, 4, or 5 substituents independently selected from the group consisting of F, Cl, Br, methyl, ethyl, n-propyl, and isopropyl;

and in each case, if present, in the aforementioned groups I.) II.) and III.) the residue(s)

$R^5$ stands for a methyl or ethyl residue;
$R^6$ stands for a methyl or ethyl residue;
$R^7$ stands for a hydrogen residue

or for a methyl or ethyl residue;

$R^8$ stands for a methyl or ethyl residue

or for an unsubstituted phenyl residue;

$R^9$ stands for a methyl or ethyl residue;
$R^{10}$ stands for a hydrogen residue

or for a methyl or ethyl residue;

$R^{11}$ stands for a methyl or ethyl residue;

or for a phenyl residue, which can be substituted with 1, 2, 3, 4, or 5 substituents independently selected from the group consisting of F, Cl, Br, $-O-CH_3$, $-O-C_2H_5$, $-O-CH(CH_3)_2$, $-O-C(CH_3)_3$, and phenyl;

$R^{12}$ stands for a hydrogen residue or for a methyl residue;
$R^{13}$ stands for a hydrogen residue or for a methyl residue;
$R^{14}$ stands for a hydrogen residue

or for a methyl or ethyl residue
or for an unsubstituted phenyl residue;

$R^{15}$ stands for a hydrogen residue or for a methyl residue.
$R^{16}$ stands for a methyl or ethyl residue

or for an unsubstituted phenyl residue;

$R^{17}$ stands for an unsubstituted phenyl residue;
$R^{18}$ stands for an alkyl residue selected from the group consisting of methyl, ethyl, n-propyl, isopropyl, n-butyl, isobutyl, and tert.-butyl;
$R^{19}$ stands for a hydrogen residue

or for a methyl or ethyl residue
or for an unsubstituted phenyl residue;

$R^{20}$ stands for a residue selected from the group consisting of cyclopentyl, cyclohexyl, and hydantoin;

or for a phenyl residue, which can be substituted with 1, 2, 3, 4, or 5 substituents independently selected from the group consisting of F, Cl, Br, $-O-CH_3$, $-O-C_2H_5$, $-O-CH(CH_3)_2$, $-O-C(CH_3)_3$, and phenyl;

$R^{21}$ stands for a phenyl residue, which can be substituted with 1, 2, 3, 4, or 5 substituents independently selected from the group consisting of F, Cl, Br, and $-CF_3$;
$R^{22}$ stands for a phenyl residue, wherein the phenyl residue is in each case substituted with 1, 2, 3, 4, or 5 substituents independently selected from the group consisting of F, Cl, Br, -CN, $-CF_3$, $-NO_2$, $-O-CF_3$, $-S-CF_3$, methyl, ethyl, n-propyl, isopropyl, n-butyl, sec-butyl, isobutyl, tert.-butyl, -C(=O)-OH, and $-C(=O)-O-CH_3$ $-C(=O)-O-C_2H_5$;

or for an unsubstituted naphthyl residue;

$R^{23}$ stands for an unsubstituted phenyl residue

and

$R^{24}$ stands for a methyl or ethyl residue

or for an unsubstituted phenyl residue.

**13.** Substituted 1,4,8-triazaspiro[4.5]decan-2-one compounds of the general formula I according to one or more of the previous claims 1 to 12 and selected from the group consisting of

(1) 3-(*S*)-Benzyl-8-(2-ethylbutyryl)-1,4,8-triazaspiro[4.5]decan-2-one,
(2) 8-(2,4-Dimethoxybenzoyl)-3-(*S*)-(2-methylsulfanylethyl)-1,4,8-triazaspiro[4.5]decan-2-one,
(3) 3-(*S,R*)-Benzyl-8-(2-ethylbutyryl)-1,4,8-triazaspiro[4.5]decan-2-one,
(4) 8-Acetyl-3-(*S*)-benzyl-1,4,8-triazaspiro[4.5]decan-2-one,
(5) 3-(*S,R*)-Benzyl-8-(4-chlorobenzoyl)-1,4,8-triazaspiro[4.5]decan-2-one,
(6) 3-(*S*)-Benzyl-8-(4-chlorobenzoyl)-1,4,8-triazaspiro[4.5]decan-2-one,
(7) 8-(2-ethylbutyryl)-3-(*S*)-(2-methylsulfanylethyl)-1,4,8-triazaspiro[4.5]decan-2-one,
(8) 1,3-(*S*)-Dibenzyl-8-(2-ethylbutyryl)-1,4,8-triazaspiro[4.5]decan-2-one,
(9) 8-Acetyl-1,3-(*S*)-dibenzyl-1,4,8-triazaspiro[4.5]decan-2-one,
(10) 3-(*S,R*)-Benzyl-8-(4-chlorobenzoyl)-1-(4-methoxybenzyl)-1,4,8-triazaspiro[4.5]decan-2-one,
(11) 3-(*S,R*)-Benzyl-8-(4-chlorobenzoyl)-1-(4-fluorobenzyl)-1,4,8-triazaspiro[4.5]decan-2-one,
(12) 1,3-(*S,R*)-Dibenzyl-8-(4-chlorobenzoyl)-1,4,8-triazaspiro[4.5]decan-2-one,
(13) 3-(*S,R*)-Benzyl-8-(2-ethylbutyryl)-1-(4-methoxybenzyl)-1,4,8-triazaspiro[4.5]decan-2-one,
(14) 3-(*S,R*)-Benzyl-8-(2-ethylbutyryl)-1-(4-fluorobenzyl)-1,4,8-triazaspiro[4.5]decan-2-one,
(15) 1,3-(*S,R*)-Dibenzyl-8-(2-ethylbutyryl)-1,4,8-triazaspiro[4.5]decan-2-one,
(16) 1-Benzyl-8-(2-ethylbutyryl)-3-(*S*)-(2-methylsulfanylethyl)-1,4,8-triazaspiro[4.5]decan-2-one,
(17) 1,3-(*S*)-Dibenzyl-8-(4-chlorobenzoyl)-1,4,8-triazaspiro[4.5]decan-2-one,
(18) 1,3-(*S*)-Dibenzyl-8-(2-ethylbutyryl)-1,4,8-triazaspiro[4.5]decan-2-one,
(19) 8-Acetyl-1,3-(*S*)-dibenzyl-1,4,8-triazaspiro[4.5]decan-2-one,
(20) 3-(*S,R*)-Benzyl-8-(4-chlorobenzoyl)-1-(4-methoxybenzyl)-1,4,8-triazaspiro[4.5]decan-2-one,
(21) 3-(*S,R*)-Benzyl-8-(4-chlorobenzoyl)-1-(4-fluorobenzyl)-1,4,8-triazaspiro[4.5]decan-2-one,
(22) 1,3-(*S,R*)-Dibenzyl-8-(4-chlorobenzoyl)-1,4,8-triazaspiro[4.5]decan-2-one,
(23) 3-(*S,R*)-Benzyl-8-(2-ethylbutyryl)-1-(4-methoxybenzyl)-1,4,8-triazaspiro[4.5]decan-2-one,

(24) 3-(*S,R*)-Benzyl-8-(2-ethylbutyryl)-1-(4-fluorobenzyl)-1,4,8-triazaspiro[4.5]decan-2-one,
(25) 1,3-(*S,R*)-Dibenzyl-8-(2-ethylbutyryl)-1,4,8-triazaspiro[4.5]decan-2-one,
(26) 1-Benzyl-8-(2-ethylbutyryl)-3-(*S*)-(2-methylsulfanylethyl)-1,4,8-triazaspiro[4.5]decan-2-one,
(27) 1,3-(*S*)-Dibenzyl-8-(4-chlorobenzoyl)-1,4,8-triazaspiro[4.5]decan-2-one,
(28) 1-Benzyl-8-(2,4-Dimethoxybenzoyl)-3-(*S*)-(2-methylsulfanylethyl)-1,4,8-triazaspiro[4.5]decan-2-one,
(29) 1,3-(*S*)-Dibenzyl-1,4,8-triazaspiro[4.5]decan-2-one,
(30) 1-Benzyl-3-(*S*)-(2-methylsulfanylethyl)-1,4,8-triazaspiro[4.5]decan-2-one,
(31) 1,3-(*S*)-Dibenzyl-8-butyryl-1,4,8-triazaspiro[4.5]decan-2-one,
(32) 1,3-(*S*)-Dibenzyl-8-(3-fluoro-4-trifluoromethylbenzyl)-1,4,8-triazaspiro[4.5]decan-2-one,
(33) 1,3-(*S*)-Dibenzyl-8-(2,3-difluorobenzoyl)-1,4,8-triazaspiro[4.5]decan-2-one,
(34) 1,3-(*S*)-Dibenzyl-8-[2-(4-chlorophenoxy)-acetyl]-1,4,8-triazaspiro[4.5]decan-2-one,
(35) 1,3-(*S*)-Dibenzyl-8-diphenylacetyl-1,4,8-triazaspiro[4.5]decan-2-one,
(36) 1,3-(*S*)-Dibenzyl-8-(2-phenoxyacetyl)-1,4,8-triazaspiro[4.5]decan-2-one,
(37) 1,3-(*S*)-Dibenzyl-8-(3-phenylpropionyl)-1,4,8-triazaspiro[4.5]decan-2-one,
(38) 1,3-(*S*)-Dibenzyl-8-(naphthalin-2-carbonyl)-1,4,8-triazaspiro[4.5]decan-2-one,
(39) 1,3-(*S*)-Dibenzyl-8-(furan-2-carbonyl)-1,4,8-triazaspiro[4.5]decan-2-one,
(40) 1,3-(*S*)-Dibenzyl-8-(3-methoxybenzoyl)-1,4,8-triazaspiro[4.5]decan-2-one,
(41) 1,3-(*S*)-Dibenzyl-8-(4-fluorobenzoyl)-1,4,8-triazaspiro[4.5]decan-2-one,
(42) 1-Benzyl-8-(4-fluorobenzoyl)-3-(*S*)-(2-methylsulfanylethyl)-1,4,8-triazaspiro[4.5]decan-2-one,
(43) 1,3-(*S*)-Dibenzyl-8-butyryl-1,4,8-triazaspiro[4.5]decan-2-one,
(44) 1,3-(*S*)-Dibenzyl-8-(3-fluoro-4-trifluoromethylbenzoyl)-1,4,8-triazaspiro[4.5]decan-2-one,
(45) 1,3-(*S*)-Dibenzyl-8-(2,3-difluorobenzoyl)-1,4,8-triazaspiro[4.5]decan-2-one,
(46) 1,3-(*S*)-Dibenzyl-8-[2-(4-chlorophenoxy)-acetyl]-1,4,8-triazaspiro[4.5]decan-2-one,
(47) 1,3-(*S*)-Dibenzyl-8-diphenylacetyl-1,4,8-triazaspiro[4.5]decan-2-one,
(48) 1,3-(*S*)-Dibenzyl-8-(2-phenoxyacetyl)-1,4,8-triazaspiro[4.5]decan-2-one,
(49) 1,3-(*S*)-Dibenzyl-8-(3-phenylpropionyl)-1,4,8-triazaspiro[4.5]decan-2-one,
(50) 1,3-(*S*)-Dibenzyl-8-(naphthalin-2-carbonyl)-1,4,8-triazaspiro[4.5]decan-2-one,
(51) 1,3-(*S*)-Dibenzyl-8-(furan-2-carbonyl)-1,4,8-triazaspiro[4.5]decan-2-one,
(52) 1,3-(*S*)-Dibenzyl-8-(3-methoxybenzoyl)-1,4,8-triazaspiro[4.5]decan-2-one,
(53) 1,3-(*S*)-Dibenzyl-8-(4-fluorobenzoyl)-1,4,8-triazaspiro[4.5]decan-2-one,
(54) 1-Benzyl-8-(4-fluorobenzoyl)-3-(*S*)-(2-methylsulfanylethyl)-1,4,8-triazaspiro[4.5]decan-2-one,
(55) N-[4-(3-Isobutyl-2-oxo-1-prop-2-ynyl-1,4,8-triazaspiro[4.5]decan-8-carbonyl)-phenyl]-acetamide,
(56) 1-(2-Phenoxyethyl)-8-(thiophene-2-carbonyl)-1,4,8-triazaspiro[4.5]decan-2-one,
(57) 2-(2-Oxo-8-pent-4-enoyl-1,4,8-triazaspiro[4.5]dec-1-ylmethyl)-benzonitrile,
(58) 8-(2,4-Dimethoxybenzoyl)-1-methyl-3-(2-methylsulfanylethyl)-1,4,8-triazaspiro[4.5]decan-2-one,
(59) 2-[8-(2-Ethylbutyryl)-2-oxo-1,4,8-triazaspiro[4.5]dec-1-ylmethyl]-benzonitrile,
(60) 4-(2-Isobutyl-3-oxo-1,4,8-triazaspiro[4.5]decan-8-carbonyl)-benzonitrile,
(61) 8-(6-Chloropyridine-3-carbonyl)-3-isobutyl-1,4,8-triazaspiro[4.5]decan-2-one,
(62) 2-[8-(2-Methylpentanoyl)-2-oxo-1,4,8-triazaspiro[4.5]dec-1-ylmethyl]-benzonitrile,
(63) 1-Benzyl-8-(biphenyl-4-carbonyl)-1,4,8-triazaspiro[4.5]decan-2-one,
(64) 3-Isobutyl-8-(5-methylisoxazole-3-carbonyl)-1,4,8-triazaspiro[4.5]decan-2-one,
(65) Ethyl 3-oxo-3-[2-oxo-l-(2-phenoxyethyl)-1,4,8-triazaspiro[4.5]dec-8-yl]-propionate,
(66) 8-(2-Chlorobenzoyl)-3-isopropyl-1,4,8-triazaspiro[4.5]decan-2-one,
(67) 8-Cyclopentanecarbonyl-3-isopropyl-1,4,8-triazaspiro[4.5]decan-2-one,
(68) 8-(Furan-2-carbonyl)-1-methyl-3-(2-methylsulfanylethyl)-1,4,8-triazaspiro[4.5]decan-2-one,
(69) 3-Benzyl-8-(2-ethylsulfanylpyridine-3-carbonyl)-1-methyl-1,4,8-triazaspiro[4.5]decan-2-one,
(70) 3-Benzyl-8-(4-chlorobenzoyl)-1,4,8-triazaspiro[4.5]decan-2-one,
(71) 8-(2-Benzyloxyacetyl)-3-isobutyl-1,4,8-triazaspiro[4.5]decan-2-one,
(72) 3-Benzyl-8-(2-methoxyacetyl)-1-prop-2-ynyl-1,4,8-triazaspiro[4.5]decan-2-one,
(73) 8-(5-tert.-Butyl-2-methyl-2H-pyrazole-3-carbonyl)-3-isobutyl-1,4,8-triazaspiro[4.5]decan-2-one,
(74) 2-{8-[3-(2-chloro-6-fluorophenyl)-5-methylisoxazole-4-carbonyl]-2-oxo-1,4,8-triazaspiro[4.5]dec-1-ytmethyl}-benzonitri!e,
(75) 3-Benzyl-8-(2-chlorobenzoyl)-1,4,8-triazaspiro[4.5]decan-2-one,
(76) 3-Benzyl-8-(3-dimethylaminobenzoyl)-1-methyl-1,4,8-triazaspiro[4.5]decan-2-one,
(77) 8-(3-Methylbenzoyl)-1-(2-phenoxyethyl)-1,4,8-triazaspiro[4.5]decan-2-one,
(78) 3-Isopropyl-1-(3-methylbut-2-enyl)-8-(pyridine-4-carbonyl)-1,4,8-triazaspiro[4.5]decan-2-one,
(79) 1-Benzyl-8-(4-methyl[1,2,3]thiadiazole-5-carbonyl)-1,4,8-triazaspiro[4.5]decan-2-one,
(80) 2-{8-[3-(2-chlorophenyl)-acryloyl]-2-oxo-1,4,8-triazaspiro[4.5]dec-1-ylmethyl}-benzonitrile,

(81) 8-(3-Chlorobenzoyl)-3-isobutyl-1,4,8-triazaspiro[4.5]decan-2-one,
(82) Ethyl 2-(2-benzyl-3-oxo-1,4,8-triazaspiro[4.5]dec-8-yl)-2-oxo-l-phenylacetate,
(83) 8-(3,5-Dimethoxybenzoyl)-3-isobutyl-1,4,8-triazaspiro[4.5]decan-2-one,
(84) 3-Benzyl-8-(isoxazole-5-carbonyl)-1,4,8-triazaspiro[4.5]decan-2-one,
(85) 8-(3-Chlorothiophene-2-carbonyl)-3-isobutyl-1,4,8-triazaspiro[4.5]decan-2-one,
(86) 3-Isopropyl-8-pentafluorobenzoyl-1,4,8-triazaspiro[4.5]decan-2-one,
(87) 8-(2,5-Dimethylfuran-3-carbonyl)-3-isobutyl-1,4,8-triazaspiro[4.5]decan-2-one,
(88) 1-Butyl-8-[2-(3,4-dimethoxyphenyl)-acetyl]-3-(2-methylsulfanylethyl)-1,4,8-triazaspiro[4.5]decan-2-one,
(89) 1-Benzyl-3-isopropyl-8-(pyridin-4-carbonyl)-1,4,8-triazaspiro[4.5]decan-2-one,
(90) 1,3-Dibenzyl-8-(3-dimethylaminobenzoyl)-1,4,8-triazaspiro[4.5]decan-2-one,
(91) 5-{2-[1-(4-Fluorobenzyl)-3-(2-methylsulfanylethyl)-2-oxo-1,4,8-triazaspiro[4.5]dec-8-yl]-2-oxoethyl}-imidazolidine-2,4-dione,
(92) 8-(Biphenyl-4-carbonyl)-1-(4-fluorobenzyl)-3-isobutyl-1,4,8-triazaspiro[4.5]decan-2-one,
(93) 2-[2-Oxo-8-(2-propylpentanoyl)-1,4,8-triazaspiro[4.5]dec-1-ylmethyl]-benzonitrile,
(94) 2-[8-(Furan-2-carbonyl)-3-isopropyl-2-oxo-1,4,8-triazaspiro[4.5]dec-1-ylmethyl]-benzonitrile,
(95) 8-[2-(4-Chlorophenoxy)-acetyl]-3-isobutyl-1-prop-2-ynyl-1,4,8-triazaspiro[4.5]decan-2-one,
(96) 1,3-Dibenzyl-8-(4-bromobenzoyl)-1,4,8-triazaspiro[4.5]decan-2-one,
(97) 8-(3-Difluoromethylsulfanylbenzoyl)-3-isobutyl-1,4,8-triazaspiro[4.5]decan-2-one,
(98) 8-(2,3-Dimethylbenzoyl)-3-isopropyl-1,4,8-triazaspiro[4,5]decan-2-one,
(99) 3-Benzyl-8-(2,3-dimethylbenzoyl)-1,4,8-triazaspiro[4.5]decan-2-one,
(100) 1-(4-Fluorobenzyl)-3-(2-methylsulfanylethyl)-8-(pyridin-2-carbonyl)-1,4,8-triazaspiro[4.5]decan-2-one,
(101) 3-Benzyl-8-(3,3-dimethylbutyryl)-1,4,8-triazaspiro[4.5]decan-2-one,
(102) 2-[8-(3-Dimethylaminobenzoyl)-3-isobutyl-2-oxo-1,4,8-triazaspiro[4.5]dec-1-ylmethyl]-benzonitrile,
(103) 3-[8-(2-Methoxyacetyl)-3-(2-methylsulfanylethyl)-2-oxo-1,4,8-triazaspiro[4,5]dec-1-ylmethyl]-benzonitrile,
(104) Ethyl 2-(3-benzyl-1-methyl-2-oxo-1,4,8-triazaspiro[4,5]dec-8-yl)-2-oxo-1-phenylacetate,
(105) 2-[8-[2-(2-Methoxyethoxy)-acetyl]-3-(2-methylsulfanylethyl)-2-oxo-1,4,8-triazaspiro[4.5]dec-1-ylmethyl]-benzonitrile,
(106) 3-Benzyl-8-[3-(2-chloro-6-fluorophenyl)-5-methylisoxazole-4-carbonyl]-1,4,8-triazaspiro[4.5]decan-2-one,
(107) 8-(2-Chloro-6-fluorobenzoyl)-3-isopropyl-1,4,8-triazaspiro[4.5]decan-2-one,
(108) 1-Methyl-3-(2-methylsulfanylethyl)-8-(2-phenoxyacetyl)-1,4,8-triazaspiro[4.5]decan-2-one,
(109) 8-(2-Chloropyridine-3-carbonyl)-1-(4-fluorobenzyl)-3-isobutyl-1,4,8-triazaspiro[4,5]decan-2-one,
(110) 8-(2-Ethylbutyryl)-1-methyl-3-(2-methylsulfanylethyl)-1,4,8-triazaspiro[4,5]decan-2-one,
(111) 1-Methyl-3-(2-methylsulfanylethyl)-8-(2-phenoxypyridine-3-carbonyl)-1,4,8-triazaspiro[4,5]decan-2-one,
(112) 8-(3-Fluorobenzoyl)-1-methyl-3-(2-methylsulfanylethyl)-1,4,8-triazaspiro[4.5]decan-2-one,
(113) 3-Benzyl-8-(naphthalene-2-carbonyl)-1,4,8-triazaspiro[4.5]decan-2-one,
(114) 8-Cyclohexanecarbonyl-1-(2-phenoxyethyl)-1,4,8-triazaspiro[4.5]decan-2-one,
(115) 8-(2-Phenoxyacetyl)-1-(2-phenoxyethyl)-1,4,8-triazaspiro[4.5]decan-2-one,
(116) 4-[1-Butyl-3-(2-methylsulfanylethyl)-2-oxo-1,4,8-triazaspiro[4.5]decan-8-carbonyl]-benzonitrile,
(117) 3-Benzyl-8-(3,3-dimethylbutyryl)-1-methyl-1,4,8-triazaspiro[4.5]decan-2-one,
(118) 3-Benzyl-8-(4-propylbenzoyl)-1,4,8-triazaspiro[4.5]decan-2-one,
(119) 3-Isopropyl-8-(2-phenoxypropionyl)-1,4,8-triazaspiro[4.5]decan-2-one,
(120) 1-Butyl-8-hexanoyl-3-isopropyl-1,4,8-triazaspiro[4.5]decan-2-one,
(121) 8-(4-Bromo-3-methylbenzoyl)-3-isopropyl-1,4,8-triazaspiro[4.5]decan-2-one,
(122) 8-[3-(2-Chloro-6-fluorophenyl)-5-methylisoxazole-4-carbonyl]-3-isopropyl-1-(2-phenoxyethyl)-1,4,8-triazaspiro[4.5]decan-2-one,
(123) 1-(2-Fluorobenzyl)-3-isobutyl-8-(3-methylbenzoyl)-1,4,8-triazaspiro[4.5]decan-2-one,
(124) 8-(2-Ethylhexanoyl)-1-(2-phenoxyethyl)-1,4,8-triazaspiro[4.5]decan-2-one,
(125) 3-Benzyl-8-(3,4-difluorobenzoyl)-1,4,8-triazaspiro[4.5]decan-2-one,
(126) 3-Benzyl-8-(4-ethoxybenzoyl)-1-methyl-1,4,8-triazaspiro[4.5]decan-2-one,
(127) 1-Benzyl-8-(6-chloropyridine-3-carbonyl)-1,4,8-triazaspiro[4.5]decan-2-one,
(128) 8-(3-Dimethylaminobenzoyl)-1-(3,5-dimethylbenzyl)-3-(2-methylsulfanylethyl)-1,4,8-triazaspiro[4.5]decan-2-one,
(129) 3-[8-(Benzo[1,3]dioxol-5-carbonyl)-3-(2-methylsulfanylethyl)-2-oxo-1,4,8-triazaspiro[4.5]dec-1-ylmethyl]-benzonitrile,
(130) 3-Benzyl-1-methyl-8-pent-4-enoyl-1,4,8-triazaspiro[4.5]decan-2-one,
(131) 8-(4-Ethoxybenzoyl)-1-methyl-3-(2-methylsulfanylethyl)-1,4,8-triazaspiro[4.5]decan-2-one,

(132) 3-Benzyl-8-(2-benzyloxyacetyl)-1-methyl-1,4,8-triazaspiro[4.5]decan-2-one,
(133) 8-(3,4-Difluorobenzoyl)-1-(2-phenoxyethyl)-1,4,8-triazaspiro[4.5]decan-2-one,
(134) 3-Benzyl-1-methyl-8-(2-methylsulfanylpyridin-3-carbonyl)-1,4,8-triazaspiro[4.5]decan-2-one,
(135) 1-Butyl-8-(4-methoxybenzoyl)-3-(2-methylsulfanylethyl)-1,4,8-triazaspiro[4.5]decan-2-one,
(136) 1,3-Dibenzyl-8-(2-ethylsulfanylpyridin-3-carbonyl)-1,4,8-triazaspiro[4.5]decan-2-one,
(137) 3-Benzyl-8-(3-difluoromethylsulfanylbenzoyl)-1,4,8-triazaspiro[4.5]decan-2-one,
(138) 1-(4-Fluorobenzyl)-8-(furan-2-carbonyl)-3-(2-methylsulfanylethyl)-1,4,8-triazaspiro[4.5]decan-2-one,
(139) 3-Benzyl-8-(3-fluoro-4-methylbenzoyl)-1-methyl-1,4,8-triazaspiro[4.5]decan-2-one,
(140) 8-[3-(2-Chloro-6-fluorophenyl)-5-methylisoxazole-4-carbonyl]-3-isopropyl-1,4,8-triazaspiro[4.5]decan-2-one,
(141) 1-Butyl-8-(6-Chloro-2H-chromane-3-carbonyl)-3-(2-methylsulfanylethyl)-1,4,8-triazaspiro[4.5]decan-2-one,
(142) 3-[8-(3-Methoxybenzoyl)-3-(2-methylsulfanylethyl)-2-oxo-1,4,8-triazaspiro[4.5]dec-1-ylmethyl]-benzonitrile,
(143) 8-Cyclobutanecarbonyl-3-(2-methylsulfanylethyl)-1-prop-2-ynyl-1,4,8-triazaspiro[4.5]decan-2-one,
(144) 3-Benzyl-1-butyl-8-(furan-2-carbonyl)-1,4,8-triazaspiro[4.5]decan-2-one,
(145) 1-Benzyl-8-(3-chlorothiophene-2-carbonyl)-3-isobutyl-1,4,8-triazaspiro[4.5]decan-2-one,
(146) 3-Benzyl-8-(2,5-bis-trifluoromethylbenzoyl)-1-butyl-1,4,8-triazaspiro[4.5]decan-2-one,
(147) 8-(3-Chloro-2-fluorobenzoyl)-1-(3,5-dimethylbenzyl)-3-(2-methylsulfanylethyl)-1,4,8-triazaspiro[4.5]decan-2-one,
(148) 1-Benzyl-8-(2-chloropyridin-3-carbonyl)-3-isopropyl-1,4,8-triazaspiro[4.5]decan-2-one,
(149) 3-Isobutyi-8-pentafluorobenzoyl-1-prop-2-ynyl-1,4,8-triazaspiro[4.5]decan-2-one,
(150) 8-(2-Benzyloxyacetyl)-1-butyl-3-(2-methylsulfanylethyl)-1,4,8-triazaspiro[4,5]decan-2-one,
(151) 8-(Furan-2-carbonyl)-3-isobutyl-1-prop-2-ynyl-1,4,8-triazaspiro[4.5]decan-2-one,
(152) 1-Butyl-3-(2-methylsulfanylethyl)-8-(4-phenoxybutyryl)-1,4,8-triazaspiro[4.5]decan-2-one,
(153) 3-Benzyl-8-(6-chloropyridine-3-carbonyl)-1-methyl-1,4,8-triazaspiro[4.5]decan-2-one,
(154) 1-(2-Fluorobenzyl)-3-(2-methylsulfanylethyl)-8-(2-phenoxypyridine-3-carbonyl)-1,4,8-triazaspiro[4.5]decan-2-one,
(155) 1-(2-Fluorobenzyl)-3-isobutyl-8-(2-methyl-6-trifluoromethylpyridine-3-carbonyl)-1,4,8-triazaspiro[4.5]decan-2-one,
(156) 8-[2-(3-Chlorophenoxy)-acetyl]-3-isopropyl-1-(2-phenoxyethyl)-1,4,8-triazaspiro[4.5]decan-2-one,
(157) 8-(2,3-Dimethylbenzoyl)-1-methyl-3-(2-methylsulfanylethyl)-1,4,8-triazaspiro[4.5]decan-2-one,
(158) 3-Isopropyl-8-(2-methyl-6-trifluoromethylpyridine-3-carbonyl)-1-(2-phenoxyethyl)-1,4,8-triazaspiro[4.5]decan-2-one,
(159) 3-Benzyl-1-methyl-8-(5-methyl-2-phenyl-2H-[1,2,3]triazole-4-carbonyl)-1,4,8-triazaspiro[4.5]decan-2-one,
(160) 8-[3-(2-Chlorophenyl)-5-methylisoxazole-4-carbonyl]-1-(3,5-dimethylbenzyl)-3-(2-methylsulfanylethyl)-1,4,8-triazaspiro[4.5]decan-2-one,
(161) 1-(2-Fluorobenzyl)-3-(2-methylsulfanylethyl)-8-(pyridine-2-carbonyl)-1,4,8-triazaspiro[4.5]decan-2-one,
(162) 1-Benzyl-3-(2-methylsulfanylethyl)-8-(2-phenoxypyridine-3-carbonyl)-1,4,8-triazaspiro[4.5]decan-2-one,
(163) 1,3-Dibenzyl-8-(3-chlorothiophene-2-carbonyl)-1,4,8-triazaspiro[4.5]decan-2-one,
(164) 3-Benzyl-8-(4-tert.-butylbenzoyl)-1-methyl-1.4,8-triazaspiro[4.5]decan-2-one,
(165) 2-[3-Isobutyl-8-(2-methoxyacetyl)-2-oxo-1,4,8-triazaspiro[4.5]dec-1-ylmethyl]-benzonitrile,
(166) 3-Benzyl-1-butyl-8-(5-fluoro-2-trifluoromethylbenzoyl)-1,4,8-triazaspiro[4.5]decan-2-one,
(167) 3-Benzyl-8-[2-(4-methoxyphenyl)-acetyl]-1-methyl-1,4,8-triazaspiro[4.5]decan-2-one,
(168) 1,3-Dibenzyl-8-(4-methyl[1,2,3]thiadiazole-5-carbonyl)-1,4,8-triazaspiro[4.5]decan-2-one,
(169) 1-Benzyl-3-isopropyl-8-(2-methyl-6-trifluoromethylpyridine-3-carbonyl)-1 ,4,8-triazaspiro[4.5]decan-2-one,
(170) 1-Benzyl-8-(4-ethoxybenzoyl)-3-isobutyl-1,4,8-triazaspiro[4.5]decan-2-one,
(171) 3-Benzyl-1-butyl-8-cyclohexanecarbonyl-1,4,8-triazaspiro[4.5]decan-2-one,
(172) 3-[8-[3-(2-Chloro-6-fluorophenyl)-5-methylisoxazole-4-carbonyl]-3-(2-methylsulfanylethyl)-2-oxo-1,4,8-triazaspiro[4.5]dec-1-ylmethyl]-benzonitrile,
(173) 1-(2-Fluorobenzyl)-3-isobutyl-8-pent-4-enoyl-1,4,8-triazaspiro[4.5]decan-2-one,
(174) 1-Methyl-3-(2-methylsulfanylethyl)-8-(2-propylpentanoyl)-1,4,8-triazaspiro[4.5]decan-2-one,
(175) 3-Benzyl-1-methyl-8-(2-phenoxyacetyl)-1,4,8-triazaspiro[4.5]decan-2-one,
(176) 3-Isobutyl-1-prop-2-ynyl-8-(3-trifluoromethylbenzoyl)-1,4,8-triazaspiro[4.5]decan-2-one,
(177) 3-Benzyl-8-(furan-2-carbonyl)-1-prop-2-ynyl-1,4,8-triazaspiro[4.5]decan-2-one,
(178) 1-Methyl-3-(2-methylsulfanylethyl)-8-(naphthalene-1-carbonyl)-1,4,8-triazaspiro[4.5]decan-2-one,

(179) 3-Benzyl-1-butyl-8-(3-cyclopentylpropionyl)-1,4,8-triazaspiro[4.5]decan-2-one,
(180) 1-(3,5-Dimethylbenzyl)-3-(2-methylsulfanylethyl)-8-pentanoyl-1,4,8-triazaspiro[4.5]decan-2-one,
(181) 3-Benzyl-1-butyl-8-(2-methoxyacetyl)-1,4,8-triazaspiro[4.5]decan-2-one,
(182) 3-Benzyl-8-(3-fluoro-4-trifluoromethylbenzoyl)-1-methyl-1,4,8-triazaspiro[4.5]decan-2-one,
(183) 1-Benzyl-8-(3-difluoromethylsulfanylbenzoyl)-3-isopropyl-1,4,8-triazaspiro[4.5]decan-2-one,
(184) 8-(2-Chloro-6-fluoro-3-methylbenzoyl)-1-methyl-3-(2-methylsulfanylethyl)-1,4,8-triazaspiro[4.5]decan-2-one,
(185) Methyl 4-[3-isopropyl-8-(2-methylsulfanylpyridine-3-carbonyl)-2-oxo-1,4,8-triazaspiro[4.5]dec-1-ylmethyl]benzoate,
(186) 8-[2-(2,5-Dimethoxyphenyl)-acetyl]-1-(2-fluorobenzyl)-3-(2-methylsulfanylethyl)-1 ,4,8-triazaspiro[4.5] decan-2-one,
(187) 8-(5-tert.-Butyl-2-methyffuran-3-carbonyl)-3-isopropyl-1-(2-phenoxyethyl)-1,4,8-triazaspiro[4.5]decan-2-one,
(188) 8-(2-Cyclopentoacetyl)-1-(4-fluorobenzyl)-3-isobutyl-1,4,8-triazaspiro[4.5]decan-2-one,
(189) Methyl 4-[8-(3,3-dimethylbutyryl)-3-isopropyl-2-oxo-1,4,8-triazaspiro[4.5]dec-1-ylmethyl]benzoate,
(190) 3-[8-Cyclopropanecarbonyl-3-(2-methylsulfanylethyl)-2-oxo-1,4,8-triazaspiro[4.5]dec-1-ylmethyl]-benzonitrile,
(191) 3-[3-(2-Methylsulfanylethyl)-2-oxo-8-(3-trifluoromethoxybenzoyl)-1,4,8-triazaspiro[4.5]dec-1-ylmethyl]-benzonitrile,
(192) 1-Butyl-8-(2-cyclopentylacetyl)-3-isopropyl-1,4,8-triazaspiro[4.5]decan-2-one,
(193) 3-Benzyl-1-butyl-8-(pyridine-2-carbonyl)-1,4,8-triazaspiro[4.5]decan-2-one,
(194) 3-Benzyl-8-[3-(2-chlorophenyl)-acryloyl]-1-methyl-1,4,8-triazaspiro[4.5]decan-2-one,
(195) Methyl 4-[8-(3-fluoro-4-trifluoromethylbenzoyl)-3-isopropyl-2-oxo-1,4,8-triazaspiro[4.5]dec-1-ylmethyl] benzoate,
(196) 8-[3-(2,6-Dichlorophenyl)-5-methylisoxazole-4-carbonyl]-1-methyl-3-(2-methylsulfanylethyl)-1,4,8-triazaspiro[4.5]decan-2-one,
(197) 1-Butyl-8-cyclohexanecarbonyl-3-(2-methylsulfanylethyl)-1,4,8-triazaspiro[4.5]decan-2-one,
(198) 3-Benzyl-1-butyl-8-(4-iodobenzoyl)-1,4,8-triazaspiro[4.5]decan-2-one
(199) 1-Methyl-3-(2-methylsulfanylethyl)-8-[3-(3-trifluoromethylphenyl)-acryloyl]-1,4,8-triazaspiro[4.5]decan-2-one,
(200) 1,3-Dibenzyl-8-(4-phenoxybutyryl)-1,4,8-triazaspiro[4.5]decan-2-one
(201) 3-Benzyl-8-(2-chloro-6-fluorobenzoyl)-1-prop-2-ynyl-1,4,8-triazaspiro[4.5]decan-2-one,
(202) Methyl 4-[8-(2-chloropyridine-3-carbonyl)-3-isopropyl-2-oxo-1,4,8-triazaspiro[4.5]dec-1-ylmethyl]benzoate,
(203) 8-(2,5-Dimethylfuran-3-carbonyl)-3-isopropyl-1-(2-phenoxyethyl)-1,4,8-triazaspiro[4.5]decan-2-one,
(204) 8-(Biphenyl-4-carbonyl)-3-(2-methylsulfanylethyl)-1-prop-2-ynyl-1,4,8-triazaspiro[4.5]decan-2-one,
(205) 8-(3-Chlorothiophene-2-carbonyl)-3-(2-methylsulfanylethyl)-1-prop-2-ynyl-1,4,8-triazaspiro[4.5]decan-2-one,
(206) 1-(4-Fluorobenzyl)-8-[2-(4-methoxyphenyl)-acetyl]-3-(2-methylsulfanylethyl)-1,4,8-triazaspiro[4.5]decan-2-one,
(207) 1-Benzyl-3-isopropyl-8-(4-methyl[1,2,3]thiadiazole-5-carbonyl)-1,4,8-triazaspiro[4.5]decan-2-one,
(208) 2-[3-Isopropyl-8-(2-methylsulfanylpyridin-3-carbonyl)-2-oxo-1,4,8-triazaspiro[4.5]dec-1-ylmethyl]-benzonitrile,
(209) 3-[8-(5-tert.-Butyl-2-methyl-2H-pyrazole-3-carbonyl)-3-isobutyl-2-oxo-1,4,8-triazaspiro[4.5]dec-1-ylmethyl]-benzonitrile,
(210) 1-Butyl-8-(2,5-dimethylfuran-3-carbonyl)-3-(2-methylsulfanylethyl)-1,4,8-triazaspiro[4.5]decan-2-one,
(211) 8-(3-Cyclopentylpropionyl)-1-(2-fluorobenzyl)-3-isobutyl-1,4,8-triazaspiro[4.5]decan-2-one,
(212) 1-Benzyl-8-(3-cyclopentylpropionyl)-3-(2-methylsulfanylethyl)-1,4,8-triazaspiro[4.5]decan-2-one,
(213) 3-(2-Methylsulfanylethyl)-8-(4-phenoxybutyryl)-1-prop-2-ynyl-1,4,8-triazaspiro[4.5]decan-2-one,
(214) 1,3-Dibenzyl-8-(3-phenylacryloyl)-1,4,8-triazaspiro[4.5]decan-2-one,
(215) 3-Benzyl-8-(6-chloro-2-fluoro-3-methylbenzoyl)-1-methyl-1,4,8-triazaspiro[4.5]decan-2-one,
(216) 8-[3-(2-Chlorophenyl)-acryloyl]-3-isopropyl-1-(2-phenoxyethyl)-1,4,8-triazaspiro[4.5]decan-2-one,
(217) 2-[3-Isopropyl-2-oxo-8-(3-phenylacryloyl)-1,4,8-triazaspiro[4.5]dec-1-ylmethyl]-benzonitrile,
(218) 3-Benzyl-1-methyl-8-(4-methyl-3-nitrobenzoyl)-1,4,8-triazaspiro[4.5]decan-2-one,
(219) 1-Benzyl-8-(furan-2-carbonyl)-3-isopropyl-1,4,8-triazaspiro[4.5]decan-2-one,
(220) 1-Butyl-8-(3,5-dimethoxybenzoyl)-3-(2-methylsulfanylethyl)-1,4,8-triazaspiro[4.5]decan-2-one,
(221) 1,3-Dibenzyl-8-(3,3-dimethylbutyryl)-1,4,8-triazaspiro[4.5]decan-2-one,
(222) 8-(2,6-Difluoro-3-methylbenzoyl)-1-methyl-3-(2-methylsulfanylethyl)-1,4,8-triazaspiro[4.5]decan-2-one,

(223) 2-[8-(2-Chloro-6-fluorobenzoyl)-3-(2-methylsulfanylethyl)-2-oxo-1,4,8-triazaspiro[4.5]dec-1-ylmethyl]-benzonitrile,
(224) 8-[3-(2-Chloro-6-fluorophenyl)-5-methylisoxazole-4-carbonyl]-3-isopropyl-1-(3-methylbut-2-enyl)-1,4,8-triazaspiro[4.5]decan-2-one,
(225) 3-Isobutyl-1-prop-2-ynyl-8-(4-trifluoromethoxybenzoyl)-1,4,8-triazaspiro[4.5]decan-2-one,
(226) 1-Benzyl-8-(2-chloro-6-fluoro-3-methylbenzoyl)-1,4,8-triazaspiro[4.5]decan-2-one,
(227) Benzyl 2-(1-benzyl-3-isopropyl-2-oxo-1,4,8-triazaspiro[4.5]decan-8-carbonyl)benzoate,
(228) 1,3-Dibenzyl-8-(2-phenylbutyryl)-1,4,8-triazaspiro[4.5]decan-2-one,
(229) 3-Benzyl-1-methyl-8-(4-nitrobenzoyl)-1,4,8-triazaspiro[4.5]decan-2-one,
(230) 3-Benzyl-1-methyl-8-(5-methylisoxazole-3-carbonyl)-1,4,8-triazaspiro[4.5]decan-2-one,
(231) 1-Benzyl-8-(6-chloro-2-fluoro-3-methylbenzoyl)-3-(2-methylsulfanylethyl)-1,4,8-triazaspiro[4.5]decan-2-one,
(232) 1-Benzyl-8-(3-fluoro-4-trifluoromethylbenzoyl)-3-isobutyl-1,4,8-triazaspiro[4.5]decan-2-one,
(233) 3-Benzyl-8-(6-chloro-2H-chromane-3-carbonyl)-1-methyl-1,4,8-triazaspiro[4.5]decan-2-one,
(234) 3-Benzyl-1-butyl-8-(4-methyl[1,2,3]thiadiazole-5-carbonyl)-1,4,8-triazaspiro[4.5]decan-2-one,
(235) 3-Benzyl-8-(4-methyl[1,2,3]thiadiazole-5-carbonyl)-1-prop-2-ynyl-1,4,8-triazaspiro[4.5]decan-2-one,
(236) 2-[8-(6-Chloro-2H-chromane-3-carbonyl)-3-isopropyl-2-oxo-1,4,8-triazaspiro[4.5]dec-1-ylmethyl]-benzonitrile,
(237) 2-[8-(5-Methylisoxazole-3-carbonyl)-3-(2-methylsulfanylethyl)-2-oxo-1,4,8-triazaspiro[4.5]dec-1-ylmethyl]-benzonitrile,
(238) 2-[8-(3-Chloro-2-fluorobenzoyl)-3-isopropyl-2-oxo-1,4,8-triazaspiro[4.5]dec-1-ylmethyl]-benzonitrile,
(239) Methyl 4-[8-(5-tert.-butyl-2-methyl-2H-pyrazole-3-carbonyl)-3-isopropyl-2-oxo-1,4,8-triazaspiro[4.5]dec-1-ylmethyl]benzoate,
(240) 3-Benzyl-1-butyl-8-(5-tert.-butyl-2-methyl-2H-pyrazole-3-carbonyl)-1,4,8-triazaspiro[4.5]decan-2-one,
(241) 3-Benzyl-1-butyl-8-(2-chloro-4-trifluoromethylpyrimidine-5-carbonyl)-1,4,8-triazaspiro[4.5]decan-2-one,
(242) 3-Benzyl-8-(5-methylisoxazole-3-carbonyl)-1-prop-2-ynyl-1,4,8-triazaspiro[4.5]decan-2-one,
(243) Methyl 4-[8-(2-chloropyridine-3-carbonyl)-2-oxo-1,4,8-triazaspiro[4.5]dec-1-ylmethyl]benzoate,
(244) 8-(2-tert.-Butyl-5-methyl-2H-pyrazole-3-carbonyl)-1-(2-fluorobenzyl)-1,4,8-triazaspiro[4.5]decan-2-one,
(245) Methyl 4-[8-(2-methylsulfanylpyridine-3-carbonyl)-2-oxo-1,4,8-triazaspiro[4.5]dec-1-ylmethyl]benzoate,
(246) Butyl 4-[8-(4-acetylaminobenzoyl)-3-isobutyl-2-oxo-1,4,8-triazaspiro[4.5]dec-1-yl]acetate,
(247) Ethyl [8-(4-acetylaminobenzoyl)-3-benzyl-2-oxo-1,4,8-triazaspiro[4.5]dec-1-yl]acetate,
(248) Butyl 4-[8-(2-ethylsulfanylpyridine-3-carbonyl)-3-isobutyl-2-oxo-1,4,8-triazaspiro[4.5]dec-1-yl]acetate,
(249) Methyl 4-[8-(4-methyl[1,2,3]thiadiazole-5-carbonyl)-2-oxo-1,4,8-triazaspiro[4.5]dec-1-ylmethyl]benzoate,
(250) 4-[1-Allyl-3-(2-methylsulfanylethyl)-2-oxo-1,4,8-triazaspiro[4.5]decan-8-carbonyl]-benzolsulfonamide,
(251) Methyl 4-(8-cyclobutanecarbonyl-2-oxo-1,4,8-triazaspiro[4.5]dec-1-ylmethyl)benzoate,
(252) Ethyl 2-[1-allyl-3-(2-methylsulfanylethyl)-2-oxo-1,4,8-triazaspiro[4.5]dec-8-yl]-2-oxo-acetate,
(253) 8-(Biphenyl-4-carbonyl)-1-(2-fluorobenzyl)-1,4,8-triazaspiro[4.5]decan-2-one,
(254) Ethyl [3-(2-Methylsulfanylethyl)-2-oxo-8-propionyl-1,4,8-triazaspiro[4.5]dec-1-yl]acetate,
(255) 8-(Benzo[1,3]dioxole-5-carbonyl)-1-(2,6-dichlorobenzyl)-3-isobutyl-1,4,8-triazaspiro[4.5]decan-2-one,
(256) 1-Allyl-8-(biphenyl-4-carbonyl)-3-isopropyl-1,4,8-triazaspiro[4.5]decan-2-one,
(257) Ethyl [3-benzyl-8-(biphenyl-4-carbonyl)-2-oxo-1,4,8-triazaspiro[4.5]dec-1-yl]acetate,
(258) Ethyl [8-(3-dimethylaminobenzoyl)-3-(2-methylsulfanylethyl)-2-oxo-1,4,8-triazaspiro[4.5]dec-1-yl]acetate,
(259) 3-(2-Oxo-8-pentanoyl-1,4,8-triazaspiro[4.5]dec-1-ylmethyl)-benzonitrile,
(260) Methyl 4-(8-cyclopentanecarbonyl-2-oxo-1,4,8-triazaspiro[4.5]dec-1-ylmethyl)benzoate,
(261) Ethyl 4-[1-(2-fluorobenzyl)-2-oxo-1,4,8-triazaspiro[4.5]dec-8-yl]-4-oxo-butanoate,
(262) 1-(2-Fluorobenzyl)-8-(3,4,5-trimethoxybenzoyl)-1,4,8-triazaspiro[4.5]decan-2-one,
(263) Methyl 4-[8-(2-chloropyridine-4-carbonyl)-2-oxo-1,4,8-triazaspiro[4.5]dec-1-ylmethyl]benzoate,
(264) 3-[8-(3,5-Bis-trifluoromethylbenzoyl)-2-oxo-1,4,8-triazaspiro[4.5]dec-1-ylmethyl]-benzonitrile,
(265) 3-Benzyl-1-(2-fluorobenzyl)-8-(2-methoxybenzoyl)-1,4,8-triazaspiro[4.5]decan-2-one,
(266) 8-(Benzo[1,2,5]oxadiazole-5-carbonyl)-1-(2,6-dichlorobenzyl)-3-isobutyl-1,4,8-triazaspiro[4.5]decan-2-one,
(267) Ethyl [3-benzyl-2-oxo-8-(4-sulfamoylbenzoyl)-1,4,8-triazaspiro[4.5]dec-1-yl]acetate,
(268) 3-[2-Oxo-8-(3,4,5-trimethoxybenzoyl)-1,4,8-triazaspiro[4.5]dec-1-ylmethyl]-benzonitrile,
(269) Ethyl 2-[1-(4-acetoxybutyl)-3-isobutyl-2-oxo-1,4,8-triazaspiro[4.5]dec-8-yl]-1,1-dimethyl-2-oxo-acetate,
(270) 8-(6-Chloropyridine-3-carbonyl)-1-(2,6-dichlorobenzyl)-3-isobutyl-1,4,8-triazaspiro[4.5]decan-2-one,
(271) 8-(2-Ethoxybenzoyl)-1-(2-fluorobenzyl)-1,4,8-triazaspiro[4.5]decan-2-one,
(272) 1-Allyl-8-cyclopropanecarbonyl-3-(2-methylsulfanylethyl)-1,4,8-triazaspiro[4.5]decan-2-one,

(273) 3-[3-Isopropyl-8-(2-methoxyacetyl)-2-oxo-1,4,8-triazaspiro[4.5]dec-1-ylmethyl]-benzonitrile,
(274) Methyl 4-(2-oxo-8-phenylacetyl-1,4,8-triazaspiro[4.5]dec-1-ylmethyl)benzoate,
(275) Ethyl 2-[1-(3-cyanobenzyl)-2-oxo-1,4,8-triazaspiro[4.5]dec-8-yl]-2-oxo-1-phenylacetate,
(276) 1-(2-Fluorobenzyl)-8-(4-methoxybenzoyl)-1,4,8-triazaspiro[4.5]decan-2-one,
(277) Methyl 4-(8-cyclohexanecarbonyl-2-oxo-1,4,8-triazaspiro[4.5]dec-1-ylmethyl)benzoate,
(278) 1-(2-Fluorobenzyl)-8-pent-4-enoyl-1,4,8-triazaspiro[4.5]decan-2-one,
(279) Ethyl [3-isobutyl-8-(3-ethylbutyryl)-2-oxo-1,4,8-triazaspiro[4.5]dec-1-yl]acetate,
(280) 1-Allyl-8-(3,3-dimethylbutyryl)-3-(2-methylsulfanylethyl)-1,4,8-triazaspiro[4.5]decan-2-one,
(281) 1-Allyl-3-(2-methylsulfanylethyl)-8-(2-methylsulfanylpyridin-3-carbonyl)-1,4,8-triazaspiro[4.5]decan-2-one,
(282) Ethyl [3-isobutyl-8-(2-Methylpentanoyl)-2-oxo-1,4,8-triazaspiro[4.5]dec-1-yl]acetate,
(283) Methyl 4-[8-(5-tert.-butyl-2-methylfuran-3-carbonyl)-2-oxo-1,4,8-triazaspiro[4.5]dec-1-ylmethyl]benzoate,
(284) Ethyl (3-benzyl-8-cyclopropanecarbonyl-2-oxo-1,4,8-triazaspiro[4.5]dec-1-yl)acetate,
(285) Ethyl [3-benzyl-8-(3-methylbutyryl)-2-oxo-1,4,8-triazaspiro[4.5]dec-1-yl]acetate,
(286) 1-(2,6-Dichlorobenzyl)-8-(2,5-dimethylfuran-3-carbonyl)-3-isobutyl-1,4,8-triazaspiro[4.5]decan-2-one,
(287) 1-Allyl-3-isopropyl-8-[2-(3-methoxyphenyl)-acetyl]-1,4,8-triazaspiro[4.5]decan-2-one,
(288) Ethyl [8-(4-tert.-butylbenzoyl)-3-(2-methylsulfanylethyl)-2-oxo-1,4,8-triazaspiro[4.5]dec-1-yl]acetate,
(289) 3-(2-Methylsulfanylethyl)-1-(2-nitrobenzyl)-8-(2-phenoxypyridine-3-carbonyl)-1,4,8-triazaspiro[4.5]decan-2-one,
(290) Ethyl (3-benzyl-2-oxo-8-pentanoyl-1,4,8-triazaspiro[4.5]dec-1-yl)acetate,
(291) 8-(2-Chloropyridine-4-carbonyl)-1-(2-fluorobenzyl)-1,4,8-triazaspiro[4.5]decan-2-one.
(292) Ethyl [8-(3-methylbutyryl)-3-(2-methylsulfanylethyl)-2-oxo-1,4,8-triazaspiro[4.5]dec-1-yl]acetate,
(293) 1-Allyl-3-(2-methylsulfanylethyl)-8-pentafluorobenzoyl-1,4,8-triazaspiro[4.5]decan-2-one,
(294) 1-(2-Fluorobenzyl)-8-(2-trifluoromethylbenzoyl)-1,4,8-triazaspiro[4.5]decan-2-one,
(295) 8-(Benzo[1,2,5]oxadiazole-5-carbonyl)-1-(2-fluorobenzyl)-1,4,8-triazaspiro[4.5]decan-2-one,
(296) Methyl 4-[8-(4-tert.-butylbenzoyl)-2-oxo-1,4,8-triazaspiro[4.5]dec-1-ylmethyl]benzoate,
(297) Ethyl [3-benzyl-2-oxo-8-(1-phenyl-5-propyl-1H-pyrazole-4-carbonyl)-1,4,8-triazaspiro[4.5]dec-1-yl]acetate,
(298) 1-Allyl-3-isopropyl-8-pent-4-enoyl-1,4,8-triazaspiro[4.5]decan-2-one,
(299) 3-[2-Oxo-8-(2-trifluoromethylbenzoyl)-1,4,8-triazaspiro[4.5]dec-1-ylmethyl]-benzonitrile,
(300) 8-(2-Dimethylaminoacetyl)-1-(3,5-dimethylbenzyl)-3-isobutyl-1,4,8-triazaspiro[4.5]decan-2-one,
(301) Butyl [8-(2-ethoxybenzoyl)-3-isobutyl-2-oxo-1,4,8-triazaspiro[4.5]dec-1-yl]acetate,
(302) 1-(2-Fluorobenzyl)-8-(3-methoxybenzoyl)-1,4,8-triazaspiro[4.5]decan-2-one,
(303) Ethyl [8-(furan-2-carbonyl)-3-isobutyl-2-oxo-1,4,8-triazaspiro[4.5]dec-1-yl]acetate,
(304) 8-(5-tert.-Butyl-2-methyl-2H-pyrazole-3-carbonyl)-1-(2-fluorobenzyl)-1,4,8-triazaspiro[4.5]decan-2-one,
(305) 1-(2-Fluorobenzyl)-8-(thiophene-2-carbonyl)-1,4,8-triazaspiro[4.5]decan-2-one,
(306) Ethyl [3-benzyl-8-(2-fluorobenzoyl)-2-oxo-1,4,8-triazaspiro[4.5]dec-1-yl]acetate,
(307) Ethyl [8-(isoxazole-5-carbonyl)-3-(2-methylsulfanylethyl)-2-oxo-1,4,8-triazaspiro[4.5]dec-1-yl]acetate,
(308) 1-(2,6-Dichlorobenzyl)-3-isobutyl-8-(4-propylbenzoyl)-1,4,8-triazaspiro[4.5]decan-2-one,
(309) Ethyl [8-[2-(2,5-dimethoxyphenyl)-acetyl]-3-(2-methylsulfanylethyl)-2-oxo-1,4,8-triazaspiro[4.5]dec-1-yl] acetate,
(310) Ethyl (3-benzyl-8-cyclobutanecarbonyl-2-oxo-1,4,8-triazaspiro[4.5]dec-1-yl)acetate,
(311) 1-(2-Fluorobenzyl)-8-[2-(4-methoxyphenyl)-acetyl]-1,4,8-triazaspiro[4.5]decan-2-one,
(312) 1-Allyl-3-(2-methylsulfanylethyl)-8-(2-methyl-6-trifluoromethylpyridine-3-carbonyl)-1,4,8-triazaspiro[4.5] decan-2-one,
(313) Ethyl [3-(2-methylsulfanylethyl)-2-oxo-8-(4-trifluoromethylbenzoyl)-1,4,8-triazaspiro[4.5]dec-1-yl]acetate,
(314) 1-Allyl-3-(2-methylsulfanylethyl)-8-(1-phenyl-5-propyl-1H-pyrazole-4-carbonyl)-1,4,8-triazaspiro[4.5]decan-2-one,
(315) 1-(2,6-Dichlorobenzyl)-8-(2,3-difluoro-4-methylbenzoyl)-3-isobutyl-1,4,8-triazaspiro[4.5]decan-2-one,
(316) 1-Allyl-3-isopropyl-8-(2-methylbenzoyl)-1,4,8-triazaspiro[4.5]decan-2-one,
(317) 8-(2-Chloro-5-trifluoromethylbenzoyl)-1-(2,6-dichlorobenzyl)-3-isobutyl-1,4,8-triazaspiro[4.5]decan-2-one,
(318) 1-Allyl-8-[2-(3-methoxyphenyl)-acetyl]-3-(2-methylsulfanylethyl)-1,4,8-triazaspiro[4.5]decan-2-one,
(319) 1-Allyl-3-(2-methylsulfanylethyl)-8-(3-phenylpropionyl)-1,4,8-triazaspiro[4.5]decan-2-one,
(320) Ethyl [3-benzyl-8-(2-benzyloxyacetyl)-2-oxo-1,4,8-triazaspiro[4.5]dec-1-yl]acetate,
(321) 1-Allyl-8-(5-tert.-butyl-2-methyl-2H-pyrazole-3-carbonyl)-3-(2-methylsulfanylethyl)-1,4,8-triazaspiro[4.5] decan-2-one,

(322) Ethyl 1,1-dimethyl-2-[3-(2-methylsulfanylethyl)-1-(2-nitrobenzyl)-2-oxo-1,4,8-triazaspiro[4.5]dec-8-yl]-2-oxo-acetate,
(323) Benzyl 2-[1-ethoxycarbonylmethyl-3-(2-methylsulfanylethyl)-2-oxo-1,4,8-triazaspiro[4.5]decan-8-carbonyl]benzoate,
(324) 1-Allyl-3-isopropyl-8-(4-methyl[1,2,3]thiadiazole-5-carbonyl)-1,4,8-triazaspiro[4.5]decan-2-one,
(325) 1-Allyl-8-(2,5-dimethylfuran-3-carbonyl)-3-(2-methylsulfanylethyl)-1,4,8-triazaspiro[4.5]decan-2-one,
(326) 1-Allyl-8-(benzo[1,2,5]oxadiazole-5-carbonyl)-3-(2-methylsulfanylethyl)-1,4,8-triazaspiro[4.5]decan-2-one,
(327) 1-(2-Fluorobenzyl)-8-(4-phenoxybutyryl)-1,4,8-triazaspiro[4.5]decan-2-one,
(328) 1-Allyl-8-(2-cydopentylacetyl)-3-(2-methylsulfanylethyl)-1,4,8-triazaspiro[4.5]decan-2-one,
(329) Ethyl [3-benzyl-8-(naphthalene-2-carbonyl)-2-oxo-1,4,8-triazaspiro[4.5]dec-1-yl]acetate,
(330) 8-[3-(2-Chloro-6-fluorophenyl)-5-methylisoxazole-4-carbonyl]-1-(2-fluorobenzyl)-1,4,8-triazaspiro[4.5]decan-2-one,
(331) 3-[8-(3,5-Dimethoxybenzoyl)-3-isopropyl-2-oxo-1,4,8-triazaspiro[4.5]dec-1-ylmethyl]-benzonitrile,
(332) 3-(2-Methylsulfanylethyl)-1-(2-nitrobenzyl)-8-(1-phenyl-5-propyl-1H-pyrazole-4-carbonyl)-1,4,8-triazaspiro[4.5]decan-2-one,
(333) Ethyl {3-benzyl-8-[3-(2-chloro-6-fluorophenyl)-5-methylisoxazole-4-carbonyl]-2-oxo-1,4,8-triazaspiro[4.5]dec-1-yl}acetate,
(334) 1-(2-Fluorobenzyl)-8-(3-fluoro-4-trifluoromethylbenzoyl)-1,4,8-triazaspiro[4.5]decan-2-one,
(335) Ethyl [8-[1-(4-chlorophenyl)-5-trifluoromethyl-1H-pyrazole-4-carbonyl]-3-(2-methylsulfanylethyl)-2-oxo-1,4,8-triazaspiro[4.5]dec-1-yl]acetate,
(336) 3-[8-(Naphthalene-1-carbonyl)-2-oxo-1,4,8-triazaspiro[4.5]dec-1-ylmethyl]-benzonitrile,
(337) Ethyl [8-(3,3-dimethylbutyryl)-3-(2-methylsulfanylethyl)-2-oxo-1,4,8-triazaspiro[4.5]dec-1-yl]acetate,
(338) 8-Acetyl-3-(2-methylsulfanylethyl)-1-naphthalen-2-ylmethyl-1,4,8-triazaspiro[4.5]decan-2-one,
(339) Ethyl [3-benzyl-8-(3-cyanobenzoyl)-2-oxo-1,4,8-triazaspiro[4.5]dec-1-yl]acetate,
(340) 8-(5-tert.-Butyl-2-methyl-2H-pyrazole-3-carbonyl)-1-(2,6-dichlorobenzyl)-3-isobutyl-1,4,8-triazaspiro[4.5]decan-2-one,
(341) 4-[3-Benzyl-1-(2-fluorobenzyl)-2-oxo-1,4,8-triazaspiro[4.5]decan-8-carbonyl]-benzonitrile,
(342) Methyl 4-[3-benzyl-1-(2-fluorobenzyl)-2-oxo-1,4,8-triazaspiro[4.5]dec-8-yl]-4-oxo-butyrate,
(343) 3-(2-Methylsulfanylethyl)-1-naphthalen-2-ylmethyl-8-(3,4,5-trimethoxybenzoyl)-1,4,8-triazaspiro[4.5]decan-2-one,
(344) Ethyl [3-benzyl-8-(isoxazole-5-carbonyl)-2-oxo-1,4,8-triazaspiro[4.5]dec-1-yl]acetate,
(345) 8-(3-Difluoromethylsulfanylbenzoyl)-1-(2-fluorobenzyl)-1,4,8-triazaspiro[4.5]decan-2-one,
(346) Ethyl [3-benzyl-8-(2,3-dimethylbenzoyl)-2-oxo-1,4,8-triazaspiro[4.5]dec-1-yl]acetate,
(347) 1-(2-Fluorobenzyl)-8-(4-propylbenzoyl)-1,4,8-triazaspiro[4.5]decan-2-one,
(348) 3-[8-(4-Iodobenzoyl)-2-oxo-1,4,8-triazaspiro[4.5]dec-1-ylmethyl]-benzonitrile,
(349) 1-(2-Fluorobenzyl)-8-(2-propylpentanoyl)-1,4,8-triazaspiro[4.5]decan-2-one,
(350) 3-[8-(2,6-Difluoro-3-methylbenzoyl)-3-isopropyl-2-oxo-1,4,8-triazaspiro[4.5]dec-1-ylmethyl]-benzonitrile,
(351) 3-(2-Methylsulfanylethyl)-8-(4-methyl[1,2,3]thiadiazole-5-carbonyl)-1-(2-nitrobenzyl)-1,4,8-triazaspiro[4.5]decan-2-one,
(352) Butyl 4-[3-isobutyl-8-(4-methyl[1,2,3]thiadiazole-5-carbonyl)-2-oxo-1,4,8-triazaspiro[4.5]dec-1-yl]acetate,
(353) 8-[2-(4-Chlorophenoxy)-acetyl]-3-(2-methylsulfanylethyl)-1-naphthalen-2-ylmethyl-1,4,8-triazaspiro[4.5]decan-2-one,
(354) Ethyl [3-benzyl-2-oxo-8-(3-trifluoromethylbenzoyl)-1,4,8-triazaspiro[4.5]dec-1-yl]acetate,
(355) 8-(4-Bromobenzoyl)-3-(2-methylsulfanylethyl)-1-naphthalen-2-ylmethyl-1,4,8-triazaspiro[4.5]decan-2-one,
(356) Ethyl [8-(2-chloropyridine-4-carbonyl)-3-(2-methylsulfanylethyl)-2-oxo-1,4,8-triazaspiro[4.5]dec-1-yl]acetate,
(357) 1-Allyl-8-(3,5-dimethoxybenzoyl)-3-(2-methylsulfanylethyl)-1,4,8-triazaspiro[4.5]decan-2-one,
(358) 3-[8-(4-Methyl-3-nitrobenzoyl)-2-oxo-1,4,8-triazaspiro[4.5]dec-1-ylmethyl]-benzonitrile,
(359) 8-(4-tert.-Butylbenzoyl)-1-(2-fluorobenzyl)-1,4,8-triazaspiro[4.5]decan-2-one,
(360) 1-Allyl-3-isopropyl-8-(5-methylisoxazole-3-carbonyl)-1,4,8-triazaspiro[4.5]decan-2-one,
(361) 1-Allyl-3-(2-methylsulfanylethyl)-8-(2-trifluoromethylbenzoyl)-1,4,8-triazaspiro[4.5]decan-2-one,
(362) Ethyl [3-benzyl-8-(3-cyclopentylpropionyl)-2-oxo-1,4,8-triazaspiro[4.5]dec-1-yl]acetate,
(363) 3-Benzyl-8-(3,5-difluorobenzoyl)-1-(2-fluorobenzyl)-1,4,8-triazaspiro[4.5]decan-2-one,
(364) Ethyl [3-benzyl-8-(5-fluoro-2-trifluoromethylbenzoyl)-2-oxo-1,4,8-triazaspiro[4.5]dec-1-yl]acetate,
(365) Ethyl [3-benzyl-2-oxo-8-(3,4,5-trimethoxybenzoyl)-1,4,8-triazaspiro[4.5]dec-1-yl]acetate,

(366) Benzyl 2-[1-(2-fluorobenzyl)-2-oxo-1,4,8-triazaspiro[4.5]decan-8-carbonyl]benzoate,
(367) 1-(2-Fluorobenzyl)-8-(2-methylbenzoyl)-1,4,8-triazaspiro[4.5]decan-2-one,
(368) 1-(2-Fluorobenzyl)-8-(2-phenylbutyryl)-1,4,8-triazaspiro[4.5]decan-2-one,
(369) 1-Allyl-8-(6-chloropyridine-3-carbonyl)-3-isopropyl-1,4,8-triazaspiro[4.5]decan-2-one,
(370) Ethyl [8-(2,5-dimethylfuran-3-carbonyl)-3-(2-methylsulfanylethyl)-2-oxo-1,4,8-triazaspiro[4.5]dec-1-yl]acetate,
(371) Ethyl [8-(5-tert.-butyl-2-methyl-2H-pyrazole-3-carbonyl)-3-(2-methylsulfanylethyl)-2-oxo-1,4,8-triazaspiro[4.5]dec-1-yl]acetate,
(372) Benzyl 2-[1-allyl-3-(2-methylsulfanylethyl)-2-oxo-1,4,8-triazaspiro[4.5]decan-8-carbonyl]benzoate,
(373) Ethyl 2-[1-allyl-3-(2-methylsulfanylethyl)-2-oxo-1,4,8-triazaspiro[4.5]dec-8-yl]-2-oxo-1-phenylacetate,
(374) 3-[8-(2-Chloro-6-fluorobenzoyl)-3-isopropyl-2-oxo-1,4,8-triazaspiro[4.5]dec-1-ylmethyl]-benzonitrile,
(375) 3-(2-Methylsulfanylethyl)-1-(2-nitrobenzyl)-8-(3-phenylpropionyl)-1,4,8-triazaspiro[4.5]decan-2-one,
(376) 3-[8-(2,3-Dimethylbenzoyl)-3-isopropyl-2-oxo-1,4,8-triazaspiro[4.5]dec-1-ylmethyl]-benzonitrile,
(377) Ethyl [8-(5-fluoro-2-trifluoromethylbenzoyl)-3-(2-methylsulfanylethyl)-2-oxo-1,4,8-triazaspiro[4.5]dec-1-yl]acetate,
(378) Ethyl [8-cyclopentanecarbonyl-3-(2-methylsulfanylethyl)-2-oxo-1,4,8-triazaspiro[4.5]dec-1-yl]acetate,
(379) Ethyl [3-(2-methylsulfanylethyl)-2-oxo-8-(thiophene-2-carbonyl)-1,4,8-triazaspiro[4.5]dec-1-yl]acetate,
(380) 1-Allyl-8-[1-(4-chlorophenyl)-5-trifluoromethyl-1H-pyrazole-4-carbonyl]-3-(2-methylsulfanylethyl)-1,4,8-triazaspiro[4.5]decan-2-one,
(381) 1-Allyl-3-(2-methylsulfanylethyl)-8-(naphthalene-1-carbonyl)-1,4,8-triazaspiro[4.5]decan-2-one,
(382) 1-(2-fluorobenzyl)-8-(4-trifluoromethoxybenzoyl)-1,4,8-triazaspiro[4.5]decan-2-one,
(383) 3-Benzyl-8-(2-benzyloxyacetyl)-1-(2-fluorobenzyl)-1,4,8-triazaspiro[4.5]decan-2-one,
(384) 3-[8-(2-chloro-6-fluoro-3-methylbenzoyl)-3-isopropyl-2-oxo-1,4,8-triazaspiro[4.5]dec-1-ylmethyl]-benzonitrile,
(385) 1-Allyl-8-(2-chloro-5-trifluoromethylbenzoyl)-3-isopropyl-1,4,8-triazaspiro[4.5]decan-2-one,
(386) 8-(3-Methylbenzoyl)-3-(2-methylsulfanylethyl)-1-naphthalen-2-ylmethyl-1,4,8-triazaspiro[4.5]decan-2-one,
(387) 1-(3,5-Dimethylbenzyl)-8-(2-ethylbutyryl)-3-isobutyl-1,4,8-triazaspiro[4.5]decan-2-one,
(388) 8-(5-tert.-Butyl-2-methyl-2H-pyrazole-3-carbonyl)-3-(2-methylsulfanylethyl)-1-(2-nitrobenzyl)-1,4,8-triazaspiro[4.5]decan-2-one,
(389) Benzyl 2-[1-(3-cyanobenzyl)-3-isopropyl-2-oxo-1,4,8-triazaspiro[4.5]decan-8-carbonyl]benzoate,
(390) 8-(4-Methyl-3-nitrobenzoyl)-3-(2-methylsulfanylethyl)-1-(2-nitrobenzyl)-1,4,8-triazaspiro[4.5]decan-2-one,
(391) 1-(2,6-Dichlorobenzyl)-3-isobutyl-8-(3,4,5-trimethoxybenzoyl)-1,4,8-triazaspiro[4.5]decan-2-one,
(392) 3-{8-[2-(2-Bromophenyl)-acetyl]-3-isopropyl-2-oxo-1,4,8-triazaspiro[4.5]dec-1-ylmethyl}-benzonitrile,
(393) 3-[8-(2,3-Dichlorobenzoyl)-3-isopropyl-2-oxo-1,4,8-triazaspiro[4.5]dec-1-ylmethyl]-benzonitrile,
(394) 1-Allyl-8-(6-chloro-2H-chromane-3-carbonyl)-3-isopropyl-1,4,8-triazaspiro[4.5]decan-2-one,
(395) Ethyl {3-benzyl-8-[2-(4-methoxyphenyl)-acetyl]-2-oxo-1,4,8-triazaspiro[4.5]dec-1-yl}acetate,
(396) 3-[3-Isopropyl-2-oxo-8-(1-phenyl-5-propyl-1H-pyrazole-4-carbonyl)-1,4,8-triazaspiro[4.5]dec-1-ylmethyl]-benzonitrile,
(397) 8-(3-Cyclopentylpropionyl)-1-(2-fluorobenzyl)-1,4,8-triazaspiro[4.5]decan-2-one,
(398) 1-Allyl-8-(3-difluoromethylsulfanylbenzoyl)-3-isopropyl-1,4,8-triazaspiro[4.5]decan-2-one,
(399) 3-[8-(2-chloro-4-nitrobenzoyl)-3-isopropyl-2-oxo-1,4,8-triazaspiro[4.5]dec-1-ylmethyl]-benzonitrile,
(400) Ethyl [8-(2-ethoxybenzoyl)-3-(2-methylsulfanylethyl)-2-oxo-1,4,8-triazaspiro[4.5]dec-1-yl]acetate,
(401) 8-(2,5-Bis-trifluoromethylbenzoyl)-1-(2-fluorobenzyl)-1,4,8-triazaspiro[4.5]decan-2-one,
(402) 8-(2-Chloropyridine-4-carbonyl)-3-(2-methylsulfanylethyl)-1-(2-nitrobenzyl)-1,4,8-triazaspiro[4.5]decan-2-one,
(403) Ethyl {3-benzyl-8-[3-(2,6-dichlorophenyl)-5-methylisoxazole-4-carbonyl]-2-oxo-1,4,8-triazaspiro[4.5]dec-1-yl}acetate,
(404) 1-(3,5-Dimethylbenzyl)-3-isobutyl-8-(2-trifluoromethylbenzoyl)-1,4,8-triazaspiro[4.5]decan-2-one,
(405) 8-(3,5-Dimethoxybenzoyl)-3-(2-methylsulfanylethyl)-1-(2-nitrobenzyl)-1,4,8-triazaspiro[4.5]decan-2-one,
(406) Benzyl 2-[3-benzyl-1-(2-fluorobenzyl)-2-oxo-1,4,8-triazaspiro[4.5]decan-8-carbonyl]benzoate,
(407) 3-[8-(Benzo[1,2,5]oxadiazole-5-carbonyl)-3-isopropyl-2-oxo-1,4,8-triazaspiro[4.5]dec-1-ylmethyl]-benzonitrile,
(408) Ethyl [3-(2-methylsulfanylethyl)-2-oxo-8-(4-trifluoromethoxybenzoyl)-1,4,8-triazaspiro[4.5]dec-1-yl]acetate,
(409) 1-Allyl-3-isopropyl-8-[2-(4-methoxyphenyl)-acetyl]-1,4,8-triazaspiro[4.5]decan-2-one,
(410) 3-(2-Methylsulfanylethyl)-1-(2-nitrobenzyl)-8-(4-propylbenzoyl)-1,4,8-triazaspiro[4.5]decan-2-one,

(411) 3-Benzyl-8-(4-tert.-butylbenzoyl)-1-(2-fluorobenzyl)-1,4,8-triazaspiro[4.5]decan-2-one,
(412) 1-Allyl-8-(2,6-difluoro-3-methylbenzoyl)-3-(2-methylsulfanylethyl)-1,4,8-triazaspiro[4.5]decan-2-one,
(413) 8-(3,5-Bis-trifluoromethylbenzoyl)-1-(2-fluorobenzyl)-1,4,8-triazaspiro[4.5]decan-2-one,
(414) 3-[8-(4-Iodobenzoyl)-3-isopropyl-2-oxo-1,4,8-triazaspiro[4.5]dec-1-ylmethyl]-benzonitrile,
(415) 3-(2-Methylsulfanylethyl)-1-naphthalen-2-ylmethyl-8-(thiophene-2-carbonyl)-1,4,8-triazaspiro[4.5]decan-2-one,
(416) Ethyl [3-benzyl-8-(2-chloropyridine-4-carbonyl)-2-oxo-1,4,8-triazaspiro[4.5]dec-1-yl]acetate,
(417) Ethyl [3-(2-methylsulfanylethyl)-8-(4-nitrobenzoyl)-2-oxo-1,4,8-triazaspiro[4.5]dec-1-yl]acetate,
(418) 8-(2-Ethylhexanoyl)-3-(2-methylsulfanylethyl)-1-(2-nitrobenzyl)-1,4,8-triazaspiro[4.5]decan-2-one,
(419) 1-(2,6-Dichlorobenzyl)-3-isobutyl-8-(thiophene-2-carbonyl)-1,4,8-triazaspiro[4.5]decan-2-one,
(420) Ethyl [3-benzyl-8-(2-chloro-4-nitrobenzoyl)-2-oxo-1,4,8-triazaspiro[4.5]dec-1-yl]acetate,
(421) 3-Benzyl-8-(3,5-bis-trifluoromethylbenzoyl)-1-(2-fluorobenzyl)-1,4,8-triazaspiro[4.5]decan-2-one,
(422) Ethyl [3-benzyl-8-(4-bromo-3-methylbenzoyl)-2-oxo-1,4,8-triazaspiro[4.5]dec-1-yl]acetate,
(423) 8-Cyclohexanecarbonyl-3-(2-methylsulfanylethyl)-1-(2-nitrobenzyl)-1,4,8-triazaspiro[4.5]decan-2-one,
(424) 8-(2,5-Dimethylfuran-3-carbonyl)-3-(2-methylsulfanylethyl)-1-(2-nitrobenzyl)-1,4,8-triazaspiro[4.5]decan-2-one,
(425) 8-(3-Difluoromethylsulfanylbenzoyl)-3-(2-methylsulfanylethyl)-1-(2-nitrobenzyl)-1,4,8-triazaspiro[4.5]decan-2-one,
(426) 8-(Furan-2-carbonyl)-3-(2-methylsulfanylethyl)-1-(2-nitrobenzyl)-1,4,8-triazaspiro[4.5]decan-2-one,
(427) Ethyl [3-benzyl-8-(2,3-difluorobenzoyl)-2-oxo-1,4,8-triazaspiro[4.5]dec-1-yl]acetate,
(428) 3-[3-Isopropyl-8-(4-methyl[1,2,3]thiadiazole-5-carbonyl)-2-oxo-1,4,8-triazaspiro[4.5]dec-1-ylmethyl]-benzonitrile,
(429) 3-[8-(3-Chloro-2-fluorobenzoyl)-3-isopropyl-2-oxo-1,4,8-triazaspiro[4.5]dec-1-ylmethyl]-benzonitrile,
(430) Ethyl [3-benzyl-8-(naphthalene-1-carbonyl)-2-oxo-1,4,8-triazaspiro[4.5]dec-1-yl]acetate,
(431) 8-(4-tert.-Butylbenzoyl)-3-(2-methylsulfanylethyl)-1-(2-nitrobenzyl)-1,4,8-triazaspiro[4.5]decan-2-one,
(432) 3-[3-Benzyl-1-(2-fluorobenzyl)-2-oxo-1,4,8-triazaspiro[4.5]decan-8-carbonyl]-benzonitrile,
(433) 1-(2,6-Dichlorobenzyl)-8-(furan-2-carbonyl)-3-isobutyl-1,4,8-triazaspiro[4.5]decan-2-one,
(434) 8-(2,6-Dichlorobenzoyl)-1-(2-fluorobenzyl)-1,4,8-triazaspiro[4.5]decan-2-one,
(435) 1-(3,5-Dimethylbenzyl)-8-(3-fluoro-4-trifluoromethylbenzoyl)-3-isobutyl-1,4,8-triazaspiro[4.5]decan-2-one,
(436) 1-(2-Fluorobenzyl)-8-(3-fluoro-4-methylbenzoyl)-1,4,8-triazaspiro[4.5]decan-2-one,
(437) 1-Allyl-8-(3-cyclopentylpropionyl)-3-(2-methylsulfanylethyl)-1,4,8-triazaspiro[4.5]decan-2-one,
(438) 8-[2-(3-Chlorophenoxy)-acetyl]-3-(2-methylsulfanylethyl)-1-(2-nitrobenzyl)-1,4,8-triazaspiro[4.5]decan-2-one,
(439) 1-(3,5-Dimethylbenzyl)-3-isobutyl-8-(2-methylsulfanylpyridine-3-carbonyl)-1,4,8-triazaspiro[4.5]decan-2-one,
(440) 3-(2-Methylsulfanylethyl)-1-(2-nitrobenzyl)-8-propionyl-1,4,8-triazaspiro[4.5]decan-2-one,
(441) 3-[8-(3,4-Dimethoxybenzoyl)-3-isopropyl-2-oxo-1,4,8-triazaspiro[4.5]dec-1-ylmethyl]-benzonitrile,
(442) 3-(2-Methylsulfanylethyl)-8-(naphthalene-2-carbonyl)-1-naphthalen-2-ylmethyl-1,4,8-triazaspiro[4.5]decan-2-one,
(443) 8-(6-Chloro-2H-chromane-3-carbonyl)-3-(2-methylsulfanylethyl)-1-(2-nitrobenzyl)-1,4,8-triazaspiro[4.5]decan-2-one,
(444) Ethyl {3-benzyl-2-oxo-8-[3-(3-trifluoromethylphenyl)-acryloyl]-1,4,8-triazaspiro[4.5]dec-1-yl}acetate,
(445) 1-(3,5-Dimethylbenzyl)-3-isobutyl-8-(3-phenylacryloyl)-1,4,8-triazaspiro[4.5]decan-2-one,
(446) 3-[3-Isopropyl-2-oxo-8-(2-phenoxypropionyl)-1,4,8-triazaspiro[4.5]dec-1-ylmethyl]-benzonitrile,
(447) 3-[3-Isopropyl-2-oxo-8-(4-trifluoromethoxybenzoyl)-1,4,8-triazaspiro[4.5]dec-1-ylmethyl]-benzonitrile,
(448) 1-Allyl-3-(2-methylsulfanylethyl)-8-(3-nitrobenzoyl)-1,4,8-triazaspiro[4.5]decan-2-one,
(449) Ethyl [3-benzyl-8-(3,4-dichlorobenzoyl)-2-oxo-1,4,8-triazaspiro[4.5]dec-1-yl]acetate,
(450) 1-(2-Fluorobenzyl)-8-[3-(3-trifluoromethylphenyl)-acryloyl]-1,4,8-triazaspiro[4.5]decan-2-one,
(451) 1-Allyl-8-(3,5-bis-trifluoromethylbenzoyl)-3-(2-methylsulfanylethyl)-1,4,8-triazaspiro[4.5]decan-2-one,
(452) 8-(3-Cyclopentylpropionyl)-3-(2-methylsulfanylethyl)-1-(2-nitrobenzyl)-1,4,8-triazaspiro[4.5]decan-2-one,
(453) 1-(3,5-Dimethylbenzyl)-3-isobutyl-8-(4-nitrobenzoyl)-1,4,8-triazaspiro[4.5]decan-2-one,
(454) 8-(3-Cyclopentylpropionyl)-1-(3,5-dimethylbenzyl)-3-isobutyl-1,4,8-triazaspiro[4.5]decan-2-one,
(455) 3-[3-Isopropyl-8-(isoxazole-5-carbonyl)-2-oxo-1,4,8-triazaspiro[4.5]dec-1-ylmethyl]-benzonitrile und
(456) 3-Benzyl-1-(2-fluorobenzyl)-8-(2-methyl-6-trifluoromethylpyridine-3-carbonyl)-1,4,8-triazaspiro[4.5]decan-2-one,

in each case optionally in the form of one of the pure stereoisomers thereof, particularly enantiomers or diastereoisomers thereof, the racemates thereof or in the form of a mixture of stereoisomers thereof, particularly the enantiomers and/or diastereoisomers, in any desired mixing ratio, or in each case in the form of corresponding salts, or in each case in the form of corresponding solvates.

**14.** A process for the production of the substituted 1,4,8-triazaspiro[4.5]decan-2-one compounds according to one or more of claims 1 to 13, **characterized in that** a protected piperidin-4-one of the general formula II,

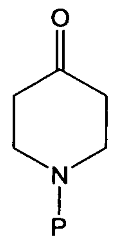

II

in which P stands for a protective group, is converted, by reaction with at least one compound of the general formula III,

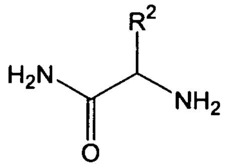

III

in which $R^2$ has the meaning according to one or more of claims 1 to 13, into at least one compound of the general formula IV,

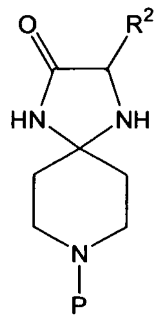

IV

in which P and $R^2$ have the aforementioned meanings, which is optionally purified and/or optionally isolated, and optionally converted, by reaction with at least one compound of the general formula $R^1$-$X^1$, in which $R^1$ has the meaning according to one or more of claims 1 to 13 and $X^1$ stands for a suitable leaving group, preferably for a halogen residue, optionally in the presence of at least one base, into at least one compound of the general formula V,

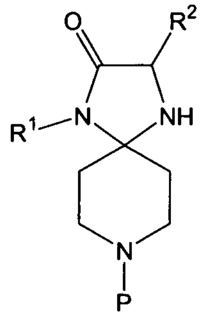

V

in which $R^1$, $R^2$ and P have the aforementioned meanings, which compound is optionally purified and/or optionally isolated, and optionally converted, by splitting-off the protective group P, into at least one compound of the general formula VI,

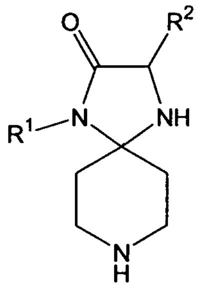

VI

which compound is optionally purified and/or optionally isolated, and optionally at least one compound of the general formula IV, V or VI is converted, by reaction with a carboxylic acid derivative of the general formula $R^4$-(C=O)-$X^2$, a carboxylic anhydride of the general formula ($R^4$-(C=O)$)_2$O or a compound of the general formula $R^3$-$X^3$, in which the residue $R^3$ has the meaning according to one of claims 1 to 13 with the exception of hydrogen and -(C=O)-$R^4$, $R^4$ in each case has the meaning according to one or more of claims 1 to 13 and $X^2$ and $X^3$ in each case stand for a suitable leaving group, preferably for a halogen residue, into at least one compound according to one or more of claims 1 to 13, and the latter compound is optionally purified and/or optionally isolated,
or
4-piperidone of the formula VII, optionally in the form of a corresponding salt,

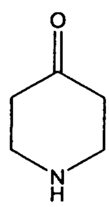

VII

is converted, by reaction with a carboxylic acid derivative of the general formula $R^4$-(C=O)-$X^2$, a carboxylic anhydride of the general formula ($R^4$-(C=O)$)_2$O or a compound of the general formula $R^3$-$X^3$,
in which $R^3$ has the meaning according to one or more of claims 1 to 13 with the exception of hydrogen and (C=O)-$R^4$, the residue $R^4$ in each case has the aforementioned meaning and $X^2$ and $X^3$ each stand for a suitable leaving group, preferably for a halogen residue, into a compound of the general formula VIII,

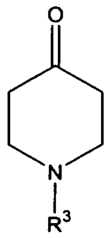

VIII

in which $R^3$ has the meaning according to one or more of claims 1 to 13, this being optionally purified and/or isolated and converted, by reaction with at least one compound of the general formula III

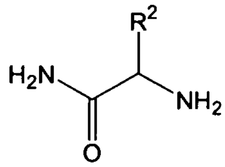

III

in which $R^2$ has the aforementioned meaning, into at least one compound of the general formula IX,

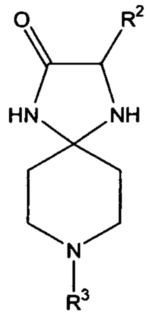

IX

in which $R^2$ and $R^3$ each have the aforementioned meanings, this being optionally purified and/or isolated and optionally converted, by reaction with at least one compound of the general formula $R^1$-$X^1$
in which $R^1$ and $X^1$ have the aforementioned meanings, optionally in the presence of at least one base, into at least one compound according to one or more of claims 1 to 13, which is optionally purified and/or isolated.

**15.** A medicament containing at least one substituted 1,4,8-triazaspiro[4.5]decan-2-one compound according to one or more of claims 1 to 13 and optionally one or more pharmaceutically acceptable adjuvants.

**16.** A medicament according to claim 15 for regulation, particularly inhibition, of noradrenalin reuptake (noradrenalin-uptake), for regulation, particularly inhibition, of 5-hydroxy tryptophan reuptake (5-HT uptake), for opioid receptor regulation, particularly for μ-opioid receptor regulation, and/or for batrachotoxin (BTX) receptor regulation.

**17.** A medicament according to claim 15 or claim 16 for prophylaxis and/or treatment of disturbances in food intake, preferably selected from the group consisting of bulimia, anorexia, obesity, and cachexia, preferably obesity.

**18.** A medicament according to claim 15 or claim 16 for prophylaxis and/or treatment of pain, preferably for treatment

and prophylaxis of acute pain, chronic pain, neuropathic pain, and/or cluster headache.

**19.** A medicament according to claim 15 or claim 16 for prophylaxis and/or treatment of depression.

**20.** A medicament according to claim 15 or claim 16 for combined prophylaxis and/or treatment of depression and pain, preferably for combined treatment of depression and pain selected from the group consisting of acute pain, chronic pain, neuropathic pain and cluster headache.

**21.** A medicament according to claim 15 or claim 16 for prophylaxis and/or treatment of abuse of alcohol and/or drugs and/or medicines, for prophylaxis and/or treatment of addiction to alcohol and/or drugs and/or medicines, for prophylaxis and/or treatment of inflammations, for prophylaxis and/or treatment of lethargy, for prophylaxis and/or treatment of ischemia, for prophylaxis and/or treatment of catalepsy, for vigilance enhancement, for libido enhancement, for anxiolysis, for prophylaxis and/or treatment of neurodegenerative disorders, preferably one or more neurodegenerative disorders selected from the group consisting of Parkinson's disease, Huntington's chorea, Alzheimer's disease and multiple sclerosis, and/or for local anaesthesia.

**22.** The use of at least one substituted 1,4,8-triazaspiro[4.5]decan-2-one compound according to any one of claims 1 to 13 including the excluded compounds for the production of a medicament for regulation of noradrenalin reuptake (noradrenalin uptake), preferably for inhibition of noradrenalin reuptake (noradrenalin uptake).

**23.** The use of at least one substituted 1,4,8-triazaspiro[4.5]decan-2-one compound according to any one of claims 1 to 13 including the excluded compounds for the production of a medicament for regulation of 5-hydroxy tryptophan reuptake (5-HT uptake), preferably for inhibition of 5-hydroxy tryptophan reuptake (5-HT uptake).

**24.** The use at least one substituted 1,4,8-triazaspiro[4.5]decan-2-one compound according to any one of claims 1 to 13 including the excluded compounds for the production of a medicament for opioid receptor regulation, preferably for $\mu$-opioid receptor regulation.

**25.** The use of at least one substituted 1,4,8-triazaspiro[4.5]decan-2-one compound according to any one of claims 1 to 13 including the excluded compounds for the production of a medicament for batrachotoxin (BTX) receptor regulation.

**26.** The use of at least one substituted 1,4,8-triazaspiro[4.5]decan-2-one compound of the general formula I according to any one of claims 1 to 13 including the excluded compounds for the production of a medicament for prophylaxis and/or treatment of disturbances in food intake, preferably selected from the group consisting of bulimia, anorexia, obesity, and cachexia, particularly preferably obesity.

**27.** The use of at least one substituted 1,4,8-triazaspiro[4.5]decan-2-one compound of the general formula I according to any one of claims 1 to 13 including the excluded compounds listed under B) but excluding the compounds listed under A) for the production of a medicament for prophylaxis and/or treatment of pain.

**28.** The use of at least one substituted 1,4,8-triazaspiro[4.5]decan-2-one compound according to any one of claims 1 to 13 including the excluded compounds for the production of a medicament for prophylaxis and/or treatment of acute pain, chronic pain, neuropathic pain and/or cluster headache.

**29.** The use of at least one substituted 1,4,8-triazaspiro[4.5]decan-2-one compound according to any one of claims 1 to 13 including the excluded compounds listed under B) but excluding the compounds listed under A) for the production of a medicament for prophylaxis and/or treatment of depression.

**30.** The use of at least one substituted 1,4,8-triazaspiro[4.5]decan-2-one compound according to any one of claims 1 to 13 including the excluded compounds for prophylaxis and/or treatment of the abuse of drugs and medicines, for prophylaxis and/or treatment of addiction to drugs and/or medicines, for prophylaxis and/or treatment of multiple sclerosis, for prophylaxis and/or treatment of lethargy, for prophylaxis and/or treatment of catalepsy, for vigilance enhancement, for libido enhancement, for anxiolysis, for prophylaxis and/or treatment of ischemia and/or for local anaesthesia.

**31.** The use of at least one substituted 1,4,8-triazaspiro[4.5]decan-2-one compound according to any one of claims 1 to 13 including the excluded compounds listed under B) but excluding the compounds listed under A) for the production

of a medicament for prophylaxis and/or treatment of alcohol abuse, for prophylaxis and/or treatment of alcohol addiction and/or for prophylaxis and/or treatment of inflammations.

**32.** The use of at least one substituted 1,4,8-triazaspiro[4.5]decan-2-one compound according to any one of claims 1 to 13 for the production of a medicament for prophylaxis and/or treatment of neurodegenerative disorders, preferably one or more disorders selected from the group consisting of Huntington's chorea, Alzheimer's disease and Parkinson's disease.

## Revendications

**1.** Composés substitués de 1,4,8-triazaspiro[4,5]décan-2-one de formule générale I,

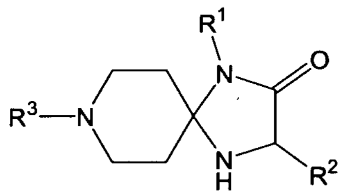

où

$R^1$ représente un radical hydrogène, un radical alkyle linéaire ou ramifié, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui est lié via un groupe alkylène linéaire ou ramifié, présentant le cas échéant au moins un hétéroatome comme élément de chaîne, un radical -C(=O)-$OR^5$ lié via un groupe alkylène linéaire ou ramifié ou un radical -O-(C=O)-$R^6$ lié via un groupe alkylène linéaire ou ramifié,
$R^2$ représente un radical hydrogène, un radical alkyle linéaire ou ramifié, non substitué ou au moins monosubstitué, présentant le cas échéant au moins $R^3$ un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui peut être lié via un groupe alkylène linéaire ou ramifié, présentant le cas échéant au moins un hétéroatome comme élément de chaîne, représente un radical hydrogène, un radical alkyle linéaire ou ramifié, non substitué ou au moins monosubstitué, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément de chaîne, un radical aryle ou hétéroaryle non substitué ou au moins monosubstitué, qui est lié via un groupe alkylène linéaire ou ramifié, non substitué ou au moins monosubstitué, présentant le cas échéant au moins un hétéroatome comme élément de chaîne et le cas échéant condensé avec un système monocyclique non substitué ou au moins monosubstitué, un radical cycloaliphatique non substitué ou au moins monosubstitué, présentant le cas échéant au moins un hétéroatome comme élément de cycle, le cas échéant lié via un groupe alkylène linéaire ou ramifié, non substitué ou au moins monosubstitué, présentant le cas échéant au moins un hétéroatome comme élément de chaîne, qui peut être ponté avec un groupe alkylène linéaire ou ramifié, non substitué ou au moins monosubstitué, présentant le cas échéant au moins $R^4$ un hétéroatome comme élément de cycle et/ou qui peut être condensé avec un système monocyclique non substitué ou au moins monosubstitué, ou un groupe -C(=O)-$R^4$, représente un radical alkyle linéaire ou ramifié, non substitué ou au moins monosubstitué, présentant le cas échéant au moins un hétéroatome comme élément de chaîne, un radical alcényle linéaire ou ramifié, non substitué ou au moins monosubstitué, présentant le cas échéant au moins un hétéroatome comme élément de chaîne, un radical alcynyle linéaire ou ramifié, non substitué ou au moins monosubstitué, présentant le cas échéant au moins un hétéroatome comme élément

de chaîne,

représente un radical aryle ou hétéroaryle non substitué ou au moins monosubstitué, qui peut être lié via un groupe alkylène, alcénylène ou alcynylène linéaire ou ramifié, non substitué ou au moins monosubstitué, présentant le cas échéant au moins un hétéroatome comme élément de chaîne et/ou condensé avec un système monocyclique non substitué ou au moins monosubstitué, représente un radical cycloaliphatique non substitué ou au moins monosubstitué, présentant le cas échéant au moins un hétéroatome comme élément de cycle, le cas échéant lié via un groupe alkylène linéaire ou ramifié, non substitué ou au moins monosubstitué, présentant le cas échéant au moins un hétéroatome comme élément de chaîne, qui peut être au moins ponté une fois par un groupe alkylène linéaire ou ramifié, non substitué ou au moins monosubstitué et/ou condensé avec un système monocyclique non substitué ou au moins monosubstitué,

représente un radical -C(=O)-$OR^7$ lié via un groupe alkylène linéaire ou ramifié, non substitué ou au moins monosubstitué, un radical -O-(C=O)-$R^8$ lié via un groupe alkylène linéaire ou ramifié, non substitué ou au moins monosubstitué ou un radical- (C=O)-$R^9$ lié via un groupe alkylène linéaire ou ramifié, non substitué ou au moins monosubstitué,

$R^5$ et $R^6$ représentent indépendamment l'un de l'autre à chaque fois un radical alkyle linéaire ou ramifié, un radical alcényle linéaire ou ramifié ou un radical alcynyle linéaire ou ramifié,

$R^7$ et $R^8$ représentent indépendamment l'un de l'autre à chaque fois un radical hydrogène, un radical alkyle linéaire ou ramifié, un radical alcényle linéaire ou ramifié, un radical alcynyle linéaire ou ramifié ou un radical aryle ou hétéroaryle non substitué ou au moins monosubstitué,

$R^9$ représente un radical alkyle linéaire ou ramifié, un radical alcényle linéaire ou ramifié ou un radical alcynyle linéaire ou ramifié,

le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants,

où "au moins monosubstitué" associé à un radical alkyle, alcényle ou alcynyle signifie un radical alkyle, alcényle ou alcynyle monosubstitué ou polysubstitué, les substituants étant à chaque fois choisis indépendamment l'un de l'autre dans le groupe constitué par F, Cl, Br, I, $C_{1-6}$-alcoxy, -$NO_2$, -OH, -SH, -(C=O)-OH, -(C=O)-O-$C_{1-4}$-alkyle, -O-(C=O)-$C_{1-4}$-alkyle, -$CH_2$-O-$CH_2$-phényle, -CN, -$CF_3$, -$CHF_2$, -$CH_2F$, phényle non substitué et -$NR^aR^b$, où $R^a$ et $R^b$ peuvent être choisis à chaque fois indépendamment l'un de l'autre, dans le groupe constitué par H, méthyle, éthyle, n-propyle, iso-propyle, n-butyle, sec-butyle, tert-butyle, -(C=O)-O-$C_{1-4}$-alkyle et phényle non substitué ;

où "au moins monosubstitué" en association avec un radical cycloaliphatique signifie un radical cycloaliphatique monosubstitué ou polysubstitué, les substituants étant choisis à chaque fois indépendamment l'un de l'autre dans le groupe constitué par F, Cl, Br, $C_{1-6}$-alcoxy, oxo, $C_{1-6}$-alkyle, hydroxy, -CN, -$CF_3$, -$CHF_2$, $CH_2F$, phényle non substitué, -$NR^aR^b$, où $R^a$ et $R^b$ peuvent à chaque fois être choisis indépendamment l'un de l'autre dans le groupe constitué par H, méthyle, éthyle, n-propyle, iso-propyle et phényle non substitué, thioxo (=S), I, -$SF_5$, -$NO_2$, -O-$CF_3$, -S-$CF_3$, -SH, -S-$C_{1-5}$-alkyle, -C(=O)-H, -C(=O)-$C_{1-5}$-alkyle, -C(=O)-OH, -C(=O)-O-$C_{1-5}$-alkyle, -($CH_2$)-C(=O)-OH, -($CH_2$)-C(=O)-O-$C_{1-5}$-alkyle, -($CH_2$)-benzo[b]furannyle, -O-phényle, -O-benzyle, phényle, benzyle, naphtyle et -($CH_2$)-naphtyle, la partie cyclique des radicaux -O-phényle, -O-benzyle, phényle, -($CH_2$)-benzo[b]furannyle, benzyle, naphtyle et -($CH_2$)-naphtyle pouvant être chaque fois être substituée par 1, 2, 3, 4 ou 5 substituants choisis indépendamment l'un de l'autre dans le groupe constitué par F, Cl, Br, -OH, -$CF_3$, -$SF_5$, -CN, -$NO_2$, -O-$C_{1-5}$-alkyle, -$C_{1-5}$-alkyle, -O-$CF_3$, -S-$CF_3$, phényle et -O-benzyle ; où "au moins monosubstitué" en association avec un radical aryle ou hétéroaryle signifie un radical aryle ou hétéroaryle monosubstitué ou polysubstitué, les substituants étant choisis à chaque fois indépendamment l'un de l'autre de préférence dans le groupe constitué par F, Cl, Br, I, -CN, -$NO_2$, $C_{1-5}$-alkyle, OH, SH, $C_{1-5}$-alcoxy, $C_{1-5}$-perfluoroalcoxy, -C(=O)-$C_{1-5}$-alkyle, -C(=O)-O-$C_{1-5}$-alkyle, -$(CH_2)_{1-3}$-O-(C=O)-phényle, -S-$C_{1-5}$-alkyle, -S$(=O)_2$-$C_{1-6}$-alkyle, -S$(=O)_2$-$NH_2$, -NH-(C=O)-$CH_3$, -S-$CHF_2$, -S-$CH_2F$, -C(=O)-$C_{1-5}$-perfluoroalkyle, -$CF_3$, $CHF_2$, $CH_2F$, phényle, phénoxy et -$NR^aR^b$, où $R^a$ et $R^b$ peuvent à chaque fois être choisis indépendamment l'un de l'autre dans le groupe constitué par H, méthyle, éthyle, n-propyle, iso-propyle et phényle non substitué, -$SF_5$, -O-$C_{2-5}$-alcényle, -O-$CF_3$, -O-$CHF_2$, -O-$CH_2F$, -C(=O)-OH, -O-C(=O)-$C_{1-5}$-alkyle, -O-C(=O)-phényle, -($CH_2$)-O-C(=O)-$C_{1-5}$-alkyle, -NH-$C_{1-5}$-alkyle, -N$(C_{1-5}\text{-alkyle})_2$, -NH-C(=O)-O-$C_{1-5}$-alkyle, -C(=O)-H, -C(=O)-$C_{1-5}$-alkyle, -C(=O)-$NH_2$, -C(=O)-NH-$C_{1-5}$-alkyle, C(=O)-N-$(C_{1-5}\text{-alkyle})_2$, -S$(=O)_2$-NH-$C_{1-5}$-alkyle, -S$(=O)_2$-NH-phényle, -($CH_2$)-benzo[b]furannyle, -O-phényle, -S-phényle, -S-benzyle, -O-benzyle, phényle et benzyle, où la partie cyclique des radicaux -S$(=O)_2$-NH-phényle, -O-phényle, -S-phényle, -S-benzyle, -O-benzyle, phényle, -($CH_2$)-benzo[b]furannyle et benzyle peut être substituée par 1, 2, 3, 4 ou 5 substituants choisis indépendamment l'un de l'autre dans le groupe constitué par F, Cl, Br, -OH, -$CF_3$, -$SF_5$, -CN, -$NO_2$, -O-$C_{1-5}$-alkyle, -$C_{1-5}$-alkyle, -O-$CF_3$, -S-$CF_3$, phényle et -O-benzyle et où les substituants phényle et phénoxy eux-mêmes peuvent être

monosubstitués ou polysubstitués, de manière identique ou différente par un substituant choisi dans le groupe constitué par F, Cl, Br, -S-$CHF_2$, -$CF_3$, -$CHF_2$, -$CH_2F$, -CN, -$NO_2$, $C_{1-5}$-alkyle et $C_{1-5}$-alcoxy ;
où "au moins monosubstitué" en association avec un système monocyclique signifie un système monocyclique monosubstitué ou polysubstitué, les substituants étant choisis à chaque fois indépendamment l'un de l'autre de préférence dans le groupe constitué par F, Cl, Br, I, -CN, -$NO_2$, oxo, $C_{1-5}$-alkyle, $C_{1-5}$-alcoxy, $C_{1-5}$-perfluoroalcoxy, -C(=O)$C_{1-5}$-alkyle, -C(=O)-O-$C_{1-5}$-alkyle, -S(=O)$_2$-$C_{1-6}$-alkyle, -C(=O)-$C_{1-5}$-perfluoroalkyle, -$CF_3$, $CHF_2$, $CH_2F$ et -$NR^aR^b$, où $R^a$ et $R^b$ peuvent à chaque fois être choisis indépendamment l'un de l'autre dans le groupe constitué par H, méthyle, éthyle, n-propyle, iso-propyle et phényle non substitué, thioxo (=S), -$SF_5$, -OH, -O-$C_{2-5}$-alcényle, -$NH_2$, -$NO_2$, -O-$CF_3$, -O-$CHF_2$, -O-$CH_2F$, -S-$CF_3$, -S-$CHF_2$, -S-$CH_2F$,- SH, -S-$C_{1-5}$-alkyle, -C(=O)-OH, -O-C(=O)-$C_{1-5}$-alkyle, -O-C(=O)-phényle, -($CH_2$)-O-C(=O)-$C_{1-5}$-alkyle, -($CH_2$)-O-C(=O)-phényle, -NH-$C_{1-5}$-alkyle, -N($C_{1-5}$-alkyle)$_2$, -NH-C(=O)-O-$C_{1-5}$-alkyle, -NH-C(=O)-$C_{1-5}$-alkyle, -C(=O)-H, -C(=O)-$NH_2$, -C (=O)-NH-$C_{1-5}$-alkyle, C(=O)-N-($C_{1-5}$-alkyle)$_2$, -S(=O)$_2$-$NH_2$, -S(=O)$_2$-NH-$C_{1-5}$-alkyle, -S(=O)$_2$-NH-phényle, -($CH_2$)-benzo[b]furannyle, -O-phényle, -S-phényle, -S-benzyle, -O-benzyle, phényle et benzyle, la partie cyclique des radicaux -O-C(=O)-phényle, -($CH_2$)-O-C(=O)-phényle, -S(=O)$_2$-NH-phényle, -O-phényle, -S-phényle, -S-benzyle, -O-benzyle, phényle, -($CH_2$)-benzo[b]furannyle et benzyle pouvant être substituée par 1, 2, 3, 4 ou 5 substituants choisis indépendamment l'un de l'autre dans le groupe constitué par F, Cl, Br, -OH, -$CF_3$, -$SF_5$, -CN, -$NO_2$, -O-$C_{1-5}$-alkyle, -$C_{1-5}$-alkyle, -O-$CF_3$, -S-$CF_3$, phényle et -0-benzyle ;
où "au moins monosubstitué" en association avec un groupe alkylène, alcénylène ou alcynylène linéaire ou ramifié signifie un groupe alkylène, alcénylène ou alcynylène monosubstitué ou polysubstitué linéaire ou ramifié, les substituants étant choisis à chaque fois indépendamment l'un de l'autre de préférence dans le groupe constitué par F, Cl, Br, $C_{1-6}$-alcoxy, hydroxy, CN, $CF_3$, $CHF_2$, $CH_2F$, phényle non substitué et -$NR^aR^b$, où $R^a$ et $R^b$ peuvent à chaque fois être choisis indépendamment l'un de l'autre dans le groupe constitué par H, $C_{1-3}$-alkyle et phényle non substitué ;

à l'exception des composés de formule générale I, dans lesquels

A)

$R^1$ représente un radical hydrogène, un radical $C_{1-6}$-alkyle, un radical $C_{1-6}$-alcoxy, un radical $C_{2-6}$-alcényle, un radical $C_{2-6}$-alcynyle ou un radical $C_{1-6}$-alkyle monosubstitué par un radical phényle,
$R^2$ représente un radical hydrogène, un radical $C_{1-6}$-alkyle, un radical $C_{1-6}$-alcoxy, un radical $C_{2-6}$-hydroxyalkyle, un radical $C_{2-6}$-alcényle, un radical $C_{2-6}$-alcynyle, un radical phényle, un radical $C_{1-6}$-alkyle monosubstitué, disubstitué ou trisubstitué par un radical phényle, un radical -O-$C_{1-6}$-alcanoyle, un radical -O-$C_{1-5}$-alkyle, un radical -O-$C_{2-6}$-hydroxyalkyle, un radical -O-$C_{2-6}$-alcényle, un radical -O-$C_{2-6}$-alcynyle ou un radical -O-$C_{1-6}$-alkyle monosubstitué, disubstitué ou trisubstitué par un radical phényle,
$R^3$ représente un radical hydrogène, un radical $C_{1-6}$-alkyle, un radical $C_{2-6}$-alcényle, un radical $C_{2-6}$-alcynyle, un radical $C_{3-7}$-cycloalkyle, un radical $C_{1-6}$-alkyle substitué par 1-6 atomes d'halogène, un radical hydroxy-$C_{1-6}$-alkyle, un radical $C_{1-6}$-alcoxy, un radical $C_{1-5}$-alkylthio, un radical $C_{1-6}$-alcoxy-$C_{1-6}$-alkyle, un radical carboxy-$C_{1-6}$-alkyle, un radical ($C_{1-6}$-alcoxy)carbonyl-$C_{1-6}$-alkyle, un radical amino-$C_{1-6}$-alkyle, un radical mono-($C_{1-6}$-alkyl)-amino-$C_{1-6}$-alkyle, un radical di- ($C_{1-6}$-alkyl)-amino-$C_{1-6}$-alkyle, un radical 2-oxopyrrolidin-1-ylméthyle, un radical aryle, un radical diarylméthylol, un radical $C_{1-6}$-alkyle substitué par un ou deux radicaux aryle, un radical $C_{1-6}$-alcanoyle ou un radical arylcarbonyle, où aryle représente à chaque fois un radical phényle non substitué ou un radical phényle substitué par 1-3 substituants choisis dans le groupe constitué par halogène, $C_{1-6}$-alkyle, $C_{1-6}$-alcoxy et $CF_3$

ainsi que leurs sels, énantiomères et racémates physiologiquement acceptables, et
B)
les radicaux $R^1$, $R^2$ et $R^3$ représentent à chaque fois un radical hydrogène ou un radical hydrocarboné ainsi qu'à chaque fois leurs sels.

**2.** Composés selon la revendication 1,
**caractérisés**
**en ce que** $R^1$ représente un radical hydrogène, un radical $C_{1-10}$-alkyle linéaire ou ramifié, non substitué présentant le cas échéant au moins un hétéroatome comme élément de chaîne, un radical $C_{2-10}$-alcényle linéaire ou ramifié, non substitué ou au moins monosubstitué, présentant le cas échéant au moins un hétéroatome comme élément de chaîne, un radical $C_{2-10}$-alcynyle linéaire ou ramifié, non substitué ou au moins monosubstitué, présentant le cas échéant au moins un hétéroatome comme élément de chaîne, un radical aryle ou hétéroaryle de 5 à 14 chaînons, non substitué ou au moins monosubstitué, qui est lié via un groupe $C_{1-5}$-alkylène linéaire ou ramifié, présentant le

cas échéant au moins un hétéroatome comme élément de chaîne, un radical -C(=O)-OR$^5$ lié via un groupe $C_{1-5}$-alkylène linéaire ou ramifié
ou un radical -O-C(=O)-R$^6$ lié via un groupe $C_{1-5}$-alkylène linéaire ou ramifié,
de préférence un radical hydrogène, un radical $C_{1-5}$-alkyle linéaire ou ramifié, non substitué, un radical $C_{2-5}$-alcényle linéaire ou ramifié, non substitué, un radical $C_{2-5}$-alcynyle linéaire ou ramifié, non substitué, un radical phényle ou naphtyle non substitué
ou au moins monosubstitué, qui est lié via un groupe $C_{1-5}$-alkylène linéaire ou ramifié, présentant le cas échéant un ou plusieurs atomes d'oxygène comme élément de chaîne, un radical -C(=O)-OR$^5$ lié via un groupe $C_{1-4}$-alkylène linéaire ou ramifié ou un radical -O-C(=O)-R$^6$ lié via un groupe $C_{1-4}$-alkylène linéaire ou ramifié,
de manière particulièrement préférée un radical hydrogène, un radical $C_{1-4}$-alkyle linéaire ou ramifié, non substitué, un radical $C_{2-5}$-alcényle linéaire ou ramifié, non substitué, un radical $C_{2-3}$-alcynyle linéaire ou ramifié, non substitué, un radical phényle
ou naphtyle qui est lié via un pont -($CH_2$)-, -($CH_2$)$_2$-, -($CH_2$)$_3$- ou -($CH_2$)$_2$-O- et le cas échéant monosubstitué ou polysubstitué, de manière identique ou différente, par un substituant choisi dans le groupe constitué par F, Cl, Br, -CN, -$NO_2$, $C_{1-5}$-alkyle, $C_{1-5}$-alcoxy, -C(=O)-$C_{1-5}$-alkyle, -C(=O)-O-$C_{1-5}$-alkyle, -S(=O)$_2$-$C_{1-6}$-alkyle,- C (=O)-$C_{1-5}$-perfluoroalkyle, -$CF_3$, -$CHF_2$ et -$CH_2$F, un radical -C(=O)-OR$^5$ lié via un groupe -($CH_2$)- ou un radical -O-C(=O)-R$^6$ lié via un groupe -($CH_2$)-, -($CH_2$)$_2$-, -($CH_2$)$_3$- ou -($CH_2$)$_4$-.

**3.** Composés selon la revendication 1 ou 2,
**caractérisés**
**en ce que** le radical R$^2$ représente un radical hydrogène, un radical $C_{1-10}$-alkyle linéaire ou ramifié, non substitué ou au moins monosubstitué, présentant le cas échéant au moins un hétéroatome comme élément de chaîne, un radical $C_{2-10}$-alcényle linéaire ou ramifié, non substitué ou au moins monosubstitué, présentant le cas échéant au moins un hétéroatome comme élément de chaîne, un radical $C_{2-10}$-alcynyle linéaire ou ramifié, non substitué ou au moins monosubstitué, présentant le cas échéant au moins un hétéroatome comme élément de chaîne ou un radical aryle ou hétéroaryle de 5 à 14 chaînons, non substitué ou au moins monosubstitué, qui peut être lié via un groupe $C_{1-5}$-alkylène linéaire ou ramifié, présentant le cas échéant au moins un hétéroatome comme élément de chaîne, de préférence un radical hydrogène, un radical $C_{1-5}$-alkyle linéaire ou ramifié, non substitué, présentant le cas échant un ou plusieurs atomes d'oxygène et/ou un
ou plusieurs atomes de soufre comme élément de chaîne,
ou un radical phényle ou naphtyle, qui peut être monosubstitué ou polysubstitué, de manière identique ou différente, par un substituant choisi dans le groupe constitué par F, Cl, Br, -CN, -$NO_2$, $C_{1-5}$-alkyle, $C_{1-5}$-alcoxy, -C(=O)-$C_{1-5}$-alkyle, -C(=O)-O-$C_{1-5}$-alkyle, -S(=O)$_2$-$C_{1-6}$-alkyle, -C(=O)-$C_{1-5}$-perfluoroalkyle, -$CF_3$, -$CHF_2$ et -$CH_2$F et/ou lié via un pont -($CH_2$)-, -($CH_2$)$_2$-, -($CH_2$)$_3$- ou -($CH_2$)$_2$-O-,
de manière particulièrement préférée un radical hydrogène, un radical $C_{1-5}$-alkyle linéaire ou ramifié, non substitué, présentant le cas échéant un ou plusieurs atomes de soufre comme élément de chaîne, ou un radical phényle, où le radical phényle peut être monosubstitué ou polysubstitué, de manière identique ou différente, par un substituant choisi dans le groupe constitué par F, Cl, Br, -CN, -$NO_2$, $C_{1-5}$-alkyle, $C_{1-5}$-alcoxy, -C(=O)-$C_{1-5}$-alkyle, -C(=O)-O-$C_{1-5}$-alkyle, -S(=O)$_2$-$C_{1-6}$-alkyle, -C(=O)-$C_{1-5}$-perfluoroalkyle, -$CF_3$, -$CHF_2$ et -$CH_2$F et/ou lié via un pont -($CH_2$)-.

**4.** Composés selon l'une ou plusieurs des revendications 1 à 3, **caractérisés**
**en ce que** R$^3$ représente un radical hydrogène, un radical $C_{1-10}$-alkyle linéaire ou ramifié, non substitué
ou au moins monosubstitué, présentant le cas échéant au moins un hétéroatome comme élément de chaîne, un radical $C_{2-10}$-alcényle linéaire ou ramifié, non substitué ou au moins monosubstitué, présentant le cas échéant au moins un hétéroatome comme élément de chaîne, un radical $C_{2-10}$-alcynyle linéaire ou ramifié, non substitué ou au moins monosubstitué, présentant le cas échéant au moins un hétéroatome comme élément de chaîne, un radical aryle ou hétéroaryle de 5 à 14 chaînons, non substitué ou au moins monosubstitué, qui est lié via un groupe $C_{1-5}$-alkylène linéaire ou ramifié, non substitué ou au moins monosubstitué, présentant le cas échéant au moins un hétéroatome comme élément de chaîne et le cas échéant condensé avec un système monocyclique non substitué ou au moins monosubstitué, un radical $C_{3-8}$-cycloaliphatique non substitué ou au moins monosubstitué, présentant le cas échéant au moins un hétéroatome comme élément de cycle, qui peut être lié via un groupe $C_{1-5}$-alkylène linéaire ou ramifié, non substitué ou au moins monosubstitué, présentant le cas échéant au moins un hétéroatome comme élément de chaîne et/ou condensé avec un système monocyclique non substitué ou au moins monosubstitué, ou un groupe -C(=O)-R$^4$,
de préférence un radical hydrogène, un radical biphényle, qui est lié via un pont -($CH_2$)-, -($CH_2$)$_2$- ou -($CH_2$)$_3$-, ou un groupe -C(=O)-R$^4$, de manière particulièrement préférée un groupe -C(=O)-R$^4$.

**5.** Composés selon l'une ou plusieurs des revendications 1 à 4, **caractérisés**

**en ce que** le radical $R^4$

représente un radical $C_{1-10}$-alkyle linéaire ou ramifié, non substitué ou au moins monosubstitué, présentant le cas échéant au moins un hétéroatome comme élément de chaîne, un radical $C_{2-10}$-alcényle linéaire ou ramifié, non substitué ou au moins monosubstitué, présentant le cas échéant au moins un hétéroatome comme élément de chaîne, un radical $C_{2-10}$-alcynyle linéaire ou ramifié, non substitué ou au moins monosubstitué, présentant le cas échéant au moins un hétéroatome comme élément de chaîne,

représente un radical aryle ou hétéroaryle de 5 à 14 chaînons non substitué ou au moins monosubstitué, qui peut être lié via un groupe $C_{1-5}$-alkylène, $C_{2-5}$-alcénylène ou $C_{2-5}$-alcynylène linéaire ou ramifié, non substitué ou au moins monosubstitué, présentant le cas échéant au moins un hétéroatome comme élément de chaîne et/ou condensé avec un système monocyclique non substitué ou au moins monosubstitué, représente un radical $C_{3-8}$-cycloaliphatique non substitué ou au moins monosubstitué, présentant le cas échéant au moins un hétéroatome comme élément de cycle, le cas échéant lié via un groupe $C_{1-6}$-alkylène linéaire ou ramifié, non substitué ou au moins monosubstitué, présentant le cas échéant au moins un hétéroatome comme élément de chaîne, qui peut être au moins ponté une fois par un groupe $C_{1-6}$-alkylène linéaire ou ramifié, non substitué ou au moins monosubstitué et/ou condensé avec un système monocyclique non substitué ou au moins monosubstitué,

représente un radical -C(=O)-OR$^7$ lié via un groupe $C_{1-5}$-alkylène linéaire ou ramifié, non substitué ou au moins monosubstitué, un radical -O-(C=O)-R$^8$ lié via un groupe $C_{1-5}$-alkylène linéaire ou ramifié, non substitué ou au moins monosubstitué ou un radical -(C=O)-R$^9$ lié via un groupe $C_{1-5}$-alkylène linéaire ou ramifié, non substitué ou au moins monosubstitué,

représente de préférence un radical $C_{1-10}$-alkyle linéaire ou ramifié présentant le cas échéant un ou plusieurs atomes d'oxygène et/ou un ou plusieurs atomes de soufre comme élément de chaîne, qui peut être non substitué ou au moins monosubstitué, de manière identique ou différente, par un substituant choisi dans le groupe constitué par phényle, di-($C_{1-5}$-alkylamino), carboxy et -NH-(C=O)-O-$C_{1-5}$-alkyle, représente un radical $C_{2-5}$-alcényle linéaire ou ramifié, non substitué, représente un radical phényle, un radical naphtyle, un radical furyle (furannyle), un radical thiényle (thiophényle) ou un radical [1,2,3]-triazolyle, un radical benzo[1,3]dioxolyle, un radical benzo[1,2,5]oxadiazolyle, un radical chromanyle, un radical pyrimidinyle, un radical pyrazolyle, un radical pyridinyle, un radical isoxazolyle, ou un radical [1,2,3]-thiadiazolyle, où le radical cyclique peut être lié à chaque fois via un pont $-(CH_2)-$, $-(CH_2)-O-$, -CH=CH-, $-(CH_2)_2-$, $-(CH_2)_2-O-$, ou $-(CH_2)_3$ et/ou être à chaque fois monosubstitué ou polysubstitué, de manière identique ou différente, par un substituant choisi dans le groupe constitué par F, Cl, Br, I, -CN, $-NO_2$, $C_{1-5}$-alkyle, $C_{1-5}$-alcoxy, $C_{1-5}$-perfluoroalcoxy, -C(=O)-$C_{1-5}$-alkyle, -C(=O)-O-$C_{1-5}$-alkyle, $-(CH_2)_{1-3}$-O-C(=O)-phényle, $-N(CH_3)_2$, $-S(=O)_2-C_{1-6}$-alkyle, $-S(=O)_2-NH_2$, -C(=O)-$C_{1-5}$-perfluoroalkyle, -S-$C_{1-5}$-alkyle, $-S-CH_2F$, $-S-CHF_2$, $-SCF_3$, $-CF_3$, $-NH-(C=O)-CH_3$, $-CHF_2$, $-CH_2F$, phényle et phénoxy, où les substituants phényle et phénoxy mentionnés en dernier lieu peuvent eux-mêmes être monosubstitués ou polysubstitués, de manière identique ou différente, par un substituant choisi dans le groupe constitué par F, Cl, Br, $-S-CHF_2$, $-CF_3$, $-CHF_2$, $-CH_2F$, -CN, $-NO_2$, $C_{1-5}$-alkyle et $C_{1-5}$-alcoxy, représente un radical cycloaliphatique présentant le cas échéant au moins un hétéroatome comme élément de cycle, le cas échéant lié via un pont $-(CH_2)-$, $-(CH_2)_2-$ ou - $(CH_2)_3-$, de 3, 4, 5, 6, 7 ou 8 chaînons, qui peut être ponté au moins un fois par un groupe $-(C(CH_3)_2)-$ ou $-(CH_2)-$ et/ou non substitué ou monosubstitué ou polysubstitué, de manière identique ou différente, par un substituant choisi dans le groupe constitué par $C_{1-5}$-alkyle, $C_{1-5}$-alcoxy, $-CF_3$, $-CHF_2$, $-CH_2F$ et oxo, ou représente un radical -C(=O)-OR$^7$ lié via un groupe $C_{1-5}$-alkylène linéaire ou ramifié, non substitué ou au moins monosubstitué, un radical -O-(C=O)-R$^8$ lié via un groupe $C_{1-5}$-alkylène linéaire ou ramifié, non substitué ou au moins monosubstitué ou un radical-(C=O)-R$^9$ lié via un groupe $C_{1-5}$-alkylène linéaire ou ramifié, non substitué ou au moins monosubstitué,

représente de manière particulièrement préférée un radical $C_{1-8}$-alkyle linéaire ou ramifié, présentant le cas échéant un ou plusieurs atomes d'oxygène et/ou un ou plusieurs atomes de soufre comme élément de chaîne, qui peut être non substitué ou au moins monosubstitué, de manière identique ou différente, par un substituant choisi dans le groupe constitué par phényle non substitué, diméthylamino, carboxy et $-NH-(C=O)-O-C(CH_3)_3$. représente un radical $C_{2-4}$-alcényle linéaire ou ramifié, non substitué, représente un radical phényle, un radical naphtyle, un radical furyle (furannyle), un radical thiényle (thiophényle), un radical [1,2,3]-triazolyle, un radical chromanyle, un radical benzo [1,3]dioxolyle, un radical benzo[1,2,5]oxadiazolyle, un radical pyrimidinyle, un radical pyrazolyle, un radical pyridinyle, un radical isoxazolyle, ou un radical [1,2,3]-thiadiazolyle, où le radical cyclique peut être lié à chaque fois via un pont $-(CH_2)-$, $-(CH_2)-O-$, -CH=CH-, $-(CH_2)_2-$, $-(CH_2)_2-O-$, ou $-(CH_2)_3$ et/ou être monosubstitué ou polysubstitué, de manière identique ou différente, par un substituant choisi dans le groupe constitué par F, Cl, Br, I, méthyle, éthyle, iso-propyle, n-propyle, sec-butyle, tert-butyle, n-butyle, méthoxy, éthoxy, $-CF_3$, $-OCF_3$, $-CH_2-O-C(=O)$-phényle, $-NO_2$, $-S-CF_3$, $-S-CHF_2$, $-N(CH_3)_2$, $-NH-CO-CH_3$, -CN, $-SO_2-NH_2$, phényle et phénoxy, où les substituants phényle et phénoxy mentionnés en dernier lieu peuvent eux-mêmes être monosubstitués ou polysubstitués, de manière identique ou différente, par un substituant choisi dans le groupe constitué par F, Cl, Br, $-CH_3$ et $-OCH_3$,

représente un radical cyclopropyle, cyclobutyle, cyclopentyle, cyclohexyle, imidazolidine, 4,7,7-triméthyl-3-oxo-2-oxa-bicyclo-[2,2,1]-heptyle ou adamantyle le cas échéant lié via un pont-$(CH_2)$-, -$(CH_2)_2$- ou -$(CH_2)_3$-, qui est non substitué ou monosubstitué ou polysubstitué, de manière identique ou différente, par un substituant choisi dans le groupe constitué par $C_{1-5}$-alkyle, $C_{1-5}$-alcoxy, -$CF_3$, -$CHF_2$, -$CH_2F$ et oxo,
représente un radical -C(=O)-$OR^7$ lié via un groupe -$(CH_2)$-, -$(CH_2)_2$-, -C(H)(phényl)-, ou -$C(CH_3)_2$-, représente un radical -O-(C=O)-$R^8$ lié via un groupe -$(CH_2)$-, -$(CH_2)_2$-, -C(H)(phényl)-, ou -$C(CH_3)_2$-, ou représente un radical -(C=O)-$R^9$ lié via un groupe -$(CH_2)$- ou -$(CH_2)_2$-,
représente de manière tout particulièrement préférée un radical $C_{6-8}$-alkyle linéaire ou ramifié, présentant le cas échéant un ou plusieurs atomes d'oxygène et/ou un ou plusieurs atomes de soufre comme élément de chaîne, qui peut être non substitué ou au moins monosubstitué, de manière identique ou différente, par un substituant choisi dans le groupe constitué par phényle non substitué, diméthylamino, carboxy et -NH-(C=O)-$OC(CH_3)_3$, représente un radical $C_{2-4}$-alcényle linéaire ou ramifié, non substitué, représente un radical naphtyle, un radical furyle (furannyle), un radical thiényle (thiophényle), un radical [1,2,3]-triazolyle, un radical chromanyle, un radical benzo[1,3]dioxolyle, un radical benzo[1,2,5]oxadiazolyle, un radical pyrimidinyle, un radical pyrazolyle, un radical pyridinyle, un radical isoxazolyle ou un radical [1,2,3]-thiadiazolyle, où le radical cyclique peut être lié à chaque fois via un pont -$(CH_2)$-, -$(CH_2)$-O-, -CH=CH-, -$(CH_2)_2$-, -$(CH_2)_2$-O-, ou -$(CH_2)_3$- et/ou peut être monosubstitué ou polysubstitué, de manière identique ou différente, par un substituant choisi dans le groupe constitué par F, Cl, Br, I, méthyle, éthyle, iso-propyle, n-propyle, sec-butyle, tert-butyle, n-butyle, méthoxy, éthoxy, -$CF_3$, -$OCF_3$, -$CH_2$-O-C(=O)-phényle, -$NO_2$, -S-$CF_3$, -S-$CHF_2$, -$N(CH_3)_2$, -NH-CO-$CH_3$, -CN, -$SO_2$-$NH_2$, phényle et phénoxy, où les substituants phényle et phénoxy mentionnés en dernier lieu peuvent eux-mêmes être monosubstitués ou polysubstitués, de manière identique ou différente, par un substituant choisi dans le groupe constitué par F, Cl, Br, -$CH_3$ et -$OCH_3$, représente 2-trifluorométhoxyphényle, 3-trifluorométhoxyphényle, 4-trifluorométhoxyphényle, 2-$CF_3$-S-phényle, 3-$CF_3$-S-phényle, 4-$CF_3$-S-phényle, 2-cyanophényle, 3-cyanophényle, 4-cyanophényle, 2-nitrophényle, 3-nitrophényle, 4-nitrophényle, 2-$CHF_2$-S-phényle, 3-$CHF_2$-S-phényle, 4-$CHF_2$-S-phényle, 2-diméthylaminophényle, 3-diméthylaminophényle, 4-diméthylaminophényle, 2-phényl-(C=O)-O-$CH_2$-phényle, 3-phényl-(C=O)-O-$CH_2$-phényle, 4-phényl-(C=O)- O-$CH_2$-phényle, 2-$CH_3$-(C=O)-NH-phényle, 3-$CH_3$-(C=O)-NH-phényle, 4-$CH_3$-(C=O)-NH-phényle, 2-phénylphényle, 3-phénylphényle, 4-phénylphényle, 2-$NH_2$-$SO_2$-phényle, 3-$NH_2$-$SO_2$-phényle, 4-$NH_2$-$SO_2$-phényle, pentafluorophényle, 4-méthyl-3-nitrophényle, 2-fluorobenzyle, 3-fluorobenzyle, 4-fluorobenzyle, 2-chlorobenzyle, 3-chlorobenzyle, 4-chlorobenzyle, 2-bromobenzyle, 3-bromobenzyle, 4-bromobenzyle, 2-méthylbenzyle, 3-méthylbenzyle, 4-méthylbenzyle, 2-méthoxybenzyle, 3-méthoxybenzyle, 4-méthoxybenzyle, 3,4-diméthoxybenzyle, 2,5-diméthoxybenzyle, -CH=CH-phényle, où le radical phényle peut être monosubstitué ou polysubstitué de manière identique ou différente en position 2, 3, ou 4 par un radical choisi dans le groupe constitué par F, Cl, Br et $CF_3$, représente -$CH_2$-O-phényle, où le radical phényle peut être monosubstitué ou polysubstitué de manière identique ou différente, en 2ème, 3ème ou 4ème position, par un radical choisi dans le groupe constitué par F, Cl, Br et $CF_3$, représente un radical cyclopropyle, cyclobutyle, cyclopentyle, cyclohexyle, imidazolidine, 4,7,7-triméthyl-3-oxo-2-oxabicyclo-[2,2,1]-heptyle ou adamantyle le cas échéant lié via un pont -$(CH_2)$-, -$(CH_2)_2$- ou -$(CH_2)_3$-, où les radicaux cycliques peuvent à chaque fois être non substitués ou monosubstitués ou polysubstitués, de manière identique ou différente, par un substituant choisi dans le groupe constitué par $C_{1-5}$-alkyle, $C_{1-5}$-alcoxy, -$CF_3$, $CHF_2$, $CH_2F$ et oxo, représente un radical -C(=O)-$OR^7$ lié via un groupe -$(CH_2)$-, -$(CH_2)_2$-, -C(H)(phényl)- ou -$C(CH_3)_2$, représente un radical -O-(C=O)-$R^8$ lié via un groupe -$(CH_2)$-, -$(CH_2)_2$-, -C(H)(phényl)- ou -$C(CH_3)_2$, ou représente un radical -(C=O)-$R^9$ lié via un groupe -$(CH_2)$- ou -$(CH_2)_2$-.

6. Composés selon l'une ou plusieurs des revendications 1 à 5, **caractérisés en ce que** les radicaux $R^5$ et $R^6$, à chaque fois indépendamment l'un de l'autre, représentent un radical $C_{1-5}$-alkyle linéaire ou ramifié, un radical $C_{2-5}$-alcényle linéaire ou ramifié ou un radical $C_{2-5}$-alcynyle linéaire ou ramifié, de préférence un radical méthyle, éthyle, n-propyle ou iso-propyle.

7. Composés selon l'une ou plusieurs des revendications 1 à 6, **caractérisés en ce que** les radicaux $R^7$ et $R^8$, à chaque fois indépendamment l'un de l'autre, représentent un radical hydrogène, un radical $C_{1-5}$-alkyle linéaire ou ramifié, un radical $C_{2-5}$-alcényle linéaire ou ramifié, un radical $C_{2-5}$-alcynyle linéaire ou ramifié ou un radical phényle ou naphtyle le cas échéant au moins monosubstitué, de préférence un radical méthyle, éthyle, n-propyle, iso-propyle ou un radical phényle non substitué.

8. Composés selon l'une ou plusieurs des revendications 1 à 7, **caractérisés en ce que** le radical $R^9$ représente un radical $C_{1-5}$-alkyle linéaire ou ramifié, un radical $C_{2-5}$-alcényle linéaire ou ramifié, un radical $C_{2-5}$-alcynyle linéaire ou ramifié, de préférence un radical méthyle, éthyle, n-propyle ou iso-propyle.

9. Composés selon l'une ou plusieurs des revendications 1 à 8, **caractérisés en ce que**

$R^1$ représente un radical hydrogène, un radical $C_{1-4}$-alkyle linéaire ou ramifié, non substitué, un radical $C_{2-5}$-alcényle linéaire ou ramifié, non substitué, un radical $C_{2-3}$-alcynyle linéaire ou ramifié, non substitué, un radical phényle ou naphtyle qui est non substitué ou monosubstitué ou polysubstitué, de manière identique ou différente, par un substituant choisi dans le groupe constitué par F, Cl, Br, -CN, $-NO_2$, $C_{1-5}$-alkyle, $C_{1-5}$-alcoxy, -C(=O)-$C_{1-5}$-alkyle, -C(=O)-O-$C_{1-5}$-alkyle, $-S(=O)_2$-$C_{1-6}$-alkyle, -C(=O)-$C_{1-5}$-perfluoroalkyle, $-CF_3$, $-CHF_2$ et $-CH_2F$ et/ou lié via un pont $-(CH_2)-$, $-(CH_2)_2-$, $-(CH_2)_3-$ ou $-(CH_2)_2$-O-, un radical C(=O)-$OR^5$ lié via un groupe $-(CH_2)-$ ou un radical -O-C(=O)-$R^6$ lié via un groupe $-(CH_2)-$, $-(CH_2)_2-$, $-(CH_2)_3-$ ou $-(CH_2)_4-$,
$R^2$ représente un radical hydrogène, un radical $C_{1-5}$-alkyle linéaire ou ramifié, non substitué, présentant le cas échéant un ou plusieurs atomes de soufre comme élément de chaîne, ou un radical phényle, où le radical phényle peut être monosubstitué ou polysubstitué, de manière identique ou différente, par un substituant choisi dans le groupe constitué par F, Cl, Br, -CN, $-NO_2$, $C_{1-5}$-alkyle, $C_{1-5}$-alcoxy, -C(=O)-$C_{1-5}$-alkyle, -C(=O)-O-$C_{1-5}$-alkyle, $-S(=O)_2$-$C_{1-6}$-alkyle, -C(=O)-$C_{1-5}$-perfluoroalkyle, $-CF_3$, $CHF_2$ et $CH_2F$ et/ou lié via un pont $-(CH_2)-$,
$R^3$ représente un radical hydrogène, un radical biphényle, qui est lié via un pont $-(CH_2)-$, $-(CH_2)_2-$ ou $-(CH_2)_3-$, ou un groupe C(=O)-$R^9$,
$R^4$ représente un radical choisi dans le groupe constitué par méthyle, éthyle, iso-propyle, n-propyle, sec-butyle, isobutyle, tert-butyle, n-butyle, n-pentyle, 1-méthylbutyle, 2-diméthylpropyle, 1-éthylpropyle, 1-propylbutyle, 1-éthylpentyle, diméthylaminométhyle, $-CH_2-CH_2-CH=CH_2$, $-CH_2-O-CH_3$, $-CH_2-O-CH_2-CH_2-O-CH_3$, $-CH_2-CH_2$-phényle, $-CH_2$-O-phényle, $-CH_2-O-CH_2$-phényle, $-CH_2-CH_2-CH_2$-O-phényle, -C(H)(phényl)($C_2H_5$), -C(H)($CH_3$)-O-phényle, $-CH_2-CH_2-(C=O)-CH_3$, $-CH_2-O-(C=O)-CH_3$, $-CH_2-CH_2-(C=O)-O-C_2H_5$, $-CH_2$-O-(C=O)-phényle, $-CH_2-(C=O)-O-CH_2-CH_3$, -C(H)(phényl)-(C=O)-$CH_3$, -C(H)(phényl)-O-(C=O)-$CH_3$, -C$(CH_3)_2$-O-(C=O)-$CH_3$, -C(H)(NH-(C=O)-O-$(CH_3)_3$)($CH_2-O-CH_2$-phényl), -C$(CH_3)_2$-$CH_2$-COOH, phényle non substitué, 2-fluorophényle, 3-fluorophényle, 4-fluorophényle, 2-chlorophényle, 3-chlorophényle, 4-chlorophényle, 2-bromophényle, 3-bromophényle, 4-bromophényle, 2-iodophényle, 3-iodophényle, 4-iodophényle, 2-méthylphényle, 3-méthylphényle, 4-méthylphényle, 2-éthylphényle, 3-éthylphényle, 4-éthylphényle, 2-n-propylphényle, 3-n-propylphényle, 4-n-propylphényle, 2-tert-butylphényle, 3-tert-butylphényle, 4-tert-butylphényle, 2-méthoxyphényle, 3-méthoxyphényle, 4-méthoxyphényle, 2-éthoxyphényle, 3-éthoxyphényle, 4-éthoxyphényle, 2-trifluorométhylphényle, 3-trifluorométhylphényle, 4-trifluorométhylphényle, 2-trifluorométhoxyphényle, 3-trifluorométhoxyphényle, 4-trifluorométhoxyphényle, 2-$CF_3$-S-phényle, 3-$CF_3$-S-phényle, 4-$CF_3$-S-phényle, 2-cyanophényle, 3-cyanophényle, 4-cyanophényle, 2-nitrophényle, 3-nitrophényle, 4-nitrophényle, 2-$CHF_2$-S-phényle, 3-$CHF_2$-S-phényle, 4-$CHF_2$-S-phényle, 2-diméthylaminophényle, 3-diméthylaminophényle, 4-diméthylaminophényle, 2-phényl-(C=O)-O-$CH_2$-phényle, 3-phényl-(C=O)-O-$CH_2$-phényle, 4-phényl-(C=O)-O-$CH_2$-phényle, 2-$CH_3$-(C=O)-NH-phényle, 3-$CH_3$-(C=O)-NH-phényle, 4-$CH_3$-(C=O)-NH-phényle, 2-phénylphényle, 3-phénylphényle, 4-phénylphényle, 2-$NH_2$-$SO_2$-phényle, 3-$NH_2$-$SO_2$-phényle, 4-$NH_2$-$SO_2$-phényle, 2,4-diméthoxyphényle, 2,5-diméthoxyphényle, 3,5-diméthoxyphényle, 3,4-diméthoxyphényle, 2,3-diméthylphényle, 2,3-dichlorophényle, 3,4-dichlorophényle, 2,6-dichlorophényle, 2,3-difluorophényle, 3,4-difluorophényle, 3,5-difluorophényle, pentafluorophényle, 2-chloro-6-fluorophényle, 4-bromo-3-méthylphényle, 3-fluoro-4-méthylphényle, 3-chloro-2-fluorophényle, 2-chloro-4-nitrophényle, 5-fluoro-2-trifluorométhylphényle, 3-fluoro-4-trifluorométhyle, 4-méthyl-3-nitrophényle, 2-chloro-5-trifluorométhyle, 2,5-bistrifluorométhylphényle, 3,5-bistrifluorométhylphényle, 2-chloro-6-fluoro-3-méthylphényle, 6-chloro-2-fluoro-3-méthylphényle, 2,6-difluoro-4-méthylphényle, 2,6-difluoro-3-méthylphényle, 3,4,5-triméthoxyphényle, 2,3-difluoro-4-méthylphényle, 2-fluorobenzyle, 3-fluorobenzyle, 4-fluorobenzyle, 2-chlorobenzyle, 3-chlorobenzyle, 4-chlorobenzyle, 2-bromobenzyle, 3-bromobenzyle, 4-bromobenzyle, 2-méthylbenzyle, 3-méthylbenzyle, 4-méthylbenzyle, 2-méthoxybenzyle, 3-méthoxybenzyle, 4-méthoxybenzyle, 3,4-diméthoxybenzyle, 2,5-diméthoxybenzyle, -CH=CH-phényle, où le radical phényle peut être monosubstitué ou polysubstitué, de manière identique ou différente, en position 2, 3 ou 4, par un radical choisi dans le groupe constitué par F, Cl, Br et $CF_3$, représente $-CH_2$-O-phényle, où le radical phényle peut être monosubstitué ou polysubstitué, de manière identique ou différente, en position 2, 3 ou 4, par un radical choisi dans le groupe constitué par F, Cl, Br et $CF_3$, représente 1-naphtyle, 2-naphtyle, 2-thiényle, 3-thiényle, 3-chlorothién-2-yle, 2-furannyle, 3-furannyle, 2,5-diméthyl-furann-3-yle, 5-tert-butyl-2-méthylfurann-3-yle, pyridin-2-yle, pyridin-3-yle, pyridin-4-yle, 2-chloropyridin-4-yle, 6-chloropyridin-3-yle, 2-chloropyridin-3-yle, 2-éthylsulfanylpyridin-3-yle, 2-phénoxypyridin-3-yle, 2-méthylsulfanylpyridin-3-yle, 2-méthyl-6-trifluorométhylpyridin-3-yle, isoxazol-5-yle, 5-méthylisoxazol-3-yle, 3-(2-chloro-6-fluorophényl)-5-méthylisoxazol-4-yle, 3-(2-chlorophényl)-5-méthylisoxazol-4-yle, 3-(2,6-dichlorophényl)-5-méthylisoxazol-4-yle, 5-tert-butyl-2-méthyl-2H-pyrazol-3-yle, 1-phényl-5-n-propyl-1H-pyrazol-4-yle, 1-(4-chlorophényl)-5-trifluorométhyl-1H-pyrazol-4-yle, 2-tert-butyl-5-méthyl-2H-pyrazol-3-yle, 5-méthyl-2-phényl-2H-[1,2,3]triazol-4-yle, 4-méthyl-[1,2,3]thiadiazol-5-yle, 2-chloro-4-trifluorométhylpyrimidin-5-yle, 2-chloro-4-trifluorométhylpyrimidin-5-yle, benzo[1,2,5]oxadiazol-5-yle, benzo[1,3]dioxol-5-yle, 6-chloro-2H-chroman-3-yle, imidazolidin-2,4-dion-5-ylméthyle, cyclopropyle, cyclobutyle, représente cyclopentyle, cyclohexyle, 4,7,7-triméthyl-3-oxo-2-oxabicyclo[2,2,1]heptyle et adamantyle, le cas échéant lié via un pont $-(CH_2)-$ou $-(CH_2)_2-$.

**10.** Composés selon l'une ou plusieurs des revendications 1 à 9, **caractérisés en ce que**

I.)

$R^1$ représente un radical aryle substitué présentant 6 ou 10 chaînons ;
représente un radical hétéroaryle de 5 à 14 chaînons, le cas échéant substitué ;
représente $-(CH_2)_m-O-C(=O)-R^5$ avec m = 1, 2, 3, 4 ou 5 ;
représente $-(CH_2)_n-C(=O)-O-R^6$ avec n = 1, 2, 3, 4 ou 5 ;
représente - $(CH_2)-R^{22}$ ;
ou représente $-(CH_2)-(CH_2)_c-U-(CH_2)_d-R^{23}$ avec c = 0 ou 1 et d = 0 ou 1, où U représente O, S, NH, N $(CH_3)$ ou $N(C_2H_5)$ ;
$R^2$ représente un radical hydrogène ;
représente $-(CH_2)-(CH_2)_e-V_f-(CH_2)_g-R^{24}$ avec e = 0 ou 1, f = 0 ou 1 et g = 0 ou 1, où V représente O, S, NH, $N(CH_3)$ ou $N(C_2H_5)$ ;
représente un radical $C_{1-10}$-alkyle, $C_{2-10}$-alcényle ou $C_{2-10}$-alcynyle à chaque fois linéaire ou ramifié, le cas échéant à chaque fois substitué ;
ou représente un radical aryle ou hétéroaryle de 5 à 14 chaînons, le cas échéant substitué ;
$R^3$ représente un groupe $-C(=O)-R^4-$ ;

et

$R^4$ représente $-(CHR^{10})-P_s-(CH_2)C(CH_2)_u-Q_v-R^{11}$ avec s = 0 ou 1, t = 0 ou 1, u = 0 ou 1 et v = 0 ou 1, où P et Q représentent, indépendamment l'un de l'autre, à chaque fois O, S, NH, $N(CH_3)$ ou $N(C_2H_5)$ et la somme de s et v est égale à 1 ;
représente $-(CR^{12}R^{13})-(CH_2)_w-(CH_2)_x-C(=O)-O-R^7$ avec w = 0 ou 1 et x = 0 ou 1 ;
représente $-(CR^{14}R^{15})-(CH_2)_y-O-C(=O)-R^8$ avec y = 0 ou 1 ;
représente $-(CHR^{16})-(CH_2)_z-C(=O)-R^9$ avec z = 0 ou 1 ;
représente $-CH[(CH_2)-T_a-(CH_2)_b-R^{17}][NH-C(=O)-O-R^{18}]$ avec a = 0 ou 1 et b = 0 ou 1, où T représente O, S, NH, $N(CH_3)$ ou $N(C_2H_5)$ ;
représente $-CHR^{19}R^{20}$ ou $-(CH_2)-CHR^{19}R^{20}$ ;
représente $-(CH=CH)-R^{21}$ ;
représente un radical $C_{1-10}$-alkyle, $C_{2-10}$-alcényle ou $C_{2-10}$-alcynyle à chaque fois linéaire ou ramifié, le cas échéant à chaque fois substitué ;
représente un radical cycloaliphatique insaturé ou saturé, le cas échéant substitué, de 3, 4, 5, 6, 7, 8 ou 9 chaînons, qui peut être condensé avec un système monocyclique saturé ou insaturé, le cas échéant aromatique, le cas échéant substitué et/ou ponté par un ou deux groupes $C_{1-5}$-alkylène linéaires ou ramifiés, le cas échéant substitués,
ou représente un radical aryle ou hétéroaryle de 5 à 14 chaînons, le cas échéant substitué, qui peut être condensé avec un système monocyclique saturé
ou insaturé, le cas échéant substitué ;

ou II.)

$R^1$ représente un radical hydrogène ;
représente un radical $C_{1-10}$-alkyle, $C_{2-10}$-alcényle ou $C_{2-10}$-alcynyle à chaque fois linéaire ou ramifié, à chaque fois non substitué ;
ou représente un radical phényle ou benzyle non substitué ;
$R^2$ représente un radical hydrogène ;
représente $-(CH_2)-(CH_2)_e-V_f-(CH_2)_g-R^{24}$ avec e = 0 ou 1, f = 0 ou 1 et g = 0 ou 1, où V représente 0, S, NH, $N(CH_3)$ ou $N(C_2H_5)$ ;
représente un radical $C_{1-10}$-alkyle, $C_{2-10}$-alcényle ou $C_{2-10}$-alcynyle à chaque fois linéaire ou ramifié, le cas échéant à chaque fois substitué ;
ou représente un radical aryle ou hétéroaryle de 5 à 14 chaînons, le cas échéant substitué ;
$R^3$ représente un groupe $-C(=O)-R^4$ ;

et

$R^4$ représente $-(CHR^{10})-P_s-(CH_2)_t-(CH_2)_u-Q_v-R^{11}$ avec s = 0 ou 1, t = 0 ou 1, u = 0 ou 1 et v = 0 ou 1, où

P et Q représentent, indépendamment l'un de l'autre, à chaque fois O, S, NH, $N(CH_3)$ ou $N(C_2H_5)$ et la somme de s et v est égale à 1 ;
représente $-(CR^{12}R^{13})-(CH_2)_w-(CH_2)_x-C(=O)-O-R^7$ avec w = 0 ou 1 et x = 0 ou 1 ;
représente $-(CR^{14}R^{15})-(CH_2)_y-O-C(=O)-R^8$ avec y = 0 ou 1 ;
représente $-(CHR^{16})-(CH_2)_z-C(=O)-R^9$ avec z = 0 ou 1 ;
représente $-CH[(CH_2)-T_a-(CH_2)_b-R^{17}][NH-C(=O)-O-R^{18}]$ avec a = 0 ou 1 et b = 0 ou 1, où T représente O, S, NH, $N(CH_3)$ ou $N(C_2H_5)$ ;
représente $-CHR^{19}R^{20}$ ou $-(CH_2)-CHR^{19}R^{20}$ ;
représente $-(CH{=}CH)-R^{21}$ ;
représente un radical $C_{1-10}$-alkyle linéaire ou ramifié, substitué ;
représente un radical $C_{2-10}$-alcényle linéaire ou ramifié, le cas échéant substitué ;
représente un radical $C_{2-10}$-alcynyle linéaire ou ramifié, le cas échéant substitué ;
représente un radical cycloaliphatique insaturé ou saturé, le cas échéant substitué, de 3, 4, 5, 6, 7, 8 ou 9 chaînons, qui peut être condensé avec un système monocyclique saturé ou insaturé, le cas échéant aromatique, le cas échéant substitué et/ou ponté par un ou deux groupes $C_{1-5}$-alkylène linéaires ou ramifiés, le cas échéant substitués ; représente un radical phényle, qui est substitué par 1, 2, 3, 4 ou 5 substituants choisis indépendamment l'un de l'autre dans le groupe constitué par -CN, $-SF_5$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-C_{1-5}$-alkyle, -C(=O)-OH, $-C(=O)-O-C_{1-5}$-alkyle, $-O-C(=O)-C_{1-5}$-alkyle, -O-C(=O)-phényle, $-(CH_2)-O-C(=O)-C_{1-5}$-alkyle, $-(CH_2)$-O-C(=O)-phényle, $-NH-C_{1-5}$-alkyle, $-N(C_{1-5}\text{-alkyle})_2$, $-NH-C(=O)-O-C_{1-5}$-alkyle, $-NH-C(=O)-C_{1-5}$-alkyle, -C(=O)-H, $-C(=O)-C_{1-5}$-alkyle, $-C(=O)-NH_2$, $-C(=O)-NH-C_{1-5}$-alkyle, $C(=O)-N-(C_{1-5}\text{-alkyle})_2$, $-S(=O)_2-NH_2$, $-S(=O)_2-NH-C_{1-5}$-alkyle, $S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-C_{1-5}$-alkyle, $-C_{1-5}$-alkyle, $-O-CF_3$, $-S-CF_3$, phényle et -O-benzyle,
représente un radical choisi dans le groupe constitué par 1,2,3,4,5-pentafluorophényle, 1,2,3,4-tétrafluorophényle, 4-méthyl-3-nitrophényle, 5-méthyl-3-nitrophényle, 6-méthyl-3-nitrophényle et 2-méthyl-3-nitrophényle ;
représente un radical naphtyle non substitué ;
représente un radical aryle de 6 à 10 chaînons, le cas échéant substitué, qui est condensé avec un système monocyclique saturé ou insaturé, le cas échéant substitué ;
ou représente un radical hétéroaryle de 5 à 14 chaînons, le cas échéant substitué, qui peut être condensé avec un système monocyclique saturé ou insaturé, le cas échéant substitué ;

ou III.)

$R^1$ représente un radical hydrogène ;
représente un radical $C_{1-10}$-alkyle, $C_{2-10}$-alcényle ou $C_{2-10}$-alcynyle à chaque fois linéaire ou ramifié, à chaque fois non substitué ;
représente un radical aryle ou hétéroaryle de 5 à 14 chaînons, le cas échéant substitué ;
représente $-(CH_2)_m-O-C(=O)-R^5$ avec m = 1, 2, 3, 4 ou 5 ;
représente $-(CH_2)_n-C(=O)-O-R^6$ avec n = 1, 2, 3, 4 ou 5 ;
représente un radical benzyle non substitué ;
représente $-(CH_2)-R^{22}$ ;
ou représente $-(CH_2)-(CH_2)_c-U-(CH_2)_d-R^{23}$ avec c = 0 ou 1 et d = 0 ou 1, où U représente O, S, NH, $N(CH_3)$ ou $N(C_2H_5)$ ;
$R^2$ représente $-(CH_2)-(CH_2)_e-V-(CH_2)_g-R^{24}$ avec e = 0 ou 1 et g = 0 ou 1, où V représente S, NH, $N(CH_3)$ ou $N(C_2H_5)$ ;
$R^3$ représente un groupe $-C(=O)-R^4$ ;

et

$R^4$ représente $-(CHR^{10})-P_s-(CH_2)_t-(CH_2)_u-Q_v-R^{11}$ avec s = 0 ou 1, t = 0 ou 1, u = 0 ou 1 et v = 0 ou 1, où P et Q représentent, indépendamment l'un de l'autre, à chaque fois 0, S, NH, $N(CH_3)$ ou $N(C_2H_5)$ et la somme de s et v est égale à 1 ;
représente $-(CR^{12}R^{13})-(CH_2)_w-(CH_2)_x-C(=O)-O-R^7$ avec w = 0 ou 1 et x = 0 ou 1 ;

représente $-(CR^{14}R^{15})-(CH_2)_y-O-C(=O)-R^8$ avec y = 0 ou 1 ;
représente $-(CHR^{16})-(CH_2)_z-C(=O)-R^9$ avec z = 0 ou 1 ;
représente $-CH[(-CH_2)-T_a-(CH_2)_b-R^{17}][NH-C(=O)-O-R^{18}]$ avec a = 0 ou 1 et b = 0 ou 1, où T représente O, S, NH, $N(CH_3)$ ou $N(C_2H_5)$ ;
représente $-CHR^{19}R^{20}$ ou $-(CH_2)-CHR^{19}R^{20}$ ;
représente $-(CH=CH)-R^{21}$ ;
représentent un radical $C_{1-10}$-alkyle, $C_{2-10}$-alcényle ou $C_{2-10}$-alcynyle à chaque fois linéaire ou ramifié, le cas échéant à chaque fois substitué ;
représente un radical cycloaliphatique insaturé ou saturé, le cas échéant substitué, de 3, 4, 5, 6, 7, 8 ou 9 chaînons, qui peut être condensé avec un système monocyclique saturé ou insaturé, le cas échéant aromatique, le cas échéant substitué et/ou ponté par un ou deux groupes $C_{1-5}$-alkylène linéaires ou ramifiés, le cas échéant substitués,
ou représente un radical aryle ou hétéroaryle de 5 à 14 chaînons, le cas échéant substitué, qui peut être condensé avec un système monocyclique saturé
ou insaturé, le cas échéant substitué ;

et à chaque fois - pour autant qu'ils soient présents - dans les groupes I.) II.) et III.) les radicaux

$R^5$, $R^6$, $R^9$ et $R^{18}$ indépendamment l'un de l'autre, à chaque fois
représentent un radical $C_{1-10}$-alkyle, $C_{2-10}$-alcényle ou $C_{2-10}$-alcynyle à chaque fois linéaire ou ramifié, le cas échéant à chaque fois substitué ;
$R^7$ représente un radical hydrogène ;
représente un radical $C_{1-10}$-alkyle, $C_{2-10}$-alcényle ou $C_{2-10}$-alcynyle à chaque fois linéaire ou ramifié, le cas échéant à chaque fois substitué ;
ou représente un radical aryle ou hétéroaryle de 5 à 14 chaînons, le cas échéant substitué ;
$R^8$, $R^{16}$ et $R^{24}$ indépendamment l'un de l'autre, à chaque fois
représentent un radical $C_{1-10}$-alkyle, $C_{2-10}$-alcényle
ou $C_{2-10}$-alcynyle à chaque fois linéaire ou ramifié, le cas échéant à chaque fois substitué ;
ou représentent un radical aryle ou hétéroaryle de 5 à 14 chaînons, le cas échéant substitué ;
$R^{10}$, $R^{12}$ et $R^{13}$, indépendamment l'un de l'autre, à chaque fois
représentent un radical hydrogène ;
représentent un radical $C_{1-10}$-alkyle, $C_{2-10}$-alcényle ou $C_{2-10}$-alcynyle à chaque fois linéaire ou ramifié, le cas échéant à chaque fois substitué ;
$R^{11}$ représente un radical $C_{1-10}$-alkyle, $C_{2-10}$-alcényle ou $C_{2-10}$-alcynyle à chaque fois linéaire ou ramifié, le cas échéant à chaque fois substitué ;
représente un radical cycloaliphatique insaturé ou saturé, le cas échéant substitué, de 3, 4, 5, 6, 7, 8 ou 9 chaînons ;
ou représente un radical aryle le cas échéant substitué, présentant 6 ou 10 chaînons ;
$R^{14}$, $R^{15}$ et $R^{19}$, indépendamment l'un de l'autre, à chaque fois
représentent un radical hydrogène ;
représentent un radical $C_{1-10}$-alkyle, $C_{2-10}$-alcényle
ou $C_{2-10}$-alcynyle à chaque fois linéaire ou ramifié, le cas échéant à chaque fois substitué ;
ou représentent un radical aryle le cas échéant substitué, présentant 6 ou 10 chaînons ;
$R^{17}$ et $R^{21}$, indépendamment l'un de l'autre, à chaque fois
représentent un radical aryle le cas échéant substitué, présentant 6 ou 10 chaînons ;
$R^{20}$ représente un radical cycloaliphatique insaturé ou saturé, le cas échéant substitué, de 3, 4, 5, 6, 7, 8 ou 9 chaînons ;
ou représente un radical aryle le cas échéant substitué, présentant 6 ou 10 chaînons ;
$R^{22}$ représente un radical aryle substitué présentant 6 chaînons ;
représente un radical aryle le cas échéant substitué présentant 10 chaînons ;
ou représente un radical hétéroaryle le cas échéant substitué ;

et

$R^{23}$ représente un radical aryle ou hétéroaryle de 5 à 14 chaînons, le cas échéant substitué ;

où, à chaque fois
les radicaux $C_{1-10}$-alkyle susmentionnés peuvent être non substitués ou à chaque fois substitués par 1, 2, 3, 4

ou 5 substituants, choisis indépendamment l'un de l'autre dans le groupe constitué par F, Cl, Br, I, -CN, $-NO_2$, -OH, -SH, $-NH_2$ et $-N(C_{1-5}$-alkyl)($C_{1-5}$-alkyle) ;

les radicaux $C_{2-10}$-alcényle susmentionnés peuvent être non substitués ou à chaque fois substitués par 1, 2, 3, 4 ou 5 substituants, choisis indépendamment l'un de l'autre dans le groupe constitué par F, Cl, Br, I, -CN, $-NO_2$, -OH, -SH, $-NH_2$ et $-N(C_{1-5}$-alkyl)($C_{1-5}$-alkyle) ;

les radicaux $C_{2-10}$-alcynyle susmentionnés peuvent être non substitués ou à chaque fois substitués par 1, 2, 3, 4 ou 5 substituants, choisis indépendamment l'un de l'autre dans le groupe constitué par F, Cl, Br, I, -CN, $-NO_2$, -OH, -SH, $-NH_2$ et $-N(C_{1-5}$-alkyl)($C_{1-5}$-alkyle) ;

les groupes $C_{1-5}$-alkylène susmentionnés peuvent être non substitués ou à chaque fois substitués par 1, 2, 3, 4 ou 5 substituants, choisis indépendamment l'un de l'autre dans le groupe constitué par F, Cl, Br, -OH, -SH, $-NH_2$, -CN, $-NO_2$ et phényle ;

les radicaux cycloaliphatiques susmentionnés peuvent être non substitués ou à chaque fois substitués par 1, 2, 3, 4 ou 5 substituants choisis indépendamment l'un de l'autre dans le groupe constitué par oxo (=O), thioxo (=S), F, Cl, Br, I, -CN, $-CF_3$, $-SF_5$, -OH, -O-$C_{1-5}$-alkyle, $-NH_2$, $-NO_2$, $-O-CF_3$, $-S-CF_3$, -SH, -S-$C_{1-5}$-alkyle, -$C_{1-5}$-alkyle, -C(=O)-H, -C(=O)-$C_{1-5}$-alkyle, -C(=O)-OH, -C(=O)-O-$C_{1-5}$-alkyle, $-(CH_2)$-C(=O)-OH, $-(CH_2)$-C(=O)-O-$C_{1-5}$-alkyle, -NH-$C_{1-5}$-alkyle, -N($C_{1-5}$-alkyle)$_2$, $-(CH_2)$-benzo[b]furannyle, -O-phényle, -O-benzyle, phényle, benzyle, naphtyle et $-(CH_2)$-naphtyle, où la partie cyclique des radicaux -O-phényle, -0-benzyle, phényle, $-(CH_2)$-benzo[b]furannyle, benzyle, naphtyle et $-(CH_2)$-naphtyle peut à chaque fois être substituée par 1, 2, 3, 4 ou 5 substituants choisis indépendamment l'un de l'autre dans le groupe constitué par F, Cl, Br,- OH, $-CF_3$, $-SF_5$, -CN, $-NO_2$, -O-$C_{1-5}$-alkyle, -$C_{1-5}$-alkyle, $-O-CF_3$, $-S-CF_3$, phényle et -O-benzyle,

et les radicaux cycloaliphatiques susmentionnés peuvent à chaque fois présenter 1, 2, 3, 4 ou 5 hétéroatome (s) choisi(s) indépendamment l'un de l'autre dans le groupe constitué par oxygène, azote et soufre ;

le cycle des systèmes monocycliques susmentionnés peut le cas échéant être non substitué ou substitué par 1, 2, 3, 4 ou 5 substituants choisis indépendamment l'un de l'autre dans le groupe constitué par oxo (=O), thioxo (=S), F, Cl, Br, I, -CN, $-CF_3$, $-SF_5$, -OH, -O-$C_{1-5}$-alkyle, -O-$C_{2-5}$-alcényle, $-NH_2$, $-NO_2$, $-O-CF_3$, $-O-CHF_2$, $-O-CH_2F$, $-S-CF_3$, $-S-CHF_2$, $-S-CH_2F$, -SH, -S-$C_{1-5}$-alkyle, -$C_{1-5}$-alkyle, -C(=O)-OH, -C(=O)-O-$C_{1-5}$-alkyle, -O-C (=O)-$C_{1-5}$-alkyle, -O-C(=O)-phényle, $-(CH_2)$-O-C(=O)-$C_{1-5}$-alkyle, $-(CH_2)$-O-C(=O)-phényle, -NH-$C_{1-5}$-alkyle, -N($C_{1-5}$-alkyle)$_2$, -NH-C(=O)-O-$C_{1-5}$-alkyle, -NH-C(=O)-$C_{1-5}$-alkyle, -C(=O)-H, -C(=O)-$C_{1-5}$-alkyle, $-C(=O)-NH_2$, -C(=O)-NH-$C_{1-5}$-alkyle, C(=O)-N-($C_{1-5}$-alkyle)$_2$, $-S(=O)_2-NH_2$, $-S(=O)_2$-NH-$C_{1-5}$-alkyle, $-S(=O)_2$-NH-phényle, $-(CH_2)$-benzo[b]furannyle, -O-phényle, -S-phényle, -S-benzyle, -0-benzyle, phényle et benzyle, la partie cyclique des radicaux -O-C(=O)-phényle, $-(CH_2)$-O-C(=O)-phényle, $-S(=O)_2$-NH-phényle, -0-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, -O-$C_{1-5}$-alkyle, -$C_{1-5}$-alkyle, $-O-CF_3$, $-S-CF_3$, phényle et -O-benzyle,

et le cycle des systèmes monocycliques susmentionnés peut à chaque fois présenter 5, 6 ou 7 chaînons et à chaque fois 1, 2, 3, 4 ou 5 hétéroatome(s) comme élément(s) de cycle, qui sont choisis, indépendamment l'un de l'autre, dans le groupe constitué par oxygène, azote et soufre ;

et, sauf indication contraire, les radicaux aryle ou hétéroaryle susmentionnés peuvent être non substitués ou substitués à chaque fois par 1, 2, 3, 4 ou 5 substituants choisis indépendamment l'un de l'autre dans le groupe constitué par F, Cl, Br, I, -CN, $-CF_3$, $-SF_5$, -OH, -O-$C_{1-5}$-alkyle, -O-$C_{2-5}$-alcényle, $-NH_2$, $-NO_2$, $-O-CF_3$, $-O-CHF_2$, $-O-CH_2F$, $-S-CF_3$, $-S-CHF_2$, $-S-CH_2F$, -SH, -S-$C_{1-5}$-alkyle, -$C_{1-5}$-alkyle, -C(=O)-OH, -C(=O)-O-$C_{1-5}$-alkyle, -O-C(=O)-$C_{1-5}$-alkyle, -O-C(=O)-phényle, $-(CH_2)$-O-C(=O)-$C_{1-5}$-alkyle, $-(CH_2)$-O-C(=O)-phényle, -NH-$C_{1-5}$-alkyle, -N($C_{1-5}$-alkyle)$_2$, -NH-C(=O)-O-$C_{1-5}$-alkyle, -NH-C(=O)-$C_{1-5}$-alkyle, -C(=O)-H, -C(=O)-$C_{1-5}$-alkyle, $-C(=O)-NH_2$, -C(=O)-NH-$C_{1-5}$-alkyle, C(=O)-N-($C_{1-5}$-alkyle)$_2$, $-S(=O)_2-NH_2$, $-S(=O)_2$-NH-$C_{1-5}$-alkyle, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, -O-$C_{1-5}$-alkyle, -$C_{1-5}$-alkyle, $-O-CF_3$, $-S-CF_3$, phényle et -O-benzyle,

et

les radicaux hétéroaryle susmentionnés peuvent à chaque fois présenter 1, 2, 3, 4 ou 5 hétéroatome(s) comme élément(s) de cycle, qui est/sont choisi(s) indépendamment l'un de l'autre dans le groupe constitué par oxygène, azote et soufre ;

le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

**11.** Composés selon la revendication 10,
**caractérisés en ce que**

I.)

$R^1$ représente un radical phényle ou naphtyle, qui est substitué à chaque fois par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, -C (=O) $-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, $-(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$,-CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -0-benzyle,
représente un radical hétéroaryle choisi dans le groupe constitué par thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, oxazolyle, isoxazolyle, pyridazinyle, pyrimidinyle, indazolyle, quinazolinyle, quinoléinyle et isoquinoléinyle, où le radical hétéroaryle peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, - $(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, -S $(=O)_2-NH_2$, $-S(=O)_2-NH-CH_3$, $-S(=O)_2-NH-C_2H_5$, $-S(=O)_2$-NH-phényle, - $(CH_2)$-benzo[b]furannyle, -O-phényle, -S-phényle, -S-benzyle, -0-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,
représente $-(CH_2)_m-O-C(=O)-R^5$ avec m = 1, 2, 3, 4 ou 5 ;
représente $-(CH_2)_n-C(=O)-O-R^6$ avec n = 1, 2, 3, 4 ou 5 ;
représente $-(CH_2)-R^{22}$ ;
ou représente $-(CH_2)-O-R^{23}$, $-(CH_2)-S-R^{23}$, $-(CH_2)-N(CH_3)-R^{23}$, $-(CH_2)-(CH_2)-O-R^{23}$, $-(CH_2)-(CH_2)-S-R^{23}$, $-(CH_2)-(CH_2)-NH-R^{23}$, $-(CH_2)-(CH_2)-N(CH_3)-R^{23}$, $-(CH_2)-(CH_2)-O-(CH_2)-R^{23}$, $-(CH_2)-(CH_2)-S-(CH_2)-R^{23}$ ou $-(CH_2)-(CH_2)-N(CH_3)-R^{23}$ ;
$R^2$ représente un radical hydrogène ;
représente $-(CH_2)-R^{24}$, $-(CH_2)-O-R^{24}$, $-(CH_2)-S-R^{24}$, $-(CH_2)-N(CH_3)-R^{24}$, $-(CH_2)-(CH_2)-R^{24}$, $-(CH_2)-(CH_2)-O-R^{24}$, $-(CH_2)-(CH_2)-S-R^{24}$, $-(CH_2)-(CH_2)-NH-R^{24}$, $-(CH_2)-(CH_2)-N(CH_3)-R^{24}$, $-(CH_2)-(CH_2)-(CH_2)-R^{24}$, $-(CH_2)-(CH_2)-O-(CH_2)-R^{24}$, $-(CH_2)-(CH_2)-S-(CH_2)-R^{24}$ ou $-(CH_2)-(CH_2)-N(CH_3)-R^{24}$ ;
représente un radical choisi dans le groupe constitué par méthyle, éthyle, n-propyle, isopropyle, n-butyle, sec-butyle, isobutyle, tert-butyle, n-pentyle, sec-pentyle, $-(CH_2)-(CH_2)-(C(CH_3)_3)$, n-hexyle, n-heptyle, n-octyle, $-(CH_2)-(CH)(C_2H_5)-(CH_2)-(CH_2)-(CH_2)-(CH_3)$, vinyle, 1-propényle, 2-propényle, 1-buténylе, 2-buténylе, 3-buténylе, 2-méthyl-2-buténylе, 1-propynyle, 2-propynyle, 1-butynyle, 2-butynyle et 3-butynyle,
où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;

représente un radical choisi dans le groupe constitué par phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, oxazolyle, isoxazolyle, pyridazinyle, pyrimidinyle, indazolyle, quinazolinyle, quinoléinyle et isoquinoléinyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, $-(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C (=0) -NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -0-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -0-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,

$R^3$ représente un groupe $-C(=O)-R^4$ ;

et

$R^4$ représente $-(CHR^{10})-O-R^{11}$, $-(CHR^{10})-S-R^{11}$, $-(CHR^{10})-NH-R^{11}$, $-(CHR^{10})-O-(CH_2)-R^{11}$, $-(CHR^{10})-S-(CH_2)-R^{11}$, $- (CHR^{10})-NH-(CH_2)-R^{11}$, $-(CHR^{10})-N(CH_3)-(CH_2)-R^{11}$, $-(CHR^{10})-(CH_2)-(CH_2)-O-R^{11}$, $-(CHR^{10})-(CH_2)-(CH_2)-S-R^{11}$, $-(CHR^{10})-(CH_2)-(CH_2)-NH-R^{11}$ $-(CHR^{10})-(CH_2)-(CH_2)-N(C_2H_5)-R^{11}$, $-(CHR^{10})-O-(CH_2)-(CH_2)-O-R^{11}$, $-(CHR^{10})-S-(CH_2)-(CH_2)-S-R^{11}$, $-(CHR^{10})-O-(CH_2)-(CH_2)-S-R^{11}$ ou $-(CHR^{10})-S-(CH_2)-(CH_2)-O-R^{11}$,

représente $-(CR^{12}R^{13})-C(=O)-O-R^7$, $-(CR^{12}R^{13})-(CH_2)-C(=O)-O-R^7$ ou $-(CR^{12}R^{13})-(CH_2)-(CH_2)-C(=O)-O-R^7$ ; représente $-(CR^{14}R^{15})-O-C(=O)-R^8$ ou $-(CR^{14}R^{15})-(CH_2) -O-C(=O)-R^8$ ;

représente $-(CHR^{16})-C(=O)-R^9$ ou $-(CHR^{16})-(CH_2)-C(=O)-R^9$ ;

représente $-CH[(CH_2)-O-(CH_2)-R^{17}][NH-C(=O)-O-R^{18}]$ ou $-CH[(CH_2)-S-(CH_2)-R^{17}][NH-C(=O)-O-R^{18}]$ ;

représente $-CHR^{19}R^{20}$ ou $-(CH_2)-CHR^{19}R^{20}$ ;

représente $-(CH=CH)-R^{21}$ ;

représente un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, le radical pouvant à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;

représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et 7,7-diméthyl-2-oxabicyclo[2,2,1]heptyle, où le radical (hétéro)cycloaliphatique peut à chaque fois être substitué par 1, 2, 3, 4 ou 5 substituants choisis indépendamment l'un de l'autre dans le groupe constitué par oxo (=0), thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, méthyle, éthyle, n-propyle, isopropyle, n-butyle, sec-butyle, isobutyle, tert-butyle, -C(=O)-H, $-C(=O)-CH_3$, $-C(=O)-C_2H_5$, -C(=O)-OH, $-C(=O)-O-CH_3$, $-C(=O)-O-C_2H_5$, $-C(=O)-O-C(CH_3)_3$, $-(CH_2)-C(=O)-OH$, $-(CH_2)-C(=O)-O-CH_3$, $-(CH_2)-C(=O)-O-C_2H_5$, $-N(CH_3)_2$, $-N(C_2H_5)_2$, $-N(H)(CH_3)$, $-N(H)(C_2H_5)$, $-(CH_2)$-benzo[b]furannyle, -0-phényle, -0-benzyle, phényle, benzyle, naphtyle et $-(CH_2)$-naphtyle, la partie cyclique des radicaux -O-phényle, -O-benzyle, phényle, $-(CH_2)$-benzo[b]furannyle, benzyle, naphtyle et $-(CH_2)$-naphtyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, méthyle, éthyle, n-propyle, isopropyle, n-butyle, sec-butyle, isobutyle, tert-butyle, $-O-CF_3$, $-S-CF_3$, phényle et -O-benzyle,

ou représente un radical choisi dans le groupe constitué par phényle, naphtyle, (1,3)-benzodioxolyle,

(1,4)-benzodioxanyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrimidinyle, indazolyle, quinazolinyle, quinoléinyle, isoquinoléinyle, phénazinyle, phénothiazinyle, oxadiazolyle,

benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, $-(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -0-benzyle,

ou II.)

$R^1$ représente un radical hydrogène ;

représente un radical choisi dans le groupe constitué par méthyle, éthyle, n-propyle, isopropyle, n-butyle, sec-butyle, isobutyle, tert-butyle, n-pentyle, sec-pentyle, $-(CH_2)-(CH_2)-(C(CH_3)_3)$, n-hexyle, n-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle, 3-butényle, 2-méthyl-2-butényle, 1-propynyle, 2-propynyle, 1-butynyle, 2-butynyle et 3-butynyle ;

ou représente un radical phényle ou benzyle non substitué ;

$R^2$ représente un radical hydrogène ;

représente $-(CH_2)-R^{24}$, $-(CH_2)-O-R^{24}$, $-(CH_2)-S-R^{24}$, $-(CH_2)-N(CH_3)-R^{24}$, $-(CH_2)-(CH_2)-R^{24}$, $-(CH_2)-(CH_2)-O-R^{24}$, $-(CH_2)-(CH_2)-S-R^{24}$, $-(CH_2)-(CH_2)-NH-R^{24}$, $-(CH_2)-(CH_2)-N(CH_3)-R^{24}$, $-(CH_2)-(CH_2)-(CH_2)-R^{24}$, $-(CH_2)-(CH_2)-O-(CH_2)-R^{24}$, $-(CH_2)-(CH_2)-S-(CH_2)-R^{24}$ ou $-(CH_2)-(CH_2)-N(CH_3)-R^{24}$ ;

représente un radical choisi dans le groupe constitué par méthyle, éthyle, n-propyle, isopropyle, n-butyle, sec-butyle, isobutyle, tert-butyle, n-pentyle, sec-pentyle, $-(CH_2)-(CH_2)-(C(CH_3)_3)$, n-hexyle, n-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle, 3-butényle, 2-méthyl-2-butényle, 1-propynyle, 2-propynyle, 1-butynyle, 2-butynyle et 3-butynyle,

où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;

ou représente un radical choisi dans le groupe constitué par phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, oxazolyle, isoxazolyle, pyridazinyle, pyrimidinyle, indazolyle, quinazolinyle, quinoléinyle et isoquinoléinyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, - $(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, - $(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH,

$-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,
$R^3$ représente un groupe $-C(=O)-R^4$,

et

$R^4$ représente $-(CHR^{10})-O-R^{11}$, $-(CHR^{10})-S-R^{11}$, $-(CHR^{10})-NH-R^{11}$, $-(CHR^{10})-O-(CH_2)-R^{11}$, $-(CHR^{10})-S-(CH_2)-R^{11}$, $-(CHR^{10})-NH-(CH_2)-R^{11}$, $-(CHR^{10})-N(CH_3)-(CH_2)-R^{11}$, $-(CHR^{10})-(CH_2)-(CH_2)-O-R^{11}$, $-(CHR^{10})-(CH_2)-(CH_2)-S-R^{11}$, $-(CHR^{10})-(CH_2)-(CH_2)-NH-R^{11}$-$(CHR^{10})-(CH_2)-(CH_2)-N(C_2H_5)-R^{11}$, $-(CHR^{10})-O-(CH_2)-(CH_2)-O-R^{11}$, $-(CHR^{10})-S-(CH_2)-(CH_2)-S-R^{11}$, $-(CHR^{10})-O-(CH_2)-(CH_2)-S-R^{11}$ ou $-(CHR^{10})-S-(CH_2)-(CH_2)-O-R^{11}$,
représente $-(CR^{12}R^{13})-C(=O)-O-R^7$, $-(CR^{12}R^{13})-(CH_2)-C(=O)-O-R^7$ ou $-(CR^{12}R^{13})-(CH_2)-(CH_2)-C(=O)-O-R^7$ ; représente $-(CR^{14}R^{15})-O-C(=O)-R^8$ ou $-(CR^{14}R^{15})-(CH_2)$ $-O-C(=O)-R^8$ ;
représente $-(CHR^{16})-C(=O)-R^9$ ou $-(CHR^{16})-(CH_2)-C(=O)-R^9$ ;
représente $-CH[(CH_2)-O-(CH_2)-R^{17}][NH-C(=O)-O-R^{18}]$ ou $-CH[(CH_2)-S-(CH_2)-R^{17}][NH-C(=O)-O-R^{18}]$ ; représente $-CHR^{19}R^{20}$ ou $-(CH_2)-CHR^{19}R^{20}$ ;
représente $-(CH=CH)-R^{21}$ ;
représente un radical alkyle choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle et $-(CH_2)-(CH)(C_2H_5)-(CH_2)-(CH_2)-(CH_2)-(CH_3)$,
où le radical alkyle est à chaque fois substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;
représente un radical choisi dans le groupe constitué par vinyle, 1-propényle, 2-propényle, 1-buténylе, 2-buténylе, 3-buténylе, 1-propynyle, 2-propynyle, 1-butynyle, 2-butynyle et 3-butynyle,
où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;
représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle,
tétrahydropyrannyle, azépanyle, diazépanyle, dithiolanyle, adamantyle et 7,7-diméthyl-2-oxabicyclo[2,2,1]heptyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, méthyle, éthyle, n-propyle, isopropyle, n-butyle, sec-butyle, isobutyle, tert-butyle, -C(=O)-H, $-C(=O)-CH_3$, $-C(=O)-C_2H_5$, -C(=O)-OH, $-C(=O)-O-CH_3$, $-C(=O)-O-C_2H_5$, $-C(=O)-O-C(CH_3)_3$, $-(CH_2)-C(=O)-OH$, $-(CH_2)-C(=O)-O-CH_3$, $-(CH_2)-C(=O)-O-C_2H_5$, $-N(CH_3)_2$, $-N(C_2H_5)_2$, $-N(H)(CH_3)$, $-N(H)(C_2H_5)$, $-(CH_2)$-benzo[b]furannyle, -O-phényle, -O-benzyle, phényle, benzyle, naphtyle et $-(CH_2)$-naphtyle, la partie cyclique des radicaux -O-phényle, -O-benzyle, phényle, $-(CH_2)$-benzo[b]furannyle, benzyle, naphtyle et $-(CH_2)$-naphtyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, méthyle, éthyle, n-propyle, isopropyle, n-butyle, sec-butyle, isobutyle, tert-butyle, $-O-CF_3$, $-S-CF_3$, phényle et -O-benzyle,
représente un radical phényle, où le radical phényle est à chaque fois substitué par 1, 2, 3, 4
ou 5 substituants choisis indépendamment l'un de l'autre dans le groupe constitué par -CN, $-SF_5$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, - $(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$,- C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl,

Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,

représente un radical choisi dans le groupe constitué par 1,2,3,4,5-pentafluorophényle, 1,2,3,4-tétrafluorophényle, 4-méthyl-3-nitrophényle, 5-méthyl-3-nitrophényle, 6-méthyl-3-nitrophényle et 2-méthyl-3-nitrophényle ;

représente un radical naphtyle non substitué ;

représente un radical choisi dans le groupe constitué par (1,3)-benzodioxolyle et (1,4)-benzodioxanyle, où le radical peut à chaque fois être substitué par 1, 2, 3, 4 ou 5 substituants choisis dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle ;

ou représente un radical hétéroaryle choisi dans le groupe constitué par thiophényle, furannyle, pyrrolyle, pyrazolyle, pyrazinyle, pyrannyle, triazolyle, pyridinyle, imidazolyle, indolyle,

iso-indolyle, benzo[b]furannyle, benzo[b]thiophényle, thiazolyle, thiadiazolyle, oxazolyle, isoxazolyle, pyridazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical hétéroaryle peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, $-(CH_2)-O-C(=O)$-phényle, $-N(CH_3)_2$, $-N(C_2H_5)_2$, $-N(H)(CH_3)$, $-N(H)(C_2H_5)$,$-NH-C(=O)-O-CH_3$,$-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)-O-C(=O)$-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,

ou III.)

$R^1$ représente un radical hydrogène ;

représente un radical choisi dans le groupe constitué par méthyle, éthyle, n-propyle, isopropyle, n-butyle, sec-butyle, isobutyle, tert-butyle, n-pentyle, sec-pentyle, $-(CH_2)-(CH_2)-(C(CH_3)_3)$, n-hexyle, n-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle, 3-butényle, 2-méthyl-2-butényle, 1-propynyle, 2-propynyle, 1-butynyle, 2-butynyle et 3-butynyle ;

représente un radical choisi dans le groupe constitué par phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, oxazolyle, isoxazolyle, pyridazinyle, pyrimidinyle, indazolyle, quinazolinyle, quinoléinyle et isoquinoléinyle, où le radical hétéroaryle peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, - $(CH_2)-O-C(=O)$-phényle, -N $(CH_3)_2$, -N $(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle,- O-benzyle, phényle et benzyle, la partie cyclique des radicaux -O-C(=O)-phényle, $-(CH_2)-O-C(=O)$-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle,

isopropyle, n-butyle, sec-butyle, isobutyle, tert-butyle, $-O-CF_3$, $-S-CF_3$, phényle et -O-benzyle,
représente $-(CH_2)_m-O-C(=O)-R^5$ avec m = 1, 2, 3, 4 ou 5 ;
représente $-(CH_2)_n-C(=O)-O-R^6$ avec n = 1, 2, 3, 4 ou 5 ; représente un radical benzyle non substitué ;
représente $-(CH_2)-R^{22}$ ;
ou représente $-(CH_2)-O-R^{23}$, $-(CH_2)-S-R^{23}$, $-(CH_2)-N(CH_3)-R^{23}$, $-(CH_2)-(CH_2)-O-R^{23}$, $-(CH_2)-(CH_2)-S-R^{23}$, $-(CH_2)-(CH_2)-NH-R^{23}$, $-(CH_2)-(CH_2)-N(CH_3)-R^{23}$, $-(CH_2)-(CH_2)-O-(CH_2)-R^{23}$,$-(CH_2)-(CH_2)-S-(CH_2)-R^{23}$ ou $-(CH_2)-(CH_2)-N(CH_3)-R^{23}$ ;
$R^2$ représente $-(CH_2)-S-R^{24}$, $-(CH_2)-N(CH_3)-R^{29}$, $-(CH_2)-(CH_2)-S-R^{24}$, $-(CH_2)-(CH_2)-NH-R^{24}$, $-(CH_2)-(CH_2)-N(CH_3)-R^{24}$, $-(CH_2)-(CH_2)-S-(CH_2)-R^{24}$ ou $-(CH_2)-(CH_2)-N(CH_3)-R^{24}$ ;
$R^3$ représente un groupe $-C(=O)-R^4$ ;

et

$R^4$ représente $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$, $-(CHR^{10})-(CH_2)-(CH_2)-O-R^{11}$, $-(CHR^{10})-(CH_2)-(CH_2)-S-R^{11}$ ou $-(CHR^{10})-O-(CH_2)-(CH_2)-O-R^{11}$,
représente $-(CR^{12}R^{13})-C(=O)-O-R^7$ ou $-(CR^{12}R^{13})-(CH_2)-C(=O)-O-R^7$ ;
représente $-(CR^{14}R^{15})-O-C(=O)-R^8$ ou $-(CR^{19}R^{15})-(CH_2)-O-C(=O)-R^8$ ;
représente $-(CHR^{16})-C(=O)-R^9$ ;
représente $-CH[(CH_2)-O-(CH_2)-R^{17}][NH-C(=O)-O-R^{18}]$ ;
représente $-CHR^{19}R^{20}$ ou $-(CH_2)-CHR^{19}R^{20}$ ;
représente $-(CH=CH)-R^{21}$ ;
représente un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2-(CH)(C_2H_5)-(CH_2)-(CH_2)-(CH_2)-(CH_3)$, vinyle, 1-propényle, 2-propényle, 1-butényle, 2-butényle et 3-butényle, le radical pouvant à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;
représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle, dithiolanyle, adamantyle et 7,7-diméthyl-2-oxabicyclo[2,2,1]heptyle, où le radical (hétéro)cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, méthyle, éthyle, n-propyle, isopropyle, n-butyle, sec-butyle, isobutyle, tert-butyle, -C(=O)-H, $-C(=O)-CH_3$, $-C(=O)-C_2H_5$, -C(=O)-OH, $-C(=O)-O-CH_3$, $-C(=O)-O-C_2H_5$, $-C(=O)-O-C(CH_3)_3$, $-(CH_2-C(=O)-OH$, $-(CH_2)-C(=O)-O-CH_3$, $-(CH_2)-C(=O)-O-C_2H_5$, $-N(CH_3)_2$, $-N(C_2H_5)_2$, $-N(H)(CH_3)$, $-N(H)(C_2H_5)$,- $(CH_2)$-benzo[b]furannyle, -O-phényle, -O-benzyle, phényle, benzyle, naphtyle et $-(CH_2)$-naphtyle, la partie cyclique des radicaux -O-phényle, -O-benzyle, phényle, $-(CH_2)$-benzo[b]furannyle, benzyle, naphtyle et $-(CH_2)$-naphtyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, méthyle, éthyle, n-propyle, isopropyle, n-butyle, sec-butyle, isobutyle, tert-butyle, $-O-CF_3$, $-S-CF_3$, phényle et -O-benzyle,
représente un radical choisi dans le groupe constitué par phényle, naphtyle, (1,3)-benzodioxolyle, (1,4)-benzodioxanyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, $-(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$,- $NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo

[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,

et à chaque fois - pour autant qu'ils soient présents - dans les groupes I.) II.) et III.) les radicaux

$R^5$, $R^6$, $R^9$ et $R^{18}$, indépendamment l'un de l'autre, à chaque fois
représentent un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;
$R^7$ représente un radical hydrogène ;
représente un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, le radical pouvant à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;
ou représente un radical choisi dans le groupe constitué par phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C(=O)-phényle, $-(CH_2)-O-C(=O)-CH_3$, $-(CH_2)-O-C(=O)-C_2H_5$, $-(CH_2)$-O-C(=O)-phényle, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$,- $S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,
$R^8$ $R^{16}$ et $R^{24}$, indépendamment l'un de l'autre, à chaque fois
représentent un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et $-N(CH_3)(C_2H_5)$ ;
ou représentent un radical choisi dans le groupe constitué par phényle, naphtyle, thiophényle, furannyle, pyrrolyle, pyrazolyle, pyrannyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle, quinoléinyle, isoquinoléinyle, phénazinyle, phénothiazinyle, oxadiazolyle, benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, -O-C (=O)-phényle, $-(CH_2)-O-C(=O)-CH_3$, $-(CH_2)-O-C(=O)-C_2H_5$, $-(CH_2)$-O-C(=O)-phényle, $-N(CH_3)_2$, -N $(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, -NH-C(=O) $C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$, $-S(=O)_2-NH-C_2H_5$, $-S(=O)_2$-NH-phényle, $-(CH_2)$-benzo[b]furannyle, -O-phényle, -S-phényle, -S-benzyle, -O-benzyle, phényle et benzyle, la partie cyclique des radicaux -O-C(=O)-phényle, $-(CH_2)$-O-C(=O)-phényle, $-S(=O)_2$-NH-phényle, -O-phényle, -S-phényle, -S-benzyle, -O-benzyle, phényle, $-(CH_2)$-benzo[b]furannyle et benzyle pouvant à chaque fois être substituée par 1, 2, 3, 4 ou 5 substituants choisis indépendamment l'un de l'autre dans le groupe constitué par F, Cl, Br, -OH, $-CF_3$, $-SF_5$, -CN, $-NO_2$, $-O-CH_3$, $-O-C_2H_5$, $-O-CH(CH_3)_2$, $-O-C(CH_3)_3$, méthyle, éthyle, n-propyle, isopropyle, n-butyle, sec-butyle, isobutyle, tert-butyle, $-O-CF_3$, $-S-CF_3$, phényle et -O-benzyle,

$R^{10}$, $R^{12}$ et $R^{13}$, indépendamment l'un de l'autre, à chaque fois

représentent un radical hydrogène ou représentent un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, -N $(C_2H_5)_2$ et $-N(CH_3)(C_2H_5)$ ;

$R^{11}$ représente un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, le radical pouvant à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et -N $(CH_3)(C_2H_5)$ ;

représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, pyrrolidinyle, pipéridinyle, morpholinyle, pipérazinyle, azépanyle et diazépanyle, où le radical (hétéro)cycloaliphatique peut à chaque fois être substitué par 1, 2, 3, 4 ou 5 substituants choisis indépendamment l'un de l'autre dans le groupe constitué par oxo (=0), thioxo (=S), F, Cl, Br, I,- CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, méthyle, éthyle, n-propyle, isopropyle, n-butyle, sec-butyle, isobutyle, tert-butyle, -C(=O)-H, $-C(=O)-CH_3$, -C $(=O)-C_2H_5$, -C(=O)-OH, $-C(=O)-O-CH_3$, $-C(=O)-O-C_2H_5$ et $-C(=O)-O-C(CH_3)_3$ ;

ou représente un radical phényle ou naphtyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle et phényle ;

$R^{14}$, $R^{15}$ et $R^{19}$, indépendamment l'un de l'autre, à chaque fois

représentent un radical hydrogène ;

représentent un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, n-octyle, $-(CH_2)-(CH)(C_2H_5)-(CH_2)-(CH_2)-(CH_2)-(CH_3)$, vinyle, 1-propényle, 2-propényle, 1-butényle, 2-butényle et 3-butényle, où le radical peut à chaque fois être substitué par 1, 2, 3, 4 ou 5 substituants choisis indépendamment l'un de l'autre dans le groupe constitué par F, Cl, Br, I, -CN, $-NO_2$, -OH, -SH, $-NH_2$, $-N(CH_3)_2$, $-N(C_2H_5)_2$ et -N $(CH_3)(C_2H_5)$ ;

ou représentent un radical phényle ou naphtyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH,- $O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle

et tert-butyle ;

$R^{20}$ représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, imidazolidinyle, pyrrolidinyle, pipéridinyle, morpholinyle, pipérazinyle, azépanyle et diazépanyle, où le radical (hétéro) cycloaliphatique peut à chaque fois être substitué par 1, 2, 3, 4 ou 5 substituants choisis indépendamment l'un de l'autre dans le groupe constitué par oxo (=O), thioxo (=S), F, Cl, Br, I, -CN, $-CF_3$, $-SF_5$, -OH, $-O-CH_3$, $-O-C_2H_5$, $-O-C(CH_3)_3$, $-NH_2$, $-NO_2$, $-O-CF_3$, $-S-CF_3$, -SH, $S-CH_3$, $-S-C_2H_5$, $-S-C(CH_3)_3$, méthyle, éthyle, n-propyle, isopropyle, n-butyle, sec-butyle, isobutyle, tert-butyle, -C(=O)-H, $-C(=O)-CH_3$, $-C(=O)-C_2H_5$, -C(=O)-OH, $-C(=O)-O-CH_3$, $-C(=O)-O-C_2H_5$ et $-C(=O)-O-C(CH_3)_3$ ;

ou représente un radical phényle ou naphtyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle et phényle ;

$R^{17}$ et $R^{21}$, indépendamment l'un de l'autre, à chaque fois

représentent un radical phényle ou naphtyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle et tert-butyle ;

$R^{22}$ représente un radical phényle, où le radical phényle est à chaque fois substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$, $-C(=O)-O-C(CH_3)_3$, $-O-C(=O)-CH_3$, $-O-C(=O)-C_2H_5$, $-N(CH_3)_2$, $-N(C_2H_5)_2$, $-N(H)(CH_3)$, $-N(H)(C_2H_5)$, $-NH-C(=O)-O-CH_3$, $-NH-C(=O)-O-C_2H_5$, $-NH-C(=O)-CH_3$, $-NH-C(=O)-C_2H_5$, -C(=O)-H, $-C(=O)-CH_3$, $-C(=O)-C_2H_5$, $-C(=O)-NH_2$, $-C(=O)-NH-CH_3$, $-C(=O)-NH-C_2H_5$, $-S(=O)_2-NH_2$, $-S(=O)_2-NH-CH_3$ et $-S(=O)_2-NH-C_2H_5$,

représente un radical naphtyle, où le radical naphtyle peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle et tert-butyle ;

ou représente un radical hétéroaryle, choisi dans le groupe constitué par thiophényle, furannyle, pyrrolyle, pyrazolyle, pyrannyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle et quinoléinyle, où le radical hétéroaryle peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$ et $-C(=O)-O-C(CH_3)_3$,

et

$R^{23}$ représente un radical choisi dans le groupe constitué par phényle, naphtyle, thiophényle, furannyle, pyrrolyle, pyrazolyle, pyrannyle, triazolyle, pyridinyle, imidazolyle, indolyle, iso-indolyle, benzo[b]furannyle, benzo[b]thiophényle, thiazolyle, thiadiazolyle, oxazolyle, isoxazolyle, pyridazinyle, pyrazinyle, pyrimidinyle, indazolyle, quinazolinyle et quinoléinyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-SF_5$, -OH, $-O-CH_3$, $-O-C_2H_5$, $-O-CH(CH_3)_2$, $-O-C(CH_3)_3$, $-O-CH_2-CH_2-CH_2-CH_3$, $-O-CH_2-CH=CH_2$, $-NH_2$, $-NO_2$, $-O-CF_3$, $-O-CHF_2$, $-O-CH_2F$, $-S-CF_3$, $-S-CHF_2$, $-S-CH_2F$, -SH, $-S-CH_3$, $-S-C_2H_5$, $-S-CH(CH_3)_2$, $-S-C(CH_3)_3$, $-S-CH_2-CH_2-CH_2-CH_3$, méthyle, éthyle, n-propyle, isopropyle, n-butyle, sec-butyle, isobutyle, tert-butyle, -C(=O)-OH, $-C(=O)-O-CH_3$, $-C(=O)-O-C_2H_5$ et $-C(=O)-O-C(CH_3)_3$.

**12.** Composés selon la revendication 10 ou 11,
**caractérisés en ce que**

I.)

$R^1$ représente $-(CH_2)_m-O-C(=O)-R^5$ avec m = 1, 2, 3 ou 4 ;
représente $-(CH_2)_n-C(=O)-O-R^6$ avec n = 1, 2 ou 3 ;
représente $-(CH_2)-R^{22}$ ;
ou représente $-(CH_2)-(CH_2)-O-R^{23}$, $-(CH_2)-(CH_2)-S-R^{23}$, $-(CH_2)-(CH_2)-NH-R^{23}$ ou $-(CH_2)-(CH_2)-N(CH_3)-R^{23}$ ;
$R^2$ représente un radical hydrogène ;
représente $-(CH_2)-R^{24}$, $-(CH_2)-(CH_2)-R^{24}$, $-(CH_2)-(CH_2)-O-R^{24}$, $-(CH_2)-(CH_2)-S-R^{24}$ ou $-(CH_2)-(CH_2)-(CH_2)-R^{24}$ ;
représente un radical alkyle choisi dans le groupe constitué par méthyle, éthyle, n-propyle, isopropyle, n-butyle, sec-butyle, isobutyle, tert-butyle, n-pentyle et sec-pentyle ;
$R^3$ représente un groupe $-C(=O)-R^4$,

et

$R^4$ représente $-(CHR^{10})-O-R^{11}$, $-(CHR^{10})-S-R^{11}$, $-(CHR^{10})-O-(CH_2)-R^{11}$, $-(CHR^{10})-S-(CH^2)-R^{11}$, $-(CHR^{10})-(CH_2)-(CH_2)-O-R^{11}$, $-(CHR^{10})-(CH_2)-(CH_2)-S-R^{11}$ ou $-(CHR^{10})-O-(CH_2)-(CH_2)-O-R^{11}$,
représente $-(CR^{12}R^{13})-C(=O)-O-R^7$ ou $-(CR^{12}R^{13})-(CH_2)-C(=O)-O-R^7$ ;
représente $-(CR^{14}R^{15})-O-C(=O)-R^8$ ou $-(CR^{14}R^{15})-(CH_2)-O-C(=O)-R^8$ ;
représente $-(CHR^{16})-C(=O)-R^9$ ;
représente $-CH[(CH_2)-O-(CH_2)-R^{17}]$ $[NH-C(=O)-O-R^{18}]$ ;
représente $-CHR^{19}R^{20}$ ou-$(CH_2)-CHR^{19}R^{20}$ ;
représente $-(CH=CH)-R^{21}$ ;
représente un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, $-(CH_2)-N(CH_3)_2$,- $(CH_2)-(CH_2)-N(CH_3)_2$, 1-buté-nyle, 2-butényle et 3-butényle ;
représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, adamantyle et 7,7-diméthyl-3-oxo-2-oxabicyclo[2,2,1]heptyle, où le radical cycloaliphatique peut à chaque fois être substitué par 1, 2, 3, 4 ou 5 substituants méthyle, éthyle et n-propyle ;
ou représente un radical choisi dans le groupe constitué par phényle, naphtyle, (1,3)-benzodioxolyle, (1,4)-benzodioxanyle, thiophényle, furannyle, pyrazolyle, triazolyle, pyridinyle, imidazolyle, indolyle, thiazolyle, thiadiazolyle, oxazolyle, isoxazolyle, pyrimidinyle, oxadiazolyle, benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-O-CH_3$, $-O-C_2H_5$, $-NO_2$, $-O-CF_3$, $-O-CHF_2$, $-O-CH_2F$, $-S-CF_3$, $-S-CHF_2$, $-S-CH_2F$, $-S-CH_3$, $-S-C_2H_5$, $-S-CH(CH_3)_2$, $-S-C(CH_3)_3$, méthyle, éthyle, n-propyle, isopropyle, n-butyle, sec-butyle, isobutyle, tert-butyle, $-(CH_2)-O-C(=O)-CH_3$, $-(CH_2)-O-C(=O)-C_2H_5$, $-(CH_2)-O-C(=O)$-phényle, $-N(CH_3)_2$, $-N(C_2H_5)_2$, $-NH-C(=O)-CH_3$, $-NH-C(=O)-C_2H_5$, $-S(=O)_2-NH_2$, -O-phényle et phényle, la partie cyclique des radicaux -O-phényle et phényle pouvant à chaque fois être substituée par 1, 2, 3, 4 ou 5 substituants choisis indépendamment l'un de l'autre dans le groupe constitué par F, Cl, Br, méthyle, éthyle, n-propyle et isopropyle.

ou II.)

$R^1$ représente un radical hydrogène ;
représente un radical choisi dans le groupe constitué par méthyle, éthyle, n-propyle, isopropyle, n-butyle, sec-butyle, isobutyle, tert-butyle, 1-propényle, 2-propényle, 1-butényle, 2-butényle, 3-butényle, 2-méthyl-2-butényle, 1-propynyle et 2-propynyle ;
ou représente un radical benzyle non substitué ;
$R^2$ représente un radical hydrogène ;
représente $-(CH_2)-R^{24}$, $-(CH_2)-(CH_2)-R^{24}$, $-(CH_2)-(CH_2)-O-R^{24}$, $-(CH_2)-(CH_2)-S-R^{24}$ ou $-(CH_2)-(CH_2)-(CH_2)-R^{24}$ ;
ou représente un radical alkyle choisi dans le groupe constitué par méthyle, éthyle, n-propyle, isopropyle,

n-butyle, sec-butyle, isobutyle, tert-butyle, n-pentyle et sec-pentyle ;
$R^3$ représente un groupe $-C(=O)-R^4$,

et

$R^4$ représente $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$, $-(CHR^{10})-(CH_2)-(CH_2)-O-R^{11}$, $-(CHR^{10})-(CH_2)-(CH_2)-S-R^{11}$ ou $-(CHR^{10})-O-(CH_2)-(CH_2)-O-R^{11}$,
représente $-(CR^{12}R^{13})-C(=O)-O-R^7$ ou $-(CR^{12}R^{13})-(CH_2)-C(=O)-O-R^7$ ;
représente $-(CR^{14}R^{15})-O-C(=O)-R^8$ ou $-(CR^{19}R^{15})-(CH_2)-O-C(=O)-R^8$ ;
représente $-(CHR^{16})-C(=O)-R^9$ ;
représente $-CH[(CH_2)-O-(CH_2)-R^{17}]$ $[NH-C(=O)-O-R^{18}]$ ;
représente $-CHR^{19}R^{20}$ ou $-(CH_2)-CHR^{19}R^{20}$ ;
représente $-(CH=CH)-R^{21}$ ;
représente un radical choisi dans le groupe constitué par $-(CH_2)-N(CH_3)_2$, $-(CH_2)-(CH_2)-N(CH_3)_2$, 1-butényle, 2-butényle et 3-butényle ; représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, adamantyle et 7,7-diméthyl-3-oxo-2-oxabicyclo[2,2,1]heptyle, où le radical cycloaliphatique peut à chaque fois être substitué par 1, 2, 3, 4 ou 5 substituants méthyle, éthyle et n-propyle ; représente un radical phényle, où le radical phényle est à chaque fois substitué par 1, 2, 3, 4
ou 5 substituants choisis indépendamment l'un de l'autre dans le groupe constitué par -CN, $-NO_2$, $-O-CF_3$, $-O-CHF_2$, $-O-CH_2F$, $-S-CF_3$, $-S-CHF_2$, $-S-CH_2F$, $-(CH_2)-O-C(=O)-CH_3$, $-(CH_2)-O-C(=O)-C_2H_5$, $-(CH_2)-O-C(=O)$-phényle, $-N(CH_3)_2$, $-N(C_2H_5)_2$, $-N(H)(CH_3)$, $-N(H)(C_2H_5)$, $-NH-C(=O)-CH_3$, $-NH-C(=O)-C_2H_5$, $-S(=O)_2-NH_2$, $-S(=O)_2-NH-CH_3$, $-S(=O)_2-NH-C_2H_5$ et phényle, la partie cyclique des radicaux $-(CH_2)-O-C(=O)$-phényle et phényle pouvant à chaque fois être substituée par 1, 2, 3, 4 ou 5 substituants choisis indépendamment l'un de l'autre dans le groupe constitué par F, Cl, Br, méthyle, éthyle, n-propyle, isopropyle, n-butyle, sec-butyle, isobutyle et tert-butyle,
représente un radical choisi dans le groupe constitué par 1,2,3,4,5-pentafluorophényle, 1,2,3,4-tétrafluorophényle, 4-méthyl-3-nitrophényle, 5-méthyl-3-nitrophényle, 6-méthyl-3-nitrophényle et 2-méthyl-3-nitrophényle ;
représente un radical naphtyle non substitué ;
représente un radical non substitué choisi dans le groupe constitué par (1,3)-benzodioxolyle et (1,4)-benzodioxanyle,
ou représente un radical hétéroaryle, choisi dans le groupe constitué par thiophényle, furannyle, pyrazolyle, triazolyle, pyridinyle, imidazolyle, indolyle, thiazolyle, thiadiazolyle, oxazolyle, isoxazolyle, pyrimidinyle, oxadiazolyle, benzo[1,2,5]oxadiazolyle et chromanyle, où le radical hétéroaryle peut être substitué par 1, 2, 3, 4 ou 5 substituants choisis indépendamment l'un de l'autre dans le groupe constitué par F, Cl, Br, I, -CN, $-CF_3$, $-O-CH_3$, $-O-C_2H_5$, $-NO_2$, $-O-CF_3$, $-O-CHF_2$, $-O-CH_2F$, $-S-CF_3$, $-S-CHF_2$, $-S-CH_2F$, $-S-CH_3$, $-S-C_2H_5$, $-S-CH(CH_3)_2$, $-S-C(CH_3)_3$, méthyle, éthyle, n-propyle, isopropyle, n-butyle, sec-butyle, isobutyle, tert-butyle, -O-phényle et phényle, la partie cyclique des radicaux -O-phényle et phényle pouvant à chaque fois être substituée par 1, 2, 3, 4 ou 5 substituants choisis indépendamment l'un de l'autre dans le groupe constitué par F, Cl, Br, méthyle, éthyle, n-propyle et isopropyle ;

ou III.)

$R^1$ représente un radical hydrogène ;
représente un radical choisi dans le groupe constitué par méthyle, éthyle, n-propyle, isopropyle, n-butyle, sec-butyle, isobutyle, tert-butyle, 1-propényle, 2-propényle, 1-butényle, 2-butényle, 3-butényle, 2-méthyl-2-butényle, 1-propynyle et 2-propynyle ;
représente $-(CH_2)_m-O-C(=O)-R^5$ avec m = 1, 2, 3 ou 4 ;
représente $-(CH_2)_n-C(=O)-O-R^6$ avec n = 1, 2 ou 3 ;
représente $-(CH_2)_n-C(=O)-O-R^6$ avec n = 1, 2 ou 3 ;
représente un radical benzyle non substitué ;
représente $-(CH_2)-R^{22}$ ;
ou représente $-(CH_2)-(CH_2)-O-R^{23}$, $-(CH_2)-(CH_2)-S-R^{23}$, $-(CH_2)-(CH_2)-NH-R^{23}$ ou $-(CH_2)-(CH_2)-N(CH_3)-R^{23}$ ;
$R^2$ représente $-(CH_2)-(CH_2)-S-R^{24}$ ;
$R^3$ représente un groupe $-C(=O)-R^4$ ;

et

$R^4$ représente $-(CHR^{10})-O-R^{11}$, $-(CHR^{10})-S-R^{11}$, $-(CHR^{10})-O-(CH_2)-R^{11}$, $-(CHR^{10})-S-(CH_2)-R^{11}$, $-(CHR^{10})-(CH_2)-(CH_2)-O-R^{11}$, $-(CHR^{10})-(CH_2)-(CH_2)-S-R^{11}$ ou $-(CHR^{10})-O-(CH_2)-(CH_2)-O-R^{11}$,
représente $-(CR^{12}R^{13})-C(=O)-O-R^7$ ou $-(CR^{12}R^{13})-(CH_2)-C(=O)-O-R^7$ ;
représente $-(CR^{14}R^{15})-O-C(=O)-R^8$ ou $-(CR^{14}R^{15})-(CH_2)$ $-O-C(=O)-R^8$ ;
représente $-(CHR^{16})-C(=O)-R^9$ ;
représente $-CH[(CH_2)-O-(CH_2)-R^{17}]$ $[NH-C(=O)-O-R^{18}]$ ;
représente $-CHR^{19}R^{20}$ ou $-(CH_2)-CHR^{19}R^{20}$ ;
représente $-(CH=CH)-R^{21}$ ;
représente un radical choisi dans le groupe constitué par méthyle, éthyle, n-propyle, isopropyle, n-butyle, sec-butyle, isobutyle, tert-butyle, n-pentyle, 2-pentyle, 3-pentyle, $-(CH_2)-(C(CH_3)_3)$, n-hexyle, 3-hexyle, $-(CH_2)-(CH_2)-(C(CH_3)_3)$, n-heptyle, 3-heptyle, 4-heptyle, $-(CH_2)-N(CH_3)_2$, $-(CH_2)-(CH_2)-N(CH_3)_2$, 1-buté-nyle, 2-butényle et 3-butényle ;
représente un radical (hétéro)cycloaliphatique choisi dans le groupe constitué par cyclopropyle, cyclobutyle, cyclopentyle, cyclohexyle, cycloheptyle, cyclopentényle, cyclohexényle, cycloheptényle, adamantyle et 7,7-diméthyl-3-oxo-2-oxabicyclo[2,2,1]heptyle, où le radical cycloaliphatique peut à chaque fois être substitué par 1, 2, 3, 4 ou 5 substituants méthyle, éthyle et n-propyle ;
ou représente un radical choisi dans le groupe constitué par phényle, naphtyle, (1,3)-benzodioxolyle, (1,4)-benzodioxanyle, thiophényle, furannyle, pyrazolyle, triazolyle, pyridinyle, imidazolyle, indolyle, thiazolyle, thiadiazolyle, oxazolyle, isoxazolyle, pyrimidinyle, oxadiazolyle, benzo[1,2,5]oxadiazolyle et chromanyle, où le radical peut à chaque fois être substitué par 1, 2, 3, 4 ou 5 substituants choisis indépendamment l'un de l'autre dans le groupe constitué par F, Cl, Br, I, -CN, $-CF_3$, $-O-CH_3$, $-O-C_2H_5$, $-NO_2$, $-O-CF_3$, $-O-CHF_2$, $-O-CH_2F$, $-S-CF_3$, $-S-CHF_2$, $-S-CH_2F$, $-S-CH_3$, $-S-C_2H_5$, $-S-CH(CH_3)_2$, $-S-C(CH_3)_3$, méthyle, éthyle, n-propyle, isopropyle, n-butyle, sec-butyle, isobutyle, tert-butyle, $-(CH_2)-O-C(=O)-CH_3$, $-(CH_2)-O-C(=O)-C_2H_5$, $-(CH_2)-O-C(=O)$-phényle, $-N(CH_3)_2$, $-N(C_2H_5)_2$, $-NH-C(=O)-CH_3$, $-NH-C(=O)-C_2H_5$, $-S(=O)_2-NH_2$, -O-phényle et phényle, la partie cyclique des radicaux -0-phényle et phényle pouvant à chaque fois être substituée par 1, 2, 3, 4 ou 5 substituants choisis indépendamment l'un de l'autre dans le groupe constitué par F, Cl, Br, méthyle, éthyle, n-propyle et isopropyle ; et à chaque fois - pour autant qu'ils soient présents - dans les groupes I.) II.) et III.) les radicaux
$R^5$ représente un radical méthyle ou éthyle ;
$R^6$ représente un radical méthyle ou éthyle ;
$R^7$ représente un radical hydrogène
ou un radical méthyle ou éthyle ;
$R^8$ représente un radical méthyle ou éthyle
ou un radical phényle non substitué ;
$R^9$ représente un radical méthyle ou éthyle ;
$R^{10}$ représente un radical hydrogène
ou un radical méthyle ou éthyle ;
$R^{11}$ représente un radical méthyle ou éthyle ;
ou représente un radical phényle, qui peut être substitué par 1, 2, 3, 4 ou 5 substituants choisis indépendamment l'un de l'autre dans le groupe constitué par F, Cl, Br, $-O-CH_3$, $-O-C_2H_5$, $-O-CH(CH_3)_2$, $-O-C(CH_3)$ et phényle ;
$R^{12}$ représente un radical hydrogène ou un radical méthyle ;
$R^{13}$ représente un radical hydrogène ou un radical méthyle ;
$R^{14}$ représente un radical hydrogène ;
représente un radical méthyle ou éthyle
ou un radical phényle non substitué ;
$R^{15}$ représente un radical hydrogène ou un radical méthyle ;
$R^{16}$ représente un radical méthyle ou éthyle
ou un radical phényle non substitué ;
$R^{17}$ représente un radical phényle non substitué ;
$R^{18}$ représente un radical alkyle choisi dans le groupe constitué par méthyle, éthyle, n-propyle, isopropyle, n-butyle, isobutyle et tert-butyle ;
$R^{19}$ représente un radical hydrogène ;
représente un radical méthyle ou éthyle
ou un radical phényle non substitué ;
$R^{20}$ représente un radical choisi dans le groupe constitué par cyclopentyle, cyclohexyle et hydantoïne ;

ou représente un radical phényle, qui peut être substitué par 1, 2, 3, 4 ou 5 substituants choisis indépendamment l'un de l'autre dans le groupe constitué par F, Cl, Br, $-O-CH_3$, $-O-C_2H_5$, $-O-CH(CH_3)_2$, $-O-C(CH_3)_3$ et phényle ;

$R^{21}$ représente un radical phényle, qui peut être substitué par 1, 2, 3, 4 ou 5 substituants choisis indépendamment l'un de l'autre dans le groupe constitué par F, Cl, Br et $-CF_3$.

$R^{22}$ représente un radical phényle, où le radical phényle est à chaque fois substitué par 1, 2, 3, 4 ou 5 substituants choisis indépendamment l'un de l'autre dans le groupe constitué par F, Cl, Br, -CN, $-CF_3$, $-NO_2$, $-O-CF_3$, $-S-CF_3$, méthyle, éthyle, n-propyle, isopropyle, n-butyle, sec-butyle, isobutyle, tert-butyle, -C(=O)-OH, $-C(=O)-O-CH_3$ et $-C(=O)-O-C_2H_5$ ;

ou représente un radical naphtyle non substitué ;

$R^{23}$ représente un radical phényle non substitué

et

$R^{24}$ représente un radical méthyle ou éthyle

ou un radical phényle non substitué.

**13.** Composés substitués de 1,4,8-triazaspiro[4,5]décan-2-one de formule générale I selon l'une ou plusieurs des revendications 1 à 12 choisis dans le groupe constitué par

1. la 3-(S)-benzyl-8-(2-éthylbutyryl)-1,4,8-triazaspiro[4,5]décan-2-one,
2. la 8-(2,4-diméthoxybenzoyl)-3-(S)-(2-méthylsulfanyléthyl)-1,4,8-triazaspiro[4,5]décan-2-one,
3. la 3-(S,R)-benzyl-8-(2-éthylbutyryl)-1,4,8-triazaspiro[4,5]décan-2-one,
4. la 8-acétyl-3-(S)-benzyl-1,4,8-triazaspiro[4,5]décan-2-one,
5. la 3-(S,R)-benzyl-8-(4-chlorobenzoyl)-1,4,8-triazaspiro[4,5]décan-2-one,
6. la 3-(S)-benzyl-8-(4-chlorobenzoyl)-1,4,8-triazaspiro[4,5]décan-2-one,
7. la 8-(2-éthylbutyryl)-3-(S)-(2-méthylsulfanyléthyl)-1,4,8-triazaspiro[4,5]décan-2-one,
8. la 1,3-(S)-dibenzyl-8-(2-éthylbutyryl)-1,4,8-triazaspiro[4,5]décan-2-one,
9. la 8-acétyl-1,3-(S)-dibenzyl-1,4,8-triazaspiro[4,5]décan-2-one,
10. la 3-(S,R)-benzyl-8-(4-chlorobenzoyl)-1-(4-méthoxybenzyl)-1,4,8-triazaspiro[4,5]décan-2-one,
11. la 3-(S,R)-benzyl-8-(4-chlorobenzoyl)-1-(4-fluorobenzyl)-1,4,8-triazaspiro[4,5]décan-2-one,
12. la 1,3-(S,R)-dibenzyl-8-(4-chlorobenzoyl)-1,4,8-triazaspiro[4,5]décan-2-one,
13. la 3-(S,R)-benzyl-8-(2-éthylbutyryl)-1-(4-méthoxybenzyl)-1,4,8-triazaspiro[4,5]décan-2-one,
14. la 3-(S,R)-benzyl-8-(2-éthylbutyryl)-1-(4-fluorobenzyl)-1,4,8-triazaspiro[4,5]décan-2-one,
15. la 1,3-(S,R)-dibenzyl-8-(2-éthylbutyryl)-1,4,8-triazaspiro[4,5]décan-2-one,
16. la 1-benzyl-8-(2-éthylbutyryl)-3-(S)-(2-méthylsulfanyléthyl)-1,4,8-triazaspiro[4,5]décan-2-one,
17. la 1,3-(S)-dibenzyl-8-(4-chlorobenzoyl)-1,4,8-triazaspiro[4,5]décan-2-one,
18. la 1,3-(S)-dibenzyl-8-(2-éthylbutyryl)-1,4,8-triazaspiro[4,5]décan-2-one,
19. la 8-acétyl-1,3-(S)-dibenzyl-1,4,8-triazaspiro[4,5]décan-2-one,
20. la 3-(S,R)-benzyl-8-(4-chlorobenzoyl)-1-(4-méthoxybenzyl)-1,4,8-triazaspiro[4,5]décan-2-one,
21. la 3-(S,R)-benzyl-8-(4-chlorobenzoyl)-1-(4-fluorobenzyl)-1,4,8-triazaspiro[4,5]décan-2-one,
22. la 1,3-(S,R)-dibenzyl-8-(4-chlorobenzoyl)-1,4,8-triazaspiro[4,5]décan-2-one,
23. la 3-(S,R)-benzyl-8-(2-éthylbutyryl)-1-(4-méthoxybenzyl)-1,4,8-triazaspiro[4,5]décan-2-one,
24. la 3-(S,R)-benzyl-8-(2-éthylbutyryl)-1-(4-fluorobenzyl)-1,4,8-triazaspiro[4,5]décan-2-one,
25. la 1,3-(S,R)-dibenzyl-8-(2-éthylbutyryl)-1,4,8-triazaspiro[4,5]décan-2-one,
26. la . 1-benzyl-8-(2-éthylbutyryl)-3-(S)-(2-méthylsulfanyléthyl)-1,4,8-triazaspiro[4,5]décan-2-one,
27. la 1,3-(S)-dibenzyl-8-(4-chlorobenzoyl)-1,4,8-triazaspiro[4,5]décan-2-one,
28. la 1-benzyl-8-(2,4-diméthoxybenzoyl)-3-(S)-(2-méthylsulfanyléthyl)-1,4,8-triazaspiro[4,5]décan-2-one,
29. la 1,3-(S)-dibenzyl-1,4,8-triazaspiro[4,5]décan-2-one,
30. la 1-benzyl-3-(S)-(2-méthylsulfanyléthyl)-1,4,8-triazaspiro[4,5]décan-2-one,
31. la 1,3-(S)-dibenzyl-8-butyryl-1,4,8-triazaspiro[4,5]décan-2-one,
32. la 1,3-(S)-dibenzyl-8-(3-fluoro-4-trifluorométhylbenzoyl)-1,4,8-triazaspiro[4,5]décan-2-one,
33. la 1,3-(S)-dibenzyl-8-(2,3-difluorobenzoyl)-1,4,8-triazaspiro[4,5]décan-2-one,
34. la 1,3-(S)-dibenzyl-8-[2-(4-chlorophénoxy)-acétyl]-1,4,8-triazaspiro[4,5]décan-2-one,
35. la 1,3-(S)-dibenzyl-8-diphénylacétyl-1,4,8-triazaspiro[4,5]décan-2-one,
36. la 1,3-(S)-dibenzyl-8-(2-phénoxyacétyl)-1,4,8-triazaspiro[4,5]décan-2-one,
37. la 1,3-(S)-dibenzyl-8-(3-phénylpropionyl)-1,4,8-triazaspiro[4,5]décan-2-one,

38. la 1,3-(S)-dibenzyl-8-(naphtalène-2-carbonyl)-1,4,8-triazaspiro[4,5]décan-2-one,
39. la 1,3-(S)-dibenzyl-8-(furanne-2-carbonyl)-1,4,8-triazaspiro[4,5]décan-2-one,
40. la 1,3-(S)-dibenzyl-8-(3-méthoxybenzoyl)-1,4,8-triazaspiro[4,5]décan-2-one,
41. la 1,3-(S)-dibenzyl-8-(4-fluorobenzoyl)-1,4,8-triazaspiro[4,5]décan-2-one,
42. la 1-benzyl-8-(4-fluorobenzoyl)-3-(S)-(2-méthylsulfanyléthyl)-1,4,8-triazaspiro[4,5]décan-2-one,
43. la 1,3-(S)-dibenzyl-8-butyryl-1,4,8-triazaspiro[4,5]décan-2-one,
44. la 1,3-(S)-dibenzyl-8-(3-fluoro-4-trifluorométhylbenzoyl)-1,4,8-triazaspiro[4,5]décan-2-one,
45. la 1,3-(S)-dibenzyl-8-(2,3-difluorobenzoyl)-1,4,8-triazaspiro[4,5]décan-2-one,
46. la 1,3-(S)-dibenzyl-8-[2-(4-chlorophénoxy)-acétyl]-1,4,8-triazaspiro[4,5]décan-2-one,
47. la 1,3-(S)-dibenzyl-8-diphénylacétyl-1,4,8-triazaspiro[4,5]décan-2-one,
48. la 1,3-(S)-dibenzyl-8-(2-phénoxyacétyl)-1,4,8-triazaspiro[4,5]décan-2-one,
49. la 1,3-(S)-dibenzyl-8-(3-phénylpropionyl)-1,4,8-triazaspiro[4,5]décan-2-one,
50. la 1,3-(S)-dibenzyl-8-(naphtalène-2-carbonyl)-1,4,8-triazaspiro[4,5]décan-2-one,
51. la 1,3-(S)-dibenzyl-8-(furanne-2-carbonyl)-1,4,8-triazaspiro[4,5]décan-2-one,
52. la 1,3-(S)-dibenzyl-8-(3-méthoxybenzoyl)-1,4,8-triazaspiro[4,5]décan-2-one,
53. la 1,3-(S)-dibenzyl-8-(4-fluorobenzoyl)-1,4,8-triazaspiro[4,5]décan-2-one,
54. la 1-benzyl-8-(4-fluorobenzoyl)-3-(S)-(2-méthylsulfanyléthyl)-1,4,8-triazaspiro[4,5]décan-2-one,
55. le N-[4-(3-isobutyl-2-oxo-1-prop-2-ynyl-1,4,8-triazaspiro[4,5]décane-8-carbonyl)-phényl]-acétamide,
56. la 1-(2-phénoxyéthyl)-8-(thiophène-2-carbonyl)-1,4,8-triazaspiro[4,5]décan-2-one,
57. le 2-(2-oxo-8-pent-4-énoyl-1,4,8-triazaspiro[4,5]déc-1-ylméthyl)-benzonitrile,
58. la 8-(2,4-diméthoxybenzoyl)-1-méthyl-3-(2-méthylsulfanyléthyl)-1,4,8-triazaspiro[4,5]décan-2-one,
59. le 2-[8-(2-éthylbutyryl)-2-oxo-1,4,8-triazaspiro[4,5]déc-1-ylméthyl]-benzonitrile,
60. le 4-(2-isobutyl-3-oxo-1,4,8-triazaspiro[4,5]décane-8-carbonyl)-benzonitrile,
61. la 8-(6-chloropyridine-3-carbonyl)-3-isobutyl-1,4,8-triazaspiro[4,5]décan-2-one,
62. le 2-[8-(2-méthylpentanoyl)-2-oxo-1,4,8-triazaspiro[4,5]déc-1-ylméthyl]-benzonitrile,
63. la 1-benzyl-8-(biphényl-4-carbonyl)-1,4,8-triazaspiro[4,5]décan-2-one,
64. la 3-isobutyl-8-(5-méthylisoxazole-3-carbonyl)-1,4,8-triazaspiro[4,5]décan-2-one,
65. l'ester éthylique de l'acide 3-oxo-3-[2-oxo-1-(2-phénoxyéthyl)-1,4,8-triazaspiro[4,5]déc-8-yl]-propionique,
66. la 8-(2-chlorobenzoyl)-3-isopropyl-1,4,8-triazaspiro[4,5]décan-2-one,
67. la 8-cyclopentanecarbonyl-3-isopropyl-1,4,8-triazaspiro[4,5]décan-2-one,
68. la 8-(furanne-2-carbonyl)-1-méthyl-3-(2-méthylsulfanyléthyl)-1,4,8-triazaspiro[4,5]décan-2-one,
69. la 3-benzyl-8-(2-éthylsulfanylpyridine-3-carbonyl)-1-méthyl-1,4,8-triazaspiro[4,5]décan-2-one,
70. la 3-benzyl-8-(4-chlorobenzoyl)-1,4,8-triazaspiro[4,5]décan-2-one,
71. la 8-(2-benzyloxyacétyl)-3-isobutyl-1,4,8-triazaspiro[4,5]décan-2-one,
72. la 3-benzyl-8-(2-méthoxyacétyl)-1-prop-2-ynyl-1,4,8-triazaspiro[4,5]décan-2-one,
73. la 8-(5-tert-butyl-2-méthyl-2H-pyrazole-3-carbonyl)-3-isobutyl-1,4,8-triazaspiro[4,5]décan-2-one,
74. le 2-{8-[3-(2-chloro-6-fluorophényl)-5-méthylisoxazole-4-carbonyl]-2-oxo-1,4,8-triazaspiro[4,5]déc-1-ylméthyl}-benzonitrile,
75. la 3-benzyl-8-(2-chlorobenzoyl)-1,4,8-triazaspiro[4,5]décan-2-one,
76. la 3-benzyl-8-(3-diméthylaminobenzoyl)-1-méthyl-1,4,8-triazaspiro[4,5]décan-2-one,
77. la 8-(3-méthylbenzoyl)-1-(2-phénoxyéthyl)-1,4,8-triazaspiro[4,5]décan-2-one,
78. la 3-isopropyl-1-(3-méthylbut-2-ényl)-8-(pyridine-4-carbonyl)-1,4,8-triazaspiro[4,5]décan-2-one,
79. la 1-benzyl-8-(4-méthyl-[1,2,3]thiadiazole-5-carbonyl)-1,4,8-triazaspiro[4,5]décan-2-one,
80. le 2-{8-[3-(2-chlorophényl)-acryloyl]-2-oxo-1,4,8-triazaspiro[4,5]déc-1-ylméthyl}-benzonitrile,
81. la 8-(3-chlorobenzoyl)-3-isobutyl-1,4,8-triazaspiro[4,5]décan-2-one,
82. l'ester 2-(2-benzyl-3-oxo-1,4,8-triazaspiro[4,5]déc-8-yl)-2-oxo-1-phényléthylique de l'acide acétique,
83. la 8-(3,5-diméthoxybenzoyl)-3-isobutyl-1,4,8-triazaspiro[4,5]décan-2-one,
84. la 3-benzyl-8-(isoxazole-5-carbonyl)-1,4,8-triazaspiro[4,5]décan-2-one,
85. la 8-(3-chlorothiophène-2-carbonyl)-3-isobutyl-1,4,8-triazaspiro[4,5]décan-2-one,
86. la 3-isopropyl-8-pentafluorobenzoyl-1,4,8-triazaspiro[4,5]décan-2-one,
87. la 8-(2,5-diméthylfuranne-3-carbonyl)-3-isobutyl-1,4,8-triazaspiro[4,5]décan-2-one,
88. la 1-butyl-8-[2-(3,4-diméthoxyphényl)-acétyl]-3-(2-méthylsulfanyléthyl)-1,4,8-triazaspiro[4,5]décan-2-one,
89. la 1-benzyl-3-isopropyl-8-(pyridine-4-carbonyl)-1,4,8-triazaspiro[4,5]décan-2-one,
90. la 1,3-dibenzyl-8-(3-diméthylaminobenzoyl)-1,4,8-triazaspiro[4,5]décan-2-one,
91. la 5-{2-[1-(4-fluorobenzyl)-3-(2-méthylsulfanyléthyl)-2-oxo-1,4,8-triazaspiro[4,5]déc-8-yl]-2-oxoéthyl}-imidazolidine-2,4-dione,
92. la 8-(biphényl-4-carbonyl)-1-(4-fluorobenzyl)-3-isobutyl-1,4,8-triazaspiro[4,5]décan-2-one,
93. le 2-[2-oxo-8-(2-propylpentanoyl)-1,4,8-triazaspiro[4,5]déc-1-ylméthyl]-benzonitrile,

94. le 2-[8-(furanne-2-carbonyl)-3-isopropyl-2-oxo-1,4,8-triazaspiro[4,5]déc-1-ylméthyl]-benzonitrile,
95. la 8-[2-(4-chlorophénoxy)-acétyl]-3-isobutyl-1-prop-2-ynyl-1,4,8-triazaspiro[4,5]décan-2-one,
96. la 1,3-dibenzyl-8-(4-bromobenzoyl)-1,4,8-triazaspiro[4,5]décan-2-one,
97. la 8-(3-difluorométhylsulfanylbenzoyl)-3-isobutyl-1,4,8-triazaspiro[4,5]décan-2-one,
98. la 8-(2,3-diméthylbenzoyl)-3-isopropyl-1,4,8-triazaspiro[4,5]décan-2-one,
99. la 3-benzyl-8-(2,3-diméthylbenzoyl)-1,4,8-triazaspiro[4,5]décan-2-one,
100. la 1-(4-fluorobenzyl)-3-(2-méthylsulfanyléthyl)-8-(pyridine-2-carbonyl)-1,4,8-triazaspiro[4,5]décan-2-one,
101. la 3-benzyl-8-(3,3-diméthylbutyryl)-1,4,8-triazaspiro[4,5]décan-2-one,
102. le 2-[8-(3-diméthylaminobenzoyl)-3-isobutyl-2-oxo-1,4,8-triazaspiro[4,5]déc-1-ylméthyl]-benzonitrile,
103. le 3-[8-(2-méthoxyacétyl)-3-(2-méthylsulfanyléthyl)-2-oxo-1,4,8-triazaspiro[4,5]déc-1-ylméthyl]-benzonitrile,
104. l'ester 2-(3-benzyl-1-méthyl-2-oxo-1,4,8-triazaspiro[4,5]déc-8-yl)-2-oxo-1-phényléthylique de l'acide acétique,
105. le 2-[8-[2-(2-méthoxyéthoxy)-acétyl]-3-(2-méthylsulfanyléthyl)-2-oxo-1,4,8-triazaspiro[4,5]déc-1-ylméthyl]-benzonitrile,
106. la 3-benzyl-8-[3-(2-chloro-6-fluorophényl)-5-méthylisoxazole-4-carbonyl]-1,4,8-triazaspiro[4,5]décan-2-one,
107. la 8-(2-chloro-6-fluorobenzoyl)-3-isopropyl-1,4,8-triazaspiro[4,5]décan-2-one,
108. la 1-méthyl-3-(2-méthylsulfanyléthyl)-8-(2-phénoxyacétyl)-1,4,8-triazaspiro[4,5]décan-2-one,
109. la 8-(2-chloropyridine-3-carbonyl)-1-(4-fluorobenzyl)-3-isobutyl-1,4,8-triazaspiro[4,5]décan-2-one,
110. la 8-(2-éthylbutyryl)-1-méthyl-3-(2-méthylsulfanyléthyl)-1,4,8-triazaspiro[4,5]décan-2-one,
111. la 1-méthyl-3-(2-méthylsulfanyléthyl)-8-(2-phénoxypyridine-3-carbonyl)-1,4,8-triazaspiro[4,5]décan-2-one,
112. la 8-(3-fluorobenzoyl)-1-méthyl-3-(2-méthylsulfanyléthyl)-1,4,8-triazaspiro[4,5]décan-2-one,
113. la 3-benzyl-8-(naphtalène-2-carbonyl)-1,4,8-triazaspiro[4,5]décan-2-one,
114. la 8-cyclohexanecarbonyl-1-(2-phénoxyéthyl)-1,4,8-triazaspiro[4,5]décan-2-one,
115. la 8-(2-phénoxyacétyl)-1-(2-phénoxyéthyl)-1,4,8-triazaspiro[4,5]décan-2-one,
116. le 4-[1-butyl-3-(2-méthylsulfanyléthyl)-2-oxo-1,4,8-triazaspiro[4,5]décane-8-carbonyl]-benzonitrile,
117. la 3-benzyl-8-(3,3-diméthylbutyryl)-1-méthyl-1,4,8-triazaspiro[4,5]décan-2-one,
118. la 3-benzyl-8-(4-propylbenzoyl)-1,4,8-triazaspiro[4,5]décan-2-one,
119. la 3-isopropyl-8-(2-phénoxypropionyl)-1,4,8-triazaspiro[4,5]décan-2-one,
120. la 1-butyl-8-hexanoyl-3-isopropyl-1,4,8-triazaspiro[4,5]décan-2-one,
121. la 8-(4-bromo-3-méthylbenzoyl)-3-isopropyl-1,4,8-triazaspiro[4,5]décan-2-one,
122. la 8-[3-(2-chloro-6-fluorophényl)-5-méthylisoxazole-4-carbonyl]-3-isopropyl-1-(2-phénoxyéthyl)-1,4,8-triazaspiro[4,5]décan-2-one,
123. la 1-(2-fluorobenzyl)-3-isobutyl-8-(3-méthylbenzoyl)-1,4,8-triazaspiro[4,5]décan-2-one,
124. la 8-(2-éthylhexanoyl)-1-(2-phénoxyéthyl)-1,4,8-triazaspiro[4,5]décan-2-one,
125. la 3-benzyl-8-(3,4-difluorobenzoyl)-1,4,8-triazaspiro[4,5]décan-2-one,
126. la 3-benzyl-8-(4-éthoxybenzoyl)-1-méthyl-1,4,8-triazaspiro[4,5]décan-2-one,
127. la 1-benzyl-8-(6-chloropyridine-3-carbonyl)-1,4,8-triazaspiro[4,5]décan-2-one,
128. la 8-(3-diméthylaminobenzoyl)-1-(3,5-diméthylbenzyl)-3-(2-méthylsulfanyléthyl)-1,4,8-triazaspiro[4,5]décan-2-one,
129. le 3-[8-(benzo[1,3]dioxole-5-carbonyl)-3-(2-méthylsulfanyléthyl)-2-oxo-1,4,8-triazaspiro[4,5]déc-1-ylméthyl]-benzonitrile,
130. la 3-benzyl-1-méthyl-8-pent-4-énoyl-1,4,8-triazaspiro[4,5]décan-2-one,
131. la 8-(4-éthoxybenzoyl)-1-méthyl-3-(2-méthylsulfanyléthyl)-1,4,8-triazaspiro[4,5]décan-2-one,
132. la 3-benzyl-8-(2-benzyloxyacétyl)-1-méthyl-1,4,8-triazaspiro[4,5]décan-2-one,
133. la 8-(3,4-difluorobenzoyl)-1-(2-phénoxyéthyl)-1,4,8-triazaspiro[4,5]décan-2-one,
134. la 3-benzyl-1-méthyl-8-(2-méthylsulfanylpyridine-3-carbonyl)-1,4,8-triazaspiro[4,5]décan-2-one,
135. la 1-butyl-8-(4-méthoxybenzoyl)-3-(2-méthylsulfanyléthyl)-1,4,8-triazaspiro[4,5]décan-2-one,
136. la 1,3-dibenzyl-8-(2-éthylsulfanylpyridine-3-carbonyl)-1,4,8-triazaspiro[4,5]décan-2-one,
137. la 3-benzyl-8-(3-difluorométhylsulfanylbenzoyl)-1,4,8-triazaspiro[4,5]décan-2-one,
138. la 1-(4-fluorobenzyl)-8-(furanne-2-carbonyl)-3-(2-méthylsulfanyléthyl)-1,4,8-triazaspiro[4,5]décan-2-one,
139. la 3-benzyl-8-(3-fluoro-4-méthylbenzoyl)-1-méthyl-1,4,8-triazaspiro[4,5]décan-2-one,
140. la 8-[3-(2-chloro-6-fluorophényl)-5-méthylisoxazole-4-carbonyl]-3-isopropyl-1,4,8-triazaspiro[4,5]décan-2-one,
141. la 1-butyl-8-(6-chloro-2H-chromane-3-carbonyl)-3-(2-méthylsulfanyléthyl)-1,4,8-triazaspiro[4,5]décan-2-one,

142. le 3-[8-(3-méthoxybenzoyl)-3-(2-méthylsulfanyléthyl)-2-oxo-1,4,8-triazaspiro[4,5]déc-1-ylméthyl]-benzonitrile,
143. la 8-cyclobutanecarbonyl-3-(2-méthylsulfanyléthyl)-1-prop-2-ynyl-1,4,8-triazaspiro[4,5]décan-2-one,
144. la 3-benzyl-1-butyl-8-(furanne-2-carbonyl)-1,4,8-triazaspiro[4,5]décan-2-one,
145. la 1-benzyl-8-(3-chlorothiophène-2-carbonyl)-3-isobutyl-1,4,8-triazaspiro[4,5]décan-2-one,
146. la 3-benzyl-8-(2,5-bistrifluorométhylbenzoyl)-1-butyl-1,4,8-triazaspiro[4,5]décan-2-one,
147. la 8-(3-chloro-2-fluorobenzoyl)-1-(3,5-diméthylbenzyl)-3-(2-méthylsulfanyléthyl)-1,4,8-triazaspiro[4,5]décan-2-one,
148. la 1-benzyl-8-(2-chloropyridine-3-carbonyl)-3-isopropyl-1,4,8-triazaspiro[4,5]décan-2-one,
149. la 3-isobutyl-8-pentafluorobenzoyl-1-prop-2-ynyl-1,4,8-triazaspiro[4,5]décan-2-one,
150. la 8-(2-benzyloxyacétyl)-1-butyl-3-(2-méthylsulfanyléthyl)-1,4,8-triazaspiro[4,5]décan-2-one,
151. la 8-(furanne-2-carbonyl)-3-isobutyl-1-prop-2-ynyl-1,4,8-triazaspiro[4,5]décan-2-one,
152. la 1-butyl-3-(2-méthylsulfanyléthyl)-8-(4-phénoxybutyryl)-1,4,8-triazaspiro[4,5]décan-2-one,
153. la 3-benzyl-8-(6-chloropyridine-3-carbonyl)-1-méthyl-1,4,8-triazaspiro[4,5]décan-2-one,
154. la 1-(2-fluorobenzyl)-3-(2-méthylsulfanyléthyl)-8-(2-phénoxypyridine-3-carbonyl)-1,4,8-triazaspiro[4,5]décan-2-one,
155. la 1-(2-fluorobenzyl)-3-isobutyl-8-(2-méthyl-6-trifluorométhylpyridine-3-carbonyl)-1,4,8-triazaspiro[4,5]décan-2-one,
156. la 8-[2-(3-chlorophénoxy)-acétyl]-3-isopropyl-1-(2-phénoxyéthyl)-1,4,8-triazaspiro[4,5]décan-2-one,
157. la 8-(2,3-diméthylbenzoyl)-1-méthyl-3-(2-méthylsulfanyléthyl)-1,4,8-triazaspiro[4,5]décan-2-one,
158. la 3-isopropyl-8-(2-méthyl-6-trifluorométhylpyridine-3-carbonyl)-1-(2-phénoxyéthyl)-1,4,8-triazaspiro[4,5]décan-2-one,
159. la 3-benzyl-1-méthyl-8-(5-méthyl-2-phényl-2H-[1,2,3]triazole-4-carbonyl)-1,4,8-triazaspiro[4,5]décan-2-one,
160. la 8-[3-(2-chlorophényl)-5-méthylisoxazole-4-carbonyl]-1-(3,5-diméthylbenzyl)-3-(2-méthylsulfanyléthyl)-1,4,8-triazaspiro[4,5]décan-2-one,
161. la 1-(2-fluorobenzyl)-3-(2-méthylsulfanyléthyl)-8-(pyridine-2-carbonyl)-1,4,8-triazaspiro[4,5]décan-2-one,
162. la 1-benzyl-3-(2-méthylsulfanyléthyl)-8-(2-phénoxypyridine-3-carbonyl)-1,4,8-triazaspiro[4,5]décan-2-one,
163. la 1,3-dibenzyl-8-(3-chlorothiophène-2-carbonyl)-1,4,8-triazaspiro[4,5]décan-2-one,
164. la 3-benzyl-8-(4-tert-butylbenzoyl)-1-méthyl-1,4,8-triazaspiro[4,5]décan-2-one,
165. le 2-[3-isobutyl-8-(2-méthoxyacétyl)-2-oxo-1,4,8-triazaspiro[4,5]déc-1-ylméthyl]-benzonitrile,
166. la 3-benzyl-1-butyl-8-(5-fluoro-2-trifluorométhylbenzoyl)-1,4,8-triazaspiro[4,5]décan-2-one,
167. la 3-benzyl-8-[2-(4-méthoxyphényl)-acétyl]-1-méthyl-1,4,8-triazaspiro[4,5]décan-2-one,
168. la 1,3-dibenzyl-8-(4-méthyl-[1,2,3]thiadiazole-5-carbonyl)-1,4,8-triazaspiro[4,5]décan-2-one,
169. la 1-benzyl-3-isopropyl-8-(2-méthyl-6-trifluorométhylpyridine-3-carbonyl)-1,4,8-triazaspiro[4,5]décan-2-one,
170. la 1-benzyl-8-(4-éthoxybenzoyl)-3-isobutyl-1,4,8-triazaspiro[4,5]décan-2-one,
171. la 3-benzyl-1-butyl-8-cyclohexanecarbonyl-1,4,8-triazaspiro[4,5]décan-2-one,
172. le 3-[8-[3-(2-chloro-6-fluorophényl)-5-méthylisoxazole-4-carbonyl]-3-(2-méthylsulfanyléthyl)-2-oxo-1,4,8-triazaspiro[4,5]déc-1-ylméthyl]-benzonitrile,
173. la 1-(2-fluorobenzyl)-3-isobutyl-8-pent-4-énoyl-1,4,8-triazaspiro[4,5]décan-2-one,
174. la 1-méthyl-3-(2-méthylsulfanyléthyl)-8-(2-propylpentanoyl)-1,4,8-triazaspiro[4,5]décan-2-one,
175. la 3-benzyl-1-méthyl-8-(2-phénoxyacétyl)-1,4,8-triazaspiro[4,5]décan-2-one,
176. la 3-isobutyl-1-prop-2-ynyl-8-(3-trifluorométhylbenzoyl)-1,4,8-triazaspiro[4,5]décan-2-one,
177. la 3-benzyl-8-(furanne-2-carbonyl)-1-prop-2-ynyl-1,4,8-triazaspiro[4,5]décan-2-one,
178. la 1-méthyl-3-(2-méthylsulfanyléthyl)-8-(naphtalène-1-carbonyl)-1,4,8-triazaspiro[4,5]décan-2-one,
179. la 3-benzyl-1-butyl-8-(3-cyclopentylpropionyl)-1,4,8-triazaspiro[4,5]décan-2-one,
180. la 1-(3,5-diméthylbenzyl)-3-(2-méthylsulfanyléthyl)-8-pentanoyl-1,4,8-triazaspiro[4,5]décan-2-one,
181. la 3-benzyl-1-butyl-8-(2-méthoxyacétyl)-1,4,8-triazaspiro[4,5]décan-2-one,
182. la 3-benzyl-8-(3-fluoro-4-trifluorométhylbenzoyl)-1-méthyl-1,4,8-triazaspiro[4,5]décan-2-one,
183. la 1-benzyl-8-(3-difluorométhylsulfanylbenzoyl)-3-isopropyl-1,4,8-triazaspiro[4,5]décan-2-one,
184. la 8-(2-chloro-6-fluoro-3-méthylbenzoyl)-1-méthyl-3-(2-méthylsulfanyléthyl)-1,4,8-triazaspiro[4,5]décan-2-one,
185. l'ester méthylique de l'acide 4-[3-isopropyl-8-(2-méthylsulfanylpyridine-3-carbonyl)-2-oxo-1,4,8-triazaspiro[4,5]déc-1-ylméthyl]-benzoïque,
186. la 8-[2-(2,5-diméthoxyphényl)-acétyl]-1-(2-fluorobenzyl)-3-(2-méthylsulfanyléthyl)-1,4,8-triazaspiro[4,5]décan-2-one,

187. la 8-(5-tert-butyl-2-méthylfuranne-3-carbonyl)-3-isopropyl-1-(2-phénoxyéthyl)-1,4,8-triazaspiro[4,5]décan-2-one,
188. la 8-(2-cyclopentylacétyl)-1-(4-fluorobenzyl)-3-isobutyl-1,4,8-triazaspiro[4,5]décan-2-one,
189. l'ester méthylique de l'acide 4-[8-(3,3-diméthylbutyryl)-3-isopropyl-2-oxo-1,4,8-triazaspiro[4,5]déc-1-ylméthyl]-benzoïque,
190. le 3-[8-cyclopropanecarbonyl-3-(2-méthylsulfanyléthyl)-2-oxo-1,4,8-triazaspiro[4,5]déc-1-ylméthyl]-benzonitrile,
191. le 3-[3-(2-méthylsulfanyléthyl)-2-oxo-8-(3-trifluorométhoxybenzoyl)-1,4,8-triazaspiro[4,5]déc-1-ylméthyl]-benzonitrile,
192. la 1-butyl-8-(2-cyclopentylacétyl)-3-isopropyl-1,4,8-triazaspiro[4,5]décan-2-one,
193. la 3-benzyl-1-butyl-8-(pyridine-2-carbonyl)-1,4,8-triazaspiro[4,5]décan-2-one,
194. la 3-benzyl-8-[3-(2-chlorophényl)-acryloyl]-1-méthyl-1,4,8-triazaspiro[4,5]décan-2-one,
195. l'ester méthylique de l'acide 4-[8-(3-fluoro-4-trifluorométhylbenzoyl)-3-isopropyl-2-oxo-1,4,8-triazaspiro[4,5]déc-1-ylméthyl]-benzoïque,
196. la 8-[3-(2,6-dichlorophényl)-5-méthylisoxazole-4-carbonyl]-1-méthyl-3-(2-méthylsulfanyléthyl)-1,4,8-triazaspiro[4,5]décan-2-one,
197. la 1-butyl-8-cyclohexanecarbonyl-3-(2-méthylsulfanyléthyl)-1,4,8-triazaspiro[4,5]décan-2-one,
198. la 3-benzyl-1-butyl-8-(4-iodobenzoyl)-1,4,8-triazaspiro[4,5]décan-2-one
199. la 1-méthyl-3-(2-méthylsulfanyléthyl)-8-[3-(3-trifluorométhylphényl)-acryloyl]-1,4,8-triazaspiro[4,5]décan-2-one,
200. la 1,3-dibenzyl-8-(4-phénoxybutyryl)-1,4,8-triazaspiro[4,5]décan-2-one,
201. la 3-benzyl-8-(2-chloro-6-fluorobenzoyl)-1-prop-2-ynyl-1,4,8-triazaspiro[4,5]décan-2-one,
202. l'ester méthylique de l'acide 4-[8-(2-chloropyridine-3-carbonyl)-3-isopropyl-2-oxo-1,4,8-triazaspiro[4,5]déc-1-ylméthyl]-benzoïque,
203. la 8-(2,5-diméthylfuranne-3-carbonyl)-3-isopropyl-1-(2-phénoxyéthyl)-1,4,8-triazaspiro[4,5]décan-2-one,
204. la 8-(biphényl-4-carbonyl)-3-(2-méthylsulfanyléthyl)-1-prop-2-ynyl-1,4,8-triazaspiro[4,5]décan-2-one,
205. la 8-(3-chlorothiophène-2-carbonyl)-3-(2-méthylsulfanyléthyl)-1-prop-2-ynyl-1,4,8-triazaspiro[4,5]décan-2-one,
206. la 1-(4-fluorobenzyl)-8-[2-(4-méthoxyphényl)-acétyl]-3-(2-méthylsulfanyléthyl)-1,4,8-triazaspiro[4,5]décan-2-one,
207. la 1-benzyl-3-isopropyl-8-(4-méthyl-[1,2,3]thiadiazole-5-carbonyl)-1,4,8-triazaspiro[4,5]décan-2-one,
208. le 2-[3-isopropyl-8-(2-méthylsulfanylpyridine-3-carbonyl)-2-oxo-1,4,8-triazaspiro[4,5]déc-1-ylméthyl]-benzonitrile,
209. le 3-[8-(5-tert-butyl-2-méthyl-2H-pyrazole-3-carbonyl)-3-isobutyl-2-oxo-1,4,8-triazaspiro[4,5]déc-1-ylméthyl]-benzonitrile,
210. la 1-butyl-8-(2,5-diméthylfuranne-3-carbonyl)-3-(2-méthylsulfanyléthyl)-1,4,8-triazaspiro[4,5]décan-2-one,
211. la 8-(3-cyclopentylpropionyl)-1-(2-fluorobenzyl)-3-isobutyl-1,4,8-triazaspiro[4,5]décan-2-one,
212. la 1-benzyl-8-(3-cyclopentylpropionyl)-3-(2-méthylsulfanyléthyl)-1,4,8-triazaspiro[4,5]décan-2-one,
213. la 3-(2-méthylsulfanyléthyl)-8-(4-phénoxybutyryl)-1-prop-2-ynyl-1,4,8-triazaspiro[4,5]décan-2-one,
214. la 1,3-dibenzyl-8-(3-phénylacryloyl)-1,4,8-triazaspiro[4,5]décan-2-one,
215. la 3-benzyl-8-(6-chloro-2-fluoro-3-méthylbenzoyl)-1-méthyl-1,4,8-triazaspiro[4,5]décan-2-one,
216. la 8-[3-(2-chlorophényl)-acryloyl]-3-isopropyl-1-(2-phénoxyéthyl)-1,4,8-triazaspiro[4,5]décan-2-one,
217. le 2-[3-isopropyl-2-oxo-8-(3-phénylacryloyl)-1,4,8-triazaspiro[4,5]déc-1-ylméthyl]-benzonitrile,
218. la 3-benzyl-1-méthyl-8-(4-méthyl-3-nitrobenzoyl)-1,4,8-triazaspiro[4,5]décan-2-one,
219. la 1-benzyl-8-(furanne-2-carbonyl)-3-isopropyl-1,4,8-triazaspiro[4,5]décan-2-one,
220. la 1-butyl-8-(3,5-diméthoxybenzoyl)-3-(2-méthylsulfanyléthyl)-1,4,8-triazaspiro[4,5]décan-2-one,
221. la 1,3-dibenzyl-8-(3,3-diméthylbutyryl)-1,4,8-triazaspiro[4,5]décan-2-one,
222. la 8-(2,6-difluoro-3-méthylbenzoyl)-1-méthyl-3-(2-méthylsulfanyléthyl)-1,4,8-triazaspiro[4,5]décan-2-one,
223. le 2-[8-(2-chloro-6-fluorobenzoyl)-3-(2-méthylsulfanyléthyl)-2-oxo-1,4,8-triazaspiro[4,5]déc-1-ylméthyl]-benzonitrile,
224. la 8-[3-(2-chloro-6-fluorophényl)-5-méthylisoxazole-4-carbonyl]-3-isopropyl-1-(3-méthylbut-2-ényl)-1,4,8-triazaspiro[4,5]décan-2-one,
225. la 3-isobutyl-1-prop-2-ynyl-8-(4-trifluorométhoxybenzoyl)-1,4,8-triazaspiro[4,5]décan-2-one,
226. la 1-benzyl-8-(2-chloro-6-fluoro-3-méthylbenzoyl)-1,4,8-triazaspiro[4,5]décan-2-one,
227. l'ester 2-(1-benzyl-3-isopropyl-2-oxo-1,4,8-triazaspiro[4,5]décane-8-carbonyl)-benzylique de l'acide benzoïque,
228. la 1,3-dibenzyl-8-(2-phénylbutyryl)-1,4,8-triazaspiro[4,5]décan-2-one,

229. la 3-benzyl-1-méthyl-8-(4-nitrobenzoyl)-1,4,8-triazaspiro[4,5]décan-2-one,
230. la 3-benzyl-1-méthyl-8-(5-méthylisoxazole-3-carbonyl)-1,4,8-triazaspiro[4,5]décan-2-one,
231. la 1-benzyl-8-(6-chloro-2-fluoro-3-méthylbenzoyl)-3-(2-méthylsulfanyléthyl)-1,4,8-triazaspiro[4,5]décan-2-one,
232. la 1-benzyl-8-(3-fluoro-4-trifluorométhylbenzoyl)-3-isobutyl-1,4,8-triazaspiro[4,5]décan-2-one,
233. la 3-benzyl-8-(6-chloro-2H-chromane-3-carbonyl)-1-méthyl-1,4,8-triazaspiro[4,5]décan-2-one,
234. la 3-benzyl-1-butyl-8-(4-méthyl-[1,2,3]thiadiazole-5-carbonyl)-1,4,8-triazaspiro[4,5]décan-2-one,
235. la 3-benzyl-8-(4-méthyl-[1,2,3]thiadiazole-5-carbonyl)-1-prop-2-ynyl-1,4,8-triazaspiro[4,5]décan-2-one,
236. le 2-[8-(6-chloro-2H-chromane-3-carbonyl)-3-isopropyl-2-oxo-1,4,8-triazaspiro[4,5]déc-1-ylméthyl]-benzonitrile,
237. le 2-[8-(5-méthylisoxazole-3-carbonyl)-3-(2-méthylsulfanyléthyl)-2-oxo-1,4,8-triazaspiro[4,5]déc-1-ylméthyl]-benzonitrile,
238. le 2-[8-(3-chloro-2-fluorobenzoyl)-3-isopropyl-2-oxo-1,4,8-triazaspiro[4,5]déc-1-ylméthyl]-benzonitrile,
239. l'ester méthylique de l'acide 4-[8-(5-tert-butyl-2-méthyl-2H-pyrazole-3-carbonyl)-3-isopropyl-2-oxo-1,4,8-triazaspiro[4,5]déc-1-ylméthyl]-benzoïque,
240. la 3-benzyl-1-butyl-8-(5-tert-butyl-2-méthyl-2H-pyrazole-3-carbonyl)-1,4,8-triazaspiro[4,5]décan-2-one,
241. la 3-benzyl-1-butyl-8-(2-chloro-4-trifluorométhylpyrimidine-5-carbonyl)-1,4,8-triazaspiro[4,5]décan-2-one,
242. la 3-benzyl-8-(5-méthylisoxazole-3-carbonyl)-1-prop-2-ynyl-1,4,8-triazaspiro[4,5]décan-2-one,
243. l'ester méthylique de l'acide 4-[8-(2-chloropyridine-3-carbonyl)-2-oxo-1,4,8-triazaspiro[4,5]déc-1-ylméthyl]-benzoïque,
244. la 8-(2-tert-butyl-5-méthyl-2H-pyrazole-3-carbonyl)-1-(2-fluorobenzyl)-1,4,8-triazaspiro[4,5]décan-2-one,
245. l'ester méthylique de l'acide 4-[8-(2-méthylsulfanylpyridine-3-carbonyl)-2-oxo-1,4,8-triazaspiro[4,5]déc-1-ylméthyl]-benzoïque,
246. l'ester 4-[8-(4-acétylaminobenzoyl)-3-isobutyl-2-oxo-1,4,8-triazaspiro[4,5]déc-1-yl]-butylique de l'acide acétique,
247. l'ester éthylique de l'acide [8-(4-acétylaminobenzoyl)-3-benzyl-2-oxo-1,4,8-triazaspiro[4,5]déc-1-yl]-acétique,
248. l'ester 4-[8-(2-éthylsulfanylpyridine-3-carbonyl)-3-isobutyl-2-oxo-1,4,8-triazaspiro[4,5]déc-1-yl]-butylique de l'acide acétique,
249. l'ester méthylique de l'acide 4-[8-(4-méthyl-[1,2,3]thiadiazole-5-carbonyl)-2-oxo-1,4,8-triazaspiro[4,5]déc-1-ylméthyl]-benzoïque,
250. le 4-[1-allyl-3-(2-méthylsulfanyléthyl)-2-oxo-1,4,8-triazaspiro[4,5]décane-8-carbonyl]-benzènesulfonamide,
251. l'ester méthylique de l'acide 4-(8-cyclobutanecarbonyl-2-oxo-1,4,8-triazaspiro[4,5]déc-1-ylméthyl)-benzoïque,
252. l'ester 2-[1-allyl-3-(2-méthylsulfanyléthyl)-2-oxo-1,4,8-triazaspiro[4,5]déc-8-yl]-2-oxoéthylique de l'acide acétique,
253. la 8-(biphényl-4-carbonyl)-1-(2-fluorobenzyl)-1,4,8-triazaspiro[4,5]décan-2-one,
254. l'ester éthylique de l'acide [3-(2-méthylsulfanyléthyl)-2-oxo-8-propionyl-1,4,8-triazaspiro[4,5]déc-1-yl]-acétique,
255. la 8-(benzo[1,3]dioxole-5-carbonyl)-1-(2,6-dichlorobenzyl)-3-isobutyl-1,4,8-triazaspiro[4,5]décan-2-one,
256. la 1-allyl-8-(biphényl-4-carbonyl)-3-isopropyl-1,4,8-triazaspiro[4,5]décan-2-one,
257. l'ester éthylique de l'acide [3-benzyl-8-(biphényl-4-carbonyl)-2-oxo-1,4,8-triazaspiro[4,5]déc-1-yl]-acétique,
258. l'ester éthylique de l'acide [8-(3-diméthylaminobenzoyl)-3-(2-méthylsulfanyléthyl)-2-oxo-1,4,8-triazaspiro[4,5]déc-1-yl]-acétique,
259. le 3-(2-oxo-8-pentanoyl-1,4,8-triazaspiro[4,5]déc-1-ylméthyl)-benzonitrile,
260. l'ester méthylique de l'acide 4-(8-cyclopentanecarbonyl-2-oxo-1,4,8-triazaspiro[4,5]déc-1-ylméthyl)-benzoïque,
261. l'ester éthylique de l'acide 4-[1-(2-fluorobenzyl)-2-oxo-1,4,8-triazaspiro[4,5]déc-8-yl]-4-oxobutanoïque,
262. la 1-(2-fluorobenzyl)-8-(3,4,5-triméthoxybenzoyl)-1,4,8-triazaspiro[4,5]décan-2-one,
263. l'ester méthylique de l'acide 4-[8-(2-chloropyridine-4-carbonyl)-2-oxo-1,4,8-triazaspiro[4,5]déc-1-ylméthyl]-benzoïque,
264. le 3-[8-(3,5-bistrifluorométhylbenzoyl)-2-oxo-1,4,8-triazaspiro[4,5]déc-1-ylméthyl]-benzonitrile,
265. la 3-benzyl-1-(2-fluorobenzyl)-8-(2-méthoxybenzoyl)-1,4,8-triazaspiro[4,5]décan-2-one,
266. la 8-(benzo[1,2,5]oxadiazole-5-carbonyl)-1-(2,6-dichlorobenzyl)-3-isobutyl-1,4,8-triazaspiro[4,5]décan-2-one,
267. l'ester éthylique de l'acide [3-benzyl-2-oxo-8-(4-sulfamoylbenzoyl)-1,4,8-triazaspiro[4,5]déc-1-yl]-acétique,
268. le 3-[2-oxo-8-(3,4,5-triméthoxybenzoyl)-1,4,8-triazaspiro[4,5]déc-1-ylméthyl]-benzonitrile,
269. l'ester 2-[1-(4-acétoxybutyl)-3-isobutyl-2-oxo-1,4,8-triazaspiro[4,5]déc-8-yl]-1,1-diméthyl-2-oxoéthylique de l'acide acétique,
270. la 8-(6-chloropyridine-3-carbonyl)-1-(2,6-dichlorobenzyl)-3-isobutyl-1,4,8-triazaspiro[4,5]décan-2-one,
271. la 8-(2-éthoxybenzoyl)-1-(2-fluorobenzyl)-1,4,8-triazaspiro[4,5]décan-2-one,
272. la 1-allyl-8-cyclopropanecarbonyl-3-(2-méthylsulfanyléthyl)-1,4,8-triazaspiro[4,5]décan-2-one,
273. le 3-[3-isopropyl-8-(2-méthoxyacétyl)-2-oxo-1,4,8-triazaspiro[4,5]déc-1-ylméthyl]-benzonitrile,
274. l'ester méthylique de l'acide 4-(2-oxo-8-phénylacétyl-1,4,8-triazaspiro[4,5]déc-1-ylméthyl)-benzoïque,
275. l'ester 2-[1-(3-cyanobenzyl)-2-oxo-1,4,8-triazaspiro[4,5]déc-8-yl]-2-oxo-1-phényléthylique de l'acide acétique,
276. la 1-(2-fluorobenzyl)-8-(4-méthoxybenzoyl)-1,4,8-triazaspiro[4,5]décan-2-one,
277. l'ester méthylique de l'acide 4-(8-cyclohexanecarbonyl-2-oxo-1,4,8-triazaspiro[4,5]déc-1-ylméthyl)-benzoïque,
278. la 1-(2-fluorobenzyl)-8-pent-4-énoyl-1,4,8-triazaspiro[4,5]décan-2-one,
279. l'ester éthylique de l'acide [3-isobutyl-8-(3-méthylbutyryl)-2-oxo-1,4,8-triazaspiro[4,5]déc-1-yl]-acétique,
280. la 1-allyl-8-(3,3-diméthylbutyryl)-3-(2-méthylsulfanyléthyl)-1,4,8-triazaspiro[4,5]décan-2-one,
281. la 1-allyl-3-(2-méthylsulfanyléthyl)-8-(2-méthylsulfanylpyridine-3-carbonyl)-1,4,8-triazaspiro[4,5]décan-2-one,
282. l'ester éthylique de l'acide [3-isobutyl-8-(2-méthylpentanoyl)-2-oxo-1,4,8-triazaspiro[4,5]déc-1-yl]-acétique,
283. l'ester méthylique de l'acide 4-[8-(5-tert-butyl-2-méthylfuranne-3-carbonyl)-2-oxo-1,4,8-triazaspiro[4,5]déc-1-ylméthyl]-benzoïque,
284. l'ester éthylique de l'acide (3-benzyl-8-cyclopropanecarbonyl-2-oxo-1,4,8-triazaspiro[4,5]déc-1-yl)-acétique,
285. l'ester éthylique de l'acide [3-benzyl-8-(3-méthylbutyryl)-2-oxo-1,4,8-triazaspiro[4,5]déc-1-yl]-acétique,
286. la 1-(2,6-dichlorobenzyl)-8-(2,5-diméthylfuranne-3-carbonyl)-3-isobutyl-1,4,8-triazaspiro[4,5]décan-2-one,
287. la 1-allyl-3-isopropyl-8-[2-(3-méthoxyphényl)-acétyl]-1,4,8-triazaspiro[4,5]décan-2-one,
288. l'ester éthylique de l'acide [8-(4-tert-butylbenzoyl)-3-(2-méthylsulfanyléthyl)-2-oxo-1,4,8-triazaspiro[4,5]déc-1-yl]-acétique,
289. la 3-(2-méthylsulfanyléthyl)-1-(2-nitrobenzyl)-8-(2-phénoxypyridine-3-carbonyl)-1,4,8-triazaspiro[4,5]décan-2-one,
290. l'ester éthylique de l'acide (3-benzyl-2-oxo-8-pentanoyl-1,4,8-triazaspiro[4,5]déc-1-yl)-acétique,
291. la 8-(2-chloropyridine-4-carbonyl)-1-(2-fluorobenzyl)-1,4,8-triazaspiro[4,5]décan-2-one,
292. l'ester éthylique de l'acide [8-(3-méthylbutyryl)-3-(2-méthylsulfanyléthyl)-2-oxo-1,4,8-triazaspiro[4,5]déc-1-yl]-acétique,
293. la 1-allyl-3-(2-méthylsulfanyléthyl)-8-pentafluorobenzoyl-1,4,8-triazaspiro[4,5]décan-2-one,
294. la 1-(2-fluorobenzyl)-8-(2-trifluorométhylbenzoyl)-1,4,8-triazaspiro[4,5]décan-2-one,
295. la 8-(benzo[1,2,5]oxadiazole-5-carbonyl)-1-(2-fluorobenzyl)-1,4,8-triazaspiro[4,5]décan-2-one,
296. l'ester méthylique de l'acide 4-[8-(4-tert-butylbenzoyl)-2-oxo-1,4,8-triazaspiro[4,5]déc-1-ylméthyl]-benzoïque,
297. l'ester éthylique de l'acide [3-benzyl-2-oxo-8-(1-phényl-5-propyl-1H-pyrazole-4-carbonyl)-1,4,8-triazaspiro[4,5]déc-1-yl]-acétique,
298. la 1-allyl-3-isopropyl-8-pent-4-énoyl-1,4,8-triazaspiro[4,5]décan-2-one,
299. le 3-[2-oxo-8-(2-trifluorométhylbenzoyl)-1,4,8-triazaspiro[4,5]déc-1-ylméthyl]-benzonitrile,
300. la 8-(2-diméthylaminoacétyl)-1-(3,5-diméthylbenzyl)-3-isobutyl-1,4,8-triazaspiro[4,5]décan-2-one,
301. l'ester 4-[8-(2-éthoxybenzoyl)-3-isobutyl-2-oxo-1,4,8-triazaspiro[4,5]déc-1-yl]-butylique de l'acide acétique,
302. la 1-(2-fluorobenzyl)-8-(3-méthoxybenzoyl)-1,4,8-triazaspiro[4,5]décan-2-one,
303. l'ester éthylique de l'acide [8-(furanne-2-carbonyl)-3-isobutyl-2-oxo-1,4,8-triazaspiro[4,5]déc-1-yl]-acétique,
304. la 8-(5-tert-butyl-2-méthyl-2H-pyrazole-3-carbonyl)-1-(2-fluorobenzyl)-1,4,8-triazaspiro[4,5]décan-2-one,
305. la 1-(2-fluorobenzyl)-8-(thiophène-2-carbonyl)-1,4,8-triazaspiro[4,5]décan-2-one,
306. l'ester éthylique de l'acide [3-benzyl-8-(2-fluorobenzoyl)-2-oxo-1,4,8-triazaspiro[4,5]déc-1-yl]-acétique,
307. l'ester éthylique de l'acide [8-(isoxazole-5-carbonyl)-3-(2-méthylsulfanyléthyl)-2-oxo-1,4,8-triazaspiro[4,5]déc-1-yl]-acétique,
308. la 1-(2,6-dichlorobenzyl)-3-isobutyl-8-(4-propylbenzoyl)-1,4,8-triazaspiro[4,5]décan-2-one,

309. l'ester éthylique de l'acide [8-[2-(2,5-diméthoxyphényl)-acétyl]-3-(2-méthylsulfanyléthyl)-2-oxo-1,4,8-triazaspiro[4,5]déc-1-yl]-acétique,
310. l'ester éthylique de l'acide (3-benzyl-8-cyclobutanecarbonyl-2-oxo-1,4,8-triazaspiro[4,5]déc-1-yl)-acétique,
311. la 1-(2-fluorobenzyl)-8-[2-(4-méthoxyphényl)-acétyl]-1,4,8-triazaspiro[4,5]décan-2-one,
312. la 1-allyl-3-(2-méthylsulfanyléthyl)-8-(2-méthyl-6-trifluorométhylpyridine-3-carbonyl)-1,4,8-triazaspiro[4,5]décan-2-one,
313. l'ester éthylique de l'acide [3-(2-méthylsulfanyléthyl)-2-oxo-8-(4-trifluorométhylbenzoyl)-1,4,8-triazaspiro[4,5]déc-1-yl]-acétique,
314. la 1-allyl-3-(2-méthylsulfanyléthyl)-8-(1-phényl-5-propyl-1H-pyrazole-4-carbonyl)-1,4,8-triazaspiro[4,5]décan-2-one,
315. la 1-(2,6-dichlorobenzyl)-8-(2,3-difluoro-4-méthylbenzoyl)-3-isobutyl-1,4,8-triazaspiro[4,5]décan-2-one,
316. la 1-allyl-3-isopropyl-8-(2-méthylbenzoyl)-1,4,8-triazaspiro[4,5]décan-2-one,
317. la 8-(2-chloro-5-trifluorométhylbenzoyl)-1-(2,6-dichlorobenzyl)-3-isobutyl-1,4,8-triazaspiro[4,5]décan-2-one,
318. la 1-allyl-8-[2-(3-méthoxyphényl)-acétyl]-3-(2-méthylsulfanyléthyl)-1,4,8-triazaspiro[4,5]décan-2-one,
319. la 1-allyl-3-(2-méthylsulfanyléthyl)-8-(3-phénylpropionyl)-1,4,8-triazaspiro[4,5]décan-2-one,
320. l'ester éthylique de l'acide [3-benzyl-8-(2-benzyloxyacétyl)-2-oxo-1,4,8-triazaspiro[4,5]déc-1-yl]-acétique,
321. la 1-allyl-8-(5-tert-butyl-2-méthyl-2H-pyrazole-3-carbonyl)-3-(2-méthylsulfanyléthyl)-1,4,8-triazaspiro[4,5]décan-2-one,
322. l'ester 1,1-diméthyl-2-[3-(2-méthylsulfanyléthyl)-1-(2-nitrobenzyl)-2-oxo-1,4,8-triazaspiro[4,5]déc-8-yl]-2-oxoéthylique de l'acide acétique,
323. l'ester 2-[1-éthoxycarbonylméthyl-3-(2-méthylsulfanyléthyl)-2-oxo-1,4,8-triazaspiro[4,5]décane-8-carbonyl]-benzylique de l'acide benzoïque,
324. la 1-allyl-3-isopropyl-8-(4-méthyl-[1,2,3]thiadiazole-5-carbonyl)-1,4,8-triazaspiro[4,5]décan-2-one,
325. la 1-allyl-8-(2,5-diméthylfuranne-3-carbonyl)-3-(2-méthylsulfanyléthyl)-1,4,8-triazaspiro[4,5]décan-2-one,
326. la 1-allyl-8-(benzo[1,2,5]oxadiazole-5-carbonyl)-3-(2-méthylsulfanyléthyl)-1,4,8-triazaspiro[4,5]décan-2-one,
327. la 1-(2-fluorobenzyl)-8-(4-phénoxybutyryl)-1,4,8-triazaspiro[4,5]décan-2-one,
328. la 1-allyl-8-(2-cyclopentylacétyl)-3-(2-méthylsulfanyléthyl)-1,4,8-triazaspiro[4,5]décan-2-one,
329. l'ester éthylique de l'acide [3-benzyl-8-(naphtalène-2-carbonyl)-2-oxo-1,4,8-triazaspiro[4,5]déc-1-yl]-acétique,
330. la 8-[3-(2-chloro-6-fluorophényl)-5-méthylisoxazole-4-carbonyl]-1-(2-fluorobenzyl)-1,4,8-triazaspiro[4,5]décan-2-one,
331. le 3-[8-(3,5-diméthoxybenzoyl)-3-isopropyl-2-oxo-1,4,8-triazaspiro[4,5]déc-1-ylméthyl]-benzonitrile,
332. la 3-(2-méthylsulfanyléthyl)-1-(2-nitrobenzyl)-8-(1-phényl-5-propyl-1H-pyrazole-4-carbonyl)-1,4,8-triazaspiro[4,5]décan-2-one,
333. l'ester éthylique de l'acide {3-benzyl-8-[3-(2-chloro-6-fluorophényl)-5-méthylisoxazole-4-carbonyl]-2-oxo-1,4,8-triazaspiro[4,5]déc-1-yl}-acétique,
334. la 1-(2-fluorobenzyl)-8-(3-fluoro-4-trifluorométhylbenzoyl)-1,4,8-triazaspiro[4,5]décan-2-one,
335. l'ester éthylique de l'acide [8-[1-(4-chlorophényl)-5-trifluorométhyl-1H-pyrazole-4-carbonyl]-3-(2-méthylsulfanyléthyl)-2-oxo-1,4,8-triazaspiro[4,5]déc-1-yl]-acétique,
336. le 3-[8-(naphtalène-1-carbonyl)-2-oxo-1,4,8-triazaspiro[4,5]déc-1-ylméthyl]-benzonitrile,
337. l'ester éthylique de l'acide [8-(3,3-diméthylbutyryl)-3-(2-méthylsulfanyléthyl)-2-oxo-1,4,8-triazaspiro[4,5]déc-1-yl]-acétique,
338. la 8-acétyl-3-(2-méthylsulfanyléthyl)-1-naphtalén-2-ylméthyl-1,4,8-triazaspiro[4,5]décan-2-one,
339. l'ester éthylique de l'acide [3-benzyl-8-(3-cyanobenzoyl)-2-oxo-1,4,8-triazaspiro[4,5]déc-1-yl]-acétique,
340. la 8-(5-tert-butyl-2-méthyl-2H-pyrazole-3-carbonyl)-1-(2,6-dichlorobenzyl)-3-isobutyl-1,4,8-triazaspiro[4,5]décan-2-one,
341. le 4-[3-benzyl-1-(2-fluorobenzyl)-2-oxo-1,4,8-triazaspiro[4,5]décane-8-carbonyl]-benzonitrile,
342. l'ester méthylique de l'acide 4-[3-benzyl-1-(2-fluorobenzyl)-2-oxo-1,4,8-triazaspiro[4,5]déc-8-yl]-4-oxobutyrique,
343. la 3-(2-méthylsulfanyléthyl)-1-naphtalén-2-ylméthyl-8-(3,4,5-triméthoxybenzoyl)-1,4,8-triazaspiro[4,5]décan-2-one,
344. l'ester éthylique de l'acide [3-benzyl-8-(isoxazole-5-carbonyl)-2-oxo-1,4,8-triazaspiro[4,5]déc-1-yl]-acétique,
345. la 8-(3-difluorométhylsulfanylbenzoyl)-1-(2-fluorobenzyl)-1,4,8-triazaspiro[4,5]décan-2-one,
346. l'ester éthylique de l'acide [3-benzyl-8-(2,3-diméthylbenzoyl)-2-oxo-1,4,8-triazaspiro[4,5]déc-1-yl]-acétique,
347. la 1-(2-fluorobenzyl)-8-(4-propylbenzoyl)-1,4,8-triazaspiro[4,5]décan-2-one,
348. le 3-[8-(4-iodobenzoyl)-2-oxo-1,4,8-triazaspiro[4,5]déc-1-ylméthyl]-benzonitrile,
349. la 1-(2-fluorobenzyl)-8-(2-propylpentanoyl)-1,4,8-triazaspiro[4,5]décan-2-one,
350. le 3-[8-(2,6-difluoro-3-méthylbenzoyl)-3-isopropyl-2-oxo-1,4,8-triazaspiro[4,5]déc-1-ylméthyl]-benzonitrile,
351. la 3-(2-méthylsulfanyléthyl)-8-(4-méthyl-[1,2,3]thiadiazole-5-carbonyl)-1-(2-nitrobenzyl)-1,4,8-triazaspiro [4,5]décan-2-one,
352. l'ester 4-[3-isobutyl-8-(4-méthyl-[1,2,3]thiadiazole-5-carbonyl)-2-oxo-1,4,8-triazaspiro[4,5]déc-1-yl]-butylique de l'acide acétique,
353. la 8-[2-(4-chlorophénoxy)-acétyl]-3-(2-méthylsulfanyléthyl)-1-naphtalén-2-ylméthyl-1,4,8-triazaspiro[4,5] décan-2-one,
354. l'ester éthylique de l'acide [3-benzyl-2-oxo-8-(3trifluorométhylbenzoyl)-1,4,8-triazaspiro[4,5]déc-1-yl]-acétique,
355. la 8-(4-bromobenzoyl)-3-(2-méthylsulfanyléthyl)-1-naphtalén-2-ylméthyl-1,4,8-triazaspiro[4,5]décan-2-one,
356. l'ester éthylique de l'acide [8-(2-chloropyridine-4-carbonyl)-3-(2-méthylsulfanyléthyl)-2-oxo-1,4,8-triazaspiro[4,5]déc-1-yl]-acétique,
357. la 1-allyl-8-(3,5-diméthoxybenzoyl)-3-(2-méthylsulfanyléthyl)-1,4,8-triazaspiro[4,5]décan-2-one,
358. le 3-[8-(4-méthyl-3-nitrobenzoyl)-2-oxo-1,4,8-triazaspiro[4,5]déc-1-ylméthyl]-benzonitrile,
359. la 8-(4-tert-butylbenzoyl)-1-(2-fluorobenzyl)-1,4,8-triazaspiro[4,5]décan-2-one,
360. la 1-allyl-3-isopropyl-8-(5-méthylisoxazole-3-carbonyl)-1,4,8-triazaspiro[4,5]décan-2-one,
361. la 1-allyl-3-(2-méthylsulfanyléthyl)-8-(2-trifluorométhylbenzoyl)-1,4,8-triazaspiro[4,5]décan-2-one,
362. l'ester éthylique de l'acide [3-benzyl-8-(3-cyclopentylpropionyl)-2-oxo-1,4,8-triazaspiro[4,5]déc-1-yl]-acétique,
363. la 3-benzyl-8-(3,5-difluorobenzoyl)-1-(2-fluorobenzyl)-1,4,8-triazaspiro[4,5]décan-2-one,
364. l'ester éthylique de l'acide [3-benzyl-8-(5-fluoro-2-trifluorométhylbenzoyl)-2-oxo-1,4,8-triazaspiro[4,5]déc-1-yl]-acétique,
365. l'ester éthylique de l'acide [3-benzyl-2-oxo-8-(3,4,5-triméthoxybenzoyl)-1,4,8-triazaspiro[4,5]déc-1-yl]-acétique,
366. l'ester 2-[1-(2-fluorobenzyl)-2-oxo-1,4,8-triazaspiro[4,5]décane-8-carbonyl]-benzylique de l'acide benzoïque,
367. la 1-(2-fluorobenzyl)-8-(2-méthylbenzoyl)-1,4,8-triazaspiro[4,5]décan-2-one,
368. la 1-(2-fluorobenzyl)-8-(2-phénylbutyryl)-1,4,8-triazaspiro[4,5]décan-2-one,
369. la 1-allyl-8-(6-chloropyridine-3-carbonyl)-3-isopropyl-1,4,8-triazaspiro[4,5]décan-2-one,
370. l'ester éthylique de l'acide [8-(2,5-diméthylfuranne-3-carbonyl)-3-(2-méthylsulfanyléthyl)-2-oxo-1,4,8-triazaspiro[4,5]déc-1-yl]-acétique,
371. l'ester éthylique de l'acide [8-(5-tert-butyl-2-méthyl-2H-pyrazole-3-carbonyl)-3-(2-méthylsulfanyléthyl)-2-oxo-1,4,8-triazaspiro[4,5]déc-1-yl]-acétique,
372. l'ester 2-[1-allyl-3-(2-méthylsulfanyléthyl)-2-oxo-1,4,8-triazaspiro[4,5]décane-8-carbonyl]-benzylique de l'acide benzoïque,
373. l'ester 2-[1-allyl-3-(2-méthylsulfanyléthyl)-2-oxo-1,4,8-triazaspiro[4,5]déc-8-yl]-2-oxo-1-phényléthylique de l'acide acétique,
374. le 3-[8-(2-chloro-6-fluorobenzoyl)-3-isopropyl-2-oxo-1,4,8-triazaspiro[4,5]déc-1-ylméthyl]-benzonitrile,
375. la 3-(2-méthylsulfanyléthyl)-1-(2-nitrobenzyl)-8-(3-phénylpropionyl)-1,4,8-triazaspiro[4,5]décan-2-one,
376. le 3-[8-(2,3-diméthylbenzoyl)-3-isopropyl-2-oxo-1,4,8-triazaspiro[4,5]déc-1-ylméthyl]-benzonitrile,
377. l'ester éthylique de l'acide [8-(5-fluoro-2-trifluorométhylbenzoyl)-3-(2-méthylsulfanyléthyl)-2-oxo-1,4,8-triazaspiro[4,5]déc-1-yl]-acétique,
378. l'ester éthylique de l'acide [8-cyclopentanecarbonyl-3-(2-méthylsulfanyléthyl)-2-oxo-1,4,8-triazaspiro[4,5] déc-1-yl]-acétique,
379. l'ester éthylique de l'acide [3-(2-méthylsulfanyléthyl)-2-oxo-8-(thiophène-2-carbonyl)-1,4,8-triazaspiro[4,5] déc-1-yl]-acétique,
380. la 1-allyl-8-[1-(4-chlorophényl)-5-trifluorométhyl-1H-pyrazole-4-carbonyl]-3-(2-méthylsulfanyléthyl)-1,4,8-triazaspiro[4,5]décan-2-one,
381. la 1-allyl-3-(2-méthylsulfanyléthyl)-8-(naphtalène-1-carbonyl)-1,4,8-triazaspiro[4,5]décan-2-one,
382. la 1-(2-fluorobenzyl)-8-(4-trifluorométhoxybenzoyl)-1,4,8-triazaspiro[4,5]décan-2-one,
383. la 3-benzyl-8-(2-benzyloxyacétyl)-1-(2-fluorobenzyl)-1,4,8-triazaspiro[4,5]décan-2-one,
384. le 3-[8-(2-chloro-6-fluoro-3-méthylbenzoyl)-3-isopropyl-2-oxo-1,4,8-triazaspiro[4,5]déc-1-ylméthyl]-benzonitrile,
385. la 1-allyl-8-(2-chloro-5-trifluorométhylbenzoyl)-3-isopropyl-1,4,8-triazaspiro[4,5]décan-2-one,
386. la 8-(3-méthylbenzoyl)-3-(2-méthylsulfanyléthyl)-1-naphtalén-2-ylméthyl-1,4,8-triazaspiro[4,5]décan-2-one,
387. la 1-(3,5-diméthylbenzyl)-8-(2-éthylbutyryl)-3-isobutyl-1,4,8-triazaspiro[4,5]décan-2-one,
388. la 8-(5-tert-butyl-2-méthyl-2H-pyrazole-3-carbonyl)-3-(2-méthylsulfanyléthyl)-1-(2-nitrobenzyl)-1,4,8-triazaspiro[4,5]décan-2-one,
389. l'ester 2-[1-(3-cyanobenzyl)-3-isopropyl-2-oxo-1,4,8-triazaspiro[4,5]décane-8-carbonyl]-benzylique de l'acide benzoïque,
390. la 8-(4-méthyl-3-nitrobenzoyl)-3-(2-méthylsulfanyléthyl)-1-(2-nitrobenzyl)-1,4,8-triazaspiro[4,5]décan-2-one,
391. la 1-(2,6-dichlorobenzyl)-3-isobutyl-8-(3,4,5-triméthoxybenzoyl)-1,4,8-triazaspiro[4,5]décan-2-one,
392. le 3-{8-[2-(2-bromophényl)-acétyl]-3-isopropyl-2-oxo-1,4,8-triazaspiro[4,5]déc-1-ylméthyl}-benzonitrile,
393. le 3-[8-(2,3-dichlorobenzoyl)-3-isopropyl-2-oxo-1,4,8-triazaspiro[4,5]déc-1-ylméthyl]-benzonitrile,
394. la 1-allyl-8-(6-chloro-2H-chromane-3-carbonyl)-3-isopropyl-1,4,8-triazaspiro[4,5]décan-2-one,
395. l'ester éthylique de l'acide {3-benzyl-8-[2-(4-méthoxyphényl)-acétyl]-2-oxo-1,4,8-triazaspiro[4,5]déc-1-yl}-acétique,
396. le 3-[3-isopropyl-2-oxo-8-(1-phényl-5-propyl-1H-pyrazole-4-carbonyl)-1,4,8-triazaspiro[4,5]déc-1-ylméthyl]-benzonitrile,
397. la 8-(3-cyclopentylpropionyl)-1-(2-fluorobenzyl)-1,4,8-triazaspiro[4,5]décan-2-one,
398. la 1-allyl-8-(3-difluorométhylsulfanylbenzoyl)-3-isopropyl-1,4,8-triazaspiro[4,5]décan-2-one,
399. le 3-[8-(2-chloro-4-nitrobenzoyl)-3-isopropyl-2-oxo-1,4,8-triazaspiro[4,5]déc-1-ylméthyl]-benzonitrile,
400. l'ester éthylique de l'acide [8-(2-éthoxybenzoyl)-3-(2-méthylsulfanyléthyl)-2-oxo-1,4,8-triazaspiro[4,5]déc-1-yl]-acétique,
401. la 8-(2,5-bistrifluorométhylbenzoyl)-1-(2-fluorobenzyl)-1,4,8-triazaspiro[4,5]décan-2-one,
402. la 8-(2-chloropyridine-4-carbonyl)-3-(2-méthylsulfanyléthyl)-1-(2-nitrobenzyl)-1,4,8-triazaspiro[4,5]décan-2-one,
403. l'ester éthylique de l'acide {3-benzyl-8-[3-(2,6-dichlorophényl)-5-méthylisoxazole-4-carbonyl]-2-oxo-1,4,8-triazaspiro[4,5]déc-1-yl}-acétique,
404. la 1-(3,5-diméthylbenzyl)-3-isobutyl-8-(2-trifluorométhylbenzoyl)-1,4,8-triazaspiro[4,5]décan-2-one,
405. la 8-(3,5-diméthoxybenzoyl)-3-(2-méthylsulfanyléthyl)-1-(2-nitrobenzyl)-1,4,8-triazaspiro[4,5]décan-2-one,
406. l'ester 2-[3-benzyl-1-(2-fluorobenzyl)-2-oxo-1,4,8-triazaspiro[4,5]décane-8-carbonyl]-benzylique de l'acide benzoïque,
407. le 3-[8-(benzo[1,2,5]oxadiazole-5-carbonyl)-3-isopropyl-2-oxo-1,4,8-triazaspiro[4,5]déc-1-ylméthyl]-benzonitrile,
408. l'ester éthylique de l'acide [3-(2-méthylsulfanyléthyl)-2-oxo-8-(4-trifluorométhoxybenzoyl)-1,4,8-triazaspiro [4,5]déc-1-yl]-acétique,
409. la 1-allyl-3-isopropyl-8-[2-(4-méthoxyphényl)-acétyl]-1,4,8-triazaspiro[4,5]décan-2-one,
410. la 3-(2-méthylsulfanyléthyl)-1-(2-nitrobenzyl)-8-(4-propylbenzoyl)-1,4,8-triazaspiro[4,5]décan-2-one,
411. la 3-benzyl-8-(4-tert-butylbenzoyl)-1-(2-fluorobenzyl)-1,4,8-triazaspiro[4,5]décan-2-one,
412. la 1-allyl-8-(2,6-difluoro-3-méthylbenzoyl)-3-(2-méthylsulfanyléthyl)-1,4,8-triazaspiro[4,5]décan-2-one,
413. la 8-(3,5-bistrifluorométhylbenzoyl)-1-(2-fluorobenzyl)-1,4,8-triazaspiro[4,5]décan-2-one,
414. le 3-[8-(4-iodobenzoyl)-3-isopropyl-2-oxo-1,4,8-triazaspiro[4,5]déc-1-ylméthyl]-benzonitrile,
415. la 3-(2-méthylsulfanyléthyl)-1-naphtalén-2-ylméthyl-8-(thiophène-2-carbonyl)-1,4,8-triazaspiro[4,5]décan-2-one,
416. l'ester éthylique de l'acide [3-benzyl-8-(2-chloropyridine-4-carbonyl)-2-oxo-1,4,8-triazaspiro[4,5]déc-1-yl]-acétique,
417. l'ester éthylique de l'acide [3-(2-méthylsulfanyléthyl)-8-(4-nitrobenzoyl)-2-oxo-1,4,8-triazaspiro[4,5]déc-1-yl]-acétique,
418. la 8-(2-éthylhexanoyl)-3-(2-méthylsulfanyléthyl)-1-(2-nitrobenzyl)-1,4,8-triazaspiro[4,5]décan-2-one,
419. la 1-(2,6-dichlorobenzyl)-3-isobutyl-8-(thiophène-2-carbonyl)-1,4,8-triazaspiro[4,5]décan-2-one,
420. l'ester éthylique de l'acide [3-benzyl-8-(2-chloro-4-nitrobenzoyl)-2-oxo-1,4,8-triazaspiro[4,5]déc-1-yl]-acétique,
421. la 3-benzyl-8-(3,5-bistrifluorométhylbenzoyl)-1-(2-fluorobenzyl)-1,4,8-triazaspiro[4,5]décan-2-one,
422. l'ester éthylique de l'acide [3-benzyl-8-(4-bromo-3-méthylbenzoyl)-2-oxo-1,4,8-triazaspiro[4,5]déc-1-yl]-acétique,
423. la 8-cyclohexanecarbonyl-3-(2-méthylsulfanyléthyl)-1-(2-nitrobenzyl)-1,4,8-triazaspiro[4,5]décan-2-one,

424. la 8-(2,5-diméthylfuranne-3-carbonyl)-3-(2-méthylsulfanyléthyl)-1-(2-nitrobenzyl)-1,4,8-triazaspiro[4,5]décan-2-one,
425. la 8-(3-difluorométhylsulfanylbenzoyl)-3-(2-méthylsulfanyléthyl)-1-(2-nitrobenzyl)-1,4,8-triazaspiro[4,5]décan-2-one,
426. la 8-(furanne-2-carbonyl)-3-(2-méthylsulfanyléthyl)-1-(2-nitrobenzyl)-1,4,8-triazaspiro[4,5]décan-2-one,
427. l'ester éthylique de l'acide [3-benzyl-8-(2,3-difluorobenzoyl)-2-oxo-1,4,8-triazaspiro[4,5]déc-1-yl]-acétique,
428. le 3-[3-isopropyl-8-(4-méthyl-[1,2,3]thiadiazole-5-carbonyl)-2-oxo-1,4,8-triazaspiro[4,5]déc-1-ylméthyl]-benzonitrile,
429. le 3-[8-(3-chloro-2-fluorobenzoyl)-3-isopropyl-2-oxo-1,4,8-triazaspiro[4,5]déc-1-ylméthyl]-benzonitrile,
430. l'ester éthylique de l'acide [3-benzyl-8-(naphtalène-1-carbonyl)-2-oxo-1,4,8-triazaspiro[4,5]déc-1-yl]-acétique,
431. la 8-(4-tert-butylbenzoyl)-3-(2-méthylsulfanyléthyl)-1-(2-nitrobenzyl)-1,4,8-triazaspiro[4,5]décan-2-one,
432. le 3-[3-benzyl-1-(2-fluorobenzyl)-2-oxo-1,4,8-triazaspiro[4,5]décane-8-carbonyl]-benzonitrile,
433. la 1-(2,6-dichlorobenzyl)-8-(furanne-2-carbonyl)-3-isobutyl-1,4,8-triazaspiro[4,5]décan-2-one,
434. la 8-(2,6-dichlorobenzoyl)-1-(2-fluorobenzyl)-1,4,8-triazaspiro[4,5]décan-2-one,
435. la 1-(3,5-diméthylbenzyl)-8-(3-fluoro-4-trifluorométhylbenzoyl)-3-isobutyl-1,4,8-triazaspiro[4,5]décan-2-one,
436. la 1-(2-fluorobenzyl)-8-(3-fluoro-4-méthylbenzoyl)-1,4,8-triazaspiro[4,5]décan-2-one,
437. la 1-allyl-8-(3-cyclopentylpropionyl)-3-(2-méthylsulfanyléthyl)-1,4,8-triazaspiro[4,5]décan-2-one,
438. la 8-[2-(3-chlorophénoxy)-acétyl]-3-(2-méthylsulfanyléthyl)-1-(2-nitrobenzyl)-1,4,8-triazaspiro[4,5]décan-2-one,
439. la 1-(3,5-diméthylbenzyl)-3-isobutyl-8-(2-méthylsulfanylpyridine-3-carbonyl)-1,4,8-triazaspiro[4,5]décan-2-one,
440. la 3-(2-méthylsulfanyléthyl)-1-(2-nitrobenzyl)-8-propionyl-1,4,8-triazaspiro[4,5]décan-2-one,
441. le 3-[8-(3,4-diméthoxybenzoyl)-3-isopropyl-2-oxo-1,4,8-triazaspiro[4,5]déc-1-ylméthyl]-benzonitrile,
442. la 3-(2-méthylsulfanyléthyl)-8-(naphtalène-2-carbonyl)-1-naphtalén-2-ylméthyl-1,4,8-triazaspiro[4,5]décan-2-one,
443. la 8-(6-chloro-2H-chromane-3-carbonyl)-3-(2-méthylsulfanyléthyl)-1-(2-nitrobenzyl)-1,4,8-triazaspiro[4,5]décan-2-one,
444. l'ester éthylique de l'acide {3-benzyl-2-oxo-8-[3-(3-trifluorométhylphényl)-acryloyl]-1,4,8-triazaspiro[4,5]déc-1-yl}-acétique,
445. la 1-(3,5-diméthylbenzyl)-3-isobutyl-8-(3-phénylacryloyl)-1,4,8-triazaspiro[4,5]décan-2-one,
446. le 3-[3-isopropyl-2-oxo-8-(2-phénoxypropionyl)-1,4,8-triazaspiro[4,5]déc-1-ylméthyl]-benzonitrile,
447. le 3-[3-isopropyl-2-oxo-8-(4-trifluorométhoxybenzoyl)-1,4,8-triazaspiro[4,5]déc-1-ylméthyl]-benzonitrile,
448. la 1-allyl-3-(2-méthylsulfanyléthyl)-8-(3-nitrobenzoyl)-1,4,8-triazaspiro[4,5]décan-2-one,
449. l'ester éthylique de l'acide [3-benzyl-8-(3,4-dichlorobenzoyl)-2-oxo-1,4,8-triazaspiro[4,5]déc-1-yl]-acétique,
450. la 1-(2-fluorobenzyl)-8-[3-(3-trifluorométhylphényl)-acryloyl]-1,4,8-triazaspiro[4,5]décan-2-one,
451. la 1-allyl-8-(3,5-bistrifluorométhylbenzoyl)-3-(2-méthylsulfanyléthyl)-1,4,8-triazaspiro[4,5]décan-2-one,
452. la 8-(3-cyclopentylpropionyl)-3-(2-méthylsulfanyléthyl)-1-(2-nitrobenzyl)-1,4,8-triazaspiro[4,5]décan-2-one,
453. la 1-(3,5-diméthylbenzyl)-3-isobutyl-8-(4-nitrobenzoyl)-1,4,8-triazaspiro[4,5]décan-2-one,
454. la 8-(3-cyclopentylpropionyl)-1-(3,5-diméthylbenzyl)-3-isobutyl-1,4,8-triazaspiro[4,5]décan-2-one,
455. le 3-[3-isopropyl-8-(isoxazole-5-carbonyl)-2-oxo-1,4,8-triazaspiro[4,5]déc-1-ylméthyl]-benzonitrile et
456. la 3-benzyl-1-(2-fluorobenzyl)-8-(2-méthyl-6-trifluorométhylpyridine-3-carbonyl)-1,4,8-triazaspiro[4,5]décan-2-one,

le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants, de préférence le chlorhydrate, ou à chaque fois sous forme de solvates correspondants.

**14.** Procédé pour la préparation de composés substitués de la 1,4,8-triazaspiro[4,5]décan-2-one selon l'une ou plusieurs des revendications 1 à 13,
**caractérisé**
**en ce qu'**on transforme une pipéridin-4-one protégée de formule générale II,

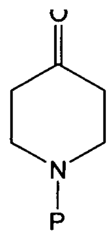

où P représente un groupe de protection, par transformation avec au moins un composé de formule générale III,

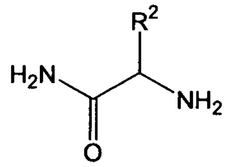

où $R^2$ a la signification selon l'une ou plusieurs des revendications 1 à 13, en au moins un composé de formule générale IV,

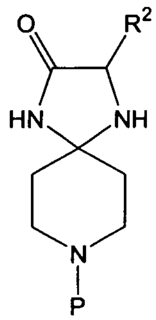

où P et $R^2$ ont la signification susmentionnée, qui est le cas échéant purifié et/ou le cas échéant isolé, et qui est transformé le cas échéant par transformation avec au moins un composé de formule générale $R^1$-$X^1$, où $R^1$ a la signification selon l'une ou plusieurs des revendications 1 à 13 et $X^1$ représente un groupe partant approprié, de préférence un radical halogène, le cas échéant en présence d'au moins une base, en au moins un composé de formule générale V,

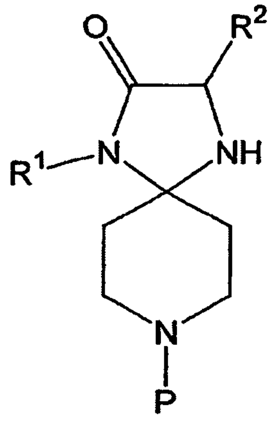

V

où $R^1$, $R^2$ et P ont la signification susmentionnée et qui est le cas échéant purifié et/ou le cas échéant isolé et le cas échéant transformé, par dissociation du groupe de protection P en au moins un composé de formule générale VI,

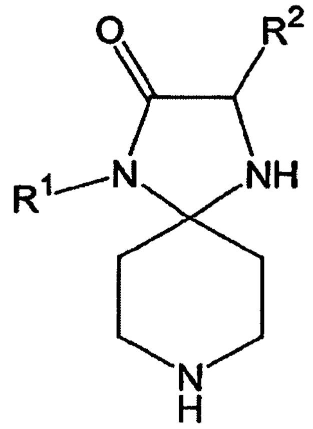

VI

on le purifie le cas échéant et/ou on l'isole le cas échéant et on transforme le cas échéant au moins un composé de formule générale IV, V ou VI, par transformation avec un dérivé d'acide carboxylique de formule générale $R^4$-(C=O)-$X^2$, un anhydride d'acide carboxylique de formule générale $(R^9\text{-(C=O)})_2O$ ou un composé de formule générale $R^3$-$X^3$, où le radical $R^3$ a la signification selon l'une quelconque des revendications 1 à 13, à l'exception de l'hydrogène et de -(C=O)-$R^4$, $R^4$ présente à chaque fois la signification selon l'une ou plusieurs des revendications 1 à 13 et $X^2$ et $X^3$ représentent à chaque fois un groupe partant approprié, de préférence un radical halogène, en au moins un composé selon l'une ou plusieurs des revendications 1 à 13, et on purifie le cas échéant et/ou on isole le cas échéant ce composé,

ou

on transforme la 4-pipéridone de formule VII, le cas échéant sous forme d'un sel correspondant,

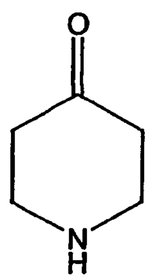

VII

par transformation avec un dérivé d'acide carboxylique de formule générale $R^4$-(C=O)-$X^2$, un anhydride d'acide carboxylique de formule générale $(R^9\text{-(C=O)})_2O$ ou un composé de formule générale $R^3$-$X^3$, où le radical $R^3$ a la signification selon l'une ou plusieurs des revendications 1 à 13, à l'exception de l'hydrogène et de -(C=O)-$R^4$, le radical $R^4$ présente à chaque fois la signification susmentionnée et $X^2$ et $X^3$ représentent à chaque fois un groupe partant approprié, de préférence un radical halogène, en au moins un composé de formule générale VIII,

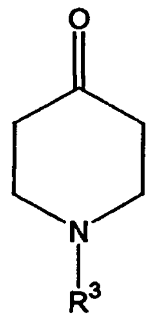

VIII

où $R^3$ a la signification selon l'une ou plusieurs des revendications 1 à 13 et on le purifie et/ou l'isole le cas échéant et on le transforme par transformation avec au moins un composé de formule générale III

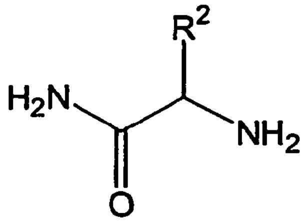

III

où $R^2$ présente la signification susmentionnée, en au moins un composé de formule générale X,

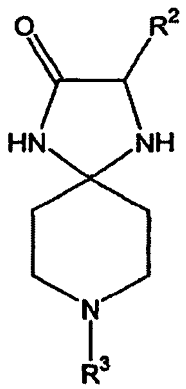

X

où $R^2$ et $R^3$ ont la signification susmentionnée, on le purifie le cas échéant et/ou on l'isole le cas échéant, et on le transforme le cas échéant par transformation avec au moins un composé de formule générale $R^1$-$X^1$, où $R^1$ et $X^1$ ont la signification susmentionnée, le cas échéant en présence d'au moins une base, en au moins un composé selon l'une ou plusieurs des revendications 1 à 13 et on le purifie et/ou on l'isole le cas échéant.

**15.** Médicament contenant au moins un composé substitué de 1,4,8-triazaspiro[4,5]décan-2-one selon l'une ou plusieurs des revendications 1 à 13 et le cas échéant un ou plusieurs adjuvants pharmaceutiquement acceptables.

**16.** Médicament selon la revendication 15 destiné à la régulation, en particulier l'inhibition, de la résorption de la noradrénaline (Noradrenalin-Uptake), destiné à la régulation, en particulier l'inhibition de la résorption du 5-hydroxytryptophane (5-HT-Uptake), destiné à la régulation du récepteur des opioïdes, en particulier la régulation du récepteur des opioïdes $\mu$ et/ou destiné à la régulation du récepteur de la batrachotoxine (BTX).

**17.** Médicament selon la revendication 15 ou 16 destiné à la prophylaxie et/ou au traitement de troubles de l'absorption alimentaire, de préférence choisis dans le groupe constitué par la boulimie, l'anorexie, l'obésité et la cachexie, de préférence l'obésité.

**18.** Médicament selon la revendication 15 ou 16 destiné à la prophylaxie et/ou au traitement de douleurs, de préférence au traitement et/ou à la prophylaxie de douleurs aiguës, de douleurs chroniques, de douleurs neuropathiques et/ou d'algies vasculaires de la face.

**19.** Médicament selon la revendication 15 ou 16 destiné à la prophylaxie et/ou au traitement de dépressions.

**20.** Médicament selon la revendication 15 ou 16 destiné à la prophylaxie et/ou au traitement combinés de dépressions et de douleurs, de préférence au traitement combiné de dépressions et de douleurs choisies dans le groupe constitué par les douleurs aiguës, les douleurs chroniques, les douleurs neuropathiques et les algies vasculaires de la face.

**21.** Médicament selon la revendication 15 ou 16 destiné à la prophylaxie et/ou au traitement de l'usage abusif d'alcool et/ou de drogues et/ou de médicaments, à la prophylaxie et/ou au traitement de la dépendance à l'alcool et/ou aux drogues et/ou aux médicaments, à la prophylaxie et/ou au traitement d'inflammations, à la prophylaxie et/ou au traitement du manque d'entrain, à la prophylaxie et/ou au traitement d'ischémies, à la prophylaxie et/ou au traitement de la catalepsie, à l'augmentation de la vigilance, à l'augmentation de la libido, à l'anxiolyse, à la prophylaxie et/ou au traitement de maladies de neurodégénérescence, de préférence d'une ou de plusieurs maladies de neurodégénérescence choisies dans le groupe constitué par la maladie de Parkinson, de Huntington, d'Alzheimer et la sclérose en plaques et/ou à l'anesthésie locale.

**22.** Utilisation d'au moins un composé substitué de 1,4,8-triazaspiro[4,5]décan-2-one selon l'une quelconque des revendications 1 à 13, y compris les composés exclus pour la préparation d'un médicament destiné à la régulation de la résorption de la noradrénaline (Noradrenalin-Uptake), de préférence à l'inhibition de la résorption de la noradrénaline (Noradrenalin-Uptake).

**23.** Utilisation d'au moins un composé substitué de 1,4,8-triazaspiro[4,5]décan-2-one selon l'une quelconque des revendications 1 à 13 y compris les composés exclus pour la préparation d'un médicament destiné à la régulation de la résorption du 5-hydroxytryptophane (5-HT-Uptake), de préférence à l'inhibition du 5-hydroxytryptophane (5-HT-Uptake).

**24.** Utilisation d'au moins un composé substitué de 1,4,8-triazaspiro[4,5]décan-2-one selon l'une quelconque des revendications 1 à 13, y compris les composés exclus pour la préparation d'un médicament destiné à la régulation du récepteur des opioïdes, de préférence la régulation du récepteur des opioïdes p.

**25.** Utilisation d'au moins un composé substitué de 1,4,8-triazaspiro[4,5]décan-2-one selon l'une quelconque des revendications 1 à 13 y compris les composés exclus pour la préparation d'un médicament destiné à la régulation du récepteur de la batrachotoxine-(BTX).

**26.** Utilisation d'au moins un composé substitué de la 1,4,8-triazaspiro[4,5]décan-2-one de formule générale I selon l'une quelconque des revendications 1 à 13, y compris les composés exclus pour la préparation d'un médicament destiné à la prophylaxie et/ou au traitement de troubles de l'absorption alimentaire, de préférence choisis dans le groupe constitué par la boulimie, l'anorexie, l'obésité et la cachexie, de manière particulièrement préférée l'obésité.

**27.** Utilisation d'au moins un composé substitué de la 1,4,8-triazaspiro[4,5]décan-2-one de formule générale I selon l'une quelconque des revendications 1 à 13, y compris les composés exclus selon B) mais à l'exception des composés selon A) pour la préparation d'un médicament destiné à la prophylaxie et/ou au traitement de douleurs.

**28.** Utilisation d'au moins un composé substitué de 1,4,8-triazaspiro[4,5]décan-2-one selon l'une quelconque des revendications 1 à 13 y compris les composés exclus pour la préparation d'un médicament destiné à la prophylaxie et/ou au traitement de douleurs aiguës, de douleurs chroniques, de douleurs neuropathiques et/ou d'algies vasculaires de la face.

**29.** Utilisation d'au moins un composé substitué de la 1,4,8-triazaspiro[4,5]décan-2-one selon l'une quelconque des revendications 1 à 13, y compris les composés exclus selon B) mais à l'exception des composés selon A) pour la préparation d'un médicament destiné à la prophylaxie et/ou au traitement de dépressions.

**30.** Utilisation d'au moins un composé substitué de 1,4,8-triazaspiro[4,5]décan-2-one selon l'une quelconque des revendications 1 à 13 y compris les composés exclus pour la prophylaxie et/ou le traitement de l'usage abusif de drogues et/ou de médicaments, la prophylaxie et/ou le traitement de la dépendance aux drogues et/ou aux médicaments, la prophylaxie et/ou le traitement de la sclérose en plaques, la prophylaxie et/ou le traitement du manque d'entrain, la prophylaxie et/ou le traitement de la catalepsie, l'augmentation de la vigilance, l'augmentation de la libido, l'anxiolyse, la prophylaxie et/ou le traitement d'ischémies et/ou l'anesthésie locale.

**31.** Utilisation d'au moins un composé substitué de la 1,4,8-triazaspiro[4,5]décan-2-one selon l'une quelconque des revendications 1 à 13, y compris les composés exclus selon B) mais à l'exception des composés selon A) pour la préparation d'un médicament destiné à la prophylaxie et/ou au traitement de l'usage abusif d'alcool, à la prophylaxie et/ou au traitement de la dépendance de l'alcool et/ou à la prophylaxie et/ou au traitement d'inflammations.

**32.** Utilisation d'au moins un composé substitué de 1,4,8-triazaspiro[4,5]décan-2-one selon l'une quelconque des revendications 1 à 13 pour la préparation d'un médicament destiné à la prophylaxie et/ou au traitement de maladies de neurodégénérescence, de préférence d'une ou de plusieurs maladies de neurodégénérescence choisies dans le groupe constitué par la maladie de Huntington, d'Alzheimer et de Parkinson.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 1207291 A


### In der Beschreibung aufgeführte Nicht-Patentliteratur


- **Y. Ishihara et al.** *Chem. Pharm. Bull.,* 1992, vol. 40 (5), 1177-1185
- **I.C. Hendershot ; J. Forsaith.** *J. Pharmacol. Exp. Ther.,* 1959, vol. 125, 237-240
- **Y. Cheng ; W.H. Prusoff.** *Biochem. Pharmacol.,* 1973, vol. 22, 3099-3108
- **E.G. Gray ; V.P. Whittaker.** The isolation of nerve endings from brain. *J. Anatomy,* 1962, vol. 96, 79-88
- **Lowry et al.** Protein measurement with the folin phenol reagent. *J. Biol. Chem.,* 1951, vol. 193, 265-275
- **M.Ch. Frink ; H.-H. Hennies ; W. Engelberger ; M. Haurand ; B. Wilffert.** *Arzneim.-Forsch./Drug Res.,* 1996, vol. 46 (III), 11, 1029-1036
- **Gray ; Whittaker.** *J. Anat.,* 1962, vol. 76, 79-88
- *Eur. J. Pharmacol.,* vol. 124, 291-298